(19) Europäisches Patentamt / European Patent Office / Office européen des brevets

(11) **EP 4 782 442 A1**

(12) **EUROPEAN PATENT APPLICATION**
published in accordance with Art. 153(4) EPC


(43) Date of publication:
**29.07.2026 Bulletin 2026/31**

(21) Application number: **24879107.1**

(22) Date of filing: **18.10.2024**

(51) International Patent Classification (IPC):
***C07D 403/12*** (2006.01) ***C07D 401/10*** (2006.01)
***C07D 405/12*** (2006.01) ***C07D 491/04*** (2006.01)
***C07D 471/04*** (2006.01) ***A61K 31/407*** (2006.01)
***A61P 35/00*** (2006.01) ***A61P 35/02*** (2006.01)
***A61K 31/506*** (2006.01) ***A61K 31/4025*** (2006.01)

(52) Cooperative Patent Classification (CPC):
**A61K 31/4025; A61K 31/407; A61K 31/506; A61P 35/00; A61P 35/02; C07D 401/10; C07D 403/12; C07D 405/12; C07D 471/04; C07D 491/04**

(86) International application number:
**PCT/CN2024/125698**

(87) International publication number:
**WO 2025/082467 (24.04.2025 Gazette 2025/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.10.2023 CN 202311355692**
**03.01.2024 CN 202410006918**
**02.02.2024 CN 202410151124**
**17.07.2024 CN 202410954855**



(71) Applicant: **Hitgen Inc.**
**Chengdu, Sichuan 610200 (CN)**

(72) Inventors:
- **LI, Jin**
  **Chengdu, Sichuan 610200 (CN)**
- **BAI, Xiaoguang**
  **Chengdu, Sichuan 610200 (CN)**
- **HONG, Xinfu**
  **Chengdu, Sichuan 610200 (CN)**
- **GONG, Yi**
  **Chengdu, Sichuan 610200 (CN)**
- **DENG, Ning**
  **Chengdu, Sichuan 610200 (CN)**
- **ZHANG, Pei**
  **Chengdu, Sichuan 610200 (CN)**
- **QU, Qingxi**
  **Chengdu, Sichuan 610200 (CN)**
- **HUANG, Xiaodong**
  **Chengdu, Sichuan 610200 (CN)**
- **ZHANG, Rui**
  **Chengdu, Sichuan 610200 (CN)**

(74) Representative: **Vitina, Maruta et al**
**Agency Tria Robit**
**Vilandes iela 5-2**
**1010 Riga (LV)**


(54) **IMMUNOMODULATORS**


(57) A compound of formula (I):

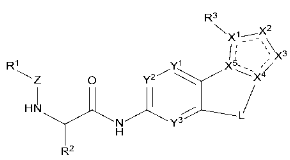

,

where X1 is C or N, $X^2$ and $X^3$ are independently O, N, $NR^X$ or $CR^X$, $X^4$ and $X^5$ are independently C or N, L is $-C_{2-6}$ alkylene or $-C_{2-6}$ alkenylene, Z is C(O), S(O) or $S(O)_2$, and $Y^1$, $Y^2$ and $Y^3$ are each independently N or $CR^Y$. This application further provides an application of the compound of formula (I) or a stereoisomer thereof in the preparation of a drug for treating a IL-17A-related disease.


EP 4 782 442 A1

## Description

### TECHNICAL FIELD

This application relates to pharmaceuticals, and more particularly to an immunomodulator and an application thereof in the preparation of a drug.

### BACKGROUND

Interleukin 17 (IL-17) is a proinflammatory cytokine that plays a role in the induction of other inflammatory cytokines, chemokine, and adhesion factor. An IL-17 family consists of cytokines that are involved in acute and chronic inflammatory responses, including interleukin 17A (IL-17A or CTLA-8), interleukin 17B (IL-17B), interleukin 17C (IL-17C), interleukin 17D (IL-17D), interleukin 17E (IL-17E or IL-25) and interleukin 17F (IL-17F). The IL-17A is expressed by T helper cell 17 (TH17), and is involved in the pathogenesis of inflammatory and autoimmune diseases. The IL-17A of human is a glycoprotein with a molecular weight of approximately 17,000 daltons. The IL-17A transmits signals into the cell through an IL-17 receptor, such as interleukin 17RA (IL-17RA) and interleukin 17RC (IL-17RC) (Wright, et al. Journal of immunology, 2008, 181: 2799-2805). A primary function of IL-17A is to coordinate local tissue inflammation through upregulation of proinflammatory and neutrophil migratory cytokines and chemokines (including IL-6, G-CSF, TNF-$\alpha$, IL-1, CXCL1, CCL2, CXCL2), and to allow activated T cells to penetrate the extracellular matrix though matrix metalloproteinases. The IL-17A has been shown to play an important role in severe asthma and chronic obstructive pulmonary disease (COPD), and those patients having severe asthma or COPD typically do not respond or respond poorly to currently available medications (Al-Ramli et al. J Allergy Clin Immunol, 2009, 123: 1185-1187). An upregulation of IL-17A is related to many diseases, such as rheumatoid arthritis (RA), bone erosion, intraperitoneal abscess, inflammatory bowel disease, allograft rejection, psoriasis, atherosclerosis, asthma, and multiple sclerosis (Gaffen, SL et al. Arthritis Research & Therapy, 2004, 6: 240-247).

The immune function is critical for maintaining homeostasis and for an effective response to disease. Moreover, aberrant immune responses are established contributing factors to the pathophysiology of autoimmune diseases. In certain disease states, some key pathways leading to these aberrant autoimmune responses have been found to be effective targets for therapeutic intervention. The IL-17A is a well-recognized proinflammatory cytokine that plays a critical role in chronic inflammation and is a major driver of tissue damage. The IL-17A induces normal immune and inflammatory responses to pathogens, but can also contribute to chronic autoimmune diseases, including psoriasis, spondyloarthritis, rheumatoid arthritis and multiple sclerosis. As a consequence, the binding of targeting IL-17A to IL-17RA is an effective strategy to treat autoimmune inflammatory diseases mediated by IL-17 A. A morbidity and severity of autoimmune encephalomyelitis can be reduced in animals through IL-17A and monoclonal antibody therapy (Komiyama Y et al. J. Immunol., 2006, 177: 566-573). The existing IL-17A antibodies have shown promising results on IL-7A-mediated inflammatory diseases, including asthma, psoriasis, rheumatoid arthritis, ankylosing spondylitis, and multiple sclerosis. The IL-17A (Cosentyx/secukinumab of Novartis) approved by the Food and Drug Administration (FDA) for the treatment of psoriasis in January 2015.

Although several IL-17A antibodies are currently on the market, known IL-17A small molecule modulators remain relatively scarce, particularly improved and/or orally bioavailable IL-17A small molecule modulators. Therefore, the development of IL-17A small molecule modulators holds significant clinical therapeutic value.

### SUMMARY

The present disclosure adopts the following technical solutions.

In a first aspect, the present disclosure provides a compound of formula (I), or a deuterated compound, a stereoisomer or a pharmaceutically acceptable salt thereof:

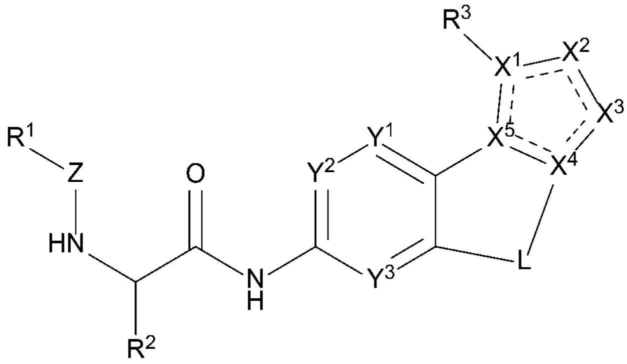

(I),

wherein $\overline{---}$ represents a single bond or a double bond;

$X^1$ is C or N;

$X^2$ and $X^3$ are independently selected from the group consisting of O, N, $NR^X$ and $CR^X$;

$X^4$ and $X^5$ are independently each C or N;

each $R^X$ is independently selected from the group consisting of hydrogen, deuterium, halogen, cyano, $-C_{1-6}$ alkyl, $-OC_{1-6}$ alkyl, $-C_{0-2}$ alkylene-(3 to 10-membered cycloalkyl), -O(3 to 10-membered cycloalkyl), $-C_{0-2}$ alkylene-(4 to 10-membered heterocycloalkyl), -O(4 to 10-membered heterocycloalkyl), deuterium-substituted $-C_{1-6}$ alkyl and halogen-substituted $-C_{1-6}$ alkyl;

$Y^1$, $Y^2$ and $Y^3$ are independently each N or $CR^Y$;

each $R^Y$ is independently selected from the group consisting of hydrogen, deuterium, halogen, cyano, $-C_{1-6}$ alkyl, deuterium-substituted $-C_{1-6}$ alkyl, halogen-substituted $-C_{1-6}$ alkyl, $-C_{0-2}$ alkylene-(3 to 10-membered cycloalkyl), $-C_{0-2}$ alkylene-(4 to 10-membered heterocycloalkyl), $-C_{0-2}$ alkylene-$OC_{1-6}$ alkyl, $-C_{0-2}$ alkylene-O(halogen-substituted $-C_{1-6}$ alkyl), $-C_{0-2}$ alkylene-O(3 to 10-membered cycloalkyl) and $-C_{0-2}$ alkylene-O(4 to 10-membered heterocycloalkyl);

Z is C(O), S(O) or $S(O)_2$;

$R^1$ is selected from the group consisting of $-C_{1-6}$ alkyl, deuterium-substituted $-C_{1-6}$ alkyl, halogen-substituted $-C_{1-6}$ alkyl, $-C_{2-6}$ alkenyl, halogen-substituted $-C_{2-6}$ alkenyl, $-C_{2-6}$ alkynyl, halogen-substituted $-C_{2-6}$ alkynyl, $-C_{0-2}$ alkylene-(3 to 10-membered cycloalkyl), $-C_{0-2}$ alkylene-(4 to 10-membered heterocycloalkyl), $-C_{0-2}$ alkylene-(5 to 10-membered aromatic ring), $-C_{0-2}$ alkylene-(5 to 10-membered heteroaromatic ring), - $C_{0-2}$ alkylene-$OR^{11}$ and $-C_{0-2}$ alkylene-$NR^{11}R^{12}$, wherein alkylene, alkyl, alkenyl, alkynyl, cycloalkyl, heterocycloalkyl, aromatic ring and heteroaromatic ring are unsubstituted or substituted by one, two or three $R^{1a}$;

$R^{11}$ and $R^{12}$ are independently selected from the group consisting of hydrogen, deuterium, $-C_{1-6}$ alkyl, deuterium-substituted $-C_{1-6}$ alkyl, halogen-substituted $-C_{1-6}$ alkyl, $-C_{0-2}$ alkylene-(3 to 10-membered cycloalkyl), $-C_{0-2}$ alkylene-(4 to 10-membered heterocycloalkyl), $-C_{0-2}$ alkylene-(5 to 10-membered aromatic ring) and $-C_{0-2}$ alkylene-(5 to 10-membered heteroaromatic ring), wherein alkyl, alkylene, cycloalkyl, heterocycloalkyl, aromatic ring and heteroaromatic ring are unsubstituted or substituted by one, two or three $R^{1a}$;

each $R^{1a}$ is independently selected from the group consisting of hydrogen, deuterium, halogen, cyano, $-C_{1-6}$ alkyl, deuterium-substituted $-C_{1-6}$ alkyl, halogen-substituted $-C_{1-6}$ alkyl, $-C_{0-4}$ alkylene-$OR^{1b}$, $-C_{0-4}$ alkylene-$SR^{1b}$, $-C_{0-2}$ alkylene-$C(O)R^{1b}$, $-C_{0-2}$ alkylene-$C(O)NR^{1b}R^{1c}$, $-C_{0-4}$ alkylene-$NR^{1b}R^{1c}$, $-C_{0-2}$ alkylene-$NR^{1b}C(O)R^{1c}$, $-C_{0-4}$ alkylene-$S(O)_2R^{1b}$, $-C_{0-4}$ alkylene-$NHS(O)_2R^{1b}$, $-C_{0-4}$ alkylene-$S(O)R^{1b}$, $-C_{0-4}$ alkylene-(3 to 10-membered cycloalkyl), $-C_{0-4}$ alkylene-(4 to 10-membered heterocycloalkyl), - $C_{0-4}$ alkylene-(5 to 10-membered aromatic ring) and $-C_{0-4}$ alkylene-(5 to 10-membered heteroaromatic ring), wherein alkyl, alkylene, cycloalkyl, heterocycloalkyl, aromatic ring and heteroaromatic ring areunsubstituted or substituted by one, two or three $R^{1d}$; or

two $R^{1a}$ linked to the same carbon atom form 3 to 10-membered cycloalkyl or 4 to 10-membered heterocycloalkyl with the carbon atom, wherein cycloalkyl and heterocycloalkyl are unsubstituted or substituted by one, two or three $R^{1e}$;

$R^{1b}$, $R^{1c}$ and $R^{1d}$ are independently selected from the group consisting of hydrogen, deuterium, $-C_{1-6}$ alkyl, deuterium-substituted $-C_{1-6}$ alkyl, halogen-substituted $-C_{1-6}$ alkyl, halogen, cyano, =O, =S, nitro, -OH, -SH, $-S(C_{1-6}$ alkyl), $-C_{0-2}$ alkylene-$O(C_{1-6}$ alkyl), $-C(O)NH_2$, $-NH_2$, $-NH(C_{1-6}$ alkyl), $-N(C_{1-6}$ alkyl)$(C_{1-6}$ alkyl), $-C_{0-2}$ alkylene-(3 to 10-membered cycloalkyl), $-C_{0-2}$ alkylene-(4 to 10-membered heterocycloalkyl), $-C_{0-2}$ alkylene-(5 to 10-membered aromatic ring) and $-C_{0-2}$ alkylene-(5 to 10-membered heteroaromatic ring);

each $R^{1e}$ is independently selected from the group consisting of hydrogen, deuterium, - $C_{1-6}$ alkyl, deuterium-substituted $-C_{1-6}$ alkyl, halogen-substituted $-C_{1-6}$ alkyl, halogen, cyano, =O, =S, nitro, -OH, -SH, $-S(C_{1-6}$ alkyl), $-C_{0-2}$ alkylene-$O(C_{1-6}$ alkyl), $-C(O)NH_2$, $-NH_2$, $-NH(C_{1-6}$ alkyl), $-N(C_{1-6}$ alkyl)$(C_{1-6}$ alkyl), $-C_{0-2}$ alkylene-(3 to 10-membered

cycloalkyl), -$C_{0-2}$ alkylene-(4 to 10-membered heterocycloalkyl), -$C_{0-2}$ alkylene-(6 to 10-membered aromatic ring) and -$C_{0-2}$ alkylene-(5 to 10-membered heteroaromatic ring);

$R^2$ is selected from the group consisting of -$C_{0-2}$ alkylene-(3 to 10-membered cycloalkyl), -$C_{0-2}$ alkylene-(4 to 10-membered heterocycloalkyl), -$C_{0-2}$ alkylene-(5 to 12-membered bridged cycloalkyl), -$C_{0-2}$ alkylene-(5 to 12-membered bridged heterocycloalkyl), -$C_{0-2}$ alkylene-(5 to 12-membered spiro cycloalkyl), -$C_{0-2}$ alkylene-(5 to 12-membered spiro heterocycloalkyl), -$C_{0-2}$ alkylene-(5 to 10-membered aromatic ring), -$C_{0-2}$ alkylene-(5 to 10-membered heteroaromatic ring), -$C_{0-2}$ alkylene-$OR^{21}$, -$C_{0-2}$ alkylene-$NR^{21}R^{22}$ and -$C_{0-2}$ alkylene-CN, wherein alkylene, cycloalkyl, heterocycloalkyl, bridged cycloalkyl, bridged heterocycloalkyl, spiro cycloalkyl, spiro heterocycloalkyl, aromatic ring and heteroaromatic ring are unsubstituted or substituted by one, two, three or four $R^{2a}$;

$R^{21}$ and $R^{22}$ are independently selected from the group consisting of hydrogen, deuterium, -$C_{1-6}$ alkyl, deuterium-substituted -$C_{1-6}$ alkyl, halogen-substituted -$C_{1-6}$ alkyl, -$C_{0-2}$ alkylene-(3 to 10-membered cycloalkyl), -$C_{0-2}$ alkylene-(4 to 10-membered heterocycloalkyl), -$C_{0-2}$ alkylene-(5 to 10-membered aromatic ring) and -$C_{0-2}$ alkylene-(5 to 10-membered heteroaromatic ring), wherein alkyl, alkylene, cycloalkyl, heterocycloalkyl, aromatic ring and heteroaromatic ring are unsubstituted or substituted by one, two or three $R^{2a}$; and

each $R^{2a}$ is independently selected from the group consisting of hydrogen, deuterium, halogen, cyano, =O, =S, nitro, -$C_{1-6}$ alkyl, deuterium-substituted -$C_{1-6}$ alkyl, halogen-substituted -$C_{1-6}$ alkyl, -$C_{0-2}$ alkylene-$OR^{2b}$, -$C_{0-2}$ alkylene-$C(O)R^{2b}$, -$C_{0-2}$ alkylene-$C(O)NR^{2b}R^{2c}$, -$C_{0-2}$ alkylene-$NR^{2b}R^{2c}$, -$C_{0-2}$ alkylene-$NR^{2b}C(O)R^{2c}$, -$C_{0-4}$ alkylene-$S(O)_2R^{2b}$, -$C_{0-4}$ alkylene-CN, -$C_{0-2}$ alkylene-(3 to 10-membered cycloalkyl), -$C_{0-2}$ alkylene-(4 to 10-membered heterocycloalkyl), -$C_{0-2}$ alkylene-(5 to 12-membered bridged cycloalkyl), -$C_{0-2}$ alkylene-(5 to 12-membered bridged heterocycloalkyl), -$C_{0-2}$ alkylene-(5 to 12-membered spiro cycloalkyl), -$C_{0-2}$ alkylene-(5 to 12-membered spiro heterocycloalkyl), -$C_{0-2}$ alkylene-(5 to 10-membered aromatic ring) and -$C_{0-2}$ alkylene-(5 to 10-membered heteroaromatic ring), wherein alkyl, alkylene, cycloalkyl, heterocycloalkyl, bridged cycloalkyl, bridged heterocycloalkyl, spiro cycloalkyl, spiro heterocycloalkyl, aromatic ring and heteroaromatic ring are unsubstituted or substituted by one, two or three $R^{2d}$; or two $R^{2a}$ linked to the same carbon atom form 3 to 10-membered cycloalkyl or 4 to 10-membered heterocycloalkyl with the carbon atom;

$R^{2b}$ and $R^{2C}$ are independently selected from the group consisting of hydrogen, deuterium, -$C_{1-6}$ alkyl, deuterium-substituted -$C_{1-6}$ alkyl, halogen-substituted -$C_{1-6}$ alkyl, -$C_{0-2}$ alkylene-(3 to 10-membered cycloalkyl), -$C_{0-2}$ alkylene-(4 to 10-membered heterocycloalkyl), -$C_{0-2}$ alkylene-(5 to 10-membered aromatic ring) and -$C_{0-2}$ alkylene-(5 to 10-membered heteroaromatic ring), wherein alkyl, alkylene, cycloalkyl, heterocycloalkyl, aromatic ring and heteroaromatic ring are unsubstituted or substituted by one, two or three $R^{2d}$;

each $R^{2d}$ is independently selected from the group consisting of hydrogen, deuterium, - $C_{1-6}$ alkyl, deuterium-substituted -$C_{1-6}$ alkyl, halogen-substituted -$C_{1-6}$ alkyl, halogen, cyano, =O, =S, nitro, -OH, -O($C_{1-6}$ alkyl), -$NH_2$, -NH($C_{1-6}$ alkyl) and -N($C_{1-6}$ alkyl)($C_{1-6}$ alkyl);

$R^3$ is selected from the group consisting of hydrogen, deuterium, halogen, cyano, -$C_{1-6}$ alkyl, deuterium-substituted -$C_{1-6}$ alkyl, halogen-substituted -$C_{1-6}$ alkyl, -$C_{0-2}$ alkylene-(3 to 10-membered cycloalkyl), -$C_{0-2}$ alkylene-(4 to 10-membered heterocycloalkyl), - $C_{0-2}$ alkylene-(5 to 10-membered aromatic ring), -$C_{0-2}$ alkylene-(5 to 10-membered heteroaromatic ring), -$C_{0-2}$ alkylene-$OR^{31}$ and -$C_{0-2}$ alkylene-$NR^{31}R^{32}$, wherein alkylene, cycloalkyl, heterocycloalkyl, aromatic ring and heteroaromatic ring are unsubstituted or substituted by one, two or three $R^{3a}$;

$R^{31}$ and $R^{32}$ are independently selected from the group consisting of hydrogen, deuterium, -$C_{1-6}$ alkyl, deuterium-substituted -$C_{1-6}$ alkyl, halogen-substituted -$C_{1-6}$ alkyl, -$C_{0-2}$ alkylene-(3 to 10-membered cycloalkyl), -$C_{0-2}$ alkylene-(4 to 10-membered heterocycloalkyl), -$C_{0-2}$ alkylene-(5 to 10-membered aromatic ring) and -$C_{0-2}$ alkylene-(5 to 10-membered heteroaromatic ring);

each $R^{3a}$ is independently selected from the group consisting of hydrogen, deuterium, halogen, cyano, =O, =S, nitro, -$C_{1-6}$ alkyl, deuterium-substituted -$C_{1-6}$ alkyl and halogen-substituted -$C_{1-6}$ alkyl;

L is -$C_{2-6}$ alkylene or -$C_{2-6}$ alkenylene, wherein one, two or three methylene groups in -$C_{2-6}$ alkylene and -$C_{2-6}$ alkenylene are unsubstituted or substituted by O, $NR^{L1}$, C(O), S(O) or $S(O)_2$, or -$C_{2-6}$ alkylene and -$C_{2-6}$ alkenylene are unsubstituted or substituted by one, two, three or four $R^L$;

each $R^{L1}$ is selected from the group consisting of hydrogen, deuterium, -$C_{1-6}$ alkyl, deuterium-substituted -$C_{1-6}$ alkyl, halogen-substituted -$C_{1-6}$ alkyl, -$C_{0-2}$ alkylene-$OC_{1-6}$ alkyl, -$C_{0-2}$ alkylene-CN, -$C_{0-2}$ alkylene-C(O)($C_{1-6}$ alkyl), -$C_{0-2}$ alkylene-O(3 to 10-membered cycloalkyl), -$C_{0-2}$ alkylene-O(4 to 10-membered heterocycloalkyl), -$C_{0-2}$ alkylene-(3 to 10-membered cycloalkyl), -$C_{0-2}$ alkylene-(3 to 10-membered heterocycloalkyl), -$C_{0-2}$ alkylene-(5 to 10-membered aromatic ring), -$C_{0-2}$ alkylene-(5 to 10-membered heteroaromatic ring) and -$C_{0-4}$ alkylene-N($C_{1-6}$ alkyl)($C_{1-6}$ alkyl), wherein alkyl, alkylene, cycloalkyl, heterocycloalkyl, aromatic ring and heteroaromatic ring are unsubstituted or substituted by one, two or three $R^{L2}$;

each $R^{L2}$ is independently selected from the group consisting of hydrogen, deuterium, halogen, cyano, =O, =S, nitro, -$C_{1-6}$ alkyl, deuterium-substituted -$C_{1-6}$ alkyl and halogen-substituted -$C_{1-6}$ alkyl; and

each $R^L$ is independently selected from the group consisting of hydrogen, deuterium, halogen, cyano, =O, =S, nitro, -$C_{1-6}$ alkyl, deuterium-substituted -$C_{1-6}$ alkyl, halogen-substituted -$C_{1-6}$ alkyl, -$C_{0-2}$ alkylene-$OC_{1-6}$ alkyl, -$C_{0-2}$

alkylene-O(3 to 10-membered cycloalkyl) and -$C_{0-2}$ alkylene-O(4 to 10-membered heterocycloalkyl); or two $R^L$ linked to the same carbon atom form 3 to 10-membered cycloalkyl or 4 to 10-membered heterocycloalkyl with the carbon atom.

In some embodiments, the compound is represented by formula (II):

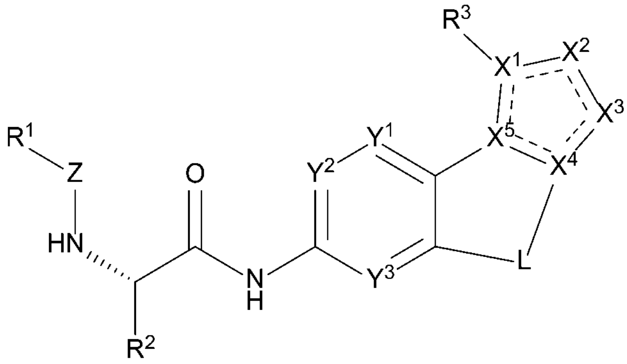

(II);

wherein $R^1$, $R^2$, $R^3$, $Y^1$, $Y^2$, $Y^3$, Z, L, $X^1$, $X^2$, $X^3$, $X^4$ and $X^5$ are as defined before.

In some embodiments, a ring consisted of $X^1$, $X^2$, $X^3$, $X^4$ and $X^5$ is a heteroaromatic ring, wherein $X^4$ and $X^5$ are not N at the same time.

In some embodiments, $X^1$ is C, $X^2$ is $NR^X$, $X^3$ is N, $X^4$ is C and $X^5$ is C;

$X^1$ is C, $X^2$ is N, $X^3$ is N, $X^4$ is C and $X^5$ is N;
$X^1$ is C, $X^2$ is N, $X^3$ is $CR^X$, $X^4$ is N and $X^5$ is C;
$X^1$ is N, $X^2$ is N, $X^3$ is $CR^X$, $X^4$ is C and $X^5$ is C; or
$X^1$ is C, $X^2$ is O, $X^3$ is N, $X^4$ is C and $X^5$ is C.

In some embodiments, the compound is selected from the group consisting of:

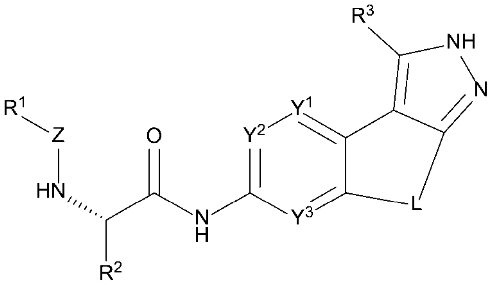

,

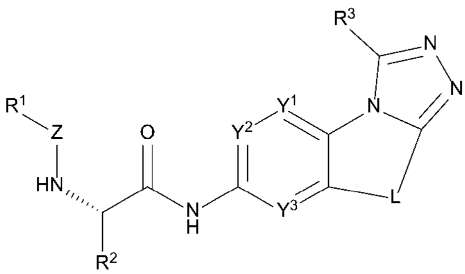

,

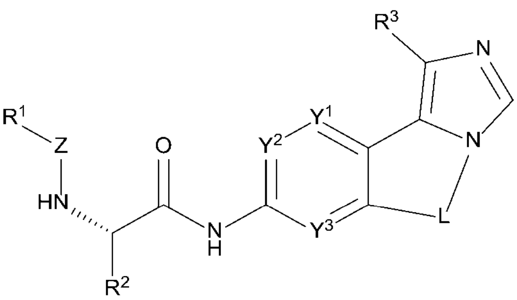

,

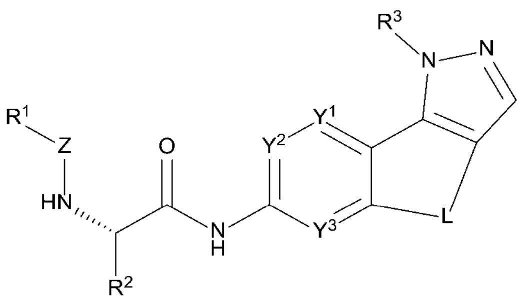

and

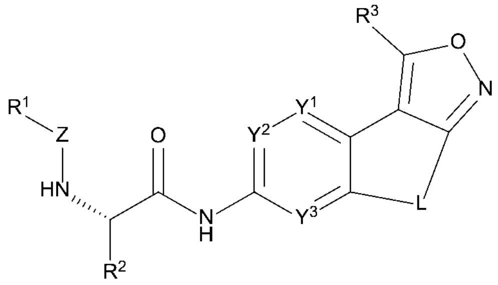

;

wherein $R^1$, $R^2$, $R^3$, $Y^1$, $Y^2$, $Y^3$, Z and L are as defined before.

In some embodiments, the compound is represented by formula (III):

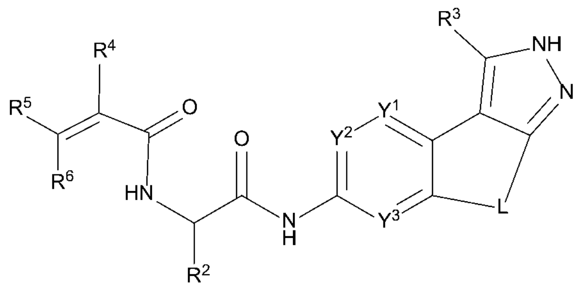

(III);

wherein $R^4$, $R^5$ and $R^6$ are independently selected from the group consisting of hydrogen, deuterium, halogen, cyano, $-C_{1-6}$ alkyl, deuterium-substituted $-C_{1-6}$ alkyl, halogen-substituted $-C_{1-6}$ alkyl, $-C_{0-4}$ alkylene-$OR^{1b}$, $-C_{0-4}$ alkylene-$SR^{1b}$, $-C_{0-2}$ alkylene-$C(O)R^{1b}$, $-C_{0-2}$ alkylene-$C(O)NR^{1b}R^{1c}$, $-C_{0-4}$ alkylene-$NR^{1b}R^{1c}$, $-C_{0-2}$ alkylene-$NR^{1b}C(O)R^{1c}$, $-C_{0-4}$ alkylene-$S(O)_2R^{1b}$, $-C_{0-4}$ alkylene-$NHS(O)_2R^{1b}$, $-C_{0-4}$ alkyleneS(O)$R^{1b}$, $-C_{0-4}$ alkylene-(3 to 10-membered cycloalkyl), $-C_{0-4}$ alkylene-(4 to 10-membered heterocycloalkyl), $-C_{0-4}$ alkylene-(6 to 10-membered aromatic ring) and $-C_{0-4}$ alkylene-(5 to 10-membered heteroaromatic ring), wherein alkyl, alkylene, cycloalkyl, heterocycloalkyl, aromatic ring and heteroaromatic ring are unsubstituted or substituted by one, two or three $R^{1d}$; and

$R^{1b}$, $R^{1c}$ and $R^{1d}$ are independently selected from the group consisting of hydrogen, deuterium, $-C_{1-6}$ alkyl, deuterium-substituted $-C_{1-6}$ alkyl, halogen-substituted $-C_{1-6}$ alkyl, halogen, cyano, =O, =S, nitro, -OH, -SH, $-S(C_{1-6}$ alkyl), $-C_{0-2}$ alkylene-$O(C_{1-6}$ alkyl), $-C(O)NH_2$, $-NH_2$, $-NH(C_{1-6}$ alkyl), $-N(C_{1-6}$ alkyl)$(C_{1-6}$ alkyl), $-C_{0-2}$ alkylene-(3 to 10-membered cycloalkyl), $-C_{0-2}$ alkylene-(4 to 10-membered heterocycloalkyl), $-C_{0-2}$ alkylene-(6 to 10-membered aromatic ring) and $-C_{0-2}$ alkylene-(5 to 10-membered heteroaromatic ring); or $R^5$ and $R^6$ linked to the same carbon atom form 3 to 10-membered cycloalkyl or 4 to 10-membered heterocycloalkyl with the carbon atom, wherein cycloalkyl and heterocycloalkyl are unsubstituted or substituted by one, two or three $R^{1e}$;

each $R^{1e}$ is independently selected from the group consisting of hydrogen, deuterium, - $C_{1-6}$ alkyl, deuterium-substituted $-C_{1-6}$ alkyl, halogen-substituted $-C_{1-6}$ alkyl, halogen, cyano, =O, =S, nitro, -OH, -SH, $-S(C_{1-6}$ alkyl), $-C_{0-2}$ alkylene-$O(C_{1-6}$ alkyl), $-C(O)NH_2$, $-NH_2$, $-NH(C_{1-6}$ alkyl), $-N(C_{1-6}$ alkyl)$(C_{1-6}$ alkyl), $-C_{0-2}$ alkylene-(3 to 10-membered cycloalkyl), $-C_{0-2}$ alkylene-(4 to 10-membered heterocycloalkyl), $-C_{0-2}$ alkylene-(6 to 10-membered aromatic ring) and $-C_{0-2}$ alkylene-(5 to 10-membered heteroaromatic ring); and

$R^2$, $R^3$, $Y^1$, $Y^2$, $Y^3$ and L are as defined before.

In some embodiments, $R^4$ is selected from the group consisting of hydrogen, deuterium, halogen, $-C_{1-3}$ alkyl, deuterium-substituted $-C_{1-3}$ alkyl and halogen-substituted $-C_{1-3}$ alkyl;

$R^5$ and $R^6$ are independently selected from the group consisting of hydrogen, deuterium, halogen, $-C_{1-3}$ alkyl, deuterium-substituted $-C_{1-3}$ alkyl, halogen-substituted $-C_{1-3}$ alkyl, 3-membered cycloalkyl, 4-membered cycloalkyl, 5-membered cycloalkyl, 6-membered cycloalkyl, 5-membered heterocycloalkyl, 6-membered heterocycloalkyl, 6-membered aromatic ring, 5-membered heteroaromatic ring and 6-membered heteroaromatic ring, wherein alkyl, alkylene, cycloalkyl, heterocycloalkyl, aromatic ring and heteroaromatic ring are unsubstituted or substituted by one, two or three $R1^d$ ; and

each $R^{1d}$ is independently selected from the group consisting of hydrogen, deuterium, - $C_{1-6}$ alkyl, deuterium-substituted $-C_{1-6}$ alkyl, halogen-substituted $-C_{1-6}$ alkyl, halogen and cyano; or $R^5$ and $R^6$ linked to the same carbon

atom form 3-membered cycloalkyl, 4-membered cycloalkyl, 5-membered cycloalkyl, 6-membered cycloalkyl, 5-membered heterocycloalkyl or 6-membered heterocycloalkyl with the carbon atom, wherein cycloalkyl and heterocycloalkyl are unsubstituted or substituted by one, two or three $R^{1e}$.

In some embodiments, $Y^1$, $Y^2$ and $Y^3$ are independently N or $CR^Y$; and each $R^Y$ is independently selected from the group consisting of hydrogen, deuterium, halogen, cyano, $-C_{1-3}$ alkyl, 3 to 6-membered cycloalkyl, deuterium-substituted $-C_{1-3}$ alkyl, halogen-substituted $-C_{1-3}$ alkyl, $-C_{0-2}$ alkylene-$OC_{1-3}$ alkyl and $-C_{0-2}$ alkylene-O(halogen-substituted $-C_{1-3}$ alkyl).

In some embodiments, $Y^1$, $Y^2$ and $Y^3$ are independently N or $CR^Y$; and each $R^Y$ is independently selected from the group consisting of hydrogen, deuterium, fluoro, chloro, bromo, cyano, methyl, ethyl, n-propyl, isopropyl, cyclopropyl, trifluoromethyl, methoxy, ethoxy and trifluoromethoxy.

In some embodiments, $Y^1$ and $Y^3$ are independently N, CH or CF;

$Y^2$ is N or $CR^Y$; and
each $R^Y$ is independently selected from the group consisting of hydrogen, deuterium, fluoro, chloro, bromo, cyano, methyl, ethyl, n-propyl, isopropyl, cyclopropyl, trifluoromethyl, methoxy, ethoxy and trifluoromethoxy.

In some embodiments, Z is C(O) or $S(O)_2$.

In some embodiments, $R^3$ is selected from the group consisting of methyl, deuterated methyl, ethyl, perdeuterated ethyl, n-propyl, isopropyl, trifluoromethyl, cyclopropyl, cyclobutyl, hydroxymethyl, methoxymethyl, deuterated methoxymethyl, amino, methylamino, dimethylamino and deuterated methylamino.

In some embodiments, $R^1$ is selected from the group consisting of benzene ring, 5-membered heteroaromatic ring and 6-membered heteroaromatic ring, wherein benzene ring and heteroaromatic ring are unsubstituted or substituted by one, two or three $R^{1a}$. In some embodiments, $R^1$ is selected from the group consisting of -methylene-benzene ring, -methylene-5-membered heteroaromatic ring, -methylene-6-membered heteroaromatic ring and -methylene-9-membered heteroaromatic ring, wherein methylene, benzene ring and heteroaromatic ring are unsubstituted or substituted by one, two or three $R^{1a}$.

In some embodiments, $R^1$ is selected from the group consisting of -difluoromethylene-benzene ring, -difluoromethylene-5-membered heteroaromatic ring, - difluoromethylene-6-membered heteroaromatic ring and -difluoromethylene-9-membered heteroaromatic ring, wherein benzene ring and heteroaromatic ring are unsubstituted or substituted by one $R^{1a}$.

In some embodiments, $R^1$ is selected from the group consisting of 8-membered fused heterocycloalkyl, 9-membered fused heterocycloalkyl, 10-membered fused heterocycloalkyl, 9-membered fused heteroaromatic ring and 10-membered fused heteroaromatic ring, wherein fused heterocycloalkyl and fused heteroaromatic ring are unsubstituted or substituted by one, two or three $R^{1a}$.

In some embodiments, $R^1$ is selected from the group consisting of 3-membered cycloalkyl, 4-membered cycloalkyl, 5-membered cycloalkyl, 6-membered cycloalkyl, 4-membered heterocycloalkyl, 5-membered heterocycloalkyl and 6-membered heterocycloalkyl, wherein cycloalkyl and heterocycloalkyl are unsubstituted or substituted by one, two or three $R^{1a}$.

In some embodiments, $R^1$ is selected from the group consisting of -methylene-3-membered cycloalkyl, -methylene-4-membered cycloalkyl, -methylene-5-membered cycloalkyl, -methylene-6-membered cycloalkyl, -methylene-4-membered heterocycloalkyl, -methylene-5-membered heterocycloalkyl, -methylene-6-membered heterocycloalkyl and -methylene-9-membered heterocycloalkyl, wherein methylene, cycloalkyl and heterocycloalkyl are unsubstituted or substituted by one, two or three $R^{1a}$.

In some embodiments, $R^1$ is selected from the group consisting of -difluoromethylene-3-membered cycloalkyl, -difluoromethylene-4-membered cycloalkyl, - difluoromethylene-5-membered cycloalkyl, -difluoromethylene-6-membered cycloalkyl, -difluoromethylene-4-membered heterocycloalkyl, -difluoromethylene-5-membered heterocycloalkyl, -difluoromethylene-6-membered heterocycloalkyl and - difluoromethylene-9-membered heterocycloalkyl, wherein cycloalkyl and heterocycloalkyl are unsubstituted or substituted by one $R^{1a}$.

In some embodiments, $R^1$ is selected from the group consisting of methyl, difluoromethyl, trifluoromethyl, ethyl, fluoroethyl, difluoroethyl, propyl, butyl, $-C_{0-2}$ alkylene-$OR^{11}$, $-C_{0-2}$ alkylene-$NR^{11}R^{12}$, vinyl, propenyl, butenyl, pentenyl and hexenyl, wherein methyl, ethyl, propyl, butyl, vinyl, propenyl, butenyl, pentenyl and hexenyl are unsubstituted or substituted by one, two or three $R^{1a}$.

In some embodiments, $R^{11}$ and $R^{12}$ are independently selected from the group consisting of hydrogen, deuterium, methyl, ethyl, n-propyl, isopropyl, deuterium-substituted methyl, deuterium-substituted ethyl, deuterium-substituted n-propyl, deuterium-substituted isopropyl, halogen-substituted methyl, halogen-substituted ethyl, halogen-substituted n-propyl and halogen-substituted isopropyl.

In some embodiments, $R^{11}$ and $R^{12}$ are independently selected from the group consisting of $-C_{0-2}$ alkylene-(3-

membered cycloalkyl), -$C_{0-2}$ alkylene-(4-membered cycloalkyl), - $C_{0-2}$ alkylene-(5-membered cycloalkyl), -$C_{0-2}$ alkylene-(6-membered cycloalkyl), -$C_{0-2}$ alkylene-(4-membered heterocycloalkyl), -$C_{0-2}$ alkylene-(5-membered heterocycloalkyl) and -$C_{0-2}$ alkylene-(6-membered heterocycloalkyl), wherein alkyl, alkylene, cycloalkyl and heterocycloalkyl are unsubstituted or substituted by one, two or three $R^{1a}$.

In some embodiments, each $R^{1a}$ is independently selected from the group consisting of hydrogen, deuterium, halogen, cyano, =O, =S, nitro, methyl, ethyl, n-propyl, isopropyl, butyl, deuterium-substituted methyl, deuterium-substituted ethyl, deuterium-substituted n-propyl, deuterium-substituted isopropyl, halogen-substituted methyl, halogen-substituted ethyl, halogen-substituted n-propyl, halogen-substituted isopropyl, and halogen-substituted butyl, wherein methyl, ethyl, n-propyl, isopropyl and butyl are unsubstituted or substituted by one, two or three $R^{1d}$.

In some embodiments, each $R^{1a}$ is independently selected from the group consisting of -$C_{0-2}$ alkylene-(3-membered cycloalkyl), -$C_{0-2}$ alkylene-(4-membered cycloalkyl), -$C_{0-2}$ alkylene-(5-membered cycloalkyl), -$C_{0-2}$ alkylene-(6-membered cycloalkyl), -$C_{0-2}$ alkylene-(4-membered heterocycloalkyl), -$C_{0-2}$ alkylene-(5-membered heterocycloalkyl), -$C_{0-3}$ alkylene-(6-membered heterocycloalkyl) and -(6-membered aromatic ring), wherein alkylene, cycloalkyl, heterocycloalkyl and aromatic ring are unsubstituted or substituted by one, two or three $R^{1d}$.

In some embodiments, each $R^{1a}$ is independently selected from the group consisting of -$C_{0-4}$ alkylene-$OR^{1b}$, -$C_{0-4}$ alkylene-$SR^{1b}$, -$C_{0-4}$ alkylene-$S(O)_2R^{1b}$, -$C_{0-4}$ alkyleneS(O)$R^{1b}$, -NHS$(O)_2R^{1b}$ and -$C_{0-3}$ alkylene-$NR^{1b}R^{1c}$, wherein alkylene is unsubstituted or substituted by one, two or three independent $R^{1d}$.

In some embodiments, $R^{1b}$, $R^{1c}$ and $R^{1d}$ are independently selected from the group consisting of hydrogen, deuterium, methyl, ethyl, n-propyl, isopropyl, butyl, deuterium-substituted methyl, deuterium-substituted ethyl, deuterium-substituted n-propyl, deuterium-substituted isopropyl, halogen-substituted methyl, halogen-substituted ethyl, halogen-substituted n-propyl, halogen-substituted isopropyl, halogen, cyano, =O, =S, nitro, -OH, -O($C_{1-6}$ alkyl), -$CH_2$-O($C_{1-6}$ alkyl), -$NH_2$, -NH($C_{1-6}$ alkyl), -N($C_{1-6}$ alkyl)($C_{1-6}$ alkyl), -(3-membered cycloalkyl), -(4-membered cycloalkyl), -(5-membered cycloalkyl), -(6-membered cycloalkyl) and -C(O)$NH_2$; or two $R^{1a}$ linked to the same carbon atom form 3-membered cycloalkyl, 4-membered cycloalkyl, 5-membered cycloalkyl, 6-membered cycloalkyl, 5-membered heterocycloalkyl and 6-membered heterocycloalkyl with the carbon atom, wherein cycloalkyl and heterocycloalkyl are unsubstituted or substituted by one, two or three independent $R^{1e}$; and

$R^{1e}$ is independently selected from the group consisting of hydrogen, deuterium, -$C_{1-3}$ alkyl, deuterium-substituted -$C_{1-3}$ alkyl, halogen-substituted -$C_{1-3}$ alkyl and halogen. In some embodiments, $R^1$ is selected from the group consisting of:

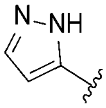, 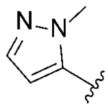, 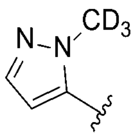, 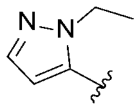, 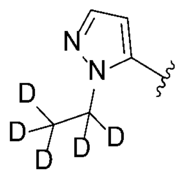, 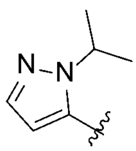, 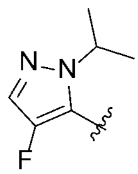,

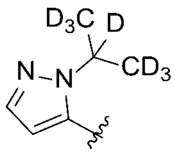, 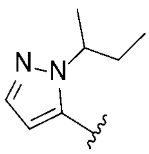, 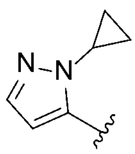, 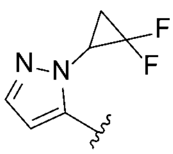, 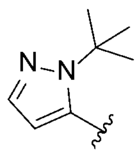, 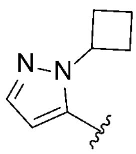,

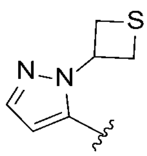, 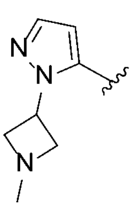, 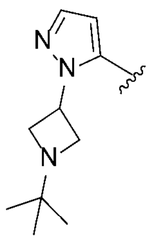, 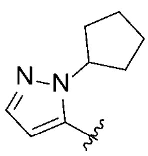, 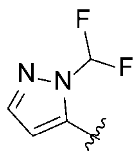, 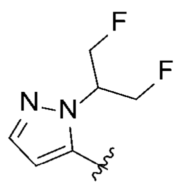,

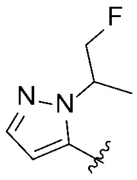 , 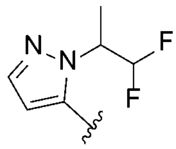 , 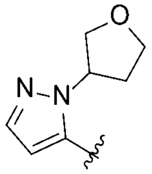 , 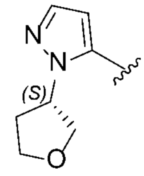 , 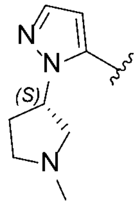 , 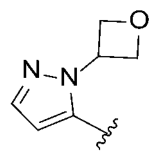 ,

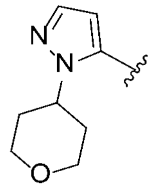 , 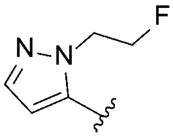 , 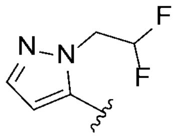 , 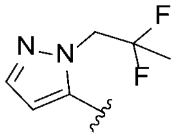 , 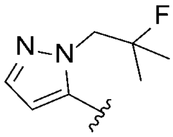 ,

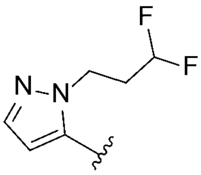 , 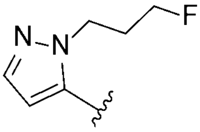 , 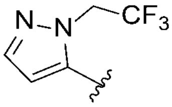 , 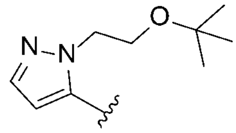 ,

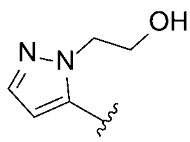 , 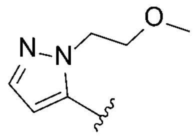 , 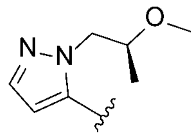 , 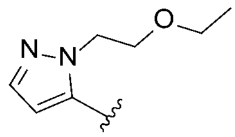 ,

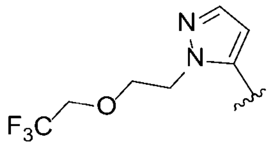 , 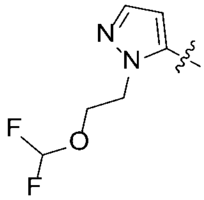 , 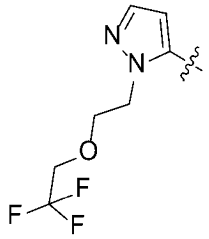 , 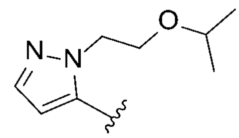 ,

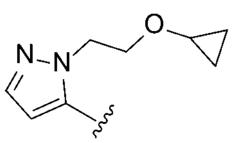 , 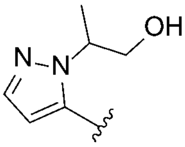 , 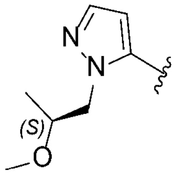 , 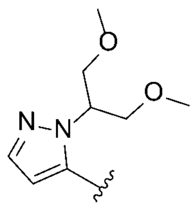 , 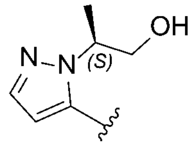 ,

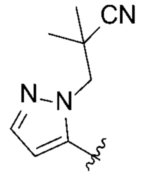 , 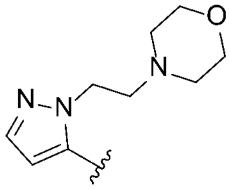 , 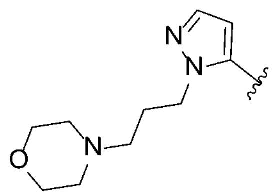 , 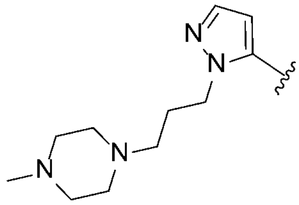 ,

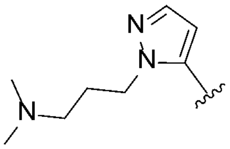, 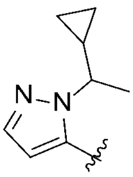, 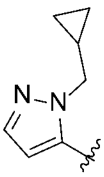, 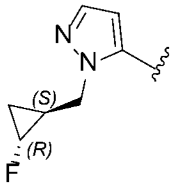, 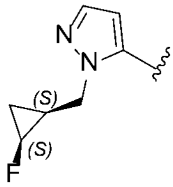, 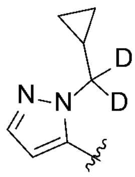,

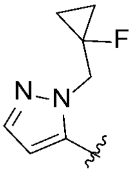, 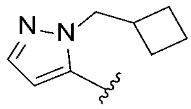, 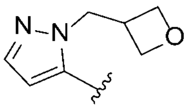, 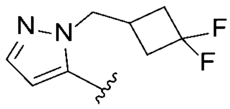, 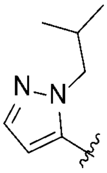, 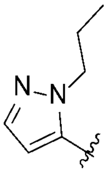,

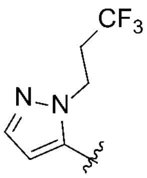, 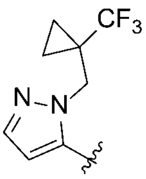, 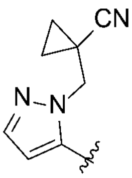, 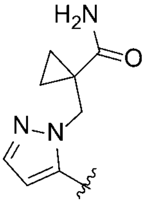, 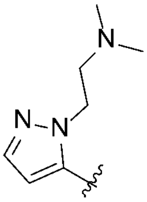, 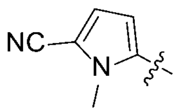,

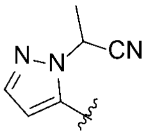, 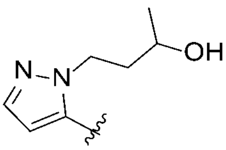, 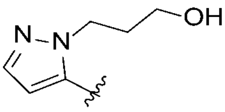, 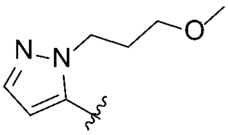, 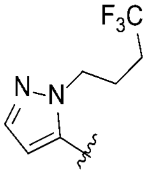,

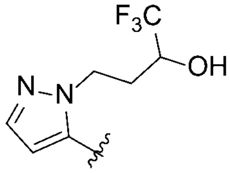, 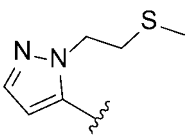, 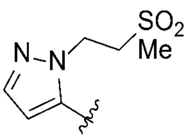, 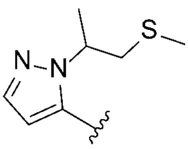, 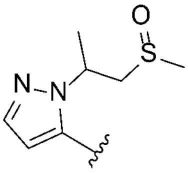,

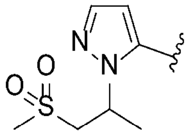, 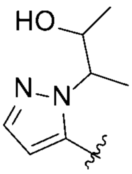, 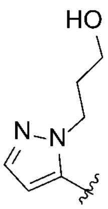, 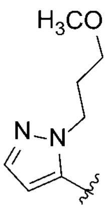, 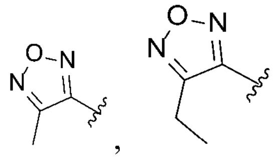, 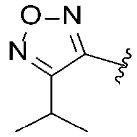,

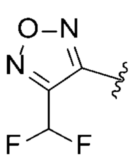, 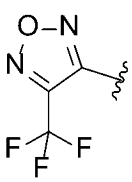, 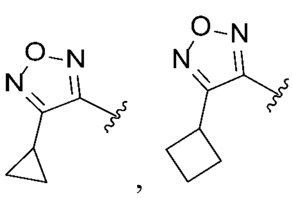, 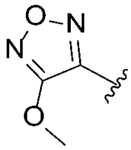, 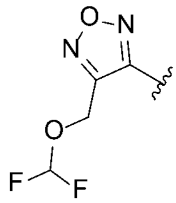,

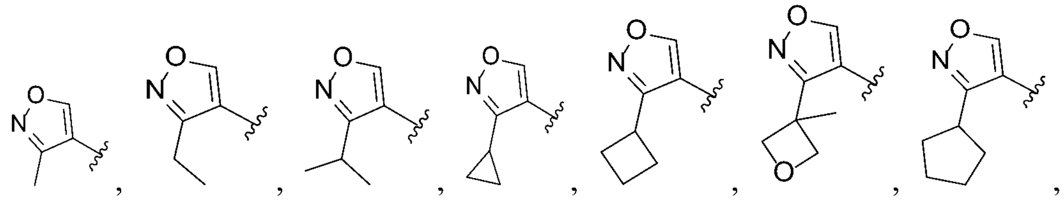

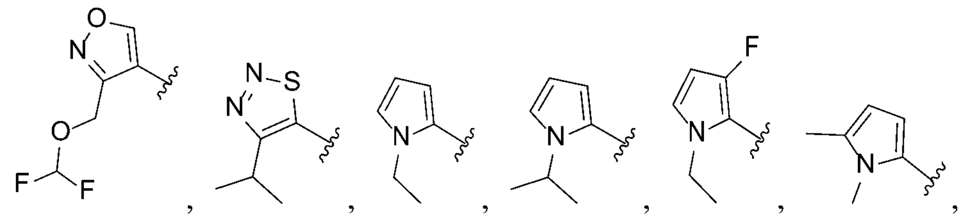

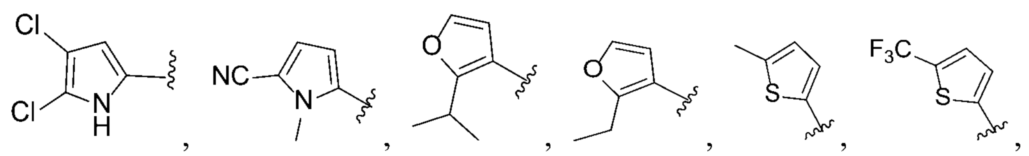

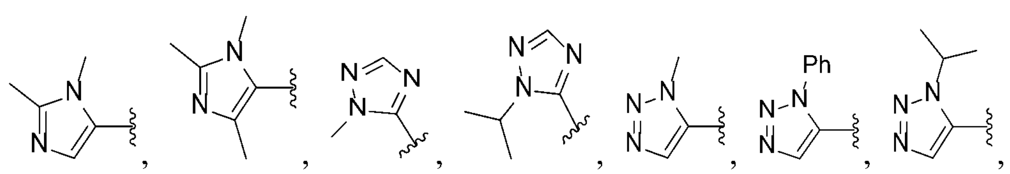

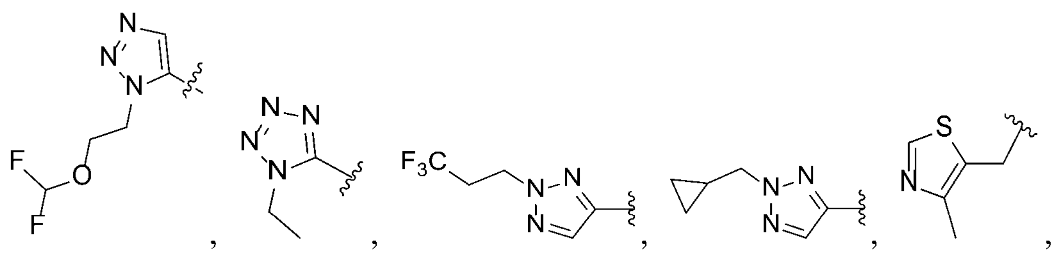

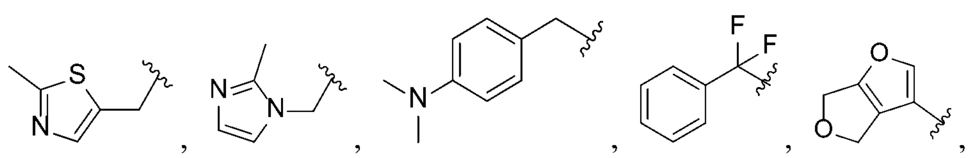

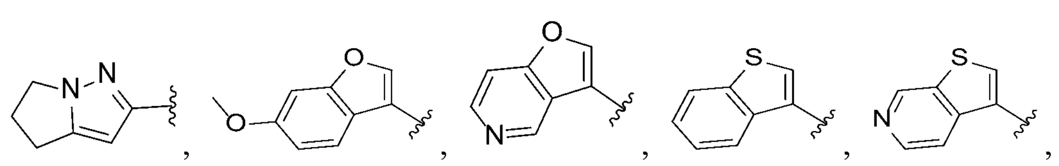

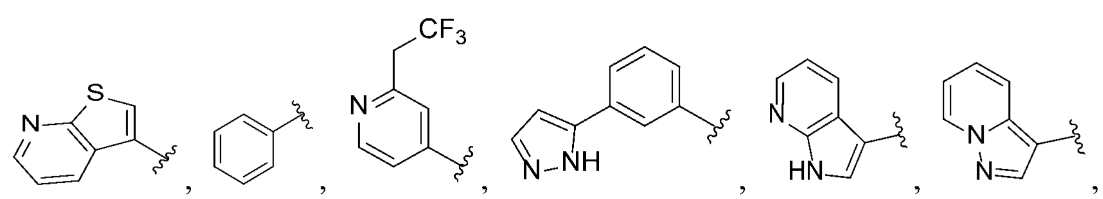

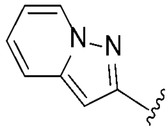, 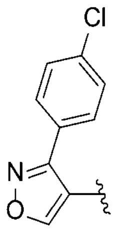, 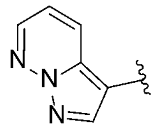, 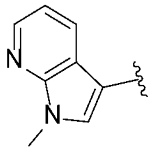, 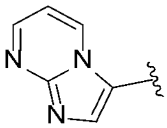, 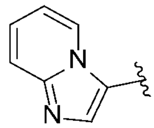,

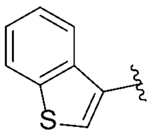, 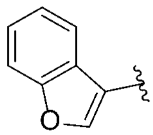, 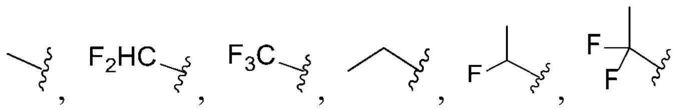, , , , , ,

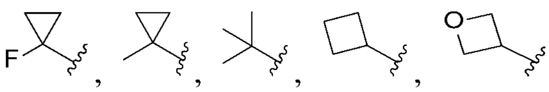, , , , , 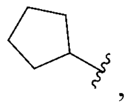, 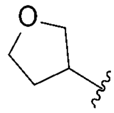, 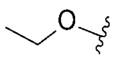, 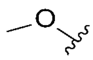,

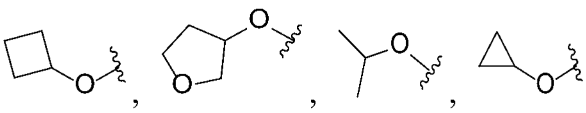, , , , 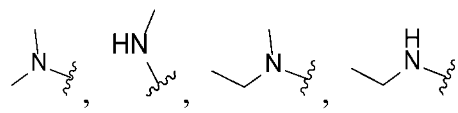, , , ,

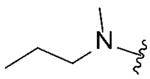, 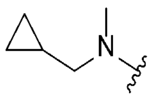, 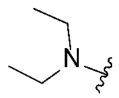, 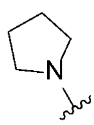, 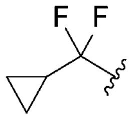, 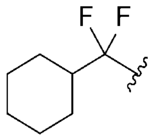, 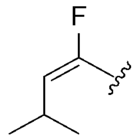,

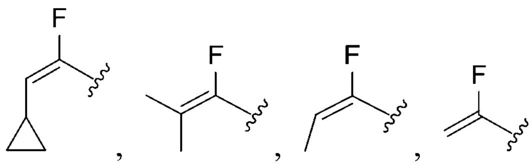, , , , 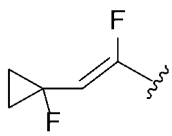, 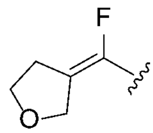, 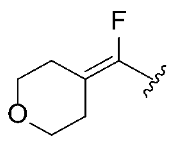,

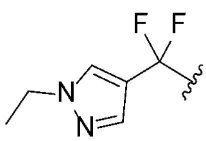, 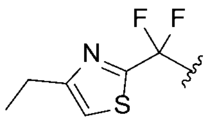, 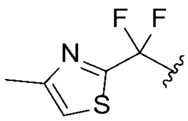, 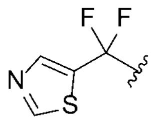, 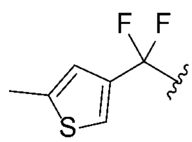,

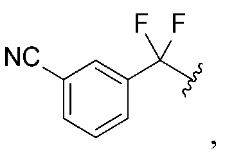, 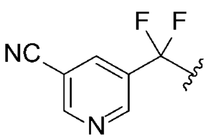, 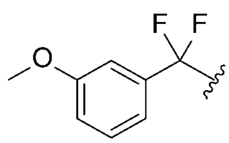, 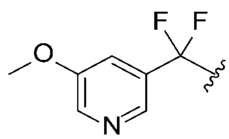,

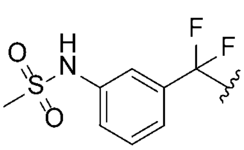, 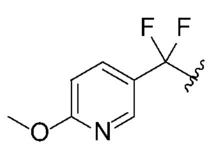, 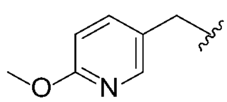, 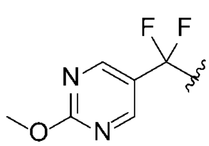,

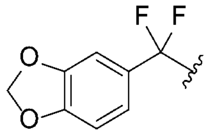 and 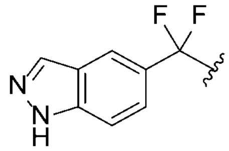.

In some embodiments, $R^2$ is selected from the group consisting of 3-membered cycloalkyl, 4-membered cycloalkyl, 5-membered cycloalkyl, 6-membered cycloalkyl, 7-membered cycloalkyl and 8-membered cycloalkyl, wherein cycloalkyl is unsubstituted or substituted by one, two, three or four $R^{2a}$.

In some embodiments, $R^2$ is selected from the group consisting of -$C_1$ alkylene-(3-membered cycloalkyl), -$C_1$ alkylene-(4-membered cycloalkyl), -$C_1$ alkylene-(5-membered cycloalkyl), -$C_1$ alkylene-(6-membered cycloalkyl), -$C_1$ alkylene-(7-membered cycloalkyl), -$C_1$ alkylene-(8-membered cycloalkyl), -$C_1$ alkylene-(benzene ring) and -$C_1$ alkylene-(heteroaromatic ring), wherein alkylene, cycloalkyl, benzene ring and heteroaromatic ring are unsubstituted or substituted by one, two, three or four $R^{2a}$.

In some embodiments, $R^2$ is

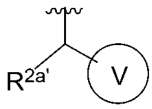,

wherein V ring is selected from the group consisting of 3-membered cycloalkyl, 4-membered cycloalkyl, 5-membered cycloalkyl, 6-membered cycloalkyl, 7-membered cycloalkyl and 4-membered heterocycloalkyl, wherein cycloalkyl is unsubstituted or substituted by one, two or three $R^{2a}$; and

$R^{2a}$ is selected from the group consisting of 3-membered cycloalkyl, 4-membered cycloalkyl, 5-membered cycloalkyl, 6-membered cycloalkyl, 7-membered cycloalkyl and 4-membered heterocycloalkyl, wherein cycloalkyl is unsubstituted or substituted by one, two or three $R^{2d}$.

In some embodiments, $R^2$ is selected from the group consisting of 6-membered fused cycloalkyl, 9-membered fused cycloalkyl, 10-membered fused cycloalkyl, 11-membered fused cycloalkyl, 9-membered fused heterocycloalkyl, 10-membered fused heterocycloalkyl, 12-membered fused heterocycloalkyl and 8-membered spiro cycloalkyl, wherein fused cycloalkyl, fused heterocycloalkyl and spiro cycloalkyl are unsubstituted or substituted by one, two, three or four $R^{2a}$.

In some embodiments, $R^2$ is selected from the group consisting of 5-membered bridged cycloalkyl, 6-membered bridged cycloalkyl, 7-membered bridged cycloalkyl, 8-membered bridged cycloalkyl, 9-membered bridged cycloalkyl, 10-membered bridged cycloalkyl, -$C_1$ alkylene-(5-membered bridged cycloalkyl), -$C_1$ alkylene-(6-membered bridged cycloalkyl), -$C_1$ alkylene-(7-membered bridged cycloalkyl), -$C_1$ alkylene-(8-membered bridged cycloalkyl), -$C_1$ alkylene-(9-membered bridged cycloalkyl) and -$C_1$ alkylene-(10-membered bridged cycloalkyl).

In some embodiments, $R^{21}$ and $R^{22}$ are independently selected from the group consisting of hydrogen, deuterium, methyl, ethyl, n-propyl, isopropyl, butyl, deuterium-substituted methyl, deuterium-substituted ethyl, deuterium-substituted n-propyl, deuterium-substituted isopropyl, halogen-substituted methyl, halogen-substituted ethyl, halogen-substituted n-propyl, halogen-substituted isopropyl, halogen-substituted butyl, 3-membered cycloalkyl, 4-membered cycloalkyl, 5-membered cycloalkyl, 6-membered cycloalkyl, 7-membered cycloalkyl, -$C_1$ alkylene-(3-membered cycloalkyl), -$C_1$ alkylene-(4-membered cycloalkyl), -$C_1$ alkylene-(5-membered cycloalkyl), -$C_1$ alkylene-(6-membered cycloalkyl) and -$C_1$ alkylene-(7-membered cycloalkyl), wherein alkylene, cycloalkyl and heterocycloalkyl are unsubstituted or substituted by one, two or three $R^{2a}$.

In some embodiments, each $R^{2a}$ is independently selected from the group consisting of hydrogen, deuterium, halogen, cyano, =O, =S, nitro, methyl, ethyl, n-propyl, isopropyl, deuterium-substituted methyl, deuterium-substituted ethyl, deuterium-substituted n-propyl, deuterium-substituted isopropyl, halogen-substituted methyl, halogen-substituted ethyl, halogen-substituted n-propyl, halogen-substituted isopropyl, - $S(O)_2R^{2b}$ and -$OR^{2b}$.

In some embodiments, each $R^{2a}$ is independently selected from the group consisting of 3-membered cycloalkyl, 4-membered cycloalkyl, 5-membered cycloalkyl, 6-membered cycloalkyl, 7-membered cycloalkyl, -$C_1$ alkylene-(3-membered cycloalkyl), -$C_1$ alkylene-(4-membered cycloalkyl), -$C_1$ alkylene-(5-membered cycloalkyl), -$C_1$ alkylene-(6-membered cycloalkyl) and -$C_1$ alkylene-(7-membered cycloalkyl), wherein alkylene, cycloalkyl and heterocycloalkyl are unsubstituted or substituted by one, two or three $R^{2d}$; or two $R^{2a}$ linked to the same carbon atom form 3 to 10-membered cycloalkyl or 4 to 10-membered heterocycloalkyl with the carbon atom.

In some embodiments, $R^{2b}$ and $R^{2c}$ are independently selected from the group consisting of hydrogen, deuterium, methyl, ethyl, n-propyl, isopropyl, deuterium-substituted methyl, deuterium-substituted ethyl, deuterium-substituted n-propyl, deuterium-substituted isopropyl, halogen-substituted methyl, halogen-substituted ethyl, halogen-substituted n-propyl, halogen-substituted isopropyl, 3-membered cycloalkyl, 4-membered cycloalkyl, 5-membered cycloalkyl, 6-membered cycloalkyl, 7-membered cycloalkyl, $-C_1$ alkylene-(3-membered cycloalkyl), $-C_1$ alkylene-(4-membered cycloalkyl), $-C_1$ alkylene-(5-membered cycloalkyl), $-C_1$ alkylene-(6-membered cycloalkyl) and $-C_1$ alkylene-(7-membered cycloalkyl), wherein alkyl, alkylene, cycloalkyl and heterocycloalkyl are unsubstituted or substituted by one, two or three $R^{2d}$.

In some embodiments, each $R^{2d}$ is independently selected from the group consisting of hydrogen, deuterium, methyl, ethyl, n-propyl, isopropyl, deuterium-substituted methyl, deuterium-substituted ethyl, deuterium-substituted n-propyl, deuterium-substituted isopropyl, halogen-substituted methyl, halogen-substituted ethyl, halogen-substituted n-propyl, halogen-substituted isopropyl, halogen, cyano, =O, =S, nitro, -OH, $-O(C_{1-6}$ alkyl), $-NH_2$, $-NH(C_{1-6}$ alkyl) and $-N(C_{1-6}$ alkyl)($C_{1-6}$ alkyl).

In some embodiments, $R^2$ is selected from the group consisting of:

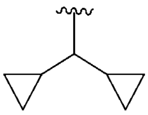, 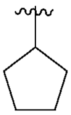, 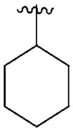, 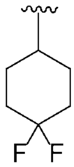, 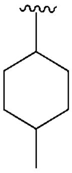, 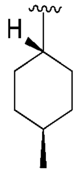, 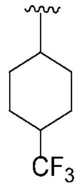, 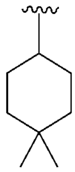, 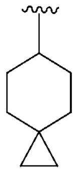, 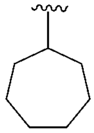,

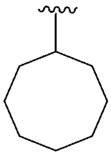, 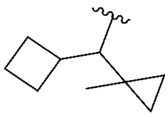, 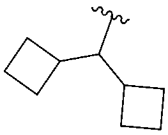, 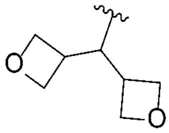, 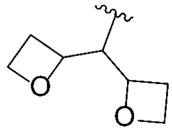,

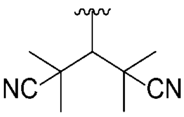, 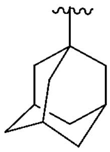, 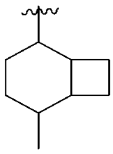, 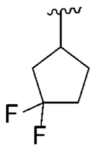, 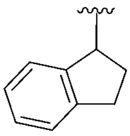, 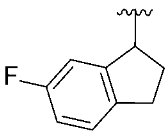, 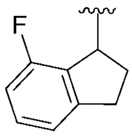

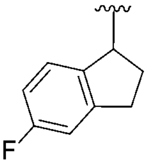, 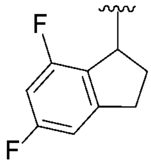, 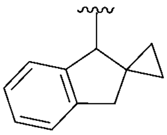, 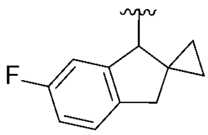, 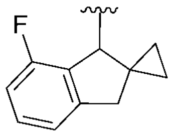,

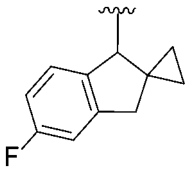, 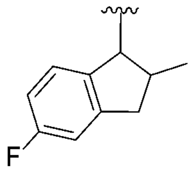, 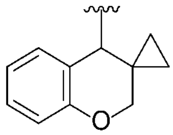, 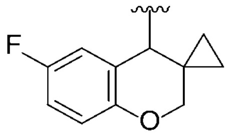,

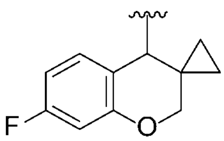, 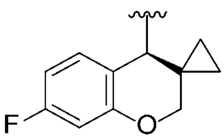, 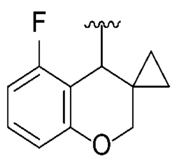, 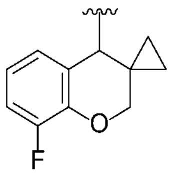,

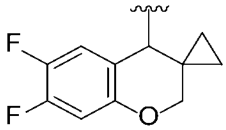, 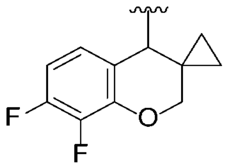, 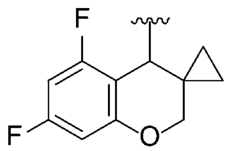, 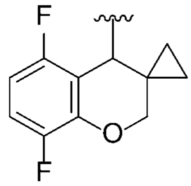,

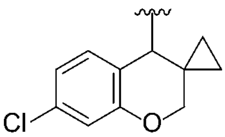, 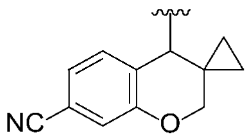, 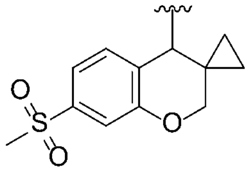, 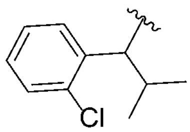,

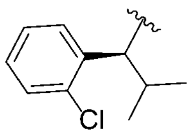, 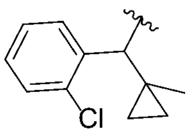, 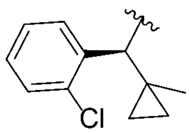, 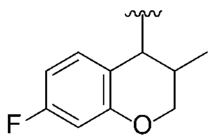, 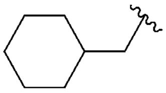,

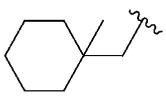, 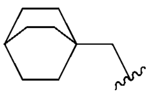, 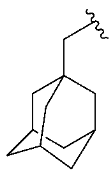, 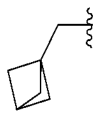, 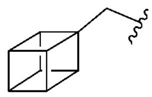, 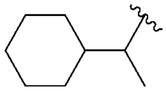,

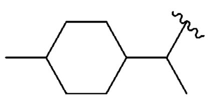, 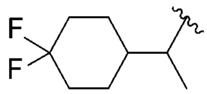, 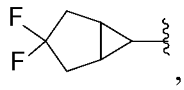, 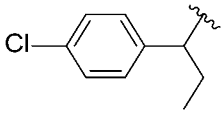,

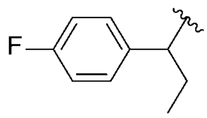, 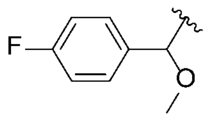, 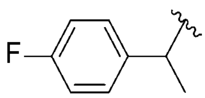, 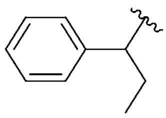, 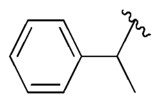,

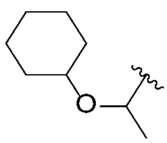, 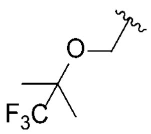, 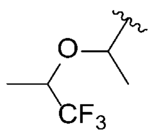, 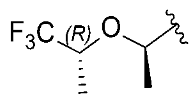, 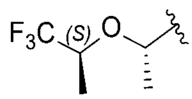,

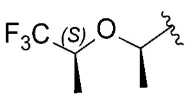, 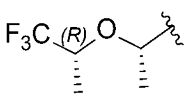, 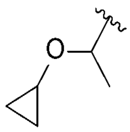, 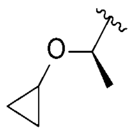, 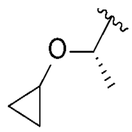, 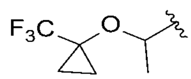,

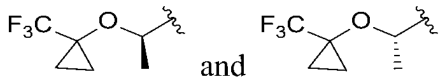 and .

In some embodiments, L is selected from the group consisting of $C_3$ alkylene, $C_4$ alkylene, $C_5$ alkylene, $C_6$ alkylene, $C_3$ alkenylene, $C_4$ alkenylene, $C_5$ alkenylene and $C_6$ alkenylene, wherein one, two or three methylene groups in alkylene and alkenylene is unsubstituted or substituted by O, $NR^{L1}$, C(O), S(O) and $S(O)_2$; or alkylene and alkenylene are

unsubstituted or substituted by one, two, three or four $R^L$.

In some embodiments, each $R^{L1}$ is selected from the group consisting of hydrogen, deuterium, methyl, ethyl, n-propyl, isopropyl, halogen-substituted methyl, halogen-substituted ethyl, halogen-substituted n-propyl, halogen-substituted isopropyl, $-C_{0-2}$ alkylene-CN, $-C_{0-2}$ alkylene-C(O)($C_{1-6}$ alkyl), $-C_{0-2}$ alkylene-(3-membered cycloalkyl), $-C_{0-2}$ alkylene-(4-membered cycloalkyl), $-C_{0-2}$ alkylene-(6-membered cycloalkyl), $-C_{0-2}$ alkylene-(6-membered heterocycloalkyl), $-C_{0-2}$ alkylene-(6-membered aryl), $-C_{0-2}$ alkylene-O($C_{1-6}$ alkyl) and $-C_{0-4}$ alkylene-N($C_{1-6}$ alkyl)($C_{1-6}$ alkyl), wherein alkyl, alkylene, cycloalkyl, heterocycloalkyl, aryl and heteroaryl are unsubstituted or substituted by one, two or three $R^{L2}$;

each $R^{L2}$ is selected from the group consisting of hydrogen, deuterium, halogen, cyano, =O, =S, nitro, $-C_{1-6}$ alkyl, deuterium-substituted $-C_{1-6}$ alkyl and halogen-substituted - $C_{1-6}$ alkyl; and

each $R^L$ is selected from the group consisting of hydrogen, deuterium, halogen, cyano, =O, =S, nitro, methyl, ethyl, n-propyl, isopropyl, deuterium-substituted methyl, deuterium-substituted ethyl, deuterium-substituted n-propyl, deuterium-substituted isopropyl, halogen-substituted methyl, halogen-substituted ethyl, halogen-substituted n-propyl and halogen-substituted isopropyl; or two $R^L$ linked to the same carbon atom form 3-membered cycloalkyl, 4-membered cycloalkyl, 5-membered cycloalkyl or 6-membered cycloalkyl with the carbon atom.

In some embodiments, L is selected from the group consisting of:

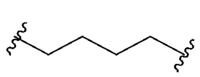 aa, 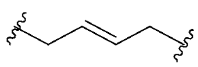 aa, 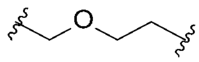 aa, 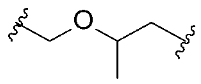 aa,

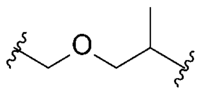 aa, 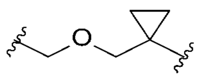 aa, 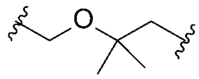 aa, 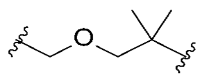 aa,

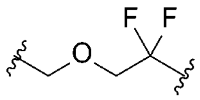 aa, 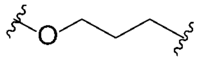 aa, 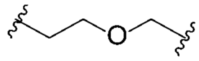 aa, 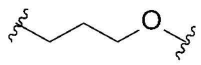 aa,

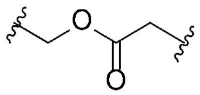 aa, 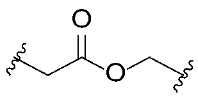 aa, 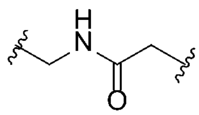 aa, 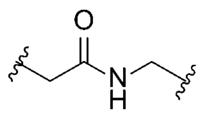 aa,

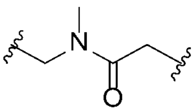 aa, 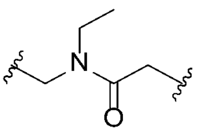 aa, 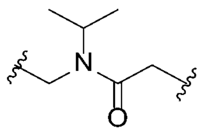 aa, 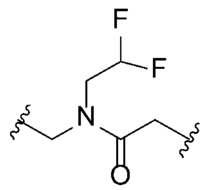 aa,

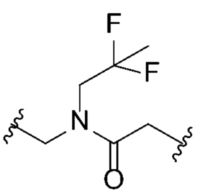 aa, 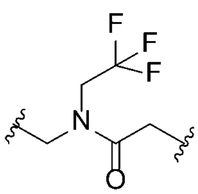 aa, 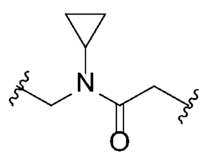 aa, 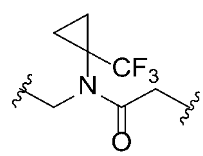 aa,

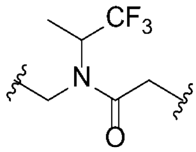 aa , 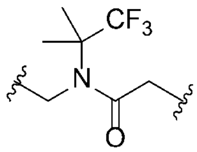 aa , 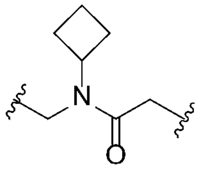 aa , 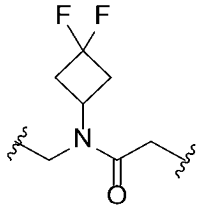 aa ,

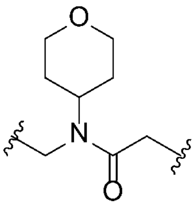 aa , 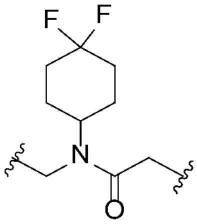 aa , 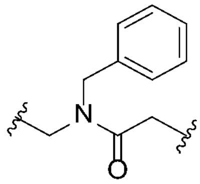 aa , 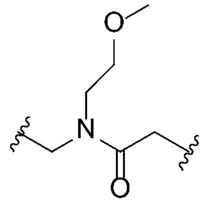 aa ,

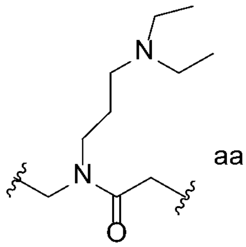 aa , 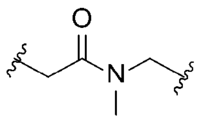 aa , 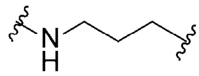 aa , 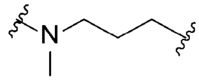 aa ,

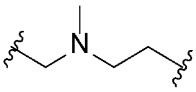 aa , 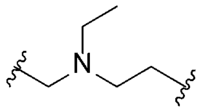 aa , 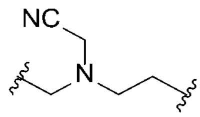 aa , 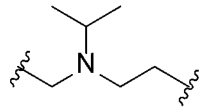 aa ,

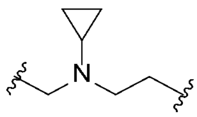 aa , 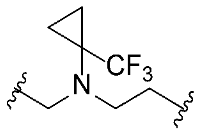 aa , 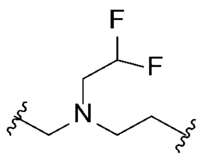 aa , 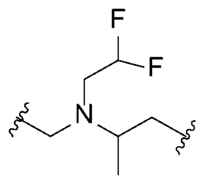 aa ,

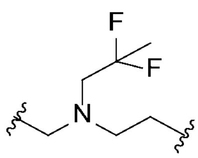 aa , 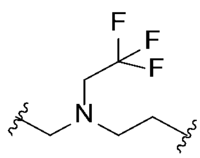 aa , 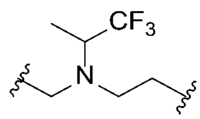 aa , 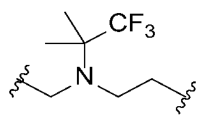 aa ,

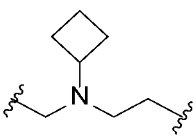 aa , 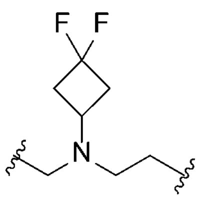 aa , 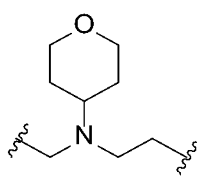 aa , 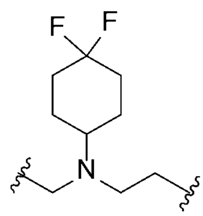 aa ,

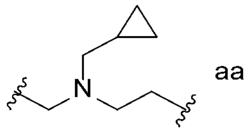, 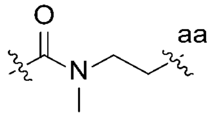, 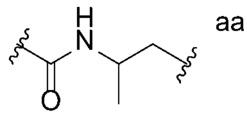, 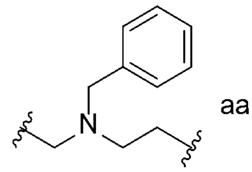

and

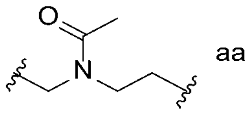,

wherein aa indicates an end of L linked to $X^4$.

In some embodiments, the compound is selected from the group consisting of:

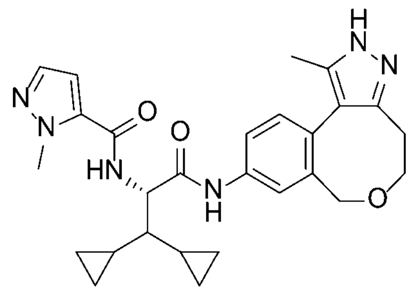, 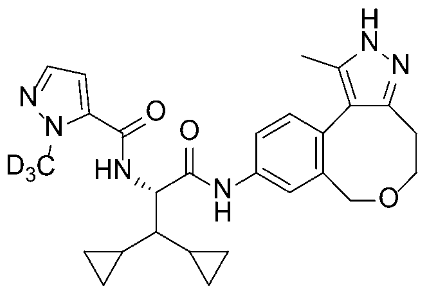,

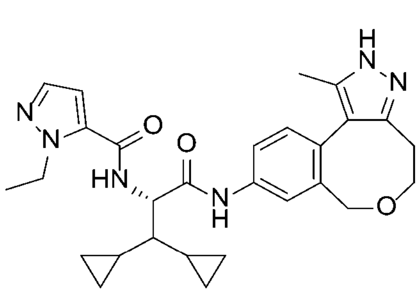, 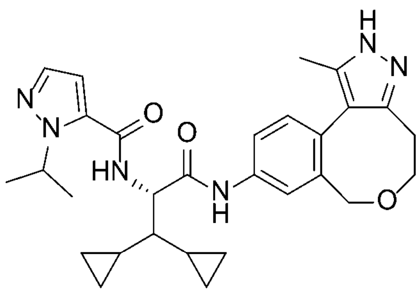,

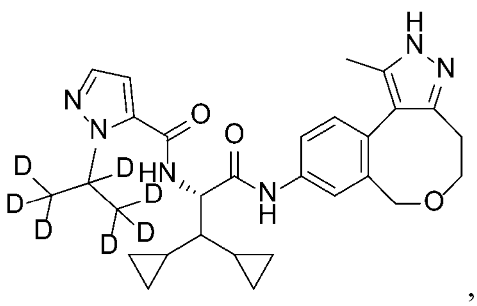, 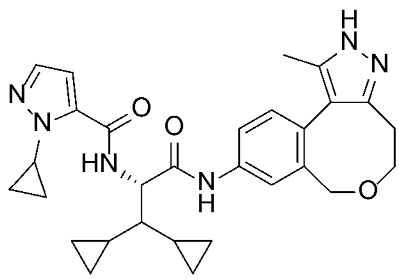,

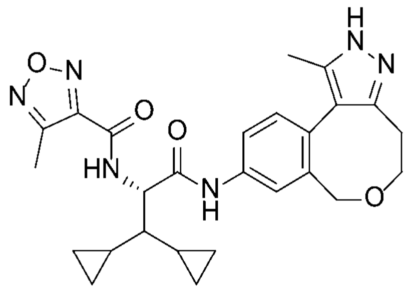
,
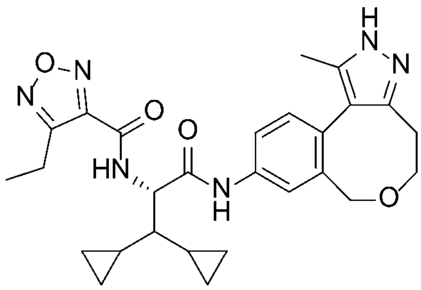
,

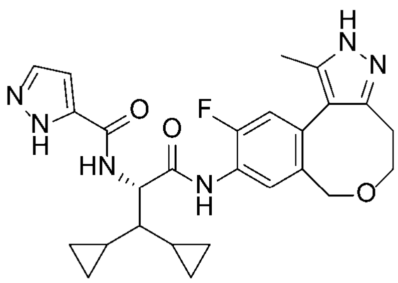
,
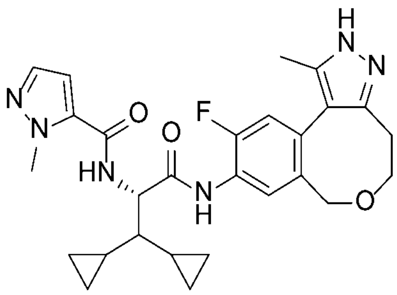
,

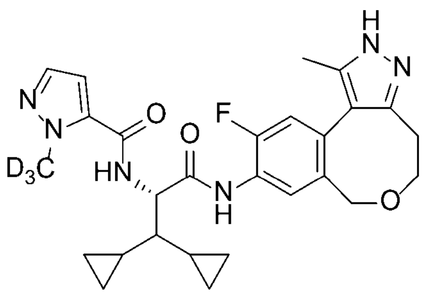
,
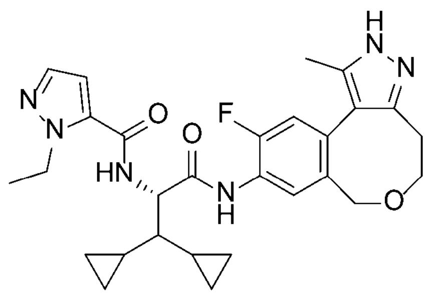
,

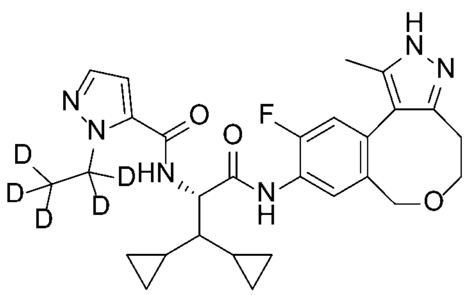
,
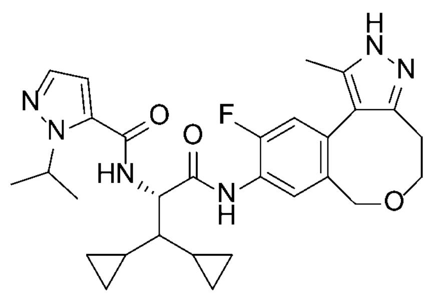
,

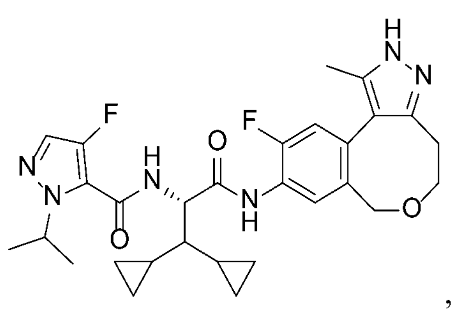
,
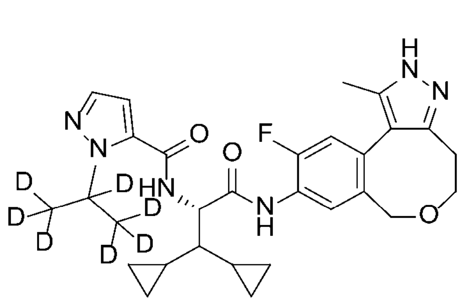
,

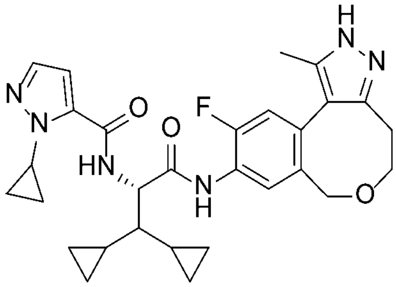 ,

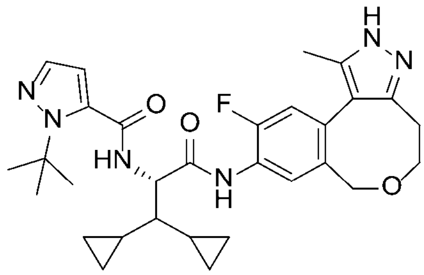 ,

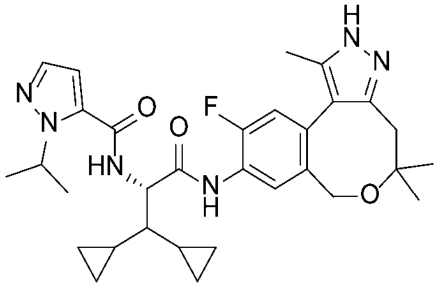 ,

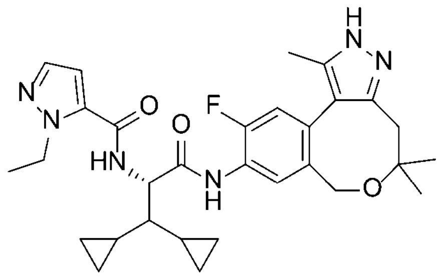 ,

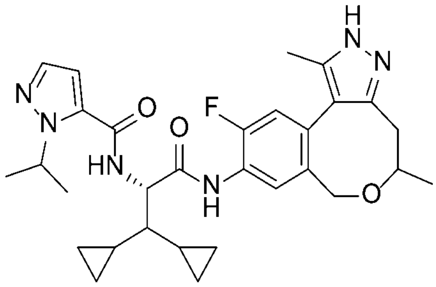 ,

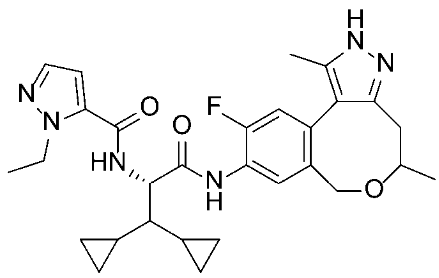 ,

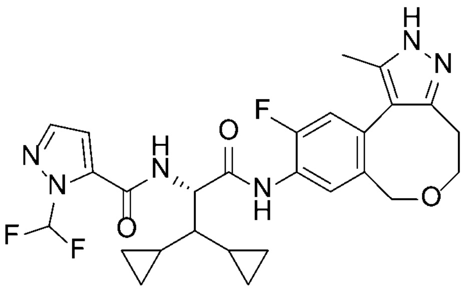 ,

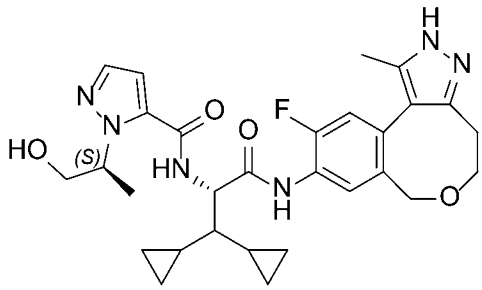 ,

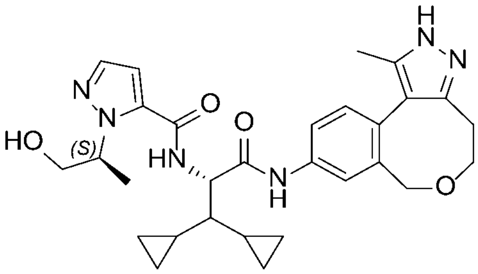 ,

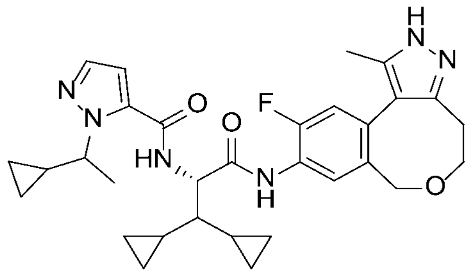 ,

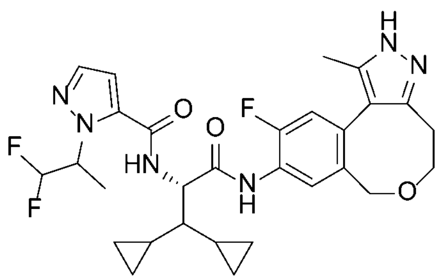,

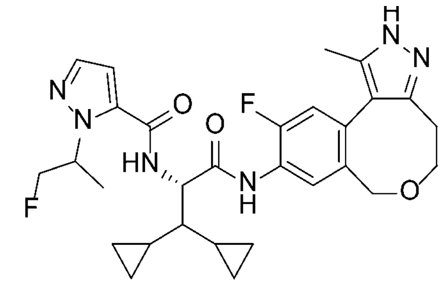,

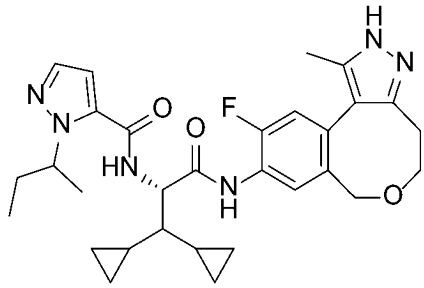,

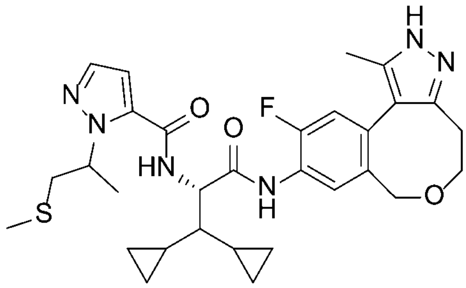,

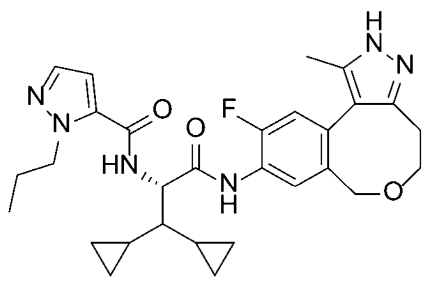,

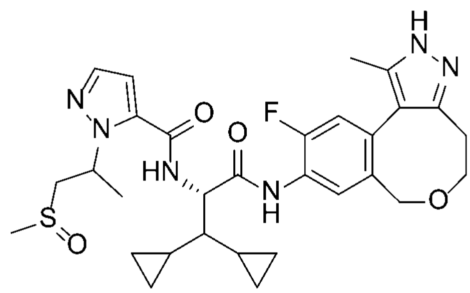,

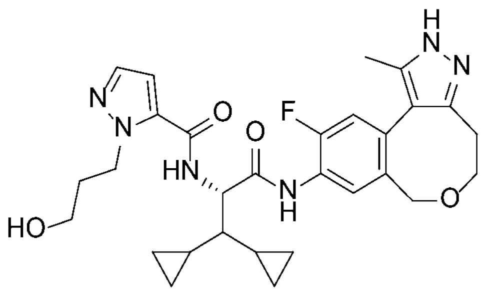,

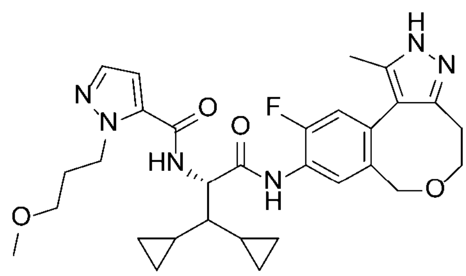,

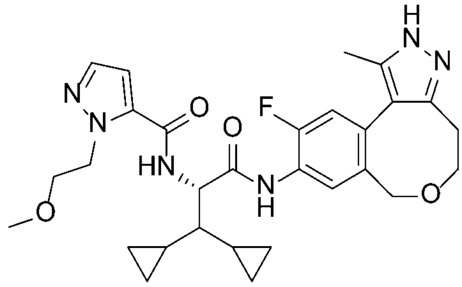,

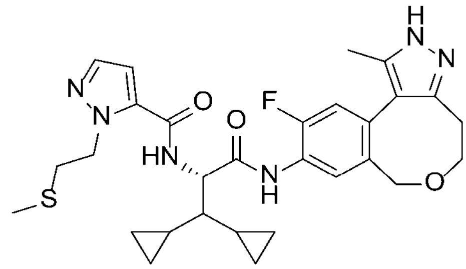,

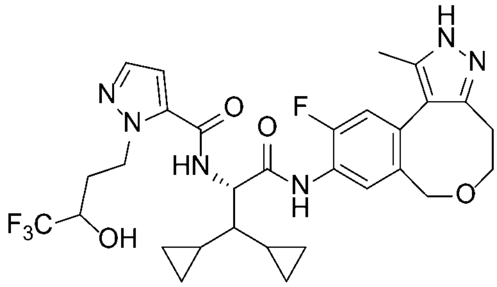

,

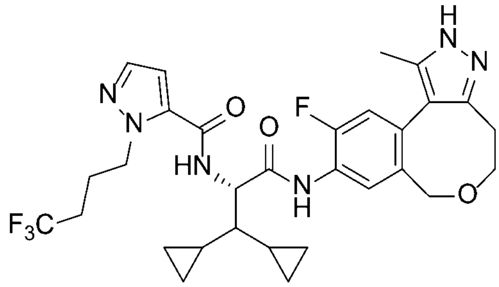

,

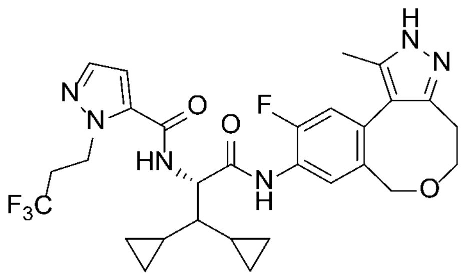

,

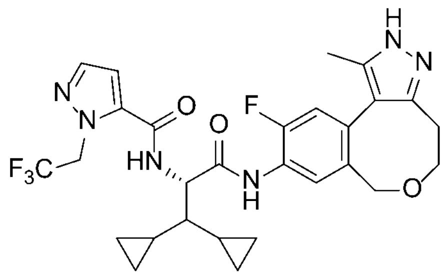

,

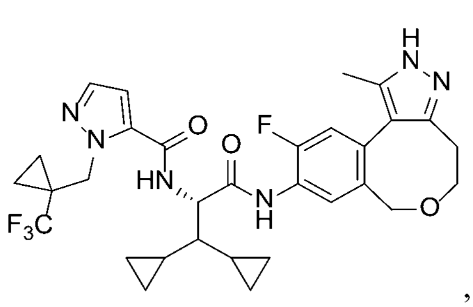

,

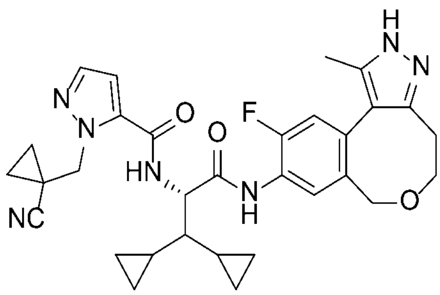

,

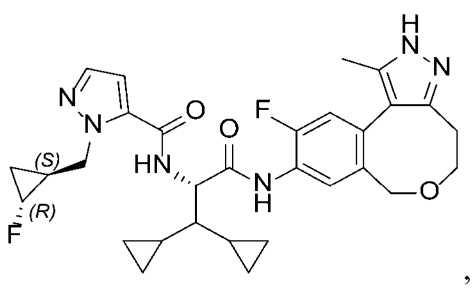

,

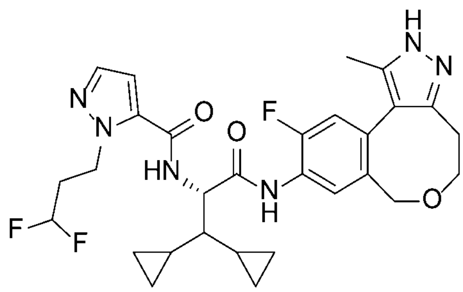

,

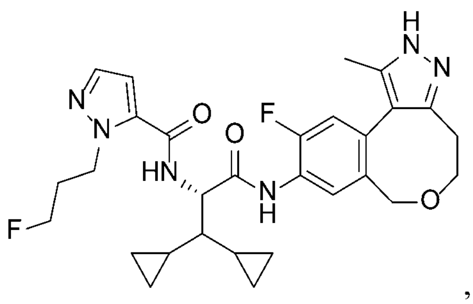

,

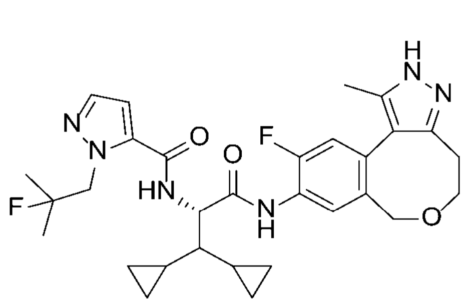

,

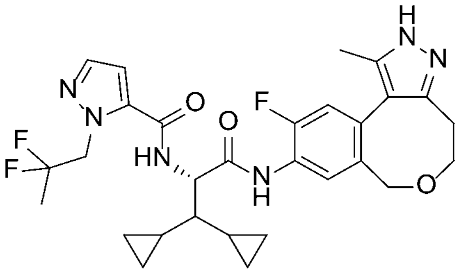
,
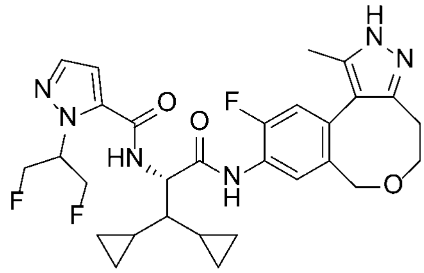
,
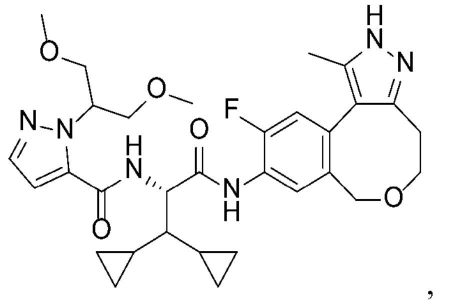
,
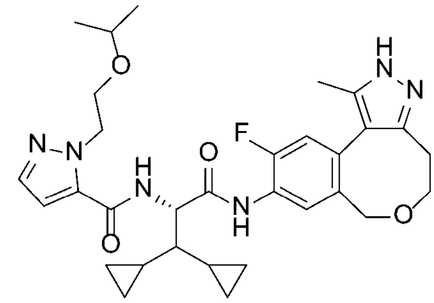
,
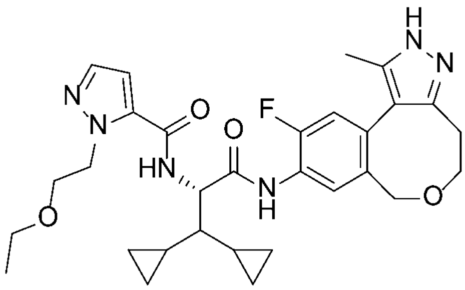
,
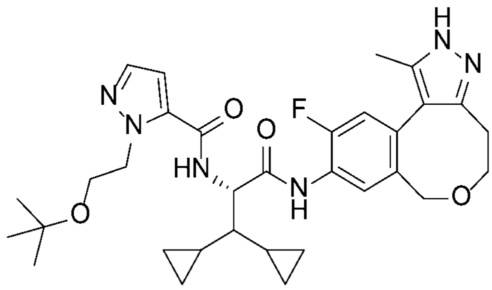
,
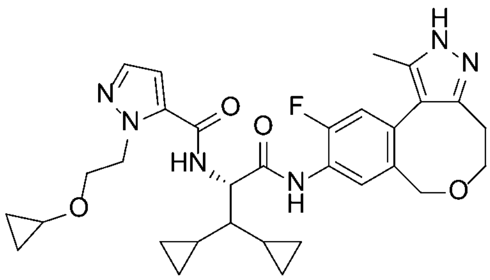
,
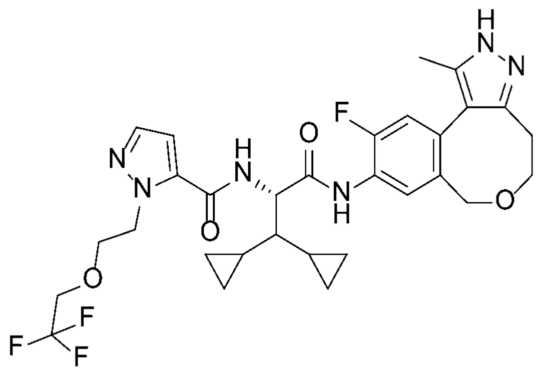
,
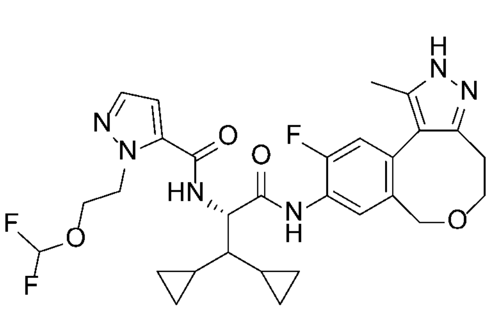
,
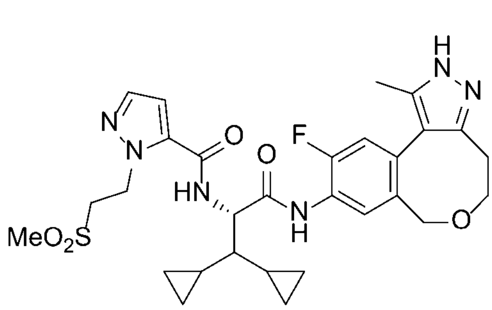
,

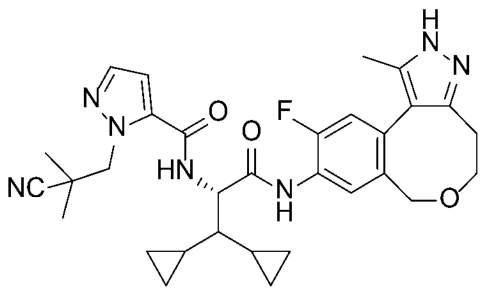
,
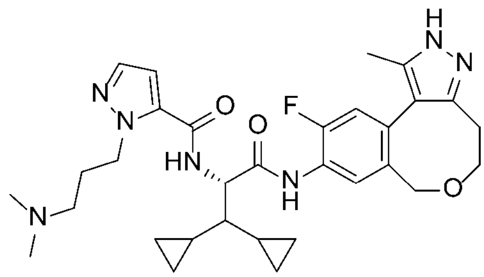
,
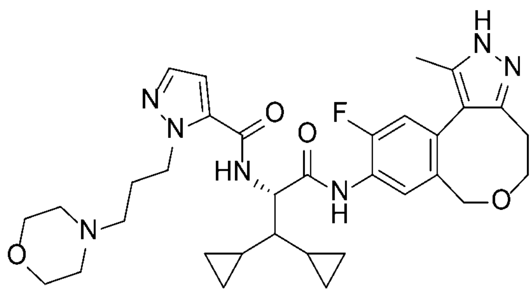
,
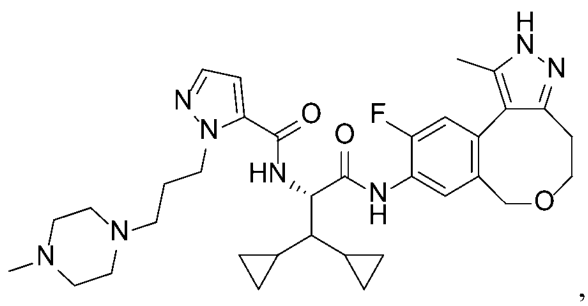
,
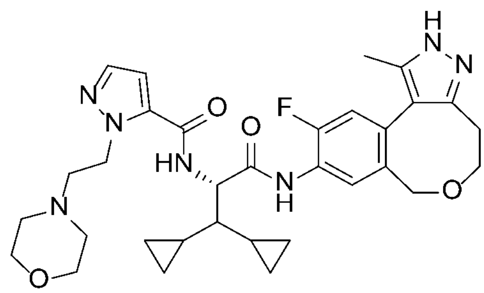
,
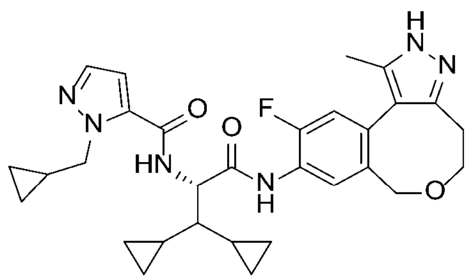
,
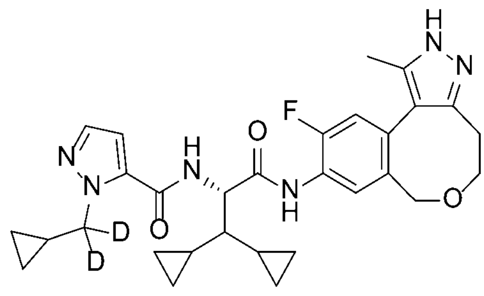
,
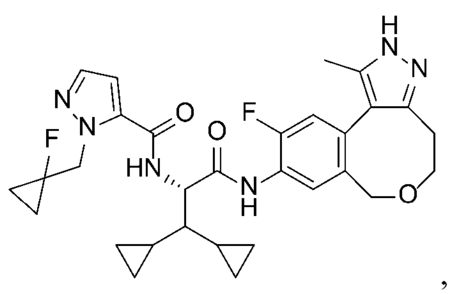
,
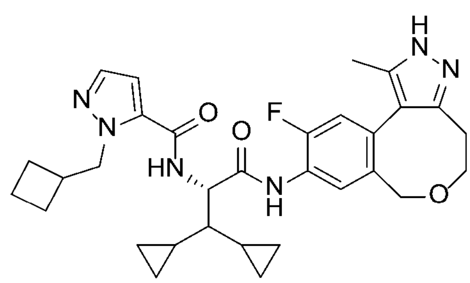
,

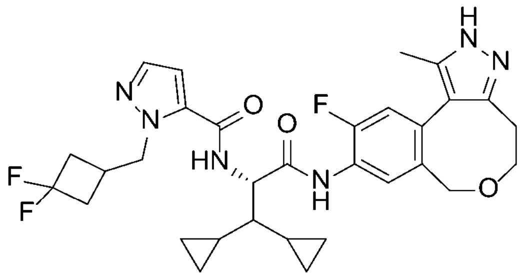

,

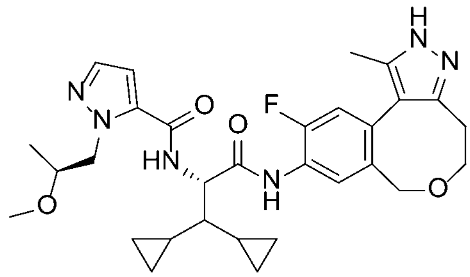

,

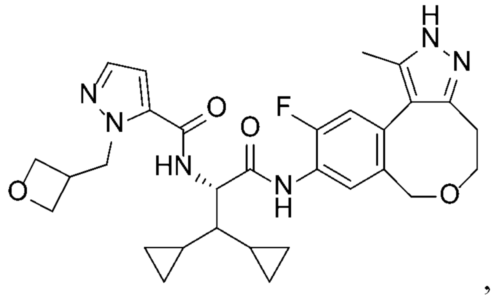

,

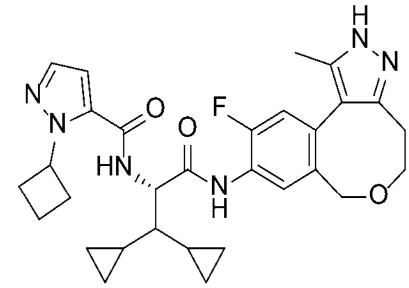

,

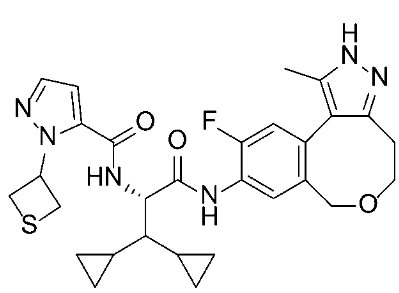

,

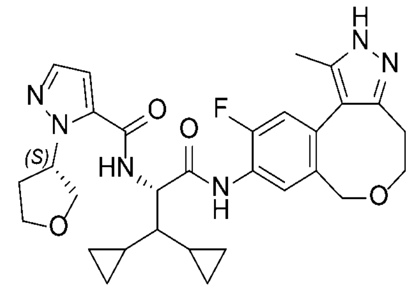

,

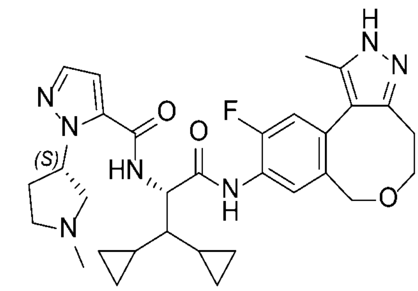

,

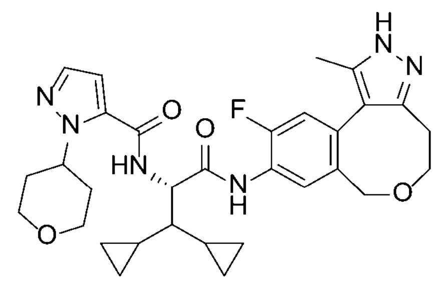

,

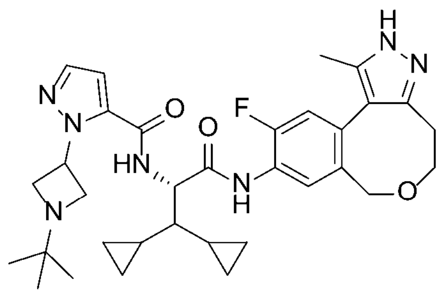

,

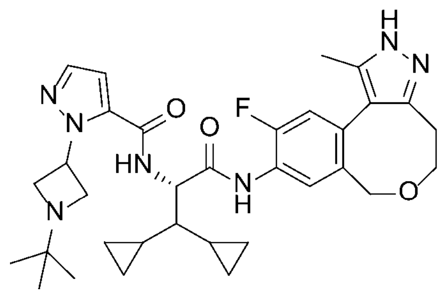

,

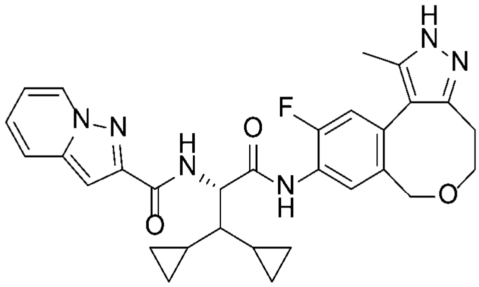
,
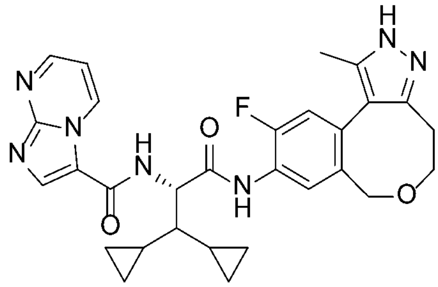
,
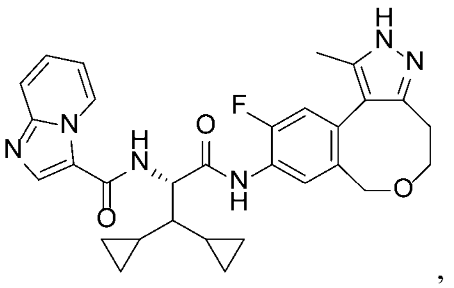
,
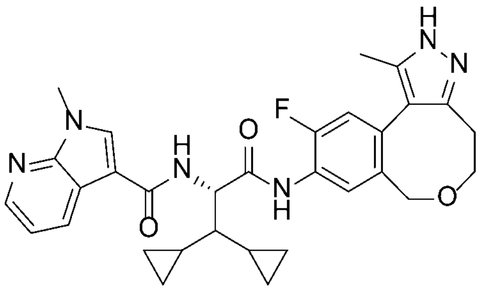
,
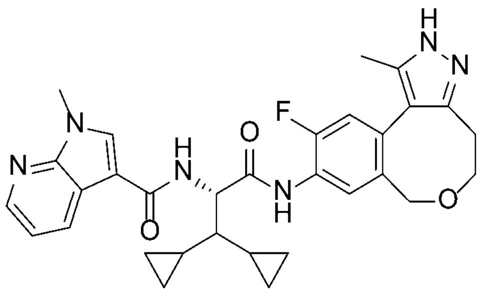
,
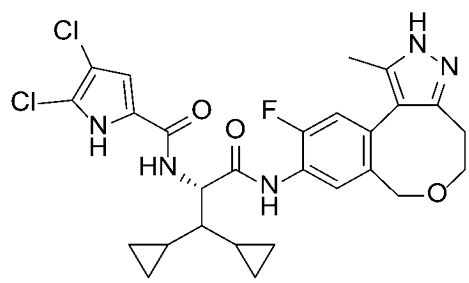
,
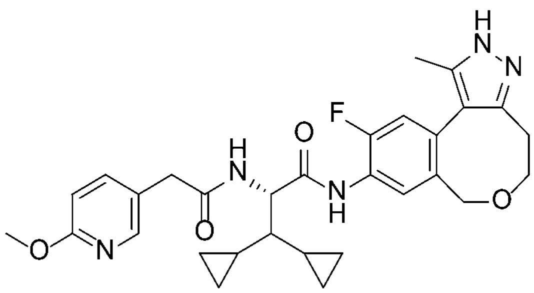
,
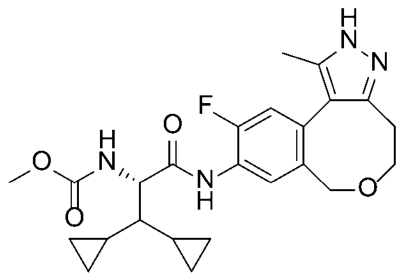
,
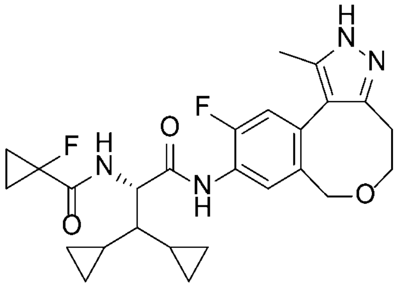
,
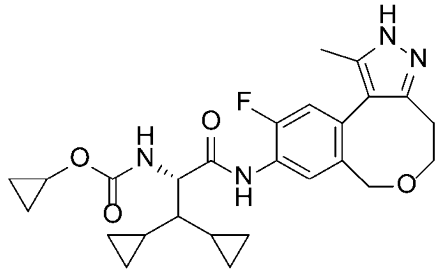
,

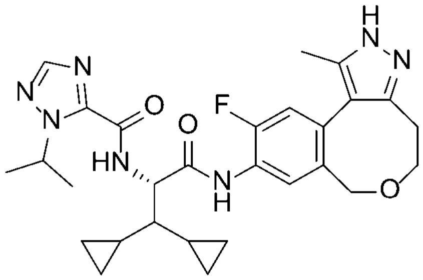,

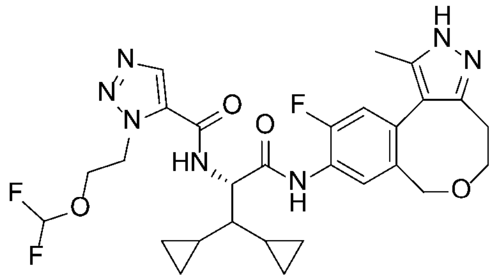,

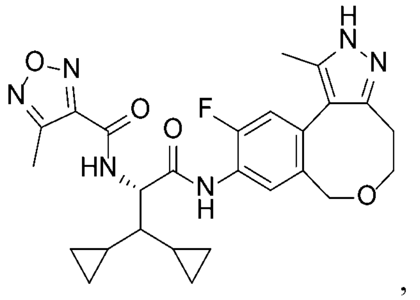,

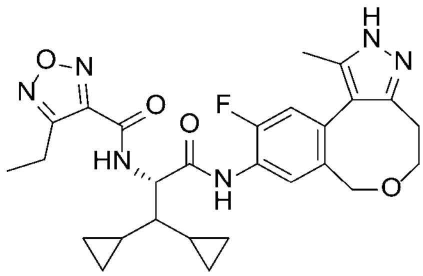,

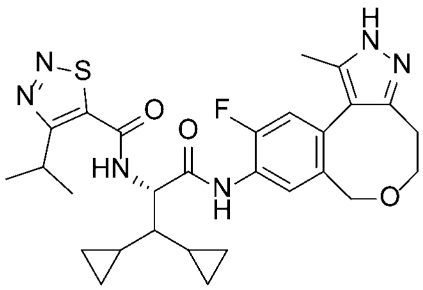,

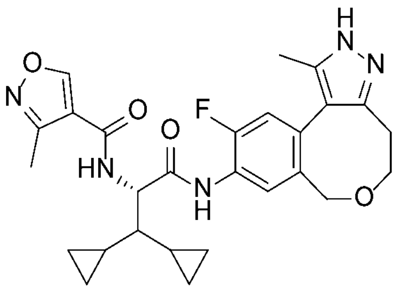,

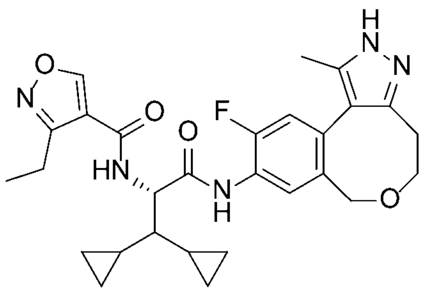,

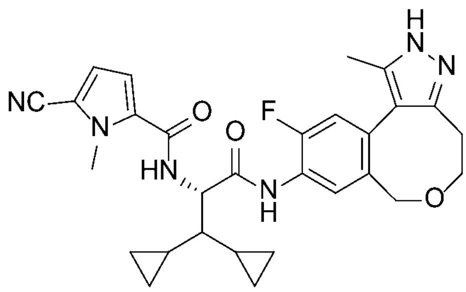,

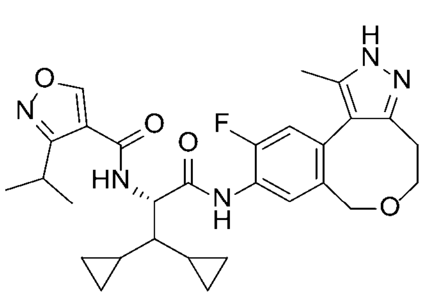,

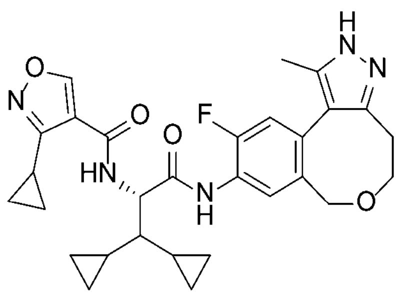,

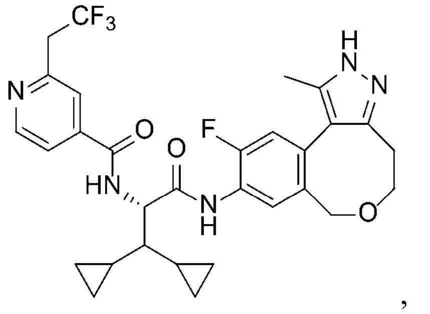
,
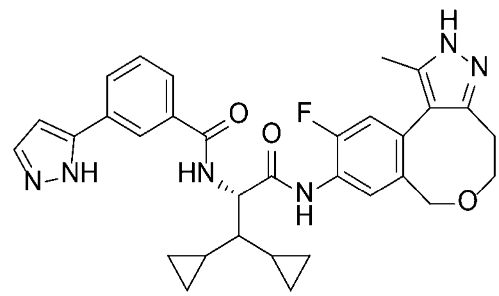
,
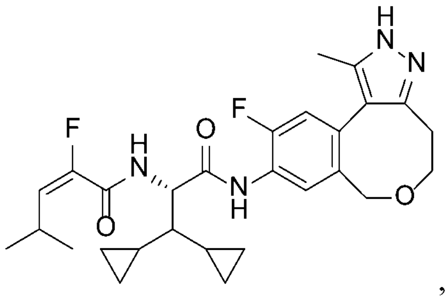
,
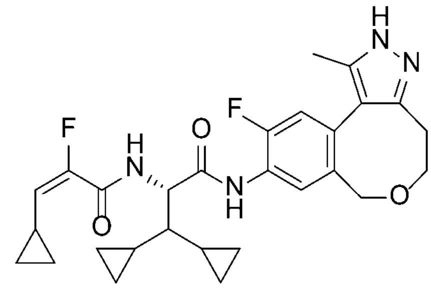
,
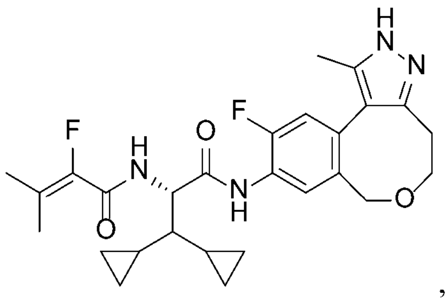
,
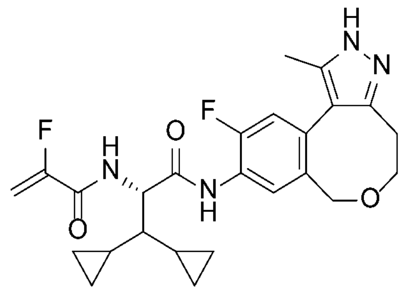
,
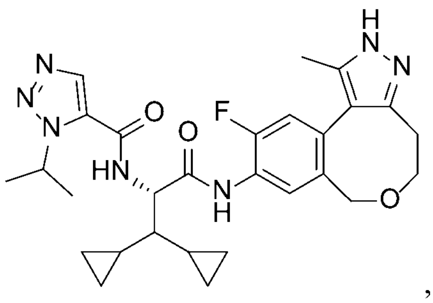
,
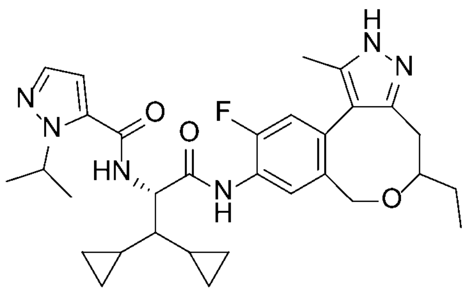
,
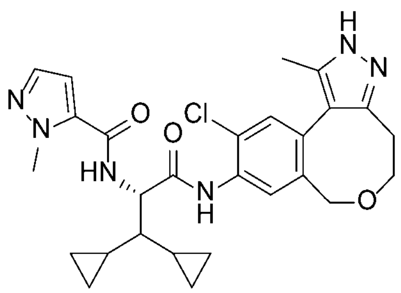
,
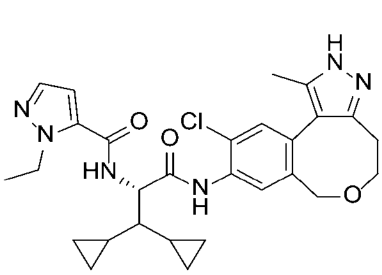
,

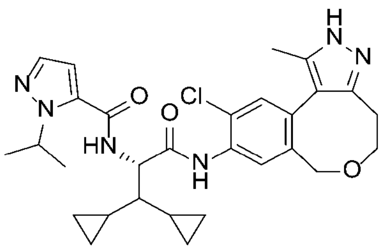
,
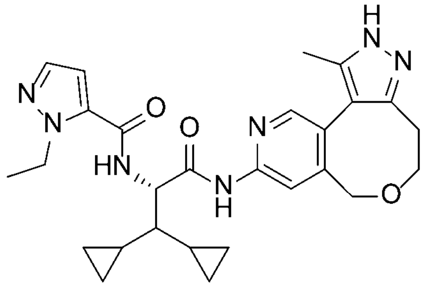
,
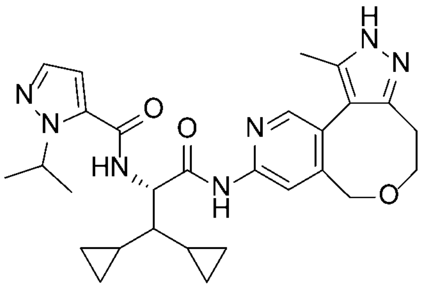
,
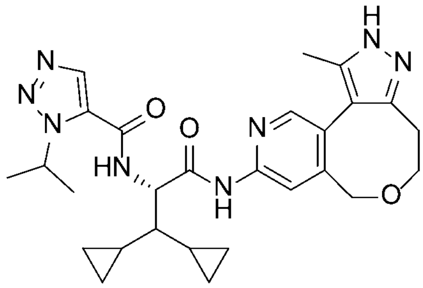
,
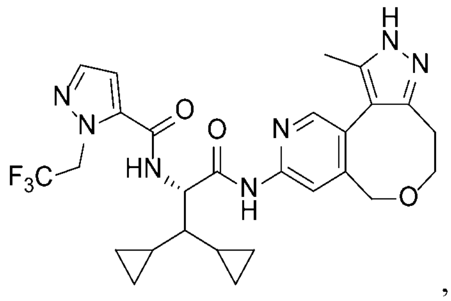
,
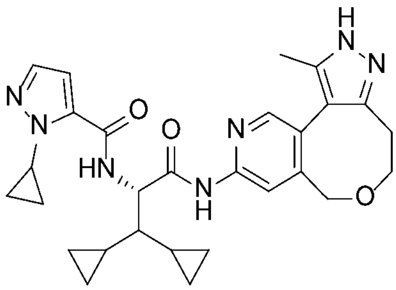
,
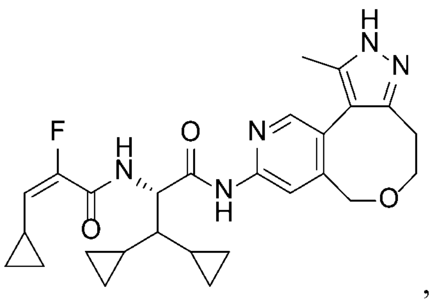
,
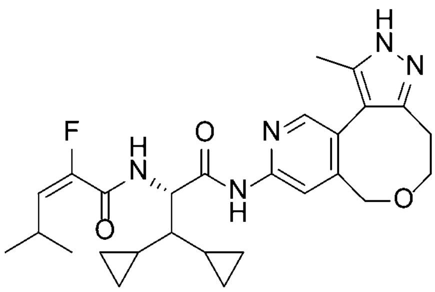
,
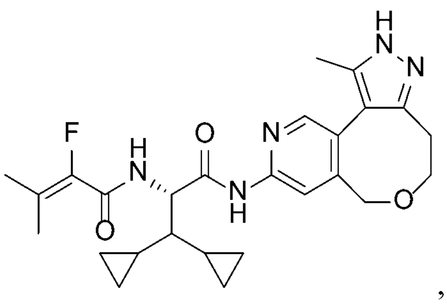
,
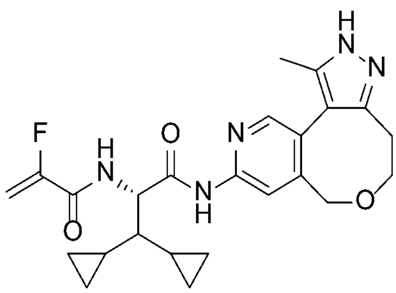
,

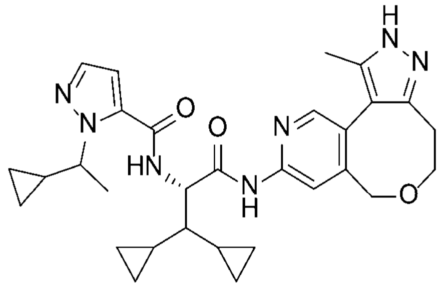
,

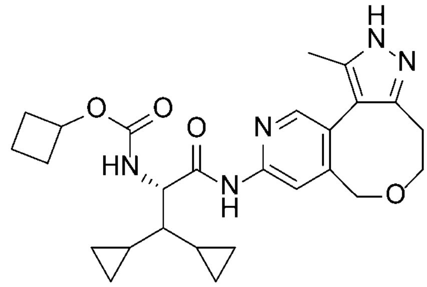
,

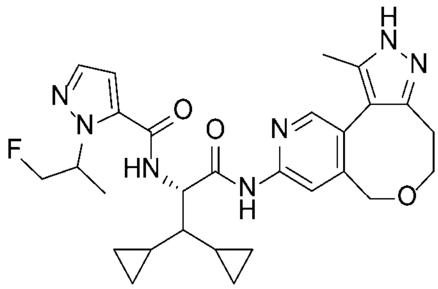
,

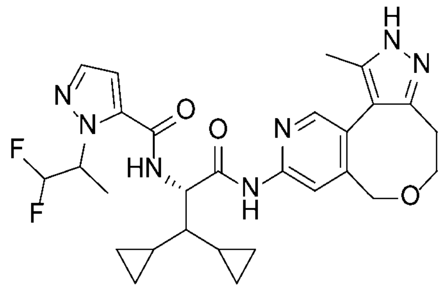
,

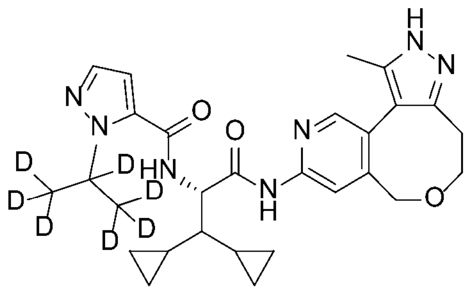
,

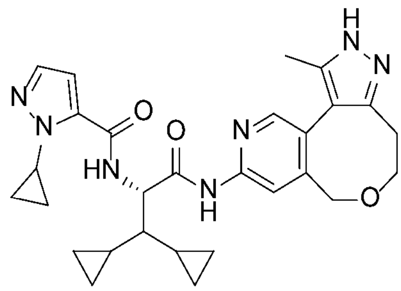
,

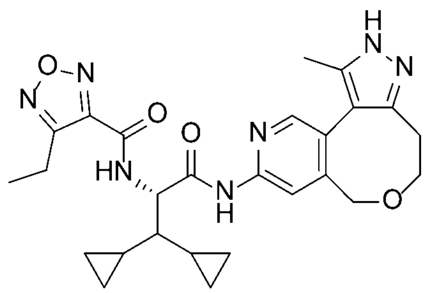
,

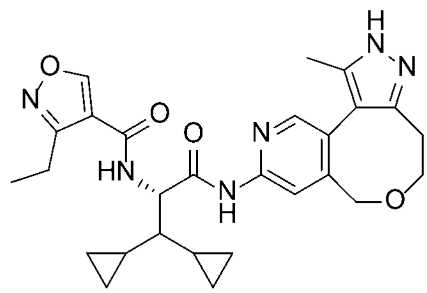
,

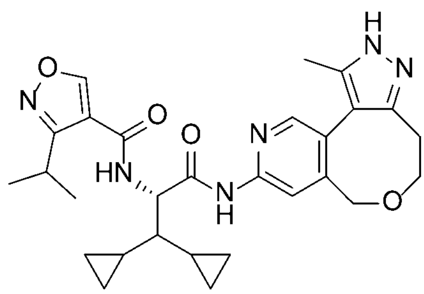
,

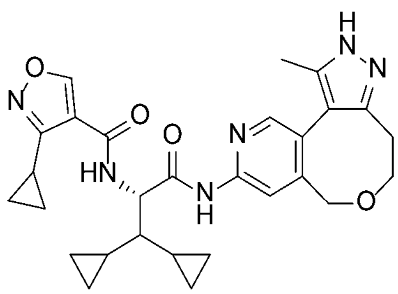
,

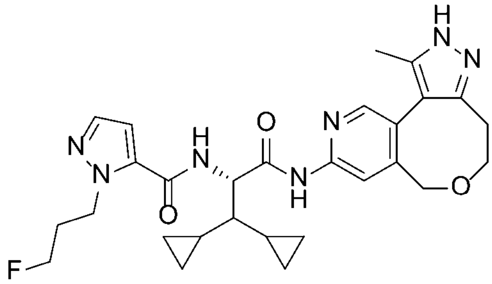 ,

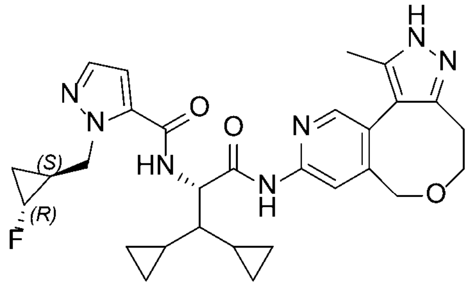 ,

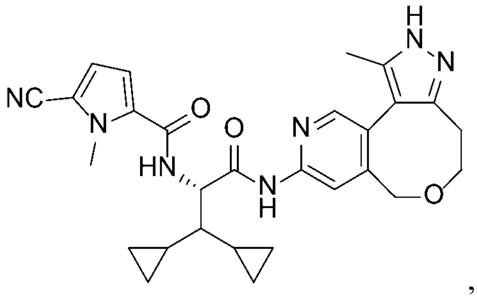 ,

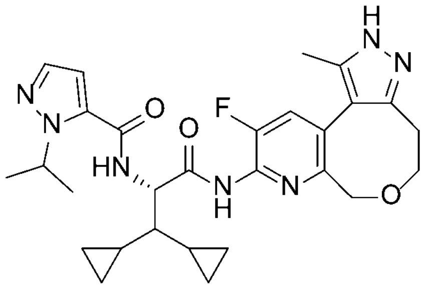 ,

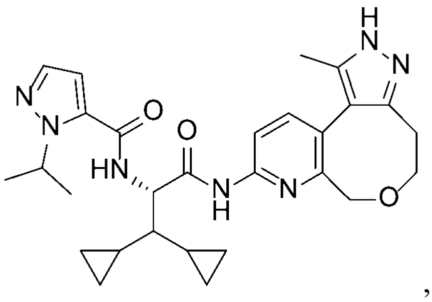 ,

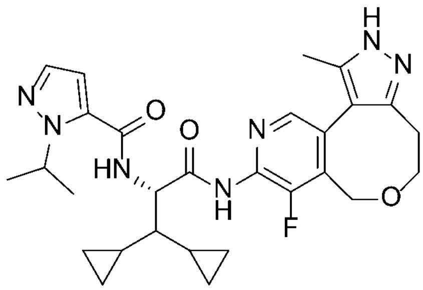 ,

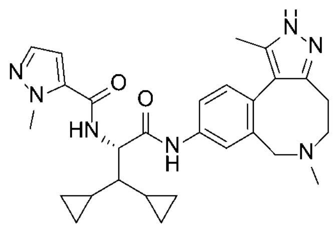 ,

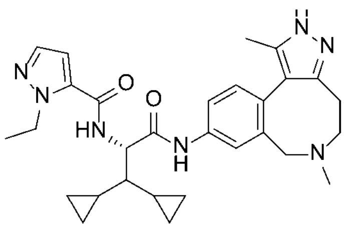 ,

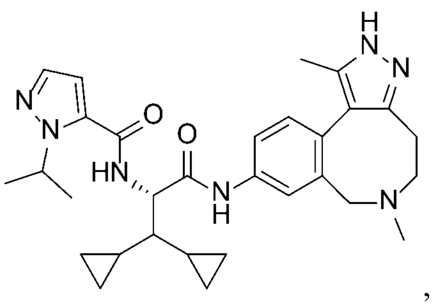 ,

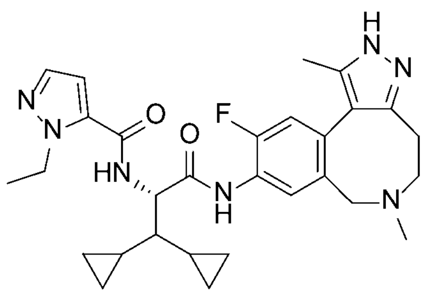 ,

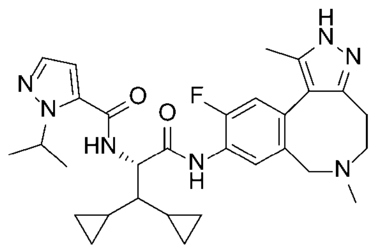,

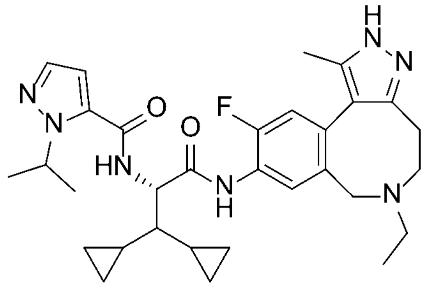,

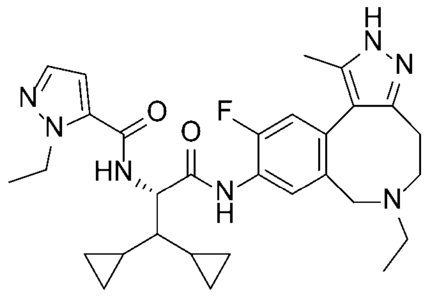,

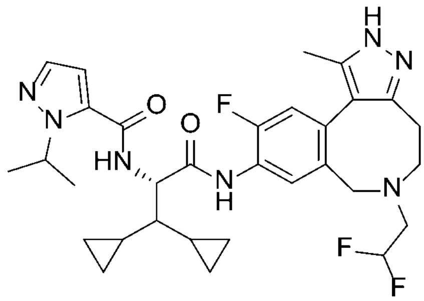,

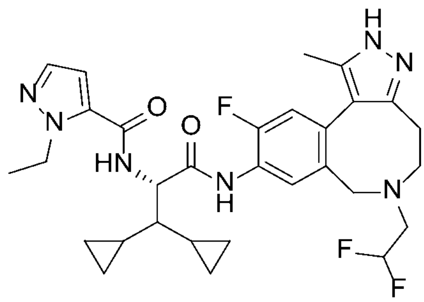,

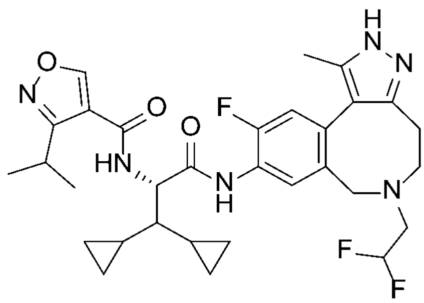,

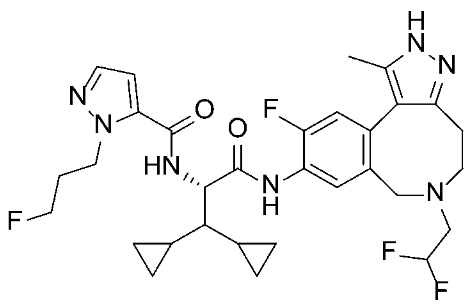,

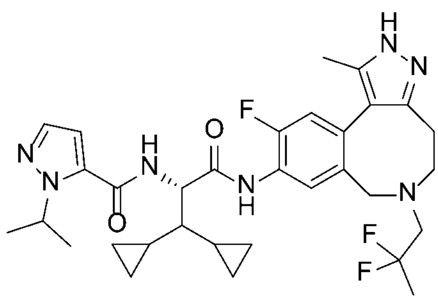,

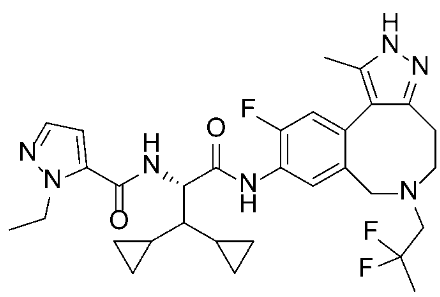,

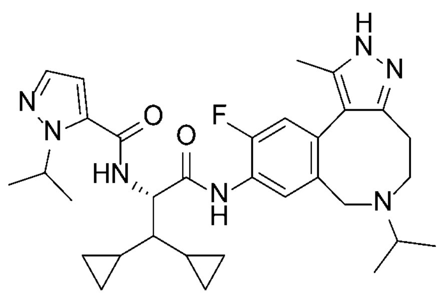,

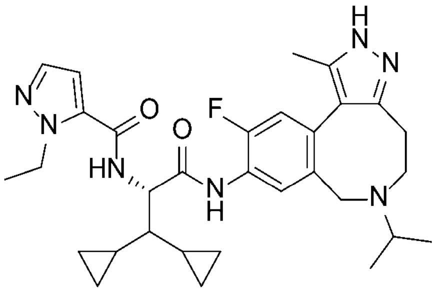,

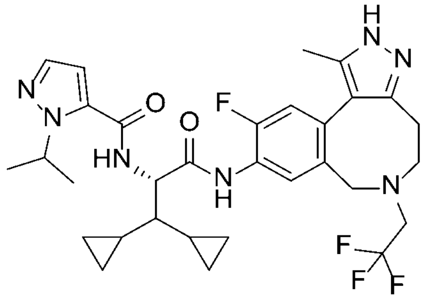,

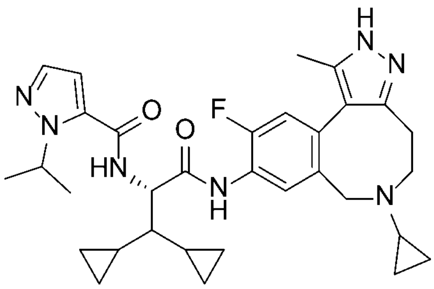,

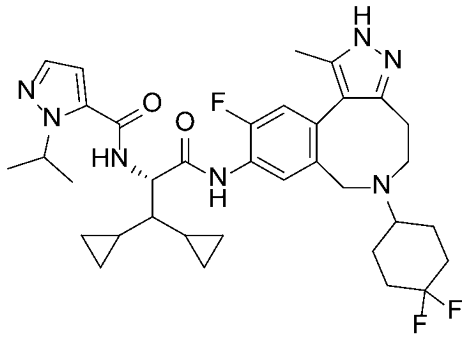,

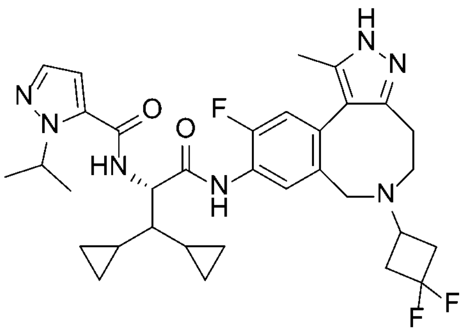,

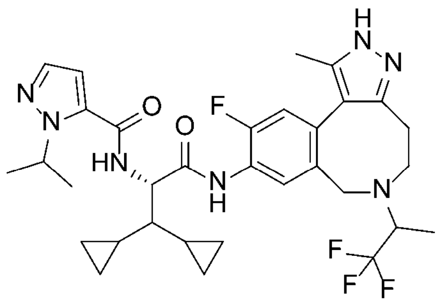,

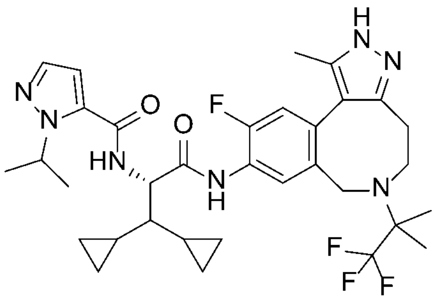,

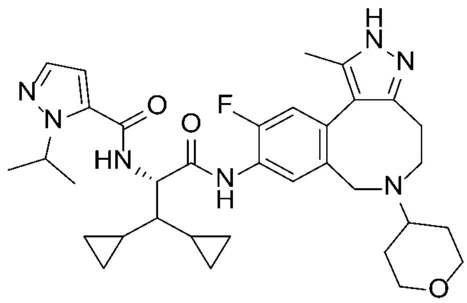,

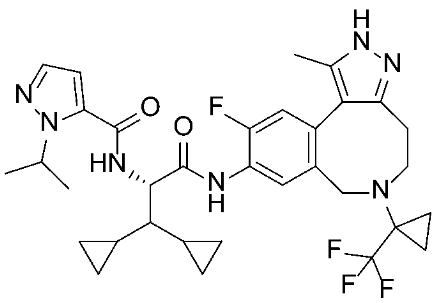,

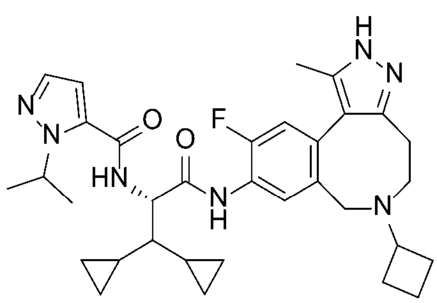,

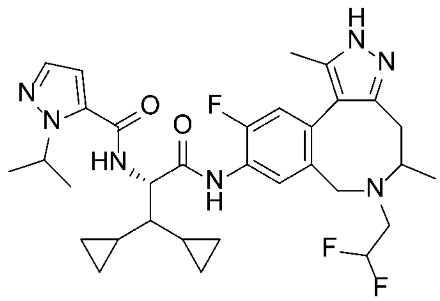,

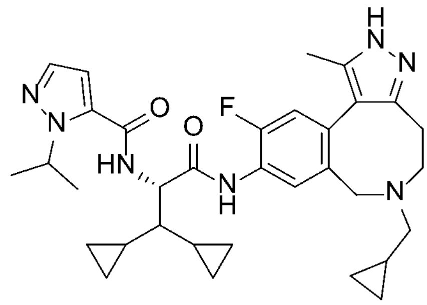,

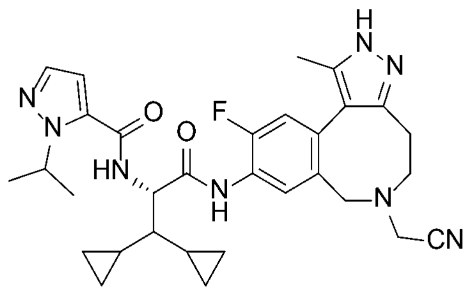,

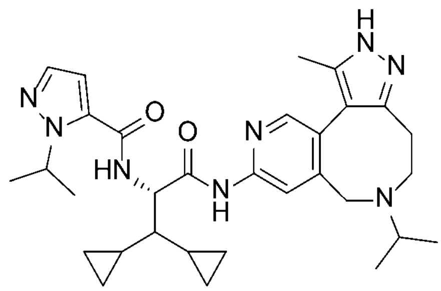,

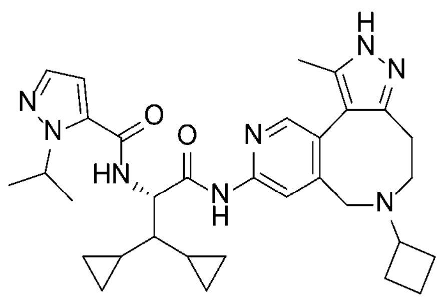,

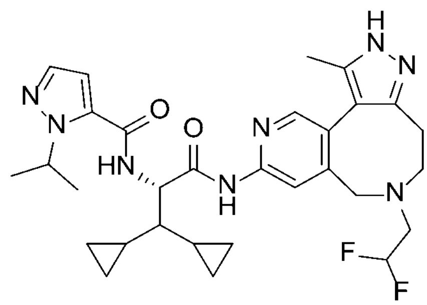,

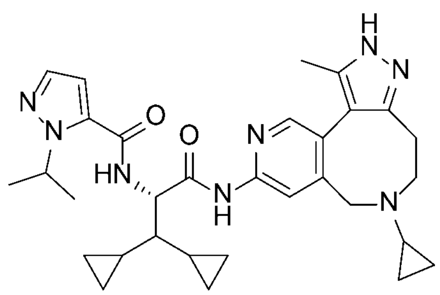,

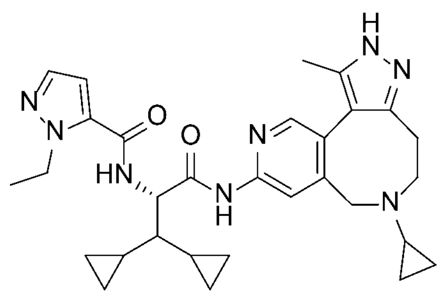,

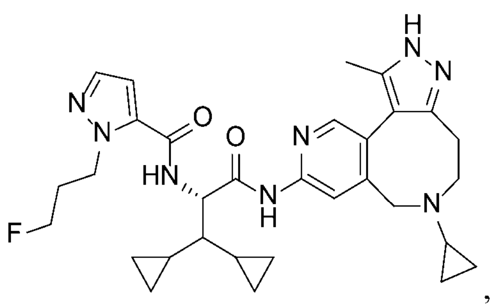,

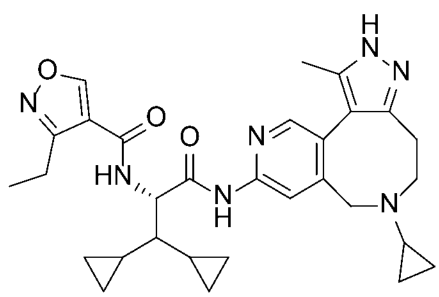,

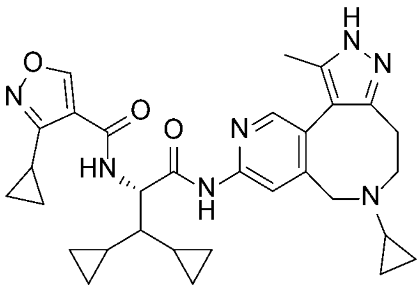,

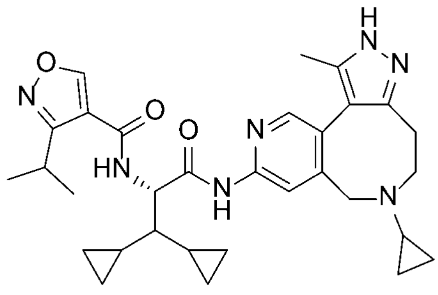,

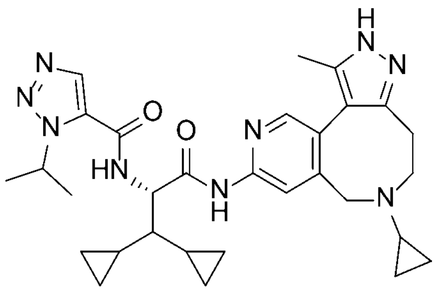,

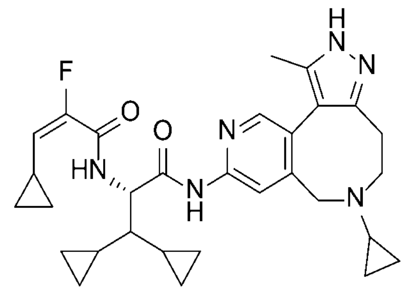,

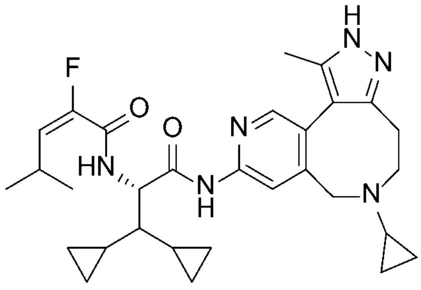,

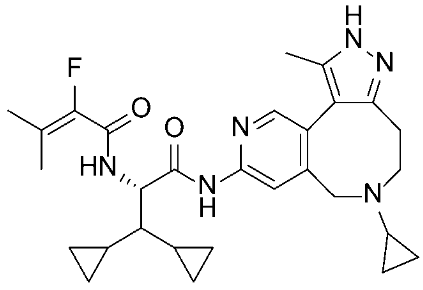,

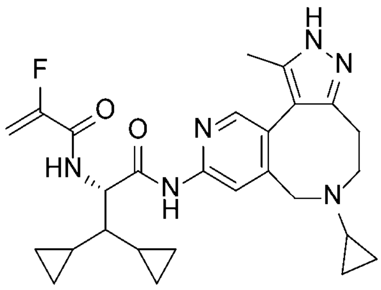,

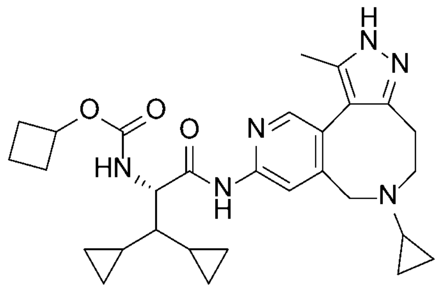,

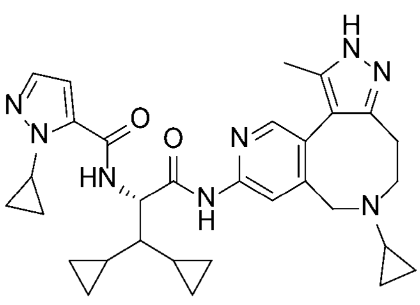,

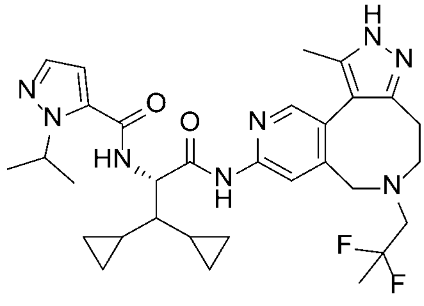,

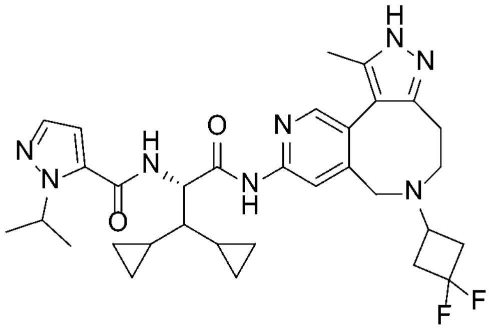,

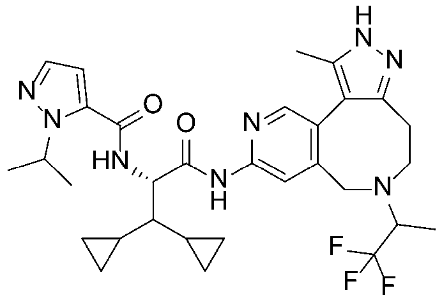,

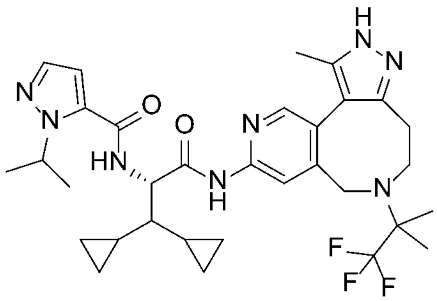,

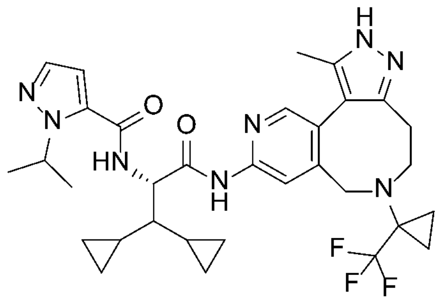,

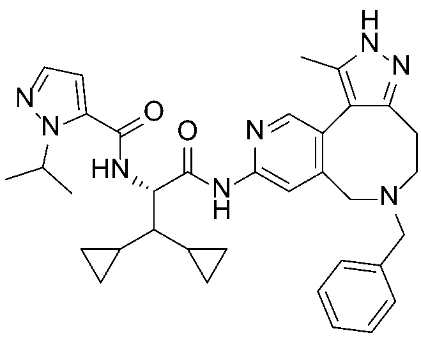,

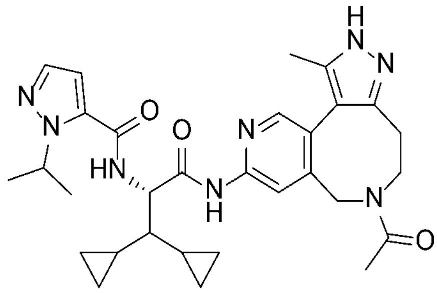,

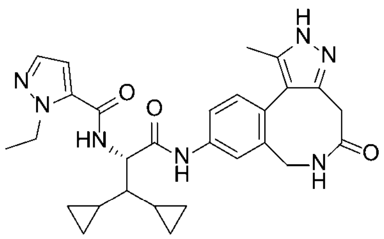,

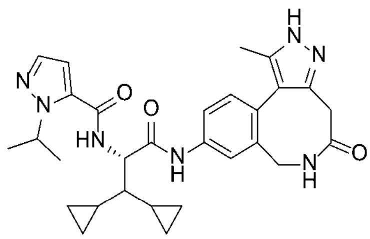,

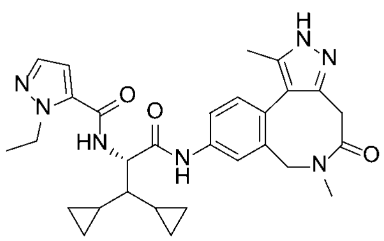,

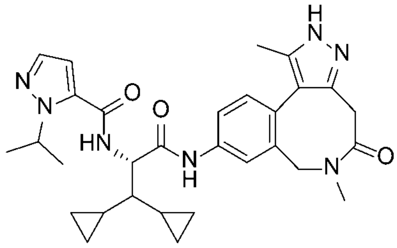,

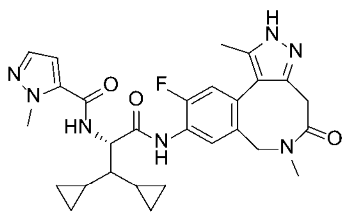
,

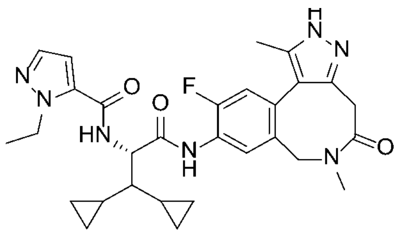
,

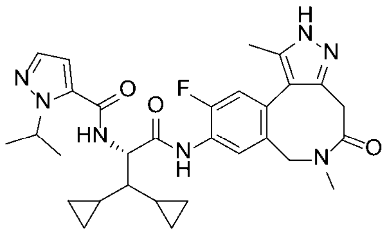
,

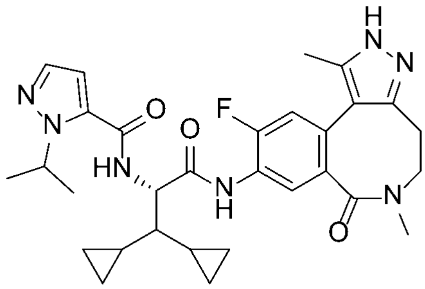
,

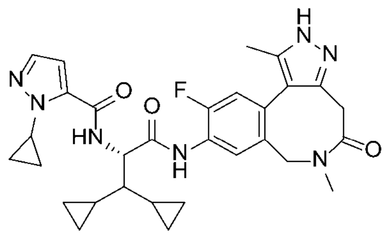
,

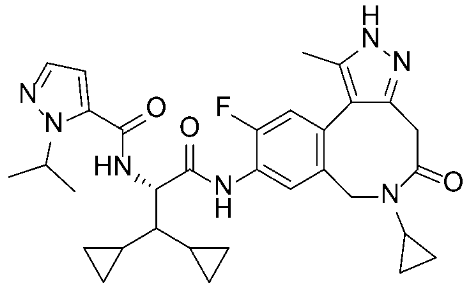
,

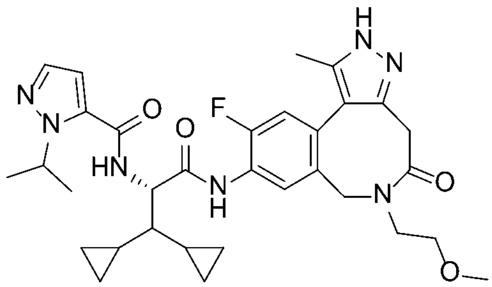
,

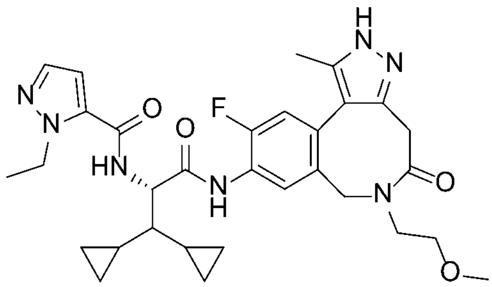
,

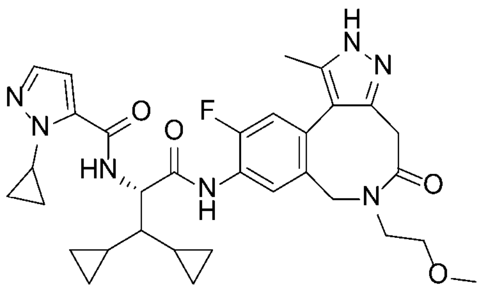
,

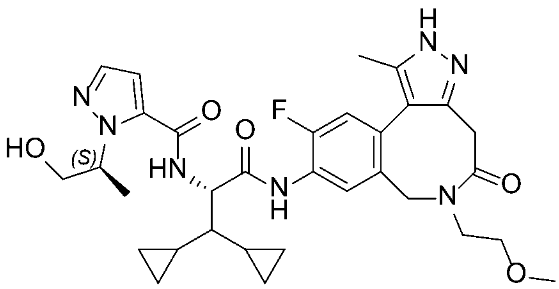
,

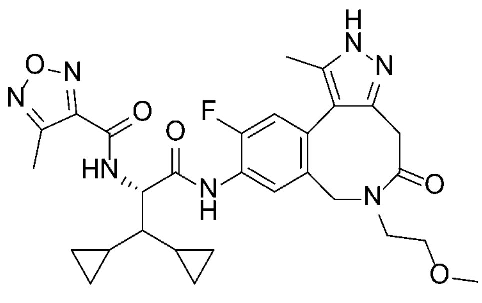,

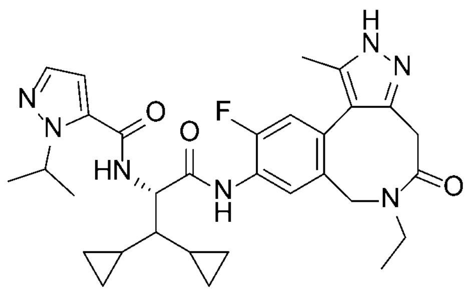,

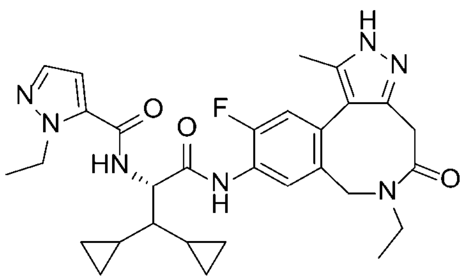,

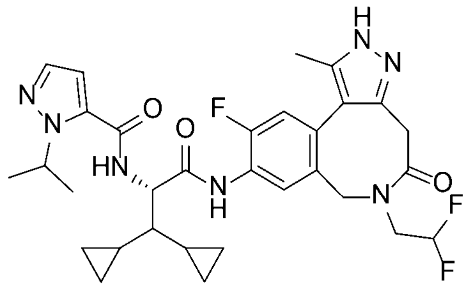,

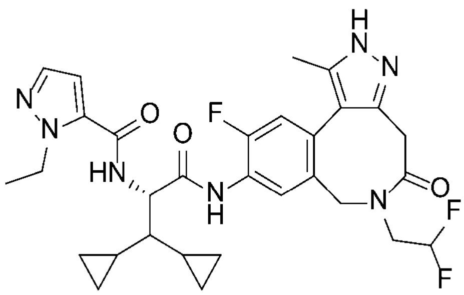,

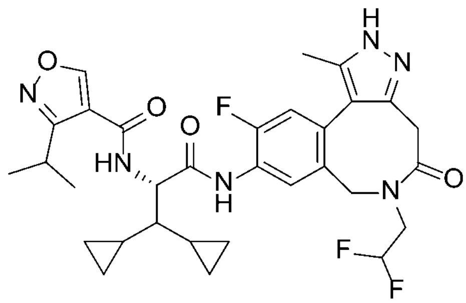,

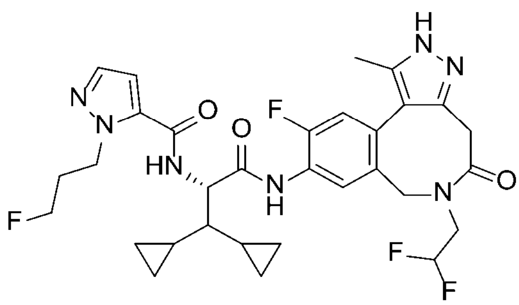,

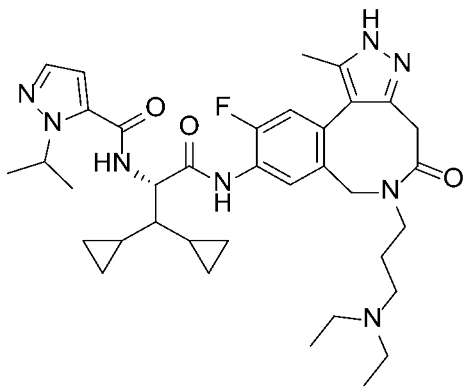,

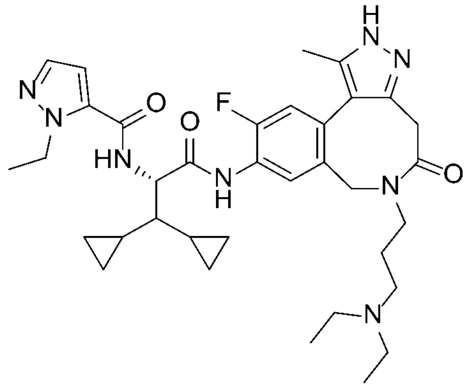,

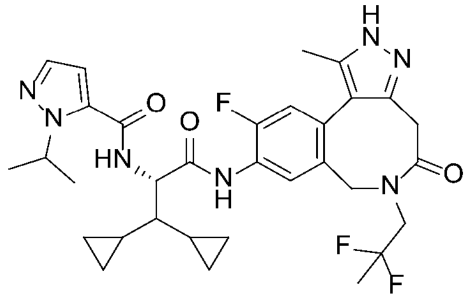,

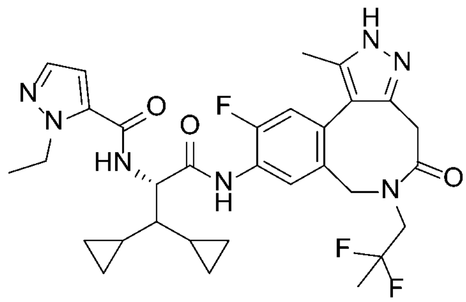,

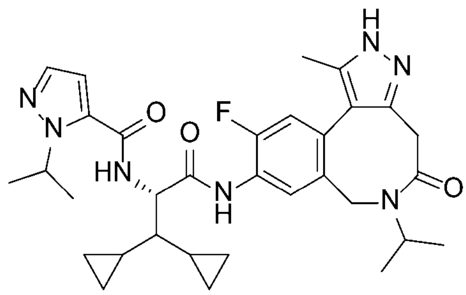,

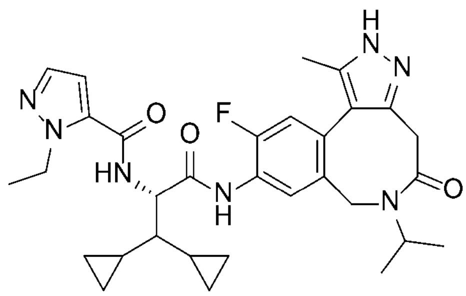,

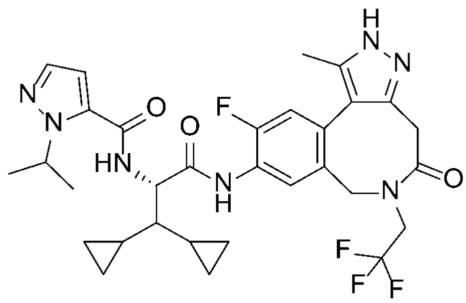,

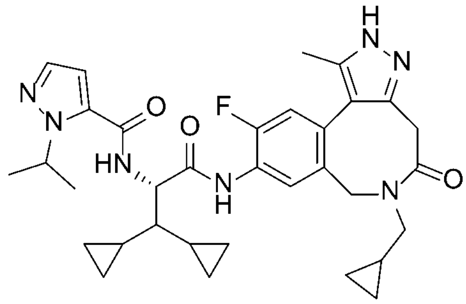,

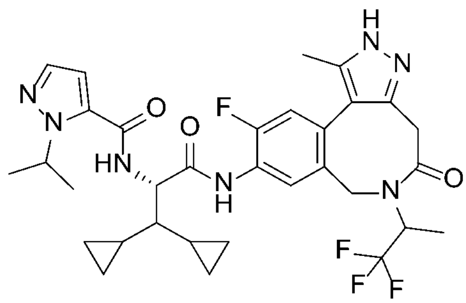,

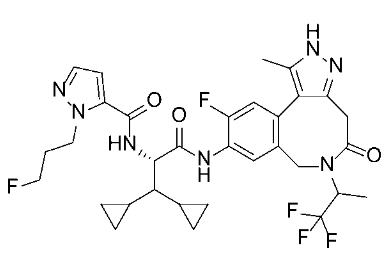,

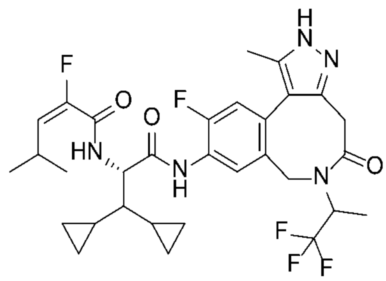,

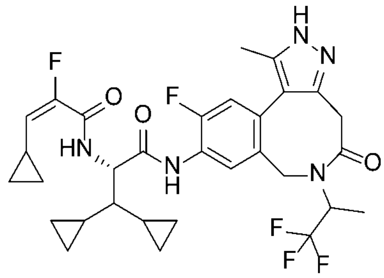,

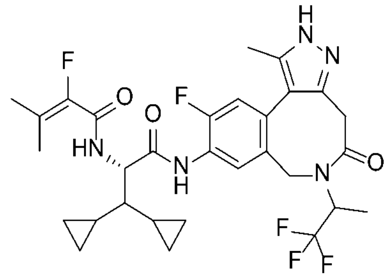,

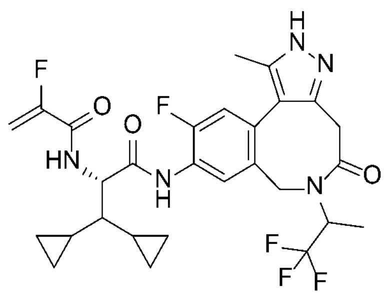,

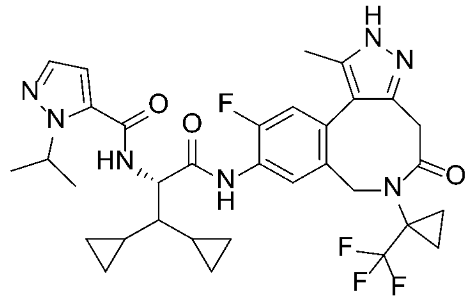,

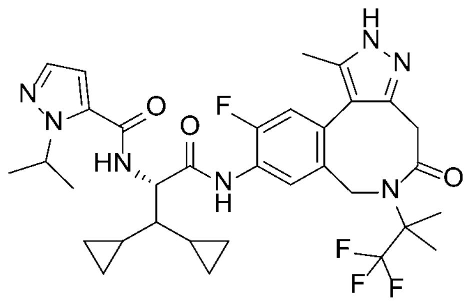,

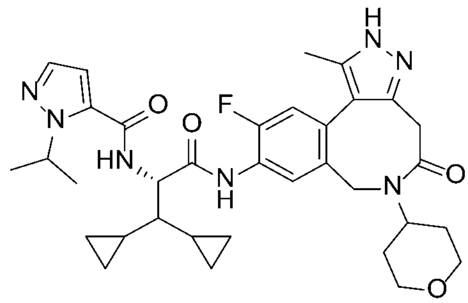,

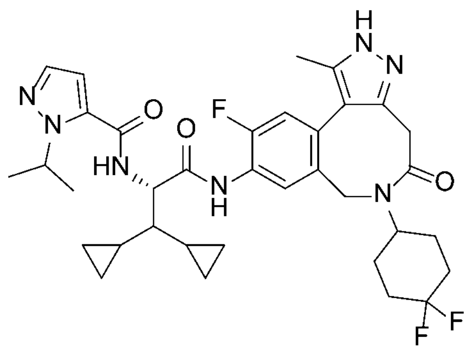,

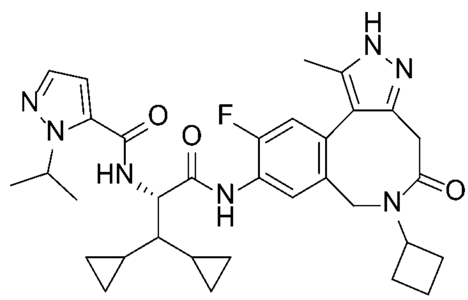,

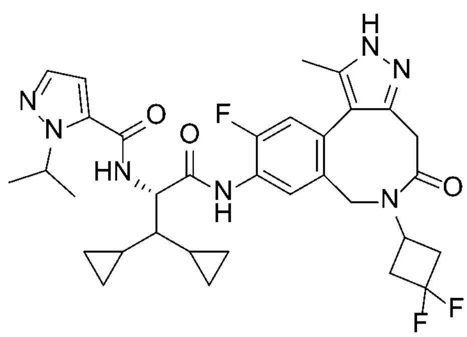,

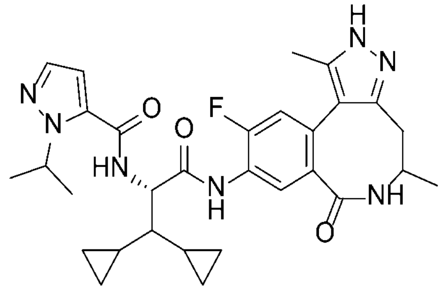,

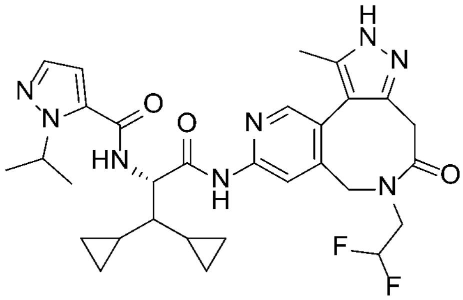

,

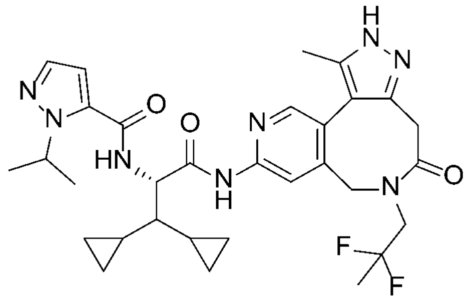

,

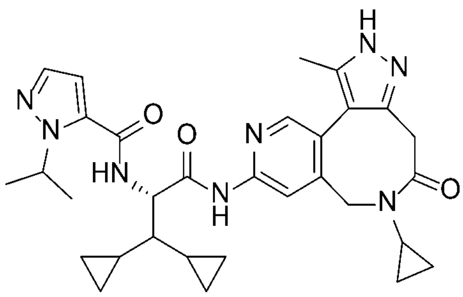

,

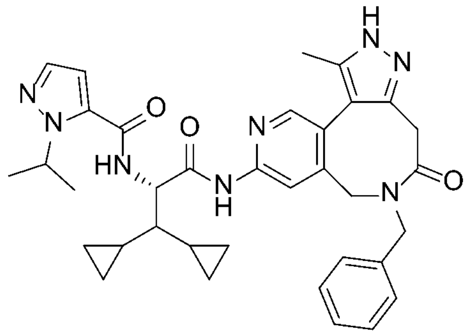

,

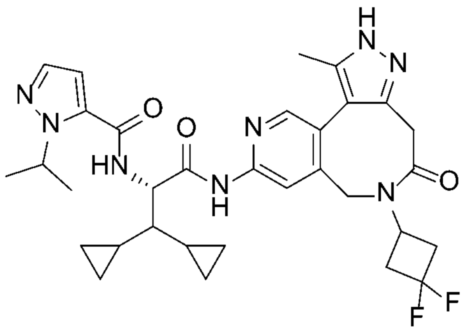

,

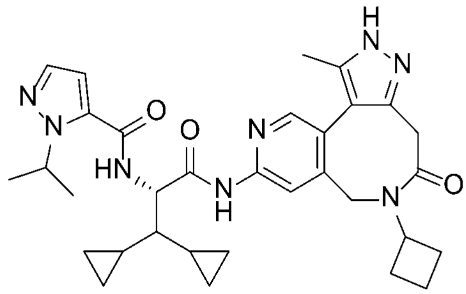

,

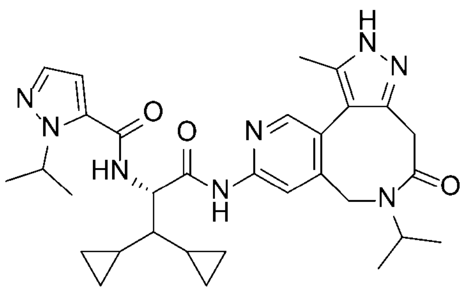

,

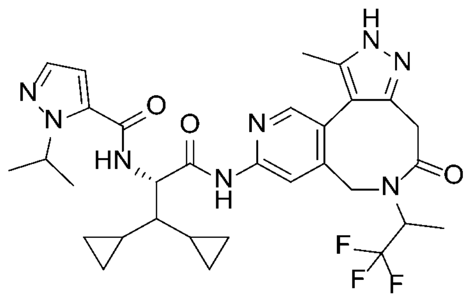

,

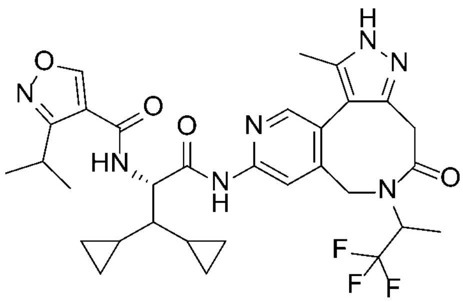

,

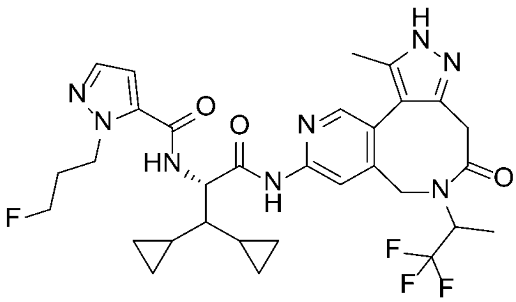

,

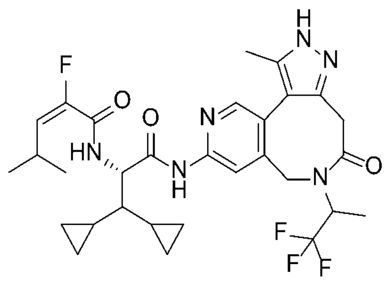
,
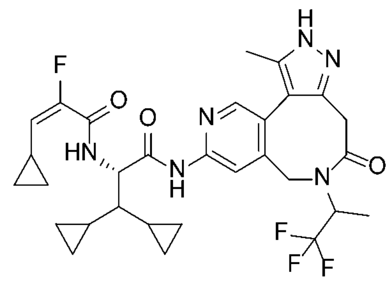
,
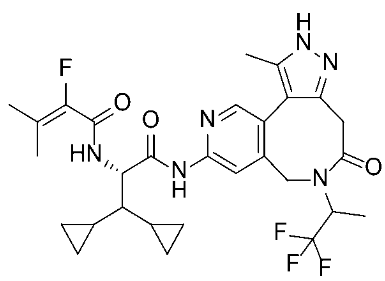
,
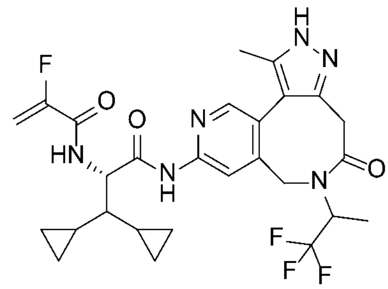
,
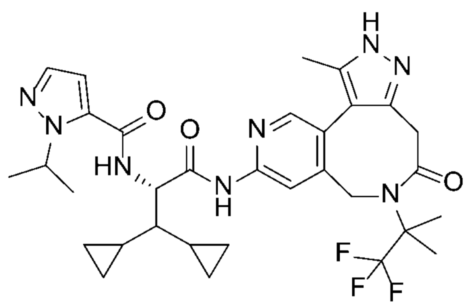
,
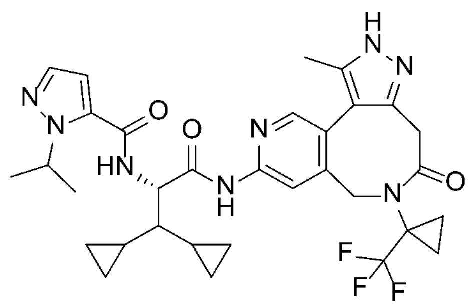
,
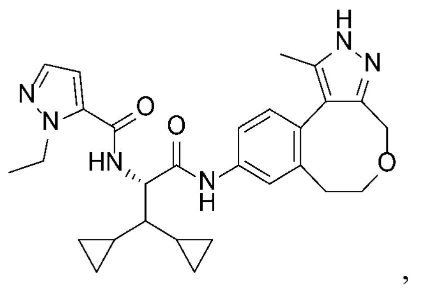
,
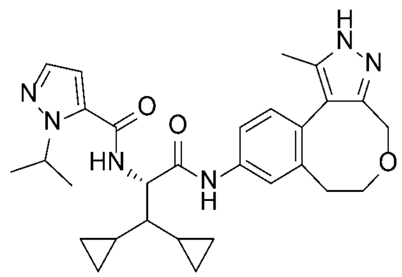
,
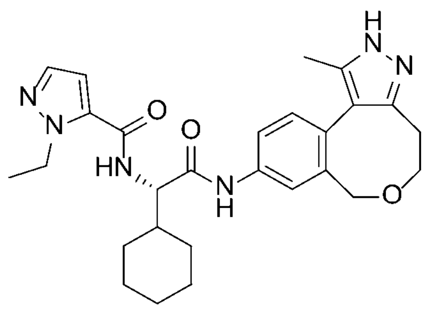
,
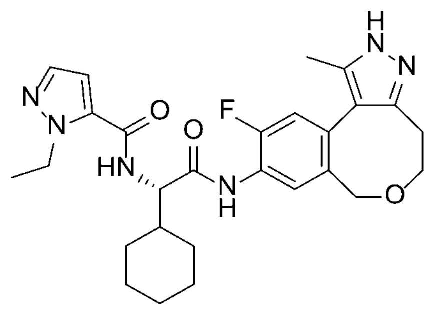
,

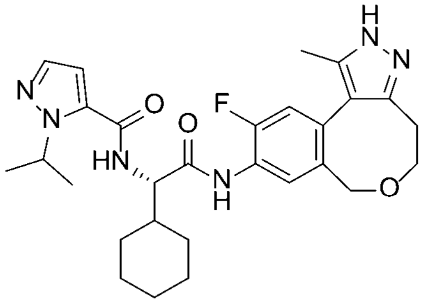
,
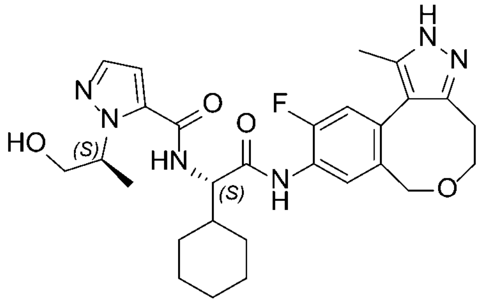
,

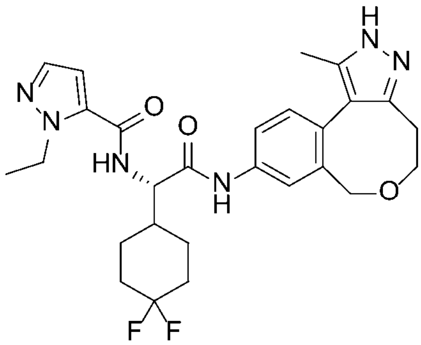
,
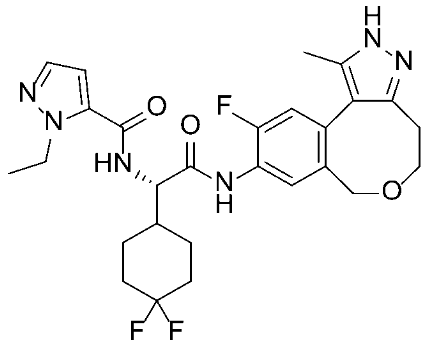
,

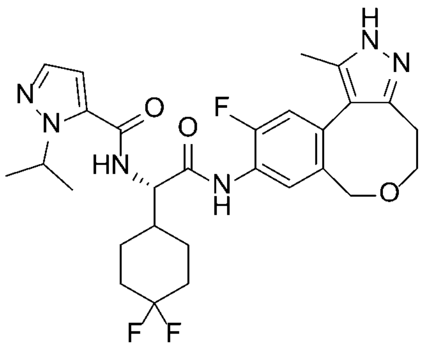
,
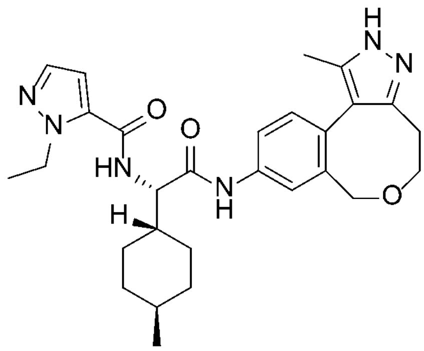
,

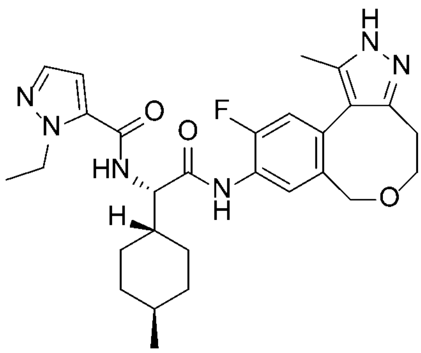
,
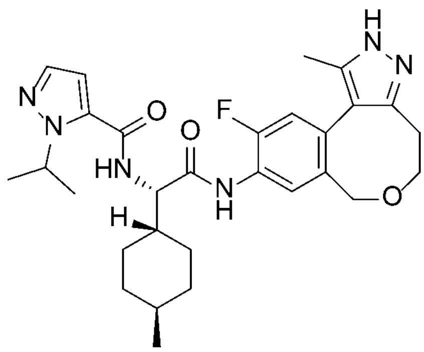
,

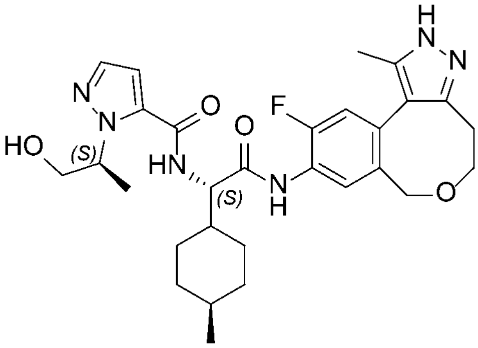
,
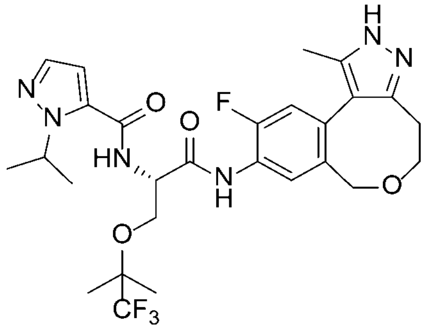
,

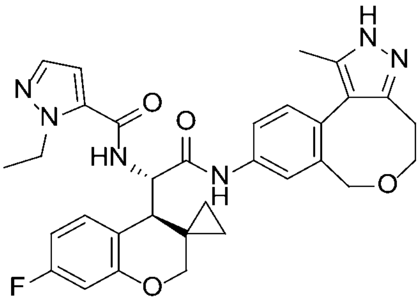
,
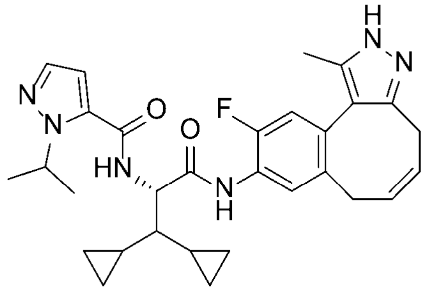
,

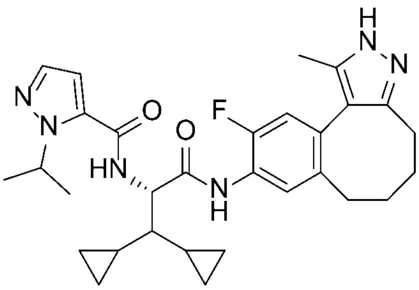
,
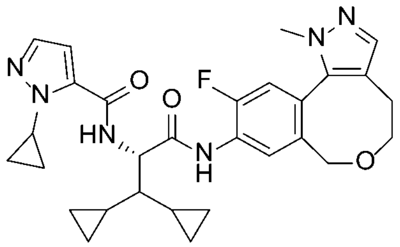

and

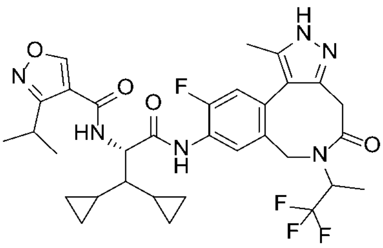
.

In a second aspect, the present disclosure provides a method for treating an interleukin-17A (IL-17A)-mediated disease in a subject in need thereof, comprising:
administering a therapeutically effective amount of the aforementioned compound, or a deuterated compound, a stereoisomer or a pharmaceutically acceptable salt thereof to the subject.
In some embodiments, the IL-17A-mediated disease is selected from the group consisting of inflammation, autoimmune disease, infectious disease, cancer, precancerous syndrome and a combination thereof.
In a third aspect, the present disclosure provides a pharmaceutical composition, comprising:

the abovementioned compound, or a deuterated compound, a stereoisomer or a pharmaceutically acceptable salt thereof; and
a pharmaceutically acceptable excipient.

In some embodiments, the IL-17A-mediated disease is selected from the group consisting of psoriasis, rheumatoid arthritis, spondyloarthritis, multiple sclerosis, psoriatic arthritis, axial spondyloarthritis, ankylosing spondylitis, hidradenitis suppurativa, systemic lupus erythematosus, palmoplantar pustulosis, atopic dermatitis, asthma and chronic obstructive pulmonary disease.

In a fourth aspect, the present disclosure provides a pharmaceutical composition, comprising:
the abovementioned compound, or a pharmaceutically acceptable salt thereof; and a pharmaceutically acceptable carrier, diluent or excipient.

In a fifth aspect, the present disclosure provides a method for treating an interleukin-17A (IL-17A)-mediated disease in a subject in need thereof, comprising:
administering a therapeutically effective amount of the aforementioned compound, or a deuterated compound, a stereoisomer, a pharmaceutically acceptable salt, a solvate, a prodrug or a metabolite thereof to the subject.

In a sixth aspect, the present disclosure provides a method for treating psoriasis in a subject in need thereof, comprising:
administering a therapeutically effective amount of the aforementioned compound or a pharmaceutically acceptable salt thereof to the subject.

In a seventh aspect, the present disclosure provides a method for treating or preventing inflammation, cancer or autoimmune disease in a subject in need thereof, comprising:
administering a therapeutically effective amount of the aforementioned compound to the subject.

In an eighth aspect, the present disclosure provides the aforementioned compound or the pharmaceutically acceptable salt thereof for treating psoriasis.

In a ninth aspect, the present disclosure provides an application of the aforementioned compound or the pharmaceutically acceptable salt thereof in the preparation of a drug for treating psoriasis.

The IL-17A-mediated disease is a disease in which IL-17a plays an important role in a pathogenesis of the disease. A primary function of IL-17A is to coordinate local tissue inflammation, thereby playing a role in various diseases. the IL-17A-mediated disease is selected from the group consisting of inflammation, autoimmune disease, infectious disease, cancer and precancerous syndrome.

The cancer or malignancy refers to any of a variety of diseases characterized by uncontrolled abnormal proliferation of cells. The affected cells of body or many characteristic structural and/or molecular features spread to other parts of the body locally or through the bloodstream and lymphatic system (i.e., metastasis). Cancer cells are cells that have undergone multiple steps of tumor progression in early, intermediate or late stages. Cancers include sarcoma, breast cancer, lung cancer, brain cancer, bone cancer, liver cancer, kidney cancer, colon cancer, and prostate cancer. In some embodiments, the compound of formula (I) is used to treat a cancer selected from the group consisting of colon cancer, brain cancer, breast cancer, fibrosarcoma, and squamous cell carcinoma. In some embodiments, the cancer is melanoma, breast cancer, colon cancer, lung cancer, and ovarian cancer. In some embodiments, the cancer being treated is a metastatic cancer.

The autoimmune diseases are caused by an immune response of body to substances and tissues normally present in the body. The autoimmune disease includes myocarditis, lupus nephritis, primary biliary cirrhosis, psoriasis, Type 1 diabetes mellitus, Grave's disease, celiac disease, Crohn's disease, autoimmune neutropenia, juvenile arthritis, rheumatoid arthritis, fibromyalgia, Guillain-Barré syndrome, multiple sclerosis, and autoimmune retinopathy. In this application, the autoimmune disease includes psoriasis and multiple sclerosis.

The inflammation includes a variety of conditions characterized by pathological inflammation of tissues, such as acne vulgaris, asthma, coeliac disease, chronic prostatitis, glomerulonephritis, inflammatory bowel disease, pelvic inflammatory disease, reperfusion injury, rheumatoid arthritis, sarcoidosis, vasculitis, airway inflammation due to house dust mites, and interstitial cystitis. There is a significant overlap between the inflammation and the autoimmune diseases. In this application, the inflammation includes asthma. The immune system is often involved in inflammatory diseases, which are manifested in allergic reactions and some myopathies. Many immune system diseases cause abnormal inflammation. The IL-17A-mediated disease also includes autoimmune inflammatory diseases.

The compounds and derivatives provided herein are named according to the nomenclature system of the International Union of Pure and Applied Chemistry (IUPAC) or Chemical Abstracts Service (CAS), Columbus, Ohio.

Unless otherwise specified, the definition of terms in this disclosure apply to the terms throughout the specification. Terms that are not specifically defined herein can be understood by the skilled in the art based on the disclosure
The term "substitute" indicates the replacement of a hydrogen atom in a molecule by a different atom or group; or the replacement of a lone pair of electrons of an atom in a molecule by another atom or group. For example, a lone pair of electrons on an S atom can be replaced with an O atom to form

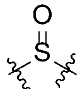 or 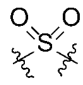.

The limitation "capable of being substituted" indicates that a "substitution" may occur, but not necessary. The description includes instances where it does or does not occur. A minimum and a maximum of a content of carbon atoms in a hydrocarbon group are indicated by the prefix. For example, a prefix $C_{a-b}$ alkyl indicates any alkyl group containing a-b carbon atoms, i.e., $C_{1-6}$ alkyl indicates that the alkyl group contains 1-6 carbon atoms.

The alkyl refers to a saturated hydrocarbon chain having the specified number of member atoms. The alkyl group can be straight-chain or branched. Representative

branched alkyl groups have one, two or three branched chains. The alkyl group may optionally be substituted by one or more substituents as defined herein. The alkyl includes methyl, ethyl, propyl (n-propyl and isopropyl), butyl (n-butyl, isobutyl and tert-butyl), pentyl (n-pentyl, isopentyl and neopentyl) and hexyl. The alkyl can also be a part of other groups, and the other groups includes -O($C_{1-6}$ alkyl).

The alkylene means a divalent saturated aliphatic hydrocarbon group having a specified number of membered atoms. The $C_{a-b}$ alkylene refers to alkylene groups with a-b carbon atoms. The alkylene includes branched and straight chain alkyl. For example, propylidene is

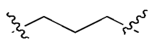,

and dimethylbutylidene is

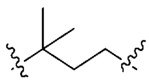 or 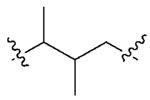.

The $C_{0-2}$ alkylene is alkylene with $C_0$, alkylene with $C_1$ (such as $-CH_2-$), or alkylene with $C_2$ (such as $-CH_2CH_2-$). $C_0$ alkylene refers to the absence of the group here, and a connection here is chemical bonding. For example, A-$C_0$ alkylene-B refers to A-B, that is, A is directly connected to B through a chemical bond.

The cycloalkyl and cycloalkane indicate a saturated or partially saturated cyclic group having a carbon atom and no heterocyclic atom, and having a single ring or a plurality of rings (including thickening and bridging). The terms "cycloalkyl" and "cycloalkane" include cycloalkenyl groups, such as cyclohexenyl. The cycloalkyl includes adamantyl, cyclopropyl, cyclobutyl, cyclohexyl, cyclopentyl, cyclooctyl, cyclopentenyl and cyclohexenyl. The cycloalkyl including a polybicycloalkyl ring system includes dicyclohexyl, dicyclopentyl and dicyclooctyl. For example,

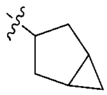 and 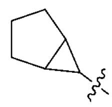

are dicyclohexyl.

The cycloalkyl and cycloalkane also include partially saturated cyclic group where an aromatic ring is fused with a non-aromatic ring, and an attachment site may be at a non-aromatic carbon atom or an aromatic carbon atom. For example, 1,2,3,4-tetrahydronaphthalen-5-yl and 5,6,7,8-tetrahydronaphthalen-5-yl.

The term "unsaturated" means that the group or molecule includes carbon-carbon double bond, carbon-carbon triple bond, carbon-oxygen double bond, carbon-sulfur double bond, carbon-nitrogen triple bond, etc. The unsaturated carbocyclic herein includes or excludes aryl groups, and the unsaturated heterocyclic includes or excludes heteroaromatic ring groups, as the skilled in the art may freely choose.

The alkenyl refers to a straight or branched hydrocarbon group having 2-10 carbon atoms, 2-6 carbon atoms or 2-4 carbon atoms, and having at least one vinyl unsaturated site (>C=C<). For example, $C_{a-b}$ alkenyl is an alkenyl with a-b carbon atoms, such as vinyl, propenyl, isopropenyl and 1,3-butadienyl.

The alkynyl is a linear monovalent hydrocarbon radical or a branched monovalent hydrocarbon radical containing at least one triple bond. The term "alkynyl" includes those alkyl having a triple bond and a double bond. For example, $C_{2-6}$ alkynyl includes ethynyl and propargyl.

The halogen is fluorine, chlorine, bromine or iodine.

The terms "haloalkyl" and "halogen-substituted alkyl" refer to alkyl in which the hydrogen atom may be replaced with one or more halogen atoms. For example, halogen-substituted $C_{1-4}$ alkyl refers to alkyl containing 1-4 carbon atoms

with hydrogen atoms substituted by one or more halogen atoms, as well as monofluoromethyl, difluoromethyl and trifluoromethyl.

The heterocyclylalkyl, heterocyclic, heterocyclic alkane indicate a saturated ring or a non-aromatic partially saturated ring containing at least one heteroatom and having a single ring or multiple rings (dense and bridged). The heteroatom includes nitrogen (N), oxygen and sulfur. For example, monovalent saturated or partially unsaturated monocyclic or bicyclic ring systems of a plurality of ring atoms, which includes 1, 2 or 3 ring heteroatoms selected from the group consisting of N, O and S, and the remaining ring atoms are carbon. Bicycles represent a chain consisting of two rings with two ring atoms in common, that is, a bridge separating the two rings is either a single bond or one or two ring atoms. The monocyclic saturated heterocyclic alkyl includes oxetanyl, azetidinyl, pyrrolidinyl, 2-oxo-pyrrolidin-3-yl, tetrahydropyranyl, tetrahydro-thienyl, pyrazolidinyl, imidazolidinyl, thiazolidinyl, piperidinyl, tetrahydropyranyl, tetrahydrothiopyranyl, piperazinyl, morpholinyl,

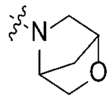,

thiomorpholinyl, 1,1-dioxo-thiomorpholin-4-yl, azepanyl, diazepanyl, homopiperazinyl and oxazepanyl. The bicyclic saturated heterocyclylalkyl includes 8-azabicyclo[3.2.1]octan, quinuclidinyl, 8-oxa-3-azabicyclo[3.2.1]octan and 9-azabicyclo[3.3.1]nonane. The partially unsaturated heterocyclylalkyl includes dihydrofuranyl, imidazolinyl, tetrahydropyridyl and dihydropyranyl. The heterocyclylalkyl also includes a partially saturated cyclic group formed by an association of an aromatic ring including at least one heteroatom with a non-aromatic ring, in which a linkage site is located at a non-aromatic carbon atom, an aromatic carbon atom or a heteroatom, such as

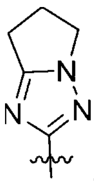, 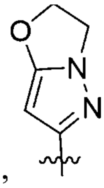, 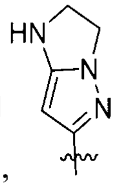

, 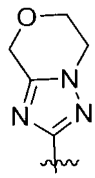 and 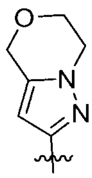.

The aryl and aromatic ring refers to an aromatic group with multiple carbon atoms. The aryl is usually a monocyclic, bicyclic or tricyclic aryl having a plurality of carbon atoms, such as benzene ring, naphthyl and tetrahydronaphthyl.

The heteroaromatic ring is an aromatic unsaturated ring containing at least one heteroatom. The heteroatom is nitrogen atoms, oxygen atoms, sulfur atoms, etc. For example, aromatic monocyclic or bicyclic hydrocarbons with a plurality of ring atoms and one or more of the ring atoms being selected from the group consisting of O, N and S. Preferably, there are 1-3 heteroatoms. The heteroaromatic ring includes pyridinyl, indolyl, quinoxalinyl, quinolinyl, isoquinolinyl, benzothienyl, benzofuranyl, benzothienyl, benzothiopyranyl, benzothiopyranyl, furanyl, pyrrolyl, thiazolyl, oxazolyl, isoxazolyl, triazolyl, tetrazolyl, pyrazolyl, imidazolyl, thienyl, oxadiazolyl, benzimidazolyl, benzothiazolyl and benzoxazolyl.

The stereoisomer includes enantiomer and diastereoisomer.

The -OR and -NRR indicate that the R group is connected to the O or N atom by a single bond.

The -C(O)R and -$S(O)_2$R indicate that the O is connected to the C or S atom by a double bond, and the R is connected to the C or S atom by a single bond.

The =O and =S indicate that the oxygen and sulfur atoms are connected to a substitution position by a double bond.

The --- and 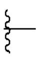 are indicate the substitution position of groups.

The deuterated compound refers to a substitution of one or more hydrogen atoms in a molecule or group by deuterium atoms, where the percentage of deuterium atoms is greater than an abundance of deuterium in nature.

The limitation "pharmaceutically acceptable" refers to a carrier, delivery agent, diluent, excipient, and/or salt are generally chemically or physically compatible with the other ingredients including a pharmaceutical dosage form, and are physiologically compatible with the receptor.

The terms "salt" and "medicinal salt" refer to the acid and/or base salts formed by the above compounds or their stereoisomers with inorganic and/or organic acids and bases. It includes amphoteric salts (internal salts) and quaternary ammonium salts such as alkyl ammonium salts. The salts can be directly obtained in a final isolation and purification of the compound, or obtained by mixing the above compounds or their stereoisomers with an appropriate amount of acid or base (e.g. in equivalent amounts). The salts may be collected by filtration as precipitates in solution, recovered by evaporation of the solvent, or freeze-dried after reaction in aqueous media. The salt provided herein includes sodium, potassium, hydrochloride, sulfate, citrate, benzenesulfonate, hydrobromide, hydrofluorate, phosphate, acetate, propionate, succinate, oxalate, malate, succinate, fumarate, maleate, tartarate and trifluoroacetate of the compound.

In some embodiments, one or more compounds of the present disclosure may be used in combination with each other. Optionally, the compounds may also be used in combination with any other active agent for the preparation of drugs or pharmaceutical compositions that modulate cellular function or treat disease. If a group of compounds is used, the compounds may be administered to the subject simultaneously, separately or in an ordered manner.

Described above are merely illustrative of the disclosure, and are not intended to limit the disclosure. Those skilled in the art could still make modifications and changes to the embodiments of the disclosure.

The disclosure will be described in detail below with reference to the embodiments and accompanying drawings. It should be understood that the scope of the present disclosure is defined by the appended claims.

## DETAILED DESCRIPTION OF EMBODIMENTS

The present disclosure will be further illustrated with reference to the embodiments. It should be noted that the embodiments cannot be understood as limitations of this disclosure. Other embodiments performed based on the following embodiments without departing from the spirit and principle of the present disclosure shall fall within the scope of the disclosure defined by the appended claims.

The structure of compound is determined by nuclear magnetic resonance (NMR) and mass spectrometry (MS). A displacement in NMR is $10^{-6}$ (ppm). NMR equipment is Bruker AvanceIII 400 MHz and Bruker Avance NEO 600 MHz. A solvent includes dimethyl sulfoxide-$d_6$ (DMSO-$d_6$), deuterated chloroform ($CDCl_3$) and deuterated methanol ($CD_3OD$). An internal standard substance is tetramethylsilane (TMS).

Liquid chromatograph mass spectrometry (LC-MS) adopted Shimadzu LC-MS 2020 (ESI). High performance liquid chromatography (HPLC) adopted Shimadzu LC-20A. Medium pressure preparative liquid chromatography (MPLC) adopted Gilson GX-281 reversed-phase chromatograph. A silica gel plate adopted Yantai Huanghai HSGF254 or Qingdao GF254 silica gel plate. A size of a product for thin layer chromatography is 0.4-0.5 mm. A column chromatography adopted Yantai Huanghai silica gel 200-300 mesh silica gel as the carrier. Supercritical fluid chromatography (SFC) analysis and preparation were performed using a SHIMADZU SFC-30A instrument.

A raw material can be synthesized using methods known in the art, or can be purchased from companies such as Anegis Chemical, Chengdu Kolon Chemical, Shaoyuan Chemical Technology, J&K Chemical Technology, etc.

Unless otherwise specified, reaction was carried out under a nitrogen atmosphere, a solution is an aqueous solution, a temperature of the reaction is room temperature, the room temperature is the most suitable reaction temperature, i.e., 20-30°C, and M is moles per liter.

DMF is N,N-dimethylformamide. DCM is dichloromethane. TFA is trifluoroacetic acid. MeOH is methanol. $Cs_2CO_3$ is cesium carbonate. DIPEA is N,N-diisopropylethylamine. HATU is O-(7-Azabenzotriazol-1-yl)-N,N,N',N'-tetramethyluronium hexafluorophosphate. SEM-Cl is 2-(trimethylsilyl) ethoxymethyl chloride. $LiAlH_4$ is lithium aluminum hydride. TBS-Cl is tert-butyldimethylchlorosilane. THF is tetrahydrofuran. NBS is N-bromosuccinimide. $(Boc)_2O$ is di-tert-butyl dicarbonate. $Na_2CO_3$ is sodium carbonate. $B_2pin_2$ is pinacol diborate. AcOK is potassium acetate. $Pd(dppf)_2Cl_2$ is [1,1'-bis(dibenzene ringphosphino)ferrocene]palladium(II) dichloride. n-BuLi is n-butyllithium. $MgSO_4$ is magnesium sulfate. CsF is cesium fluoride. TMSCN is trimethylsilyl cyanide. EA/HCl is a solution of hydrogen chloride in ethyl acetate. Cbz-Osu is N-(benzyloxycarbonyloxy)succinimide. $K_2CO_3$ is potassium carbonate. $H_2O_2$ is hydrogen peroxide. DMSO is dimethyl sulfoxide. NMP is N-methylpyrrolidone. LiOH ▪ $H_2O$ is lithium hydroxide monohydrate. $Pd(PPh_3)_4$ is tetrakis(tribenzene ringphosphine)palladium(0). $SOCl_2$ is thionyl chloride. t-BuOK is potassium tert-butoxide. t-BuOH is tert-butyl alcohol. Pd/C is palladium on carbon. $HCOONH_4$ is ammonium formate. $(COCl)_2$ is oxalyl chloride. EDCI is 1-(3-dimethylaminopropyl)-3-ethylcarbodiimide hydrochloride. DIAD is diisopropyl azodicarboxylate. $PPh_3$ is tribenzene ringphosphine. Zn is zinc powder. $NH_4Cl$ is ammonium chloride. $Ti(OEt)_4$ is titanium tetraethoxide. Raney Ni is Raney nickel. $Pd_2(dba)_3$ is tris(dibenzylideneacetone)dipalladium(0). $Pd(amphos)Cl_2$ is [1,1'-bis(di-tert-butylphosphino)ferrocene]palladium(II) dichloride. $Pd(OAc)_2$ is palladium(II) acetate. $Pd(OH)_2$ is palladium(II) hydroxide. CyJohnphos is 2-(dicyclohexylphosphino)bibenzene ring. rac-BI-DIME is 3-(tert-butyl)-4-(2,6-dimethoxybenzene ring)-2,3-dihydrobenzo[d][1,3]oxaphosphole. CbzCl is benzyl chloroformate. $NaBH(OAc)_3$ is sodium triacetoxyborohydride. $K_3PO_4$ is potas-

sium phosphate. Py is pyridine. Selectfluor is 1-(chloromethyl)-4-fluoro-1,4-diazoniabicyclo[2.2.2]octane bis(tetrafluoroborate).

**Preparation of intermediate A-1**

A preparation of intermediate A-1 is illustrated as follows:

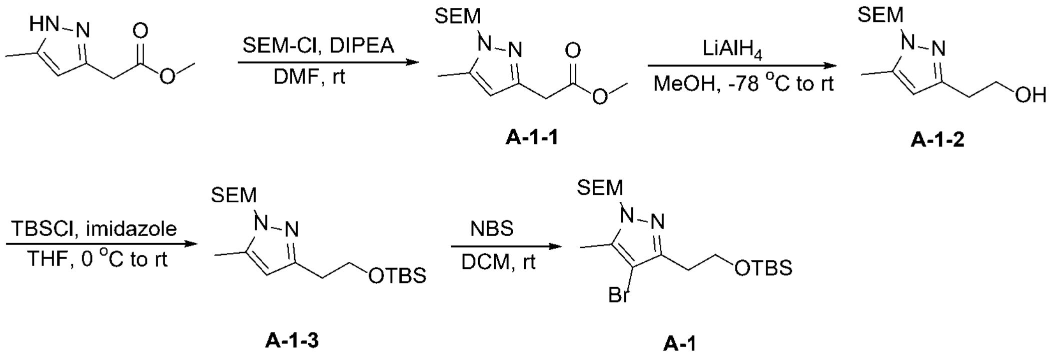


(S1) Preparation of intermediate A-1-1

To a solution of methyl 2-(5-methyl-1H-pyrazol-3-yl)acetate (2.83 g, 18.36 mmol) and DIPEA (11.86 g, 91.78 mmol, 15.99 mL) in DMF (60 mL) was dropwise added SEM-Cl (3.67 g, 22.03 mmol) at 0°C. The reaction mixture was heated to room temperature, stirred overnight, and subjected to extraction by water and EA. The EA layer was collected, washed with a NaCl solution, concentrated to dryness, and purified by silica gel column chromatography to obtain 4.6 g of intermediate A-1-1 (88% yield). MS (ESI) m/z = 285 $[M+1]^+$.

(S2) Preparation of intermediate A-1-2

To a solution of intermediate A-1-1 (4.3 g, 15.12 mmol) in THF (150 mL) was batchwise added $LiAlH_4$ (1.15 g, 30.24 mmol) at -78°C. The reaction mixture was heated to room temperature and reacted for 1 h. Upon completion, the reaction mixture was cooled to -10°C and quenched sequentially with water (2 mL), 15% aqueous NaOH solution (2 mL), and water (6 mL). After that, the reaction mixture was stirred at room temperature for 10 min and filtered. The filter cake was collected, washed with EA. The filtrate was collected, concentrated to dryness to obtain 3.8 g of intermediate A-1-2 (98% yield), which was used directly in next step without further purification. MS (ESI) m/z = 257 $[M+1]^+$.

(S3) Preparation of intermediate A-1-3

To a solution of intermediate A-1-2 (3.8 g, 14.82 mmol) in THF (50 mL) at 0°C were added TBSCl (4.47 g, 29.64 mmol) and imidazole (2.02 g, 29.64 mmol). The reaction mixture was stirred at room temperature for 2 h. Upon completion, the reaction mixture was subjected to extraction by water and EA. The EA layer was collected, washed with a saturated NaCl solution and concentrated to dryness. The residue was purified by silica gel column chromatography to obtain 4.77 g of intermediate A-1-3 (87% yield), which was used directly in next step without further purification. MS (ESI) m/z = 371 $[M+1]^+$.

(S4) Preparation of intermediate A-1

To a solution of intermediate A-1-3 (4.77 g, 12.87 mmol) in DCM (60 mL) was added NBS (2.7 g, 15.17 mmol). The reaction mixture was stirred at room temperature for 1 h. Upon completion, the reaction mixture was quenched with an aqueous sodium bicarbonate solution, and subjected to extraction by DCM. The DCM layer was collected, washed with a NaCl solution and concentrated to dryness. The residue was purified by silica gel column chromatography to obtain 5.5 g of intermediate A-1 (95% yield). MS (ESI) m/z = 449 $[M+1]^+$.

**Preparation of intermediate A-2**

A preparation of intermediate A-2 is illustrated as follows:

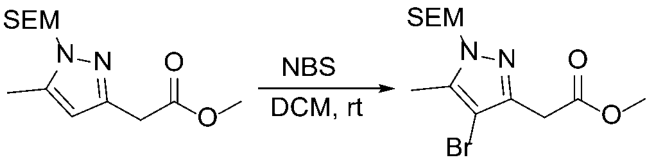


A-1-1 A-2

To a 50 mL single-necked flask were added intermediate A-1-1 (206 mg, 724.27 μmol), DCM (7 mL), and NBS (141.80 mg, 796.69 μmol) at room temperature. The reaction mixture was stirred at room temperature and reacted overnight. Upon completion, the reaction mixture was quenched with an aqueous sodium bicarbonate solution, and extracted with DCM. The DCM layer was collected, washed with a NaCl solution, dried over anhydrous sodium sulfate, filtered, and concentrated to dryness under reduced pressure. The residue was purified by silica gel column chromatography (PE/EA 10:1 to 5:1) to obtain 150 mg (412.86 μmol) of intermediate A-2 (57.0% yield). MS (ESI) m/z = 363 $[M+1]^+$.

__Preparation of intermediate A-3__

A preparation of intermediate A-3 is illustrated as follows:

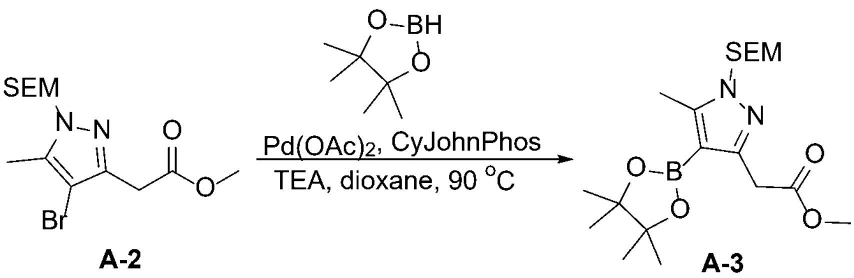


A-2 A-3

To a solution of intermediate A-2 (600 g, 1.65 mol) in 1,4-dioxane (6 L) were sequentially added pinacolborane (570.63 g, 4.46 mol), $Pd(OAc)_2$ (11.22 g, 49.54 mmol), CyJohnphos (34.73 g, 99.09 mmol), and triethylamine (250.66 g, 2.48 mol, 345.50 mL). The reaction mixture was evacuated and purged with nitrogen three times, and then heated to 90°C under a nitrogen atmosphere and stirred overnight. Upon completion, the reaction mixture was quenched with water and extracted with EA. The EA layer was collected, washed with a NaCl solution, dried over anhydrous sodium sulfate, filtered, and concentrated to dryness under reduced pressure. The residue was purified by silica gel column chromatography to obtain intermediate 540.6 g (1.32 mol) of A-3 (79.77% yield). MS (ESI) m/z = 411 $[M+1]^+$.

__Preparation of intermediate B-1__

A preparation of intermediate B-1 is illustrated as follows:

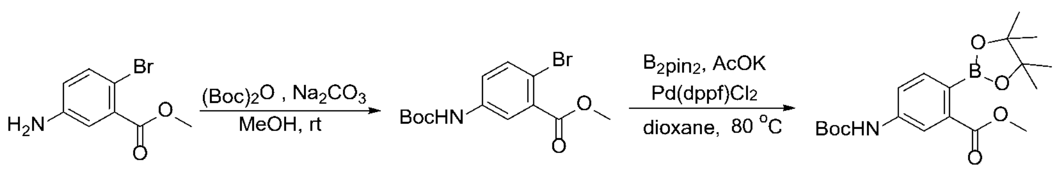


B-1-1 B-1

__(S1) Preparation of intermediate B-1-1__

To a solution of methyl 2-bromo-5-aminobenzoate (11.5 g, 49.99 mmol) in MeOH (150 mL) were added $(Boc)_2O$ (24.00 g, 109.97 mmol) and $Na_2CO_3$ (15.89 g, 149.96 mmol). The reaction mixture was stirred at room temperature and reacted overnight. Upon completion, the reaction mixture was subjected to extraction by water and EA. The EA layer was collected, washed with a NaCl solution, concentrated to dryness, and purified by silica gel column chromatography to obtain 13.9 g of intermediate B-1-1 (84% yield). MS (ESI) m/z = 330 $[M+1]^+$.

(S2) Preparation of intermediate B-1

To a solution of intermediate B-1-1 (6.6 g, 19.99 mmol) in 1,4-dioxane (70 mL) were sequentially added $B_2pin_2$ (5.58 g, 21.99 mmol), AcOK (3.92 g, 39.98 mmol), and $Pd(dppf)Cl_2$ (731.31 mg, 999.47 μmol). The reaction mixture was evacuated and purged with nitrogen three times, and then heated to 80°C under a nitrogen atmosphere and stirred overnight. Upon completion, the reaction mixture was subjected to extraction by water and EA. The EA layer was collected, washed with a NaCl solution, dried over anhydrous sodium sulfate, filtered, and concentrated to dryness under reduced pressure. The residue was purified by silica gel column chromatography to obtain 6.7 g of intermediate B-1 (89% yield). MS (ESI) m/z = 378 $[M+1]^+$.

**Preparation of intermediate B-2**

A preparation of intermediate B-2 is illustrated as follows:

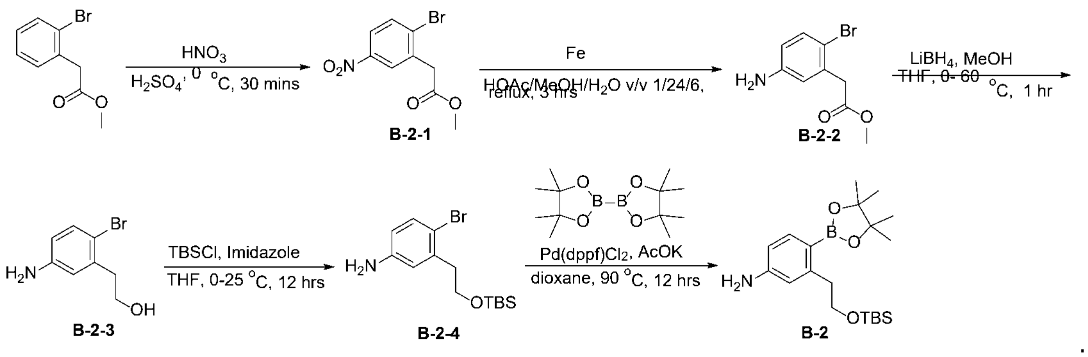


(S1) Preparation of intermediate B-2-1

To a solution of methyl 2-bromophenylacetate (10 g, 43.65 mmol) in concentrated sulfuric acid (42 mL) was dropwise added concentrated nitric acid (5.50 g, 87.31 mmol) under an ice-water bath. After addition, the reaction mixture was stirred at 0°C for 30 min. Upon completion, the reaction mixture was slowly decanted into ice-water and extracted with EA to obtain EA layers. The EA layers were combined, washed sequentially with water and a NaCl solution, dried over anhydrous sodium sulfate, filtered, and concentrated to dryness under reduced pressure. The residue was purified by silica gel column chromatography to obtain 9.8 g (35.76 mmol) of intermediate B-2-1 (81.91% yield). MS (ESI) m/z = 272 $[M-1]^-$.

(S2) Preparation of intermediate B-22

To a solution of intermediate B-2-1 (5 g, 18.24 mmol) in methanol/glacial acetic acid/water (72 mL/3 mL/18 mL) was batchwise added reduced iron powder (5.09 g, 91.22 mmol) under stirring. The reaction mixture was heated to reflux and stirred for 3 h. After the reaction was confirmed to be complete, the reaction mixture was filtered hot through Celite. The filter cake was collected, washed with EA. The filtrate was collected and concentrated to dryness under reduced pressure. The residue was dissolved in EA, washed sequentially with water and a saturated NaCl solution, dried over anhydrous sodium sulfate, filtered, and concentrated to dryness under reduced pressure. The residue was purified by silica gel column chromatography to obtain 3.95 g (16.19 mmol) of intermediate B-2-2 (88.73% yield). MS (ESI) m/z = 244 $[M+1]^+$.

(S3) Preparation of intermediate B-2-3

To a solution of intermediate B-2-2 (3.95 g, 16.19 mmol) in tetrahydrofuran (80 mL) were added methanol (726.08 mg, 22.66 mmol) and lithium borohydride (916.89 mg, 42.09 mmol) at room temperature. The reaction mixture was heated to 60°C and stirred for 2 h. Upon completion, the reaction mixture was decanted into ice-water for quenching and extracted with EA. The EA layers were collected, combined, washed sequentially with water and a NaCl solution, dried over anhydrous sodium sulfate, filtered, and concentrated to dryness under reduced pressure to obtain 3.58 g (14.89 mmol) of intermediate B-2-3 (92.02% yield). MS (ESI) m/z = 216 $[M+1]^+$.

(S4) Preparation of intermediate B-2-4

To a solution of intermediate B-2-3 (3.58 g, 16.55 mmol) in tetrahydrofuran (80 mL) were added imidazole (2.25 g, 33.10 mmol) and TBSCl (4.99 g, 33.10 mmol) under an ice-water bath. The reaction mixture was heated to room temperature and stirred for 2 h. Upon completion, the reaction mixture was decanted into saturated ammonium chloride solution for quenching and extracted with EA. The EA layers were collected, combined, washed sequentially with water and a NaCl solution, dried over anhydrous sodium sulfate, filtered, and concentrated to dryness under reduced pressure. The residue was purified by silica gel column chromatography to obtain 4.58 g (13.87 mmol) of intermediate B-2-4 (83.81% yield). MS (ESI) m/z = 330 $[M+1]^+$.

(S5) Preparation of intermediate B-2

To a 250 mL single-necked flask were added intermediate B-2-4 (3 g, 9.08 mmol), bis(pinacolato)diboron (2.54 g, 9.99 mmol), potassium acetate (1.71 g, 18.16 mmol) and 1,4-dioxane (50 mL). The reaction mixture was evacuated and purged with nitrogen three times under stirring, then heated to 90°C and stirred overnight. Upon completion, the reaction mixture was cooled and purified by silica gel column chromatography to obtain 2.12 g (5.60 mmol) of intermediate B-2 (61.71% yield). MS (ESI) m/z = 378 $[M+1]^+$.

**Preparation of intermediate B-3**

A preparation of intermediate B-3 is illustrated as follows:

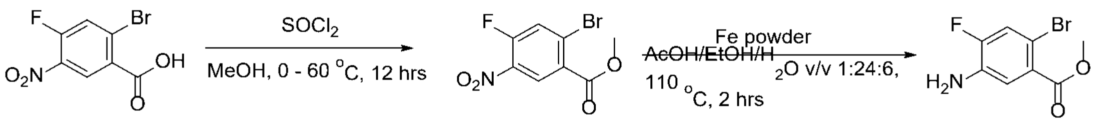


B-3-1 B-3-2

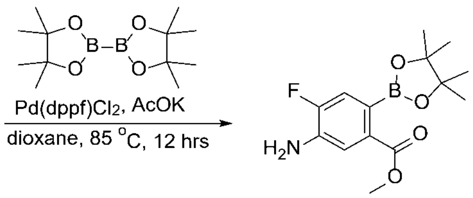


**B-3**

.

(S1) Preparation of intermediate B-3-1

To a 250 mL single-necked flask were added 2-bromo-4-fluoro-5-nitrobenzoic acid (5 g, 18.94 mmol) and methanol (100 mL), followed by dropwise addition of thionyl chloride (6.76 g, 56.82 mmol, 4.13 mL) under an ice-water bath. The reaction mixture was heated to 60°C and stirred overnight. Upon completion, the reaction mixture was cooled to room temperature and concentrated to dryness under reduced pressure. The residue was dissolved in EA and washed sequentially with an aqueous sodium bicarbonate solution and a saturated NaCl solution. The EA layer was collected, dried over anhydrous sodium sulfate, filtered, and concentrated to dryness under reduced pressure to obtain 5.03 g (18.10 mmol) of intermediate B-3-1 (95.58% yield). MS (ESI) m/z = 276 $[M-1]^-$.

(S2) Preparation of intermediate B-3-2

To a 250 mL single-necked flask were added intermediate B-3-1 (5 g, 17.98 mmol) and ethanol/glacial acetic acid/water (120 mL/5 mL/30 mL), and batchwise added reduced iron powder (5.02 g, 89.92 mmol) under stirring at room temperature. The reaction mixture was heated to reflux and stirred for 2 h. After slight cooling, the reaction mixture was filtered hot through Celite and washed with EA. The filtrate was collected, concentrated to dryness under reduced pressure. The residue was dissolved in EA, and washed sequentially with an aqueous sodium bicarbonate solution and a saturated NaCl solution. The EA layer was collected, dried over anhydrous sodium sulfate, filtered, and concentrated to dryness under reduced pressure. The residue was purified by silica gel column chromatography to obtain 4.3 g (17.34 mmol) of intermediate B-3-2 (96.40% yield). MS (ESI) m/z = 248 $[M+1]^+$.

(S3) Preparation of intermediate B-3

To a 250 mL single-necked flask were added intermediate B-3-2 (3.97 g, 16.00 mmol), bis(pinacolato)diboron (4.47 g, 17.61 mmol), potassium acetate (3.01 g, 32.01 mmol), 1,4-dioxane (100 mL) and Pd(dppf)$Cl_2$ (585.54 mg, 0.800 mmol). The reaction mixture was evacuated and purged with nitrogen three times under stirring, then heated to 85°C and stirred overnight. Upon completion, the reaction mixture was cooled and concentrated to dryness under reduced pressure. The residue was purified by silica gel column chromatography to obtain 4.83 g (13.59 mmol) of intermediate B-3 (84.89% yield). MS (ESI) m/z = 296 $[M+1]^+$.

**Preparation of intermediate B-4**

A preparation of intermediate B-4 is illustrated as follows:

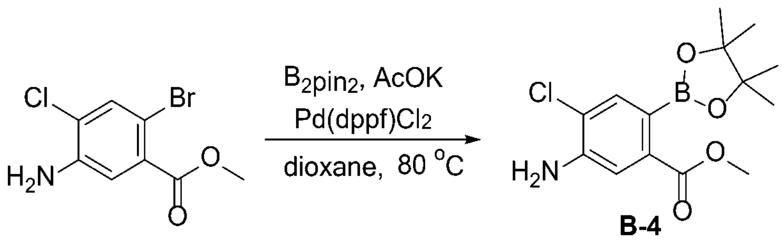


B-4

The preparation of intermediate B-4 was performed according to the synthesis method of intermediate B-1, where intermediate B-1-1 was replaced with methyl 5-amino-2-bromo-4-chlorobenzoate, with all other reagents and procedures remaining identical. MS (ESI) m/z = 312 $[M+1]^+$.

**Preparation of intermediate C-1**

A preparation of intermediate C-1 is illustrated as follows:

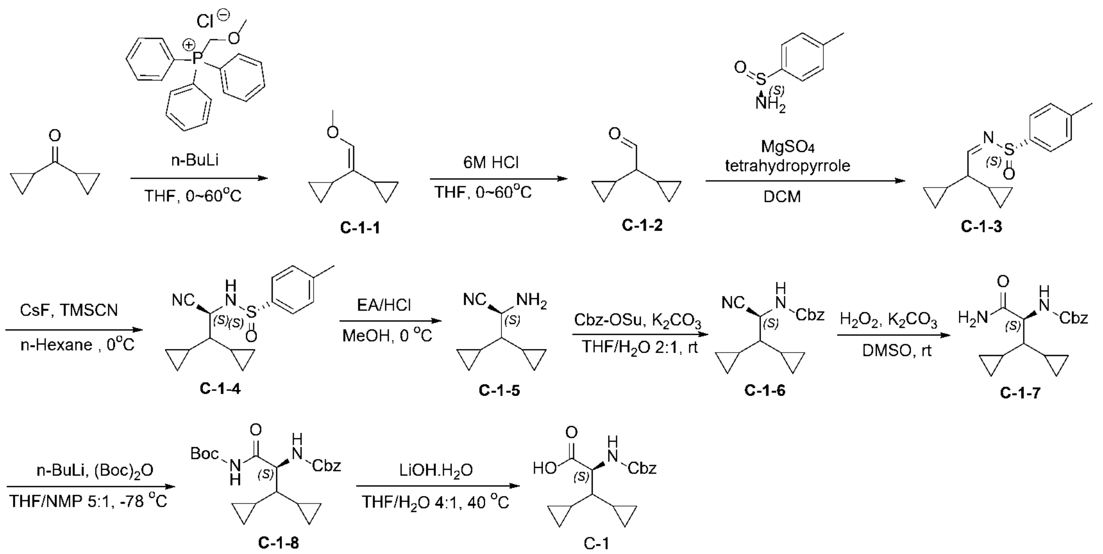


(S1) Preparation of intermediate C-1-1

Under an ice-water bath and a nitrogen atmosphere, n-BuLi (2.5 M in hexanes, 4.09 mol, 1.63 L) was dropwise added to a solution of (methoxymethyl)triphenylphosphonium chloride (300 g, 4.09 mol) in dry THF (2.4 L). The mixture was stirred at 0°C for 1 h, during which the reaction solution gradually turned dark brown. Subsequently, a solution of dicyclopropyl ketone (300 g, 2.72 mol) in THF (0.6 L) was dropwise added thereto. The reaction mixture was heated to 60°C and stirred for 4 h. Upon completion, the reaction mixture was cooled to room temperature, quenched with 30% aqueous $NH_4Cl$, and extracted with EA. The EA layers were collected, combined, washed with a saturated NaCl solution,

dried over anhydrous $Na_2SO_4$, filtered, and concentrated under reduced pressure. The residue was purified by vacuum distillation to obtain 279 g of a clear oil as intermediate C-1-1 (yield: 74.2%).

(S2) Preparation of intermediate C-1-2

To a solution of intermediate C-1-1 (279 g, 2.02 mol) in dry THF (1.7 L) was added 6 M HCl (1.7 L) under an ice-water bath. The reaction mixture was then heated to 60°C and stirred for 5 h. Upon completion, the reaction mixture was extracted with EA. The EA layers were collected, combined, dried over anhydrous $Na_2SO_4$, filtered, and concentrated under reduced pressure to obtain 239.6 g of an oily crude product as intermediate C-1-2, requiring no purification for the next step.

(S3) Preparation of intermediate C-1-3

To intermediate C-1-2 (239 g, 1.92 mol, calculated based on 100% purity) in DCM (2.5 L) in a 5 L three-necked flask were sequentially added (S)-(+)-p-toluenesulfinamide (299 g, 1.92 mol), anhydrous $MgSO_4$ (697 g, 5.79 mol), and pyrrolidine (13.73 g, 0.193 mol). The reaction mixture was stirred at room temperature overnight and filtered. The filtrate was concentrated under reduced pressure at 40°C to dryness. The residue was slurried with petroleum ether and filtered. The filtrate was concentrated and purified to 217 g of intermediate C-1-3 (yield: 43%). MS (ESI) m/z = 262 $[M+1]^+$.

(S4) Preparation of intermediate C-1-4

To a solution of intermediate C-1-3 (217 g, 0.83 mol) in n-hexane (2 L) was added CsF (252 g, 1.66 mol) under stirring. Under a nitrogen atmosphere, the reaction mixture was cooled to 0°C, and dropwise added with TMSCN (165 g, 1.66 mol). The reaction mixture was heated to room temperature and stirred overnight. Upon completion, the reaction mixture was concentrated to dryness, then slurried with n-hexane and filtered twice. The resulting solid was dried under reduced pressure to obtain 168 g of a solid as intermediate C-1-4 (yield: 70%). MS (ESI) m/z = 289 $[M+1]^+$.

(S5) Preparation of intermediate C-1-5

To a solution of intermediate C-1-4 (84 g, 2.92 mol) in MeOH (840 mL) was added HCl in EA (4 M, 146 mL, 0.583 mol) under an ice-water bath and stirring for 2 h. Upon completion, the reaction mixture was concentrated under reduced pressure to dryness. The residue was slurried and washed several times with PE, then filtered. The filter cake was dried to obtain 54.4 g of a solid as intermediate C-1-5, requiring no purification for the next step. MS (ESI) m/z = 151 $[M+1]^+$.

(S6) Preparation of intermediate C-1-6

To a solution of intermediate C-1-5 (54.4 g, 0.363 mol, calculated based on 100% purity) in THF (300 mL)/$H_2O$ (300 mL) were added $K_2CO_3$ (100.74 g, 0.73 mol) and CbzOSu (90.39 g, 0.363 mol) under an ice-water bath. The reaction mixture was stirred at 25°C for 2 h. Upon completion, the reaction mixture was extracted with EA. The EA layers were collected, combined, dried over anhydrous $Na_2SO_4$, filtered, and concentrated under reduced pressure to dryness. The residue was purified by normal-phase column chromatography to to obtain 93.6 g (0.330 mol) of a white solid as intermediate C-1-6 (yield: 90.8%). MS (ESI) m/z = 285 $[M+1]^+$.

(S7) Preparation of intermediate C-1-7

To a solution of intermediate C-1-6 (75.4 g, 0.264 mol) in DMSO (750 mL) was added $K_2CO_3$ (54.9 g, 0.398 mol), cooled to 10 °C, followed by dropwise addition of $H_2O_2$ (90 g, 0.79 mol, 30% aqueous solution). The reaction mixture was stirred at 25°C overnight. Upon completion, the reaction mixture was diluted with water and extracted with EA. The EA layers were collected, combined, dried over anhydrous $Na_2SO_4$, filtered, and concentrated under reduced pressure to dryness to obtain 73.5 g (0.242 mol) of intermediate C-1-7 (yield: 91.6%), requiring no purification for the next step. MS (ESI) m/z = 303 $[M+1]^+$.

(S8) Preparation of intermediate C-1-8

To a solution of intermediate C-1-7 (73.5 g, 0.242 mol) in dry THF (500 mL)/NMP (150 mL) at -78°C was dropwise added n-BuLi (204 mL, 0.51 mol, 2.5 M in hexanes) under a nitrogen atmosphere. The reaction mixture was stirred at -78°C for 1 h followed by addition of a solution of $(Boc)_2O$ (57.5 g, 0.26 mol) in THF (250 mL), and continuously stirred for 1 h. Upon completion, the reaction mixture was quenched with cold 30% aqueous $NH_4Cl$ and extracted with EA. The EA layers were

collected, combined, washed successively with water and a saturated NaCl solution, dried over anhydrous $Na_2SO_4$, filtered, and concentrated under reduced pressure to dryness to obtain 86 g (0.214 mol) of a crude substance as intermediate C-1-8, requiring no purification for the next step. MS (ESI) m/z = 403 $[M+1]^+$.

(S9) Preparation of intermediate C-1

To a mixture of intermediate C-1-8 (86 g, 0.214 mol) in THF (800 mL) and $H_2O$ (200 mL) was added $LiOH{\cdot}H_2O$ (17.89 g, 0.426 mol). The reaction mixture was heated to 40°C and stirred overnight. Upon completion, the solvent was removed under reduced pressure. The residue was dissolved in water, and subjected to extraction by EA to remove impurities. The aqueous layer was collected, cooled under an ice-water bath, adjusted to pH 2-3 with 6 M HCl, and extracted with EA. The organic extracts were concentrated, and purified by column chromatography to obtain 48 g (0.158 mol) of intermediate C-1 (yield: 74.07%). MS (ESI) m/z = 304 $[M+1]^+$.

**Preparation of intermediate C-2**

A preparation of intermediate C-2 is illustrated as follows:

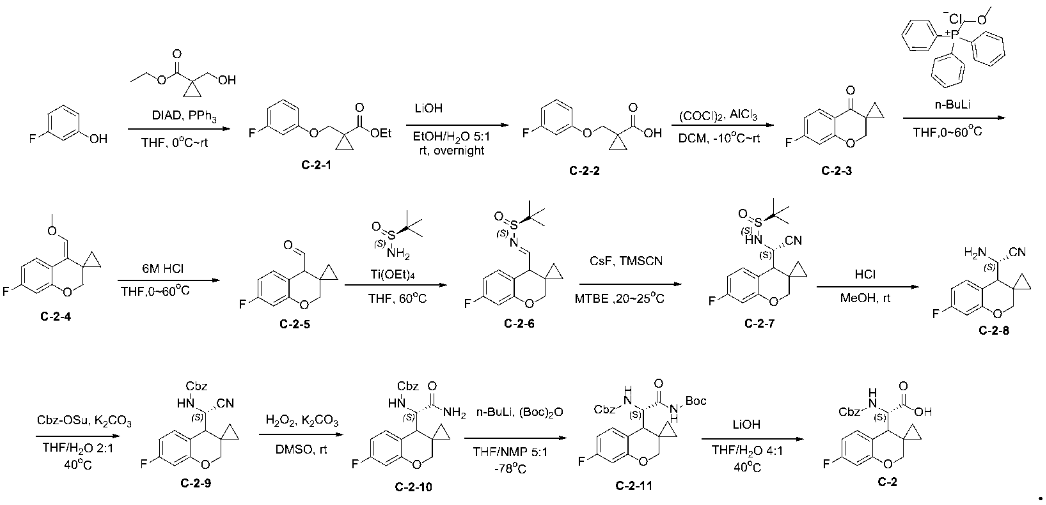


(S1) Preparation of intermediate C-2-1

To a solution of 3-fluorophenol (1063 g, 9.49 mol), ethyl 1-(hydroxymethyl)cyclopropanecarboxylate (1435 g, 9.96 mol), and $PPh_3$ (2741 g, 10.46 mol) in THF (15 L) at below 0°C was dropwise added DIAD (2099 g, 10.39 mol) under an ice-water bath. The reaction mixture was warmed naturally to room temperature and stirred overnight. After the reaction was confirmed by LCMS to be complete, the reaction mixture was concentrated under reduced pressure to dryness, added with 12 L of a solution with PE:EA = 15:1, stirred for 30 min to precipitate a large amount of solid, and passed through a short silica gel column. A silica layer was subjected to drip washing with 15 L of a solution with PE:EA = 15:1. The filtrate was concentrated under reduced pressure to dryness to obtain intermediate C-2-1, which was used directly in the next step assuming a quantitative yield. MS (ESI) m/z = 239 $[M+1]^+$.

(S2) Preparation of intermediate C-2-2

To a mixture of intermediate C-2-1 (9.49 mol, carried forward from the previous step assuming a quantitative yield) in 95% EtOH (8 L)/$H_2O$ (1.6 L) was added LiOH (988.4 g, 23.72 mol). The reaction mixture was stirred at room temperature for 12 h. Upon completion, the reaction mixture was concentrated, diluted with water, adjust to pH 3-4 with concentrated HCl, and subjected to extraction by DCM. The DCM layers were collected, combined, washed with water and a saturated NaCl solution three times, dried over sodium sulfate, filtered, and concentrated under reduced pressure to dryness. The residue was added with PE (3 L), and stirred for 30 min. The resulting solid was collected by filtration, washed with petroleum ether, and dried to obtain 1495 g (7.12 mol) of intermediate C-2-2 (two-step yield: 75%), requiring no purification for the next step. MS (ESI) m/z = 211 $[M+1]^+$.

(S3) Preparation of intermediate C-2-3

To a solution of intermediate C-2-2 (1505 g, 7.16 mol) in DCM (10 L)/DMF (50 mL) was dropwise added oxalyl chloride ($(COCl)_2$, 1365.2 g, 10.75 mol) under a nitrogen atmosphere at 0°C. The reaction mixture was stirred at 0°C for 3 h, then cooled to - 10°C, and batchwise added with $AlCl_3$ (1904.56 g, 14.32 mol). The reaction mixture was warmed slowly to room temperature and stirred for an additional 1 h. The reaction mixture was slowly decanted into ice-water. The aqueous phase was collected, extracted with DCM three times. The DCM layers were collected, combined, washed with water three times, washed with a saturated aqueous sodium bicarbonate until weakly basic, washed with a saturated NaCl solution twice, dried over anhydrous sodium sulfate, filtered, and concentrated to obtain 1200 g (6.25 mol) of crude intermediate C-2-3 (yield: 85.3%), requiring no purification for the next step.

(S4) Preparation of intermediate C-2-4

To a solution of (methoxymethyl)triphenylphosphonium chloride (232.1 g, 0.677 mol) in anhydrous THF (1,000 mL) was dropwise added n-BuLi (2.5 M in hexane, 0.677 mol, 271 mL) under an ice bath and a nitrogen atmosphere. The reaction mixture was stirred at 0°C for 1 h, during which the mixture turned dark brown. The reaction mixture was dropwise added with a THF solution of intermediate C-2-3 (100 g, 0.521 mol), heated to 60°C and stirred for 4 h. Upon completion, the reaction mixture was cooled to room temperature, quenched with 30% aqueous $NH_4Cl$, and extracted with EA. The EA layers were collected, combined, dried over anhydrous sodium sulfate, filtered, and concentrated. The residue was purified by silica gel column chromatography (0-10%, PE/EA) to obtain 114.74 g (0.521 mol) of a clear oil as intermediate C-2-4 (yield: 100%).

(S5) Preparation of intermediate C-2-5

A solution of intermediate C-2-4 (114 g, 0.518 mol) in THF (570 mL) was cooled to 0°C, and 6 M HCl (570 mL) was added dropwise to the solution while maintaining the temperature below 10°C. The reaction mixture was then heated to 60°C and stirred for 5 h. Upon completion, the reaction mixture was extracted with EA. The EA layers were collected, combined, washed once with 10% aqueous sodium bicarbonate and once with saturated aqueous sodium chloride, dried over anhydrous sodium sulfate, and concentrated to obtain 106.8 g (0.518 mol) of an oil-like substance as intermediate C-2-5 (yield: 100%), requiring no purification for the next step.

(S6) Preparation of intermediate C-2-6

To a 2,000 mL single-necked flask were added intermediate C-2-5 (106.8 g, 0.518 mol), (S)-(+)-tert-butanesulfinamide (62.78 g, 0.518 mol), anhydrous THF (1,000 mL), and $(EtO)_4Ti$ (236.3 g, 1.036 mol) under a nitrogen atmosphere. The reaction mixture was heated to 60°C and stirred for 5 h, then cooled to room temperature. The reaction mixture was added with water and EA, and filtered to remove insoluble material. The filtrate was subjected to separation. The aqueous phase was collected and extracted with EA twice. The EA layers were collected, combined and concentrated to obtain a brown oil-like substance. The brown oil-like substance was purified by silica gel column chromatography (eluent: PE/EA 0-50%) to obtain 128 g (0.414 mol) of a light yellow solid as intermediate C-2-6 (yield: 80%). MS (ESI) m/z = 310 $[M+1]^+$.

(S7) Preparation of intermediate C-2-7

To a solution of intermediate C-2-6 (128 g, 0.414 mol) in MTBE (2,560 mL) were added CsF (125.78 g, 0.828 mol) and TMSCN (82.1 g, 0.828 mol) at room temperature. The reaction mixture was stirred at 20-25°C overnight. Upon completion, a large amount of solid precipitated. The solid was collected by filtration, and dissolved in water and EA. The aqueous phase was collected, and extracted with EA twice. The EA layers were collected, dried over anhydrous sodium sulfate, filtered, and concentrated. The residue was added with MTBE (5 v/m), heated to reflux and slurried for 1 h, cooled to room temperature, stirred for 2 h, and filtered to obtain 37.6 g (0.112 mol) of an off-white solid as intermediate C-2-7 (yield: 27%). MS (ESI) m/z = 337 $[M+1]^+$.

(S8) Preparation of intermediate C-2-8

56 mL of 4 M HCl in EA (0.224 mol) was dropwise added to a solution of intermediate C-2-7 (37.6 g, 0.112 mol) in MeOH (376 mL). The reaction mixture was stirred at room temperature for 2 h. Upon completion, the reaction mixture was concentrated. The residue was slurried and washed once with MTBE, then filtered and dried to obtain 25.52 g (0.110 mol) of intermediate C-2-8 (yield: 98%), requiring no purification for the next step. MS (ESI) m/z = 233 $[M+1]^+$.

(S9) Preparation of intermediate C-2-9

To a solution of intermediate C-2-8 (25.52 g, 0.110 mol) in THF (153 mL)/$H_2O$ (77 mL) were added $K_2CO_3$ (45.6 g, 0.330 mol) and Cbz-Osu (54.82 g, 0.220 mol) at room temperature. The reaction mixture was stirred at 40°C overnight. Upon completion, the reaction mixture was extracted with EA. The EA layers were collected, combined and concentrated. The residue was purified by MPLC to obtain 38.66 g (0.106 mol) of an oil-like substance as intermediate C-2-9 (yield: 96.02%). MS (ESI) m/z = 367 $[M+1]^+$.

(S10) Preparation of intermediate C-2-10

$K_2CO_3$ (14.65 g, 0.106 mol) was added To a solution of intermediate C-2-9 (38.66 g, 0.106 mol) in DMSO (387 mL) were added $K_2CO_3$ (14.65 g, 0.106 mol) and dropwise added $H_2O_2$ (24 g, 30%, 0.212 mol). The reaction mixture was stirred at 25°C for 2 h. Upon completion, the reaction mixture was added with a large amount of water to dilute, so as to precipitate a large amount of white solid. The solid was collected by filtration and the filter cake was thoroughly washed with water. The filter cake was then dissolved in an appropriate amount of EA, washed once with water and once with a saturated NaCl solution, dried over anhydrous sodium sulfate, filtered, and concentrated to obtain 39.93 g (0.104 mol) of a solid as intermediate C-2-10 (yield: 98%). MS (ESI) m/z = 385 $[M+1]^+$.

(S11) Preparation of intermediate C-2-11

To a solution of intermediate C-2-10 (39.93 g, 0.104 mol) in anhydrous THF (400 mL)/NMP (80 mL) at -78°C was dropwise added n-BuLi (2.5 M in hexane, 96 mL, 0.239 mol) under a nitrogen atmosphere. The reaction mixture was maintained at -78°C and stirred for 1 h. The reaction mixture was added with a THF solution of $(Boc)_2O$ (29.06 g, 0.135 mol), and further stirred for 1 h. Upon completion, the reaction mixture was carefully quenched by slow addition of cold 30% aqueous $NH_4Cl$, extracted with EA, and concentrated to obtain 50.4 g (0.104 mol) of intermediate C-2-11 (yield: 100.00%), requiring no purification for the next step. MS (ESI) m/z = 485 $[M+1]^+$.

(S12) Preparation of intermediate C-2

To a solution of intermediate C-2-11 (50.4 g, 0.104 mol) in THF (806 mL)/$H_2O$ (201 mL) was added $LiOH\cdot H_2O$ (8.67 g, 0.208 mol). The reaction mixture was heated to 40°C and stirred overnight. The aqueous layer was collected, adjusted to pH 3-4 with 2 M HCl, and extracted with EA twice. The EA layers were collected, combined, washed once with a saturated NaCl solution, dried over anhydrous sodium sulfate, filtered, and concentrated under reduced pressure. The residue was dissolved in EA followed by dropwise addition of diisopropylamine (12.6 g, 0.125 mol) at room temperature under stirring, further stirred for 2 h and filtered to obtain a white solid. The white solid was dissolved in an appropriate amount of water and EA. The aqueous layer was collected, and adjusted to pH 3-4 with 2 M HCl. The EA layer was collected, washed with a saturated NaCl solution, dried over anhydrous sodium sulfate, filtered, and concentrated to dryness to obtain 27 g (70 mmol) of purified intermediate C-2 (two-step yield: 67.3%). MS (ESI) m/z = 386 $[M+1]^+$. $^1H$ NMR (400 MHz, Methanol-$d_4$) δ 7.30 (q, *J* = 7.7, 6.6 Hz, 5H), 7.02 (dd, *J* = 8.5, 6.6 Hz, 1H), 6.52 (dd, *J* = 10.6, 2.6 Hz, 1H), 6.44 (td, *J* = 8.5, 2.7 Hz, 1H), 5.06 (d, *J* = 12.5 Hz, 1H), 4.96 (d, *J* = 12.6 Hz, 1H), 4.68 (dd, *J* = 11.3, 2.0 Hz, 1H), 4.54 (d, *J* = 7.6 Hz, 1H), 3.27 (d, *J* = 1.8 Hz, 1H), 2.38 (d, *J* = 7.5 Hz, 1H), 0.94-0.79 (m, 2H), 0.75-0.64 (m, 1H), 0.63-0.53 (m, 2H), 0.46-0.35 (m, 1H).

Data of the optical rotation is as follows. A temperature was 25°C; a concentration was 0.002 g/100 mL; a solvent was methanol; a specific rotation was -132.8°; and a chiral purity was 98%.

**Preparation of intermediate C-3**

A structure of intermediate C-3 is as follows:

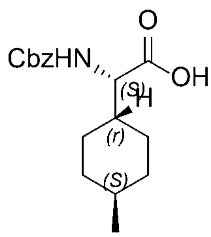

**C-3**

.

The preparation of intermediate C-3 was performed with reference to International patent application No. UCB-WO2020/11731 A1 to obtain intermediate C-3. MS (ESI) m/z = 306 $[M+1]^+$.

**Preparation of intermediate C-4**

A structure of intermediate C-4 is as follows:

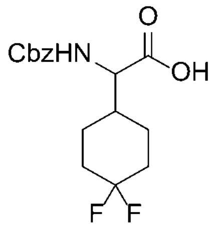

**C-4**

.

The preparation of intermediate C-4 was performed with reference to International patent application No. WO2020/146194 A1, where a double bond reduction was carried out employing Pd/C hydrogenation, with all other reagents and procedures remaining identical. MS (ESI) m/z = 328 $[M+1]^+$.

**Preparation of intermediate C-5**

A preparation of intermediate C-5 is illustrated as follows:

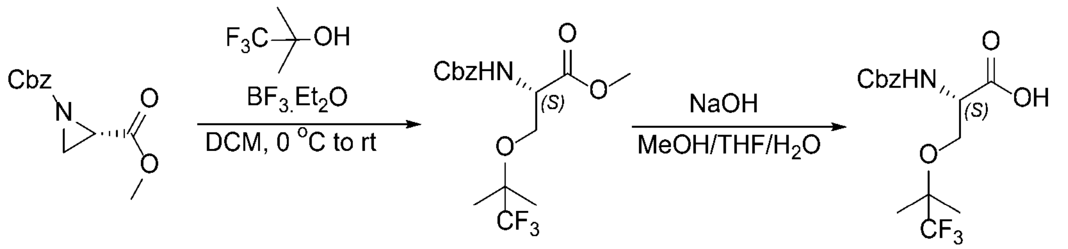


.

(S1) Preparation of intermediate C-5-1

To a three-necked flask were added (S)-(-)-methyl N-Z-aziridine-2-carboxylate (10 g, 42.55 mmol), 2-trifluoromethyl-2-propanol (27.23 g, 212.77 mmol), and DCM (90 mL). The flask was evacuated and purged with nitrogen three times. The reaction mixture was cooled to 0°C, and dropwise added with boron trifluoride etherate (4 mL). The reaction mixture was heated to room temperature and stirred for 6 h. Upon completion, the reaction mixture was cooled under an ice-water bath, quenched with methanol, and then concentrated to dry under reduced pressure. The residue was purified by silica gel column chromatography to obtain 400 mg (1.10 mmol) of intermediate C-5-1 (yield: 2.59%). MS (ESI) m/z = 364 $[M+1]^+$.

(S2) Preparation of intermediate C-5

To a single-necked flask were added intermediate C-5-1 (400 mg, 1.10 mmol) and THF/MeOH (10 mL/10 mL). The reaction mixture was cooled under an ice-water bath, and added with an aqueous solution of sodium hydroxide (79.27 mg, 1.98 mmol, 10 mL). The reaction mixture was heated to room temperature and stirred for 1 h. Upon completion, a pH of the rection mixture was adjusted to weakly basic with 6 M HCl. The organic solvent in the rection mixture was removed under reduced pressure at 0°C. The residue was diluted with water and extracted with EA twice to obtain EA layers, and the EA layers were discarded. The aqueous phase was then adjusted to pH 4 with 6 M HCl and extracted with EA. The EA layer was collected, dried over anhydrous sodium sulfate, filtered, and concentrated to dry under reduced pressure to obtain 298 mg (853.13 μmol) of intermediate C-5 (yield: 86.10%). MS (ESI) m/z = 350 $[M+1]^+$.

**Preparation of intermediate D-1**

A preparation of intermediate D-1 is illustrated as follows:

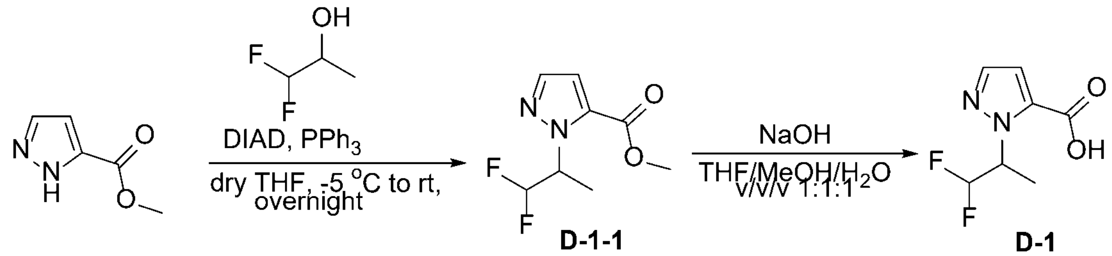


D-1-1 D-1

(S1) Preparation of intermediate D-1-1

To a three-necked flask were added methyl 1H-pyrazole-3-carboxylate (1.31 g, 10.41 mmol), triphenylphosphine (3.28 g, 12.49 mmol) and anhydrous THF (80 mL). The three-necked flask was evacuated and purged with nitrogen three times. 1,1-Difluoro-2-propanol (1 g, 10.41 mmol) was then added by syringe. The reaction mixture was cooled to -5°C, and dropwise added with DIAD (2.52 g, 12.49 mmol). The reaction mixture was heated to room temperature and stirred overnight. Upon completion, the reaction mixture was directly purified by silica gel column chromatography to obtain 1.5 g (3.82 mmol) of intermediate D-1-1 (yield: 36.70%, purity: 52%) as a first less polar spot. Another regioisomer exhibited higher polarity. Since the product obtained in this step was impure, the structure was confirmed by Nuclear Overhauser Effect (NOE) after hydrolysis in the next step. MS (ESI) m/z = 205 $[M+1]^+$.

(S2) Preparation of intermediate D-1

To a single-necked flask were added intermediate D-1-1 (1.5 g, 3.82 mmol, 52% purity) and THF/MeOH (12 mL/12 mL). The reaction mixture was cooled under an ice-water bath, and added with an aqueous solution of sodium hydroxide (305.62 mg, 7.64 mmol, 12 mL). The reaction mixture was heated to room temperature and stirred for 1 h. Upon completion, the organic solvent in the reaction mixture was removed under reduced pressure. The residue was diluted with water and extracted with EA twice to obtain EA layers, and the EA layers were discarded. The aqueous phase was then adjusted to pH 4 with 6 M HCl to precipitate a large amount of white solid. The solid was collected by filtration under reduced pressure, washed with water, and dried to obtain 0.56 g (2.39 mmol) of intermediate D-1 (yield: 62.44%, purity: 81%). Its structure was confirmed by NOE. MS (ESI) m/z = 191 $[M+1]^+$. $^1H$ NMR (400 MHz, DMSO-$d_6$) $\delta$ 13.58 (s, 1H), 7.66 (t, $J$ = 2.3 Hz, 1H), 6.89 (d, $J$ = 2.0 Hz, 1H), 6.27 (tdd, $J$ = 55.6, 5.0, 2.4 Hz, 1H), 5.85 - 5.67 (m, 1H), 1.54 (dd, $J$ = 7.0, 1.2 Hz, 3H).

**Preparation of intermediates D-2 to D-43**

The preparation of intermediates D-2 to D-43 was performed according to the synthesis method of intermediate D-1, in which 1,1-difluoro-2-propanol in step (S1) was correspondingly replaced with an alcohol from the following table, with all other reagents and procedures remaining identical.

| Alcohol | Structure of intermediate | $^1$HNMR and/or MS (ESI) |
|---|---|---|
| 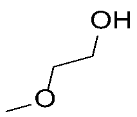 | 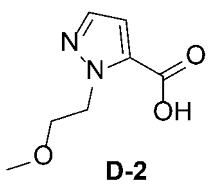 D-2 | MS (ESI) m/z =171 $[M+1]^+$ |
| 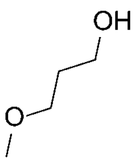 | 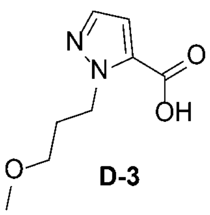 D-3 | MS (ESI) m/z = 185 $[M+1]^+$ |

(continued)

| Alcohol | Structure of intermediate | 1HNMR and/or MS (ESI) |
|---|---|---|
| 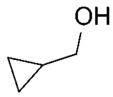 | 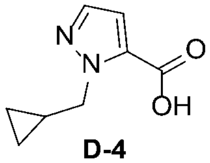 D-4 | MS (ESI) m/z =167 [M+1]+ |
| 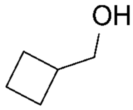 | 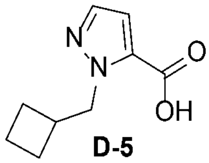 D-5 | MS (ESI) m/z =181 [M+1]+ |
| 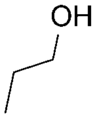 | 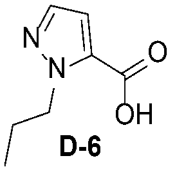 D-6 | MS (ESI) m/z =155 [M+1]+ |
| 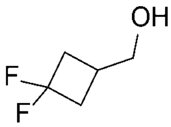 | 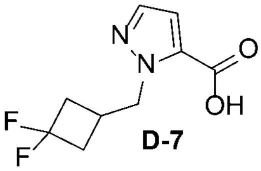 D-7 | MS (ESI) m/z =217 [M+1]+ |
| 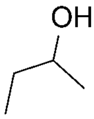 | 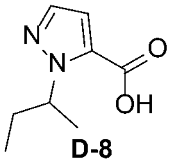 D-8 | MS (ESI) m/z =169 [M+1]+ |
| 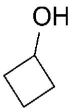 | 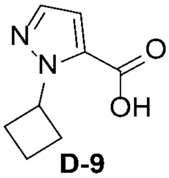 D-9 | MS (ESI) m/z =167 [M+1]+ |
| 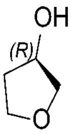 | 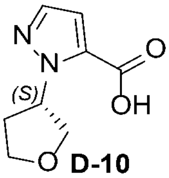 D-10 | MS (ESI) m/z =183 [M+1]+ |
| 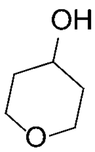 | 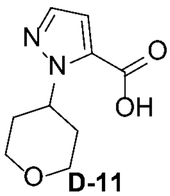 D-11 | MS (ESI) m/z =197 [M+1]+ |
| 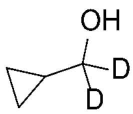 | 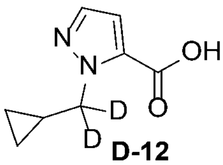 D-12 | MS (ESI) m/z =169 [M+1]+ |

(continued)

| Alcohol | Structure of intermediate | 1HNMR and/or MS (ESI) |
|---|---|---|
| 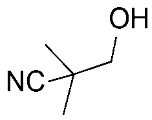 | 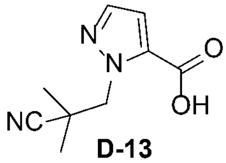 D-13 | MS (ESI) m/z =194 [M+1]$^+$ |
| 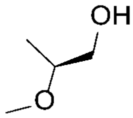 | 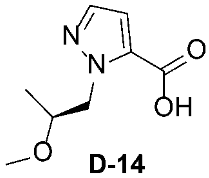 D-14 | MS (ESI) m/z =185 [M+1]$^+$ |
| 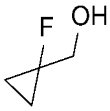 | 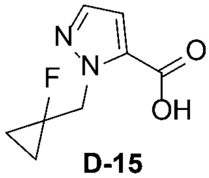 D-15 | MS (ESI) m/z =185 [M+1]$^+$ |
| 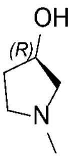 | 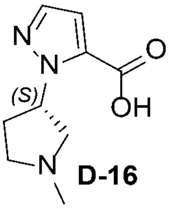 D-16 | MS (ESI) m/z =196 [M+1]$^+$ |
| 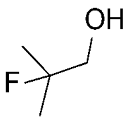 | 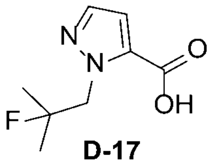 D-17 | MS (ESI) m/z =187 [M+1]$^+$ |
| 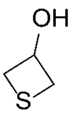 | 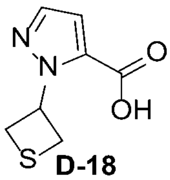 D-18 | MS (ESI) m/z =185 [M+1]$^+$ |
| 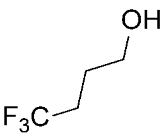 | 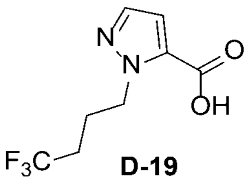 D-19 | MS (ESI) m/z =223 [M+1]$^+$ |
| 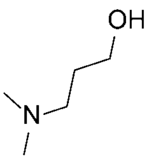 | 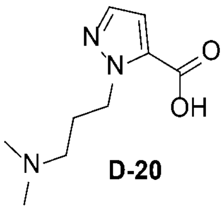 D-20 | MS (ESI) m/z =198 [M+1]$^+$ |

(continued)

| Alcohol | Structure of intermediate | 1HNMR and/or MS (ESI) |
|---|---|---|
| 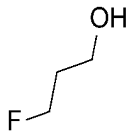 | 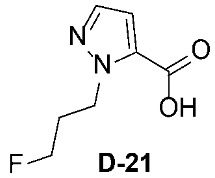 D-21 | MS (ESI) m/z =173 $[M+1]^+$ |
| 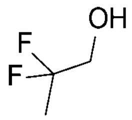 | 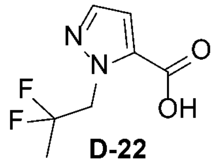 D-22 | MS (ESI) m/z =191 $[M+1]^+$ |
| 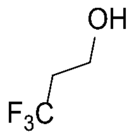 | 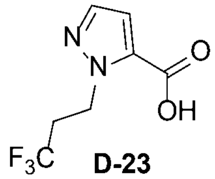 D-23 | MS (ESI) m/z =209 $[M+1]^+$ |
| 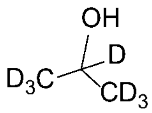 | 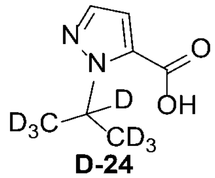 D-24 | MS (ESI) m/z =162 $[M+1]^+$ |
| 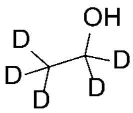 | 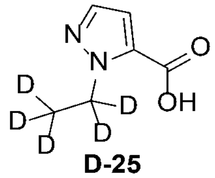 D-25 | MS (ESI) m/z =146 $[M+1]^+$ |
| 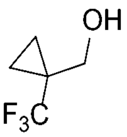 | 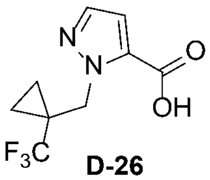 D-26 | MS (ESI) m/z =235 $[M+1]^+$ |
| 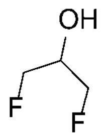 | 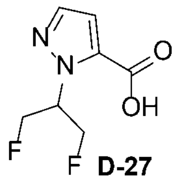 D-27 | MS (ESI) m/z =191 $[M+1]^+$ |
| 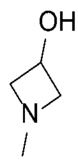 | 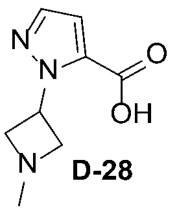 D-28 | MS (ESI) m/z =182 $[M+1]^+$ |

(continued)

| Alcohol | Structure of intermediate | $^1$HNMR and/or MS (ESI) |
|---|---|---|
| 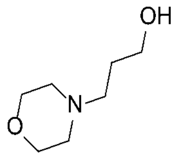 | 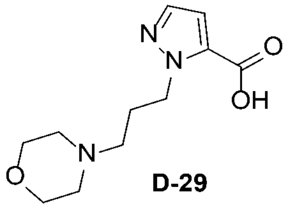 D-29 | MS (ESI) m/z =240 [M+1]$^+$ |
| 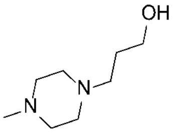 | 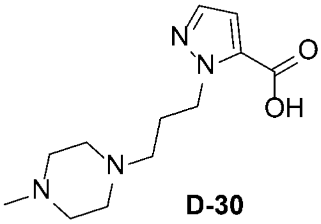 D-30 | MS (ESI) m/z =253 [M+1]$^+$ |
| 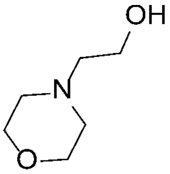 | 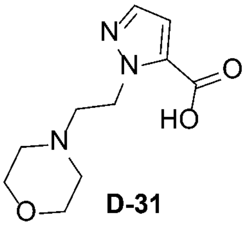 D-31 | MS (ESI) m/z =226 [M+1]$^+$ |
| 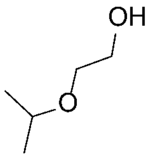 | 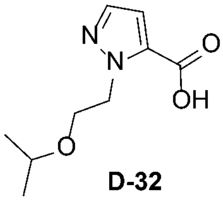 D-32 | MS (ESI) m/z =199 [M+1]$^+$ |
| 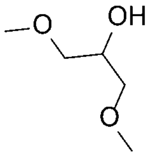 | 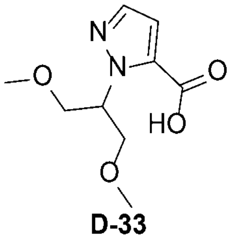 D-33 | MS (ESI) m/z =215 [M+1]$^+$ |
| 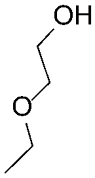 | 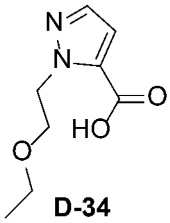 D-34 | MS (ESI) m/z =185 [M+1]$^+$ |
| 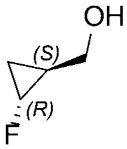 | 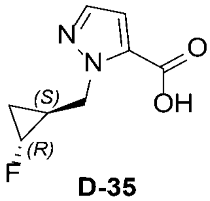 D-35 | MS (ESI) m/z =185 [M+1]$^+$ |

(continued)

| Alcohol | Structure of intermediate | $^1$HNMR and/or MS (ESI) |
|---|---|---|
| 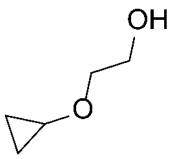 | 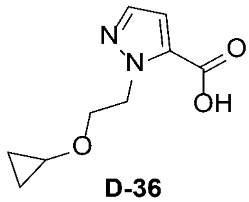 **D-36** | MS (ESI) m/z =197 $[M+1]^+$ |
| 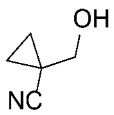 | 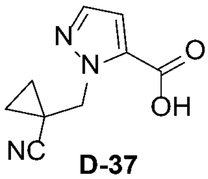 **D-37** | MS (ESI) m/z =192 $[M+1]^+$ |
| 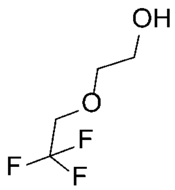 | 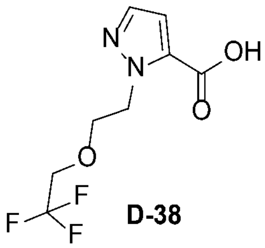 **D-38** | MS (ESI) m/z =239 $[M+1]^+$ |
| 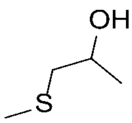 | 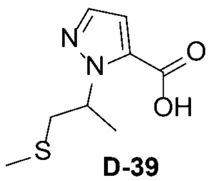 **D-39** | MS (ESI) m/z =201 $[M+1]^+$ |
| 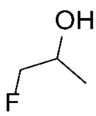 | 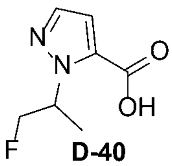 **D-40** | MS (ESI) m/z =173 $[M+1]^+$ |
| 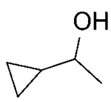 | 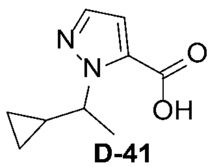 **D-41** | MS (ESI) m/z =181 $[M+1]^+$ |
| 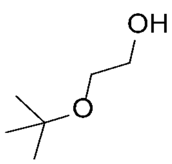 | 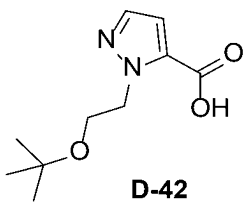 **D-42** | MS (ESI) m/z =213 $[M+1]^+$ |
| 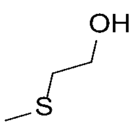 | 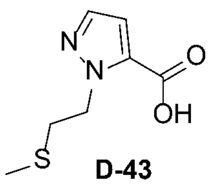 **D-43** | MS (ESI) m/z =187 $[M+1]^+$ |

**Preparation of intermediate D-44**

A preparation of intermediate D-44 is illustrated as follows:

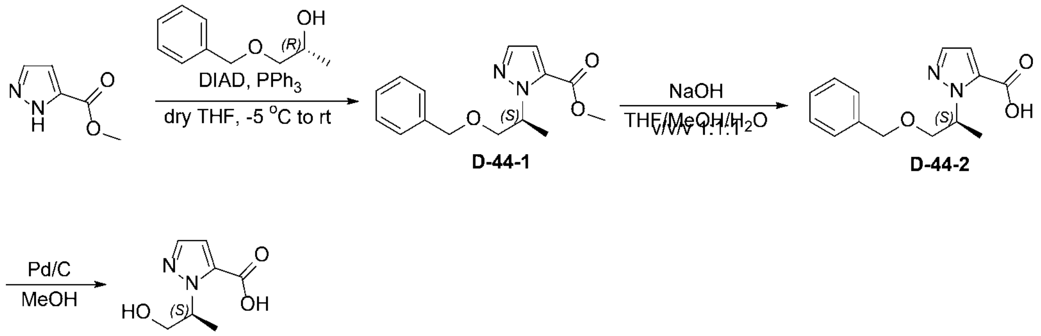


D-44

(S1-S2) Preparation of intermediate D-44-2

The preparation of intermediate D-44-2 was performed according to the synthesis method of intermediate D-1, in which 1,1-difluoro-2-propanol in step (S1) was replaced with (R)-(-)-1-benzyloxy-2-propanol, with all other reagents and procedures remaining identical. MS (ESI) m/z = 261 $[M+1]^+$.

(S3) Preparation of intermediate D-44

To a single-neck flask were added intermediate D-44-2 (2 g, 7.68 mmol), methanol (100 mL) and Pd/C (200 mg, 10% w/w). The reaction mixture was evacuated and purged with hydrogen three times, and stirred at room temperature overnight under a hydrogen balloon atmosphere. Upon completion, the reaction mixture was filtered through diatomite, and the filter cake was washed with methanol. The filtrate was concentrated under reduced pressure to dryness to obtain 1.30 g (7.64 mmol) of intermediate D-44 (yield: 99.43%). MS (ESI) m/z = 171 $[M+1]^+$.

**Preparation of intermediate D-45**

A structure of intermediate D-45 is as follows:

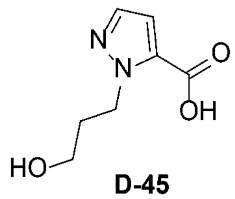

D-45

The preparation of intermediate D-45 was performed according to the synthesis method of intermediate D-44, in which (R)-(-)-1-benzyloxy-2-propanol in step (S1) was replaced with 3-benzyloxy-1-propanol, with all other reagents and procedures remaining identical. MS (ESI) m/z = 171 $[M+1]^+$.

**Preparation of intermediate D-46**

A preparation of intermediate D-46 is illustrated as follows:

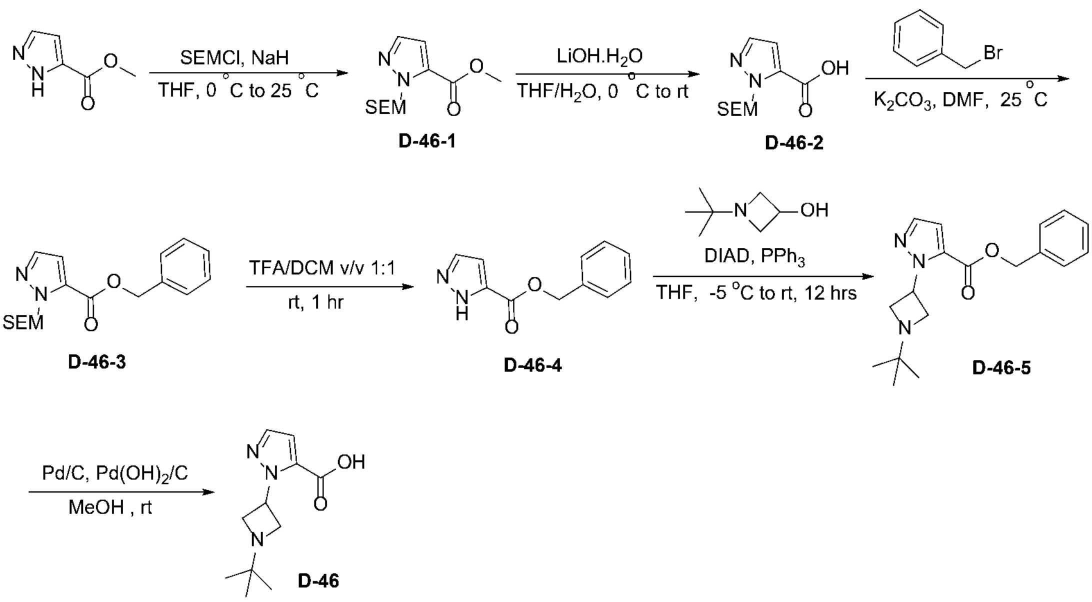


(S1) Preparation of intermediate D-46-1

To a three-neck flask were added methyl 1H-pyrazole-5-carboxylate (30 g, 237.88 mmol) and dry THF (300 mL). The reaction mixture was evacuated and purged with nitrogen three times, cooled to 0°C under an ice-water bath, and batchwise added with sodium hydride (14.27 g, 356.82 mmol). The reaction mixture was evacuated and purged with hydrogen three times, stirred at 0°C for 30 min, and dropwise added with SEM-Cl (47.59 g, 285.46 mmol) at 0°C. The reaction mixture was heated to room temperature and stirred for 3 h. Upon completion, the reaction mixture was cooled to 0°C under an ice-water bath, slowly decanted into an ice-cold ammonium chloride solution for quenching, and extracted with EA twice. The EA layers were collected, combined, washed with a saturated NaCl solution twice, dried over anhydrous sodium sulfate, and filtered. The filtrate was concentrated under reduced pressure to dryness. The residue was purified by silica gel column chromatography to obtain 55 g (214.53 mmol) of intermediate D-46-1 (yield: 90.18%). MS (ESI) m/z = 257 $[M+1]^+$.

(S2) Preparation of intermediate D-46-2

To a single-neck flask were added intermediate D-46-1 (60 g, 234.03 mmol) and THF/MeOH (100 mL/300 mL). The reaction mixture was cooled under an ice-water bath, and added with an aqueous solution of lithium hydroxide (29.46 g, 702.10 mmol, 200 mL). The reaction mixture was warmed to room temperature and stirred for 2 h. Upon completion, the organic solvent in the reaction mixture was removed under reduced pressure. The residue was diluted with water and extracted with EA twice to obtain EA layers, and the EA layers were discarded. The aqueous phase was then adjusted to pH 4 with 6 M HCl to precipitate a large amount of white solid. The solid was collected by filtration under reduced pressure, washed with water, and dried to obtain 50 g (206.32 mmol) of intermediate D-46-2 (yield: 88.16%). MS (ESI) m/z = 243 $[M+1]^+$.

(S3) Preparation of intermediate D-46-3

To a single-neck flask were added intermediate D-46-2 (62 g, 255.83 mmol) and DMF (1,000 mL). The mixture solution was cooled under an ice-water bath, and added with potassium carbonate (45.96 g, 332.58 mmol). The reaction mixture was warmed to room temperature, stirred for 30 min, cooled again under an ice-water bath, and dropwise added with benzyl bromide (113.77 g, 665.16 mmol). The reaction mixture was heated to room temperature and stirred overnight. Upon completion, the reaction mixture was decanted into ice-water and extracted with EA twice. The EA layers were collected, combined, washed with a saturated NaCl solution three times, dried over anhydrous sodium sulfate, and filtered. The filtrate was concentrated under reduced pressure to dryness. The residue was purified by silica gel column chromatography to obtain 60 g (180.47 mmol) of intermediate D-46-3 (yield: 70.54%). MS (ESI) m/z = 333 $[M+1]^+$.

(S4) Preparation of intermediate D-46-4

To a single-neck flask were added intermediate D-46-3 (20 g, 60.24 mmol) and DCM (150 mL). The reaction mixture was cooled under an ice-water bath, and added with trifluoroacetic acid (150 mL). The reaction mixture was warmed to room temperature and stirred for 1 h, and then concentrated under reduced pressure to dryness. The residue was dissolved in DCM, followed by adjustment to pH 8 with aqueous sodium bicarbonate under stirring. The DCM layer was separated, collected, washed with a saturated NaCl solution twice, dried over anhydrous sodium sulfate, and filtered. The filtrate was concentrated under reduced pressure to dryness to obtain 10.98 g (54.34 mmol) of intermediate D-46-4 (yield: 90.20%). MS (ESI) m/z = 203 $[M+1]^+$.

(S5) Preparation of intermediate D-46-5

The preparation of intermediate D-46-5 was performed according to the synthesis method of intermediate D-1, in which 1,1-difluoro-2-propanol in step (S1) was replaced with N-tert-butyl-3-hydroxyazetidine, with all other reagents and procedures remaining identical. MS (ESI) m/z = 314 $[M+1]^+$.

(S6) Preparation of intermediate D-46

To a single-neck flask were added intermediate D-46-5 (300 mg, 957.26 μmol), methanol (10 mL), Pd/C (45 mg, 15% w/w) and $Pd(OH)_2$ (45 mg, 15% w/w). The reaction mixture was evacuated and purged with hydrogen three times, and stirred at room temperature for 3 h under a hydrogen balloon atmosphere. Upon completion, the reaction mixture was filtered through diatomite. The filter cake was washed with methanol. The filtrate was concentrated under reduced pressure to dryness to obtain 190 mg (850.98 μmol) of intermediate D-46 (yield: 88.90%). MS (ESI) m/z = 224 $[M+1]^+$.

## Preparation of intermediate D-47

A preparation of intermediate D-47 is illustrated as follows:

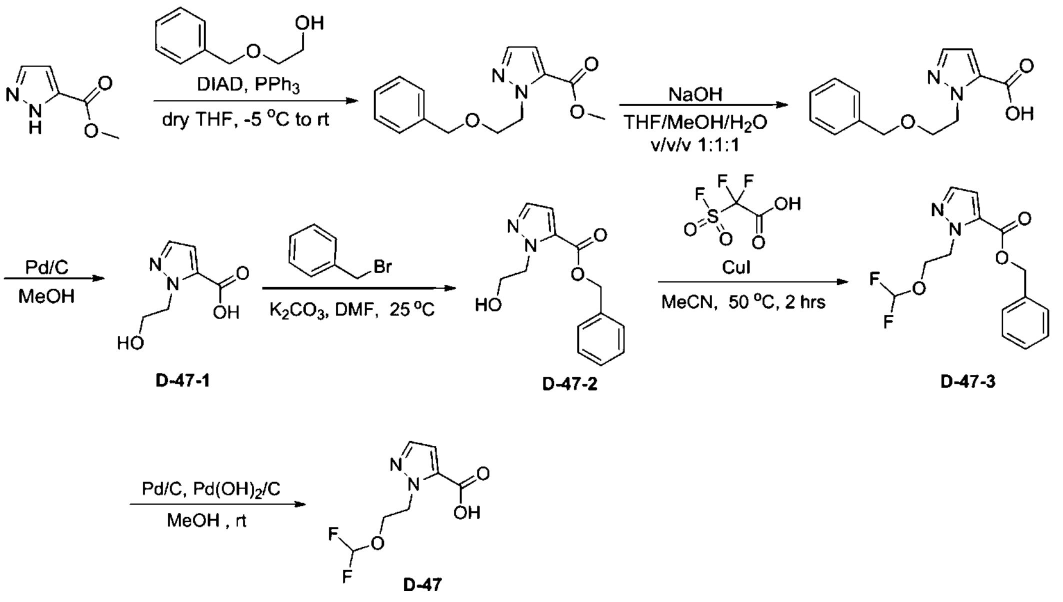


.

(S1-S3) Preparation of intermediate D-47-1

The preparation of intermediate D-47-1 was performed according to the synthesis method of intermediate D-44, in which (R)-(-)-1-benzyloxy-2-propanol in step (S1) was replaced with 2-benzyloxy-1-ethanol, with all other reagents and procedures remaining identical. MS (ESI) m/z = 157 $[M+1]^+$.

(S4) Preparation of intermediate D-47-2

The preparation of intermediate D-47-2 was performed according to the synthesis method of intermediate D-46, in which intermediate D-46-2 in step (S3) was replaced with D-47-1, with all other reagents and procedures remaining identical. MS (ESI) m/z = 247 $[M+1]^+$.

(S5) Preparation of intermediate D-47-3

To a single-neck flask were added intermediate D-47-2 (498.98 mg, 2.80 mmol), acetonitrile (20 mL), and copper(I) iodide (88.94 mg, 466.98 μmol). A solution of 2-fluorosulfonyldifluoroacetic acid (498.98 mg, 2.80 mmol) in acetonitrile (5 mL) was added dropwise to the mixture solution. The reaction mixture was heated to 50°C and stirred for 2 h. Upon completion, the reaction mixture was quenched with an aqueous sodium bicarbonate solution and extracted with EA. The EA layer was collected, washed with a NaCl solution, dried over anhydrous sodium sulfate, and filtered. The filtrate was concentrated under reduced pressure to dryness. The residue was purified by silica gel column chromatography to obtain 145 mg (489.42 μmol) of intermediate D-47-3 (yield: 26.20%). MS (ESI) m/z = 297 $[M+1]^+$.

(S6) Preparation of intermediate D-47

The preparation of intermediate D-47 was performed according to the synthesis method of intermediate D-47-3, in which intermediate D-46-5 in step (S6) was replaced with D-47-3, with all other reagents and procedures remaining identical. MS (ESI) m/z = 207 $[M+1]^+$.

**Preparation of intermediate D-48**

A preparation of intermediate D-48 is illustrated as follows:

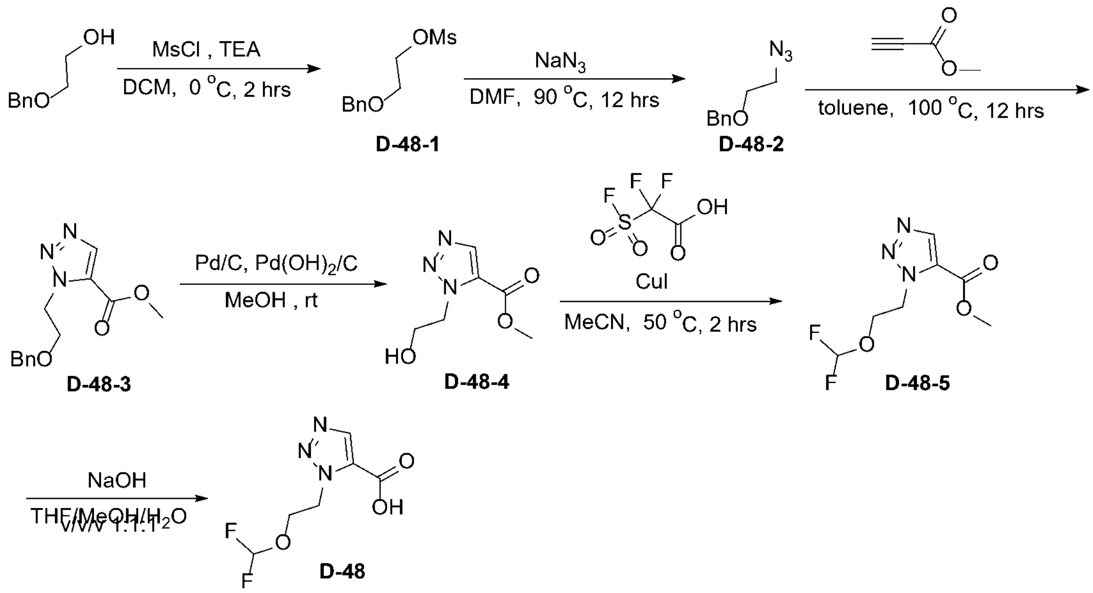


(S1) Preparation of intermediate D-48-1

To a three-neck flask were added 2-benzyloxyethanol (3 g, 19.71 mmol), DCM (100 mL) and triethylamine (7.98 g, 78.85 mmol, 11.00 mL). The reaction mixture was cooled to 0°C under an ice-water bath, dropwise added with methanesulfonyl chloride (4.52 g, 39.42 mmol), warmed to room temperature and stirred for 2 h, quenched with water, and extracted with EA. The EA layers were collected, combined, washed with an aqueous sodium bicarbonate solution, dried over anhydrous sodium sulfate, and filtered. The filtrate was concentrated under reduced pressure to dryness to obtain 5.7 g (19.55 mmol) of intermediate D-48-1 (yield: 99.20%).

(S2) Preparation of intermediate D-48-2

To a single-neck flask were added intermediate D-48-1 (5.7 g, 19.55 mmol), DMF (50 mL), and sodium azide (1.29 g, 19.80 mmol). The reaction mixture was heated to 90°C and stirred for 12 h. Upon completion, the reaction mixture was cooled, quenched with an aqueous sodium bicarbonate solution, and extracted with EA. The EA layers were collected, combined, washed with a saturated NaCl solution, dried over anhydrous sodium sulfate, and filtered. The filtrate was concentrated under reduced pressure to dryness to obtain 2.95 g (16.65 mmol) of intermediate D-48-2 (yield: 84.07%).

(S3) Preparation of intermediate D-48-3

To a single-neck flask were added intermediate D-48-2 (2.95 g, 16.65 mmol), toluene (40 mL) and methyl propiolate (1.40 g, 16.65 mmol). The reaction mixture was heated to 100°C and stirred for 12 h. Upon completion, the reaction mixture was cooled and concentrated under reduced pressure to dryness. The residue was purified by silica gel column chromatography, and the less polar product fraction was collected. The structure was confirmed by $^1H$ NMR and NOE to obtain 749 mg (2.87 mmol) of intermediate D-48-3 (yield: 17.22%). MS (ESI) m/z = 262 $[M+1]^+$. $^1H$ NMR (600 MHz, Methanol-$d_4$) δ 8.15 (s, 1H), 7.29 - 7.19 (m, 3H), 7.17 - 7.12 (m, 2H), 4.96 (t, $J$ = 5.3 Hz, 2H), 4.43 (s, 2H), 3.87 (t, $J$ = 5.3 Hz, 2H), 3.83 (s, 3H).

(S4) Preparation of intermediate D-48-4

To a single-neck flask were added intermediate D-48-3 (749 mg, 2.87 mmol), methanol (20 mL), Pd/C (150 mg, 20% w/w) and $Pd(OH)_2$ (150 mg, 20% w/w). The reaction mixture was evacuated and purged with hydrogen three times, then stirred at room temperature for 12 h under a hydrogen balloon atmosphere. Upon completion, the reaction mixture was filtered through Celite and washed with methanol. The filtrate was concentrated under reduced pressure to dryness to obtain 541 mg (2.84 mmol) of intermediate D-48-4 (yield: 99.24%). MS (ESI) m/z = 172 $[M+1]^+$.

(S5) Preparation of intermediate D-48-5

The preparation of intermediate D-48-5 was performed according to the synthesis method of intermediate D-47, in which intermediate D-47-2 in step (S5) was replaced with D-48-4, with all other reagents and procedures remaining identical. MS (ESI) m/z = 222 $[M+1]^+$.

(S6) Preparation of intermediate D-48

The preparation of intermediate D-48 was performed according to the synthesis method of intermediate D-1, in which intermediate D-1-1 in step (S2) was replaced with D-48-5, with all other reagents and procedures remaining identical. MS (ESI) m/z = 208 $[M+1]^+$.

**Preparation of intermediate D-49**

A preparation of intermediate D-49 is illustrated as follows:

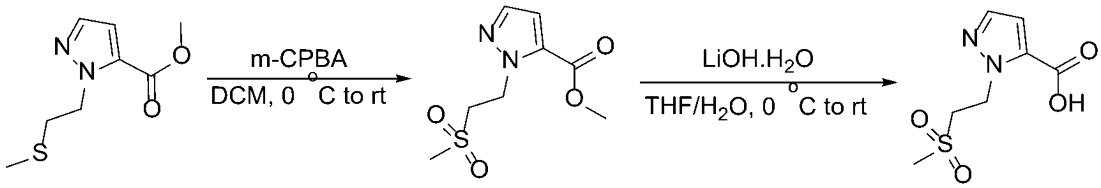


D-43-1 D-49-1 D-49

(S1) Preparation of intermediate D-49-1

To a single-neck flask were added intermediate D-43-1 (189 mg, 943.78 μmol) and DCM (6 mL). The reaction mixture was cooled to 0°C, added with m-CPBA (210.77 mg, 1.04 mmol), heated to room temperature, stirred for 3 h, cooled to 0°C, quenched with a saturated aqueous sodium sulfite solution, and extracted with DCM. The DCM layer was washed with a saturated NaCl solution, dried over anhydrous sodium sulfate, and filtered. The filtrate was concentrated under reduced pressure to dryness. The residue was purified by silica gel column chromatography to obtain 190 mg (818.06 μmol) of intermediate D-49-1 (yield: 86.68%). MS (ESI) m/z = 233 $[M+1]^+$.

(S2) Preparation of intermediate D-49

To a single-neck flask were added intermediate D-49-1 (730 mg, 3.14 mmol) and THF/MeOH (5 mL/5 mL). The reaction mixture was cooled under an ice-water bath, and added with an aqueous solution of lithium hydroxide monohydrate (395.68 mg, 9.43 mmol, 5 mL). The reaction mixture was warmed to room temperature and stirred for 2 h. Upon completion, the organic solvent in the reaction mixture was removed under reduced pressure. The residue was diluted with water and extracted twice with EA to obtain EA layers. The EA layers were discarded. The aqueous phase was then adjusted to pH 4 with 6 M HCl to precipitate a large amount of white solid. The solid was collected by filtration under reduced pressure, washed with water, and dried to obtain 650 mg (2.98 mmol) of intermediate D-49 (yield: 94.76%). MS (ESI) m/z = 219 $[M+1]^+$.

**Preparation of intermediate D-50**

A preparation of intermediate D-50 is illustrated as follows:

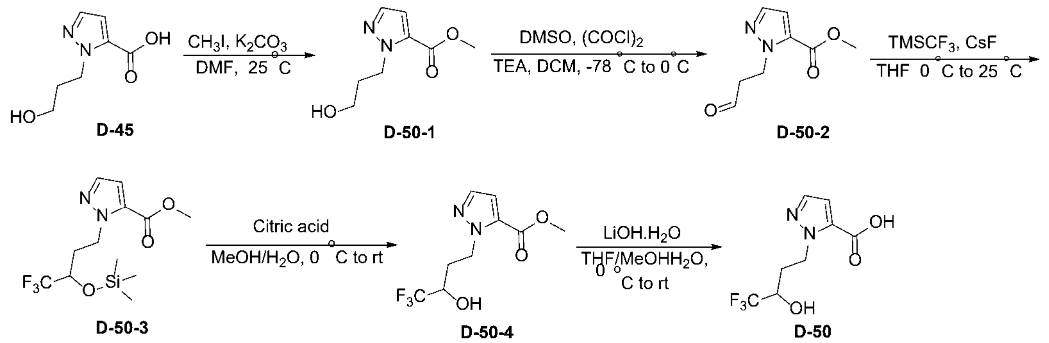

D-45 D-50-1 D-50-2 D-50-3 D-50-4 D-50

(S1) Preparation of intermediate D-50-1

To a single-neck flask were added intermediate D-45 (2 g, 11.76 mmol) and DMF (39 mL). The mixture was cooled under an ice-water bath, and added with potassium carbonate (3.25 g, 23.52 mmol). The reaction mixture was then warmed to room temperature and stirred for 30 min, cooled again under an ice-water bath, and dropwise added with methyl iodide (1.84 g, 12.94 mmol). The reaction mixture was warmed to room temperature and stirred overnight. Upon completion, the reaction mixture was decanted into ice-water and extracted with EA twice. The EA layers were collected, combined, washed with a saturated NaCl solution three times, dried over anhydrous sodium sulfate, and filtered. The filtrate was concentrated under reduced pressure to dryness. The residue was purified by silica gel column chromatography to obtain 2.09 g (11.36 mmol) of intermediate D-50-1 (yield: 96.64%). MS (ESI) m/z = 185 $[M+1]^+$.

(S2) Preparation of intermediate D-50-2

To a three-neck flask were added oxalyl chloride (206.73 mg, 1.63 mmol) and DCM (4 mL). The mixture was cooled to -60°C in a dry ice-ethanol bath, and dropwise added with DMSO (254.52 mg, 3.26 mmol). The mixture was maintained at -60°C and stirred for 30 min, and dropwise added with a solution of intermediate D-50-1 (100 mg, 542.91 μmol) in DCM. The reaction mixture was stirred at the same temperature for 1 h. Triethylamine (329.62 mg, 3.26 mmol) was then added dropwise into the reaction mixture. The reaction mixture was gradually warmed to 0°C and stirred for 30 min. Upon completion, the reaction mixture was decanted into ice-water and extracted with DCM twice. The DCM layers were collected, combined, washed with a saturated NaCl solution three times, dried over anhydrous sodium sulfate, and filtered. The filtrate was concentrated under reduced pressure to dryness to obtain crude intermediate D-50-2 (100 mg, 542.91 μmol).

(S3) Preparation of intermediate D-50-3

To a single-neck flask were added intermediate D-50-2 (100 mg, 542.91 μmol), THF (2 mL), cesium fluoride (4.17 mg, 27.45 μmol) and trimethyl(trifluoromethyl)silane (85.86 mg, 603.81 μmol). The reaction mixture was stirred at room temperature for 2 h, quenched with water, and extracted with EA twice. The EA layers were collected, combined, washed with a saturated NaCl solution three times, dried over anhydrous sodium sulfate, and filtered. The filtrate was concentrated under reduced pressure to dryness. The residue was purified by silica gel column chromatography to obtain 40 mg (158.61

μmol) of intermediate D-50-3 (yield: 28.90%). MS (ESI) m/z = 253 [M+1]+.

(S4) Preparation of intermediate D-50-4

To a single-neck flask were added intermediate D-50-3 (40 mg, 156.61 μmol) and $H_2O$/MeOH (1 mL/1 mL). The reaction mixture was cooled under an ice-water bath, and added with a citric acid (66.6 mg, 317.2 μmol) solution in MeOH (1 mL). The reaction mixture was warmed to room temperature and stirred for 2 h. Upon completion, the organic solvent in the reaction mixture was removed under reduced pressure. The residue was diluted with water and extracted with EA twice to obtain EA layers. The EA layers were collected, combined, washed with a saturated NaCl solution twice, dried over anhydrous sodium sulfate, and filtered. The filtrate was concentrated under reduced pressure to dryness to obtain intermediate D-50-4, requiring no further purification for the next step.

(S5) Preparation of intermediate D-50

To a single-neck flask were added intermediate D-50-4 and THF/MeOH (1 mL/1 mL). The reaction mixture was cooled under an ice-water bath, and added with an aqueous solution of lithium hydroxide monohydrate (19.97 mg, 475.83 μmol, 1 mL). The reaction mixture was warmed to room temperature and stirred for 2 h. Upon completion, the organic solvent in the reaction mixture was removed under reduced pressure. The residue was diluted with water and extracted with EA twice to obtain EA layers, and the EA layers were discarded. The aqueous phase was then adjusted to pH 4 with 6 M HCl and extracted with EA twice. The EA layers were collected, combined, washed with a saturated NaCl solution twice, dried over anhydrous sodium sulfate, and filtered. The filtrate was concentrated under reduced pressure to dryness to obtain 25 mg (104.97 μmol) of intermediate D-50 (yield: 66.18%). MS (ESI) m/z = 239 [M+1]+.

**Preparation of intermediate D-51**

A preparation of intermediate D-51 is illustrated as follows:

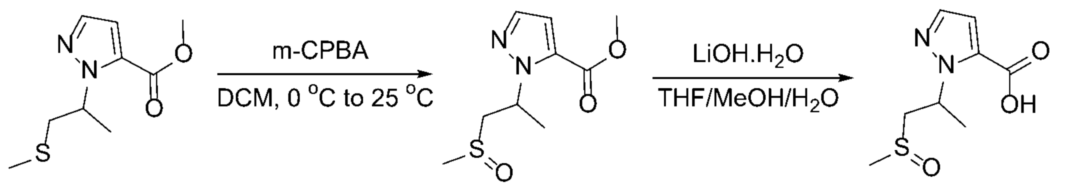


D-39-1 D-51-1 D-51

(S1) Preparation of intermediate D-51-1

To a single-neck flask were added intermediate D-39-1 (280 mg, 1.31 mmol) and DCM (6 mL). The reaction mixture was cooled to 0°C, and added with m-CPBA (247.55 mg, 1.44 mmol). The reaction mixture was warmed to room temperature and stirred for 3 h. Upon completion, the reaction mixture was cooled to 0°C, quenched with saturated aqueous sodium sulfite solution, and extracted with DCM. The DCM layer was collected, washed with a saturated NaCl solution, dried over anhydrous sodium sulfate, and filtered. The filtrate was concentrated under reduced pressure to dryness. The residue was purified by silica gel column chromatography to obtain 248.39 mg (1.08 mmol) of intermediate D-51-1 (yield: 82.54%). MS (ESI) m/z = 231 [M+1]+.

(S2) Preparation of intermediate D-51

To a single-neck flask were added intermediate D-51-1 (200 mg, 868.49 μmol), THF/MeOH/$H_2O$ (3 mL/3 mL/3 mL) and lithium hydroxide hydrate (3.25 g, 23.52 mmol). The reaction mixture was stirred at room temperature for 2 h. Upon completion, the organic solvent in the reaction mixture was removed under reduced pressure. The residue was diluted with an appropriate amount of water, adjusted to pH 4 with 6 M HCl, and extracted with EA twice. The EA layers were collected, combined, washed with a saturated NaCl solution, dried over anhydrous sodium sulfate, and filtered. The filtrate was concentrated under reduced pressure to dryness to obtain 120 mg (554.90 μmol) of crude intermediate D-51 (yield: 63.89%). MS (ESI) m/z = 217 [M+1]+.

Intermediate D-51 was directly used in the next step without further purification.

**Preparation of intermediate D-52**

A preparation of intermediate D-52 is illustrated as follows:

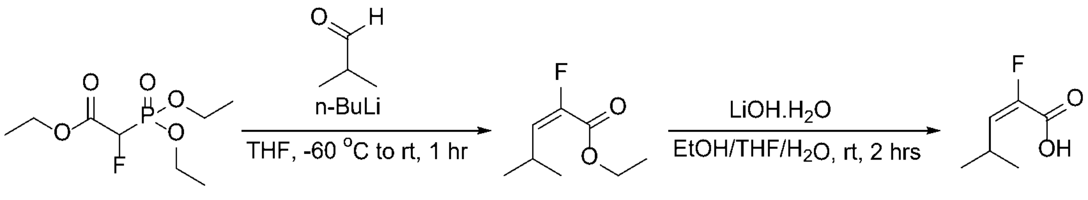


**D-52-1** **D-52**

(S1) Preparation of intermediate D-52-1

To a three-neck flask were added triethyl 2-fluoro-2-phosphonoacetate (1 g, 4.13 mmol) and dry THF (10 mL). The reaction mixture was evacuated and purged with nitrogen three times, cooled to -60°C under a dry ice-ethanol bath, and dropwise added with n-BuLi (1.65 mL, 4.13 mmol). The reaction mixture was maintained at -60°C and stirred for 10 min, and dropwise added with isobutyraldehyde (238.19 mg, 3.30 mmol). The reaction mixture was gradually warmed to room temperature and stirred for 1 h. Upon completion, the reaction mixture was decanted into saturated aqueous ammonium chloride solution for quenching, and extracted with DCM. The DCM layer was collected, washed with a saturated NaCl solution, dried over anhydrous sodium sulfate, and filtered. The filtrate was concentrated under reduced pressure to dryness to obtain 436 mg (2.72 mmol) of crude intermediate D-52-1 (yield: 65.92%), which was directly used in the next step without further purification.

(S2) Preparation of intermediate D-52

To a single-neck flask were added intermediate D-52-1 (436 mg, 2.72 mmol), THF/EtOH/$H_2O$ (8 mL/0.8 mL/3 mL) and lithium hydroxide hydrate (125.75 mg, 2.99 mmol). The reaction mixture was stirred at room temperature for 2 h. Upon completion, the pH was adjusted to 4 with 6 M HCl under an ice-water bath, followed by extraction with DCM twice. The DCM layers were collected, combined, washed with a saturated NaCl solution, dried over anhydrous sodium sulfate, and filtered. The filtrate was concentrated under reduced pressure to dryness to obtain 337 mg (2.55 mmol) of crude intermediate D-52 (yield: 93.70%). MS (ESI) m/z = 131 $[M-1]^+$. Intermediate D-52 was directly used in the next step without further purification.

**Preparation of intermediate D-53**

A structure of intermediate D-53 is as follows:

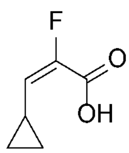

**D-53**

The preparation of intermediate D-53 was performed according to the synthesis method of intermediate D-52, in which isobutyraldehyde in step (S1) was replaced with cyclopropanecarboxaldehyde, with all other reagents and procedures remaining identical. MS (ESI) m/z = 129 $[M-1]^+$. Intermediate D-53 was used directly in the next step without purification.

**Preparation of intermediate D-54**

A structure of intermediate D-54 is as follows:

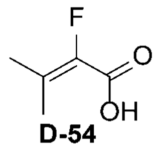

**D-54**

The preparation of intermediate D-54 was performed according to the synthesis method of intermediate D-52, in which isobutyraldehyde in step (S1) was replaced with acetone, with all other reagents and procedures remaining identical. MS (ESI) m/z = 117 [M-1]$^+$. Intermediate D-54 was used directly in the next step without purification.

**Preparation of intermediate D-55**

A preparation of intermediate D-55 is illustrated as follows:

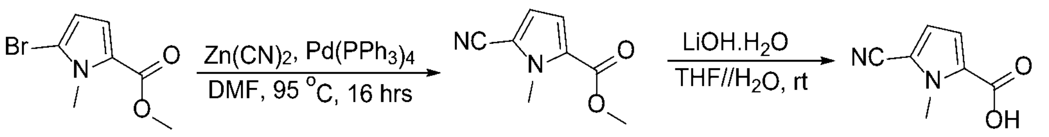


**D-55-1** **D-55**

(S1) Preparation of intermediate D-55-1

To a single-neck flask were added methyl 5-bromo-1-methyl-1H-pyrrole-2-carboxylate (1 g, 4.59 mmol), DMF (15 mL), zinc cyanide (807.76 mg, 6.88 mmol) and $Pd(PPh_3)_4$ (264.85 mg, 229.31 μmol). The reaction mixture was evacuated and purged with nitrogen three times, heated to 95°C and stirred for 16 h. Upon completion, the reaction mixture was quenched with water, and extracted with EA twice. The EA layers were collected, combined, washed with a saturated NaCl solution, dried over anhydrous sodium sulfate, and filtered. The filtrate was concentrated under reduced pressure to dryness. The residue was purified by silica gel column chromatography to obtain 680 mg (4.14 mmol) of intermediate D-55-1 (yield: 90.32%). $^1$H NMR (600 MHz, Chloroform-*d*) δ 6.89 (d, *J* = 4.3 Hz, 1H), 6.74 (d, *J* = 4.3 Hz, 1H), 4.07 (s, 3H), 3.87 (s, 3H).

(S2) Preparation of intermediate D-55

To a single-neck flask were added intermediate D-55-1 (630 mg, 3.84 mmol), THF/$H_2O$ (6 mL/6 mL) and lithium hydroxide hydrate (319.76 mg, 7.68 mmol). The reaction mixture was stirred at room temperature for 2 h. Upon completion, the organic solvent in the reaction mixture was removed under reduced pressure. The residue was diluted with an appropriate amount of water and adjusted to pH 4 with 6 M HCl to precipitate a large amount of white solid. The solid was collected by filtration, washed with water, and dried to obtain 527 mg (3.51 mmol) of intermediate D-55 (yield: 91.47%). MS (ESI) m/z = 151 [M+1]$^+$.

**Preparation of intermediate D-56**

A preparation of intermediate D-56 is illustrated as follows:

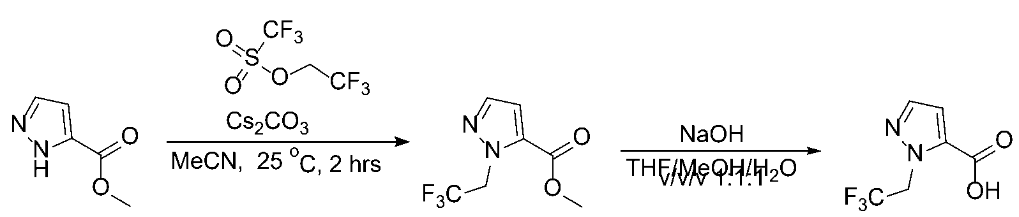


**D-56-1** **D-56**

(S1) Preparation of intermediate D-56-1

To a single-neck flask were added methyl 1H-pyrazole-3-carboxylate (1.40 g, 11.09 mmol) and acetonitrile (50

mL). To the mixture solution were added cesium carbonate (3.62 g, 11.09 mmol) under stirring and dropwise added ethyl trifluoromethanesulfonate (1.97 g, 11.09 mmol) under an ice-water bath. The reaction mixture was stirred at room temperature for 2 h. Upon completion, the reaction mixture was filtered through Celite, and the filtrate was concentrated under reduced pressure to dryness. The residue was purified by silica gel column chromatography to obtain 1.94 g (9.34 mmol) of intermediate D-56-1 (yield: 84.21%). MS (ESI) m/z = 209 $[M+1]^+$.

(S2) Preparation of intermediate D-56

The preparation of intermediate D-56 was performed according to the synthesis method of intermediate D-1, in which D-1-1 in step (S2) was replaced with D-56-1, with all other reagents and procedures remaining identical. MS (ESI) m/z = 195 $[M+1]^+$.

**Preparation of intermediate D-57**

A preparation of intermediate D-57 is illustrated as follows:

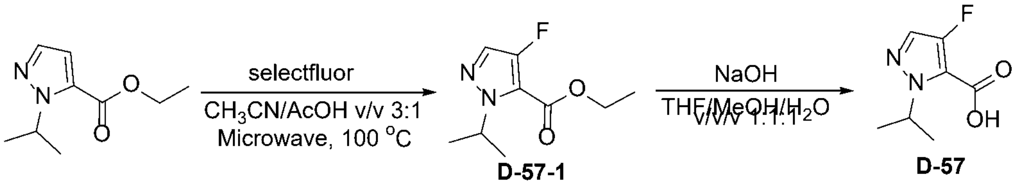


(S1) Preparation of intermediate D-57-1

To a 30 mL microwave tube were added ethyl 1-isopropyl-1H-pyrazole-5-carboxylate (500 mg, 2.74 mmol), acetonitrile/glacial acetic acid (6.9 mL/2.3 mL) and selectfluor (1.46 g, 4.12 mmol). The reaction mixture was purged with nitrogen for 1 min. The reaction mixture was then placed in a microwave reactor, heated to 100°C, and stirred for 6 h. LCMS and TLC indicated that only about half of the starting material had reacted. The reaction mixture was quenched with water, adjusted to pH 8 with an aqueous potassium carbonate solution, and extracted with DCM. The DCM layer was collected, dried over anhydrous sodium sulfate and filtered. The filtrate was concentrated under reduced pressure to dryness. The residue was purified by silica gel column chromatography to obtain 0.2 g (998.95 μmol) of intermediate D-57-1 (yield: 36.41 %). MS (ESI) m/z = 201 $[M+1]^+$.

(S2) Preparation of intermediate D-57

The preparation of intermediate D-57 was performed according to the synthesis method of intermediate D-1, in which D-1-1 in step (S2) was replaced with D-57-1, with all other reagents and procedures remaining identical. MS (ESI) m/z = 173 $[M+1]^+$.

**Preparation of intermediate D-58**

A preparation of intermediate D-58 is illustrated as follows:

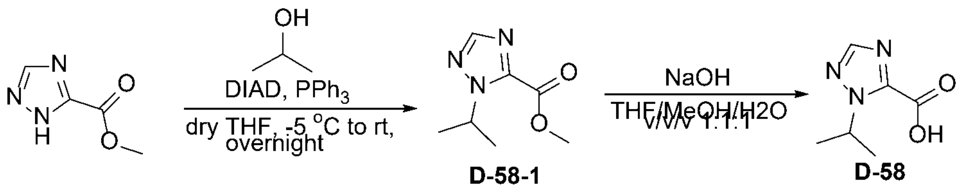


The preparation of intermediate D-58 was performed according to the synthesis method of intermediate D-1, in which methyl 1H-pyrazole-3-carboxylate in step (S1) was replaced with methyl 1,2,4-triazole-3-carboxylate, and 1,1-difluoro-2-propanol was replaced with isopropanol, with all other reagents and procedures remaining identical. Intermediate D-58-1 was confirmed by $^1H$ NMR and NOE. MS (ESI) m/z = 156 $[M+1]^+$. D-58-1: $^1H$ NMR (600 MHz, Methanol-$d_4$) δ 8.05 (s, 1H), 5.55 (p, $J$ = 6.6 Hz, 1H), 3.99 (s, 3H), 1.51 (d, $J$ = 6.6 Hz, 6H).

**Preparation of intermediate D-59**

A preparation of intermediate D-59 is illustrated as follows:

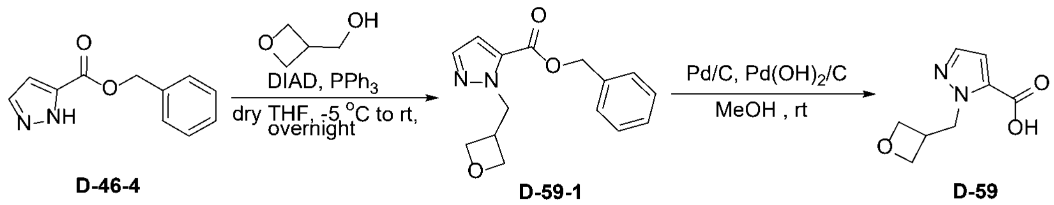


The preparation of intermediate D-59 was performed according to the synthesis method of intermediate D-46, in which N-tert-butyl-3-hydroxyazetidine in step (S5) was replaced with oxetan-3-ylmethanol, with all other reagents and procedures remaining identical. MS (ESI) m/z = 183 $[M+1]^+$.

**Preparation of intermediate E-1**

A preparation of intermediate E-1 is illustrated as follows:

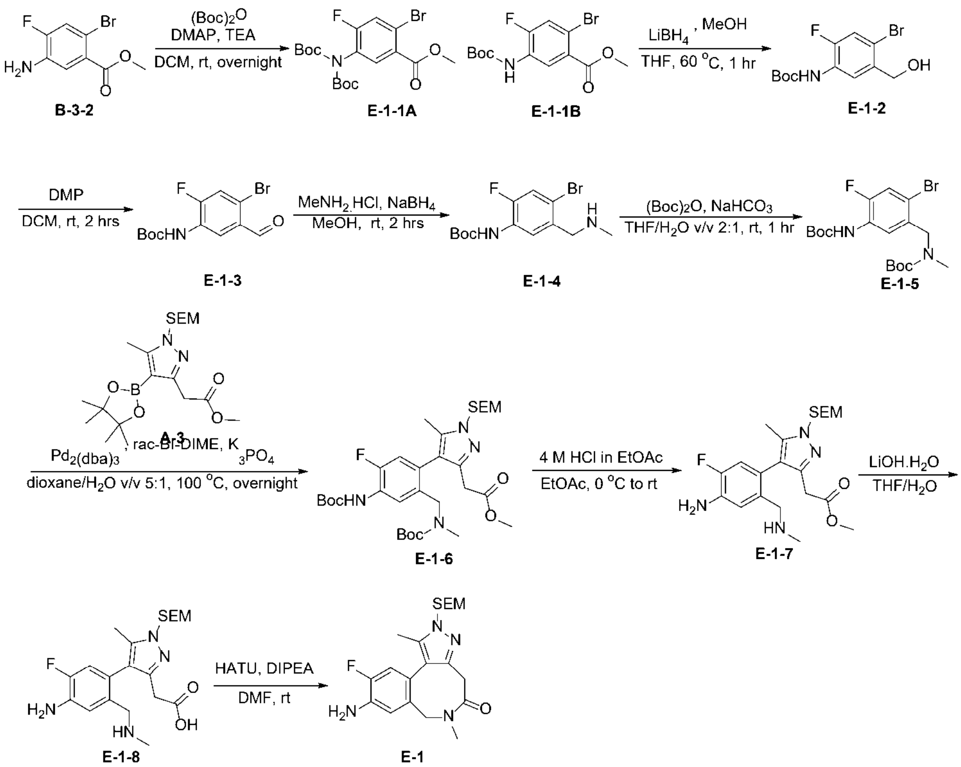


(S1) Preparation of intermediate E-1-1A and E-1-1B

To a single-neck flask were added intermediate B-3-2 (247 g, 995.77 mmol) and DCM (2 L). DMAP (24.4 g, 199.72 mmol) was added to the mixture solution at room temperature, followed by addition of Boc anhydride (24.4 g, 199.72 mmol) under an ice-water bath. The reaction mixture was stirred at room temperature overnight. Upon completion, the reaction mixture was concentrated under reduced pressure to dryness. The residue was purified by silica gel column chromatography to obtain 280.8 g (626.39 mmol) of intermediate E-1-1A (yield: 62.91%, MS (ESI) m/z = 448 $[M+1]^+$) and 79.5 g (228.34 mmol) of intermediate E-1-1B (yield: 22.93%, MS (ESI) m/z = 348 $[M+1]^+$).

(S2) Preparation of intermediate E-1-2

To a single-neck flask were added intermediate E-1-1A (280.8 g, 626.39 mmol) and THF (2 L). Methanol (28 g,

873.91 mmol) and lithium borohydride (41 g, 1.88 mol) were added to the mixture solution at room temperature. The reaction mixture was heated to 60°C and stirred for 1 h. Upon completion, the reaction mixture was cooled, quenched with a saturated aqueous ammonium chloride solution, and extracted with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 153.7 g (480.08 mmol) of intermediate E-1-2 (yield: 76.64%). MS (ESI) m/z = 320 $[M+1]^+$.

(S3) Preparation of intermediate E-1-3

To a single-neck flask were added intermediate E-1-2 (13.56 g, 42.35 mmol) and DCM (140 mL). DMP (21.56 g, 50.83 mmol) was batchwise added to the mixture solution under an ice-water bath. The reaction mixture was stirred at room temperature for 2 h. Upon completion, the reaction mixture was quenched with 1 M $Na_2S_2O_3$ solution and 1 M $NaHCO_3$ solution, stirred for 30 min, and extracted with DCM. The DCM layer was collected, washed with a saturated NaCl solution, dried over anhydrous sodium sulfate, and filtered. The filtrate was concentrated under reduced pressure to dryness. The residue was purified by silica gel column chromatography to obtain 12 g (37.72 mmol) of intermediate E-1-3 (yield: 89.06%). MS (ESI) m/z = 318 $[M+1]^+$.

(S4) Preparation of intermediate E-1-4

To a single-neck flask were added intermediate E-1-3 (4.42 g, 13.89 mmol), methanol (50 mL) and methylamine hydrochloride (862.97 mg, 27.79 mmol) at room temperature. The reaction mixture was stirred at room temperature for 1 h, cooled to 0°C under an ice-water bath, and batchwise added with sodium borohydride (1.05 g, 27.79 mmol). The reaction mixture was warmed to room temperature and stirred for 2 h. Upon completion, the reaction mixture was cooled and quenched with saturated aqueous ammonium chloride solution, followed by extraction with EA. The EA layer was collected, washed with a saturated NaCl solution, dried over anhydrous sodium sulfate, and filtered. The filtrate was concentrated under reduced pressure to dryness to obtain 4.62 g (13.87 mmol) of crude intermediate E-1-4 (yield: 99.80%), which was directly used in the next step without further purification. MS (ESI) m/z = 333 $[M+1]^+$.

(S5) Preparation of intermediate E-1-5

To a single-neck flask were added intermediate E-1-4 (4.28 g, 12.85 mmol) and THF/water (40 mL/20 mL). Sodium bicarbonate (2.16 g, 25.69 mmol) and di-tert-butyl dicarbonate (4.21 g, 19.27 mmol) were sequentially added to the mixture solution at room temperature. The reaction mixture was stirred at room temperature for 2 h. Upon completion, the reaction mixture was diluted with water and extracted with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 5.33 g (12.30 mmol) of intermediate E-1-5 (yield: 95.76%). MS (ESI) m/z = 433 $[M+1]^+$.

(S6) Preparation of intermediate E-1-6

To a single-neck flask were added intermediate E-1-5 (5.0 g, 11.57 mmol), intermediate A-3 (4.75 g, 11.57 mmol), dioxane and water (100 mL/20 mL). $Pd_2(dba)_3$ (264.76 mg, 289.25 μmol), rac-BI-DIME (190.97 mg, 578.50 μmol), and potassium phosphate (4.91 g, 23.14 mmol) were added to the mixture solution at room temperature. The reaction mixture was evacuated and purged with nitrogen three times, gradually heated to 100°C and stirred overnight. Upon completion, the reaction mixture was quenched with water and extracted with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 4.32 g (6.78 mmol) of intermediate E-1-6 (yield: 58.64%). MS (ESI) m/z = 637 $[M+1]^+$.

(S7) Preparation of intermediate E-1-7

To a single-neck flask were added intermediate E-1-6 (4.0 g, 6.29 mmol) and EA (15 mL). A solution of 4 M HCl in EA (142 mL) was added to the mixture solution under an ice-water bath. The reaction mixture was gradually warmed to room temperature and stirred for 1 h. Upon completion, the reaction mixture was cooled under an ice-water bath and slowly decanted into saturated aqueous sodium bicarbonate solution, while maintaining the pH of the solution on the basic side throughout the addition. The reaction mixture was then extracted with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 1.51 g (3.47 mmol) of

intermediate E-1-7 (yield: 55.15%). MS (ESI) m/z = 437 $[M+1]^+$.

<u>(S8) Preparation of intermediate E-1-8</u>

To a single-neck flask were added intermediate E-1-7 (1.51 g, 3.47 mmol) and THF/water (10 mL/10 mL). The reaction mixture was added with lithium hydroxide hydrate (0.29 g, 6.93 mmol) at room temperature, and stirred at room temperature for 2 h. Upon completion, the reaction mixture was adjusted to neutral pH with 2 M HCl under an ice-water bath, and concentrated under reduced pressure to dryness to obtain 1.46 g (3.47 mmol) of crude intermediate E-1-8 (calculated as quantitative yield). MS (ESI) m/z = 423 $[M+1]^+$.

<u>(S9) Preparation of intermediate E-1</u>

To a single-neck flask were added intermediate E-1-8 (1.46 g, 3.47 mmol), DMF (18 mL), HATU (1.58 g, 4.16 mmol) and DIPEA (1.34 g, 10.41 mmol) at room temperature. The reaction mixture was stirred at room temperature for 2 h. Upon completion, the reaction mixture was quenched with water and extracted with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 0.99 g (2.45 mmol) of intermediate E-1 (yield: 70.58%). MS (ESI) m/z = 405 $[M+1]^+$.

**<u>Preparation of intermediates E-2 to E-15</u>**

The preparation of intermediates E-2 to E-15 was performed according to the synthesis method of intermediate E-1, in which methylamine hydrochloride in step (S4) was correspondingly replaced with an amine from the following table, with all other reagents and procedures remaining identical.

| Amine | Structure of intermediate | $^1$HNMR and/or MS (ESI) |
|---|---|---|
| 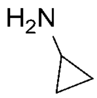 | 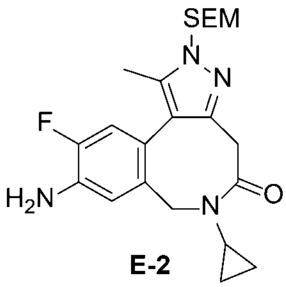 E-2 | MS (ESI) m/z =431 $[M+1]^+$ |
| 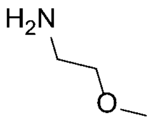 | 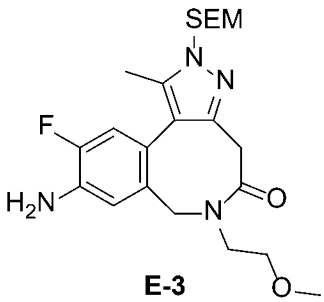 E-3 | MS (ESI) m/z =449 $[M+1]^+$ |
| 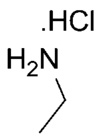 | 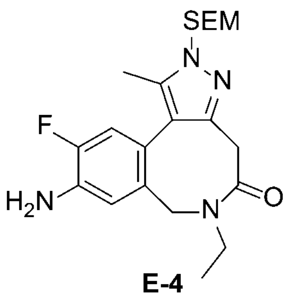 E-4 | MS (ESI) m/z =419 $[M+1]^+$ |

(continued)

| Amine | Structure of intermediate | $^{1}$HNMR and/or MS (ESI) |
|---|---|---|
| 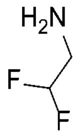 | 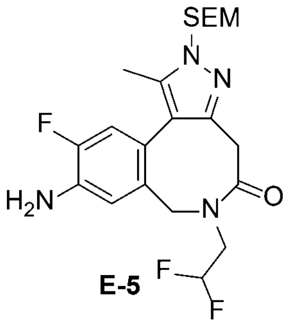 E-5 | MS (ESI) m/z =455 $[M+1]^+$ |
| 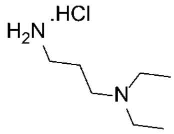 | 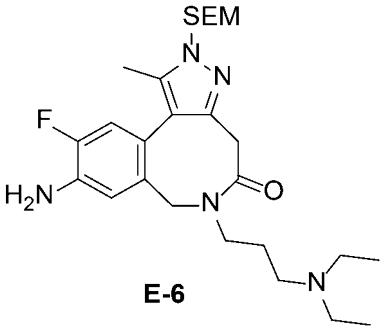 E-6 | MS (ESI) m/z =504 $[M+1]^+$ |
| 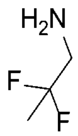 | 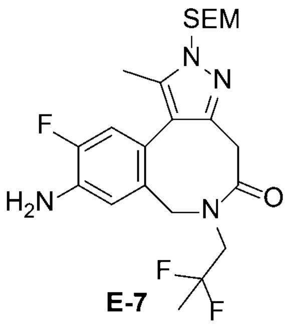 E-7 | MS (ESI) m/z =469 $[M+1]^+$ |
| 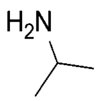 | 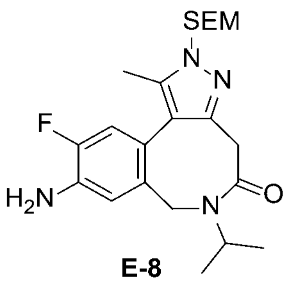 E-8 | MS (ESI) m/z =433 $[M+1]^+$ |
| 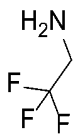 | 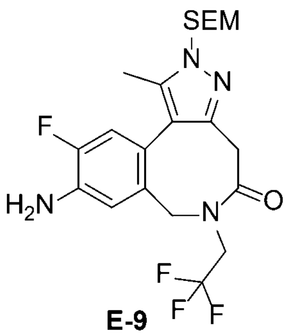 E-9 | MS (ESI) m/z =473 $[M+1]^+$ |

(continued)

| Amine | Structure of intermediate | $^{1}$HNMR and/or MS (ESI) |
|---|---|---|
| 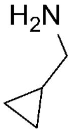 | 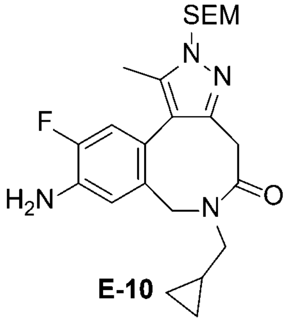 E-10 | MS (ESI) m/z =445 $[M+1]^+$ |
| 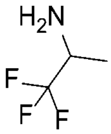 | 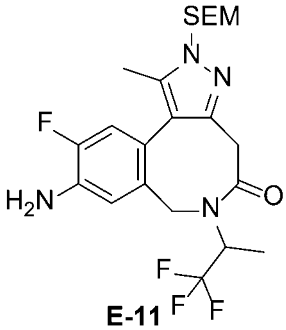 E-11 | MS (ESI) m/z =487 $[M+1]^+$ |
| 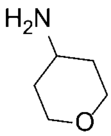 | 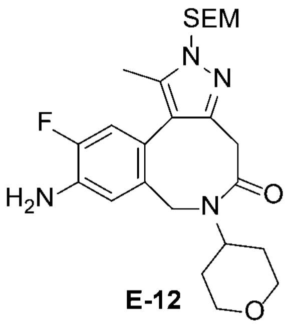 E-12 | MS (ESI) m/z =475 $[M+1]^+$ |
| 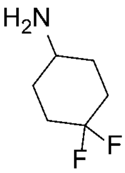 | 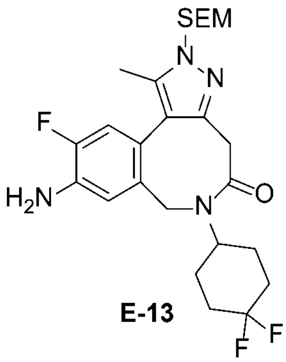 E-13 | MS (ESI) m/z =509 $[M+1]^+$ |

(continued)

| Amine | Structure of intermediate | 1HNMR and/or MS (ESI) |
|---|---|---|
| 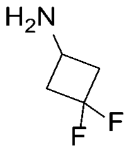 | 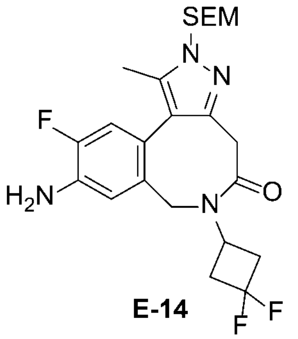 E-14 | MS (ESI) m/z =481 [M+1]+ |
| 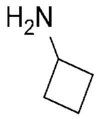 | 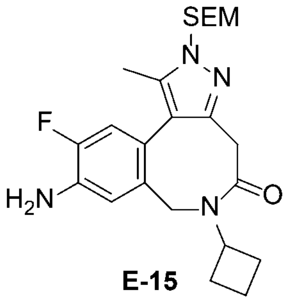 E-15 | MS (ESI) m/z =445 [M+1]+ |

## Preparation of intermediate E-16

A preparation of intermediate E-16 is illustrated as follows:

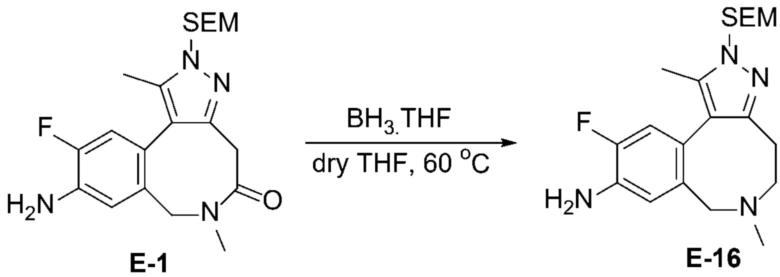


E-1 E-16

To a three-neck flask were added intermediate E-1 (0.5 g, 1.24 mmol) and dry THF (20 mL). The reaction mixture was evacuated and purged with nitrogen three times, and added with borane-THF solution (7.44 mL, 7.44 mmol) by a syringe. The reaction mixture was gradually heated to 60°C and stirred for 6 h. Upon completion, the reaction mixture was cooled, quenched by dropwise adding methanol under an ice-water bath, and concentrated under reduced pressure to dryness. The residue was purified by silica gel column chromatography to obtain 0.28 g (0.72 mmol) of intermediate E-16 (yield: 58.0%). MS (ESI) m/z = 391 [M+1]+.

## Preparation of intermediates E-17 to E-28

The preparation of intermediates E-17 to E-28 was performed according to the synthesis method of intermediate E-16, in which intermediate E-1 was correspondingly replaced with a macrocyclic amide from the following table, with all other reagents and procedures remaining identical.

| Macrocyclic amide | Structure of intermediate | $^1$HNMR and/or MS (ESI) |
|---|---|---|
| 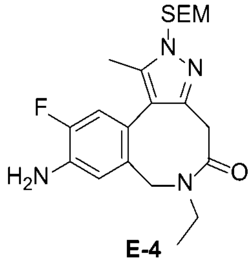 E-4 | 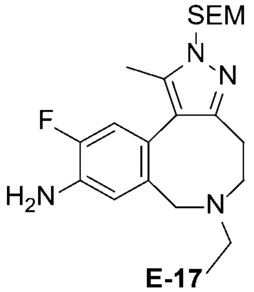 E-17 | MS (ESI) m/z =405 [M+1]$^+$ |
| 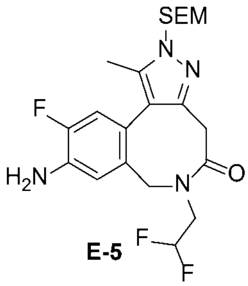 E-5 | 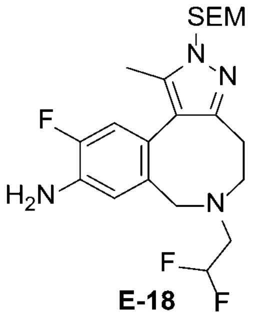 E-18 | MS (ESI) m/z =441 [M+1]$^+$ |
| 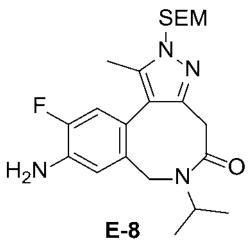 E-8 | 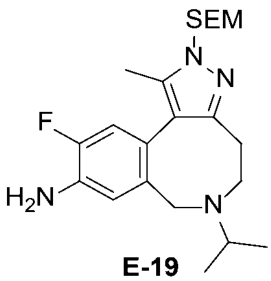 E-19 | MS (ESI) m/z =419 [M+1]$^+$ |
| 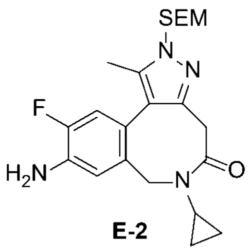 E-2 | 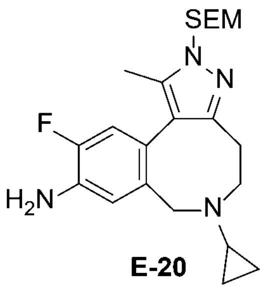 E-20 | MS (ESI) m/z =417 [M+1]$^+$ |
| 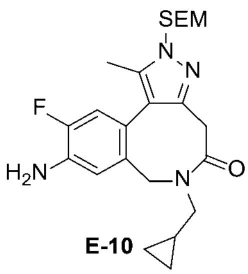 E-10 | 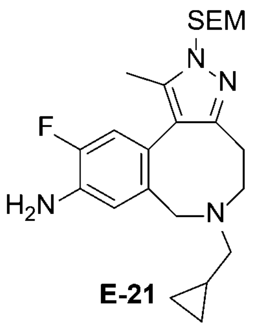 E-21 | MS (ESI) m/z =431 [M+1]$^+$ |

(continued)

| Macrocyclic amide | Structure of intermediate | $^1$HNMR and/or MS (ESI) |
|---|---|---|
| 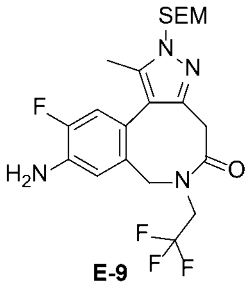 E-9 | 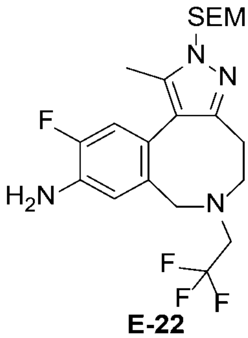 E-22 | MS (ESI) m/z =459 $[M+1]^+$ |
| 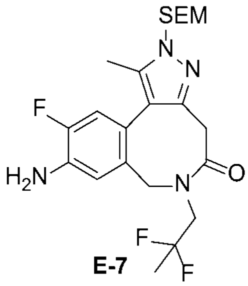 E-7 | 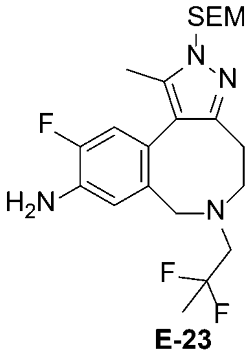 E-23 | MS (ESI) m/z =455 $[M+1]^+$ |
| 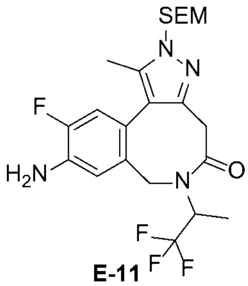 E-11 | 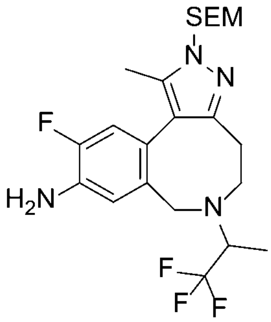 E-24 | MS (ESI) m/z =473 $[M+1]^+$ |
| 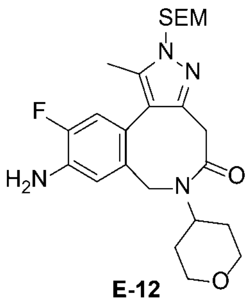 E-12 | 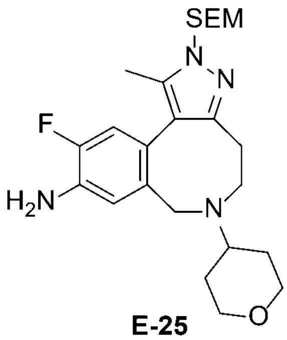 E-25 | MS (ESI) m/z =461 $[M+1]^+$ |

(continued)

| Macrocyclic amide | Structure of intermediate | $^1$HNMR and/or MS (ESI) |
|---|---|---|
| 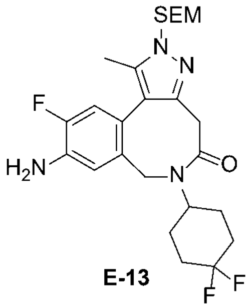 E-13 | 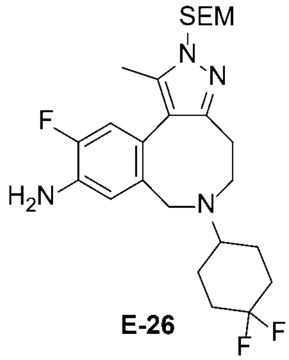 E-26 | MS (ESI) m/z =495 $[M+1]^+$ |
| 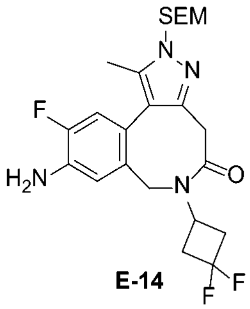 E-14 | 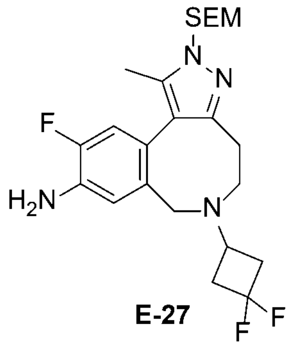 E-27 | MS (ESI) m/z =467 $[M+1]^+$ |
| 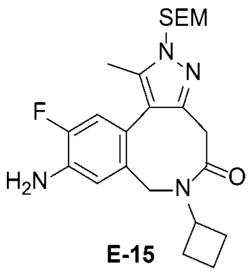 E-15 | 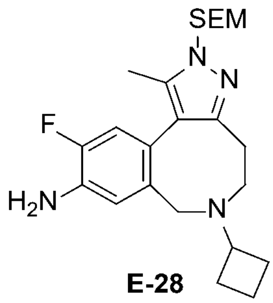 E-28 | MS (ESI) m/z =431 $[M+1]^+$ |

## Preparation of intermediate E-29

A preparation of intermediate E-29 is illustrated as follows:

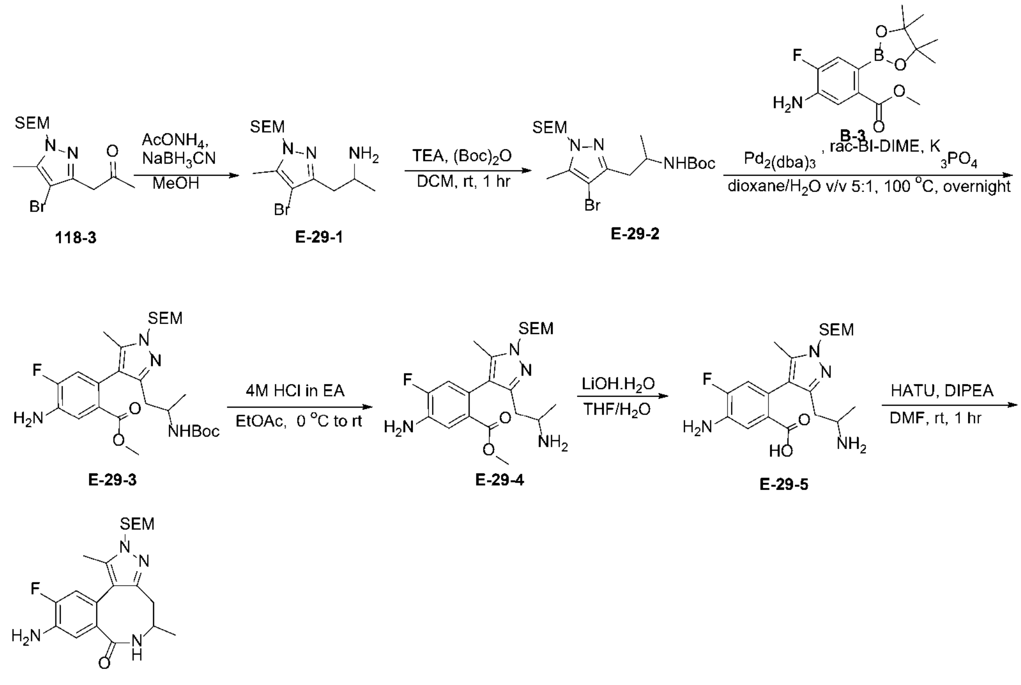


118-3 E-29-1 E-29-2

E-29-3 E-29-4 E-29-5

E-29

(S1) Preparation of intermediate E-29-1

To a single-neck flask were added intermediate 118-3 (5.6 g, 16.12 mmol) and methanol (50 mL). The reaction mixture was added with ammonium acetate (6.21 g, 80.62 mmol) at room temperature, heated to 50°C, and stirred for 3 h. The reaction mixture was cooled, batchwise added with sodium cyanoborohydride (2.03 g, 32.25 mmol), and stirred at room temperature for 2 h. Upon completion, the reaction mixture was quenched with a sodium bicarbonate solution and extracted with EA. The EA layer was collected, washed with a NaCl solution, dried over anhydrous sodium sulfate, and filtered. The filtrate was concentrated under reduced pressure to dryness. The residue was purified by MPLC to obtain 5.2 g (14.93 mmol) of intermediate E-29-1 (yield: 92.58%). MS (ESI) m/z = 348 $[M+1]^+$.

(S2) Preparation of intermediate E-29-2

To a single-neck flask were added intermediate E-29-1 (250 mg, 717.66 μmol), DCM (8 mL), triethylamine (145.24 mg, 1.44 mmol) and di-tert-butyl dicarbonate (187.95 mg, 861.19 μmol) at room temperature. The reaction mixture was stirred at room temperature for 3 h. Upon completion, the reaction mixture was quenched with water and extracted with DCM. The DCM layer was collected, washed with a NaCl solution, dried over anhydrous sodium sulfate, and filtered. The filtrate was concentrated under reduced pressure to dryness. The residue was purified by silica gel column chromatography to obtain 180 mg (401.37 μmol) of intermediate E-29-2 (yield: 55.93%). MS (ESI) m/z = 448 $[M+1]^+$.

(S3-S6) Preparation of intermediate E-29

The preparation of intermediate E-29 was performed according to the synthesis method of intermediate E-1, in which intermediate A-3 in step (S7) was replaced with intermediate E-29-2, and intermediate E-1-5 was replaced with intermediate B-3, with all other reagents and procedures remaining identical. MS (ESI) m/z = 405 $[M+1]^+$.

**Preparation of intermediate E-30**

A preparation of intermediate E-30 is illustrated as follows:

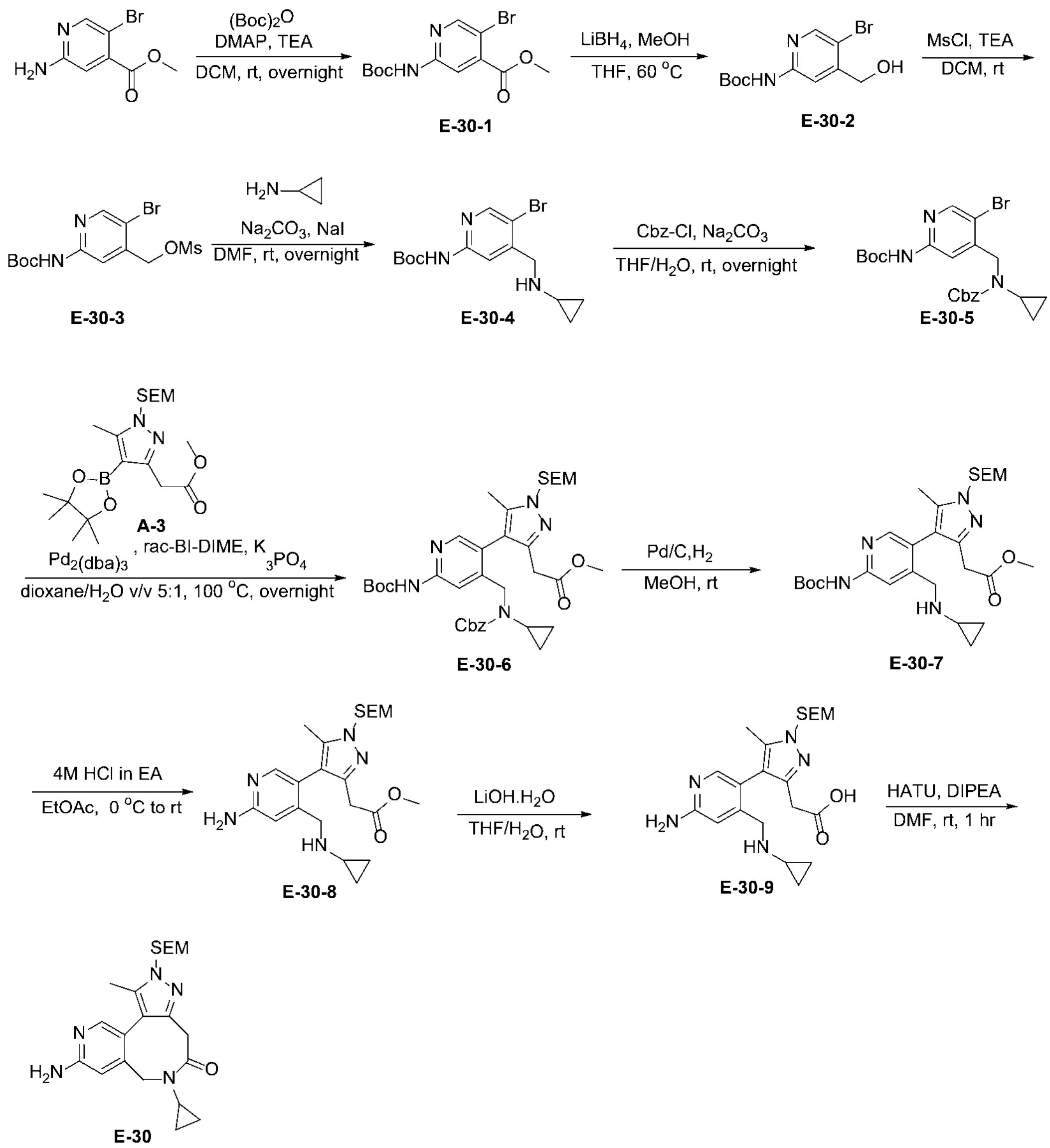


## (S1-S2) Preparation of intermediate E-30-2

The preparation of intermediate E-30-2 was performed according to the synthesis method of intermediate E-1, in which intermediate B-3-2 in step (S1) was replaced with methyl 2-amino-5-bromoisonicotinate, with all other reagents and procedures remaining identical. MS (ESI) m/z = 303 $[M+1]^+$.

## (S3) Preparation of intermediate E-30-3

To a single-neck flask were added intermediate E-30-2 (18.2 g, 60.04 mmol), DCM (300 mL) and triethylamine (12.15 g, 120.07 mmol, 16.75 mL) at room temperature, followed by dropwise addition of methanesulfonyl chloride (7.56 g, 66.04 mmol) under an ice-water bath. The reaction mixture was stirred at room temperature for 3 h. Upon completion, the reaction mixture was diluted with water and extracted with DCM. The DCM layer was collected, washed sequentially with a sodium bicarbonate solution and a NaCl solution, dried over anhydrous sodium sulfate, and filtered. The filtrate was concentrated under reduced pressure to dryness to obtain 18.8 g (49.31 mmol) of intermediate E-30-3 (yield: 82.14%), which was used directly in the next step without further purification. MS (ESI) m/z = 381 $[M+1]^+$.

## (S4) Preparation of intermediate E-30-4

To a single-neck flask were added intermediate E-30-3 (10 g, 26.32 mmol), DMF (130 mL), cyclopropylamine (18 g, 31.58 mmol), sodium carbonate (5.58 g, 52.64 mmol) and sodium iodide (3.95 g, 26.32 mmol) at room temperature. The

reaction mixture was stirred at room temperature overnight. Upon completion, the reaction mixture was quenched with water and extracted with EA. The EA layer was collected, washed with a NaCl solution, dried over anhydrous sodium sulfate, and filtered. The filtrate was concentrated under reduced pressure to dryness. The residue was purified by silica gel column chromatography to obtain 7.69 g (22.55 mmol) of intermediate E-30-4 (yield: 85.68%). MS (ESI) m/z = 342 $[M+1]^+$.

(S5) Preparation of intermediate E-30-5

To a single-neck flask were added intermediate E-30-4 (1.98 g, 5.78 mmol), THF/water (15 mL/15 mL), sodium carbonate (1.84 g, 17.35 mmol) and benzyl chloroformate (1.48 g, 8.67 mmol) at room temperature. The reaction mixture was stirred at room temperature for 2 h. Upon completion, the reaction mixture was diluted with water and extracted with EA. The EA layer was collected, washed with a NaCl solution, dried over anhydrous sodium sulfate, and filtered. The filtrate was concentrated under reduced pressure to dryness. The residue was purified by silica gel column chromatography to obtain 2.2 g (4.62 mmol) of intermediate E-30-5 (yield: 79.87%). MS (ESI) m/z = 476 $[M+1]^+$.

(S6) Preparation of intermediate E-30-6

The preparation of intermediate E-30-6 was performed according to the synthesis method of intermediate E-1, in which intermediate B-1-5 in step (S6) was replaced with intermediate E-30-5, with all other reagents and procedures remaining identical. MS (ESI) m/z = 680 $[M+1]^+$.

(S7) Preparation of intermediate E-30-7

To a single-neck flask were added intermediate E-30-6 (1.57 g, 2.31 mmol), methanol (30 mL) and Pd/C (471 mg, w/w 30%) at room temperature. The reaction mixture was evacuated and purged with hydrogen three times, and stirred at room temperature for 3 h. Upon completion, the reaction mixture was filtered through Celite and washed with methanol. The filtrate was concentrated under reduced pressure to dryness to obtain 966 mg (1.77 mmol) of intermediate E-30-7 (yield: 76.65%). MS (ESI) m/z = 546 $[M+1]^+$.

(S8-S10) Preparation of intermediate E-30

The preparation of intermediate E-30 was performed according to the synthesis method of intermediate E-1, in which intermediate B-1-6 in step (S7) was replaced with intermediate E-30-7, with all other reagents and procedures remaining identical. MS (ESI) m/z = 414 $[M+1]^+$.

**Preparation of intermediates E-31 to E-36**

The preparation of intermediates E-31 to E-36 was performed according to the synthesis method of intermediate E-30, in which cyclopropylamine in step (S4) was correspondingly replaced with an amine from the following table, with all other reagents and procedures remaining identical.

| Amine | Structure of intermediate | $^1$HNMR and/or MS (ESI) |
|---|---|---|
| 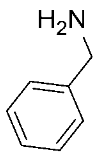 | 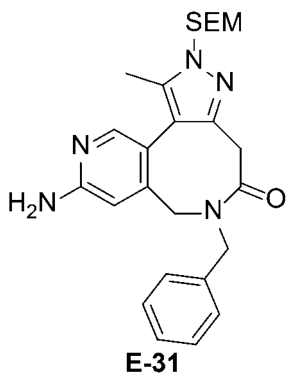 **E-31** | MS (ESI) m/z =464 $[M+1]^+$ |

(continued)

| Amine | Structure of intermediate | $^1$HNMR and/or MS (ESI) |
|---|---|---|
| 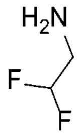 | 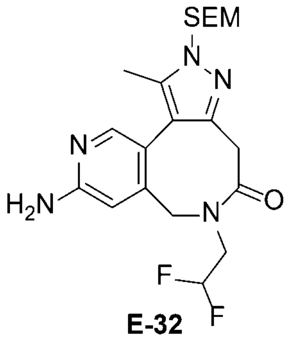 E-32 | MS (ESI) m/z =438 $[M+1]^+$ |
| 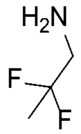 | 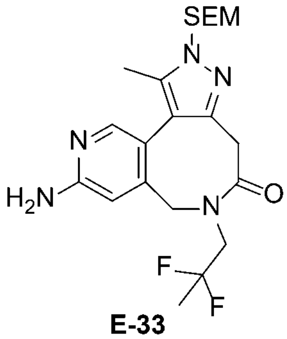 E-33 | MS (ESI) m/z =452 $[M+1]^+$ |
| 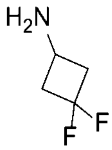 | 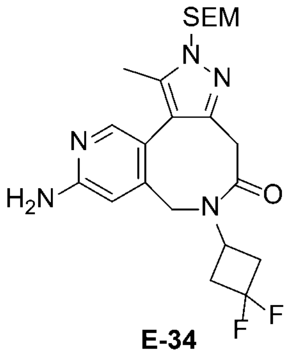 E-34 | MS (ESI) m/z =464 $[M+1]^+$ |
| 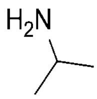 | 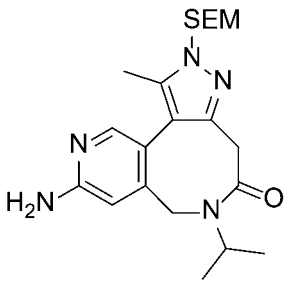 E-35 | MS (ESI) m/z =416 $[M+1]^+$ |

(continued)

| Amine | Structure of intermediate | $^1$HNMR and/or MS (ESI) |
|---|---|---|
| 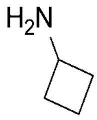 | 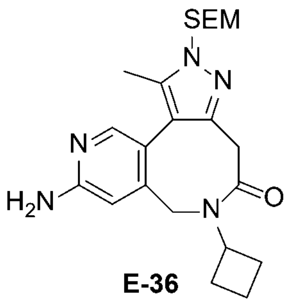 E-36 | MS (ESI) m/z =428 [M+1]$^+$ |

## Preparation of intermediates E-37 to E-42

The preparation of intermediates E-37 to E-42 was performed according to the synthesis method of intermediate E-16, in which intermediate E-1 was correspondingly replaced with a macrocyclic pyridine-amide from the following table, with all other reagents and procedures remaining identical.

| Macrocyclic pyridine-amide | Structure of intermediate | $^1$HNMR and/or MS (ESI) |
|---|---|---|
| 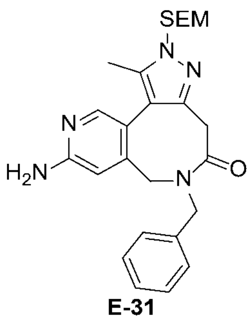 E-31 | 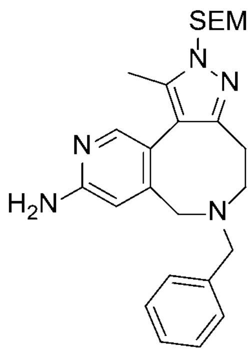 E-37 | MS (ESI) m/z =450 [M+1]$^+$ |
| 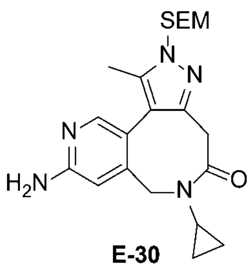 E-30 | 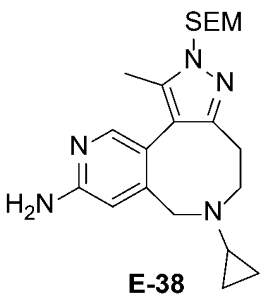 E-38 | MS (ESI) m/z =400 [M+1]$^+$ |
| 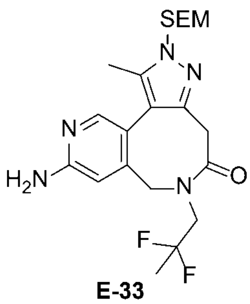 E-33 | 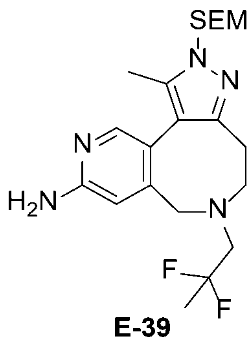 E-39 | MS (ESI) m/z =438 [M+1]$^+$ |

(continued)

| Macrocyclic pyridine-amide | Structure of intermediate | $^1$HNMR and/or MS (ESI) |
|---|---|---|
| 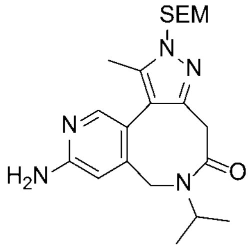 E-35 | 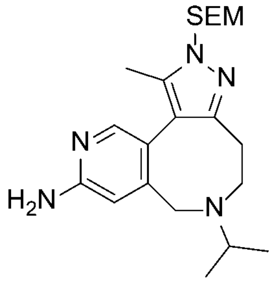 E-40 | MS (ESI) m/z =402 [M+1]$^+$ |
| 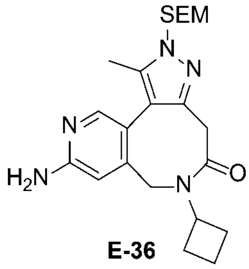 E-36 | 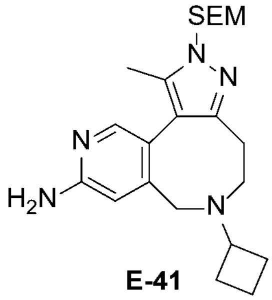 E-41 | MS (ESI) m/z =414 [M+1]$^+$ |
| 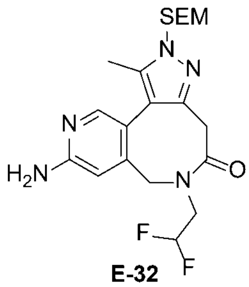 E-32 | 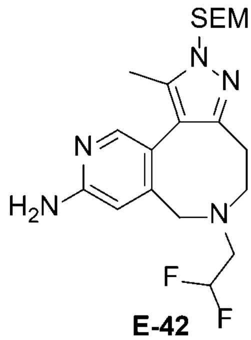 E-42 | MS (ESI) m/z =424 [M+1]$^+$ |

__Preparation of intermediate E-43__

A preparation of intermediate E-43 is illustrated as follows:

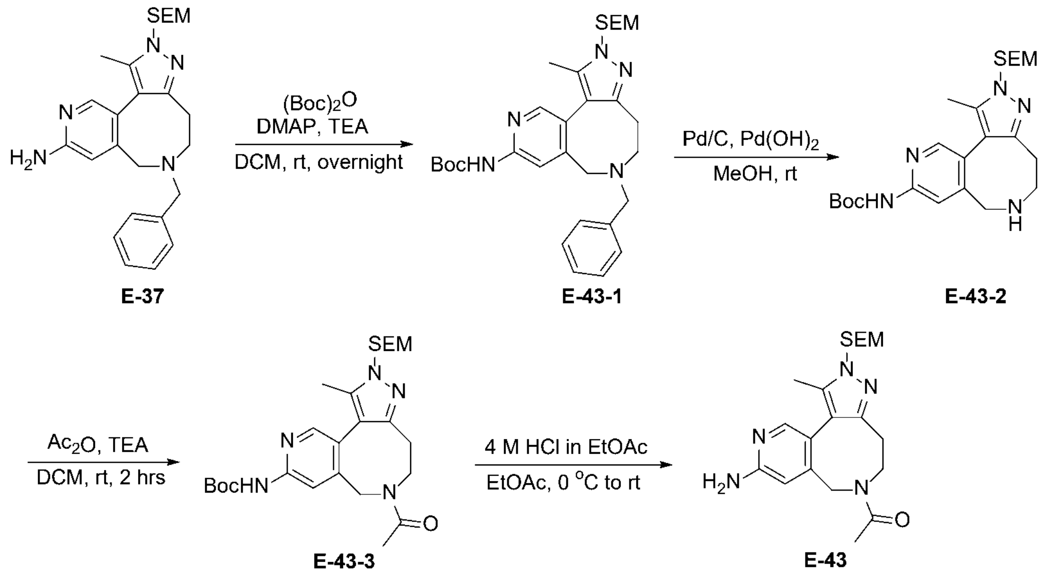


E-37 E-43-1 E-43-2

E-43-3 E-43

(S1) Preparation of intermediate E-43-1

The preparation of intermediate E-43-1 was performed according to the synthesis method of intermediate E-1, in which intermediate B-3-2 in step (S1) was replaced with intermediate E-37, with all other reagents and procedures remaining identical. MS (ESI) m/z = 550 $[M+1]^+$.

(S2) Preparation of intermediate E-43-2

To a single-neck flask were added intermediate E-43-1 (500 mg, 0.91 mmol), methanol (9 mL), Pd/C (150 mg, w/w 30%) and $Pd(OH)_2$ (150 mg, w/w 30%) at room temperature. The reaction mixture was evacuated and purged with hydrogen three times, and stirred at room temperature for 6 h. Upon completion, the reaction mixture was filtered through Celite and washed with methanol. The filtrate was concentrated under reduced pressure to dryness to obtain 384.59 mg (0.84 mmol) of intermediate E-43-2 (yield: 92%). MS (ESI) m/z = 460 $[M+1]^+$.

(S3) Preparation of intermediate E-43-3

To a single-neck flask were added intermediate E-43-2 (384.59 mg, 0.84 mmol), DCM (8 mL), triethylamine (93.32 mg, 0.92 mmol) and acetic anhydride (85.68 mg, 0.84 mmol) at room temperature. The reaction mixture was stirred at room temperature for 2 h. Upon completion, the reaction mixture was concentrated under reduced pressure to dryness. The residue was purified by reverse phase MPLC to obtain 331.71 mg (0.66 mmol) of intermediate E-43-3 (yield: 78.82%). MS (ESI) m/z = 502 $[M+1]^+$.

(S4) Preparation of intermediate E-43

The preparation of intermediate E-43 was performed according to the synthesis method of intermediate E-1, in which intermediate B-1-6 in step (S7) was replaced with intermediate E-43-3, with all other reagents and procedures remaining identical. MS (ESI) m/z = 402 $[M+1]^+$.

**Preparation of intermediate E-44**

A structure of intermediate E-44 is as follows:

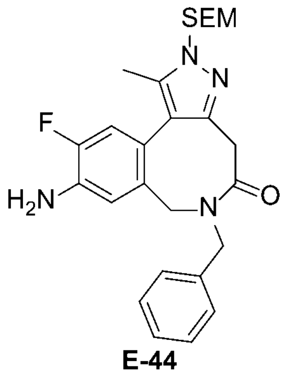

**E-44**

.

The preparation of intermediate E-44 was performed according to the synthesis method of intermediate E-1, in which methylamine hydrochloride in step (S4) was replaced with benzylamine, with all other reagents and procedures remaining identical. MS (ESI) m/z = 481 [M+1]$^+$.

**Preparation of intermediate E-45**

A structure of intermediate E-45 is as follows:

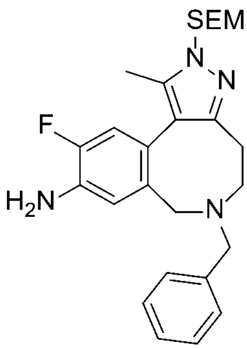

**E-45**

.

The preparation of intermediate E-45 was performed according to the synthesis method of intermediate E-16, in which intermediate E-1 was replaced with intermediate E-44, with all other reagents and procedures remaining identical. MS (ESI) m/z = 467 [M+1]$^+$.

**Preparation of intermediate E-46**

A preparation of intermediate E-46 is illustrated as follows:

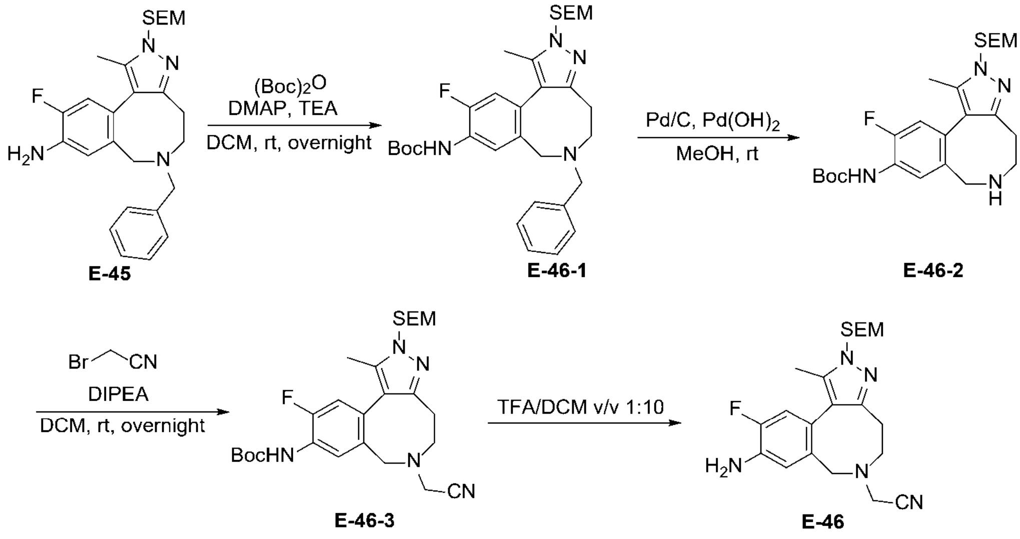

E-45 E-46-1 E-46-2 E-46-3 E-46

(S1-S2) Preparation of intermediate E-46-2

The preparation of intermediate E-46-2 was performed according to the synthesis method of intermediate E-43, in which intermediate E-37 was replaced with intermediate E-45, with all other reagents and procedures remaining identical. MS (ESI) m/z = 477 $[M+1]^+$.

(S3) Preparation of intermediate E-46-3

To a single-neck flask were added intermediate E-46-2 (400 mg, 0.84 mmol), DCM (8 mL), DIPEA (271 mg, 2.10 mmol) and bromoacetonitrile (109.96 mg, 0.92 mmol) at room temperature. The reaction mixture was stirred at room temperature overnight. Upon completion, the reaction mixture was concentrated under reduced pressure to dryness. The residue was purified by reverse phase MPLC to obtain 297.15 mg (0.58 mmol) of intermediate E-46-3 (yield: 68.69%). MS (ESI) m/z = 516 $[M+1]^+$.

(S4) Preparation of intermediate E-46

To a single-neck flask were added intermediate E-46-3 (297.15 mg, 0.58 mmol) and DCM (5 mL). TFA (0.5 mL) was added to the mixture solution under an ice-water bath. The reaction mixture was reacted at room temperature for 3 h. Upon completion, the reaction mixture was decanted into an aqueous sodium bicarbonate solution and extracted with DCM. The DCM layer was collected, dried over anhydrous sodium sulfate and filtered. The filtrate was concentrated under reduced pressure to dryness. The residue was purified by reverse phase MPLC to afford intermediate E-46. MS (ESI) m/z = 416 $[M+1]^+$.

**Preparation of intermediate E-47**

A preparation of intermediate E-47 is illustrated as follows:

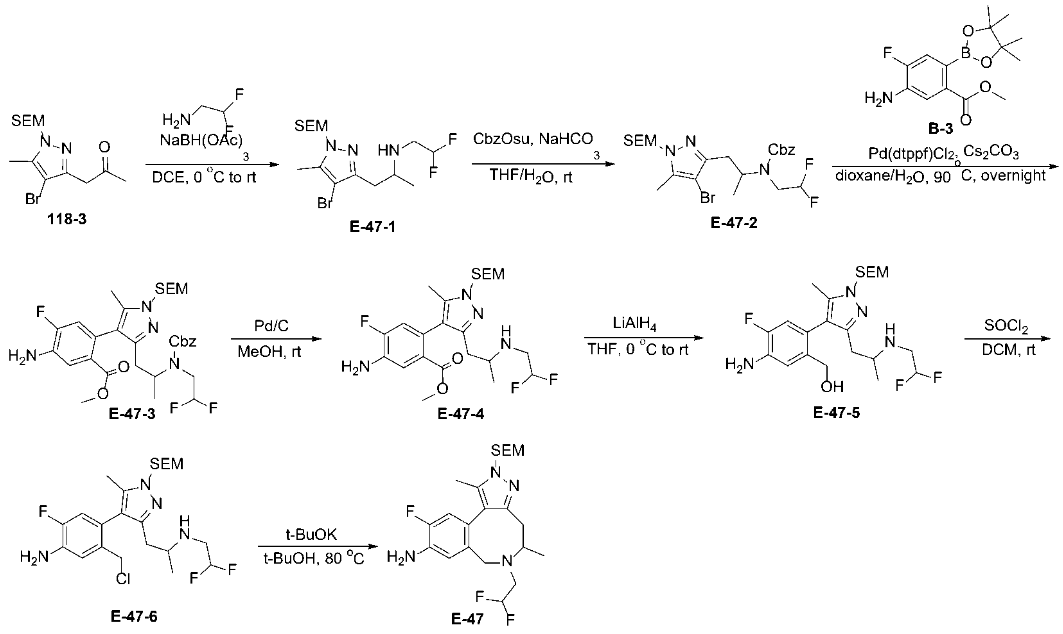


(S1) Preparation of intermediate E-47-1

To a single-neck flask were added intermediate 118-3 (1.0 g, 2.89 mmol), DCE (20 mL) and 2,2-Difluoroethylamine (0.35 g, 4.34 mmol). The reaction mixture was stirred at 40°C overnight, cooled to room temperature, and batchwise added with sodium triacetoxyborohydride (1.53 g, 7.23 mmol). The reaction mixture was then stirred at room temperature overnight. The reaction mixture was quenched with water and extracted with DCM. The DCM layer was collected, dried over anhydrous sodium sulfate and filtered. The filtrate was concentrated under reduced pressure to dryness. The residue was purified by reverse phase MPLC to obtain 800.33 mg (1.95 mmol) of intermediate E-47-1 (yield: 67.38%). MS (ESI) m/z = 412 $[M+1]^+$.

(S2) Preparation of intermediate E-47-2

To a single-neck flask were added intermediate E-47-1 (3 g, 4.36 mmol), THF/$H_2O$ (20 mL/20 mL), sodium bicarbonate (733.35 mg, 8.73 mmol) and CbzOSu (1.09 g, 4.36 mmol). The reaction mixture was stirred at room temperature overnight. The reaction mixture was diluted with water and extracted with EA. The EA layer was collected, dried over anhydrous sodium sulfate and filtered. The filtrate was concentrated under reduced pressure to dryness. The residue was purified by silica gel column chromatography to obtain 0.93 g (1.70 mmol) of intermediate E-47-2 (yield: 38.99%). MS (ESI) m/z = 546 $[M+1]^+$.

(S3) Preparation of intermediate E-47-3

To a single-neck flask were added intermediate E-47-2 (900 mg, 1.65 mmol), intermediate B-3 (487.16 mg, 1.65 mmol), $Cs_2CO_3$ (1.08 g, 3.30 mmol), 1,4-dioxane/water (15 mL/3 mL), Pd(dppf)$Cl_2$ (120.88 mg, 165.14 $\mu$mol). The reaction mixture was evacuated and purged with nitrogen three times, and stirred at 90°C overnight. Upon completion, the reaction mixture was cooled and extracted with water and EA. The EA layer was collected, washed with a NaCl solution, dried over anhydrous sodium sulfate, and filtered. The filtrate was concentrated under reduced pressure to dryness. The residue was purified by silica gel column chromatography to obtain 738.74 mg (1.17 mmol) of intermediate E-47-3 (yield: 70.56%). MS (ESI) m/z = 635 $[M+1]^+$.

(S4) Preparation of intermediate E-47-4

To a single-neck flask were added intermediate E-47-3 (738.74 mg, 1.17 mmol), methanol (10 mL) and Pd/C (221 mg, w/w 30%) at room temperature. The reaction mixture was evacuated and purged with hydrogen three times, and stirred at room temperature for 4 h. Upon completion, the reaction mixture was filtered through Celite and washed with methanol. The filtrate was concentrated under reduced pressure to dryness to obtain 530.2 mg (1.06 mmol) of intermediate E-47-4 (yield: 91%). MS (ESI) m/z = 501 $[M+1]^+$.

(S5-S7) Preparation of intermediate E-47

The preparation of intermediate E-47 was performed according to steps (S2-S4) in Example 1, in which intermediate 1-1 in step (S2) was replaced with intermediate E-47-4, with all other reagents and procedures remaining identical. MS (ESI) m/z = 455 $[M+1]^+$.

**Preparation of intermediate E-48**

A structure of intermediate E-48 is as follows:

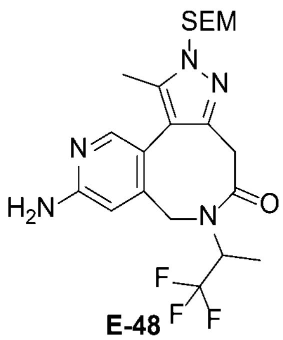

**E-48**

.

The preparation of intermediate E-48 was performed according to the synthesis method of intermediate E-30, in which cyclopropylamine in step (S4) was replaced with 1-(trifluoromethyl)ethylamine, with all other reagents and procedures remaining identical. MS (ESI) m/z = 470 $[M+1]^+$.

**Preparation of intermediate E-49**

A structure of intermediate E-49 is as follows:

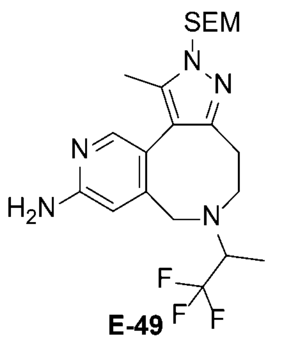

**E-49**

.

The preparation of intermediate E-49 was performed according to the synthesis method of intermediate E-16, in which intermediate E-1 was replaced with intermediate E-48, with all other reagents and procedures remaining identical. MS (ESI) m/z = 456 $[M+1]^+$.

**Preparation of intermediate E-50**

A structure of intermediate E-50 is as follows:

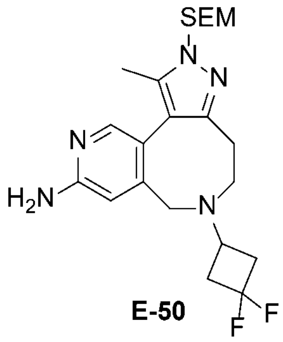

E-50

.

The preparation of intermediate E-50 was performed according to the synthesis method of intermediate E-16, in which intermediate E-1 was replaced with intermediate E-34, with all other reagents and procedures remaining identical. MS (ESI) m/z = 450 $[M+1]^+$.

**Preparation of intermediate E-51**

A structure of intermediate E-51 is as follows:

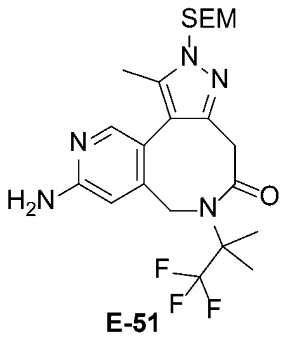

E-51

.

The preparation of intermediate E-51 was performed according to the synthesis method of intermediate E-30, in which cyclopropylamine in step (S4) was replaced with 2,2,2-trifluoro-1,1-dimethylethylamine, with all other reagents and procedures remaining identical. MS (ESI) m/z = 484 $[M+1]^+$.

**Preparation of intermediate E-52**

A structure of intermediate E-52 is as follows:

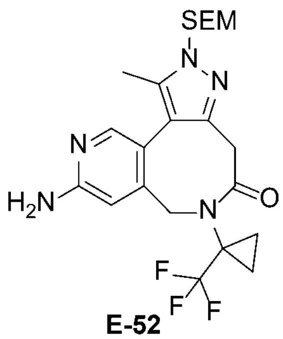

E-52

.

The preparation of intermediate E-51 was performed according to the synthesis method of intermediate E-30, in which cyclopropylamine in step (S4) was replaced with 1-(trifluoromethyl)cyclopropylamine, with all other reagents and procedures remaining identical. MS (ESI) m/z = 482 $[M+1]^+$.

**Preparation of intermediate E-53**

A structure of intermediate E-53 is as follows:

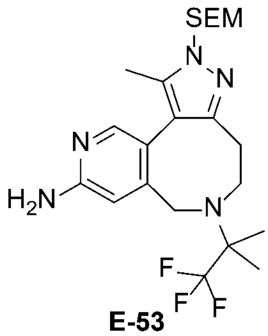

**E-53**

.

The preparation of intermediate E-53 was performed according to the synthesis method of intermediate E-16, in which intermediate E-1 was replaced with intermediate E-51, with all other reagents and procedures remaining identical. MS (ESI) m/z = 470 $[M+1]^+$.

**Preparation of intermediate E-54**

A structure of intermediate E-54 is as follows:

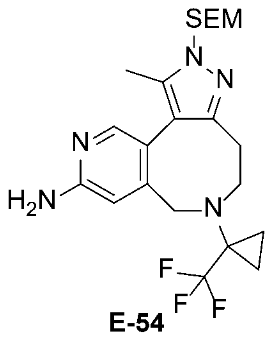

**E-54**

.

The preparation of intermediate E-54 was performed according to the synthesis method of intermediate E-16, in which intermediate E-1 was replaced with intermediate E-52, with all other reagents and procedures remaining identical. MS (ESI) m/z = 468 $[M+1]^+$.

**Preparation of intermediate E-55**

A structure of intermediate E-55 is as follows:

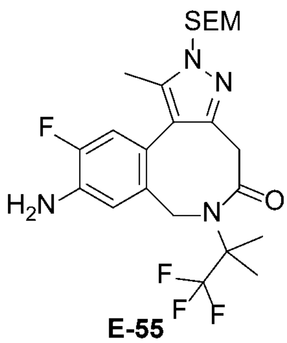

**E-55**

The preparation of intermediate E-55 was performed according to the synthesis method of intermediate E-1, in

which methylamine hydrochloride in step (S4) was replaced with 2,2,2-trifluoro-1,1-dimethylethylamine, with all other reagents and procedures remaining identical. MS (ESI) m/z = 501 [M+1]$^+$.

**Preparation of intermediate E-56**

A structure of intermediate E-56 is as follows:

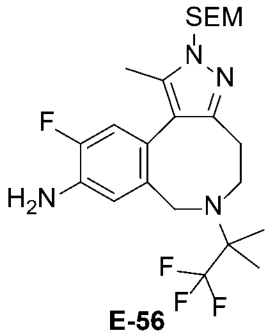

**E-56**

.

The preparation of intermediate E-56 was performed according to the synthesis method of intermediate E-16, in which intermediate E-1 was replaced with intermediate E-55, with all other reagents and procedures remaining identical. MS (ESI) m/z = 487 [M+1]$^+$.

**Preparation of intermediate E-57**

A structure of intermediate E-57 is as follows:

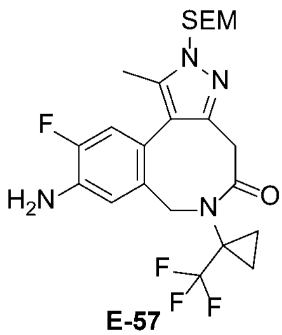

**E-57**

.

The preparation of intermediate E-57 was performed according to the synthesis method of intermediate E-1, in which methylamine hydrochloride in step (S4) was replaced with 1-(trifluoromethyl)cyclopropylamine, with all other reagents and procedures remaining identical. MS (ESI) m/z = 499 [M+1]$^+$.

**Preparation of intermediate E-58**

A structure of intermediate E-58 is as follows:

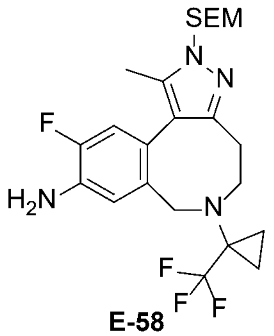

E-58

The preparation of intermediate E-58 was performed according to the synthesis method of intermediate E-16, in which intermediate E-1 was replaced with intermediate E-57, with all other reagents and procedures remaining identical. MS (ESI) m/z = 485 $[M+1]^+$.

## Example 1 Preparation of compound 1

A preparation of compound 1 is illustrated as follows:

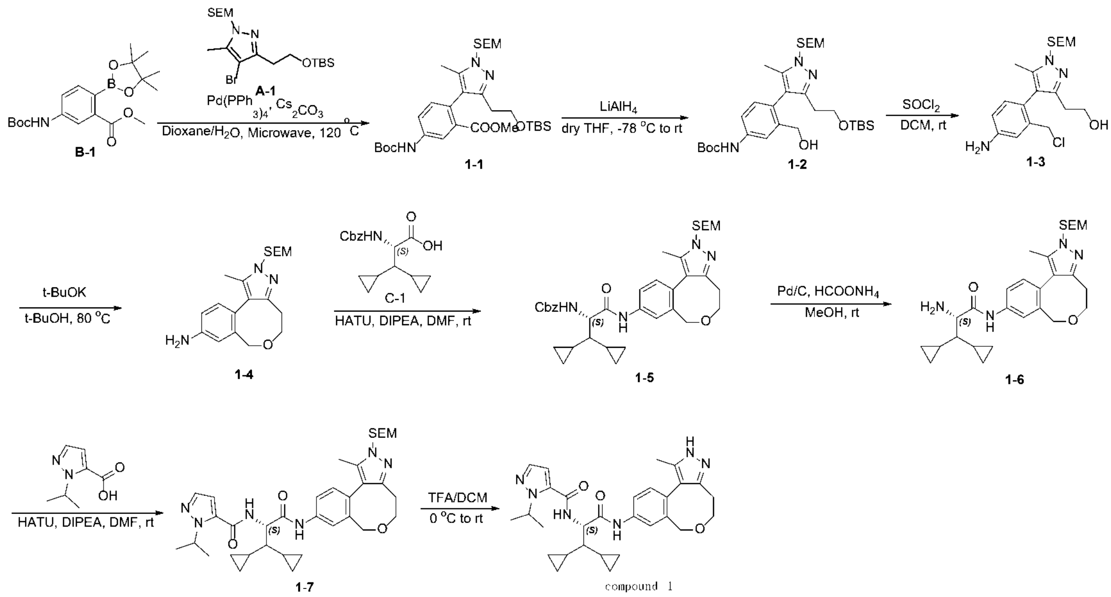


B-1, 1-1, 1-2, 1-3, 1-4, 1-5, 1-6, 1-7, compound 1

### (S1) Preparation of intermediate 1-1

To a solution of intermediate B-1 (4 g, 8.90 mmol), intermediate A-1 (4.03 g, 10.68 mmol), $Cs_2CO_3$ (5.80 g, 17.79 mmol) in 1,4-dioxane (40 mL)/water (8 mL) was $Pd(PPh_3)_4$ (1.03 g, 889.73 μmol). The reaction mixture was bubbled with nitrogen to remove oxygen and reacted in a microwave reactor at 120°C for 1 h. Upon completion, the reaction mixture was extracted with water and EA. The EA layer was collected, washed with a NaCl solution and concentrated to dryness. The residue was purified by silica gel column chromatography to obtain 2.5 g of intermediate 1-1 (yield: 45%). MS (ESI) m/z = 620 $[M+1]^+$.

### (S2) Preparation of intermediate 1-2

To a solution of intermediate 1-1 (2.5 g, 4.03 mmol) in dry THF (50 mL) was added dropwise $LiAlH_4$ (382.60 mg, 10.08 mmol, 1M in THF) at -78°C. The reaction mixture was warmed to room temperature and reacted for 2 h. Upon completion, the reaction mixture was quenched at -10°C by sequential addition of ice water (0.7 mL), 15% aqueous NaOH solution (0.7 mL), and water (2.1 mL). The mixture was filtered, and the filter cake was washed with EA. The filtrate was concentrated to dryness to obtain 1.5 g of intermediate 1-2 (yield: 63%), which was used directly in the next step without further purification. MS (ESI) m/z = 592 $[M+1]^+$.

(S3) Preparation of intermediate 1-3

To a solution of intermediate 1-2 (1.5 g, 2.53 mmol) in DCM (60 mL) was added dropwise thionyl chloride (2.7 g, 15.17 mmol) at 0°C. The reaction mixture was reacted for 1 h. Upon completion, the reaction mixture was quenched with aqueous sodium bicarbonate solution and extracted with EA. The EA layer was collected, washed with a NaCl solution and concentrated to dryness to obtain 189 mg of intermediate 1-3 (yield: 19%), which was used directly in the next step without further purification. MS (ESI) m/z = 396 $[M+1]^+$.

(S4) Preparation of intermediate 1-4

To a solution of intermediate 1-3 (189 mg, 477.28 μmol) in t-BuOH (4 mL) was added t-BuOK (58.91 mg, 525.00 μmol). The reaction mixture was heated to 80°C and reacted for 1 h. Upon completion, the reaction mixture was concentrated to dryness, extracted with water and EA. The EA layer was collected, washed with a NaCl solution and concentrated to dryness to obtain 159 mg of intermediate 1-4 (yield: 93%), which was used directly in the next step without further purification. MS (ESI) m/z = 360 $[M+1]^+$.

(S5) Preparation of intermediate 1-5

To a solution of intermediate C-1 (125 mg, 412.06 μmol) and DIPEA (176.86 mg, 1.37 mmol, 238.35 μL) in DMF (4 mL) was added HATU (169 mg, 444.47 μmol). The reaction mixture was stirred at room temperature for 5 min, and was added with intermediate 1-4 (123 mg, 342.11 μmol). The reaction mixture was stirred at room temperature for 3 h. Upon completion, the reaction mixture was extracted with water and EA. The EA layer was collected, washed with a NaCl solution and concentrated to dryness. The residue was purified by silica gel column chromatography to obtain 215 mg of intermediate 1-5 (yield: 97%). MS (ESI) m/z = 645 $[M+1]^+$.

(S6) Preparation of intermediate 1-6

To a solution of intermediate 1-5 (51 mg, 79.09 μmol) and ammonium formate (49.87 mg, 790.85 μmol) in MeOH (1 mL) was added Pd/C (20 mg, w/w 10%). The reaction mixture was stirred at room temperature for 1 h. The reaction mixture was filtered through Celite and washed with methanol. The filtrate was concentrated under reduced pressure to dryness. The residue was dissolved in EA, and washed with an aqueous sodium bicarbonate solution. The EA layer was collected, washed with a NaCl solution, dried over anhydrous sodium sulfate, and filtered. The filtrate was concentrated under reduced pressure to dryness to obtain 40 mg of intermediate 1-6 (yield: 99%), which was used directly in the next step without further purification. MS (ESI) m/z = 511 $[M+1]^+$.

(S7) Preparation of intermediate 1-7

To a solution of 1-isopropyl-1H-pyrazole-5-carboxylic acid (16 mg, 103.78 μmol) and DIPEA (40.49 mg, 313.27 μmol, 54.56 μL) in DMF (1 mL) was added HATU (42 mg, 110.46 μmol). The reaction mixture was stirred at room temperature for 5 min, and was added with intermediate 1-6 (40 mg, 78.32 μmol). The reaction mixture was stirred at room temperature for 1 h. Upon completion, the reaction mixture was extracted with water and EA. The EA layer was collected, washed with a NaCl solution and concentrated to dryness. The residue was purified by MPLC to obtain 50 mg of intermediate 1-7 (yield: 99%). MS (ESI) m/z = 647 $[M+1]^+$.

(S8) Preparation of compound 1

To a solution of intermediate 1-7 (50 mg, 77.29 μmol) in DCM (0.8 mL) was added TFA (0.8 mL). The reaction mixture was reacted at room temperature for 3 h. Upon completion, the mixture was concentrated to dryness and purified by preparative MPLC to obtain 15 mg of compound 1 (yield: 38%). MS (ESI) m/z = 517 $[M+1]^+$. $^1$H NMR (400 MHz, Methanol-$d_4$) δ 7.72 - 7.64 (m, 2H), 7.52 (d, *J* = 2.0 Hz, 1H), 7.41 - 7.27 (m, 1H), 6.80 (d, *J* = 2.1 Hz, 1H), 5.41 (sept, *J* = 6.7 Hz, 1H), 4.91 (d, *J* = 7.5 Hz, 1H), 4.33 (m, 2H), 3.72 (s, 2H), 2.69 (s, 2H), 2.26 (s, 3H), 1.45 (dd, *J* = 6.7, 5.5 Hz, 6H), 0.97 - 0.73 (m, 3H), 0.61 - 0.21 (m, 8H).

**Examples 2-7 Preparation of compounds 2-7**

The preparation of compounds 2 to 7 was performed according to the synthesis method in Example 1, in which 1-isopropyl-1H-pyrazole-5-carboxylic acid was correspondingly replaced with a carboxylic acid intermediate from the following table, with all other reagents and procedures remaining identical.

| Carboxylic acid intermediate | Structure of compound | $^1$HNMR and/or MS (ESI) |
|---|---|---|
| 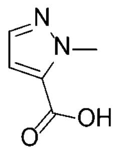 | 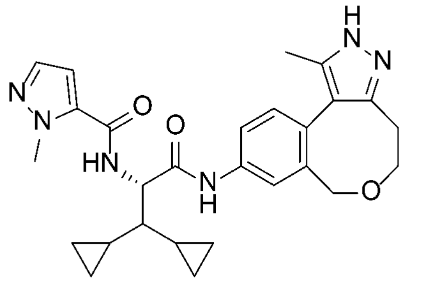 compound 2 | MS (ESI) m/z =489 $[M+1]^+$ $^1$H NMR (400 MHz, Methanol-$d_4$) δ 7.73 - 7.64 (m, 2H), 7.49 (d, *J* = 2.2 Hz, 1H), 7.35 (d, *J* = 8.2 Hz, 1H), 6.89 (d, *J* = 2.2 Hz, 1H), 4.93 (d, *J* = 7.3 Hz, 1H), 4.33 (m ,2H), 4.11 (s, 3H), 3.74 (s, 2H), 2.70 (s, 2H), 2.27 (s, 3H), 0.98 - 0.76 (m, 3H), 0.63 - 0.21 (m, 8H). |
| 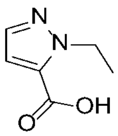 | 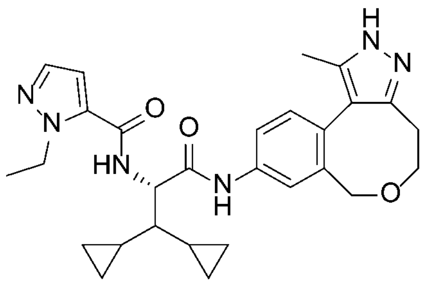 compound 3 | MS (ESI) m/z =503 $[M+1]^+$ $^1$H NMR (400 MHz, Methanol-$d_4$) δ 7.70 (d, *J* = 10.6 Hz, 2H), 7.53 (s, 1H), 7.36 (d, *J* = 8.2 Hz, 1H), 6.89 (s, 1H), 4.94 (d, *J* = 7.6 Hz, 1H), 4.56 (q, *J* = 7.3 Hz, 2H), 4.35 (s, 2H), 3.75 (s, 2H), 2.71 (s, 2H), 2.28 (s, 3H), 1.41 (t, *J* = 7.2 Hz, 3H), 0.99 - 0.75 (m, 3H) 0.64 - 0.21 (m, 8H). |
| 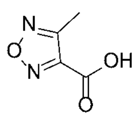 | 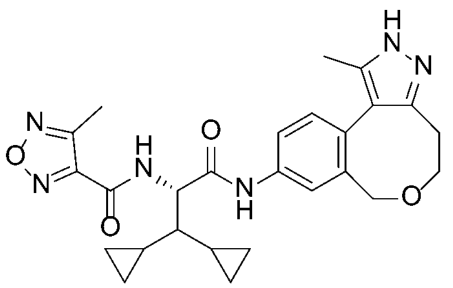 compound 4 | MS (ESI) m/z =491 $[M+1]^+$ $^1$H NMR (400 MHz, Methanol-$d_4$) δ 7.63-7.54 (m, 2H), 7.24 (s, 1H), 4.86 (d, *J* = 6.5 Hz, 1H), 4.23 (s, 2H), 3.63 (s, 2H), 2.59 (s, 2H), 2.46 (s, 3H), 2.16 (s, 3H), 0.86 - 0.65 (m, 3H), 0.53 - 0.11 (m, 8H). |
| 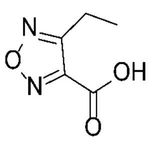 | 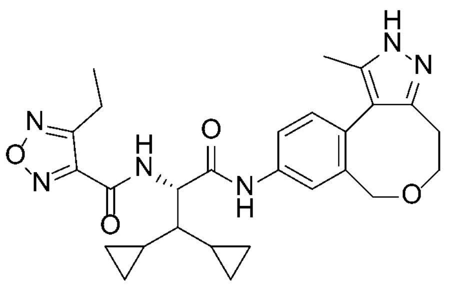 compound 5 | MS (ESI) m/z =505 $[M+1]^+$ $^1$H NMR (400 MHz, Methanol-$d_4$) δ 7.74 - 7.65 (m, 2H), 7.36 (s, 1H), 4.98 (d, *J* = 6.6 Hz, 1H), 4.35 (s, 2H), 3.75 (s, 2H), 3.03 (q, *J* = 7.5 Hz, 2H), 2.71 (s, 2H), 2.28 (s, 3H), 1.36 (t, *J* = 7.5 Hz, 3H), 0.99 - 0.77 (m, 3H), 0.65 - 0.23 (m, 8H). |
| 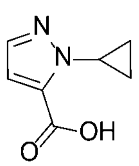 | 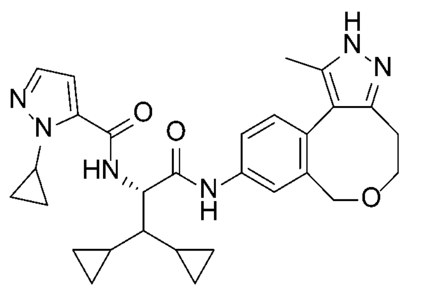 compound 6 | MS (ESI) m/z =515 $[M+1]^+$ $^1$H NMR (400 MHz, Methanol-$d_4$) δ 7.72 - 7.65 (m, 2H), 7.42 (d, *J* = 2.0 Hz, 1H), 7.34 (d, *J* = 8.2 Hz, 1H), 6.79 (d, *J* = 2.0 Hz, 1H), 4.94 (d, *J* = 7.3 Hz, 1H), 4.33 (s, 2H), 4.16 (tt, *J* = 7.5, 3.9 Hz, 1H), 3.72 (s, 2H), 2.69 (s, 2H), 2.26 (s, 3H), 1.16 - 1.10 (m, 2H), 1.01 (tt, *J* = 5.6, 2.3 Hz, 2H), 0.95 - 0.75 (m, 3H), 0.61 - 0.52 (m, 1H), 0.53 - 0.45 (m, 2H), 0.45 - 0.34 (m, 2H), 0.29 (dtd, *J* = 9.8, 7.1, 5.9, 3.1 Hz, 3H). |

(continued)

| Carboxylic acid intermediate | Structure of compound | $^1$HNMR and/or MS (ESI) |
|---|---|---|
| 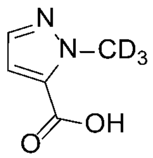 | 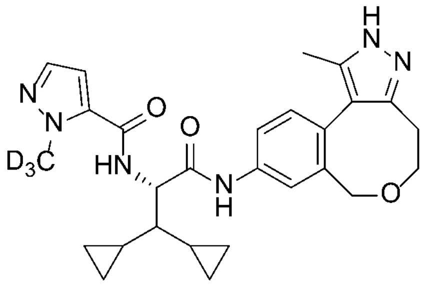 compound 7 | MS (ESI) m/z =492 [M+1]$^+$ $^1$H NMR (600 MHz, Methanol-$d_4$) δ 7.79 - 7.68 (m, 2H), 7.50 (d, *J* = 2.2 Hz, 1H), 7.38 (d, *J* = 8.3 Hz, 1H), 6.90 (d, *J* = 2.1 Hz, 1H), 4.92 (d, *J* = 7.6 Hz, 1H), 4.71 - 4.06 (m, 2H), 3.69 (d, *J* = 33.4 Hz, 2H), 2.74 (s, 2H), 2.32 (s, 3H), 0.95 - 0.83 (m, 3H), 0.60 - 0.37 (m, 5H), 0.29 (ddt, *J* = 18.0, 9.4, 4.9 Hz, 3H). |

## Examples 8-10 Preparation of compounds 8-10

The preparation of compounds 8 to 10 was performed according to the synthesis method in Example 1, in which intermediate C-1 in step (S5) was correspondingly replaced with an amino acid from the following table, and 1-isopropyl-1H-pyrazole-5-carboxylic acid in step (S7) was replaced with 1-ethyl-1H-pyrazole-5-carboxylic acid, with all other reagents and procedures remaining identical.

| Amino acid | Structure of compound | $^1$HNMR and/or MS (ESI) |
|---|---|---|
| 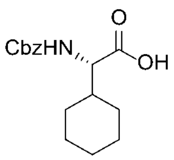 | 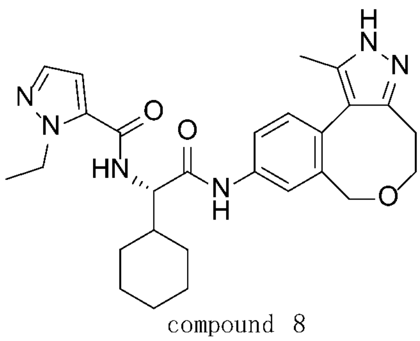 compound 8 | MS (ESI) m/z =491 [M+1]$^+$ $^1$H NMR (400 MHz, Methanol-$d_4$) δ 7.64 - 7.53 (m, 2H), 7.38 (d, *J* = 2.1 Hz, 1H), 7.22 (dd, *J* = 8.4, 2.0 Hz, 1H), 6.78 (d, *J* = 2.1 Hz, 1H), 4.51 - 4.34 (m, 3H), 4.22 (s, 2H), 3.62 (s, 2H), 2.58 (s, 2H), 2.15 (s, 3H), 1.90 - 1.78 (m, 2H), 1.76 - 1.56 (m, 4H), 1.31 - 0.98 (m, 8H) |
| 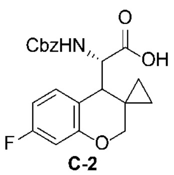 C-2 | 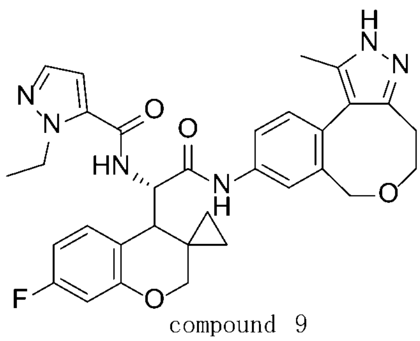 compound 9 | MS (ESI) m/z =585 [M+1]$^+$ $^1$H NMR (400 MHz, Methanol-$d_4$) δ 7.65 (d, *J* = 2.3 Hz, 1H), 7.61 (dd,*J* = 8.3, 2.3 Hz, 1H), 7.37 (d,*J* = 2.1 Hz, 1H), 7.26 (s, 1H), 7.00 (dd, *J* = 8.6, 6.6 Hz, 1H), 6.69 (d, *J* = 2.1 Hz, 1H), 6.44 (dd, *J* = 10.5, 2.6 Hz, 1H), 6.34 (td, *J* = 8.4, 2.7 Hz, 1H), 5.01 (d,*J* = 9.9 Hz, 1H), 4.89 (dd, *J* = 11.5, 2.0 Hz, 1H), 4.35 - 4.10 (m, 4H), 3.64 (s, 2H), 3.30 (dd, *J* = 11.5, 1.8 Hz, 1H), 2.60 (s, 2H), 2.50 (d, *J* = 9.9 Hz, 1H), 2.17 (s, 3H), 1.13 (t, *J* = 7.2 Hz, 3H), 0.85 (dt, *J* = 9.2, 5.0 Hz, 1H), 0.61 - 0.47 (m, 2H), 0.32 (dd, *J* = 5.6, 3.7 Hz, 1H). |

(continued)

| Amino acid | Structure of compound | $^1$HNMR and/or MS (ESI) |
|---|---|---|
| 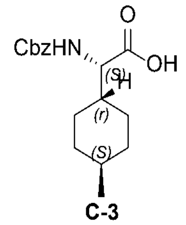 C-3 | 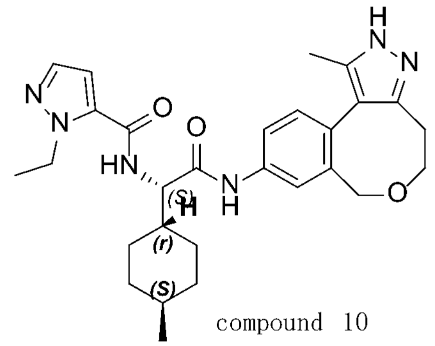 compound 10 | MS (ESI) m/z =505 [M+1]$^+$ $^1$H NMR (400 MHz, Methanol-$d_4$) δ 7.62 - 7.54 (m, 2H), 7.39 (d, *J* = 2.1 Hz, 1H), 7.23 (d, *J* = 8.3 Hz, 1H), 6.78 (d, *J* = 2.1 Hz, 1H), 4.42 (qd, *J* = 7.1, 2.3 Hz, 2H), 4.37 (d, *J* = 8.5 Hz, 1H), 4.22 (s, 2H), 3.62 (s, 2H), 2.58 (s, 2H), 2.16 (s, 3H), 1.90 - 1.62 (m, 5H), 1.35 - 1.01 (m, 6H), 0.96 - 0.75 (m, 5H). |

**Example 11 Preparation of compounds 11a and 11b**

A preparation of compounds 11a and 11b is illustrated as follows:

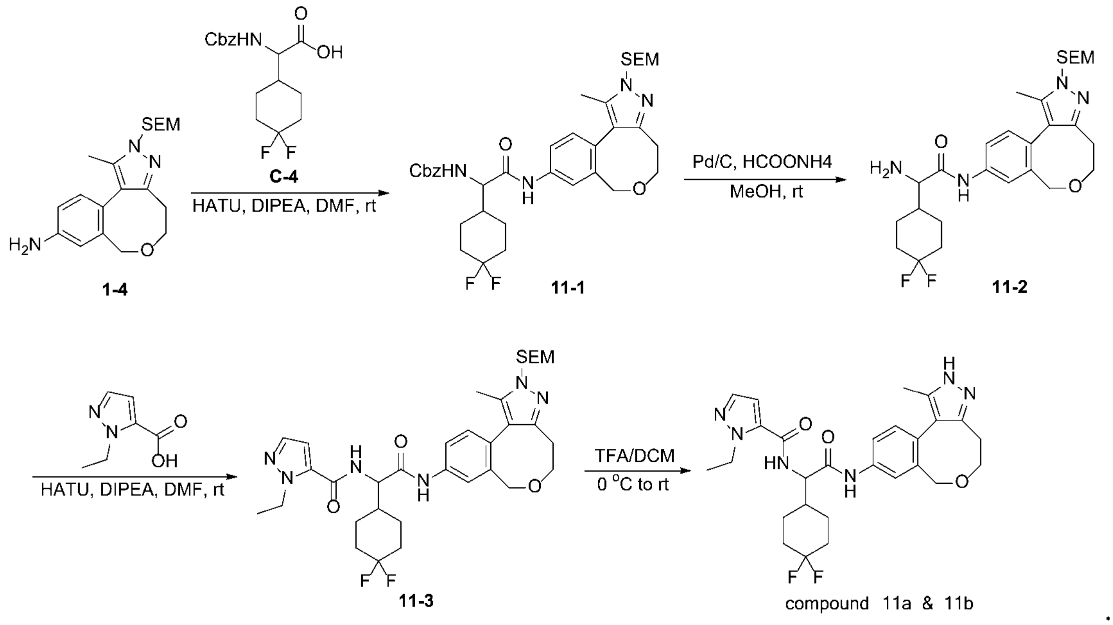


The preparation of compounds 11a and 11b was performed according to the synthesis method of compound 1, in which intermediate C-1 in step (S5) was replaced with intermediate C-4, and 1-isopropyl-1H-pyrazole-5-carboxylic acid in step (S7) was replaced with 1-ethyl-1H-pyrazole-5-carboxylic acid, with all other reagents and procedures remaining identical. After SEM group was removed, the crude product was subjected to purification by MPLC ($H_2O/CH_3CN$, 0.05% $NH_4HCO_3$) and separation by SFC to obtain compounds 11a and 11b, in which SFC was performed using a Daicel CHIRALPAK AD-3 column (3 μm, 150 mm × 3 mm) with $CO_2$ (as mobile phase A) and ethanol (as mobile phase B) (A/B = 70/30), at a flow rate of 1 mL/min and a column temperature of 40°C.

For compound 11a (P1, SFC $t_R$ = 3.054), MS (ESI) m/z = 527 [M+1]$^+$. $^1$H NMR (600 MHz, Methanol-$d_4$) δ 7.87 - 7.64 (m, 2H), 7.50 *(d, J* = 2.1 Hz, 1H), 7.36 (d, *J* = 8.3 Hz, 1H), 6.89 (d, *J* = 2.2 Hz, 1H), 4.59 - 4.48 (m, 3H), 4.38 (d, *J* = 57.2 Hz, 2H), 3.96 - 3.57 (m, 2H), 2.74 (t, *J* = 49.5 Hz, 2H), 2.30 (s, 3H), 2.14 - 2.02 (m, 3H), 2.02 - 1.95 (m, 1H), 1.91 - 1.74 (m, 3H), 1.63 - 1.44 (m, 2H), 1.37 (t, *J* = 7.2 Hz, 3H).

For compound 11b (P2, SFC $t_R$ = 4.824), MS (ESI) m/z = 527 [M+1]$^+$. $^1$H NMR (600 MHz, Methanol-$d_4$) δ 7.75 - 7.66 (m, 2H), 7.50 (d, *J* = 2.1 Hz, 1H), 7.35 (d, *J* = 8.3 Hz, 1H), 6.89 (d, *J* = 2.1 Hz, 1H), 4.59 - 4.48 (m, 3H), 4.34 (s, 2H), 3.90 - 3.57 (m, 2H), 2.89 - 2.54 (m, 2H), 2.29 (s, 3H), 2.10 (tt, *J* = 16.5, 10.3 Hz, 3H), 1.99 (d, *J* = 13.8 Hz, 1H), 1.80 (dt, *J*= 31.7, 13.3 Hz, 3H), 1.63 - 1.43 (m, 2H), 1.37 (t, *J* = 7.1 Hz, 3H).

Example 12 Preparation of compound 12

A preparation of compound 12 is illustrated as follows:

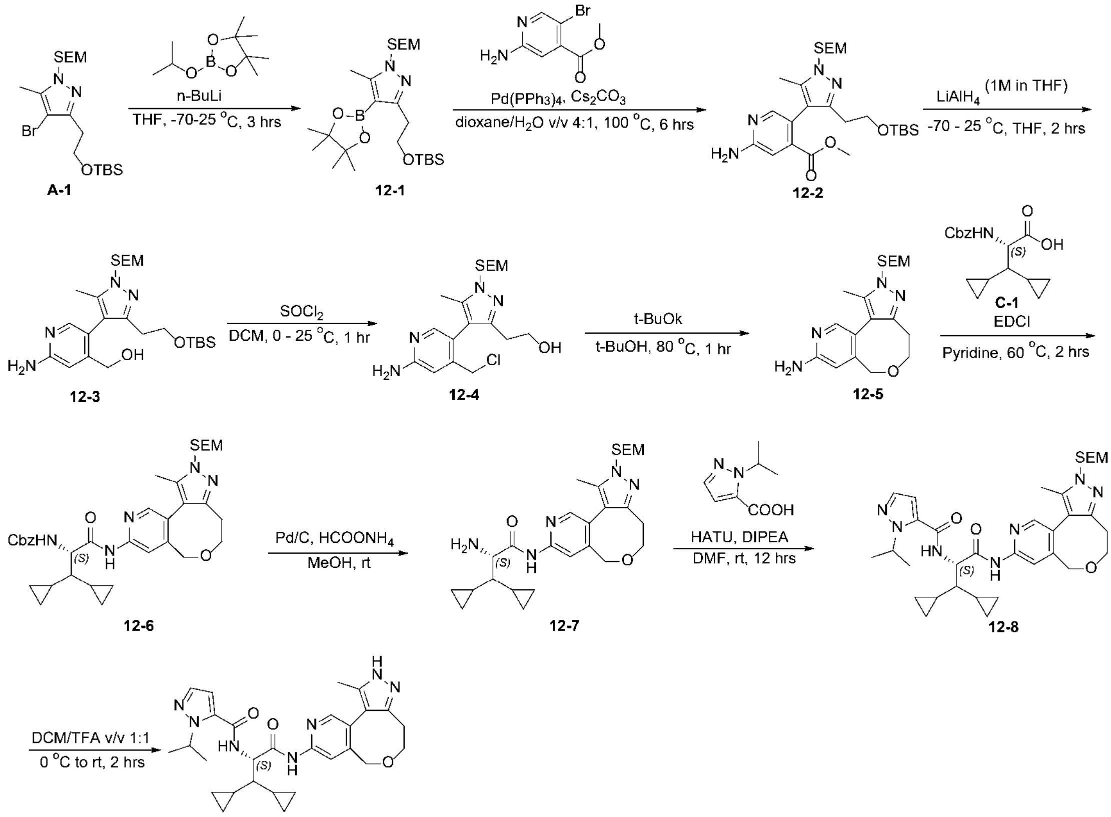


A-1, 12-1, 12-2, 12-3, 12-4, 12-5, 12-6, 12-7, 12-8

compound 12

(S1) Preparation of intermediate 12-1

To a 500 mL three-neck flask were added intermediate A-1 (11 g, 24.47 mmol) and dry THF (150 mL). The mixture was evacuated and purged with nitrogen three times, cooled to -70°C, and dropwise added with n-BuLi (24.47 mmol, 9.7 mL). The mixture was stirred at -70°C for 1 h. A solution of 2-isopropoxy-4,4,5,5-tetramethyl-1,3,2-dioxaborolane (9.10 g, 48.94 mmol) in dry THF (80 mL) was added dropwise into the mixture. The reaction mixture was warmed to room temperature and stirred for 1 h. Upon completion, the reaction mixture was decanted into saturated ammonium chloride solution to quench and extracted with EA. The EA layer was collected, washed with a NaCl solution, dried over anhydrous sodium sulfate, and filtered. The filtrate was concentrated under reduced pressure to dryness. The residue was purified by silica gel column chromatography to obtain 8.28 g (11.66 mmol) of intermediate 12-1 (yield: 47.67%). MS (ESI) m/z = 497 $[M+1]^+$.

(S2) Preparation of intermediate 12-2

To a mixture of intermediate 12-1 (5.00 g, 10.07 mmol), methyl 2-amino-5-bromoisonicotinate (1.94 g, 8.39 mmol) and $Cs_2CO_3$ (5.47 g, 16.78 mmol) in 1,4-dioxane (80 mL)/water (20 mL) was added $Pd(PPh_3)_4$ (484.82 mg, 419.40 μmol). After addition, the reaction mixture was evacuated and purged with nitrogen three times, and stirred at 100°C for 6 h. Upon completion, the reaction mixture was cooled and extracted with water and EA. The EA layer was collected, washed with a NaCl solution and concentrated to dryness. The residue was purified by silica gel column chromatography to obtain 1.40 g (1.88 mmol) of intermediate 12-2 (yield: 22.47%). MS (ESI) m/z = 521 $[M+1]^+$.

(S3-S5) Preparation of intermediate 12-5

The preparation of intermediate 12-5 was performed according to steps (S2-S4) of Example 1, in which intermediate 1-1 was replaced with intermediate 12-2, with all other reagents and procedures remaining identical. MS (ESI) m/z = 361 $[M+1]^+$.

(S6) Preparation of intermediate 12-6

To a solution of intermediate 12-5 (80 mg, 221.90 μmol) and intermediate C-1 (134.63 mg, 443.80 μmol) in pyridine (2 mL) was added EDCI (127.61 mg, 665.70 μmol). After addition, the reaction mixture was stirred at 60°C for 2 h. Upon completion, the reaction mixture was cooled and concentrated under reduced pressure to dryness. The residue was purified by MPLC ($H_2O/CH_3CN$, 0.05% TFA) to obtain 30 mg (46.45 μmol) of intermediate 12-6 (yield: 20.93%). MS (ESI) m/z = 646 $[M+1]^+$.

(S7-S9) Preparation of compound 12

The preparation of compound 12 was performed according to steps (S6-S8) of Example 1, in which intermediate 1-5 was replaced with intermediate 12-6, with all other reagents and procedures remaining identical. MS (ESI) m/z = 518 $[M+1]^+$. $^1H$ NMR (600 MHz, Methanol-$d_4$) δ 8.30 (s, 1H), 8.22 (s, 1H), 7.53 (d, *J* = 2.0 Hz, 1H), 6.81 (d, *J* = 2.1 Hz, 1H), 5.45 - 5.35 (m, 1H), 4.98 (d, *J* = 7.3 Hz, 1H), 4.66 - 4.13 (m, 2H), 3.92 - 3.57 (m, 2H), 2.88 - 2.58 (m, 2H), 2.31 (s, 3H), 1.45 (dd, *J* = 8.5, 6.6 Hz, 6H), 0.96 - 0.79 (m, 3H), 0.59 - 0.42 (m, 3H), 0.42 - 0.32 (m, 2H), 0.32 - 0.28 (m, 2H), 0.28 - 0.22 (m, 1H).

**Example 13 Preparation of compound 13**

A preparation of compound 13 is illustrated as follows:

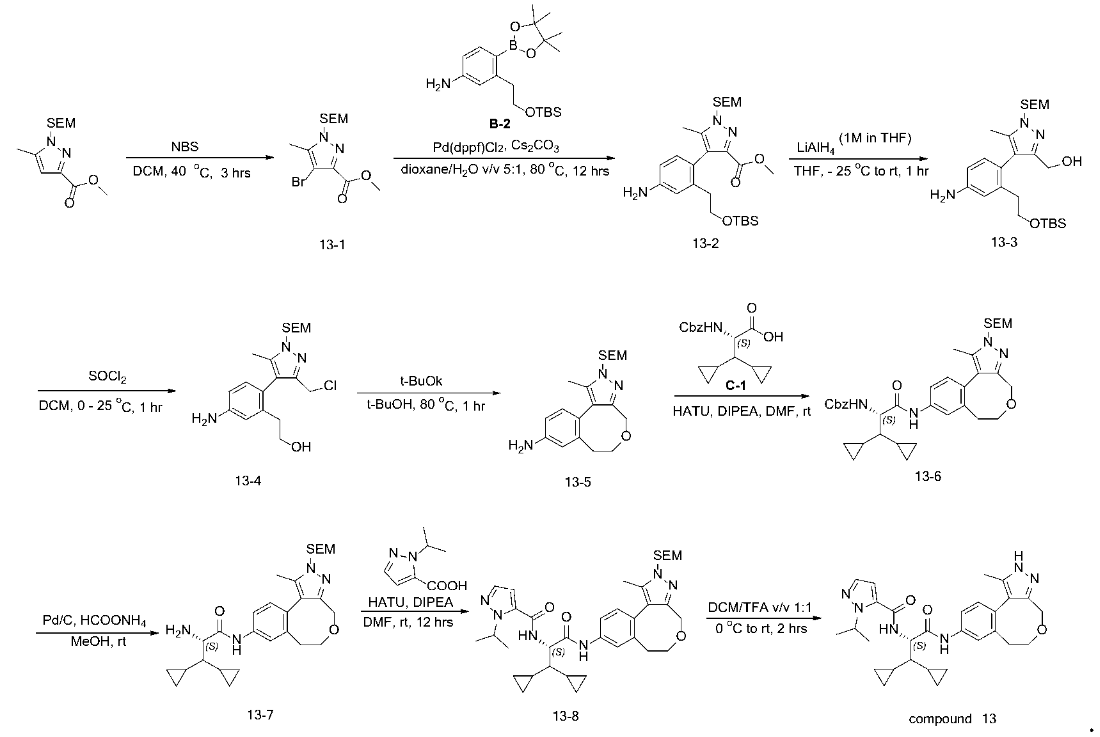


(S1) Preparation of intermediate 13-1

To a 250 mL single-neck pear-shaped flask were added methyl 5-methyl-1-((2-(trimethylsilyl)ethoxy)methyl)-1H-pyrazole-3-carboxylate (3.47 g, 12.83 mmol) and DCM (70 mL). The reaction mixture was added with NBS (4.57 g, 25.67 mmol) at room temperature, and stirred overnight. Upon completion, the reaction mixture was quenched with aqueous sodium bicarbonate solution and extracted with DCM. The DCM layer was collected, washed with a NaCl solution, dried over anhydrous sodium sulfate, and filtered. The filtrate was concentrated under reduced pressure to dryness. The residue was purified by silica gel column chromatography to obtain 791 mg (2.26 mmol) of intermediate 13-1 (yield: 17.65%). MS (ESI) m/z = 349 $[M+1]^+$.

(S2) Preparation of intermediate 13-2

To a mixture of intermediate 13-1 (741 mg, 2.12 mmol), intermediate B-2 (1.60 g, 4.24 mmol), and $Cs_2CO_3$ (1.38

g, 4.24 mmol) in 1,4-dioxane (20 mL)/water (4 mL) was added Pd(dppf)$Cl_2$ (77.61 mg, 106.07 μmol). After addition, the reaction mixture was evacuated and purged with nitrogen three times, and stirred at 80°C for 12 h. Upon completion, the reaction mixture was cooled and extracted with water and EA. The EA layer was collected, washed with a NaCl solution, dried over anhydrous sodium sulfate, and filtered. The filtrate was concentrated under reduced pressure to dryness. The residue was purified by silica gel column chromatography to obtain 332 mg (638.68 μmol) of intermediate 13-2 (yield: 30.11%). MS (ESI) m/z = 520 $[M+1]^+$.

(S3-S9) Preparation of compound 13

The preparation of compound 13 was performed according to the synthesis method in Example 1, in which intermediate 1-1 in step (S2) was replaced with intermediate 13-2, with all other reagents and procedures remaining identical. MS (ESI) m/z = 517 $[M+1]^+$. $^1$H NMR (600 MHz, Methanol-$d_4$) δ 7.64 - 7.49 (m, 3H), 7.28 (s, 1H), 6.82 (d, *J* = 2.0 Hz, 1H), 4.92 (d, *J* = 7.7 Hz, 1H), 4.23 (d, *J* = 72.7 Hz, 1H), 3.64 (s, 1H), 3.25 - 3.20 (m, 1H), 2.77 (t, *J* = 5.1 Hz, 2H), 2.31 (s, 3H), 1.48 (dd, *J* = 8.0, 6.6 Hz, 6H), 1.33 (t, *J* = 7.3 Hz, 2H), 1.00 - 0.74 (m, 3H), 0.62 - 0.36 (m, 5H), 0.35 - 0.24 (m, 3H).

**Example 14 Preparation of compound 14**

A preparation of compound 14 is illustrated as follows:

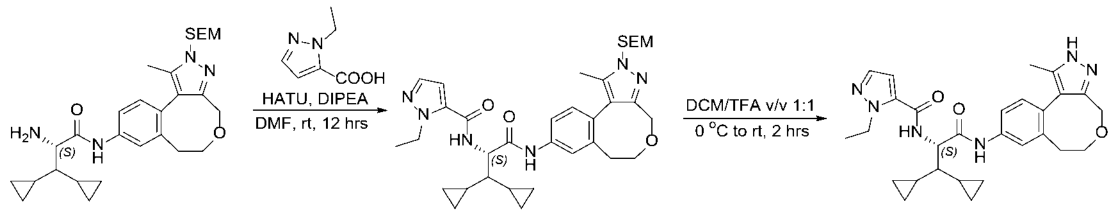


13-7 14-1 compound 14

.

The preparation of compound 14 was performed according to the synthesis method in Example 13, in which 1-isopropyl-1H-pyrazole-5-carboxylic acid in step (S8) was replaced with 1-ethyl-1H-pyrazole-5-carboxylic acid, with all other reagents and procedures remaining identical. MS (ESI) m/z = 503 $[M+1]^+$. $^1$H NMR (600 MHz, Methanol-$d_4$) δ 7.63 - 7.48 (m, 3H), 7.35 - 7.24 (m, 1H), 6.89 (d, *J* = 2.2 Hz, 1H), 4.92 (d, *J* = 7.6 Hz, 1H), 4.80 - 4.50 (m, 3H), 4.17 (s, 2H), 3.82 - 3.51 (m, 1H), 2.77 (t, *J* = 5.1 Hz, 2H), 2.31 (s, 3H), 1.41 (t, *J* = 7.2 Hz, 3H), 0.97 - 0.76 (m, 3H), 0.58 (tt, *J* = 8.5, 4.6 Hz, 1H), 0.55 - 0.46 (m, 2H), 0.45 - 0.36 (m, 2H), 0.35 - 0.24 (m, 3H).

**Example 15 Preparation of compound 15**

A preparation of compound 15 is illustrated as follows:

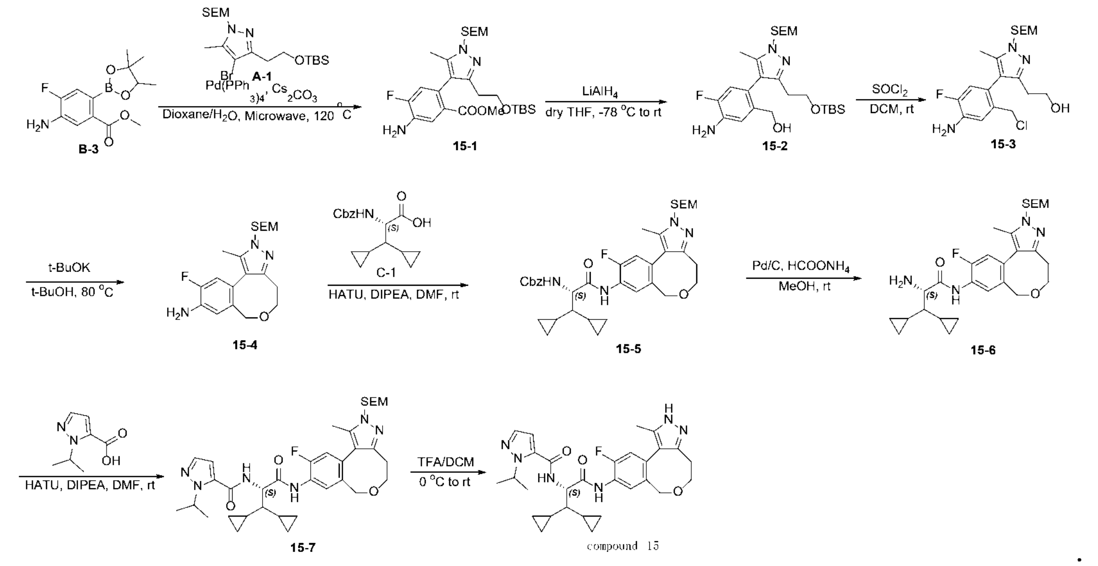


The preparation of compound 15 was performed according to the synthesis method in Example 1, in which intermediate B-1 in step (S1) was replaced with intermediate B-3, with all other reagents and procedures remaining identical. MS (ESI) m/z = 535 [M+1]+. $^1$H NMR (400 MHz, Methanol-$d_4$) δ 8.04 (d, *J* = 8.0 Hz, 1H), 7.53 (d, *J* = 2.0 Hz, 1H), 7.19 (d, *J* = 11.2 Hz, 1H), 6.80 (d *J* = 2.1 Hz, 1H), 5.48 - 5.33 (m, 1H), 5.01 (d, *J* = 7.1 Hz, 1H), 4.31 (s, 2H), 3.72 (s, 2H), 2.70 (s, 2H), 2.28 (s, 3H), 1.46 (dd, *J* = 6.7, 4.8 Hz, 6H), 1.02 - 0.79 (m, 3H), 0.61 - 0.53 (m, 1H), 0.53 - 0.41 (m, 3H), 0.41 - 0.35 (m, 1H), 0.35 - 0.21 (m, 3H).

**Examples 16-21 Preparation of compounds 16-21**

The preparation of compounds 16 to 21 was performed according to the synthesis method in Example 15, in which 1-isopropyl-1H-pyrazole-5-carboxylic acid in step (S7) was correspondingly replaced with a carboxylic acid intermediate from the following table, with all other reagents and procedures remaining identical.

| Carboxylic acid intermediate | Structure of compound | $^1$HNMR and/or MS (ESI) |
|---|---|---|
| 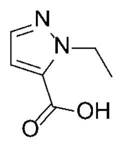 | 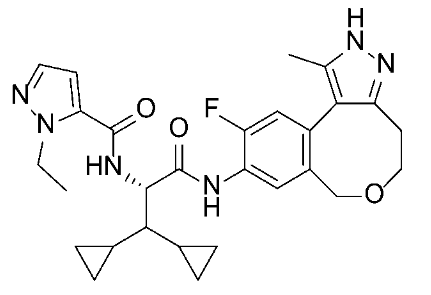 compound 16 | MS (ESI) m/z =521 [M+1]+ $^1$H NMR (400 MHz, Methanol-$d_4$) δ 8.03 (d, *J* = 8.0 Hz, 1H), 7.51 (d, *J* = 2.1 Hz, 1H), 7.19 (s, 1H), 6.87 (d, *J* = 2.1 Hz, 1H), 5.00 (d, *J* = 6.9 Hz, 1H), 4.62 - 4.48 (m, 3H), 4.43 - 4.22 (m, 1H), 3.88 - 3.53 (m, 2H), 2.70 (s, 2H), 2.28 (s, 3H), 1.40 (t, *J* = 7.1 Hz, 3H), 0.98 - 0.76 (m, 3H), 0.62 - 0.53 (m, 1H), 0.52 - 0.42 (m, 3H), 0.38 (dt, *J* = 9.4, 4.3 Hz, 1H), 0.34 - 0.23 (m, 3H). |
| 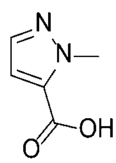 | 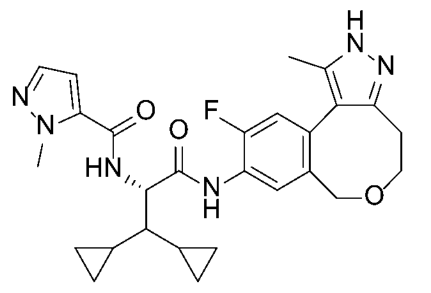 compound 17 | MS (ESI) m/z =507 [M+1]+ $^1$H NMR (600 MHz, Methanol-$d_4$) δ 8.05 (d, *J* = 7.9 Hz, 1H), 7.52 (d, *J* = 2.1 Hz, 1H), 7.21 (s, 1H), 6.91 (d, *J* = 2.1 Hz, 1H), 5.03 (d, *J* = 7.3 Hz, 1H), 4.60 (s, 2H), 4.49 - 4.21 (m, 1H), 4.14 (s, 3H), 3.74 (s, 1H), 2.73 (s, 2H), 2.31 (s, 3H), 0.97 - 0.83 (m, 3H), 0.63 - 0.56 (m, 1H), 0.56 - 0.44 (m, 3H), 0.43 - |

(continued)

| Carboxylic acid intermediate | Structure of compound | 1HNMR and/or MS (ESI) |
|---|---|---|
| | | 0.37 (m, 1H), 0.36 - 0.25 (m, 3H). |
| 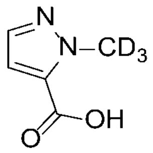 | 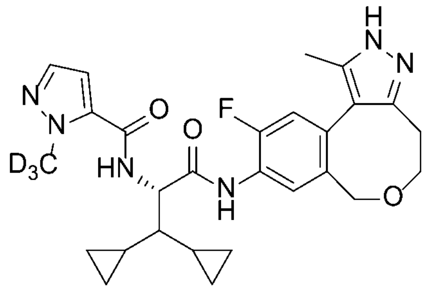 compound 18 | MS (ESI) m/z =510 [M+1]$^+$ $^1$H NMR (600 MHz, Methanol-$d_4$) δ 8.05 (d, *J* = 7.9 Hz, 1H), 7.50 (d, *J* = 2.1 Hz, 1H), 7.20 (d, *J* = 11.1 Hz, 1H), 6.90 (d, *J* = 2.1 Hz, 1H), 5.03 (d, *J*= 7.4 Hz, 1H), 4.35 (t, *J* = 140.5 Hz, 2H), 3.67 (d, *J* = *73.8* Hz, 2H), 2.72 (s, 2H), 2.29 (s, 3H), 1.03 - 0.78 (m, 3H), 0.67 - 0.35 (m, 5H), 0.35 - 0.25 (m, 3H). |
| 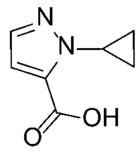 | 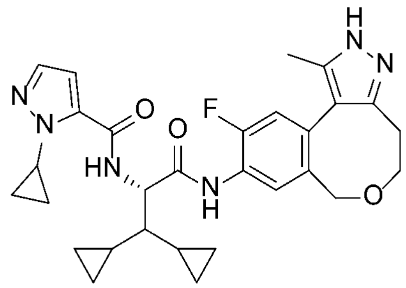 compound 19 | MS (ESI) m/z =533 [M+1]$^+$ $^1$H NMR (400 MHz, Methanol-$d_4$) δ 8.04 (d, *J* = 8.0 Hz, 1H), 7.43 (d, *J* = 2.1 Hz, 1H), 7.19 (s, 1H), 6.79 (d, *J* = 2.1 Hz, 1H), 5.03 (d, *J* = 6.9 Hz, 1H), 4.32 (s, 2H), 4.22 - 4.11 (m, 1H), 3.72 (s, 2H), 2.70 (s, 2H), 2.29 (s, 3H), 1.19 - 1.10 (m, 2H), 1.06 - 0.98 (m, 2H), 0.97 - 0.78 (m, 3H), 0.62 - 0.24 (m, 8H). |
| 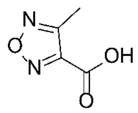 | 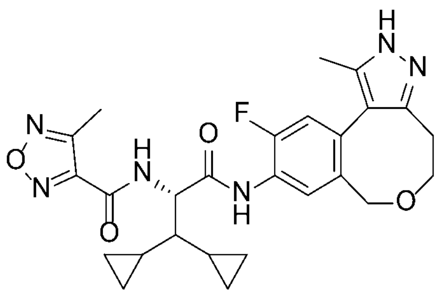 compound 20 | MS (ESI) m/z =509 [M+1]$^+$ $^1$H NMR (600 MHz, Methanol-$d_4$) δ 8.03 (d, *J* = 7.9 Hz, 1H), 7.21 (s, 1H), 5.08 (d, *J* = 6.7 Hz, 1H), 4.69 - 4.06 (m, 2H), 3.73 (s, 2H), 3.00 - 2.64 (m, 2H), 2.58 (s, 3H), 2.30 (s, 3H), 1.02 - 0.77 (m, 3H), 0.67 - 0.44 (m, 4H), 0.41 (dd, *J* = 9.4, 4.6 Hz, 1H), 0.32 (ddt, *J* = 13.1, 8.7, 4.6 Hz, 3H). |
| 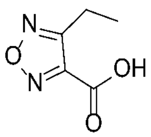 | 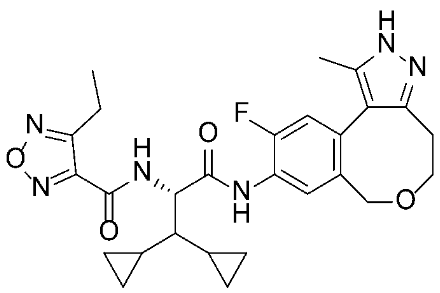 compound 21 | MS (ESI) m/z =523 [M+1]$^+$ $^1$H NMR (600 MHz, Methanol-$d_4$) δ 8.01 (d, *J* = 7.9 Hz, 1H), 7.18 (s, 1H), 5.07 (d, *J* = 6.7 Hz, 1H), 4.30 (s, 2H), 3.71 (s, 2H), 3.02 (q, *J* = 7.4, 1.1 Hz, 2H), 2.70 (s, 2H), 2.28 (s, 3H), 1.34 (t, *J* = 7.5 Hz, 3H), 0.97 - 0.76 (m, 3H), 0.60 - 0.42 (m, 4H), 0.42 - 0.36 (m, 1H), 0.35 - 0.26 (m, 3H). |

**Example 22 Preparation of compound 22**

A preparation of compound 22 is illustrated as follows:

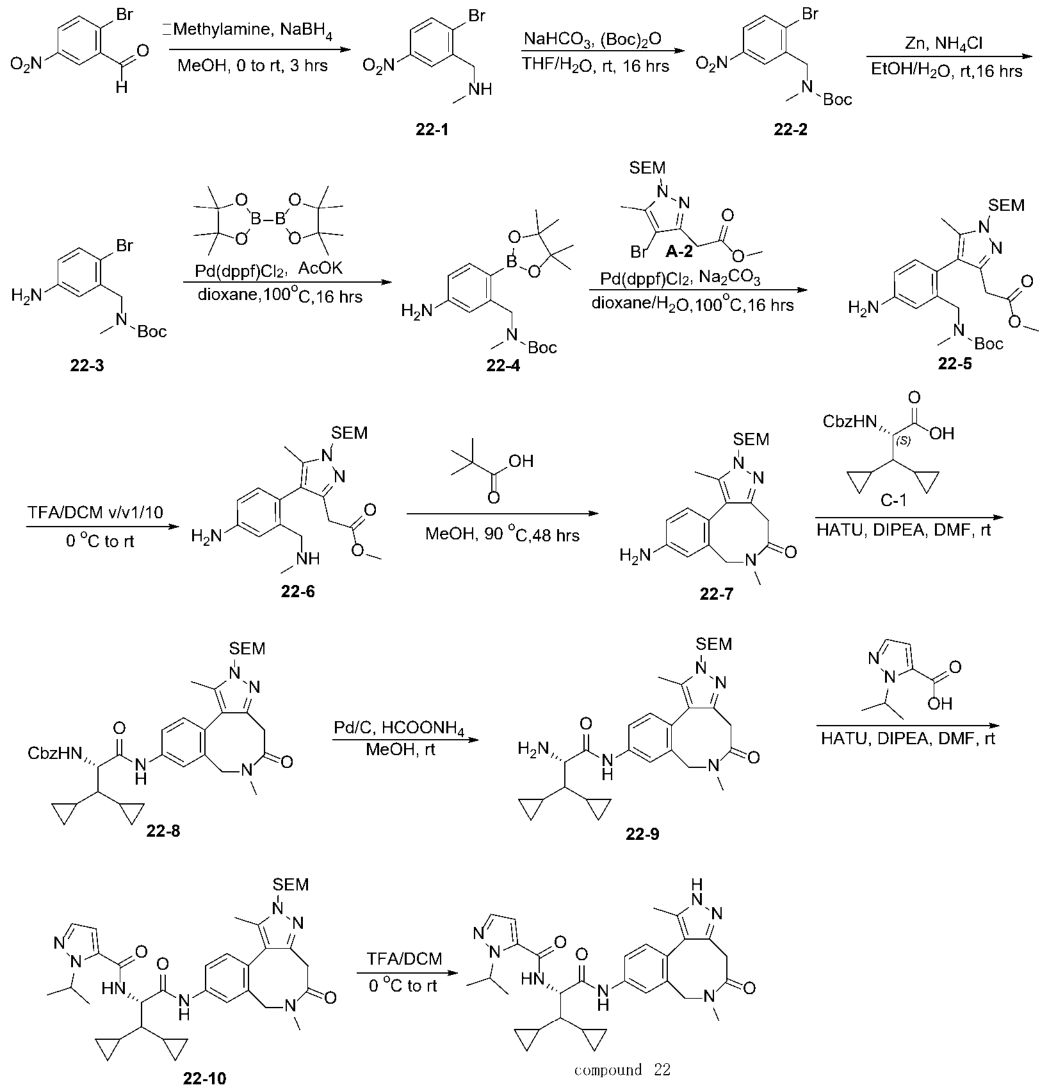


(S1) Preparation of intermediate 22-1

To a solution of 2-bromo-5-nitrobenzaldehyde (25 g, 108.69 mmol) in methanol (500 mL) was added methylamine hydrochloride (30 g, 217.38 mmol). After addition, the reaction mixture was stirred at room temperature for 1 h. The reaction mixture was cooled to 0°C under an ice-water bath, and added with sodium borohydride (8.22 g, 217.38 mmol) in portions. After addition, the reaction mixture was warmed to room temperature and stirred for 2 h. The reaction mixture was cooled to 0°C and quenched with 30% ammonium chloride solution, and stirred for 10 min. The reaction mixture was concentrated under reduced pressure to an appropriate volume, and extracted with EA. The EA layer was collected, washed sequentially with water and a saturated NaCl solution, dried over anhydrous sodium sulfate, and filtered. The filtrate was concentrated under reduced pressure to dryness. The residue was purified by silica gel column chromatography to obtain 23 g (93.85 mmol) of intermediate 22-1 (yield: 86.35%). MS (ESI) m/z = 245 $[M+1]^+$.

(S2) Preparation of intermediate 22-2

To a solution of intermediate 22-1 (23 g, 93.85 mmol) in $THF/H_2O$ (230 mL/100 mL) were added sodium bicarbonate (15.77 g, 187.70 mmol) and $(Boc)_2O$ (30.72 g, 140.77 mmol). After addition, the reaction mixture was stirred at room temperature overnight. The reaction mixture was added with water (100 mL), and extracted with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 32.3 g (93.57 mmol) of intermediate 22-2 (yield: 99.70%). MS (ESI) m/z = 345 $[M+1]^+$.

(S3) Preparation of intermediate 22-3

To a solution of intermediate 22-2 (32 g, 92.70 mmol) in EtOH/$H_2O$ (640 mL/128 mL) was added ammonium chloride (39.67 g, 741.62 mmol), followed by addition of zinc powder (30.72 g, 140.77 mmol) in portions under an ice-water bath. After addition, the reaction mixture was stirred at room temperature overnight. The reaction mixture was filtered through Celite and washed with EA. The filtrate was concentrated under reduced pressure to dryness. The residue was extracted with water and EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 20 g (63.45 mmol) of intermediate 22-3 (yield: 68.45%). MS (ESI) m/z = 315 $[M+1]^+$.

(S4) Preparation of intermediate 22-4

To a 500 mL single-neck flask were added intermediate 22-3 (23 g, 72.97 mmol), bis(pinacolato)diboron (22.24 g, 87.56 mmol), potassium acetate (14.32 g, 145.94 mmol), Pd(dppf)$Cl_2$ (5.34 g, 7.30 mmol) and dioxane (230 mL). The reaction mixture was evacuated and purged with nitrogen three times, and heated to 100°C and stirred overnight. Upon completion, the reaction mixture was cooled and concentrated under reduced pressure to an appropriate volume. The residue was extracted with EA and washed with water. The EA layer was collected, dried over anhydrous sodium sulfate and filtered. The filtrate was concentrated under reduced pressure to dryness. The residue was purified by silica gel column chromatography to obtain 22.9 g (63.21 mmol) of intermediate 22-4 (yield: 86.63%). MS (ESI) m/z = 363 $[M+1]^+$.

(S5) Preparation of intermediate 22-5

To a 500 mL single-neck flask were added intermediate 22-4 (22.43 g, 61.93 mmol), intermediate A-2 (15 g, 42.19 mmol), sodium carbonate (8.5 g, 82.57 mmol), Pd(dppf)$Cl_2$ (1.51 g, 2.06 mmol) and dioxane/water (230 mL/50 mL). The reaction mixture was evacuated and purged with nitrogen three times under stirring, and heated to 100°C and stirred overnight. Upon completion, the reaction mixture was cooled and concentrated under reduced pressure to an appropriate volume. The residue was extracted with EA and washed with water. The EA layer was collected, dried over anhydrous sodium sulfate and filtered. The filtrate was concentrated under reduced pressure to dryness. The residue was purified by silica gel column chromatography to obtain 12.41 g (23.95 mmol) of intermediate 22-5 (yield: 58%). MS (ESI) m/z = 519 $[M+1]^+$.

(S6) Preparation of intermediate 22-6

To a solution of intermediate 22-5 (2 g, 3.86 mmol) in DCM (100 mL) was added TFA (10 mL) under an ice-water bath. After addition, the reaction mixture was stirred at room temperature until the reaction was complete. Upon completion, the reaction mixture was concentrated to dryness to give a crude product. The crude product was purified by preparative MPLC ($H_2O/CH_3CN$, 0.05% $NH_4HCO_3$) to obtain 1.53 g (3.66 mmol) of intermediate 22-6 (yield: 94.98%). MS (ESI) m/z = 419 $[M+1]^+$.

(S7) Preparation of intermediate 22-7

To a 50 mL glass pressure tube were added intermediate 22-6 (649 mg, 1.55 mmol), methanol (10 mL) and pivalic acid. After addition, the reaction mixture was heated to 90°C and stirred for 12 h. Since some raw material remained unreacted, the reaction mixture was directly concentrated under reduced pressure to dryness. The residue was dissolved in DCM and washed sequentially with saturated sodium bicarbonate solution and water. The DCM layer was collected, dried over anhydrous sodium sulfate and filtered. The filtrate was concentrated under reduced pressure to dryness. The residue was purified by silica gel column chromatography to obtain 688 mg (889.90 μmol) of intermediate 22-7 (yield: 57.40%, 50% purity). MS (ESI) m/z = 387 $[M+1]^+$.

(S8-S11) Preparation of compound 22

The preparation of compound 22 was performed according to the synthesis method in Example 1, in which intermediate 1-4 in step (S5) was replaced with intermediate 22-7, with all other reagents and procedures remaining identical. MS (ESI) m/z = 544 $[M+1]^+$. $^1$H NMR (600 MHz, Methanol-$d_4$) δ 7.78 (d, *J* = 2.4 Hz, 1H), 7.61 (dd, *J* = 8.5, 2.3 Hz, 1H), 7.54 (d, *J* = 2.1 Hz, 1H), 7.44 (s, 1H), 6.83 (d, *J* = 2.0 Hz, 1H), 5.48 - 5.38 (m, 1H), 4.93 (d, *J* = 7.7 Hz, 1H), 4.51 (q, *J* = 14.7 Hz, 2H), 4.02 (d, *J* = 41.7 Hz, 2H), 2.95 (s, 3H), 2.35 (s, 3H), 1.47 (dd, *J* = 9.1, 6.7 Hz, 6H), 0.98 - 0.78 (m, 3H), 0.62 - 0.45 (m, 3H), 0.44 - 0.37 (m, 2H), 0.35 - 0.23 (m, 3H).

## Example 23 Preparation of compound 23

A preparation of compound 23 is illustrated as follows:

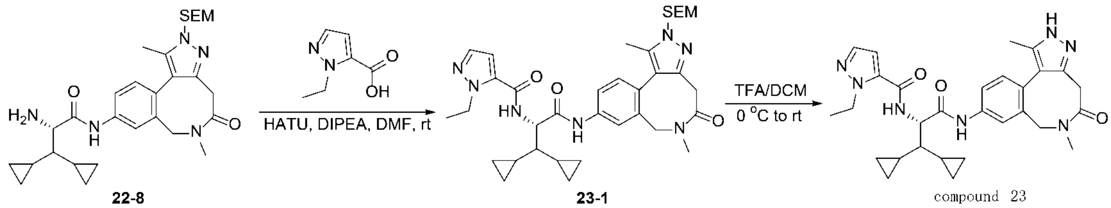


22-8 23-1 compound 23

The preparation of compound 23 was performed according to the synthesis method in Example 22, in which 1-isopropyl-1H-pyrazole-5-carboxylic acid in step (S9) was replaced with 1-ethyl-1H-pyrazole-5-carboxylic acid, with all other reagents and procedures remaining identical. MS (ESI) m/z = 530 [M+1]$^+$. $^1$H NMR (600 MHz, Methanol-$d_4$) δ 7.77 - 7.69 (m, 1H), 7.58 (s, 1H), 7.44 (d, *J* = 62.0 Hz, 2H), 6.86 (t, *J* = 3.5 Hz, 1H), 4.90 (d, *J* = 7.6 Hz, 1H), 4.58 - 4.40 (m, 4H), 3.99 (d, *J* = 67.6 Hz, 2H), 2.91 (s, 3H), 2.32 (s, 3H), 1.44 - 1.30 (m, 3H), 0.95 - 0.73 (m, 3H), 0.59 - 0.52 (m, 1H), 0.52 - 0.41 (m, 2H), 0.41 - 0.33 (m, 2H), 0.33 - 0.20 (m, 3H).

## Example 24 Preparation of compound 24

A preparation of compound 24 is illustrated as follows:

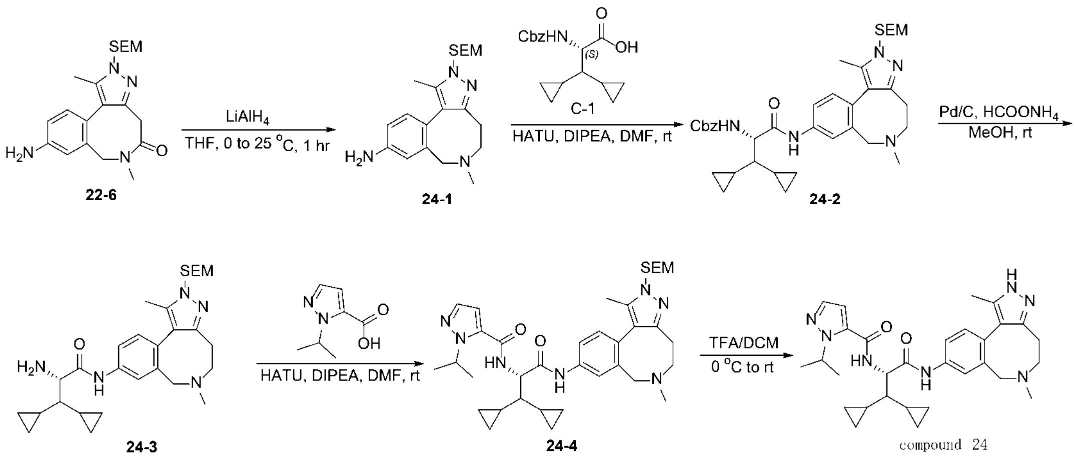


22-6 24-1 24-2 24-3 24-4 compound 24

To a solution of intermediate 22-6 (638 mg, 825.22 μmol) in dry THF (20 mL) was added dropwise $LiAlH_4$ (62.63 mg, 1.65 mmol, 1M in THF) at -78°C. After addition, the reaction mixture was warmed to room temperature and reacted for 1 h. Upon completion, the reaction mixture was quenched at -10°C by sequential addition of ice water (0.08 mL), 15% aqueous NaOH solution (0.08 mL) and water (0.24 mL), followed by stirring for 10 min. The reaction mixture was filtered through Celite, and the filter cake was washed with EA. The filtrate was concentrated to dryness to obtain 235 mg (630.74 μmol) of intermediate 24-1 (yield: 76.43%), which was used directly in the next step without further purification. MS (ESI) m/z = 373 [M+1]$^+$.

The preparation of compound 24 was performed according to the synthesis method in

Example 1, in which intermediate 1-4 in step (S5) was replaced with intermediate 24-1, with all other reagents and procedures remaining identical. MS (ESI) m/z = 530 [M+1]$^+$. $^1$H NMR (600 MHz, Methanol-$d_4$) δ 7.76 - 7.61 (m, 2H), 7.56 - 7.50 (m, 1H), 7.34 (s, 1H), 6.82 (d, *J* = 2.1 Hz, 1H), 5.43 sept, *J* = 6.8 Hz, 1H), 4.92 (d, *J* = 7.6 Hz, 1H), 3.67 (s, 2H), 3.07 - 2.56 (m, 4H), 2.45 (s, 3H), 2.25 (s, 3H), 1.48 (dd, *J* = 7.8, 6.7 Hz, 6H), 0.97 - 0.77 (m, 3H), 0.64 - 0.35 (m, 5H), 0.33 - 0.24 (m, 3H).

## Examples 25-26 Preparation of compounds 25 and 26

The preparation of compounds 25 and 26 was performed according to the synthesis method in Example 24, in which 1-isopropyl-1H-pyrazole-5-carboxylic acid in step (S4) was correspondingly replaced with a pyrazolecarboxylic acid from the following table, with all other reagents and procedures remaining identical.

| Pyrazolecarbo xylic acid | Structure of compound | 1HNMR and/or MS (ESI) |
|---|---|---|
| 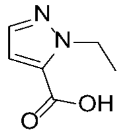 | 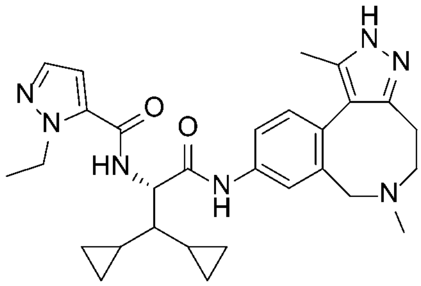 compound 25 | MS (ESI) m/z =516 [M+1]+ $^1$H NMR (600 MHz, Methanol-$d_4$) δ 7.81 - 7.62 (m, 2H), 7.53 (d, *J* = 2.1 Hz, 1H), 7.35 (s, 1H), 6.90 (d, *J* = 2.1 Hz, 1H), 4.93 (d, *J* = 7.6 Hz, 1H), 4.62 - 4.54 (m, 2H), 3.69 (s, 2H), 3.12 - 2.59 (m, 4H), 2.46 (s, 3H), 2.24 (s, 3H), 1.41 (t, *J* = 7.2 Hz, 3H), 0.99 - 0.77 (m, 3H), 0.61 - 0.36 (m, 5H), 0.34 - 0.25 (m, 3H). |
| 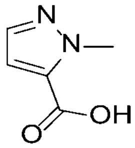 | 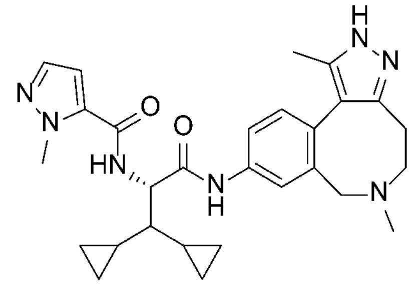 compound 26 | MS (ESI) m/z =502 [M+1]+ $^1$H NMR (600 MHz, Methanol-$d_4$) δ 8.06 (s, 1H), 7.67 (d, *J* = 8.3 Hz, 1H), 7.56 - 7.41 (m, 2H), 6.91 (d, *J* = 2.1 Hz, 1H), 4.90 (d,*J* = 7.2 Hz, 1H), 4.43 (d, *J* = 8.1 Hz, 1H), 4.13 (s, 3H), 3.67 - 3.45 (m, 1H), 3.27 (d,*J* = 10.5 Hz, 1H), 3.14 (s, 2H), 2.94 (s, 1H), 2.30 (s, 3H), 1.41 - 1.26 (m, 2H), 0.99 - 0.78 (m, 4H), 0.62 - 0.56 (m, 1H), 0.56 - 0.46 (m, 2H), 0.46 - 0.38 (m, 2H), 0.36 - 0.30 (m, 2H), 0.30 - 0.24 (m, 1H). |

**Example 27 Preparation of compound 27**

A preparation of compound 27 is illustrated as follows:

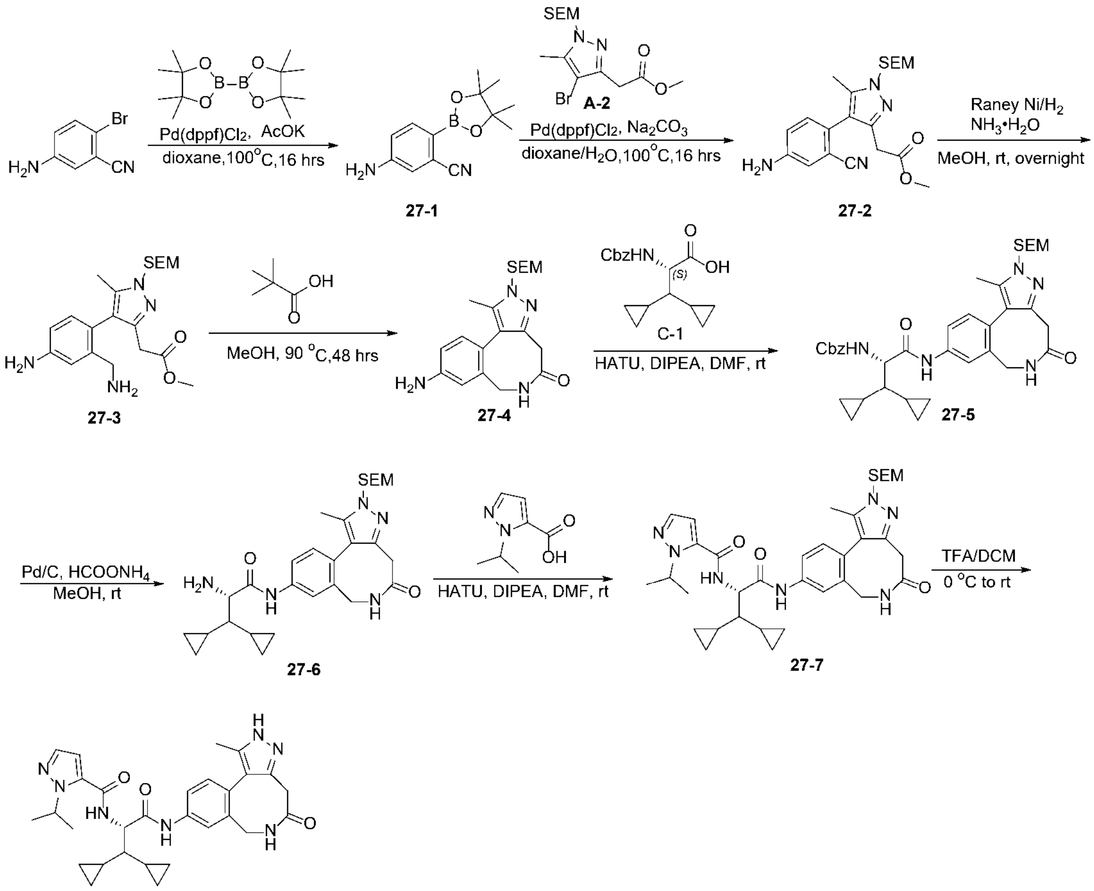


compound 27

(S1-S2) Preparation of intermediate 27-2

The preparation of intermediate 27-2 was performed according to steps (S4-S5) in Example 22, in which intermediate 22-3 in step (S4) was replaced with 5-amino-2-bromobenzonitrile, with all other reagents and procedures remaining identical. MS (ESI) m/z =401 $[M+1]^+$.

(S3) Preparation of intermediate 27-3

To a 50 mL single-neck flask were added intermediate 27-2 (300 mg, 748.98 μmol) and methanol (6 mL), followed by addition of Raney Ni (60 mg, 748.98 μmol). The reaction mixture was evacuated and purged with hydrogen, and stirred at room temperature overnight. The reaction mixture was filtered through Celite, and the filter cake was washed with methanol. The filtrate was concentrated to dryness to obtain 218 mg (538.83 μmol) of intermediate 27-3 (yield: 71.94%), which was used directly in the next step without further purification. MS (ESI) m/z = 405 $[M+1]^+$.

(S4-S8) Preparation of compound 27

The preparation of compound 27 was performed according to steps (S7-S11) in Example 22, in which intermediate 22-6 in step (S7) was replaced with intermediate 27-3, with all other reagents and procedures remaining identical. MS (ESI) m/z = 530 $[M+1]^+$. $^1$H NMR (600 MHz, Methanol-$d_4$) δ 7.80 (d, $J$ = 2.3 Hz, 1H), 7.63 (d, $J$ = 8.3 Hz, 1H), 7.54 (d, $J$ = 2.0 Hz, 1H), 7.37 (d, $J$ = 8.3 Hz, 1H), 6.81 (d, $J$ = 2.0 Hz, 1H), 5.48 - 5.35 (m, 1H), 4.91 (d, $J$=7.7 Hz, 1H), 4.05 (s, 2H), 3.55 (s, 2H), 2.32 (s, 3H), 1.47 (dd, $J$ = 8.8, 6.7 Hz, 6H), 0.98 - 0.75 (m, 3H), 0.62 - 0.35 (m, 5H), 0.34 - 0.23 (m, 3H).

**Example 28 Preparation of compound 28**

A preparation of compound 28 is illustrated as follows:

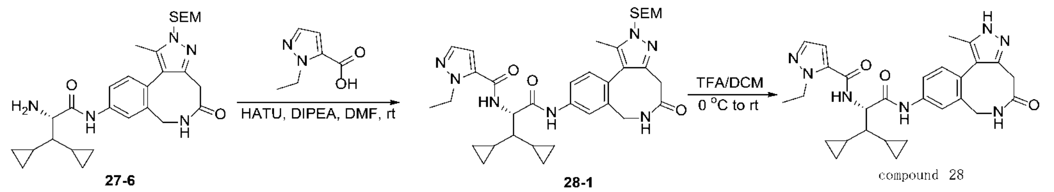


The preparation of compound 28 was performed according to the synthesis method in Example 27, in which 1-isopropyl-1H-pyrazole-5-carboxylic acid in step (S7) was replaced with 1-ethyl-1H-pyrazole-5-carboxylic acid, with all other reagents and procedures remaining identical. MS (ESI) m/z = 516 [M+1]+. $^1$H NMR (600 MHz, Methanol-$d_4$) δ 7.80 (d, *J* = 2.3 Hz, 1H), 7.63 (d, *J* = 8.3 Hz, 1H), 7.52 (d, *J* = 2.1 Hz, 1H), 7.37 (d, *J* = 7.4 Hz, 1H), 6.88 (d, *J* = 2.1 Hz, 1H), 4.92 (d, *J* = 7.6 Hz, 1H), 4.56 (qd, *J* = 7.1, 2.5 Hz, 2H), 4.05 (s, 2H), 3.55 (s, 2H), 2.32 (s, 3H), 1.40 (t, *J* = 7.2 Hz, 3H), 0.98 - 0.76 (m, 3H), 0.65 - 0.35 (m, 5H), 0.35 - 0.23 (m, 3H).

## Example 29 Preparation of compound 29

A preparation of compound 29 is illustrated as follows:

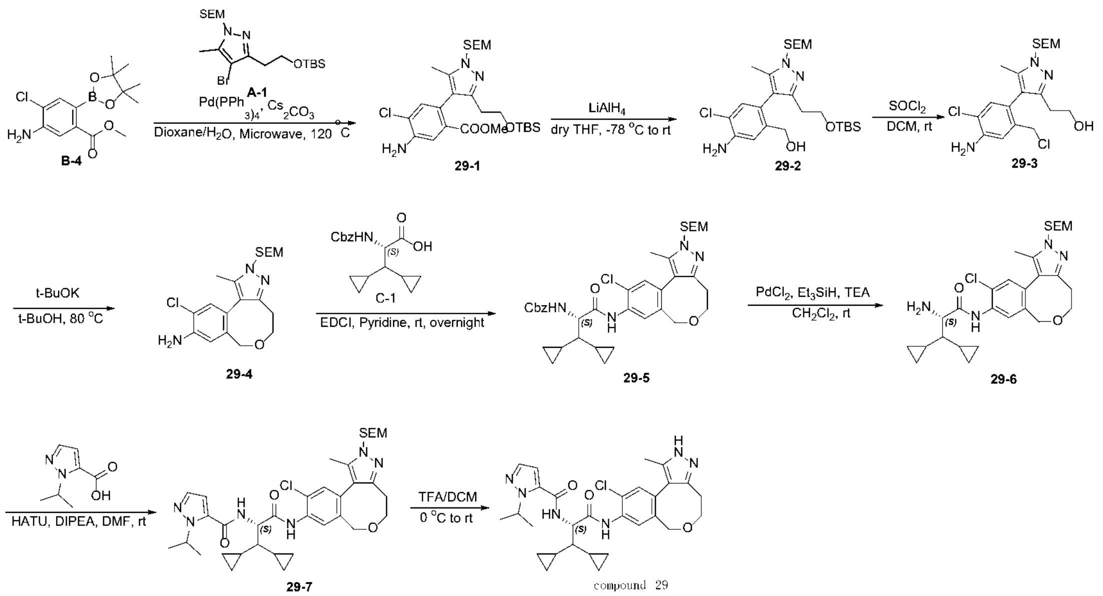


## (S1-S4) Preparation of intermediate 29-4

The preparation of intermediate 29-4 was performed according to steps (S1-S4) of Example 1, in which intermediate B-1 in step (S1) was replaced with intermediate B-4, with all other reagents and procedures remaining identical. MS (ESI) m/z = 394 [M+1]+.

## (S5) Preparation of intermediate 29-5

To a solution of intermediate 29-4 (600 mg, 1.53 mmol) and intermediate C-1 (601.37 mg, 1.98 mmol) in pyridine (16 mL) was added EDCI (1.18 g, 6.12 mmol). After addition, the reaction mixture was stirred at room temperature overnight. The reaction mixture was concentrated under reduced pressure to dryness. The residue was dissolved in EA and washed sequentially with water and a saturated NaCl solution. The EA layer was collected, dried over anhydrous sodium sulfate and filtered. The filtrate was concentrated under reduced pressure to dryness. The residue was purified by MPLC ($H_2O/CH_3CN$, 0.05% $NH_4HCO_3$) to obtain 902 mg (1.33 mmol) of intermediate 29-5 (yield: 86.98%). MS (ESI) m/z = 679 [M+1]+.

## (S6) Preparation of intermediate 29-6

To a solution of intermediate 29-5 (800 mg, 1.18 mmol) in DCM (11 mL) were added triethylamine (83.42 mg, 0.83

mmol) and $PdCl_2$ (41.44 mg, 0.24 mmol). After addition, the reaction mixture was cooled to 0°C under an ice-water bath. A solution of triethylsilane (684.40 mg, 5.90 mmol) in DCM (3 mL) was added dropwise. After addition, the reaction mixture was stirred at 0°C under nitrogen protection until the reaction was complete. The reaction mixture was filtered through Celite and washed with DCM. The filtrate was concentrated under reduced pressure to dryness. The residue was purified by MPLC ($H_2O/CH_3CN$, 0.05% $NH_4HCO_3$) to obtain 464.62 mg (0.85 mmol) of intermediate 29-6 (yield: 72.38%). MS (ESI) m/z = 545 $[M+1]^+$.

(S7-S8) Preparation of compound 29

The preparation of compound 29 was performed according to steps (S7-S8) of Example 1, in which intermediate 1-6 in step (S7) was replaced with intermediate 29-6, with all other reagents and procedures remaining identical. MS (ESI) m/z = 551 $[M+1]^+$. $^1H$ NMR (600 MHz, DMSO-$d_6$) δ 12.47 (d, *J* = 28.7 Hz, 1H), 9.68 (s, 1H), 8.44 (d, *J* = 8.7 Hz, 1H), 7.81 (s, 1H), 7.55 - 7.36 (m, 2H), 6.90 (d, *J* = 1.9 Hz, 1H), 5.51 - 5.28 (m, 1H), 4.93 (t, *J* = 8.8, 7.2 Hz, 1H), 4.20 (s, 2H), 3.60 (s, 2H), 2.72 - 2.52 (m, 2H), 2.18 (d, *J* = 35.6 Hz, 3H), 1.36 (dd, *J* = 16.6, 6.6 Hz, 6H), 1.02 - 0.78 (m, 3H), 0.51 - 0.45 (m, 1H), 0.43 - 0.28 (m, 4H), 0.28 - 0.17 (m, 3H).

**Examples 30-31 Preparation of compounds 30 and 31**

The preparation of compounds 30 and 31 was performed according to the synthesis method in Example 29, in which 1-isopropyl-1H-pyrazole-5-carboxylic acid in step (S7) was correspondingly replaced with a pyrazolecarboxylic acid from the following table, with all other reagents and procedures remaining identical.

| Pyrazolecarboxylic acid | Structure of compound | $^1$HNMR and/or MS (ESI) |
|---|---|---|
| 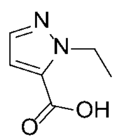 | 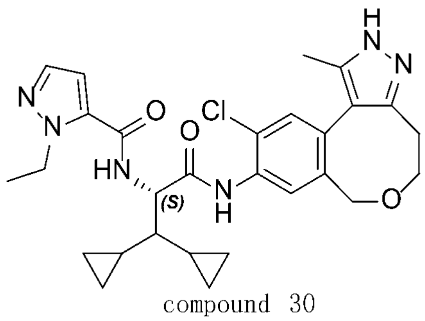 compound 30 | MS (ESI) m/z =537 $[M+1]^+$ $^1H$ NMR (600 MHz, DMSO-$d_6$) δ 12.51 (s, 1H), 9.72 (s, 1H), 8.45 (d, *J* = 8.8 Hz, 1H), 7.82 (s, 1H), 7.53 - 7.40 (m, 2H), 7.00 (d, *J* = 2.1 Hz, 1H), 4.96 (t, *J* = 8.1 Hz, 1H), 4.55 - 4.42 (m, 2H), 4.24 (s, 2H), 3.62 (s, 2H), 2.61 (s, 2H), 2.19 (d, *J* = 37.2 Hz, 3H), 1.30 (t, *J* = 7.2 Hz, 3H), 0.99 - 0.78 (m, 3H), 0.54 - 0.46 (m, 1H), 0.44 - 0.29 (m, 4H), 0.29 - 0.16 (m, 3H). |
| 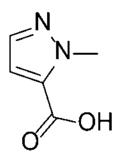 | 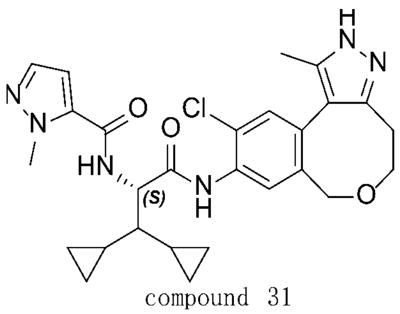 compound 31 | MS (ESI) m/z =523 $[M+1]^+$ $^1H$ NMR (600 MHz, DMSO-$d_6$) δ 12.49 (s, 1H), 9.73 (s, 1H), 8.43 (d, *J* = 8.8 Hz, 1H), 7.83 (s, 1H), 7.52 - 7.36 (m, 2H), 7.04 (d, *J* = 2.1 Hz, 1H), 4.97 (t, 1H), 4.25 (s, 2H), 4.05 (s, 3H), 3.61 (s, 2H), 2.76 - 2.51 (m, 2H), 2.20 (s, 3H), 0.99 - 0.78 (m, 3H), 0.53 - 0.46 (m, 1H), 0.43 - 0.29 (m, 4H), 0.29 - 0.17 (m, 3H). |

**Example 32 Preparation of compound 32**

A preparation of compound 32 is illustrated as follows:

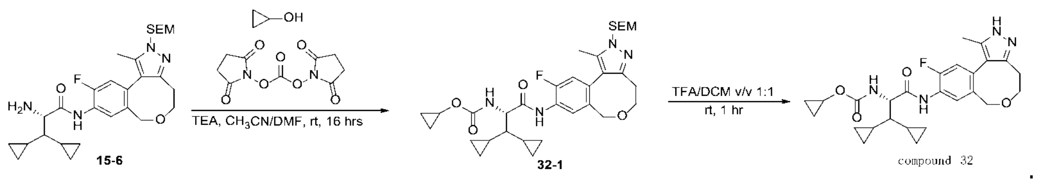

(S1) Preparation of intermediate 32-1

To a solution of cyclopropanol (6.59 mg, 113.48 μmol) and N,N'-disuccinimidyl carbonate (29.07 mg, 113.48 μmol) in acetonitrile (1 mL) was added triethylamine (9.57 mg, 94.57 μmol, 13.19 μL) at room temperature. After addition, the reaction mixture was stirred at room temperature for 2 h. The mixture was added with a solution of intermediate 15-6 (50 mg, 94.57 μmol) in DMF (0.5 mL), and stirred at room temperature overnight. The reaction mixture was directly purified by MPLC ($H_2O/CH_3CN$, 0.05% TFA) to obtain 14 mg (22.85 μmol) of intermediate 32-1 (yield: 24.16%). MS (ESI) m/z = 613 $[M+1]^+$.

(S2) Preparation of compound 32

To a solution of intermediate 32-1 (14 mg, 22.85 μmol) in DCM (1 mL) was added TFA (1 mL). After addition, the reaction mixture was stirred at room temperature for 3 h. Upon completion, the reaction mixture was concentrated to dryness and purified by preparative MPLC to obtain 5 mg (8.38 μmol) of compound 32 (yield: 36.69%). MS (ESI) m/z =483 $[M+1]^+$. $^1$H NMR (600 MHz, Methanol-$d_4$) δ 8.02 (d, *J* = 7.9 Hz, 1H), 7.21 (d, *J* = 11.4 Hz, 1H), 4.56 (d, *J* = 6.0 Hz, 1H), 4.34 (s, 2H), 4.10 - 3.99 (m, 1H), 3.73 (s, 2H), 2.72 (s, 2H), 2.30 (s, 3H), 0.90 - 0.80 (m, 2H), 0.75 - 0.62 (m, *J* = 3.8 Hz, 5H), 0.61 - 0.52 (m, 1H), 0.51 - 0.46 (m, 1H), 0.46 - 0.39 (m, 2H), 0.32 - 0.21 (m, 4H).

**Example 33 Preparation of compound 33**

A preparation of compound 33 is illustrated as follows:

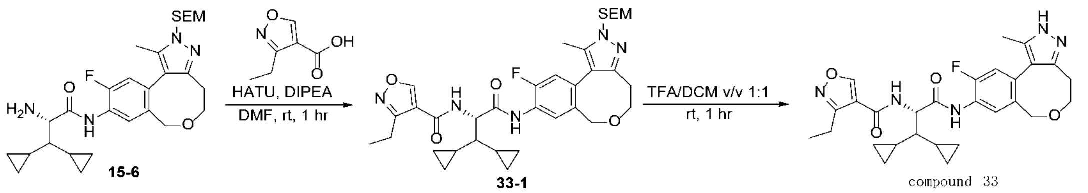


(S1) Preparation of intermediate 33-1

To a solution of 3-ethylisoxazole-4-carboxylic acid (16.01 mg, 113.48 μmol) and DIPEA (44.00 mg, 340.44 μmol, 59.30 μL) in DMF (1 mL) were added HATU (51.75 mg, 136.17 μmol) and intermediate 15-6. After addition, the reaction mixture was stirred at room temperature for 1 h. Upon completion, water (3 mL) was added to the reaction mixture to precipitate a large amount of white solid. The solid was collected by filtration, washed with water, and dried to obtain 30 mg (46.02 μmol) of intermediate 33-1 (yield: 40.56%). MS (ESI) m/z = 652 $[M+1]^+$.

(S2) Preparation of compound 33

To a solution of intermediate 33-1 (30 mg, 46.02 μmol) in DCM (1 mL) was added TFA (1 mL). After addition, the reaction mixture was stirred at room temperature for 1 h. Upon completion, the reaction mixture was concentrated to dryness under reduced pressure and purified by preparative MPLC to obtain 13 mg (20.40 μmol) of compound 33 (yield: 44.33%). MS (ESI) m/z = 522 $[M+1]^+$. $^1$H NMR (600 MHz, Methanol-$d_4$) δ 9.13 (s, 1H), 8.05 (d, *J* = 7.9 Hz, 1H), 7.22 (d, *J* = 11.3 Hz, 1H), 5.01 (dd, *J* = 7.3, 1.5 Hz, 1H), 4.33 (s, 2H), 3.78 (s, 2H), 2.93 (q, *J* = 7.5 Hz, 2H), 2.73 (s, 2H), 2.31 (s, 3H), 1.28 (t, *J* = 7.6, 1.5 Hz, 3H), 0.98 - 0.84 (m, 2H), 0.83 - 0.76 (m, 1H), 0.61 - 0.54 (m, 1H), 0.54 - 0.40 (m, 3H), 0.39 - 0.23 (m, 4H).

**Examples 34-36 Preparation of compounds 34-36**

The preparation of compounds 34-36 was performed according to the synthesis method in Example 33, in which 3-ethylisoxazole-4-carboxylic acid in step (S1) was correspondingly replaced with an isoxazolecarboxylic acid from the following table, with all other reagents and procedures remaining identical.

| Isoxazolecarbo xylic acid | Structure of compound | $^1$HNMR and/or MS (ESI) |
|---|---|---|
| 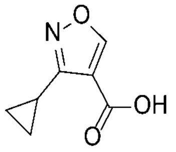 | 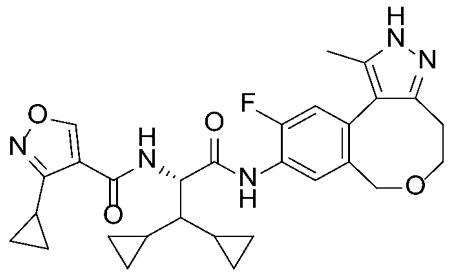 compound 34 | MS (ESI) m/z =534 $[M+1]^+$ $^1$H NMR (600 MHz, Methanol-$d_4$) δ 9.11 (s, 1H), 8.07 (d, *J* = 7.9 Hz, 1H), 7.24 (d, *J* = 11.4 Hz, 1H), 5.07 (dd, *J* = 7.2, 3.9 Hz, 1H), 4.34 (s, 2H), 3.75 (s, 2H), 2.75 (s, 2H), 2.44 - 2.36 (m, 1H), 2.33 (s, 3H), 1.13 - 1.05 (m, 2H), 1.04 - 0.99 (m, 2H), 0.99 - 0.87 (m, 2H), 0.82 (td, *J* = 9.4, 7.0 Hz, 1H), 0.63 - 0.43 (m, 4H), 0.41 - 0.27 (m, 4H). |
| 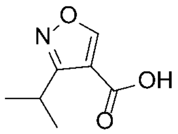 | 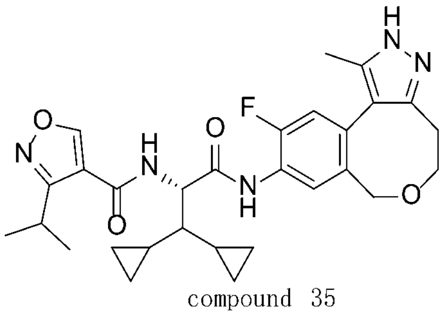 compound 35 | MS (ESI) m/z =536 $[M+1]^+$ $^1$H NMR (600 MHz, Methanol-$d_4$) δ 9.10 (s, 1H), 8.03 (d, *J*= 7.9 Hz, 1H), 7.20 (d, *J* = 11.3 Hz, 1H), 4.99 (dd, *J* = 7.1, 2.2 Hz, 1H), 4.32 (s, 2H), 3.88 - 3.58 (m, 2H), 3.50 (hept, *J* = 7.0 Hz, 1H), 2.71 (s, 2H), 2.29 (s, 3H), 1.33 (dd, *J* = 7.0, 1.3 Hz, 6H), 0.99 - 0.84 (m, 2H), 0.84 - 0.75 (m, 1H), 0.63 - 0.39 (m, 4H), 0.39 - 0.24 (m, 4H). |
| 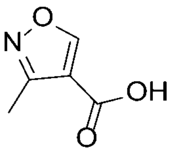 | 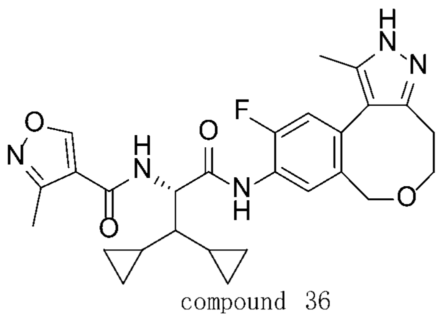 compound 36 | MS (ESI) m/z =508 $[M+1]^+$ $^1$H NMR (600 MHz, Methanol-$d_4$) δ 8.06 (d, *J* = 9.1 Hz, 2H), 7.24 (d, *J* = 11.4 Hz, 1H), 4.58 (d, *J* = 6.0 Hz, 1H), 4.36 (s, 2H), 3.70 (s, 2H), 2.73 (s, 2H), 2.31 (s, 3H), 2.19 (s, 3H), 0.93 - 0.87 (m, 1H), 0.84 - 0.74 (m, 2H), 0.67 - 0.61 (m, 1H), 0.61 - 0.48 (m, 3H), 0.39 - 0.31 (m, 3H), 0.29 - 0.23 (m, 1H). |

**Example 37 Preparation of compounds 37a and 37b**

A structure of compound 37a/37b is as follows:

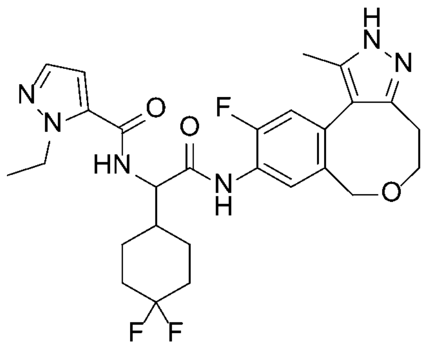

compound 37a & 37b

The preparation of compounds 37a and 37b was performed according to the synthesis method in Example 11, in which intermediate 1-4 in step (S1) was replaced with intermediate 15-4, with all other reagents and procedures remaining identical. After SEM group was removed, the crude product was subjected to purification by MPLC ($H_2O/CH_3CN$, 0.05% $NH_4HCO_3$) and separation by SFC to obtain compounds 37a and 37b, in which SFC was performed using a Daicel

CHIRALPAK AD-3 column (3 μm, 150 mm × 3 mm) with $CO_2$ (as mobile phase A) and isopropanol (as mobile phase B) (A/B = 75/25), at a flow rate of 1 mL/min and a column temperature of 40°C.

For compound 37a (P1, SFC $t_R$ = 2.114), MS (ESI) m/z = 545 $[M+1]^+$. $^1H$ NMR (600 MHz, Methanol-$d_4$) δ 7.98 (d, *J* = 7.9 Hz, 1H), 7.50 (d, *J* = 2.1 Hz, 1H), 7.19 (d, *J* = 11.2 Hz, 1H), 6.88 (d, *J* = 2.2 Hz, 1H), 4.67 (d, *J* = 8.7 Hz, 1H), 4.59 - 4.50 (m, 2H), 4.32 (s, 2H), 3.86 - 3.53 (m, 2H), 2.70 (s, 2H), 2.27 (s, 3H), 2.16 - 2.05 (m, 3H), 2.02 - 1.96 (m, 1H), 1.91 (d, *J* = 13.6 Hz, 1H), 1.88 - 1.74 (m, 2H), 1.62 - 1.45 (m, 2H), 1.38 (t, *J* = 7.2 Hz, 3H).

For compound 37b (P2, SFC $t_R$ = 5.415), MS (ESI) m/z = 545 $[M+1]^+$. $^1H$ NMR (600 MHz, Methanol-$d_4$) δ 7.98 (d, *J* = 7.9 Hz, 1H), 7.50 (d, *J* = 2.1 Hz, 1H), 7.19 (d, *J* = 11.1 Hz, 1H), 6.88 (d, *J* = 2.1 Hz, 1H), 4.68 (dd, *J* = 8.7, 1.3 Hz, 1H), 4.54 (q, *J* = 7.2 Hz, 2H), 4.31 (s, 2H), 3.71 (s, 2H), 2.69 (s, 2H), 2.27 (s, 3H), 2.15 - 2.04 (m, 3H), 2.02 - 1.96 (m, 1H), 1.93 - 1.88 (m, 1H), 1.80 (ddd, *J* = 33.2, 14.1, 8.3 Hz, 2H), 1.63 - 1.45 (m, 2H), 1.38 (t, *J* = 7.2 Hz, 3H).

**Example 38 Preparation of compounds 38a and 38b**

A structure of compound 38a/38b is as follows:

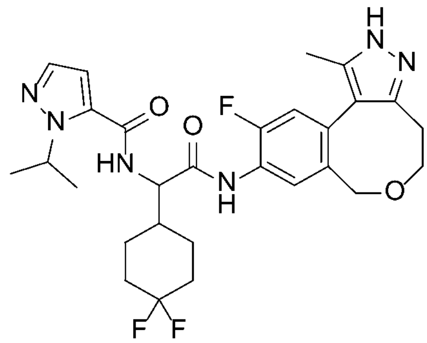

compound 38a & 38b

The preparation of compounds 38a and 38b was performed according to the synthesis method in Example 11, in which intermediate 1-4 in step (S1) was replaced with intermediate 15-4, and 1-ethyl-1H-pyrazole-5-carboxylic acid in step (S3) was replaced with 1-isopropyl-1H-pyrazole-5-carboxylic acid, with all other reagents and procedures remaining identical. After SEM group was removed, the crude product was subjected to purification by MPLC ($H_2O/CH_3CN$, 0.05% $NH_4HCO_3$) and separation by SFC to obtain compounds 38a and 38b, in which SFC was performed using a Daicel CHIRALPAK AD-3 column (3 μm, 150 mm × 3 mm) with $CO_2$ (as mobile phase A) and isopropanol (as mobile phase B) (A/B = 75/25), at a flow rate of 1 mL/min and a column temperature of 40°C.

For compound 38a (P1, SFC $t_R$ = 1.771), MS (ESI) m/z = 559 $[M+1]^+$. $^1H$ NMR (600 MHz, Methanol-$d_4$) δ 7.98 (d, *J* = 7.9 Hz, 1H), 7.51 (d, *J* = 2.0 Hz, 1H), 7.19 (d, *J* = 11.3 Hz, 1H), 6.81 (d, *J* = 2.0 Hz, 1H), 5.45 - 5.35 (m, 1H), 4.67 (dd, *J* = 8.6, 1.4 Hz, 1H), 4.30 (s, 2H), 3.72 (s, 2H), 2.69 (s, 2H), 2.27 (s, 3H), 2.16 - 2.04 (m, 3H), 1.99 (d, *J* = 13.6 Hz, 1H), 1.91 (d, *J* = 13.2 Hz, 1H), 1.88 - 1.75 (m, 2H), 1.63 - 1.49 (m, 2H), 1.45 (t, *J* = 6.7 Hz, 6H).

For compound 38b (P2, SFC $t_R$ = 2.114), MS (ESI) m/z = 559 $[M+1]^+$. $^1H$ NMR (600 MHz, Methanol-$d_4$) δ 7.98 (d, *J* = 7.9 Hz, 1H), 7.51 (d, *J* = 2.0 Hz, 1H), 7.19 (d, *J* = 11.3 Hz, 1H), 6.81 (d, *J* = 2.1 Hz, 1H), 5.45 - 5.35 (m, 1H), 4.68 (dd, *J* = 8.5, 1.1 Hz, 1H), 4.30 (s, 2H), 3.92 - 3.52 (m, 2H), 2.69 (s, 2H), 2.27 (s, 3H), 2.16 - 2.04 (m, 3H), 2.02 - 1.96 (m, 1H), 1.91 (d, *J* = 13.5 Hz, 1H), 1.81 (d, *J* = 32.3 Hz, 2H), 1.63 - 1.49 (m, 2H), 1.45 (t, *J* = 6.9 Hz, 6H).

**Example 39 Preparation of compounds 39a and 39b**

A preparation of compounds 39a and 39b is illustrated as follows:

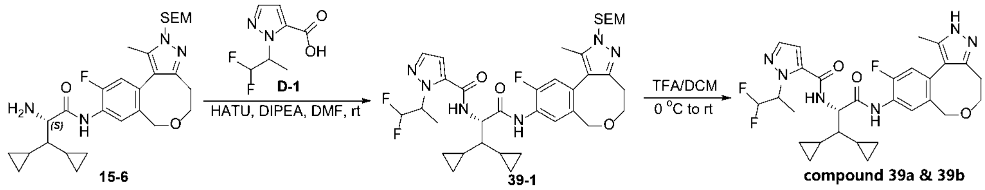


The preparation of compounds 39a and 39b was performed according to the synthesis method in Example 33, in which 3-ethylisoxazole-4-carboxylic acid in step (S1) was replaced with intermediate D-1, with all other reagents and

procedures remaining identical. After SEM group was removed, the crude product was subjected to purification by MPLC (under basic conditions) and separation by SFC to obtain compounds 39a and 39b, in which SFC was performed using a Daicel CHIRALPAK AD-3 column (3 μm, 150 mm × 3 mm) with $CO_2$ (as mobile phase A) and ethanol (as mobile phase B) (A/B = 77/23), at a flow rate of 1 mL/min and a column temperature of 40°C.

For compound 38a (Pl, SFC $t_R$ = 2.467), MS (ESI) m/z = 571 $[M+1]^+$. $^1H$ NMR (600 MHz, DMSO-$d_6$) δ 12.47 (d, *J* = 24.5 Hz, 1H), 10.02 (s, 1H), 8.63 (d, *J* = 8.8 Hz, 1H), 7.93 (d, *J* = 8.2 Hz, 1H), 7.64 (d, *J* = 2.0 Hz, 1H), 7.25 (s, 1H), 7.11 (d, *J* = 2.0 Hz, 1H), 6.24 (td, *J* = 55.8, 5.2 Hz, 1H), 5.83 - 5.72 (m, 1H), 4.99 (t, 1H), 4.34 (s, 2H), 3.61 (s, 2H), 2.61 (s, 2H), 2.21 (s, 3H), 1.53 (d, *J* = 6.9 Hz, 3H), 0.97 - 0.83 (m, 2H), 0.79 (td, *J* = 9.4, 7.8 Hz, 1H), 0.54 - 0.45 (m, 1H), 0.43 - 0.36 (m, 2H), 0.36 - 0.28 (m, 2H), 0.28 - 0.15 (m, 3H).

For compound 39b (P2, SFC $t_R$ = 3.450), MS (ESI) m/z = 571 $[M+1]^+$. $^1H$ NMR (600 MHz, DMSO-$d_6$) δ 12.40 (s, 1H), 9.99 (s, 1H), 8.53 (d, *J* = 8.7 Hz, 1H), 7.90 (d, *J* = 8.2 Hz, 1H), 7.60 (d, *J* = 2.0 Hz, 1H), 7.21 (d, *J* = 11.6 Hz, 1H), 7.09 (d, *J* = 2.0 Hz, 1H), 6.23 (td, *J* = 55.9, 5.0 Hz, 1H), 5.82 - 5.70 (m, 1H), 4.95 (t, *J* = 8.2 Hz, 1H), 4.17 (s, 2H), 3.57 (s, 2H), 2.58 (s, 2H), 2.17 (s, 3H), 1.48 (d, *J* = 7.0 Hz, 3H), 0.93 - 0.80 (m, 2H), 0.76 (q, *J* = 8.9 Hz, 1H), 0.49 - 0.41 (m, 1H), 0.38 - 0.23 (m, 4H), 0.24 - 0.11 (m, 3H).

**Examples 40-42 Preparation of compounds 40a-42b**

The preparation of compounds 40a to 42b was performed according to the synthesis method in Example 39, in which intermediate D-1 in step (S1) was replaced with pyrazolecarboxylic acid, with all other reagents and procedures remaining identical.

| Pyrazolecarboxylic acid | Structure of compound | $^1$HNMR and/or MS (ESI) |
|---|---|---|
| 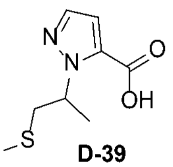 **D-39** | 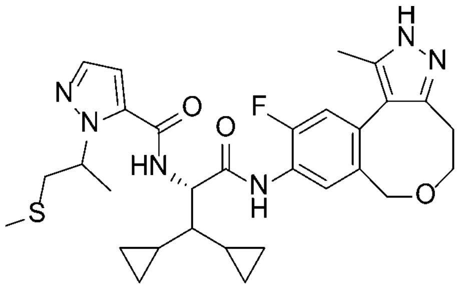 **compound 40a** | MS (ESI) m/z =581 $[M+1]^+$ $^1H$ NMR (600 MHz, Methanol-$d_4$) δ 8.04 (d, *J* = 8.0 Hz, 1H), 7.55 (d, *J* = 2.0 Hz, 1H), 7.21 - 7.17 (m, 1H), 6.79 (d, *J* = 2.0 Hz, 1H), 5.53 - 5.43 (m, 1H), 5.04 - 4.99 (m, 1H), 4.31 (s, 2H), 3.71 (s, 2H), 2.89 - 2.79 (m, 2H), 2.70 (s, 2H), 2.27 (s, 3H), 1.89 (s, 3H), 1.58 (d, *J* = 6.7 Hz, 3H), 0.94 - 0.87 (m, 2H), 0.83 (td, *J* = 9.1, 7.3 Hz, 1H), 0.59 - 0.49 (m, 3H), 0.49 - 0.37 (m, 3H), 0.31 - 0.27 (m, 2H).<br>Note: SFC was performed using a Daicel CHIRALPAK IG column (3 μm, 150 mm × 3 mm) with $CO_2$ (as mobile phase A) and ethanol (as mobile phase B) (A/B = 65/35), at a flow rate of 1 mL/min and a column temperature of 40°C. SFC $t_R$=2.397. |
| 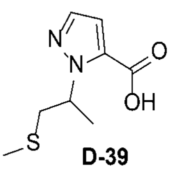 **D-39** | 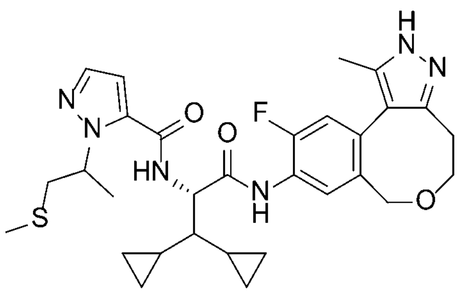 **compound 40b** | MS (ESI) m/z =581 $[M+1]^+$ $^1H$ NMR (600 MHz, Methanol-$d_4$) δ 8.04 (d, *J* = 7.9 Hz, 1H), 7.55 (d, *J* = 2.0 Hz, 1H), 7.19 (s, 1H), 6.86 (d, *J* = 2.1 Hz, 1H), 5.56 - 5.47 (m, 1H), 5.01 (d,*J* = 6.7 Hz, 1H), 4.31 (s, 2H), 3.72 (s, 2H), 2.97 (dd, *J* = 13.9, 8.7 Hz, 1H), 2.83 (dd, *J* = 13.9, 5.5 Hz, 1H), 2.70 (s, 2H), 2.29 (s, 3H), 1.93 (s, 3H), 1.56 (d, *J* = 6.7 Hz, 3H), 0.98 - 0.80 (m, 3H), 0.60 - 0.54 (m, 1H), 0.53 - 0.36 (m, 4H), 0.35 - 0.25 (m, 3H).<br>Note: SFC was performed using a Daicel CHIRALPAK IG column (3 μm, 150 mm × 3 mm) with $CO_2$ (as mobile phase A) and ethanol (as mobile phase B) (A/B = 65/35), at a flow rate of 1 mL/min and a column temperature of 40°C. SFC $t_R$=3.071. |

(continued)

| Pyrazolecarbo xylic acid | Structure of compound | $^1$HNMR and/or MS (ESI) |
|---|---|---|
| 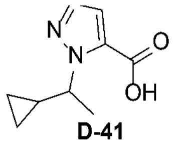 D-41 | 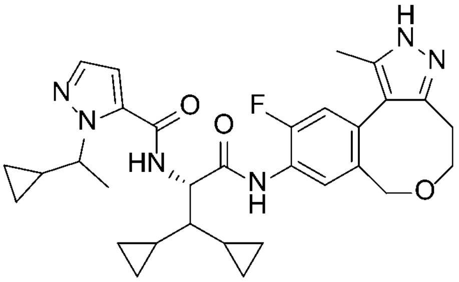 compound 41a | MS (ESI) m/z =561 [M+1]$^+$ $^1$H NMR (400 MHz, DMSO-$d_6$) $\delta$ 12.46 (d, $J$ = 21.6 Hz, 1H), 9.95 (s, 1H), 8.50 (d,$J$ = 8.8 Hz, 1H), 7.90 (d, $J$ = 8.1 Hz, 1H), 7.53 (d, $J$ = 1.9 Hz, 1H), 7.23 (d, $J$ = 13.2 Hz, 1H), 6.87 (d, $J$ = 2.0 Hz, 1H), 4.91 (t, $J$ = 8.2 Hz, 1H), 4.70 - 4.52 (m, 1H), 4.20 (s, 2H), 3.61 (s, 2H), 2.67 (p, $J$ = 1.9 Hz, 1H), 2.20 (d, $J$ = 21.6 Hz, 3H), 1.51 (d, $J$ = 6.7 Hz, 3H), 1.32 - 1.15 (m, 1H), 1.03 - 0.69 (m, 4H), 0.64 - 0.09 (m, 12H). Note: SFC was performed using a Daicel CHIRALPAK AD-3 column (3 $\mu$m, 150 mm $\times$ 3 mm) with $CO_2$ (as mobile phase A) and ethanol (as mobile phase B) (A/B = 77/23), at a flow rate of 1 mL/min and a column temperature of 40°C. SFC $t_R$=2.658. |
| 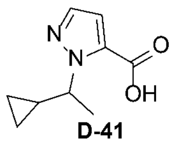 D-41 | 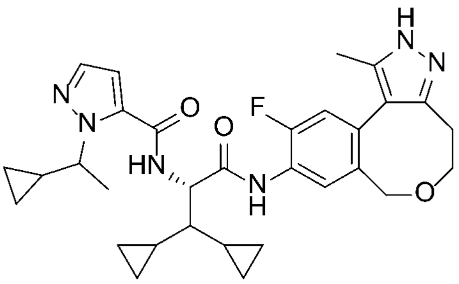 compound 41b | MS (ESI) m/z =561 [M+1]$^+$ $^1$H NMR (400 MHz, DMSO-$d_6$) $\delta$ 12.46 (d, $J$ = 21.1 Hz, 1H), 9.95 (s, 1H), 8.44 (d,$J$ = 8.7 Hz, 1H), 7.93 (d,$J$ = 8.2 Hz, 1H), 7.53 (d, $J$ = 2.0 Hz, 1H), 7.24 (s, 1H), 6.93 (d,$J$ = 2.0 Hz, 1H), 4.99 - 4.87 (m, 1H), 4.75 - 4.61 (m, 1H), 4.20 (s, 2H), 3.61 (s, 2H), 2.67 (t, $J$ = 1.9 Hz, 1H),2.19 (d, $J$ = 19.8 Hz, 3H), 1.47 (d, $J$ = 6.8 Hz, 3H), 1.38 - 1.21 (m, 1H), 1.01 - 0.73 (m, 4H), 0.62 - 0.44 (m, 2H), 0.42 - 0.16 (m, 10H). Note: SFC was performed using a Daicel CHIRALPAK AD-3 column (3 $\mu$m, 150 mm $\times$ 3 mm) with $CO_2$ (as mobile phase A) and ethanol (as mobile phase B) (A/B = 77/23), at a flow rate of 1 mL/min and a column temperature of 40°C. SFC $t_R$=3.489. |
| 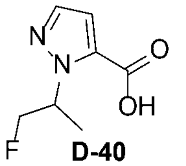 D-40 | 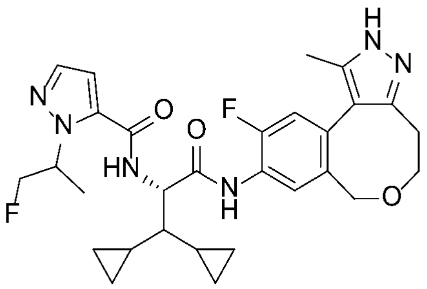 compound 42a | MS (ESI) m/z =553 [M+1]$^+$ $^1$H NMR (600 MHz, DMSO-$d_6$) $\delta$ 12.41 (s, 1H), 9.96 (s, 1H), 8.49 (d, $J$ = 8.8 Hz, 1H), 7.90 (d, $J$ = 8.2 Hz, 1H), 7.55 (d, $J$ = 2.0 Hz, 1H), 7.21 (d,$J$ = 11.7 Hz, 1H), 6.96 (d, $J$ = 2.0 Hz, 1H), 5.71 - 5.56 (m, 1H), 4.93 (t, $J$ = 8.3 Hz, 1H), 4.73 - 4.51 (m, 2H), 4.22 (s, 2H), 3.63 (s, 2H), 2.58 (s, 2H), 2.17 (s, 3H), 1.33 (dd, $J$ = 6.9, 1.6 Hz, 3H), 0.94 - 0.80 (m, 2H), 0.79 - 0.71 (m, 1H), 0.50 - 0.41 (m, 1H), 0.40 - 0.24 (m, 4H), 0.24 - 0.11 (m, 3H). Note: SFC was performed using a Daicel CHIRALPAK AD-3 column (3 $\mu$m, 150 mm $\times$ 3 mm) with $CO_2$ (as mobile phase A) and ethanol (as mobile phase B) (A/B = 77/23), at a flow rate of 1 mL/min and a column temperature of 40°C. SFC $t_R$=3.765. |

(continued)

| Pyrazolecarbo xylic acid | Structure of compound | $^1$HNMR and/or MS (ESI) |
|---|---|---|
| 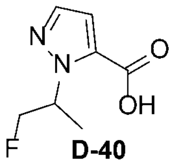 D-40 | 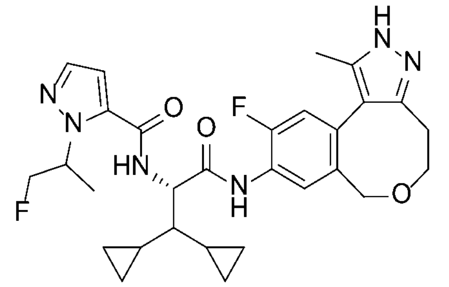 compound 42b | MS (ESI) m/z =553 [M+1]$^+$ $^1$H NMR (600 MHz, DMSO-$d_6$) δ 12.42 (s, 1H), 9.96 (s, 1H), 8.41 (d, *J* = 8.7 Hz, 1H), 7.89 (d, *J* = 8.2 Hz, 1H), 7.53 (d, *J* = 2.0 Hz, 1H), 7.20 (d, *J* = 11.6 Hz, 1H), 6.97 (d, *J* = 2.0 Hz, 1H), 5.75 - 5.60 (m, 1H), 4.92 (t, *J* = 8.2 Hz, 1H), 4.78 - 4.50 (m, 2H), 4.21 (s, 2H), 3.56 (s, 2H), 2.56 (s, 2H), 2.15 (s, 3H), 1.30 (dd, *J* = 6.9, 1.6 Hz, 3H), 0.94 - 0.78 (m, 2H), 0.74 (q, *J* = 8.9 Hz, 1H), 0.43 (s, 1H), 0.37 - 0.22 (m, 4H), 0.22 - 0.10 (m, 3H). Note: SFC was performed using a Daicel CHIRALPAK AD-3 column (3 μm, 150 mm × 3 mm) with $CO_2$ (as mobile phase A) and ethanol (as mobile phase B) (A/B = 77/23), at a flow rate of 1 mL/min and a column temperature of 40°C. SFC $t_R$=5.409. |

**Example 43 Preparation of compounds 43a, 43b and 43c**

A structure of compound 43a/43b/43c is as follows:

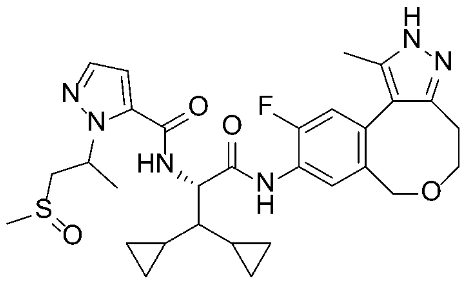

compound 43a, 43b& 43c

The preparation of compounds 43a, 43b and 43c was performed according to the synthesis method in Example 39, in which intermediate D-1 in step (S1) was replaced with intermediate D-51, with all other reagents and procedures remaining identical. After SEM group was removed, the crude product was subjected to purification by MPLC (under basic conditions) and separation by SFC to obtain compounds 43a, 43b and 43c, in which SFC was performed using a Daicel CHIRALCEL OX-3 column (3 μm, 150 mm × 3 mm) with $CO_2$ (as mobile phase A) and ethanol (as mobile phase B) (A/B = 65/35), at a flow rate of 1 mL/min and a column temperature of 40°C.

For compound 43a (P1, SFC $t_R$ = 2.467), MS (ESI) m/z = 597 [M+1]$^+$. $^1$H NMR (600 MHz, Methanol-$d_4$) δ 7.98 (d, *J* = 7.9 Hz, 1H), 7.60 (d, *J* = 2.0 Hz, 1H), 7.17 (d, *J* = 10.7 Hz, 1H), 6.83 (d, *J* = 2.0 Hz, 1H), 5.90 - 5.78 (m, 1H), 5.03 (d, *J* = 6.1 Hz, 1H), 4.31 (s, 2H), 3.71 (s, 2H), 3.59 (dd, *J* = 13.3, 10.6 Hz, 1H), 3.16 (dd, *J* = 13.3, 4.2 Hz, 1H), 2.84 - 2.62 (m, 2H), 2.57 (s, 3H), 2.28 (s, 3H), 1.63 (d, *J* = 6.7 Hz, 3H), 0.96 - 0.84 (m, 3H), 0.61 - 0.42 (m, 4H), 0.37 (dq, *J* = 9.8, 5.0 Hz, 1H), 0.29 (dtq, *J* = 23.4, 9.4, 4.7 Hz, 3H).

For compound 43b (P2, mix, SFC $t_R$ = 3.110), MS (ESI) m/z = 597 [M+1]$^+$. $^1$H NMR (600 MHz, Methanol-$d_4$) δ 8.02 (dd, *J* = 13.4, 7.9 Hz, 1H), 7.59 (s, 1H), 7.19 (s, 1H), 6.86 (dd, *J* = 15.8, 2.0 Hz, 1H), 5.88 - 5.80 (m, 1H), 5.00 (d, *J* = 7.1 Hz, 1H), 4.32 (s, 2H), 3.70 (d, *J* = 25.4 Hz, 2H), 3.56 - 3.46 (m, 1H), 3.41 - 3.32 (m, 1H), 2.63 (d, *J* = 44.0 Hz, 5H), 2.27 (d, *J* = 17.0 Hz, 3H), 1.62 (dd, *J* = 17.1, 6.7 Hz, 3H), 0.97 - 0.78 (m, 3H), 0.59 - 0.42 (m, 4H), 0.38 (dq, *J* = 9.6, 5.0 Hz, 1H), 0.29 (ddq, *J* = 22.2, 9.3, 4.8 Hz, 3H).

For compound 43c (P3, SFC $t_R$ = 3.762), MS (ESI) m/z = 597 [M+1]$^+$. $^1$H NMR (600 MHz, Methanol-$d_4$) δ 8.04 (d, *J* = 8.0 Hz, 1H), 7.58 (d, *J* = 2.0 Hz, 1H), 7.19 (d, *J* = 11.3 Hz, 1H), 6.89 (d, *J* = 2.0 Hz, 1H), 5.90 - 5.83 (m, 1H), 5.01 (d, *J* = 7.2 Hz, 1H), 4.31 (s, 2H), 3.68 (dd, *J* = 13.3, 10.5 Hz, 3H), 3.15 (dd, *J* = 13.4, 4.0 Hz, 1H), 2.89 - 2.62 (m, 2H), 2.58 (s, 3H), 2.28 (s, 3H), 1.60 (d, *J* = 6.7 Hz, 3H), 0.96 - 0.82 (m, 3H), 0.58 - 0.43 (m, 4H), 0.37 (tdd, *J* = 5.3, 3.6, 1.9 Hz, 1H), 0.32 - 0.25 (m,

3H).

## Example 44 Preparation of compounds 44a and 44b

A preparation of compounds 44a and 44b is illustrated as follows:

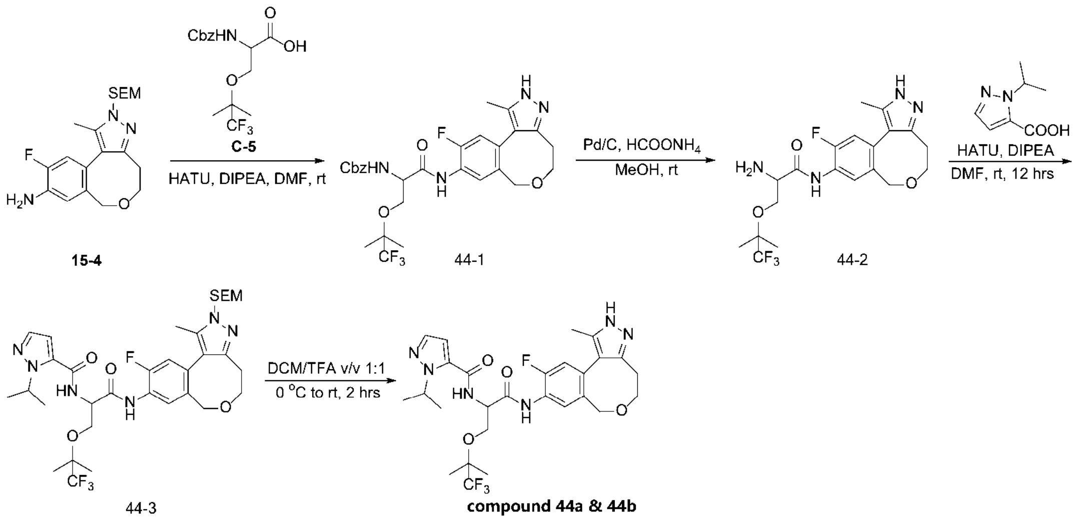


15-4 44-1 44-2 44-3 compound 44a & 44b

The preparation of compounds 44a and 44b was performed according to the synthesis method in Example 15, in which intermediate C-1 in step (S5) was replaced with intermediate C-5, with all other reagents and procedures remaining identical. After SEM group was removed, the crude product was subjected to purification by MPLC (under basic conditions) and separation by SFC to obtain compounds 44a and 44b, in which SFC was performed using a Daicel CHIRALPAK AD-3 column (3 μm, 150 mm × 3 mm) with $CO_2$ (as mobile phase A) and ethanol (as mobile phase B) (A/B = 80/20), at a flow rate of 1 mL/min and a column temperature of 40°C.

For compound 44a (P1, SFC $t_R$ = 3.283), MS (ESI) m/z = 581 $[M+1]^+$. $^1H$ NMR (600 MHz, Methanol-$d_4$) δ 8.04 (d, *J* = 7.9 Hz, 1H), 7.52 (d, *J* = 2.1 Hz, 1H), 7.18 (s, 1H), 6.80 (d, *J* = 2.0 Hz, 1H), 5.43 (h, *J* = 6.7 Hz, 1H), 4.94 (t, *J* = 6.0 Hz, 1H), 4.56 - 4.10 (m, 2H), 4.04 - 3.95 (m, 2H), 3.87 - 3.57 (m, 2H), 2.70 (s, 2H), 2.27 (d, *J* = 25.3 Hz, 3H), 1.49 - 1.44 (m, 6H), 1.42 (s, 6H).

For compound 44b (P2, SFC $t_R$ = 5.063), MS (ESI) m/z = 581 $[M+1]^+$. $^1H$ NMR (600 MHz, Methanol-$d_4$) δ 8.04 (d, *J* = 7.9 Hz, 1H), 7.52 (d, *J* = 2.1 Hz, 1H), 7.19 (s, 1H), 6.80 (d, *J* = 2.0 Hz, 1H), 5.47 - 5.40 (m, 1H), 4.94 (t, *J* = 6.0 Hz, 1H), 4.30 (s, 2H), 4.04 - 3.95 (m, 2H), 3.84 - 3.56 (m, 2H), 2.70 (s, 2H), 2.29 (s, 3H), 1.46 (dd, *J* = 6.6, 4.2 Hz, 6H), 1.42 (s, 6H).

## Examples 45-50 Preparation of compounds 45-50

The preparation of compounds 45 to 50 was performed according to the synthesis method in Example 15, in which intermediate C-1 in step (S5) was replaced with carboxylic acid intermediate, and 1-isopropyl-1H-pyrazole-5-carboxylic acid was correspondingly replaced with a pyrazolecarboxylic acid from the following table or unchanged, with all other reagents and procedures remaining identical.

| Carboxylic acid intermediate + pyrazolecarboxylic acid | Structure of compound | $^1$HNMR and/or MS (ESI) |
|---|---|---|
| 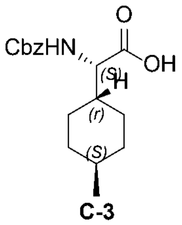 C-3 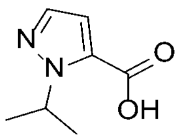 | 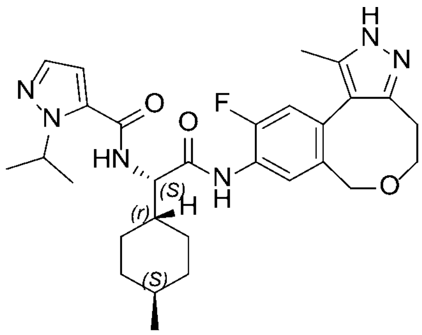 **compound 45** | MS (ESI) m/z =537 [M+1]$^+$ $^1$H NMR (600 MHz, Methanol-$d_4$) δ 7.98 (d, $J$ = 8.0 Hz, 1H), 7.51 (d,$J$ = 2.0 Hz, 1H), 7.18 (d, $J$ = 11.2 Hz, 1H), 6.80 (d, $J$ = 2.0 Hz, 1H), 5.44 - 5.34 (m, 1H), 4.56 (d, $J$ = 8.4 Hz, 1H), 4.50 - 4.03 (m, 2H), 3.71 (s, 2H), 2.69 (s, 2H), 2.27 (s, 3H), 2.02 - 1.73 (m, 5H), 1.45 (dd, $J$ = 7.9, 6.7 Hz, 6H), 1.42 - 1.35 (m, 1H), 1.34 - 1.26 (m, 1H), 1.25 - 1.16 (m, 1H), 1.05 - 0.93 (m, 2H), 0.91 (d, $J$ = 6.5 Hz, 3H). |
| 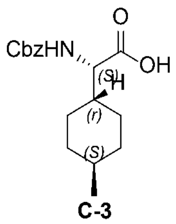 C-3 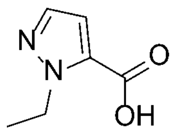 | 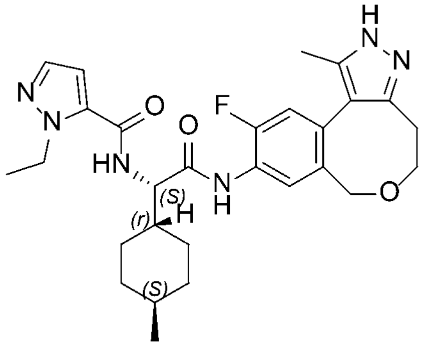 **compound 46** | MS (ESI) m/z =523 [M+1]$^+$ $^1$H NMR (600 MHz, Methanol-$d_4$) δ 7.98 (d, $J$ = 8.0 Hz, 1H), 7.49 (d, $J$ = 2.1 Hz, 1H), 7.18 (d, $J$ = 11.2 Hz, 1H), 6.87 (d, $J$ = 2.1 Hz, 1H), 4.61 - 4.50 (m, 3H), 4.30 (s, 2H), 3.71 (s, 2H), 2.69 (s, 2H), 2.27 (s, 3H), 1.98 - 1.86 (m, 2H), 1.86 - 1.76 (m, 3H), 1.38 (t, $J$ = 7.2 Hz, 4H), 1.33 - 1.25 (m, 1H), 1.23 - 1.14 (m, 1H), 1.05 - 0.94 (m, 2H), 0.91 (d, $J$ = 6.5 Hz, 3H). |
| 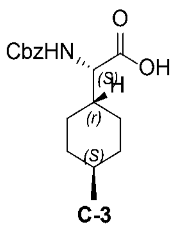 C-3 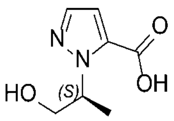 D-44 | 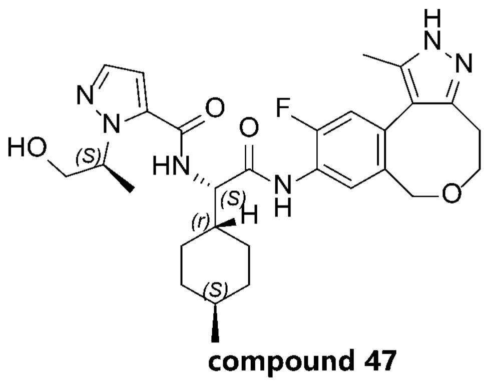 **compound 47** | MS (ESI) m/z =553 [M+1]$^+$ $^1$H NMR (600 MHz, Methanol-$d_4$) δ 7.98 (d, $J$ = 7.9 Hz, 1H), 7.53 (d, $J$ = 2.0 Hz, 1H), 7.18 (s, 1H), 6.80 (d, $J$ = 2.1 Hz, 1H), 5.39 - 5.26 (m, 1H), 4.58 (d, $J$ = 8.2 Hz, 1H), 4.31 (s, 2H), 3.84 (dd, $J$ = 11.1, 8.3 Hz, 1H), 3.76 (dd, $J$ = 11.1, 5.0 Hz, 3H), 2.69 (s, 2H), 2.28 (s, 3H), 2.00 - 1.93 (m, 1H), 1.92 - 1.71 (m, 4H), 1.44 (d, $J$ = 6.8 Hz, 3H), 1.41 - 1.26 (m, 2H), 1.26 - 1.15 (m, 1H), 1.05 - 0.93 (m, 2H), 0.91 (d, $J$= 6.5 Hz, 3H). |
| 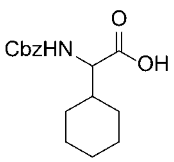 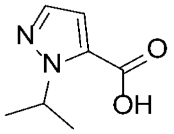 | 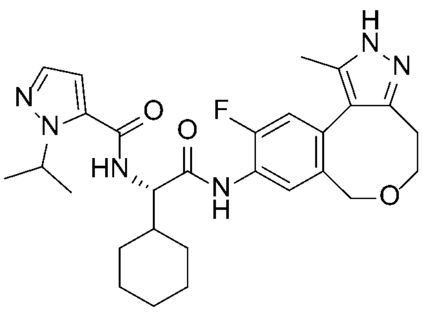 **compound 48** | MS (ESI) m/z =523 [M+1]$^+$ $^1$H NMR (600 MHz, Methanol-$d_4$) δ 7.98 (d, $J$ = 7.9 Hz, 1H), 7.50 (d,$J$ = 2.0 Hz, 1H), 7.18 (s, 1H), 6.80 (d, $J$ = 2.1 Hz, 1H), 5.44 - 5.34 (m, 1H), 4.58 (d, $J$ = 8.4 Hz, 1H), 4.47 - 4.06 (m, 2H), 3.71 (s, 2H), 2.69 (s, 2H), 2.27 (s, 3H), 1.98 - 1.90 (m, 2H), 1.86 - 1.78 (m, 3H), 1.71 (dt, $J$ = 12.6, 3.5 Hz, 1H), 1.45 (dd, $J$ = 8.5, 6.6 Hz, 6H), 1.39 - 1.22 (m, 4H), 1.22 - 1.12 (m, 1H). |

(continued)

| Carboxylic acid intermediate + pyrazolecarbo xylic acid | Structure of compound | 1HNMR and/or MS (ESI) |
|---|---|---|
| 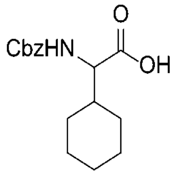 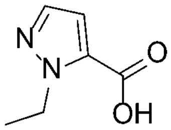 | 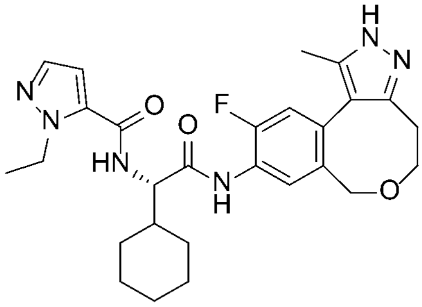 compound 49 | MS (ESI) m/z =509 [M+1]+ $^1$H NMR (600 MHz, Methanol-$d_4$) δ 7.97 (d, *J* = 7.9 Hz, 1H), 7.49 (d,*J* = 2.1 Hz, 1H), 7.18 (s, 1H), 6.87 (d, *J* = 2.1 Hz, 1H), 4.66 - 4.46 (m, 3H), 4.32 (s, 2H), 3.71 (s, 2H), 2.69 (s, 2H), 2.28 (s, 3H), 1.99 - 1.90 (m, 2H), 1.86 - 1.79 (m, 3H), 1.74 - 1.68 (m, 1H), 1.38 (t,*J* = 7.2 Hz, 3H), 1.36 - 1.29 (m, 2H), 1.25 (td, *J* = 12.8, 12.4, 3.2 Hz, 2H), 1.21 - 1.12 (m, 1H). |
| 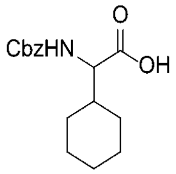 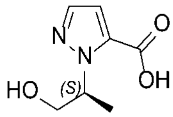 D-44 | 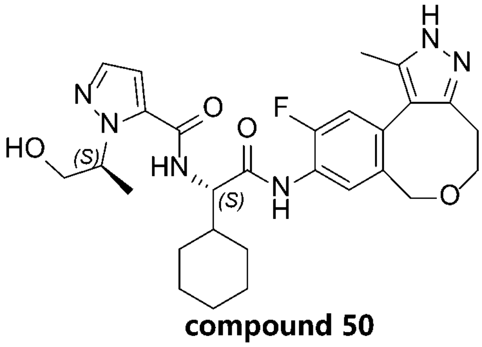 compound 50 | MS (ESI) m/z =539 [M+1]+ $^1$H NMR (600 MHz, Methanol-$d_4$) δ 7.97 (d, *J* = 7.9 Hz, 1H), 7.54 (d,*J* = 2.0 Hz, 1H), 7.18 (s, 1H), 6.80 (d, *J* = 2.1 Hz, 1H), 5.34 - 5.25 (m, 1H), 4.59 (d, *J* = 8.2 Hz, 1H), 4.50 - 4.10 (m, 2H), 3.84 (dd, *J* = 11.1, 8.3 Hz, 1H), 3.76 (dd, *J* = 11.1, 5.0 Hz, 2H), 2.69 (s, 2H), 2.28 (s, 3H), 2.00 - 1.91 (m, 2H), 1.85 - 1.78 (m, 3H), 1.71 (d, *J* = 12.5 Hz, 1H), 1.44 (d, *J* = 6.8 Hz, 3H), 1.39 - 1.13 (m, 6H). |

**Examples 51-52 Preparation of compounds 51 and 52**

The preparation of compounds 51 and 52 was performed according to the synthesis method in Example 1, in which 1-isopropyl-1H-pyrazole-5-carboxylic acid in step (S7) was correspondingly replaced with a pyrazolecarboxylic acid from the following table, with all other reagents and procedures remaining identical.

| Pyrazolecarbo xylic acid | Structure of compound | 1HNMR and/or MS (ESI) |
|---|---|---|
| 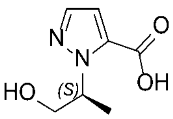 D-44 | 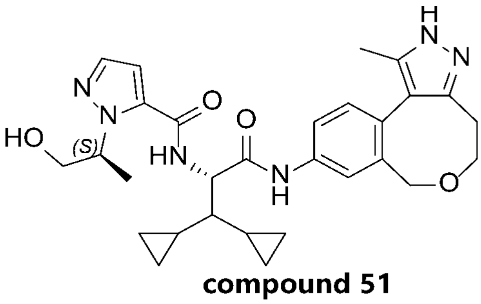 compound 51 | MS (ESI) m/z =533 [M+1]+ $^1$H NMR (600 MHz, Methanol-$d_4$) δ 7.72 - 7.65 (m, 2H), 7.55 (d, *J* = 2.0 Hz, 1H), 7.40 - 7.27 (m, 1H), 6.81 (d, *J* = 2.0 Hz, 1H), 5.36 - 5.27 (m, 1H), 4.91 (d, *J* = 7.4 Hz, 1H), 4.33 (s, 2H), 3.93 - 3.52 (m, 4H), 2.69 (s, 2H), 2.26 (s, 3H), 1.44 (d, *J* = 6.8 Hz, 3H), 0.96 - 0.76 (m, 3H), 0.61 - 0.23 (m, 8H). |

(continued)

| Pyrazolecarboxylic acid | Structure of compound | $^1$HNMR and/or MS (ESI) |
|---|---|---|
| 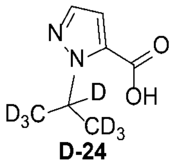 D-24 | 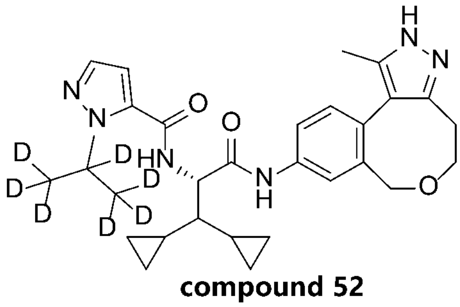 compound 52 | MS (ESI) m/z =524 [M+1]$^+$ $^1$H NMR (600 MHz, Methanol-$d_4$) δ 7.72 - 7.65 (m, 2H), 7.52 (d, *J* = 2.1 Hz, 1H), 7.41 - 7.27 (m, 1H), 6.80 (d, *J* = 2.1 Hz, 1H), 4.91 (d, *J* = 7.7 Hz, 1H), 4.32 (s, 2H), 3.72 (s, 2H), 2.69 (s, 2H), 2.26 (s, 3H), 0.95 - 0.74 (m, 3H), 0.59 - 0.33 (m, 5H), 0.27 (ddp, *J* = 23.8, 9.4, 4.9 Hz, 3H). |

**Examples 53-54 Preparation of compounds 53 and 54**

The preparation of compounds 53 and 54 was performed according to the synthesis method in Example 12, in which 1-isopropyl-1H-pyrazole-5-carboxylic acid in step (S8) was correspondingly replaced with a pyrazolecarboxylic acid from the following table, with all other reagents and procedures remaining identical.

| Pyrazolecarboxylic acid | Structure of compound | $^1$HNMR and/or MS (ESI) |
|---|---|---|
| 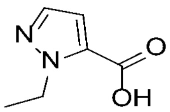 | 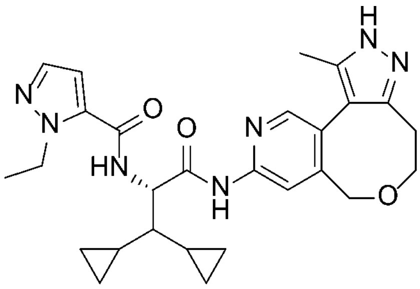 compound 53 | MS (ESI) m/z =504 [M+1]$^+$ $^1$H NMR (600 MHz, DMSO-$d_6$) δ 12.52 (d, *J* = 18.4 Hz, 1H), 10.71 (s, 1H), 8.44 (d, *J* = 8.6 Hz, 1H), 8.32 (d, *J* = 32.6 Hz, 1H), 8.13 (s, 1H), 7.50 (d, *J* = 2.0 Hz, 1H), 6.99 (d, *J* = 2.1 Hz, 1H), 4.93 (t, *J* = 8.1 Hz, 1H), 4.51 - 4.43 (m, 2H), 4.38 - 3.91 (m, 2H), 3.69 (s, 2H), 2.62 (s, 2H), 2.22 (d, *J* = 28.3 Hz, 3H), 1.28 (t, *J* = 7.1 Hz, 3H), 1.01 - 0.91 (m, 1H), 0.91 - 0.82 (m, 1H), 0.82 - 0.74 (m, 1H), 0.52 - 0.43 (m, 1H), 0.42 - 0.34 (m, 1H), 0.34 - 0.27 (m, 2H), 0.26 - 0.15 (m, 4H). |
| 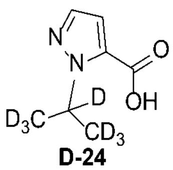 D-24 | 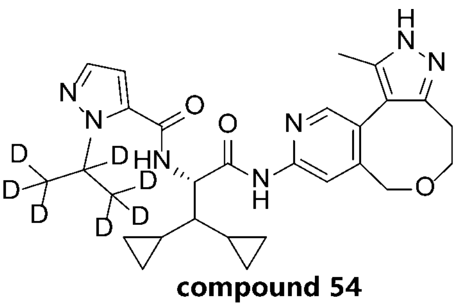 compound 54 | MS (ESI) m/z =525 [M+1]$^+$ $^1$H NMR (600 MHz, Methanol-$d_4$) δ 8.33 (d, *J* = 36.2 Hz, 1H), 8.22 (s, 1H), 7.52 (d, *J* = 2.1 Hz, 1H), 6.81 (d, *J* = 2.1 Hz, 1H), 4.99 *(d, J*= 7.2 Hz, 1H), 4.34 (s, 2H), 3.75 (s, 2H), 2.70 (s, 2H), 2.31 (s, 3H), 0.97 - 0.79 (m, 3H), 0.62 - 0.43 (m, 3H), 0.42 - 0.18 (m, 5H). |

**Examples 55-109 Preparation of compounds 55-109**

The preparation of compounds 55 to 109 was performed according to the synthesis method in Example 15, in which 1-isopropyl-1H-pyrazole-5-carboxylic acid in step (S7) was correspondingly replaced with a pyrazolecarboxylic acid from the following table, with all other reagents and procedures remaining identical.

| Pyrazolecarboxylic acid | Structure of compound | $^1$HNMR and/or MS (ESI) |
|---|---|---|
| 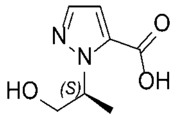 **D-44** | 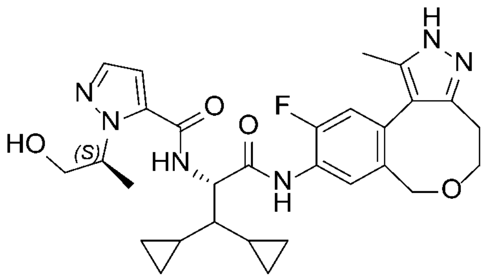 **compound 55** | MS (ESI) m/z =551 [M+1]$^+$ $^1$H NMR (400 MHz, Methanol-$d_4$) δ 8.04 (d, $J$ = 8.0 Hz, 1H), 7.55 (d, $J$ = 2.1 Hz, 1H), 7.19 (d, $J$ = 11.3 Hz, 1H), 6.81 (d, $J$ = 2.0 Hz, 1H), 5.38 - 5.26 (m, 1H), 5.00 (d, $J$ = 6.8 Hz, 1H), 4.32 (d, $J$ = 6.5 Hz, 1H), 3.87 (dd, $J$ = 11.0, 8.1 Hz, 1H), 3.77 (dd, $J$ = 11.0, 5.2 Hz, 3H), 2.70 (s, 2H), 2.28 (s, 3H), 1.45 (d, $J$ = 6.8 Hz, 3H), 1.28 (s, 1H), 0.99 - 0.77 (m, 3H), 0.57 (t, $J$ = 8.6 Hz, 1H), 0.53 - 0.40 (m, 3H), 0.40 - 0.33 (m, 1H), 0.30 (p, $J$ = 4.8 Hz, 3H). |
| 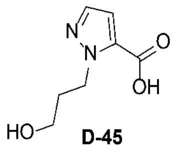 **D-45** | 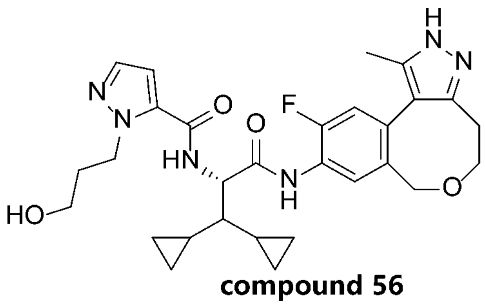 **compound 56** | MS (ESI) m/z =551 [M+1]$^+$ $^1$H NMR (600 MHz, Methanol-$d_4$) δ 8.03 (d, $J$ = 7.9 Hz, 1H), 7.53 (d, $J$ = 2.1 Hz, 1H), 7.19 (s, 1H), 6.87 (d, $J$ = 2.0 Hz, 1H), 5.01 (d, $J$ = 7.2 Hz, 1H), 4.61 (td, $J$ = 6.8, 1.4 Hz, 2H), 4.31 (s, 2H), 3.71 (s, 2H), 3.50 (t, $J$ = 6.2 Hz, 2H), 2.70 (s, 2H), 2.28 (s, 3H), 2.04 (p, $J$ = 6.6 Hz, 2H), 0.97 - 0.79 (m, 3H), 0.60 - 0.54 (m, 1H), 0.53 - 0.42 (m, 3H), 0.38 (dq, $J$ = 9.7, 4.9 Hz, 1H), 0.29 (dddd, $J$ = 14.6, 13.4, 7.3, 4.1 Hz, 3H). |
| 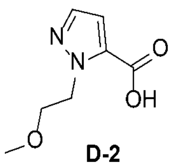 **D-2** | 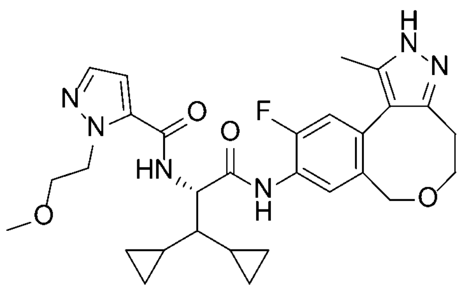 **compound 57** | MS (ESI) m/z =551 [M+1]$^+$ $^1$H NMR (400 MHz, Methanol-$d_4$) δ 8.03 (d, $J$ = 8.0 Hz, 1H), 7.53 (d, $J$ = 2.1 Hz, 1H), 7.19 (d, $J$ = 11.4 Hz, 1H), 6.84 (d, $J$ = 2.1 Hz, 1H), 5.02 (d, $J$ = 6.6 Hz, 1H), 4.75 (dt, $J$ = 14.1, 5.6 Hz, 1H), 4.66 (dt, $J$ = 14.1, 5.3 Hz, 1H), 4.31 (s, 2H), 3.73 (t, $J$ = 5.4 Hz, 4H), 3.26 (s, 3H), 2.70 (s, 2H), 2.28 (s, 3H), 0.87 (ddt, $J$ = 21.1, 9.0, 6.6 Hz, 3H), 0.59 - 0.43 (m, 4H), 0.40 - 0.26 (m, 4H). |

(continued)

| Pyrazolecarboxylic acid | Structure of compound | $^1$HNMR and/or MS (ESI) |
|---|---|---|
| 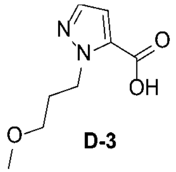 D-3 | 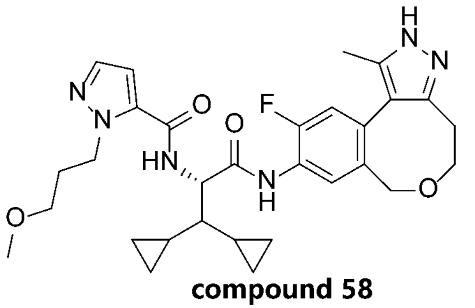 compound 58 | MS (ESI) m/z =565 [M+1]$^+$<br>$^1$H NMR (600 MHz, Methanol-$d_4$) δ 8.04 (d, *J* = 7.9 Hz, 1H), 7.51 (d, *J* = 2.1 Hz, 1H), 7.19 (s, 1H), 6.87 (d, *J* = 2.1 Hz, 1H), 5.01 (d, *J* = 7.2 Hz, 1H), 4.68 - 4.53 (m, 2H), 4.31 (s, 2H), 3.71 (s, 2H), 3.38 - 3.32 (m, 2H), 3.26 (s, 3H), 2.70 (s, 2H), 2.28 (s, 3H), 2.06 (p, *J*= 6.6 Hz, 2H), 0.95 - 0.79 (m, 3H), 0.59 - 0.54 (m, 1H), 0.53 - 0.42 (m, 3H), 0.41 - 0.36 (m, 1H), 0.34 - 0.23 (m, 3H). |
| 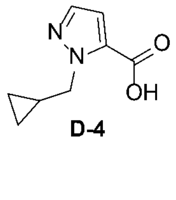 D-4 | 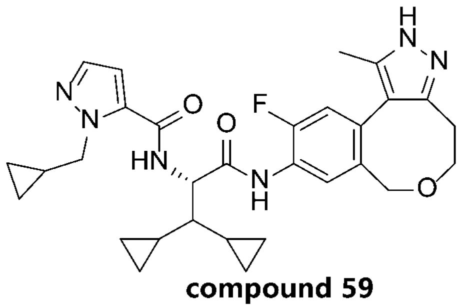 compound 59 | MS (ESI) m/z =547 [M+1]$^+$<br>$^1$H NMR (400 MHz, DMSO-$d_6$) δ 12.34 (s, 1H), 9.99 (s, 1H), 8.49 (d, *J* = 8.8 Hz, 1H), 7.92 (d, *J* = 8.2 Hz, 1H), 7.50 (d, *J* = 2.0 Hz, 1H), 7.24 (d, *J* = 11.7 Hz, 1H), 7.00 (d, *J* = 2.1 Hz, 1H), 4.96 (dd, *J* = 8.7, 7.5 Hz, 1H), 4.37 (dq, *J* = 14.3, 6.8 Hz, 2H), 4.25 (d, *J* = 40.6 Hz, 2H), 3.61 (s, 2H), 2.69 - 2.52 (m, 2H), 2.20 (s, 3H), 1.22 (ddt, *J* = 10.2, 7.4, 3.7 Hz, 1H), 1.00 - 0.75 (m, 3H), 0.49 (tq, *J* = 8.4, 3.7 Hz, 1H), 0.39 (td, *J*= 7.4, 6.5, 3.1 Hz, 4H), 0.32 (tq, *J* = 6.3, 3.4 Hz, 4H), 0.21 (dh, *J* = 12.8, 4.2 Hz, 3H). |
| 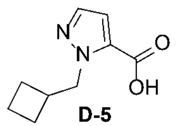 D-5 | 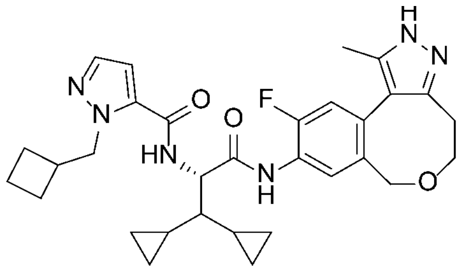 compound 60 | MS (ESI) m/z =561 [M+1]$^+$<br>$^1$H NMR (400 MHz, Methanol-$d_4$) δ 8.03 (d, *J* = 8.0 Hz, 1H), 7.49 (d, *J* = 2.1 Hz, 1H), 7.18 (d, *J* = 11.0 Hz, 1H), 6.85 (d, *J* = 2.1 Hz, 1H), 5.01 (d, *J* = 6.7 Hz, 1H), 4.62 - 4.51 (m, 3H), 4.30 (s, 2H), 3.71 (s, 2H), 2.84 - 2.61 (m, 3H), 2.27 (s, 3H), 1.93 (tdd, *J* = 7.9, 4.5, 1.6 Hz, 2H), 1.89 - 1.72 (m, 4H), 0.97 - 0.78 (m, 3H), 0.60 - 0.36 (m, 5H), 0.30 (qt, *J* = 6.9, 4.1 Hz, 3H). |

(continued)

| Pyrazolecarboxylic acid | Structure of compound | $^1$HNMR and/or MS (ESI) |
|---|---|---|
| 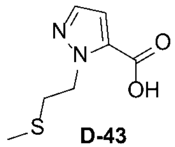 **D-43** | 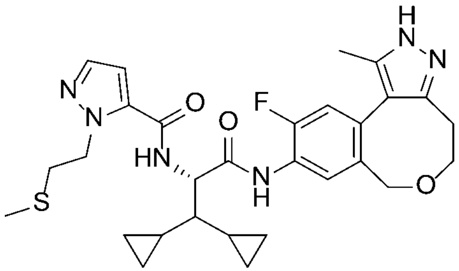 **compound 61** | MS (ESI) m/z =567 [M+1]$^+$ $^1$H NMR (600 MHz, Methanol-$d_4$) δ 8.04 (d, *J* = 7.9 Hz, 1H), 7.54 (d, *J* = 2.1 Hz, 1H), 7.18 (s, 1H), 6.88 (d, *J* = 2.1 Hz, 1H), 5.00 (d, *J* = 7.1 Hz, 1H), 4.78 - 4.65 (m, 2H), 4.32 (s, 2H), 3.71 (s, 2H), 2.93 - 2.83 (m, 2H), 2.70 (s, 2H), 2.29 (s, 3H), 2.03 (s, 3H), 0.97 - 0.78 (m, 3H), 0.59 - 0.37 (m, 5H), 0.34 - 0.25 (m, 3H). |
| 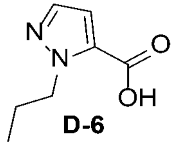 **D-6** | 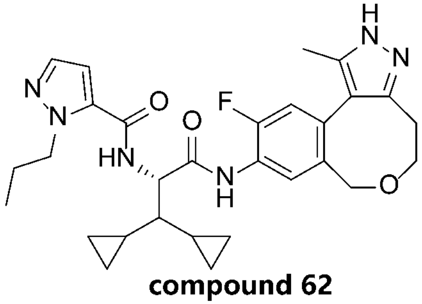 **compound 62** | MS (ESI) m/z =535 [M+1]$^+$ $^1$H NMR (600 MHz, DMSO-$d_6$) δ 12.47 (s, 1H), 9.98 (s, 1H), 8.44 (d, *J* = 8.8 Hz, 1H), 7.92 (d, *J* = 8.2 Hz, 1H), 7.49 (d, *J* = 2.0 Hz, 1H), 7.24 (d, *J* = 11.5 Hz, 1H), 7.00 (d, *J* = 2.0 Hz, 1H), 4.97 (dd, *J* = 8.8, 7.7 Hz, 1H), 4.52 - 4.37 (m, 2H), 4.15 (s, 2H), 3.61 (s, 2H), 2.58 (s, 2H), 2.20 (s, 3H), 1.73 (h, 2H), 0.97 - 0.82 (m, 2H), 0.79 (t, *J* = 7.4 Hz, 4H), 0.52 - 0.46 (m, 1H), 0.42 - 0.27 (m, 4H), 0.27 - 0.15 (m, 3H). |
| 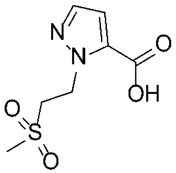 **D-49** | 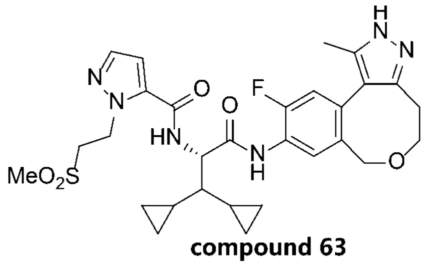 **compound 63** | MS (ESI) m/z =599 [M+1]$^+$ $^1$H NMR (400 MHz, DMSO-$d_6$) δ 7.92 (d, *J* = 8.0 Hz, 1H), 7.60 (d, *J* = 2.1 Hz, 1H), 7.24 (s, 1H), 7.06 (d, *J* = 2.1 Hz, 1H), 5.03 - 4.88 (m, 3H), 4.47 (s, 2H), 4.24 (s, 2H), 3.65 (t, *J* = 7.0 Hz, 2H), 2.93 (s, 3H), 2.73 (s, 2H), 2.23 (d, *J* = 20.3 Hz, 3H), 0.99 - 0.77 (m, 3H), 0.57 - 0.48 (m, 1H), 0.47 - 0.31 (m, 4H), 0.31 - 0.17 (m, 3H). |

(continued)

| Pyrazolecarboxylic acid | Structure of compound | $^1$HNMR and/or MS (ESI) |
|---|---|---|
| 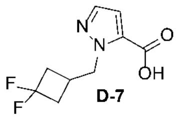 D-7 | 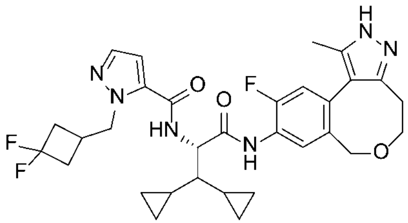 compound 64 | MS (ESI) m/z =597 [M+1]$^+$ <br> $^1$H NMR (600 MHz, Methanol-$d_4$) δ 8.03 (d, *J* = 8.0 Hz, 1H), 7.53 (d, *J* = 2.1 Hz, 1H), 7.18 (s, 1H), 6.90 (d, *J* = 2.1 Hz, 1H), 4.99 (d, *J* = 7.2 Hz, 1H), 4.66 (dd, *J* = 7.2, 4.9 Hz, 2H), 4.31 (s, 2H), 3.71 (s, 2H), 2.87 - 2.61 (m, 3H), 2.59 - 2.50 (m, 2H), 2.47 - 2.37 (m, 2H), 2.27 (d, *J* = 21.7 Hz, 3H), 0.97 - 0.80 (m, 3H), 0.60 - 0.42 (m, 4H), 0.39 (dq, *J* = 9.8, 5.0 Hz, 1H), 0.34 - 0.24 (m, 3H). |
| 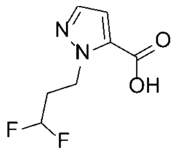 | 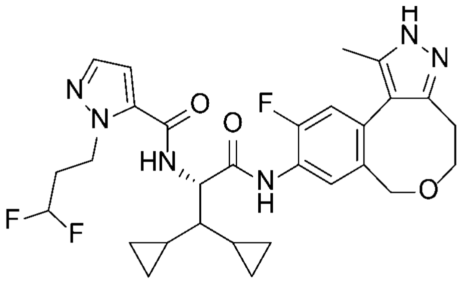 compound 65 | MS (ESI) m/z =571 [M+1]$^+$ <br> $^1$H NMR (600 MHz, DMSO-$d_6$) δ 12.38 (s, 1H), 9.98 (s, 1H), 8.48 (d, *J* = 8.8 Hz, 1H), 7.89 (d, *J* = 8.1 Hz, 1H), 7.52 (d, *J* = 2.0 Hz, 1H), 7.21 (d, *J* = 11.6 Hz, 1H), 7.05 (d, *J* = 2.1 Hz, 1H), 6.08 (tt, *J* = 56.2, 4.4 Hz, 1H), 4.96 (t, *J* = 8.3 Hz, 1H), 4.62 (td, *J* = 7.0, 2.2 Hz, 2H), 4.27 (s, 2H), 3.58 (s, 2H), 2.55 (s, 2H), 2.37 - 2.26 (m, 2H), 2.17 (s, 3H), 0.94 - 0.80 (m, 2H), 0.80 - 0.73 (m, 1H), 0.49 - 0.42 (m, 1H), 0.39 - 0.25 (m, 4H), 0.24 - 0.13 (m, 3H). |
| 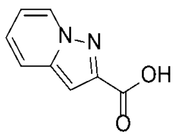 | 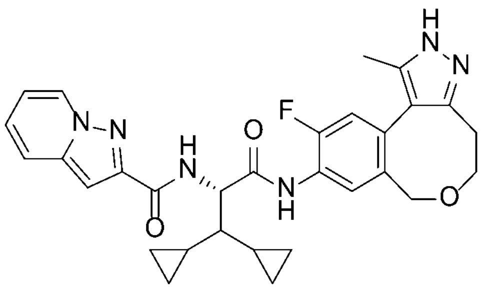 compound 66 | MS (ESI) m/z =543 [M+1]$^+$ <br> $^1$H NMR (400 MHz, Methanol-$d_4$) δ 8.62 (dt, *J* = 7.6, 1.6 Hz, 1H), 8.01 (d, *J* = 7.9 Hz, 1H), 7.85 - 7.66 (m, 1H), 7.28 (ddd, *J*= 9.2, 6.7, 1.1 Hz, 1H), 7.20 (s, 1H), 7.08 (d, *J* = 1.0 Hz, 1H), 7.02 (td, *J* = 6.9, 1.5 Hz, 1H), 5.10 (d,*J* = 5.8 Hz, 1H), 4.32 (s, 2H), 2.70 (s, 2H), 2.28 (s, 3H), 1.04 - 0.81 (m, 3H), 0.67 - 0.46 (m, 4H), 0.46 - 0.24 (m, 4H). |

(continued)

| Pyrazolecarboxylic acid | Structure of compound | $^1$HNMR and/or MS (ESI) |
|---|---|---|
| 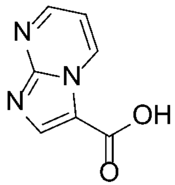 | 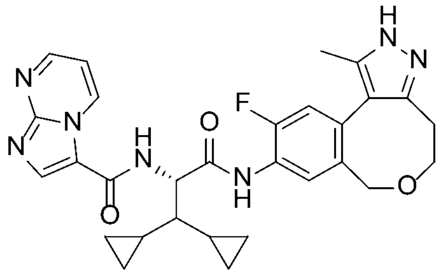 compound 67 | MS (ESI) m/z =544 [M+1]$^+$ $^1$H NMR (400 MHz, Methanol-$d_4$) δ 9.80 (ddt, *J* = 6.8, 4.6, 2.1 Hz, 1H), 8.60 - 8.50 (m, 1H), 8.05 (d, *J* = 8.0 Hz, 1H), 7.31 - 7.10 (m, 2H), 5.10 (d, *J* = 6.8 Hz, 1H), 4.31 (s, 2H), 3.71 (s, 2H), 2.70 (s, 2H), 2.28 (s, 3H), 1.08 - 0.82 (m, 3H), 0.66 - 0.39 (m, 5H), 0.32 (dd, *J* = 9.6, 5.1 Hz, 3H). |
| 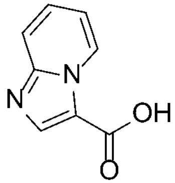 | 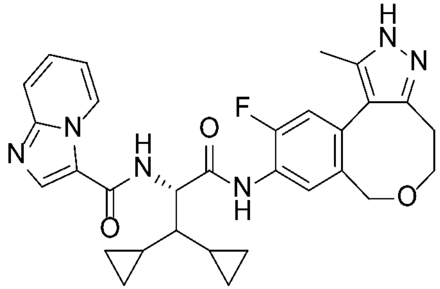 compound 68 | MS (ESI) m/z =543 [M+1]$^+$ $^1$H NMR (600 MHz, Methanol-$d_4$) δ 9.46 (d, *J* = 7.0 Hz, 1H), 8.39 (s, 1H), 8.06 (d, *J* = 7.8 Hz, 1H), 7.67 (d, *J* = 9.0 Hz, 1H), 7.54 - 7.48 (m, 1H), 7.24 - 7.15 (m, 1H), 7.12 (dd, *J* = 7.5, 6.4 Hz, 1H), 5.09 (d, *J* = 7.1 Hz, 1H), 4.31 (s, 2H), 3.71 (s, 2H), 2.70 (s, 2H), 2.27 (d,*J* = 27.0 Hz, 3H), 1.00 - 0.86 (m, 3H), 0.61 - 0.40 (m, 5H), 0.36 - 0.25 (m, 3H). |
| 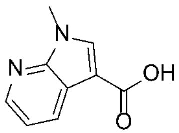 | 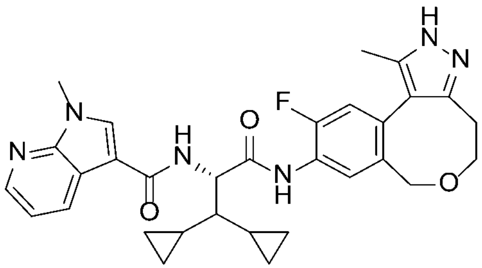 compound 69 | MS (ESI) m/z =557 [M+1]$^+$ $^1$H NMR (600 MHz, Methanol-$d_4$) δ 8.50 (dd, *J* = 8.0, 1.6 Hz, 1H), 8.33 (dd, *J* = 4.8, 1.6 Hz, 1H), 8.16 (s, 1H), 8.06 (d, *J* = 7.9 Hz, 1H), 7.26 (dd, *J*= 7.9, 4.8 Hz, 1H), 7.23 - 7.14 (m, 1H), 5.08 (d, *J* = 6.9 Hz, 1H), 4.28 (s, 2H), 3.94 (s, 3H), 3.76 (d, *J* = 63.0 Hz, 2H), 2.71 (s, 2H), 2.27 (d, *J* = 29.4 Hz, 3H), 1.02 - 0.82 (m, 3H), 0.61 - 0.37 (m, 5H), 0.31 (tdd, *J* = 12.3, 9.3, 4.7 Hz, 3H). |

(continued)

| Pyrazolecarboxylic acid | Structure of compound | $^1$HNMR and/or MS (ESI) |
|---|---|---|
| 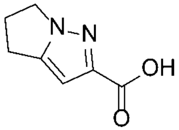 | 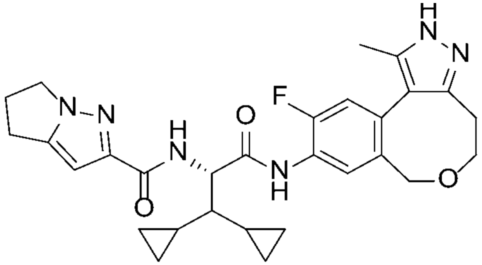 **compound 70** | MS (ESI) m/z =533 [M+1]$^+$<br>$^1$H NMR (400 MHz, Methanol-$d_4$) δ 7.99 (d, $J$ = 8.0 Hz, 1H), 7.18 (d, $J$ = 11.0 Hz, 1H), 5.00 (d, $J$ = 5.9 Hz, 1H), 4.31 (s, 2H), 4.18 (t, $J$ = 7.3 Hz, 2H), 3.71 (s, 2H), 2.94 (t, $J$ = 7.3 Hz, 2H), 2.81 - 2.57 (m, 4H), 2.27 (s, 3H), 0.92 - 0.76 (m, 3H), 0.58 - 0.42 (m, 4H), 0.37 - 0.23 (m, 4H). |
| 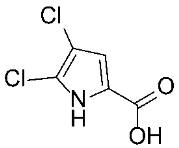 | 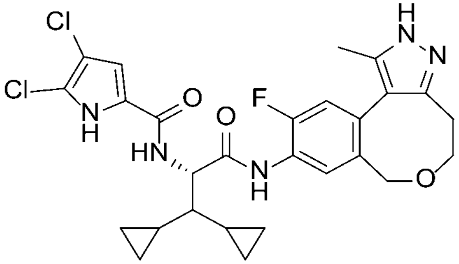 **compound 71** | MS (ESI) m/z =560 [M+1]$^+$<br>$^1$H NMR (600 MHz, Methanol-$d_4$) δ 8.02 (d, $J$ = 8.0 Hz, 1H), 7.18 (d, $J$ = 11.4 Hz, 1H), 6.91 (s, 1H), 4.97 (d,$J$ = 7.1 Hz, 1H), 4.29 (s, 2H), 3.71 (s, 2H), 2.70 (s, 2H), 2.27 (s, 3H), 0.98 - 0.75 (m, 3H), 0.56 (tq, $J$ = 8.5, 4.1 Hz, 1H), 0.49 (qd, $J$ = 8.3, 7.5, 4.4 Hz, 1H), 0.47 - 0.39 (m, 2H), 0.35 (dq, $J$ = 9.7, 4.9 Hz, 1H), 0.32 - 0.22 (m, 3H). |
| 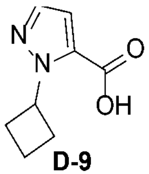 **D-9** | 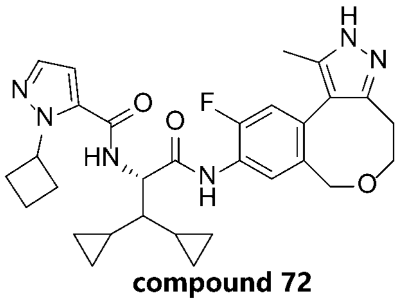 **compound 72** | MS (ESI) m/z =547 [M+1]$^+$<br>$^1$H NMR (400 MHz, DMSO-$d_6$) δ 12.47 (d, $J$ = 19.7 Hz, 1H), 9.99 (s, 1H), 8.42 (d, $J$ = 8.8 Hz, 1H), 7.92 (d, $J$ = 8.2 Hz, 1H), 7.55 (d, $J$ = 1.9 Hz, 1H), 7.24 (s, 1H), 6.95 (d, $J$ = 2.0 Hz, 1H), 5.68 - 5.47 (m, 1H), 4.94 (dd, $J$ = 8.7, 7.5 Hz, 1H), 4.28 (d, $J$ = 63.4 Hz, 2H), 3.60 (s, 2H), 2.75 - 2.53 (m, 3H), 2.42 - 2.08 (m, 6H), 1.76 (ddd, $J$ = 10.5, 8.5, 4.6 Hz, 2H), 1.00 - 0.72 (m, 3H), 0.47 (dd, $J$ = 8.6, 4.5 Hz, 1H), 0.43 - 0.13 (m, 7H). |

(continued)

| Pyrazolecarbo xylic acid | Structure of compound | $^1$HNMR and/or MS (ESI) |
|---|---|---|
| 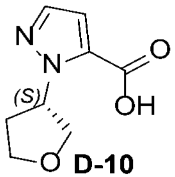 D-10 | 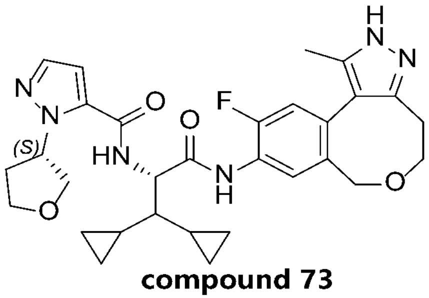 compound 73 | MS (ESI) m/z =563 [M+1]$^+$ $^1$H NMR (600 MHz, DMSO-$d_6$) δ 12.47 (s, 1H), 10.03 (s, 1H), 8.53 (d, $J$ = 8.7 Hz, 1H), 7.94 (d, $J$ = 8.2 Hz, 1H), 7.55 (d, $J$ = 2.0 Hz, 1H), 7.25 (d, $J$ = 11.6 Hz, 1H), 7.04 (d, $J$ = 2.0 Hz, 1H), 5.78 (dt, $J$ = 6.7, 4.0 Hz, 1H), 4.97 (t, $J$ = 8.2 Hz, 1H), 4.21 (s, 1H), 4.07 - 3.94 (m, 2H), 3.89 - 3.75 (m, 2H), 3.62 (s, 2H), 2.62 (p, $J$ = 1.9 Hz, 1H), 2.42 - 2.33 (m, 2H), 2.28 (ddd, $J$ = 16.9, 13.3, 8.5 Hz, 2H), 2.21 (s, 3H), 0.99 - 0.84 (m, 2H), 0.84 - 0.75 (m, 1H), 0.49 (dd, $J$ = 9.0, 4.6 Hz, 1H), 0.43 - 0.28 (m, 4H), 0.22 (dtd, $J$ = 21.0, 9.5, 4.6 Hz, 3H). |
| 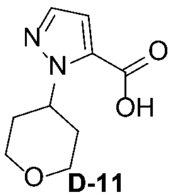 D-11 | 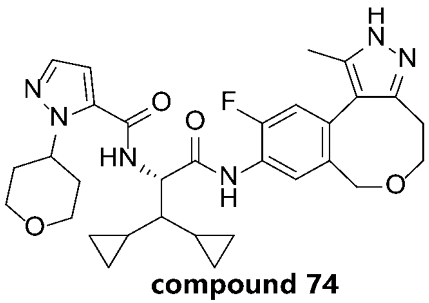 compound 74 | MS (ESI) m/z =577 [M+1]$^+$ $^1$H NMR (600 MHz, Methanol-$d_4$) δ 8.06 (d, $J$ = 7.9 Hz, 1H), 7.55 (d, $J$ = 2.0 Hz, 1H), 7.21 (d, $J$ = 11.2 Hz, 1H), 6.85 (d, $J$ = 2.1 Hz, 1H), 5.30 (tt, $J$ = 11.6, 4.1 Hz, 1H), 5.02 (d, $J$ = 7.3 Hz, 1H), 4.46 (d, $J$ = 162.8 Hz, 2H), 4.06 (td, $J$ = 11.5, 4.5 Hz, 2H), 3.83 (d, $J$= 112.9 Hz, 2H), 3.56 (tdd, $J$= 12.1, 7.2, 2.1 Hz, 2H), 2.72 (s, 2H), 2.38 - 2.17 (m, 5H), 2.02 - 1.85 (m, 2H), 1.00 - 0.79 (m, 3H), 0.63 - 0.37 (m, 5H), 0.36 - 0.25 (m, 3H). |
| 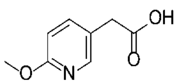 | 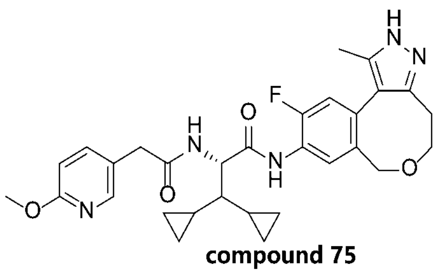 compound 75 | MS (ESI) m/z =548 [M+1]$^+$ $^1$H NMR (600 MHz, DMSO-$d_6$) δ 12.46 (d, $J$ = 31.0 Hz, 1H), 9.86 (s, 1H), 8.31 (d, $J$ = 9.0 Hz, 1H), 8.05 (d, $J$ = 2.4 Hz, 1H), 7.86 (d, $J$ = 8.1 Hz, 1H), 7.62 (dd, $J$ = 8.5, 2.5 Hz, 1H), 7.22 (s, 1H), 6.76 (d, $J$ = 8.5 Hz, 1H), 4.80 (dd, $J$ = 9.1, 6.6 Hz, 1H), 4.20 (s, 2H), 3.81 (s, 3H), 3.52 (q, $J$ = 14.4 Hz, 4H), 2.60 (s, 2H), 2.19 (d, $J$ = 30.8 Hz, 3H), 0.90 - 0.81 (m, 1H), 0.81 - 0.73 (m, 1H), 0.64 - 0.55 (m, 1H), 0.47 - 0.40 (m, 1H), 0.39 - 0.32 (m, 1H), 0.31 - 0.12 (m, 6H). |

(continued)

| Pyrazolecarboxylic acid | Structure of compound | $^1$HNMR and/or MS (ESI) |
|---|---|---|
| 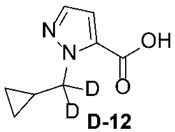 D-12 | 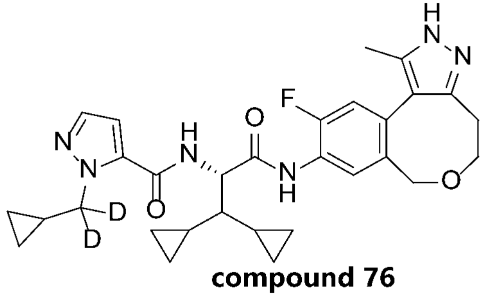 compound 76 | MS (ESI) m/z =549 [M+1]$^+$ <br> $^1$H NMR (400 MHz, DMSO-$d_6$) δ 12.46 (d, *J* = 17.7 Hz, 1H), 9.98 (s, 1H), 8.48 (d, *J* = 8.8 Hz, 1H), 7.91 (d, *J* = 8.2 Hz, 1H), 7.49 (d, *J* = 2.0 Hz, 1H), 7.24 (d, *J* = 11.7 Hz, 1H), 7.00 (d, *J* = 2.0 Hz, 1H), 5.03 - 4.89 (m, 1H), 4.20 (s, 2H), 3.61 (s, 2H), 2.76 - 2.53 (m, 2H), 2.21 (s, 3H), 1.21 (tt, *J* = 8.0, 4.8 Hz, 1H), 1.01 - 0.74 (m, 3H), 0.49 (tq, *J* = 8.2, 3.7 Hz, 1H), 0.39 (dtd, *J* = 11.0, 6.2, 3.8 Hz, 4H), 0.32 (ddt, *J* = 7.5, 5.6, 3.1 Hz, 4H), 0.21 (dh,*J* = 12.9, 4.2 Hz, 3H). |
| 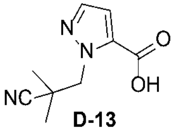 D-13 | 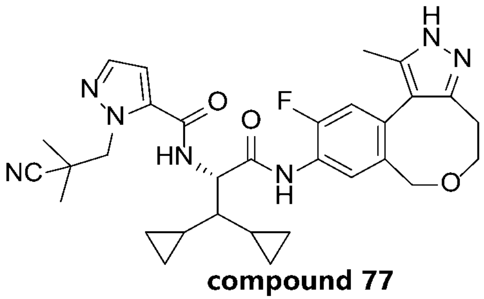 compound 77 | MS (ESI) m/z =574 [M+1]$^+$ <br> $^1$H NMR (400 MHz, Methanol-$d_4$) δ 7.99 (d, *J* = 8.0 Hz, 1H), 7.60 (d, *J* = 2.0 Hz, 1H), 7.18 (d, *J* = 11.4 Hz, 1H), 6.97 (d, *J* = 2.1 Hz, 1H), 4.99 (d, *J* = 6.2 Hz, 1H), 4.79 (d, *J* = 13.9 Hz, 2H), 4.30 (s, 2H), 3.71 (s, 2H), 2.70 (s, 2H), 2.28 (s, 3H), 1.36 (d, *J* = 3.8 Hz, 6H), 0.97 - 0.81 (m, 3H), 0.60 - 0.35 (m, 5H), 0.36 - 0.23 (m, 3H). |
| 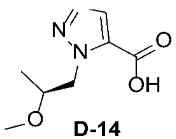 D-14 | 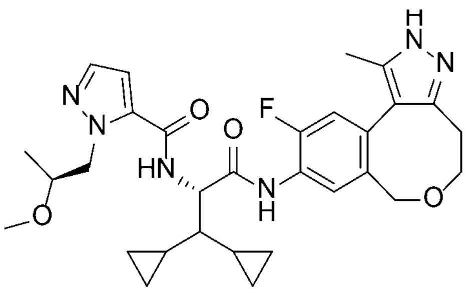 compound 78 | MS (ESI) m/z =565 [M+1]$^+$ <br> $^1$H NMR (600 MHz, Methanol-$d_4$) δ 8.03 (d, *J* = 7.9 Hz, 1H), 7.53 (t, *J* = 2.2 Hz, 1H), 7.18 (d, *J* = 11.5 Hz, 1H), 6.84 (d, *J* = 2.1 Hz, 1H), 5.04 - 4.99 (m, 1H), 4.58 (dd, *J* = 13.8, 7.3 Hz, 1H), 4.54 - 4.47 (m, 1H), 4.30 (s, 2H), 3.95 - 3.48 (m, 3H), 3.20 (d, *J* = 1.0 Hz, 3H), 2.70 (s, 2H), 2.27 (s, 3H), 1.10 (dd, *J* = 6.2, 1.3 Hz, 3H), 0.97 - 0.81 (m, 3H), 0.61 - 0.54 (m, 1H), 0.54 - 0.41 (m, 3H), 0.41 - 0.35 (m, 1H), 0.34 - 0.26 (m, 3H). |

(continued)

| Pyrazolecarbo xylic acid | Structure of compound | $^1$HNMR and/or MS (ESI) |
|---|---|---|
| 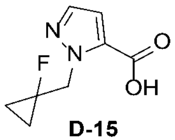 D-15 | 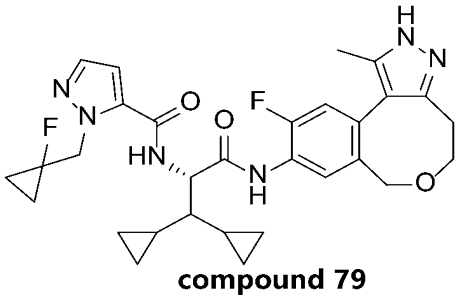 compound 79 | MS (ESI) m/z =565 [M+1]$^+$<br>$^1$H NMR (600 MHz, Methanol-$d_4$) δ 8.16 - 7.91 (m, 1H), 7.58 (s, 1H), 7.20 (s, 1H), 6.94 (s, 1H), 5.01 (s, 3H), 4.33 (s, 2H), 3.71 (s, 2H), 2.71 (s, 2H), 2.29 (s, 3H), 0.99 (d, $J$ = 18.6 Hz, 2H), 0.88 (d, $J$ = 22.3 Hz, 5H), 0.48 (dd, $J$ = 68.7, 37.0 Hz, 5H), 0.31 (s, 3H). |
| 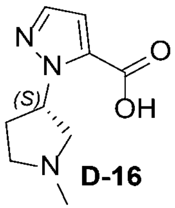 D-16 | 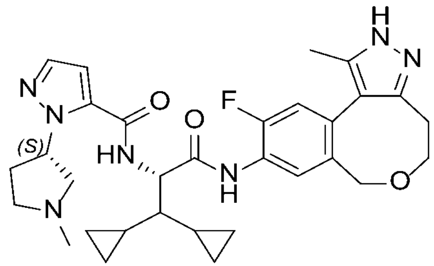 compound 80 | MS (ESI) m/z =576 [M+1]$^+$<br>$^1$H NMR (600 MHz, Methanol-$d_4$) δ 8.05 (d, $J$ = 8.0 Hz, 1H), 7.56 (d, $J$ = 2.0 Hz, 1H), 7.20 (d, $J$ = 11.3 Hz, 1H), 6.84 (d, $J$ = 2.0 Hz, 1H), 5.95 - 5.71 (m, 1H), 5.02 (d, $J$ = 7.3 Hz, 1H), 4.32 (s, 2H), 3.73 (s, 2H), 3.21 (dd, $J$ = 10.0, 7.8 Hz, 1H), 2.90 - 2.80 (m, 3H), 2.72 (s, 2H), 2.42 (s, 3H), 2.41 - 2.32 (m, 2H), 2.29 (s, 3H), 0.97 - 0.81 (m, 3H), 0.61 - 0.56 (m, 1H), 0.55 - 0.43 (m, 3H), 0.40 (dq, $J$ = 9.7, 5.0 Hz, 1H), 0.31 (dddd, $J$ = 14.8, 13.4, 7.3, 2.9 Hz, 3H). |
| 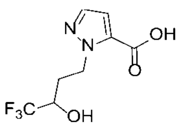 D-50 | 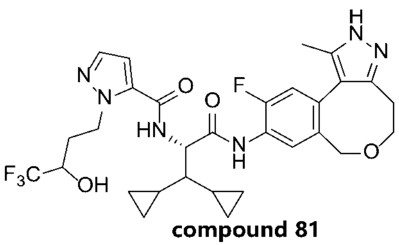 compound 81 | MS (ESI) m/z =619 [M+1]$^+$<br>$^1$H NMR (400 MHz, Methanol-$d_4$) δ 8.03 (dd, $J$ = 8.0, 2.3 Hz, 1H), 7.54 (dd, $J$ = 2.1, 1.2 Hz, 1H), 7.19 (s, 1H), 6.90 (dd, $J$ = 2.1, 0.7 Hz, 1H), 5.01 (dd,$J$ = 7.0, 2.2 Hz, 1H), 4.69 (dt, $J$ = 7.3, 4.9 Hz, 2H), 4.31 (s, 2H), 3.95 - 3.82 (m, 1H), 3.76 (d, $J$ = 36.3 Hz, 2H), 2.70 (s, 2H), 2.37 - 2.16 (m, 4H), 2.09 - 1.93 (m, 1H), 1.01 - 0.77 (m, 3H), 0.61 - 0.35 (m, 5H), 0.29 (ddt, $J$ = 9.9, 7.5, 3.9 Hz, 3H). |

(continued)

| Pyrazolecarboxylic acid | Structure of compound | $^1$HNMR and/or MS (ESI) |
|---|---|---|
| 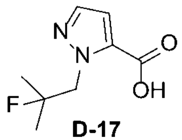 D-17 | 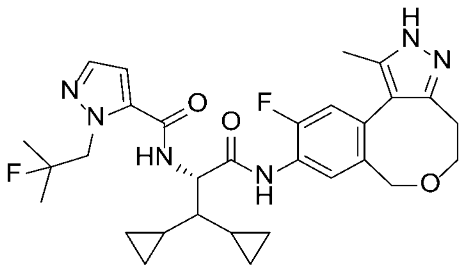 compound 82 | MS (ESI) m/z =567 [M+1]$^+$ <br> $^1$H NMR (400 MHz, Methanol-$d_4$) δ 8.03 (d, $J$ = 8.0 Hz, 1H), 7.56 (d, $J$ = 2.1 Hz, 1H), 7.18 (d, $J$ = 11.5 Hz, 1H), 6.89 (d, $J$ = 2.1 Hz, 1H), 4.98 (d, $J$ = 6.4 Hz, 1H), 4.83 - 4.69 (m, 2H), 4.31 (s, 2H), 3.72 (s, 2H), 2.87 - 2.54 (m, 2H), 2.28 (s, 3H), 1.26 (d, $J$ = 21.2 Hz, 6H), 0.98 - 0.79 (m, 3H), 0.61 - 0.35 (m, 5H), 0.30 (ddt, $J$ = 11.8, 4.2, 2.5 Hz, 3H). |
| 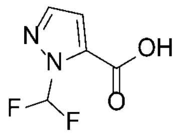 | 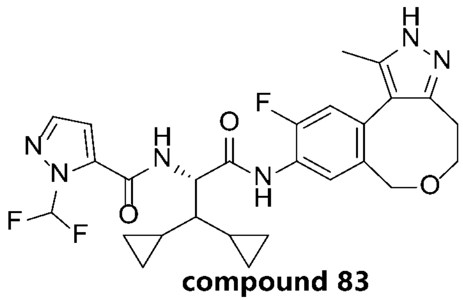 compound 83 | MS (ESI) m/z =543 [M+1]$^+$ <br> $^1$H NMR (600 MHz, DMSO-$d_6$) δ 12.47 (d, $J$ = 26.3 Hz, 1H), 10.09 (s, 1H), 8.90 (d, $J$ = 8.8 Hz, 1H), 8.32 (t, $J$ = 58.7 Hz, 1H), 7.95 - 7.88 (m, 2H), 7.35 (d, $J$ = 1.8 Hz, 1H), 7.24 (s, 1H), 5.02 (t, $J$ = 8.4 Hz, 1H), 4.30 (s, 2H), 3.61 (s, 2H), 2.59 (s, 2H), 2.18 (s, 3H), 0.97 - 0.84 (m, 2H), 0.78 (q, $J$ = 9.4, 7.9 Hz, 1H), 0.52 - 0.45 (m, 1H), 0.42 - 0.28 (m, 4H), 0.27 - 0.14 (m, 3H). |
| 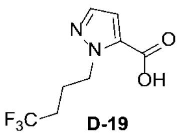 D-19 | 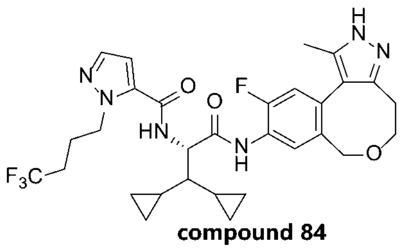 compound 84 | MS (ESI) m/z =603 [M+1]$^+$ <br> $^1$H NMR (600 MHz, DMSO-$d_6$) δ 12.47 (d, $J$ = 33.3 Hz, 1H), 10.00 (s, 1H), 8.52 (d, $J$ = 8.8 Hz, 1H), 7.92 (d, $J$ = 8.1 Hz, 1H), 7.54 (d, $J$ = 2.0 Hz, 1H), 7.24 (dd, $J$ = 27.4, 11.7 Hz, 1H), 7.05 (d, $J$ = 2.1 Hz, 1H), 4.98 (t, 1H), 4.62 - 4.48 (m, 2H), 4.20 (s, 2H), 3.72 (s, 2H), 2.61 (s, 2H), 2.27 - 2.13 (m, 5H), 2.01 - 1.92 (m, 2H), 0.97 - 0.90 (m, 1H), 0.89 - 0.83 (m, 1H), 0.82 - 0.76 (m, 1H), 0.52 - 0.45 (m, 1H), 0.42 - 0.34 (m, 2H), 0.34 - 0.27 (m, 2H), 0.27 - 0.15 (m, 3H). |

(continued)

| Pyrazolecarbo xylic acid | Structure of compound | $^1$HNMR and/or MS (ESI) |
|---|---|---|
| 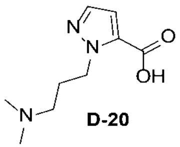 D-20 | 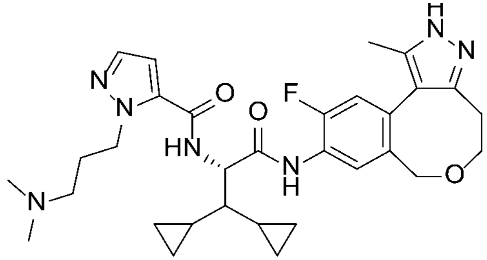 compound 85 | MS (ESI) m/z =578 [M+1]$^+$<br>$^1$H NMR (400 MHz, Methanol-$d_4$) δ 8.05 (d, *J* = 8.0 Hz, 1H), 7.52 (d, *J* = 2.1 Hz, 1H), 7.18 (d, *J* = 11.5 Hz, 1H), 6.88 (d, *J* = 2.1 Hz, 1H), 5.00 (d, *J* = 6.9 Hz, 1H), 4.64 - 4.50 (m, 2H), 4.31 (s, 2H), 3.71 (s, 2H), 2.70 (s, 2H), 2.38 - 2.30 (m, 2H), 2.27 (s, 3H), 2.20 (s, 6H), 2.08 - 1.95 (m, 2H), 0.94 - 0.81 (m, 3H), 0.60 - 0.36 (m, 5H), 0.35 - 0.22 (m, 3H). |
| 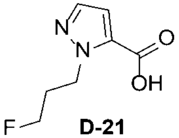 D-21 | 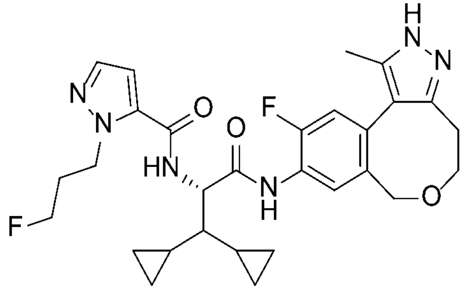 compound 86 | MS (ESI) m/z =553 [M+1]$^+$<br>$^1$H NMR (600 MHz, DMSO-$d_6$) δ 12.47 (d, *J* = 31.2 Hz, 1H), 10.00 (s, 1H), 8.47 (d, *J* = 8.8 Hz, 1H), 7.93 (d, *J* = 8.2 Hz, 1H), 7.53 (d, *J* = 2.0 Hz, 1H), 7.24 (s, 1H), 7.05 (d, *J* = 2.0 Hz, 1H), 4.98 (t, *J* = 8.3 Hz, 1H), 4.59 (t, *J* = 7.0 Hz, 2H), 4.46 (t, *J* = 5.9 Hz, 1H), 4.38 (t, *J* = 5.9 Hz, 1H), 4.21 (s, 2H), 3.61 (s, 2H), 2.61 (s, 2H), 2.20 (d, *J* = *3 1.0* Hz, 3H), 2.15 - 2.07 (m, 2H), 0.97 - 0.83 (m, 2H), 0.82 - 0.76 (m, 1H), 0.48 (tt, *J*= 8.9, 4.3 Hz, 1H), 0.42 - 0.35 (m, 2H), 0.35 - 0.27 (m, 2H), 0.27 - 0.16 (m, 3H). |
| 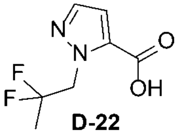 D-22 | 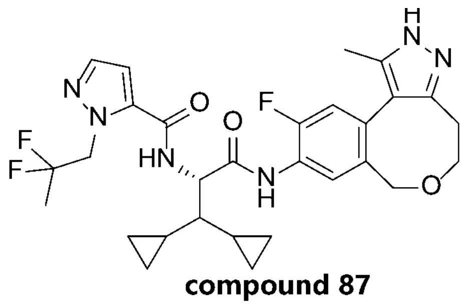 compound 87 | MS (ESI) m/z =571 [M+1]$^+$<br>$^1$H NMR (600 MHz, DMSO-$d_6$) δ 12.47 (s, 1H), 9.99 (s, 1H), 8.61 (d, *J* = 8.9 Hz, 1H), 7.91 (d, *J* = 8.2 Hz, 1H), 7.62 (d, *J* = 2.0 Hz, 1H), 7.24 (d, *J* = 11.6 Hz, 1H), 7.10 (d, *J* = 2.1 Hz, 1H), 5.17 - 5.02 (m, 2H), 4.99 (t, *J* = 8.3 Hz, 1H), 4.20 (s, 2H), 3.76 - 3.46 (m, 2H), 2.62 (s, 2H), 2.20 (s, 3H), 1.53 (t, *J* = 19.0 Hz, 3H), 0.97 - 0.76 (m, 3H), 0.52 - 0.44 (m, 1H), 0.42 - 0.27 (m, 4H), 0.27 - 0.14 (m, 3H). |

(continued)

| Pyrazolecarbo xylic acid | Structure of compound | $^1$HNMR and/or MS (ESI) |
|---|---|---|
| 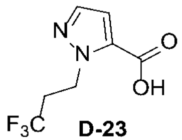 D-23 | 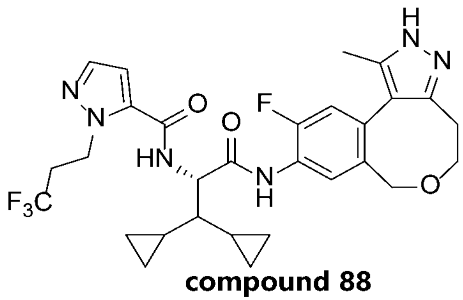 compound 88 | MS (ESI) m/z =589 [M+1]$^+$ $^1$H NMR (600 MHz, DMSO-$d_6$) δ 12.47 (d, $J$ = 32.0 Hz, 1H), 10.02 (s, 1H), 8.54 (d, $J$ = 8.8 Hz, 1H), 7.92 (d, $J$ = 8.1 Hz, 1H), 7.56 (d, $J$ = 2.0 Hz, 1H), 7.23 (s, 1H), 7.11 (d, $J$ = 2.1 Hz, 1H), 5.00 (t, $J$ = 8.3 Hz, 1H), 4.82 - 4.71 (m, 2H), 4.20 (s, 2H), 3.61 (s, 2H), 2.88 - 2.75 (m, 2H), 2.61 (s, 2H), 2.20 (d, $J$ = 32.0 Hz, 3H), 0.96 - 0.76 (m, 3H), 0.53 - 0.45 (m, 1H), 0.43 - 0.35 (m, 2H), 0.35 - 0.27 (m, 2H), 0.27 - 0.14 (m, 3H). |
| 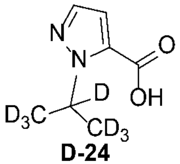 D-24 | 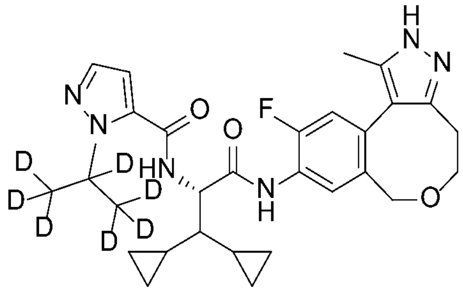 compound 89 | MS (ESI) m/z =542 [M+1]$^+$ $^1$H NMR (600 MHz, DMSO-$d_6$) δ 12.47 (d, $J$ = 32.2 Hz, 1H), 9.98 (s, 1H), 8.42 (d, $J$ = 8.8 Hz, 1H), 7.93 (d, $J$ = 8.1 Hz, 1H), 7.50 (d, $J$ = 2.0 Hz, 1H), 7.23 (s, 1H), 6.91 (d, $J$ = 2.0 Hz, 1H), 4.95 (t, $J$ = 8.8, 7.6 Hz, 1H), 4.20 (s, 2H), 3.60 (s, 2H), 2.61 (s, 2H), 2.19 (d, $J$ = 32.9 Hz, 3H), 0.99 - 0.83 (m, 2H), 0.83 - 0.74 (m, 1H), 0.54 - 0.44 (m, 1H), 0.43 - 0.27 (m, 4H), 0.27 - 0.13 (m, 3H). |
| 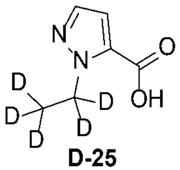 D-25 | 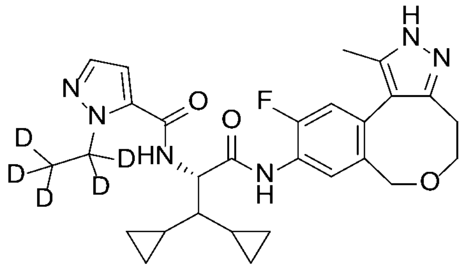 compound 90 | MS (ESI) m/z =526 [M+1]$^+$ $^1$H NMR (600 MHz, DMSO-$d_6$) δ 12.47 (d, $J$ = 25.0 Hz, 1H), 10.00 (s, 1H), 8.43 (d, $J$ = 8.8 Hz, 1H), 7.93 (d, $J$ = 8.2 Hz, 1H), 7.49 (d, $J$ = 2.0 Hz, 1H), 7.24 (s, 1H), 7.00 (d, $J$ = 2.0 Hz, 1H), 4.97 (t, $J$ = 8.3 Hz, 1H), 4.20 (s, 2H), 3.61 (s, 2H), 2.62 (s, 2H), 2.21 (s, 3H), 0.97 - 0.83 (m, 2H), 0.83 - 0.75 (m, 1H), 0.52 - 0.45 (m, 1H), 0.43 - 0.27 (m, 4H), 0.27 - 0.15 (m, 3H). |

(continued)

| Pyrazolecarboxylic acid | Structure of compound | $^1$HNMR and/or MS (ESI) |
|---|---|---|
| 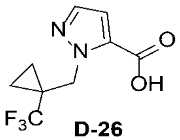 D-26 | 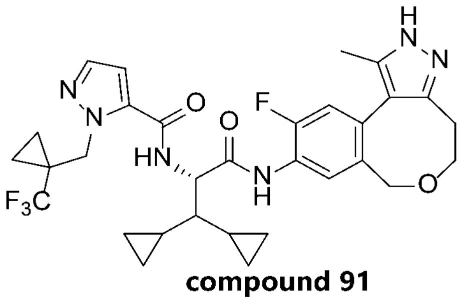 **compound 91** | MS (ESI) m/z =615 [M+1]$^+$<br>$^1$H NMR (600 MHz, DMSO-$d_6$) δ 12.47 (d, *J* = 31.7 Hz, 1H), 10.00 (s, 1H), 8.57 (d, *J* = 8.9 Hz, 1H), 7.91 (d, *J* = 8.1 Hz, 1H), 7.56 (d, *J* = 2.0 Hz, 1H), 7.23 (s, 1H), 7.04 (*d, J* = 2.0 Hz, 1H), 5.00 (t, *J* = 8.3 Hz, 1H), 4.96 - 4.87 (m, 2H), 4.20 (s, 2H), 3.61 (s, 2H), 2.61 (s, 2H), 2.20 (d, *J* = 32.0 Hz, 3H), 1.02 - 0.88 (m, 5H), 0.88 - 0.76 (m, 2H), 0.53 - 0.45 (m, 1H), 0.43 - 0.35 (m, 2H), 0.31 (s, 2H), 0.27 - 0.14 (m, 3H). |
| 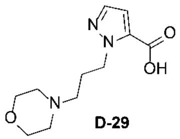 D-29 | 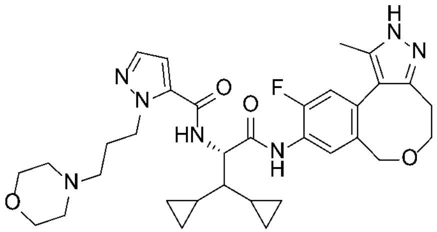 **compound 92** | MS (ESI) m/z =620 [M+1]$^+$<br>$^1$H NMR (400 MHz, Methanol-$d_4$) δ 8.06 (d, *J* = 8.0 Hz, 1H), 7.52 (d, *J* = 2.1 Hz, 1H), 7.19 (d, *J* = 11.4 Hz, 1H), 4.99 (d, *J* = 7.0 Hz, 1H), 4.66 (dt, *J* = 13.9, 7.1 Hz, 1H), 4.52 (dt, *J* = 13.6, 6.9 Hz, 1H), 4.31 (s, 2H), 3.61 (t, *J* = 4.7 Hz, 6H), 2.70 (s, 2H), 2.49 - 2.32 (m, 6H), 2.28 (s, 3H), 2.03 (q, *J*= *7.2* Hz, 2H), 0.98 - 0.76 (m, 3H), 0.64 - 0.37 (m, 5H), 0.30 (ddt, J= 10.0, 7.5, 4.7 Hz, 3H). |
| 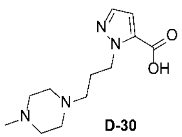 D-30 | 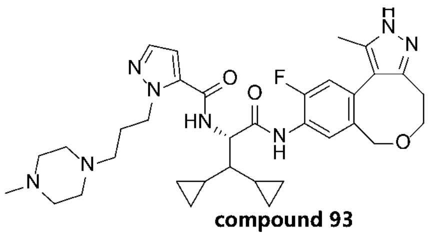 **compound 93** | MS (ESI) m/z =633 [M+1]$^+$<br>$^1$H NMR (400 MHz, Chloroform-d) δ 8.42 (dd, *J* = 8.2, 3.3 Hz, 1H), 7.49 (t, *J* = *2.6* Hz, 1H), 7.07 (dd, *J* = 11.5, 3.2 Hz, 1H), 6.62 (t, *J* = 2.6 Hz, 1H), 4.88 *(d, J* = 3.6 Hz, 1H), 4.74 *(d, J* = 6.3 Hz, 3H), 4.60 (qt, *J* = 13.5, 7.1 Hz, 2H), 4.35 (s, 2H), 3.75 (s, 2H), 2.75 (s, 2H), 2.51 - 2.35 (m, 7H), 2.31 (d, *J* = 3.2 Hz, 3H), 2.26 (d, *J* = 3.3 Hz, 3H), 2.05 (dd, *J* = 8.7, 5.8 Hz, 2H), 0.87 (p, *J* = 5.7 Hz, 3H), 0.73 - 0.49 (m, 4H), 0.40 (dh, *J* = 17.3, 4.3, 3.3 Hz, 2H), 0.26 (q, *J* = 3.9 Hz, 2H). |

(continued)

| Pyrazolecarboxylic acid | Structure of compound | $^1$HNMR and/or MS (ESI) |
|---|---|---|
| 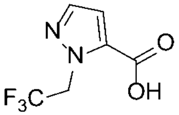 **D-56** | 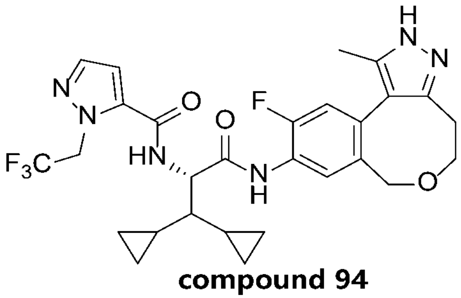 **compound 94** | MS (ESI) m/z =575 [M+1]$^+$<br>$^1$H NMR (600 MHz, Methanol-$d_4$) δ 8.03 (d, *J* = 7.9 Hz, 1H), 7.64 (d, *J* = 2.1 Hz, 1H), 7.18 (s, 1H), 7.04 (d, *J* = 2.1 Hz, 1H), 5.47 (dq, *J* = *15.0,* 8.6 Hz, 1H), 5.37 (dq, *J* = 15.0, 8.6 Hz, 1H), 5.03 (d, *J* = 7.0 Hz, 1H), 4.31 (s, 2H), 3.71 (s, 2H), 2.70 (s, 2H), 2.28 (s, 3H), 0.93 - 0.82 (m, 3H), 0.56 (tt, *J*= 7.9, 4.6 Hz, 1H), 0.52 - 0.41 (m, 3H), 0.38 *(dq, J*= 9.7, 5.0 Hz, 1H), 0.33 - 0.23 (m, 3H). |
| 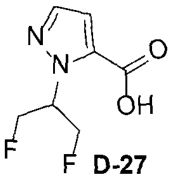 **D-27** | 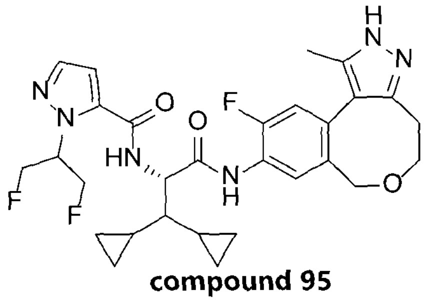 **compound 95** | MS (ESI) m/z =571 [M+1]$^+$<br>$^1$H NMR (600 MHz, Methanol-$d_4$) δ 8.04 (d, *J* = 7.9 Hz, 1H), 7.60 (d, *J* = 2.0 Hz, 1H), 7.18 (s, 1H), 6.95 (d, *J* = 2.0 Hz, 1H), 5.99 - 5.86 (m, 1H), 5.03 (d, *J* = 7.3 Hz, 1H), 4.97 - 4.86 (m, 2H), 4.83 - 4.74 (m, 2H), 4.31 (s, 2H), 3.71 (s, 2H), 2.70 (s, 2H), 2.28 (s, 3H), 0.95 - 0.79 (m, 3H), 0.61 - 0.53 (m, 1H), 0.53 - 0.41 (m, 3H), 0.40 - 0.34 (m, 1H), 0.33 - 0.23 (m, 3H). |
| 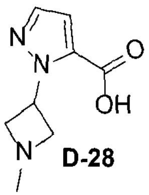 **D-28** | 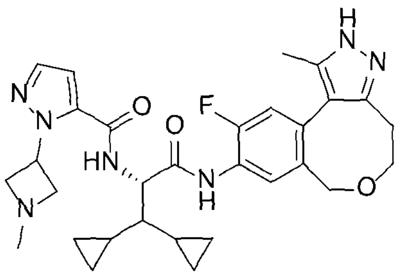 **compound 96** | MS (ESI) m/z =562 [M+1]$^+$<br>$^1$H NMR (400 MHz, Methanol-$d_4$) δ 8.03 (d, *J* = 8.0 Hz, 1H), 7.59 (d, *J* = 2.0 Hz, 1H), 7.19 (d, *J = 11.4* Hz, 1H), 6.88 (d, *J* = 2.0 Hz, 1H), 5.71 (p, *J* = 7.2 Hz, 1H), 4.98 (d, *J* = 7.1 Hz, 1H), 4.29 (t, *J* = 5.2 Hz, 2H), 3.93 - 3.84 (m, 2H), 3.69 (td, *J* = 8.8, 7.0 Hz, 4H), 2.66 (d, *J* = 29.8 Hz, 2H), 2.46 (s, 3H), 2.28 (s, 3H), 0.99 - 0.74 (m, 3H), 0.61 - 0.23 (m, 8H). |

(continued)

| Pyrazolecarbo xylic acid | Structure of compound | $^1$HNMR and/or MS (ESI) |
|---|---|---|
| 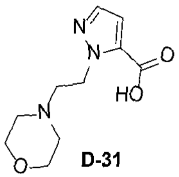 D-31 | 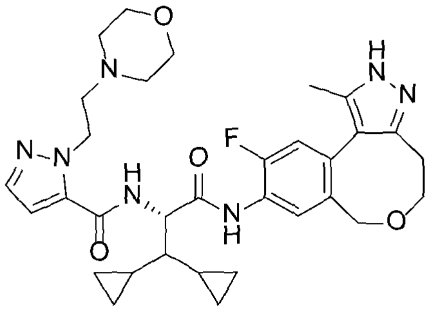 compound 97 | MS (ESI) m/z =606 [M+1]+<br>$^1$H NMR (400 MHz, Methanol-$d_4$) δ 8.04 (d, $J$ = 8.0 Hz, 1H), 7.52 (d, $J$ = 2.1 Hz, 1H), 7.19 (s, 1H), 6.86 (d, $J$ = 2.1 Hz, 1H), 5.00 (d, $J$ = 7.0 Hz, 1H), 4.79 (dt, $J$ = 13.6, 6.9 Hz, 1H), 4.59 (dt, $J$ = 13.3, 6.4 Hz, 1H), 4.30 (s, 2H), 3.71 (s, 2H), 3.58 (t, $J$ = 4.7 Hz, 4H), 2.74 (t, $J$ = 6.6 Hz, 4H), 2.53 - 2.37 (m, 4H), 2.28 (s, 3H), 0.87 (ddtd, $J$ = 21.0, 18.2, 9.3, 6.2 Hz, 3H), 0.62 - 0.36 (m, 5H), 0.30 (ddt, $J$ = 9.8, 7.1, 4.8 Hz, 3H). |
| 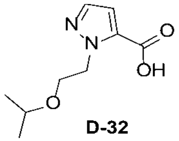 D-32 | 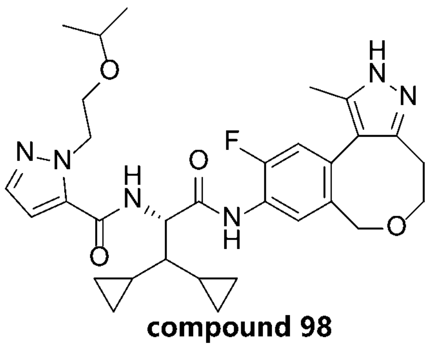 compound 98 | MS (ESI) m/z =579 [M+1]+<br>$^1$H NMR (600 MHz, Methanol-$d_4$) δ 8.04 (d, $J$ = 7.9 Hz, 1H), 7.53 (d, $J$ = 2.0 Hz, 1H), 7.18 (s, 1H), 6.85 (d, $J$ = 2.1 Hz, 1H), 5.02 (d, $J$ = 7.1 Hz, 1H), 4.78 - 4.71 (m, 1H), 4.66 - 4.56 (m, 1H), 4.31 (s, 2H), 3.82 - 3.60 (m, 4H), 3.54 - 3.45 (m, 1H), 2.70 (s, 2H), 2.28 (s, 3H), 1.01 (dd, $J$ = 6.1, 3.4 Hz, 6H), 0.97 - 0.80 (m, 3H), 0.61 - 0.35 (m, 5H), 0.34 - 0.24 (m, 3H). |
| 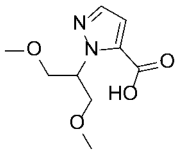 D-33 | 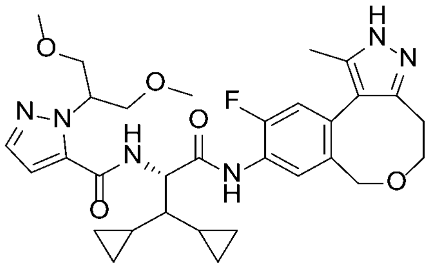 compound 99 | MS (ESI) m/z =595 [M+1]+<br>$^1$H NMR (600 MHz, Methanol-$d_4$) δ 8.02 (d, $J$ = 7.9 Hz, 1H), 7.56 (d, $J$ = 2.0 Hz, 1H), 7.18 (s, 1H), 6.80 (d, $J$ = 2.0 Hz, 1H), 5.63 (tt, $J$ = 8.0, 5.3 Hz, 1H), 5.03 (d, $J$ = 6.8 Hz, 1H), 4.30 (s, 2H), 3.91 - 3.60 (m, 6H), 3.29 (s, 3H), 3.26 (s, 3H), 2.70 (s, 2H), 2.29 (s, 3H), 0.95 - 0.79 (m, 3H), 0.61 - 0.53 (m, 1H), 0.53 - 0.41 (m, 3H), 0.39 - 0.25 (m, 4H). |

(continued)

| Pyrazolecarboxylic acid | Structure of compound | $^1$HNMR and/or MS (ESI) |
|---|---|---|
| 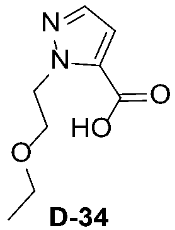 D-34 | 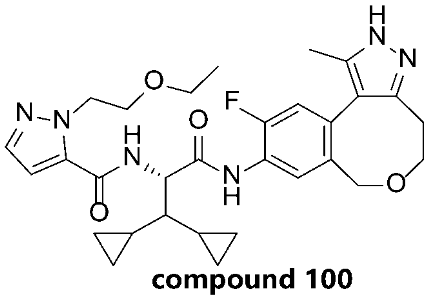 compound 100 | MS (ESI) m/z =565 [M+1]$^+$ $^1$H NMR (600 MHz, Methanol-$d_4$) δ 8.03 (d, *J* = 7.9 Hz, 1H), 7.53 (d, *J* = 2.1 Hz, 1H), 7.18 (s, 1H), 6.85 (d, *J* = 2.1 Hz, 1H), 5.02 (d, *J* = 7.1 Hz, 1H), 4.78 - 4.73 (m, 1H), 4.65 (dt, *J* = 14.0, 5.4 Hz, 1H), 4.31 (s, 2H), 3.87 - 3.52 (m, 4H), 3.43 (q, *J* = 6.8 Hz, 2H), 2.70 (s, 2H), 2.27 (d, *J* = 24.7 Hz, 3H), 1.05 (t, *J* = 7.0 Hz, 3H), 0.98 - 0.78 (m, 3H), 0.60 - 0.42 (m, 4H), 0.38 (dq, J= 9.6, 5.0 Hz, 1H), 0.34 - 0.25 (m, 3H). |
| 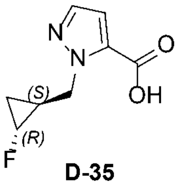 D-35 | 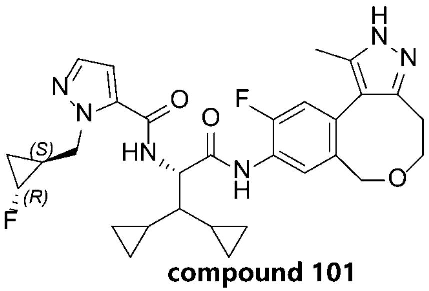 compound 101 | MS (ESI) m/z =565 [M+1]$^+$ $^1$H NMR (600 MHz, DMSO-$d_6$) δ 12.48 (s, 1H), 10.00 (s, 1H), 8.54 (dd, *J* = 8.8, 3.1 Hz, 1H), 7.92 (d, *J* = 8.0 Hz, 1H), 7.53 (t, *J* = 1.6 Hz, 1H), 7.24 (d, *J* = 11.3 Hz, 1H), 7.05 (t, *J* = 2.2 Hz, 1H), 4.97 (td, *J* = 8.2, 3.8 Hz, 1H), 4.71 (dddt, *J* = 64.4, 13.6, 6.1, 2.3 Hz, 1H), 4.37 (dd, *J*= 7.6, 2.8 Hz, 2H), 4.30 - 4.06 (m, 1H), 3.61 (s, 2H), 2.61 (p, *J* = 1.8 Hz, 2H), 2.20 (s, 3H), 1.81 - 1.63 (m, 1H), 1.32 - 1.14 (m, 1H), 1.01 (dddt, *J* = 22.0, 11.2, 7.0, 2.1 Hz, 1H), 0.97 - 0.91 (m, 1H), 0.87 (qt, *J* = 7.9, 3.3 Hz, 1H), 0.80 (q, *J* = 8.9 Hz, 1H), 0.72 (dq, *J* = 10.9, 6.6 Hz, 1H), 0.49 (tt, *J* = 8.9, 4.8 Hz, 1H), 0.39 (dh, *J* = 9.4, 5.1 Hz, 2H), 0.32 (tt, *J*= 9.0, 4.0 Hz, 2H), 0.21 (dddt, *J* = 26.4, 14.3, 9.7, 4.9 Hz, 3H). |
| 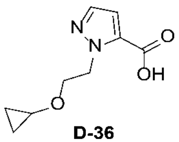 D-36 | 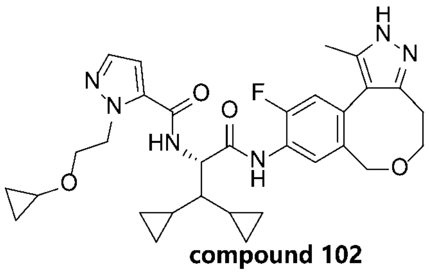 compound 102 | MS (ESI) m/z =577 [M+1]$^+$ $^1$H NMR (600 MHz, Chloroform-d) δ 8.05 (d, *J* = 7.7 Hz, 1H), 7.60 - 7.46 (m, 1H), 7.19 (s, 1H), 6.93 - 6.77 (m, 1H), 5.06 - 4.96 (m, 1H), 4.79 - 4.71 (m, 1H), 4.66 (dt, *J* = 14.3, 5.6 Hz, 1H), 3.84 (qd, *J* = 5.6, 3.5 Hz, 4H), 3.24 (qd, *J*= 5.5, 3.0 Hz, 1H), 2.70 (s, 2H), 2.28 (s, 3H), 0.98 - 0.79 (m, 3H), 0.57 (ddq, *J* = 11.3, 5.9, 3.4 Hz, 1H), 0.54 - 0.41 (m, 3H), 0.40 - 0.26 (m, 8H). |

(continued)

| Pyrazolecarboxylic acid | Structure of compound | $^1$HNMR and/or MS (ESI) |
|---|---|---|
| 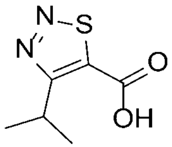 | 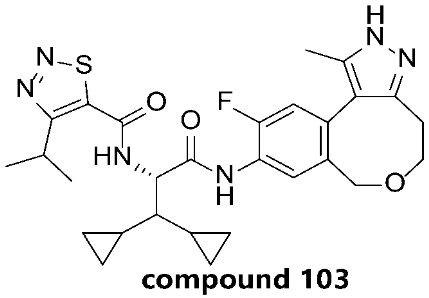 compound 103 | MS (ESI) m/z =553 [M+1]$^+$ $^1$H NMR (400 MHz, Methanol-$d_4$) δ 8.03 (d, $J$ = 8.0 Hz, 1H), 7.19 (d, $J$ = 11.4 Hz, 1H), 5.06 (d, $J$ = 7.3 Hz, 1H), 4.31 (s, 2H), 3.66 (hept, $J$ = 7.0 Hz, 3H), 2.70 (s, 2H), 2.28 (s, 3H), 1.47 (dd, $J$ = 6.9, 2.5 Hz, 6H), 0.96 - 0.76 (m, 3H), 0.63 - 0.42 (m, 4H), 0.39 - 0.25 (m, 4H). |
| 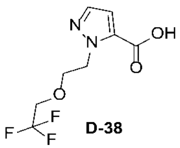 D-38 | 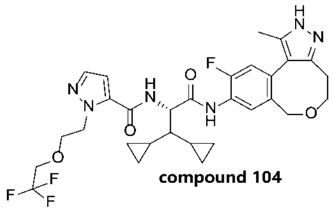 compound 104 | MS (ESI) m/z =619 [M+1]$^+$ $^1$H NMR (400 MHz, Methanol-$d_4$) δ 8.03 (d, $J$ = 7.9 Hz, 1H), 7.55 (d, $J$ = 2.1 Hz, 1H), 7.18 (d, $J$ = 11.1 Hz, 1H), 6.90 (d, $J$ = 2.1 Hz, 1H), 5.01 (d, $J$ = 6.9 Hz, 1H), 4.80 - 4.70 (m, 2H), 4.31 (s, 2H), 3.98 (t, $J$ = 5.5 Hz, 2H), 3.92 - 3.81 (m, 2H), 3.71 (s, 2H), 2.70 (s, 2H), 2.28 (s, 3H), 0.96 - 0.77 (m, 3H), 0.60 - 0.36 (m, 5H), 0.30 (tdd, $J$= 9.2, 5.8, 4.2 Hz, 3H). |
| 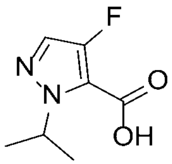 D-57 | 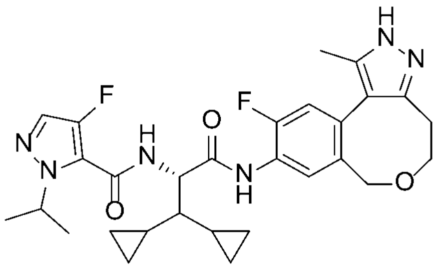 compound 105 | MS (ESI) m/z =553 [M+1]$^+$ $^1$H NMR (600 MHz, DMSO-$d_6$) δ 12.48 (d, $J$ = 29.3 Hz, 1H), 9.98 (s, 1H), 8.25 (dd, $J$ = 8.8, 2.6 Hz, 1H), 7.92 (d, $J$ = 8.1 Hz, 1H), 7.63 (d, $J$ = 4.5 Hz, 1H), 7.25 (s, 1H), 5.13 (p, $J$ = 6.6 Hz, 1H), 4.96 (dd, $J$ = 8.7, 6.2 Hz, 1H), 4.21 (s, 2H), 3.62 (s, 2H), 2.62 (s, 2H), 2.21 (d, $J$= 24.4 Hz, 3H), 1.37 (dd, $J$ = 19.2, 6.7 Hz, 6H), 0.97 - 0.76 (m, 3H), 0.54 - 0.46 (m, 1H), 0.46 - 0.40 (m, 1H), 0.40 - 0.22 (m, 6H). |

(continued)

| Pyrazolecarbo xylic acid | Structure of compound | $^1$HNMR and/or MS (ESI) |
|---|---|---|
| 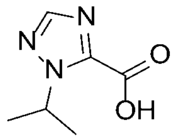 **D-58** | 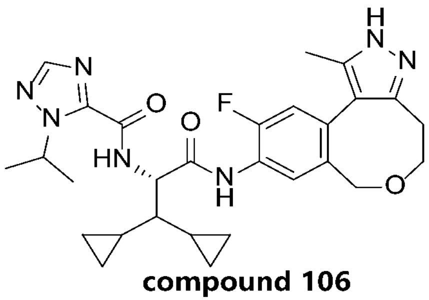 **compound 106** | MS (ESI) m/z =536 [M+1]$^+$ $^1$H NMR (600 MHz, Methanol-$d_4$) δ 8.06 - 7.92 (m, 2H), 7.18 (d, *J* = 11.3 Hz, 1H), 5.72 (hept, *J* = *6.6* Hz, 1H), 5.00 (d, *J* = 6.1 Hz, 1H), 4.33 (s, 2H), 3.71 (s, 2H), 2.70 (s, 2H), 2.27 (s, 3H), 1.50 (dd,*J* = 6.7, 2.8 Hz, 6H), 0.98 - 0.79 (m, 3H), 0.60 - 0.51 (m, 2H), 0.47 (tdd, *J* = 12.8, 6.6, 4.1 Hz, 2H), 0.33 (dddd, *J* = 26.6, 9.6, 8.0, 4.5 Hz, 4H). |
| 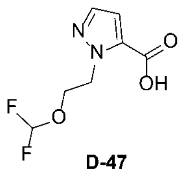 **D-47** | 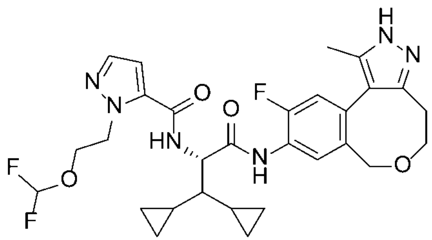 **compound 107** | MS (ESI) m/z =587 [M+1]$^+$ $^1$H NMR (600 MHz, DMSO-$d_6$) δ 12.46 (d, *J* = 33.5 Hz, 1H), 10.01 (s, 1H), 8.50 (d, *J* = 8.9 Hz, 1H), 7.92 (d, *J* = 8.1 Hz, 1H), 7.55 (d, *J* = 2.0 Hz, 1H), 7.23 (s, 1H), 7.08 (d, *J* = 2.1 Hz, 1H), 6.57 (t, *J* = 75.6 Hz, 1H), 4.99 (dd, *J* = 8.8, 7.8 Hz, 1H), 4.80 - 4.71 (m, 2H), 4.18 (t, *J* = *5.6* Hz, 4H), 3.81 - 3.46 (m, 2H), 2.79 - 2.53 (m, 2H), 2.20 (d, *J*= 31.1 Hz, 3H), 0.96 - 0.90 (m, 1H), 0.86 (qt, *J* = 8.0, 5.1 Hz, 1H), 0.82 - 0.75 (m, 1H), 0.48 *(dt, J*= 9.1, 4.7 Hz, 1H), 0.42 - 0.35 (m, 2H), 0.34 - 0.28 (m, 2H), 0.27 - 0.15 (m, 3H). |
| 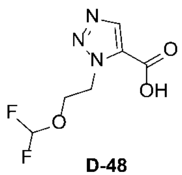 **D-48** | 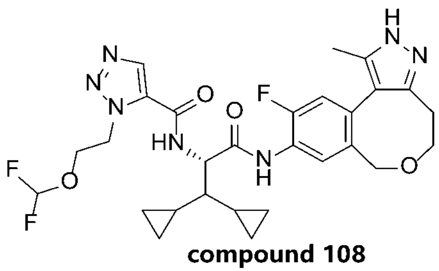 **compound 108** | MS (ESI) m/z =588 [M+1]$^+$ $^1$H NMR (600 MHz, DMSO-$d_6$) δ 12.69 - 12.29 (m, 1H), 10.06 (s, 1H), 8.91 (d, *J* = 8.9 Hz, 1H), 8.45 (s, 1H), 7.91 (d, *J* = 8.2 Hz, 1H), 7.24 (s, 1H), 6.60 (t, *J* = 75.1 Hz, 1H), 5.04 (t, *J* = 8.3 Hz, 1H), 4.95 (t, *J* = 5.3 Hz, 2H), 4.44 - 4.02 (m, 4H), 3.61 (s, 2H), 2.63 (s, 2H), 2.22 (s, 3H), 0.98 - 0.90 (m, 1H), 0.90 - 0.83 (m, 1H), 0.80 - 0.72 (m, 1H), 0.53 - 0.45 (m, 1H), 0.43 - 0.28 (m, 4H), 0.28 - 0.15 (m, 3H). |

(continued)

| Pyrazolecarboxylic acid | Structure of compound | $^{1}$HNMR and/or MS (ESI) |
|---|---|---|
| 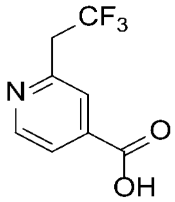 | 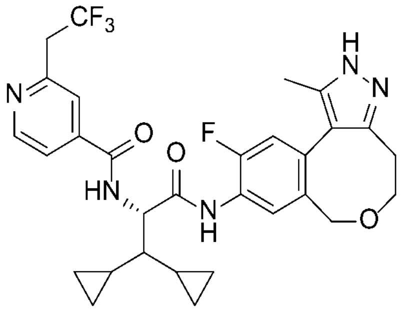 compound 109 | MS (ESI) m/z =586 [M+1]$^{+}$ $^{1}$H NMR (400 MHz, Methanol-$d_4$) δ 8.71 (dd, $J$ = 5.2, 0.8 Hz, 1H), 8.04 (d, $J$ = 8.0 Hz, 1H), 7.88 (d, $J$ = 1.5 Hz, 1H), 7.80 (dd, $J$ = 5.2, 1.6 Hz, 1H), 7.19 (d, $J$ = 11.4 Hz, 1H), 5.09 (d, $J$ = 7.2 Hz, 1H), 4.31 (s, 2H), 3.79 (q, $J$ = 10.8 Hz, 4H), 2.70 (s, 2H), 2.28 (s, 3H), 1.00 - 0.79 (m, 3H), 0.57 (qt, $J$ = 7.0, 3.1 Hz, 1H), 0.53 - 0.42 (m, 3H), 0.41 - 0.24 (m, 4H). |

**Example 110 Preparation of compound 110**

A preparation of compound 110 is illustrated as follows:

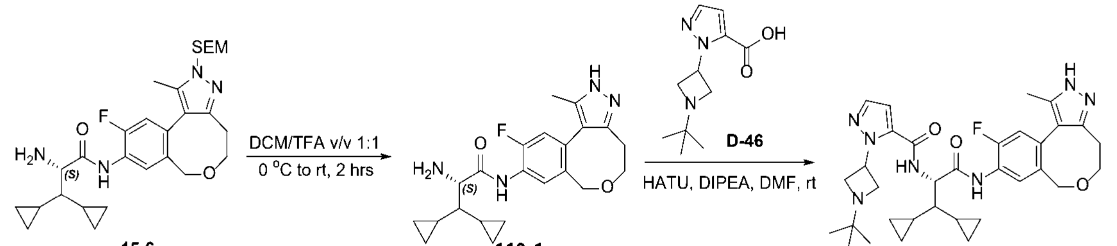


15-6 110-1 compound 110

(S1) Preparation of intermediate 110-1

To a solution of intermediate 15-6 (1.0 g, 1.89 mmol) in DCM (5 mL) was added TFA (5 mL) under an ice-water bath. After addition, the reaction mixture was stirred at room temperature for 2 h. Upon completion, the reaction mixture was concentrated to dryness under reduced pressure. The residue was purified by MPLC ($CH_3CN/H_2O$, 0.05% $NH_4HCO_3$) to obtain 534.36 mg (1.34 mmol) of intermediate 110-1 (yield: 70.89%). MS (ESI) m/z = 399 $[M+1]^+$.

(S2) Preparation of compound 110

To a solution of intermediate D-46 (30.82 mg, 138.19 μmol) and intermediate 110-1 (50 mg, 125.63 μmol) in DMF (1 mL) were added DIPEA (40.49 mg, 313.27 μmol, 54.56 μL) and HATU (42 mg, 110.46 μmol). After addition, the reaction mixture was stirred at room temperature for 1 h. Upon completion, the reaction mixture was purified by MPLC to obtain 38.12 mg (63.23 μmol) of compound 110 (yield: 50.33%). MS (ESI) m/z =604 $[M+1]^+$. $^1H$ NMR (400 MHz, Methanol-$d_4$) δ 8.03 (d, $J$ = 8.1 Hz, 1H), 7.71 (dd, $J$ = 16.7, 2.0 Hz, 1H), 7.20 (d, $J$ = 11.5 Hz, 1H), 7.02 (dd, $J$ = 12.6, 2.0 Hz, 1H), 6.07 (p, $J$ = 8.0 Hz, 1H), 4.98 (d, $J$ = 6.8 Hz, 1H), 4.83 - 4.70 (m, 2H), 4.61 (s, 1H), 4.51 - 4.15 (m, 3H), 3.72 (s, 2H), 2.70 (s, 2H), 2.28 (s, 3H), 1.38 (d, $J$ = 2.6 Hz, 9H), 1.01 - 0.79 (m, 3H), 0.63 - 0.22 (m, 8H).

**Examples 111-1115 Preparation of compounds 111-115**

The preparation of compounds 111 to 115 was performed according to the synthesis method in Example 110, in which intermediate D-46 in step (S2) was correspondingly replaced with a pyrazolecarboxylic acid from the following table, with all other reagents and procedures remaining identical.

| Pyrazolecarbo xylic acid | Structure of compound | $^1$HNMR and/or MS (ESI) |
|---|---|---|
| 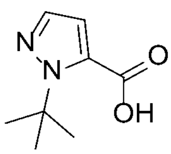 | 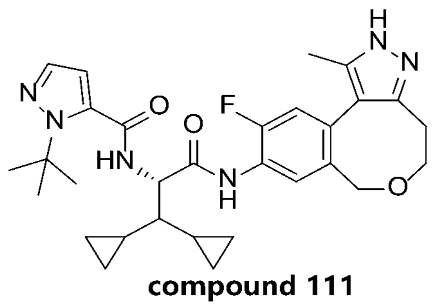 compound 111 | MS (ESI) m/z =549 $[M+1]^+$ $^1H$ NMR (600 MHz, Methanol-$d_4$) δ 8.02 (d, $J$ = 7.9 Hz, 1H), 7.42 (d, $J$ = 1.9 Hz, 1H), 7.19 (s, 1H), 6.56 (d, $J$ = 1.9 Hz, 1H), 4.97 (d, $J$ = 7.1 Hz, 1H), 4.32 (s, 2H), 3.66 (s, 2H), 2.70 (s, 2H), 2.28 (s, 3H), 1.67 (s, 9H), 0.97 - 0.79 (m, 3H), 0.62 - 0.55 (m, 1H), 0.54 - 0.47 (m, 2H), 0.47 - 0.36 (m, 2H), 0.34 - 0.26 (m, 3H). |

(continued)

| Pyrazolecarbo xylic acid | Structure of compound | $^1$HNMR and/or MS (ESI) |
| --- | --- | --- |
| 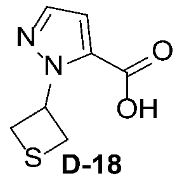 D-18 | 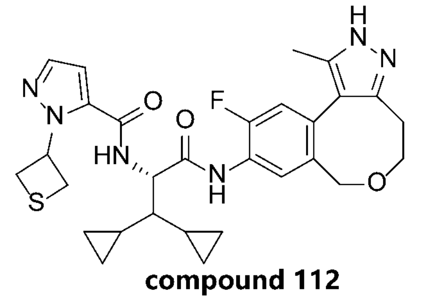 compound 112 | MS (ESI) m/z =565 [M+1]$^+$ $^1$H NMR (400 MHz, DMSO-$d_6$) δ 12.47 (d, *J* = 16.8 Hz, 1H), 10.03 (s, 1H), 8.54 (d, *J* = 8.7 Hz, 1H), 7.93 (d, *J* = 8.2 Hz, 1H), 7.65 (d, *J* = 2.0 Hz, 1H), 7.24 (d, *J* = 11.3 Hz, 1H), 7.07 (d, *J* = 2.0 Hz, 1H), 6.41 (p, *J* = 8.8 Hz, 1H), 5.04 - 4.94 (m, 1H), 4.20 (s, 2H), 4.03 (p, *J* = 0.8 Hz, 1H), 3.94 (dt, *J* = 17.9, 8.8 Hz, 2H), 3.61 (s, 2H), 3.39 - 3.35 (m, 1H), 2.60 (s, 2H), 2.21 (s, 3H), 1.00 - 0.73 (m, 3H), 0.49 (tq, *J* = 8.6, 3.8 Hz, 1H), 0.43 - 0.11 (m, 7H). |
| 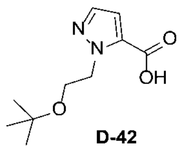 D-42 | 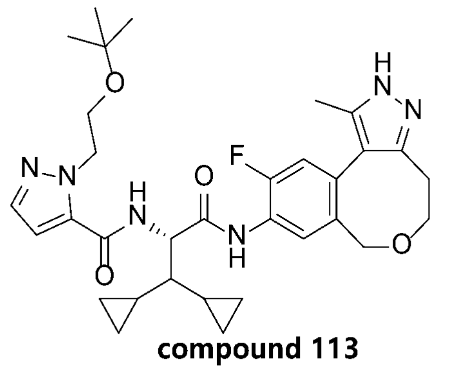 compound 113 | MS (ESI) m/z =593 [M+1]$^+$ $^1$H NMR (400 MHz, Methanol-$d_4$) δ 8.04 (dd, *J* = 8.0, 2.2 Hz, 1H), 7.52 (d, *J* = 2.1 Hz, 1H), 7.19 (d, *J* = 11.4 Hz, 1H), 6.83 (d, *J* = 2.1 Hz, 1H), 5.01 (d, *J* = 7.0 Hz, 1H), 4.73 (dt, *J* = 13.7, 5.9 Hz, 1H), 4.58 (dt, *J* = 13.7, 5.5 Hz, 1H), 4.31 (s, 2H), 3.83 - 3.57 (m, 4H), 2.70 (s, 2H), 2.28 (s, 3H), 1.03 (s, 9H), 0.94 - 0.81 (m, 3H), 0.61 - 0.34 (m, 6H), 0.29 (s, 2H). |
| 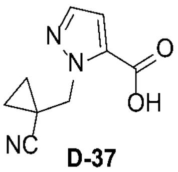 D-37 | 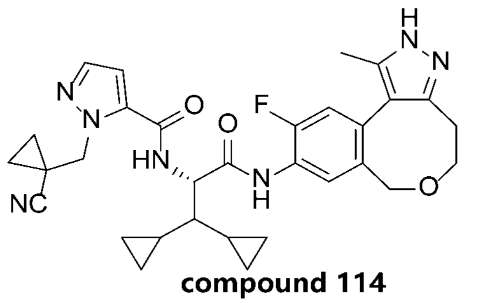 compound 114 | MS (ESI) m/z =572 [M+1]$^+$ $^1$H NMR (400 MHz, Methanol-$d_4$) δ 8.01 (d, *J* = 8.0 Hz, 1H), 7.60 (d, *J* = 2.1 Hz, 1H), 7.18 (d, *J* = 11.3 Hz, 1H), 6.97 (d, *J* = 2.0 Hz, 1H), 5.00 (d, *J* = 6.6 Hz, 1H), 4.82 (d, *J* = 14.4 Hz, 1H), 4.69 (d, *J* = 14.4 Hz, 1H), 4.31 (s, 2H), 3.71 (s, 2H), 2.70 (s, 2H), 2.28 (s, 3H), 1.36 - 1.19 (m, 4H), 1.01 - 0.77 (m, 3H), 0.62 - 0.36 (m, 5H), 0.35 - 0.20 (m, 3H). |
| 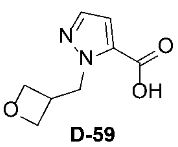 D-59 | 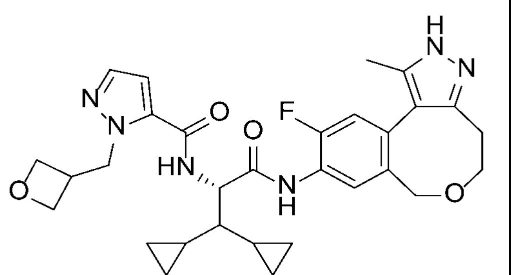 compound 115 | MS (ESI) m/z =563 [M+1]$^+$ $^1$H NMR (600 MHz, DMSO-$d_6$) δ 12.48 (d, *J* = 32.6 Hz, 1H), 10.00 (s, 1H), 8.52 (d, *J* = 8.7 Hz, 1H), 7.92 (d, *J* = 8.2 Hz, 1H), 7.52 (d, *J* = 2.0 Hz, 1H), 7.24 (s, 1H), 7.04 (d, *J* = 2.1 Hz, 1H), 4.99 - 4.93 (m, 1H), 4.83 - 4.73 (m, 2H), 4.64 - 4.54 (m, 2H), 4.40 (dt, *J* = 10.0, 6.1 Hz, 2H), 4.26 - 4.01 (m, 2H), 3.70 - 3.56 (m, 2H), 3.17 (d, *J* = 5.2 Hz, 1H), 2.55 (s, 2H), 2.20 (d, *J* = 34.9 Hz, 3H), 1.00 - 0.75 (m, 3H), 0.53 - 0.45 (m, 1H), 0.43 - 0.29 (m, 4H), 0.29 - 0.15 (m, 3H). |

**Example 116 Preparation of compound 116**

A preparation of compound 116 is illustrated as follows:

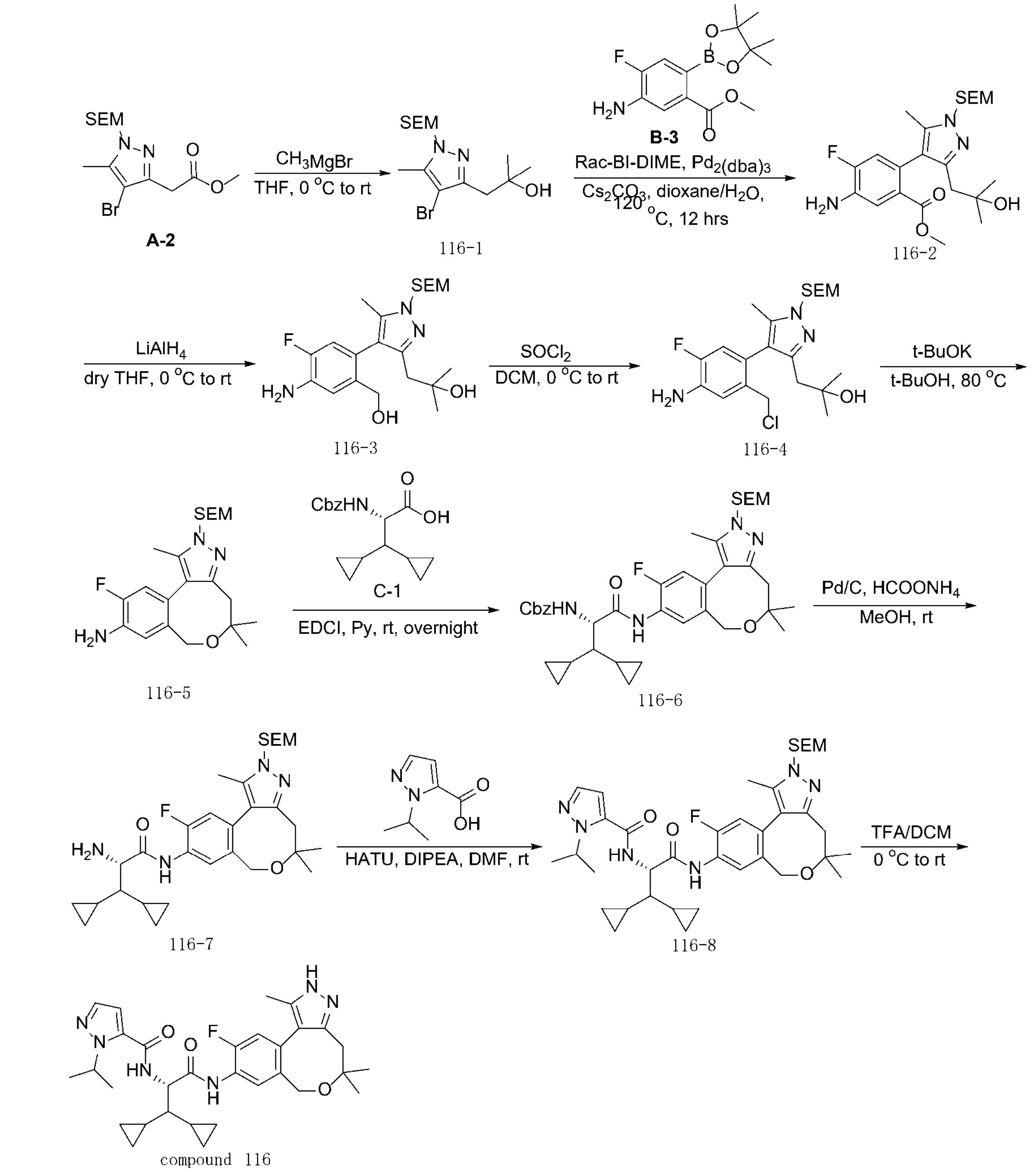

compound 116

(S1) Preparation of intermediate 116-1

To a solution of intermediate A-2 (10 g, 27.52 mmol) in dry THF (100 mL) was added dropwise methylmagnesium bromide (110.10 mmol, 37 mL) at 0°C under a nitrogen atmosphere and an ice-water bath. After addition, the reaction mixture was warmed to room temperature and stirred for 3 h. Upon completion, the reaction mixture was decanted into ice-cold aqueous ammonium chloride solution and extracted with EA. The EA layers were collected, combined, washed with a saturated NaCl solution, dried over anhydrous sodium sulfate, and filtered. The filtrate was concentrated under reduced pressure to dryness. The residue was purified by reverse-phase MPLC ($CH_3CN/H_2O$, 0.05% TFA) to obtain 5.3 g (14.59 mmol) of intermediate 116-1 (yield: 52.99%). MS (ESI) m/z = 363 $[M+1]^+$.

(S2) Preparation of intermediate 116-2

To a pear-shaped flask were added intermediate 116-1 (3.8 g, 10.46 mmol), intermediate B-3 (3.39 g, 11.50 mmol), $Pd_2(dba)_3$ (478.82 mg, 522.89 μmol), Rac-BI-DIME (345.48 mg, 1.05 mmol), and 1,4-dioxane/water (38 mL/7.6

mL). $Cs_2CO_3$ (10.96 g, 20.92 mmol) was added under stirring. The reaction mixture was evacuated and purged with nitrogen three times, heated to 100°C, and stirred for 12 h. Upon completion, the reaction mixture was filtered through Celite while hot. The filtrate was concentrated under reduced pressure to dryness. The residue was purified by silica gel column chromatography to obtain 913 mg (2.02 mmol) of intermediate 116-2 (yield: 19.29%). MS (ESI) m/z = 452 $[M+1]^+$.

(S3) Preparation of intermediate 116-3

To a solution of intermediate 116-2 (913 mg, 2.02 mmol) in dry THF (10 mL) was added dropwise $LiAlH_4$ (6.07 mmol, 6 mL, 1M in THF) at 0°C under an ice-water bath. After addition, the reaction mixture was warmed to room temperature and reacted for 2 h. Upon completion, the reaction was quenched by sequential addition of ice water (0.23 mL), 15% aqueous NaOH solution (0.23 mL) and water (0.69 mL) at -10°C. The mixture was dried over anhydrous sodium sulfate and filtered. The filter cake was washed with EA. The filtrate was concentrated under reduced pressure to dryness to obtain 570 mg (1.35 mmol) of intermediate 116-3 (yield: 66.56%). MS (ESI) m/z = 424 $[M+1]^+$, requiring no purification for the next step.

(S4-S5) Preparation of intermediate 116-5

The preparation of intermediate 116-5 was performed according to the synthesis method in Example 1, in which intermediate 1-2 was replaced with intermediate 116-3, with all other reagents and procedures remaining identical. MS (ESI) m/z = 406 $[M+1]^+$.

(S6) Preparation of intermediate 116-6

To a pear-shaped flask were added intermediate 116-5 (58 mg, 143.00 μmol), intermediate C-1 (43.38 mg, 143.00 μmol), EDCI (82.37 mg, 429.01 μmol) and pyridine (2.5 mL). The reaction mixture was stirred at room temperature overnight. Upon completion, the reaction mixture was concentrated under reduced pressure to dryness. The residue was purified by reverse-phase MPLC ($CH_3CN/H_2O$, 0.05% TFA) to obtain 57 mg (82.50 μmol) of intermediate 116-6 (yield: 57.69%). MS (ESI) m/z = 691 $[M+1]^+$.

(S7-S9) Preparation of compound 116

The preparation of compound 116 was performed according to the synthesis method in Example 1, in which intermediate 1-5 was replaced with intermediate 116-6, with all other reagents and procedures remaining identical. MS (ESI) m/z = 563 $[M+1]^+$. $^1H$ NMR (600 MHz, Methanol-$d_4$) δ 8.03 (d, *J* = 7.9 Hz, 1H), 7.52 (d, *J* = 2.0 Hz, 1H), 7.16 (s, 1H), 6.79 (d, *J*= 2.0 Hz, 1H), 5.41 (p, *J* = 6.6 Hz, 1H), 5.01 (d, *J* = 7.3 Hz, 1H), 4.26 (s, 2H), 2.59 (s, 2H), 2.29 (s, 3H), 1.46 (t, *J* = 7.0 Hz, 6H), 1.23 (d, *J* = 3.0 Hz, 6H), 0.95 - 0.80 (m, 3H), 0.60 - 0.43 (m, 4H), 0.42 - 0.35 (m, 1H), 0.34 - 0.24 (m, 3H).

**Example 117 Preparation of compound 117**

A structure of compound 117 is as follows:

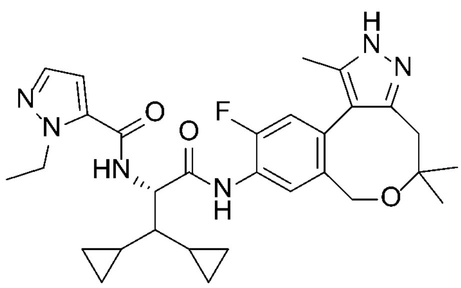

compound 117

.

The preparation of compound 117 was performed according to the synthesis method in Example 116, in which 1-isopropylpyrazole-5-carboxylic acid in step (S8) was replaced with ethylpyrazole-5-carboxylic acid, with all other reagents and procedures remaining identical. MS (ESI) m/z = 549 $[M+1]^+$. $^1H$ NMR (600 MHz, Methanol-$d_4$) δ 8.03 (d, *J* = 8.0 Hz, 1H), 7.51 (d, *J* = 2.1 Hz, 1H), 7.16 (d, *J* = 11.2 Hz, 1H), 6.87 (d, *J* = 2.1 Hz, 1H), 5.01 (d, *J* = 7.2 Hz, 1H), 4.55 (q, *J* = 7.2 Hz, 2H), 4.26 (s, 2H), 2.59 (s, 2H), 2.29 (s, 3H), 1.40 (t, *J* = 7.2 Hz, 3H), 1.23 (d, *J* = 2.9 Hz, 6H), 0.95 - 0.81 (m, 3H), 0.61 - 0.43

(m, 4H), 0.42 - 0.36 (m, 1H), 0.35 - 0.25 (m, 3H).

**Example 118 Preparation of compounds 118a and 118b**

A preparation of compounds 118a and 118b is illustrated as follows:

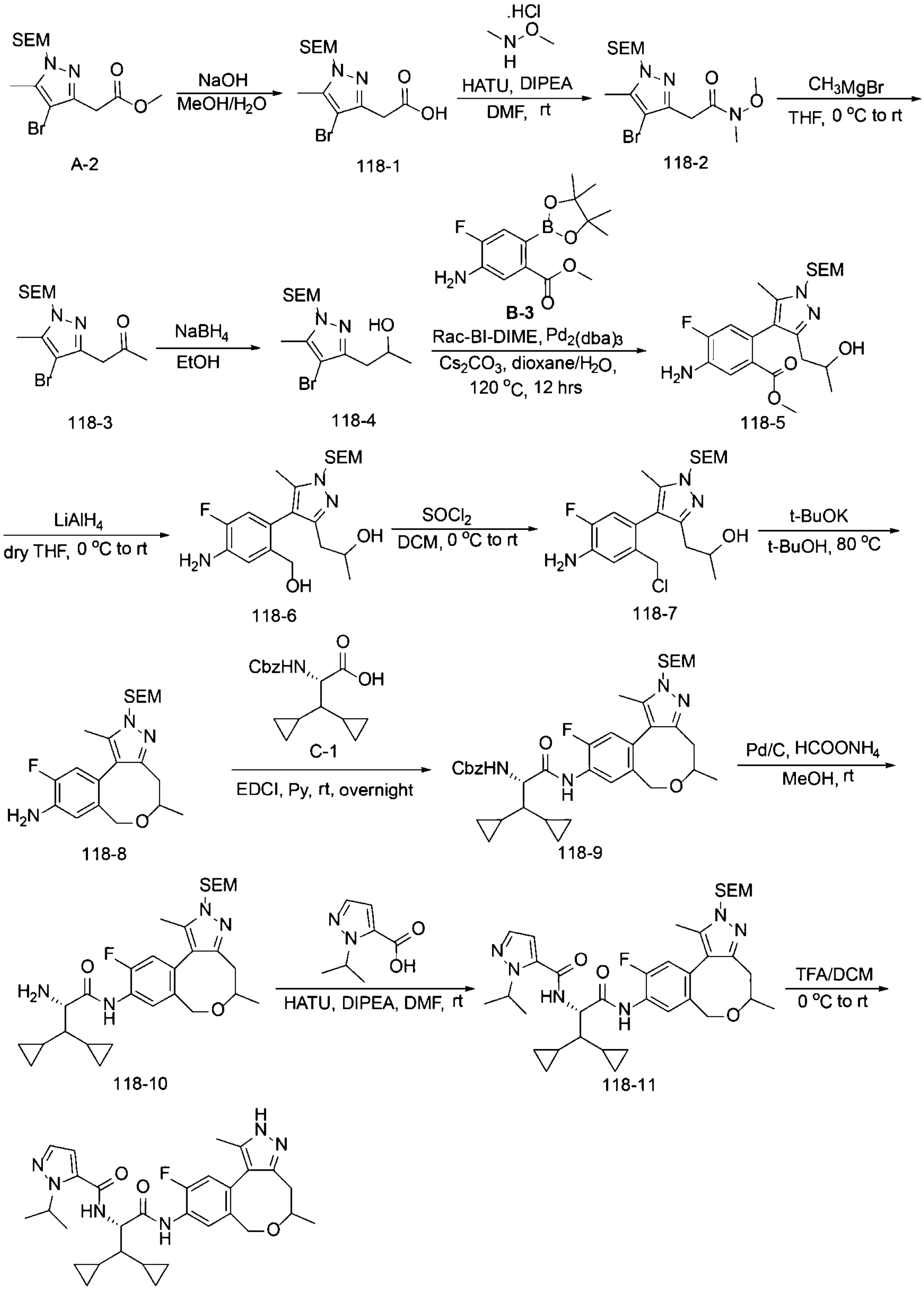

compound 118a & compound 118b

.

(S1) Preparation of intermediate 118-1

To a single-neck flask were added intermediate A-2 (30 g, 82.57 mmol) and methanol (240 mL). A solution of NaOH (4.95 g, 123.86 mmol) in water (80 mL) was added under an ice-water bath. After addition, the reaction mixture was

stirred at room temperature for 1 h. Upon completion, the reaction mixture was cooled to 0°C and adjusted to pH 4 with 6 M HCl, then extracted with EA. The EA layer was collected, washed with a saturated NaCl solution, dried over anhydrous sodium sulfate, and filtered. The filtrate was concentrated under reduced pressure to dryness to obtain 28.7 g (82.17 mmol) of intermediate 118-1 (yield: 99.51%). MS (ESI) m/z = 349 $[M+1]^+$.

(S2) Preparation of intermediate 118-2

To a single-neck flask were added intermediate 118-1 (28.7 g, 82.17 mmol), N,O-dimethylhydroxylamine hydrochloride (6.52 g, 106.82 mmol) and DMF (280 mL), followed by addition of HATU (46.86 g, 123.25 mmol) and DIPEA (31.86 g, 246.50 mmol, 42.93 mL) under an ice-water bath. After addition, the reaction mixture was stirred at room temperature for 2 h. Upon completion, the reaction mixture was decanted into ice water and extracted with EA. The EA layer was collected, washed with a saturated NaCl solution, dried over anhydrous sodium sulfate, and filtered. The filtrate was concentrated under reduced pressure to dryness. The residue was purified by reverse-phase MPLC to obtain 20 g (50.97 mmol) of intermediate 118-2 (yield: 62.04%). MS (ESI) m/z = 392 $[M+1]^+$.

(S3) Preparation of intermediate 118-3

To a solution of intermediate 118-2 (15 g, 38.23 mmol) in dry THF (150 mL) was added dropwise methylmagnesium bromide (76.46 mmol, 26 mL) at 0°C under a nitrogen atmosphere under an ice-water bath. After addition, the reaction mixture was warmed to room temperature and stirred for 2 h. Upon completion, the reaction mixture was decanted into ice-cold aqueous ammonium chloride solution and extracted with EA. The EA layers were collected, combined, washed with a saturated NaCl solution, dried over anhydrous sodium sulfate, and filtered. The filtrate was concentrated under reduced pressure to dryness. The residue was purified by reverse-phase MPLC ($CH_3CN/H_2O$, 0.05% TFA) to obtain 6.3 g (18.14 mmol) of intermediate 118-3 (yield: 47.45%). MS (ESI) m/z = 347 $[M+1]^+$.

(S4) Preparation of intermediate 118-4

To a single-neck flask were added intermediate 118-3 (4 g, 11.52 mmol) and ethanol (40 mL). Sodium borohydride (653.51 mg, 17.28 mmol) was added in portions under an ice-water bath. After addition, the reaction mixture was stirred under the ice-water bath for 1 h. Upon completion, the reaction mixture was decanted into ice-cold aqueous ammonium chloride solution to quench and extracted with EA. The EA layer was collected, washed with a saturated NaCl solution, dried over anhydrous sodium sulfate, and filtered. The filtrate was concentrated under reduced pressure to dryness to obtain 4.02 g (11.51 mmol) of intermediate 118-4 (yield: 100.00%). MS (ESI) m/z = 349 $[M+1]^+$.

(S5-S12) Preparation of compound 118a and 118b

The preparation of compounds 118a and 118b was performed according to the synthesis method in Example 116, in which intermediate 116-1 was replaced with intermediate 118-4, with all other reagents and procedures remaining identical. After SEM group was removed, the crude product was subjected to purification by MPLC ($H_2O/CH_3CN$, 0.05% $NH_4HCO_3$) and separation by SFC to obtain compounds 118a and 118b, in which SFC was performed using a Daicel CHIRALPAK AD-3 column (3 $\mu$m, 150 mm $\times$ 3 mm) with $CO_2$ (as mobile phase A) and ethanol (as mobile phase B) (A/B = 70/30), at a flow rate of 1 mL/min and a column temperature of 40°C.

For compound 118a (P1, SFC $t_R$ = 1.760), MS (ESI) m/z = 549 $[M+1]^+$. $^1$H NMR (600 MHz, Methanol-$d_4$) $\delta$ 8.04 (d, *J* = 7.9 Hz, 1H), 7.53 (d, *J* = 2.0 Hz, 1H), 7.18 (s, 1H), 6.80 (d, *J* = 2.0 Hz, 1H), 5.41 (sept, *J* = 6.7 Hz, 1H), 5.01 (d, *J* = 7.3 Hz, 1H), 4.67 (d, *J* = 12.5 Hz, 1H), 4.01 (d, *J* = 12.7 Hz, 1H), 3.64 (s, 1H), 2.86 (d, *J* = 15.0 Hz, 1H), 2.28 (s, 4H), 1.46 (t, *J* = 6.8 Hz, 6H), 1.26 (d, *J* = 6.3 Hz, 3H), 0.95 - 0.85 (m, 2H), 0.82 (td, *J* = 9.3, 7.4 Hz, 1H), 0.63 - 0.54 (m, 1H), 0.54 - 0.42 (m, 3H), 0.38 (dq, *J* = 9.7, 4.9 Hz, 1H), 0.30 (ddt, *J* = 10.7, 8.8, 5.2 Hz, 3H).

For compound 118b (P2, SFC $t_R$ = 1.880), MS (ESI) m/z = 549 $[M+1]^+$. $^1$H NMR (600 MHz, Methanol-$d_4$) $\delta$ 8.02 (d, *J* = 7.9 Hz, 1H), 7.53 (d, *J* = 2.0 Hz, 1H), 7.18 (s, 1H), 6.80 (d, *J* = 2.0 Hz, 1H), 5.41 (sept, *J* = 6.7 Hz, 1H), 5.00 (d, *J* = 7.3 Hz, 1H), 4.67 (d, *J* = 12.7 Hz, 1H), 4.02 (d, *J* = 12.8 Hz, 1H), 3.64 (s, 1H), 2.85 (d, *J* = 14.9 Hz, 1H), 2.28 (s, 4H), 1.46 (t, *J* = 7.0 Hz, 6H), 1.25 (d, *J* = 6.3 Hz, 3H), 0.97 - 0.85 (m, 2H), 0.82 (td, *J* = 9.2, 7.2 Hz, 1H), 0.57 (tq, *J* = 8.3, 4.0 Hz, 1H), 0.54 - 0.41 (m, 3H), 0.38 (dq, *J* = 9.7, 4.9 Hz, 1H), 0.29 (ddp, *J* = 18.3, 9.3, 4.7 Hz, 3H).

**Example 119 Preparation of compound 119a and 119b**

A structure of compound 119a/119b is as follows:

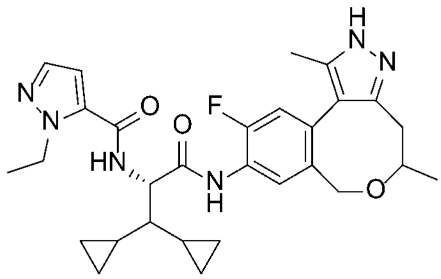

compound 119a & compound 119b

.

The preparation of compounds 119a and 119b was performed according to the synthesis method in Example 118, in which 1-isopropylpyrazole-5-carboxylic acid in step (S11) was replaced with ethylpyrazole-5-carboxylic acid, with all other reagents and procedures remaining identical. After SEM group was removed, the crude product was subjected to purification by MPLC ($H_2O/CH_3CN$, 0.05% $NH_4HCO_3$) and separation by SFC to obtain compounds 119a and 119b, in which SFC was performed using a Daicel CHIRALPAK AD-3 column (3 μm, 150 mm × 3 mm) with $CO_2$ (as mobile phase A) and ethanol (as mobile phase B) (A/B = 70/30), at a flow rate of 1 mL/min and a column temperature of 40°C.

For compound 119a (P1, SFC $t_R$ = 1.902), MS (ESI) m/z = 535 $[M+1]^+$. $^1H$ NMR (600 MHz, Methanol-$d_4$) δ 8.05 (d, *J* = *7.9* Hz, 1H), 7.53 (d, *J* = 2.1 Hz, 1H), 7.20 (s, 1H), 6.89 (d, *J* = 2.1 Hz, 1H), 5.03 (d, *J* = 7.3 Hz, 1H), 4.69 (d, *J* = 12.4 Hz, 1H), 4.57 (q, *J* = 7.2 Hz, 2H), 4.03 (d, *J* = 12.7 Hz, 1H), 3.66 (s, 1H), 2.88 (d, *J* = 15.0 Hz, 1H), 2.30 (s, 4H), 1.42 (t, *J* = 7.2 Hz, 3H), 1.28 (d, *J* = 6.3 Hz, 3H), 1.01 - 0.87 (m, 2H), 0.84 (td, *J* = 9.2, 7.3 Hz, 1H), 0.59 (tdd, *J* = 8.0, 4.9, 3.6 Hz, 1H), 0.56 - 0.43 (m, 3H), 0.40 (dq, *J* = 9.7, 4.9 Hz, 1H), 0.36 - 0.22 (m, 3H).

For compound 119b (P2, SFC $t_R$ = 2.145), MS (ESI) m/z = 535 $[M+1]^+$. $^1H$ NMR (600 MHz, Methanol-$d_4$) δ 8.01 (d, *J* = *7.9* Hz, 1H), 7.51 (d, *J* = 2.0 Hz, 1H), 7.18 (s, 1H), 6.87 (d, *J* = 2.1 Hz, 1H), 5.00 (d, *J* = 7.1 Hz, 1H), 4.67 (d, *J* = 12.7 Hz, 1H), 4.56 (q, *J* = 7.1 Hz, 2H), 4.02 (d, *J* = 12.7 Hz, 1H), 3.64 (s, 1H), 2.85 (d, *J* = 15.0 Hz, 1H), 2.28 (s, 4H), 1.40 (t, *J* = 7.2 Hz, 3H), 1.25 (d, *J* = 6.3 Hz, 3H), 0.95 - 0.85 (m, 2H), 0.83 (td, *J* = 9.3, 7.2 Hz, 1H), 0.57 (tq, *J* = 8.2, 4.0 Hz, 1H), 0.54 - 0.41 (m, 3H), 0.38 (dq, *J* = 9.7, 4.9 Hz, 1H), 0.29 (ddt, *J* = 12.2, 9.0, 5.1 Hz, 3H).

## Example 120 Preparation of compound 120a and 120b

A structure of compound 120a/120b is as follows:

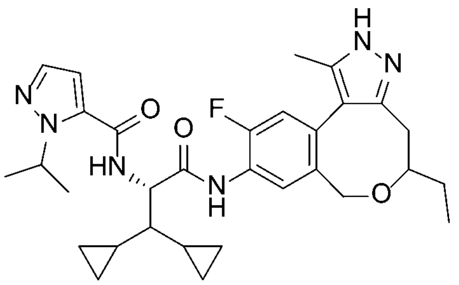

compound 120a & compound 120b

.

The preparation of compounds 120a and 120b was performed according to the synthesis method in Example 118, in which methylmagnesium bromide in step (S3) was replaced with ethylmagnesium bromide, with all other reagents and procedures remaining identical. After SEM group was removed, the crude product was subjected to purification by MPLC ($H_2O/CH_3CN$, 0.05% $NH_4HCO_3$) and separation by SFC to obtain compounds 120a and 120b, in which SFC was performed using a Daicel CHIRALPAK AS-3 column (3 μm, 150 mm × 3 mm) with $CO_2$ (as mobile phase A) and ethanol (as mobile phase B) (A/B = 80/20), at a flow rate of 1 mL/min and a column temperature of 40°C.

For compound 120a (P1, SFC $t_R$ = 1.749), MS (ESI) m/z = 563 $[M+1]^+$. $^1H$ NMR (400 MHz, Methanol-$d_4$) δ 8.04 (d, *J* = 8.0 Hz, 1H), 7.52 (d, *J* = 2.1 Hz, 1H), 7.18 (d, *J* = 11.4 Hz, 1H), 6.80 (d, *J* = 2.0 Hz, 1H), 5.41 (p, *J* = 6.7 Hz, 1H), 5.01 (d, *J* = 7.1 Hz, 1H), 4.69 (d, *J* = 12.5 Hz, 1H), 4.00 (d, *J* = 12.5 Hz, 1H), 3.34 (s, 1H), 2.88 (d, *J* = 14.9 Hz, 1H), 2.27 (s, 4H), 1.65 - 1.52 (m, 2H), 1.46 (dd, *J* = 6.7, 4.8 Hz, 6H), 1.01 (t, *J* = 7.4 Hz, 3H), 0.96 - 0.77 (m, 3H), 0.62 - 0.53 (m, 1H), 0.48 (qdd, *J* = 9.0, 5.7, 3.1 Hz, 3H), 0.38 (dt, *J* = 9.4, 4.2 Hz, 1H), 0.35 - 0.21 (m, 3H).

For compound 120b (P2, SFC $t_R$ = 1.974), MS (ESI) m/z = 563 $[M+1]^+$. $^1H$ NMR (400 MHz, Methanol-$d_4$) δ 8.03 (d, *J* = 8.0 Hz, 1H), 7.52 (d, *J* = 2.1 Hz, 1H), 7.18 (d, *J* = 11.4 Hz, 1H), 6.80 (d, *J* = 2.0 Hz, 1H), 5.50 - 5.31 (m, 1H), 5.01 (d, *J* = 7.0 Hz, 1H), 4.68 (d, *J* = 12.6 Hz, 1H), 4.00 (d, *J* = 12.6 Hz, 1H), 3.32 (s, 1H), 2.87 (d, *J* = 14.8 Hz, 1H), 2.27 (s, 4H), 1.63 - 1.52

(m, 2H), 1.46 (dd, $J$ = 6.7, 4.9 Hz, 6H), 1.01 (d, $J$ = 7.4 Hz, 3H), 0.96 - 0.79 (m, 3H), 0.61 - 0.53 (m, 1H), 0.48 (ddtd, $J$ = 13.2, 9.3, 4.6, 4.2, 2.8 Hz, 3H), 0.42 - 0.35 (m, 1H), 0.35 - 0.25 (m, 3H).

**Example 121 Preparation of compound 121**

A preparation of compound 121 is illustrated as follows:

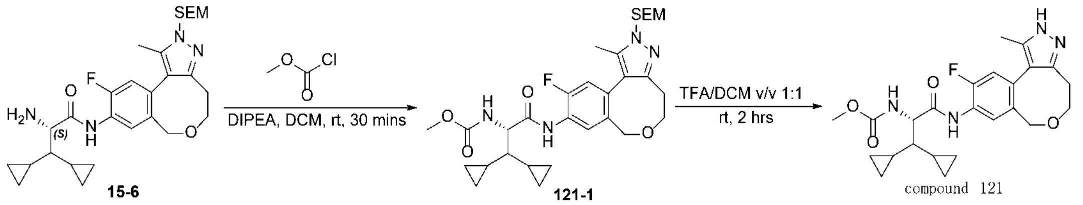


15-6 121-1 compound 121

(S1) Preparation of intermediate 121-1

To a single-neck flask were added intermediate 15-6 (50 mg, 94.57 μmol) and dry DCM (20 mL). DIPEA (24.44 mg, 189.13 μmol, 32.94 μL) was added. Methyl chloroformate (9.83 mg, 104.02 μmol) was added dropwise under an ice-water bath, and then the mixture was warmed to room temperature and stirred for 0.5 h. Upon completion, the reaction mixture was quenched by water, and extracted with DCM. The DCM layer was collected, washed with a saturated NaCl solution, dried over anhydrous sodium sulfate, and filtered. The filtrate was concentrated under reduced pressure to dryness to obtain 52 mg (88.62 μmol) of intermediate 121-1 (yield: 93.71%). MS (ESI) m/z = 587 $[M+1]^+$.

(S2) Preparation of compound 121

To a solution of intermediate 121-1 (52 mg, 88.62 μmol) in DCM (1 mL) was added TFA (1 mL). After addition, the reaction mixture was reacted at room temperature for 2 h. Upon completion, the reaction mixture was concentrated to dryness, dissolved in DCM, and adjusted to pH = 8 with triethylamine under an ice-water bath, then concentrated under reduced pressure to dryness. The residue was purified by reverse-phase MPLC ($H_2O/CH_3CN$, 0.05% $NH_4HCO_3$) to obtain 17 mg (37.24 μmol) of compound 121 (yield: 42.02%). MS (ESI) m/z = 457 $[M+1]^+$. $^1H$ NMR (600 MHz,

DMSO-$d_6$) δ 12.43 (d, $J$ = 29.0 Hz, 1H), 9.74 (s, 1H), 7.86 (d, $J$ = 8.1 Hz, 1H), 7.31 (d, $J$ = 9.0 Hz, 1H), 7.19 (s, 1H), 4.45 (dd, $J$ = 9.0, 6.4 Hz, 1H), 4.32 - 3.93 (m, 2H), 3.71 - 3.55 (m, 2H), 3.54 (s, 3H), 2.76 - 2.49 (m, 2H), 2.17 (s, 3H), 0.94 - 0.84 (m, 1H), 0.81 - 0.73 (m, 1H), 0.61 - 0.53 (m, 1H), 0.47 - 0.38 (m, 1H), 0.37 - 0.29 (m, 1H), 0.29 - 0.21 (m, 2H), 0.21 - 0.11 (m, 4H).

**Example 122 Preparation of compound 122**

A preparation of compound 122 is illustrated as follows:

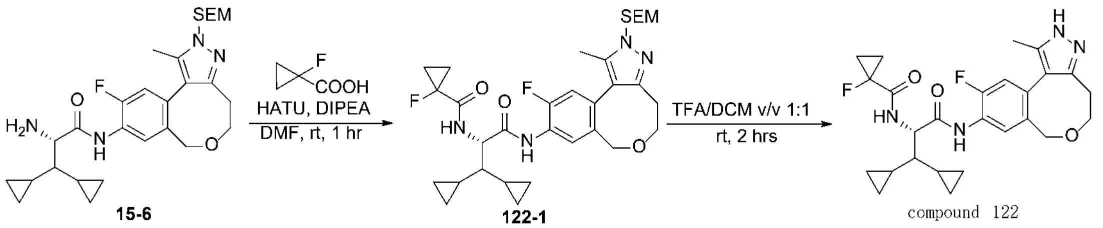


15-6 122-1 compound 122

The preparation of compound 122 was performed according to the synthesis method in Example 15, in which 1-isopropylpyrazole-5-carboxylic acid in step (S7) was replaced with 1-fluorocyclopropanecarboxylic acid, with all other reagents and procedures remaining identical. MS (ESI) m/z = 485 $[M+1]^+$.

**Example 123 Preparation of compound 123a and 123b**

A structure of compound 123a/123b is as follows:

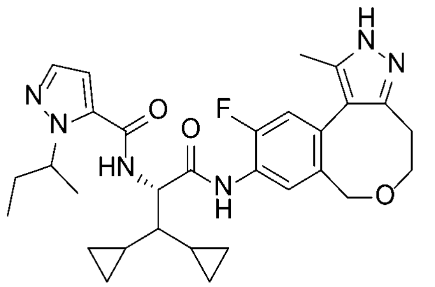

compound 123a & compound 123b

The preparation of compounds 123a and 123b was performed according to the synthesis method in Example 33, in which 3-ethylisoxazole-4-carboxylic acid in step (S1) was replaced with intermediate D-8, with all other reagents and procedures remaining identical. After SEM group was removed, the crude product was subjected to purification by MPLC (under basic conditions) and separation by SFC to obtain compounds 123a and 123b, in which SFC was performed using a Daicel CHIRALPAK IG column (3 μm, 150 mm × 3 mm) with $CO_2$ (as mobile phase A) and ethanol (as mobile phase B) (A/B = 75/25), at a flow rate of 1 mL/min and a column temperature of 40°C.

For compound 123a (P1, SFC $t_R$ = 3.993), MS (ESI) m/z = 549 $[M+1]^+$. $^1H$ NMR (400 MHz, Methanol-$d_4$) δ 8.04 (d, *J* = 8.0 Hz, 1H), 7.55 (d, *J* = 2.1 Hz, 1H), 7.19 (d, *J* = 11.4 Hz, 1H), 6.78 (d, *J* = 2.0 Hz, 1H), 5.19 (dp, *J* = 9.1, 6.5 Hz, 1H), 5.00 (d, *J* = 7.1 Hz, 1H), 4.31 (s, 2H), 3.71 (s, 2H), 2.70 (s, 2H), 2.27 (s, 3H), 1.93 (ddq, *J* = 14.6, 9.1, 7.4 Hz, 1H), 1.81 - 1.70 (m, 1H), 1.47 (d, *J* = 6.7 Hz, 3H), 0.92 - 0.81 (m, 3H), 0.72 (t, *J* = 7.4 Hz, 3H), 0.60 - 0.36 (m, 5H), 0.34 - 0.23 (m, 3H).

For compound 123b (P2, SFC $t_R$ = 4.966), MS (ESI) m/z = 549 $[M+1]^+$. $^1H$ NMR (400 MHz, Methanol-$d_4$) δ 8.04 (d, *J* = 8.0 Hz, 1H), 7.55 (d, *J* = 2.0 Hz, 1H), 7.19 (d, *J* = 11.5 Hz, 1H), 6.81 (d, *J* = 2.0 Hz, 1H), 5.22 (dp, *J* = 9.1, 6.6 Hz, 1H), 5.01 (d, *J* = 6.8 Hz, 1H), 4.31 (s, 2H), 3.72 (s, 2H), 2.70 (s, 2H), 2.28 (s, 3H), 1.96 (ddq, *J* = 14.7, 9.1, 7.4 Hz, 1H), 1.84 - 1.71 (m, 1H), 1.46 (d, J= 6.6 Hz, 3H), 0.97 - 0.81 (m, 3H), 0.74 (t, *J* = 7.4 Hz, 3H), 0.61 - 0.34 (m, 5H), 0.34 - 0.23 (m, 3H).

## Example 124 Preparation of compound 124

A structure of compound 124 is as follows:

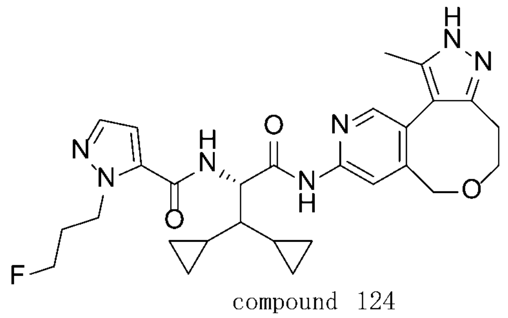

compound 124

.

The preparation of compound 124 was performed according to the synthesis method in Example 12, in which 1-isopropylpyrazole-5-carboxylic acid in step (S8) was replaced with intermediate D-21, with all other reagents and procedures remaining identical. MS (ESI) m/z = 536 $[M+1]^+$. $^1H$ NMR (600 MHz, Methanol-$d_4$) δ 8.33 (s, 1H), 8.18 (s, 1H), 7.53 (d, *J* = 2.1 Hz, 1H), 6.91 (d, *J* = 2.1 Hz, 1H), 4.98 (d, *J* = 6.6 Hz, 1H), 4.65 (td, *J* = 6.9, 2.1 Hz, 2H), 4.45 (td, *J* = 6.0, 1.6 Hz, 1H), 4.37 (td, *J* = 5.9, 1.3 Hz, 1H), 4.27 (s, 2H), 3.76 (s, 2H), 2.72 (s, 2H), 2.30 (s, 3H), 2.24 - 2.14 (m, 2H), 0.98 - 0.81 (m, 3H), 0.59 - 0.53 (m, 1H), 0.52 - 0.42 (m, 2H), 0.41 - 0.32 (m, 2H), 0.33 - 0.27 (m, 2H), 0.26 - 0.21 (m, 1H).

## Example 125 Preparation of compound 125

A structure of compound 125 is as follows:

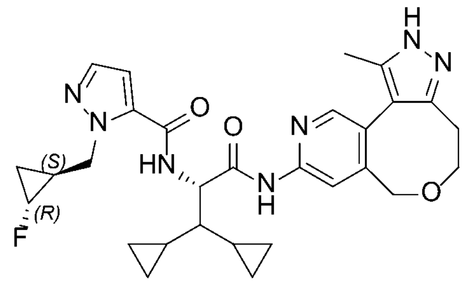

compound 125

The preparation of compound 125 was performed according to the synthesis method in Example 12, in which 1-isopropylpyrazole-5-carboxylic acid in step (S8) was replaced with intermediate D-35, with all other reagents and procedures remaining identical. MS (ESI) m/z = 548 $[M+1]^+$. $^1$H NMR (400 MHz, Methanol-$d_4$) δ 8.33 (s, 1H), 8.21 (s, 1H), 7.54 (dd, *J* = 2.2, 1.0 Hz, 1H), 6.93 (t, *J* = 1.9 Hz, 1H), 5.03 - 4.95 (m, 1H), 4.61 (dddt, *J* = 64.0, 8.7, 6.3, 2.3 Hz, 1H), 4.49 - 4.07 (m, 4H), 3.75 (s, 2H), 2.71 (s, 2H), 2.30 (s, 3H), 1.88 - 1.65 (m, 1H), 1.06 - 0.80 (m, 4H), 0.71 (dq, *J* = 10.4, 6.7 Hz, 1H), 0.61 - 0.20 (m, 8H).

## Examples 126-127 Preparation of compounds 126 and 127

A preparation of compounds 126 and 127 is illustrated as follows:

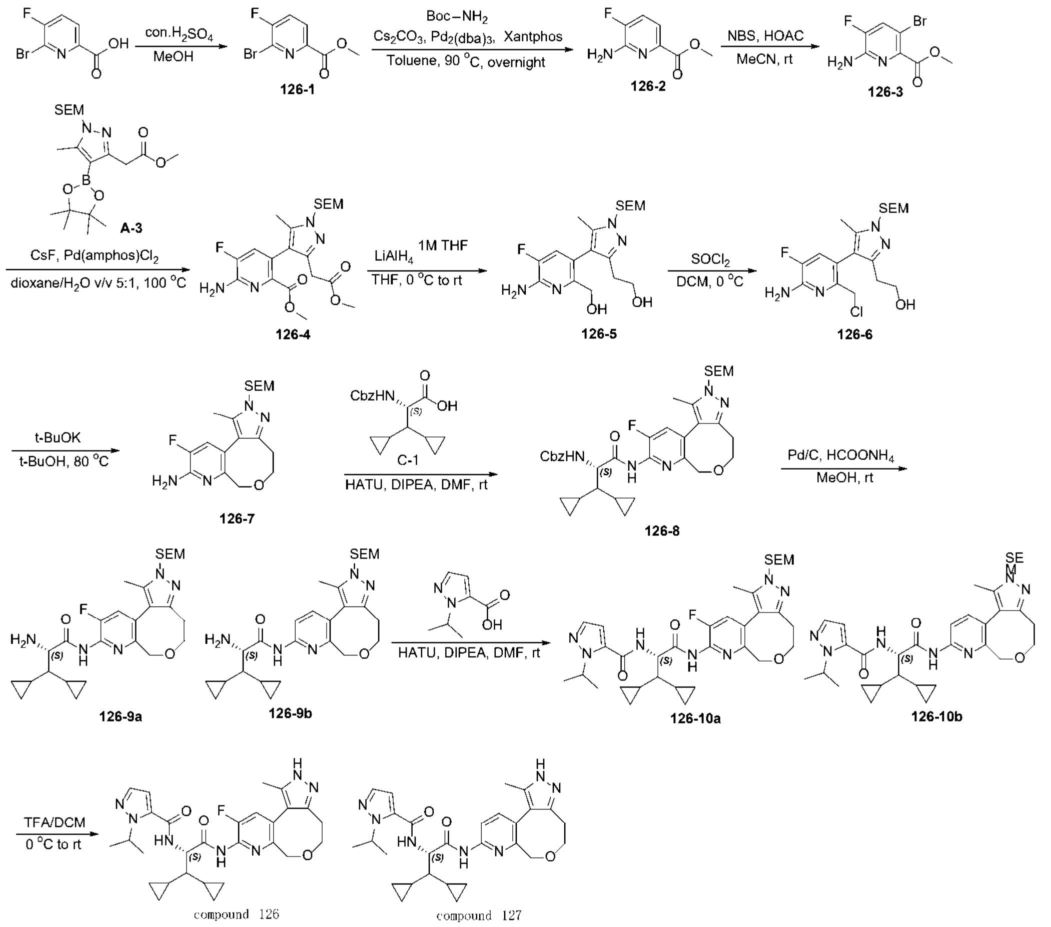


compound 126 compound 127

## (S1) Preparation of intermediate 126-1

To a single-neck flask were added 6-bromo-5-fluoro-2-pyridinecarboxylic acid (23.71 g, 107.77 mmol) and methanol (380 mL). The mixture was dropwise added with concentrated sulfuric acid (71 mL) at room temperature, and

stirred at room temperature overnight. Upon completion, the reaction mixture was decanted into ice water to quench and extracted with DCM. The DCM layer was collected, adjusted to pH 8 with a sodium bicarbonate solution, washed with a saturated NaCl solution, dried over anhydrous sodium sulfate, and filtered. The filtrate was concentrated under reduced pressure to dryness to obtain 24.58 g (105.03 mmol) of intermediate 126-1 (yield: 97.46%). MS (ESI) m/z = 234 $[M+1]^+$.

(S2) Preparation of intermediate 126-2

To a single-neck flask were added intermediate 126-1 (10 g, 42.73 mmol), Boc-$NH_2$ (6.01 g, 51.28 mmol), $Cs_2CO_3$ (27.84 g, 85.46 mmol), $Pd_2(dba)_3$ (1.96 g, 2.14 mmol), Xantphos (2.47 g, 4.27 mmol) and toluene (150 mL). The reaction mixture was evacuated and purged with nitrogen three times under stirring, heated to 90°C, and stirred overnight. Upon completion, the reaction mixture was filtered through Celite while hot. The filtrate was concentrated under reduced pressure to dryness. The residue was purified by column chromatography to obtain 2.13 g (12.52 mmol) of intermediate 126-2 (yield: 29.30%). MS (ESI) m/z = 171 $[M+1]^+$.

(S3) Preparation of intermediate 126-3

To a single-neck flask were added intermediate 126-2 (1.97 g, 11.58 mmol), glacial acetic acid (69.54 mg, 1.16 mmol), followed by addition of acetonitrile (40 mL) and NBS (2.27 g, 12.74 mmol) at room temperature. The reaction mixture was stirred for 2 h. Upon completion, the reaction was added with water to quench, and extracted with EA. The EA layer was collected, washed sequentially with sodium bicarbonate solution and a saturated NaCl solution, dried over anhydrous sodium sulfate, and filtered. The filtrate was concentrated under reduced pressure to dryness. The residue was purified by silica gel column chromatography to obtain 2.42 g (9.73 mmol) of intermediate 126-3 (yield: 84.06%). MS (ESI) m/z = 249 $[M+1]^+$. $^1$H NMR (400 MHz, DMSO-$d_6$) $\delta$ 7.82 (d, J = 10.2 Hz, 1H), 6.84 (s, 2H), 3.82 (s, 3H).

(S4) Preparation of intermediate 126-4

To a single-neck flask were added intermediate 126-3 (2.36 g, 9.48 mmol), intermediate A-3 (4.28 g, 10.42 mmol), CsF (2.88 g, 18.95 mmol), Pd(amphos)$Cl_2$ (336.46 mg, 473.83 $\mu$mol) and dioxane/water (50 mL/10 mL). The reaction mixture was evacuated and purged with nitrogen three times under stirring, heated to 100°C, and stirred overnight. Upon completion, the reaction mixture was filtered through Celite while hot. The filtrate was concentrated under reduced pressure to dryness. The residue was purified by column chromatography to obtain 3.06 g (6.76 mmol) of intermediate 126-4 (yield: 71.35%). MS (ESI) m/z = 453 $[M+1]^+$.

The preparation of compounds 126 and 127 was performed according to the synthesis method in Example 1, in which intermediate 1-1 was replaced with intermediate 126-4, with all other reagents and procedures remaining identical. However, during the hydrogenation of intermediate 126-8 with Pd/C and ammonium formate, about 1/3 of the raw material underwent de fluorination. The resulting mixture was then processed further to obtain compounds 126 and 127.

For compound 126, MS (ESI) m/z =536 $[M+1]^+$. $^1$H NMR (400 MHz, Methanol-$d_4$) $\delta$ 7.69 (d, *J* = 10.1 Hz, 1H), 7.52 (d, *J* = 2.0 Hz, 1H), 6.79 (d, *J* = 2.0 Hz, 1H), 5.41 (p, *J* = 6.7 Hz, 1H), 5.07 (s, 1H), 4.50 (d, *J* = 62.6 Hz, 2H), 3.75 (s, 2H), 2.79 (d, *J* = 51.4 Hz, 2H), 2.30 (s, 3H), 1.46 (dd, *J* = 6.6, 4.4 Hz, 6H), 0.90 (qd, *J* = 8.9, 8.3, 4.3 Hz, 3H), 0.64 - 0.41 (m, 5H), 0.36 - 0.27 (m, 3H).

For compound 127, MS (ESI) m/z =518 $[M+1]^+$. $^1$H NMR (400 MHz, Methanol-$d_4$) $\delta$ 8.19 (d, *J* = 8.4 Hz, 1H), 7.80 (d, *J* = 8.3 Hz, 1H), 7.53 (d, *J* = 2.0 Hz, 1H), 6.82 (d, *J* = 2.1 Hz, 1H), 5.40 (p, *J* = 6.6 Hz, 1H), 4.96 (d, *J* = 6.7 Hz, 1H), 4.40 (s, 2H), 3.75 (s, 2H), 2.79 (d, *J* = 58.1 Hz, 2H), 2.28 (s, 3H), 1.46 (dd, *J* = 6.6, 5.5 Hz, 6H), 1.02 - 0.76 (m, 3H), 0.61 - 0.42 (m, 3H), 0.42 - 0.21 (m, SH).

**Example 128 Preparation of compound 128**

A preparation of compound 128 is illustrated as follows:

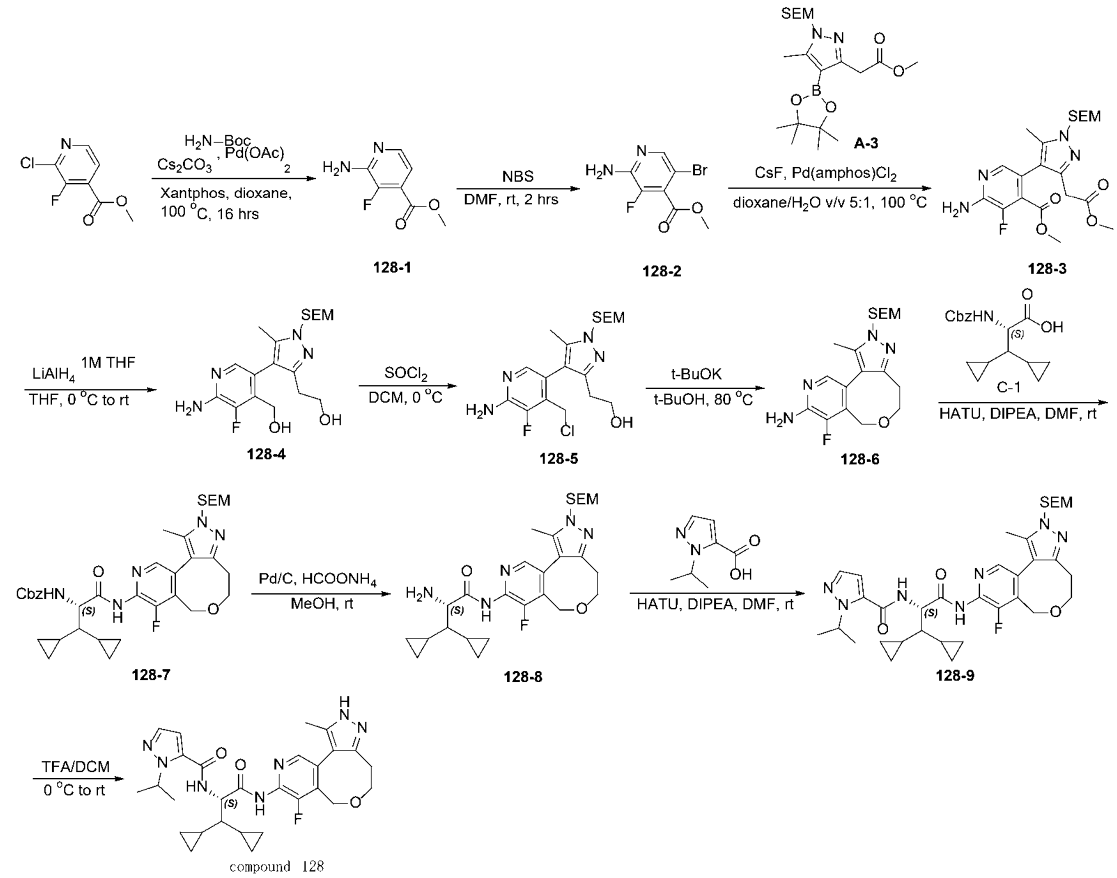


.

<u>(S1) Preparation of intermediate 128-1</u>

To a single-neck flask were added methyl 2-chloro-3-fluoroisonicotinate (9 g, 47.48 mmol), Boc-$NH_2$ (8.34 g, 71.21 mmol), $Cs_2CO_3$ (30.94 g, 94.95 mmol), $Pd(OAc)_2$ (532.94 mg, 2.37 mmol), Xantphos (2.75 g, 4.75 mmol) and dioxane (100 mL). The reaction mixture was evacuated and purged with nitrogen three times under stirring, heated to 100°C, and stirred for 16 h. Upon completion, the reaction mixture was filtered through Celite while hot. The filtrate was concentrated under reduced pressure to dryness. The residue was purified by column chromatography to obtain 1.6 g (9.40 mmol) of intermediate 128-1 (yield: 19.81%). MS (ESI) m/z = 171 $[M+1]^+$. 1H NMR (600 MHz, Chloroform-*d*) δ 7.92 (d, J = 5.2 Hz, 1H), 7.03 (t, J = 4.9 Hz, 1H), 3.95 (s, 3H).

<u>(S2) Preparation of intermediate 128-2</u>

To a single-neck flask were added intermediate 128-1 (1.6 g, 9.40 mmol) and DMF (40 mL), followed by addition of NBS (1.84 g, 10.34 mmol) at room temperature. The reaction mixture was stirred for 2 h. Upon completion, to the reaction mixture was add water to quench, followed by extraction with EA. The EA layer was collected, washed sequentially with sodium bicarbonate solution and a saturated NaCl solution, dried over anhydrous sodium sulfate, and filtered. The filtrate was concentrated under reduced pressure to dryness. The residue was purified by silica gel column chromatography to obtain 1.5 g (6.02 mmol) of intermediate 128-2 (yield: 64.05%). MS (ESI) m/z = 249 $[M+1]^+$. $^1$H NMR (600 MHz, Chloroform-*d*) δ 8.01 (s, 1H), 3.99 (s, 3H).

<u>(S3-S10) Preparation of compound 128</u>

The preparation of compound 128 was performed according to the synthesis method in Example 126, in which intermediate 126-3 was replaced with intermediate 128-2, with all other reagents and procedures remaining identical. MS (ESI) m/z =536 $[M+1]^+$. $^1$H NMR (600 MHz, Chloroform-d) δ 8.61 (s, 1H), 8.25 (s, 1H), 7.52 (d, *J* = 2.0 Hz, 1H), 7.26 (s, 1H), 7.18 (d, *J* = 8.3 Hz, 1H), 6.58 (d, *J* = 2.1 Hz, 1H), 5.49 (p, *J* = 6.7 Hz, 1H), 5.04 (s, 1H), 3.65 (d, *J* = 191.7 Hz, 5H), 2.81 (s, 1H),

2.37 (s, 3H), 1.50 (dd, *J* = 12.9, 6.6 Hz, 6H), 0.98 - 0.87 (m, 3H), 0.70 - 0.50 (m, 4H), 0.46 - 0.21 (m, 4H).

**Example 129 Preparation of compound 129**

A preparation of compound 129 is illustrated as follows:

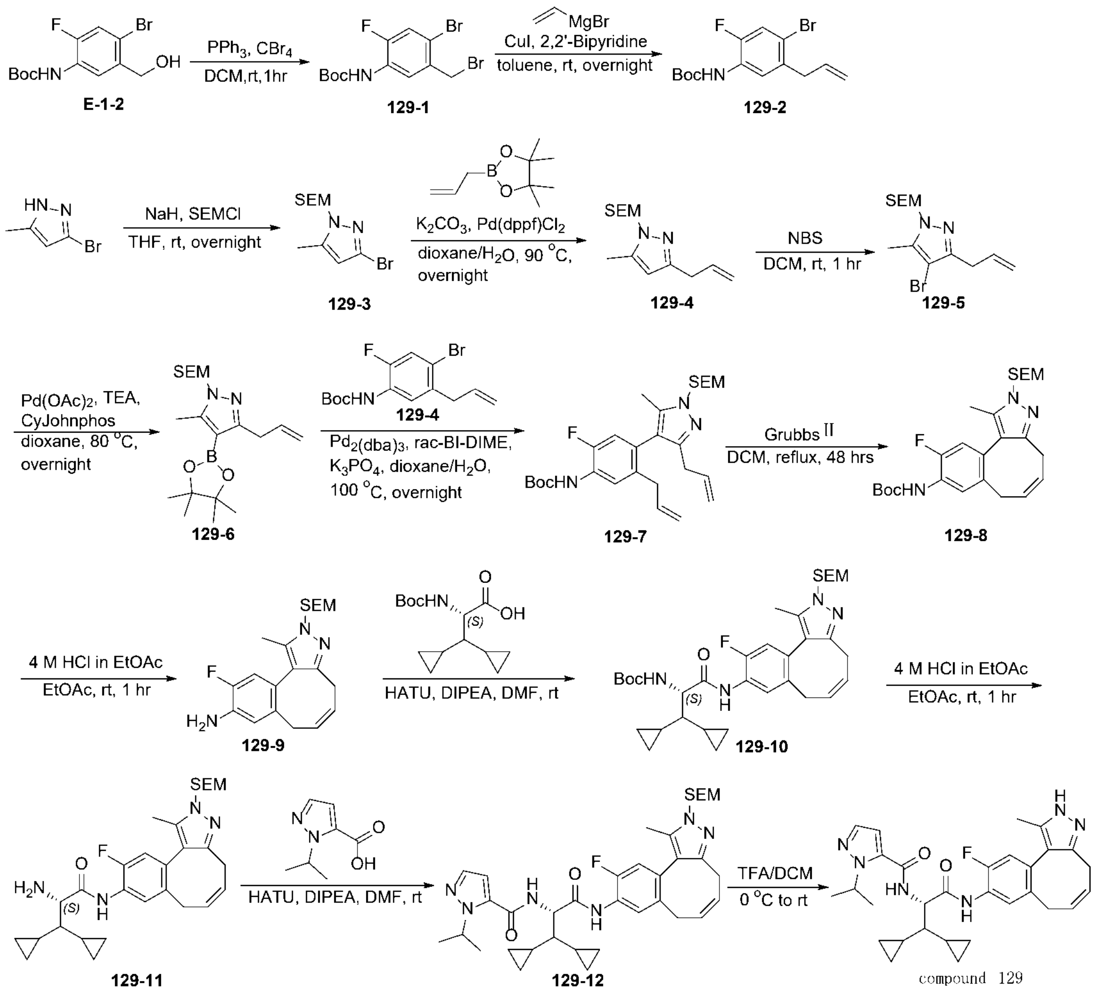


(S1) Preparation of intermediate 129-1

To a single-neck flask were added intermediate E-1-2 (5.7 g, 17.80 mmol) and DCM (50 mL), followed by addition of triphenylphosphine (7.00 g, 26.71 mmol) and carbon tetrabromide (7.68 g, 23.15 mmol) at room temperature. After addition, the reaction mixture was stirred at room temperature overnight. Upon completion, to the reaction was added water to quench, followed by extraction with DCM. The DCM layer was collected, washed with a saturated NaCl solution, dried over anhydrous sodium sulfate, and filtered. The filtrate was concentrated under reduced pressure to dryness. The residue was purified by silica gel column chromatography to obtain 4.26 g (11.12 mmol) of intermediate 129-1 (yield: 62.47%). MS (ESI) m/z = 382 $[M+1]^+$.

(S2) Preparation of intermediate 129-2

To a three-neck flask were added intermediate 129-1 (3.26 g, 8.51 mmol) and toluene (30 mL), followed by addition of copper(I) iodide (162.08 mg, 851.06 μmol) and 2,2'-bipyridine (132.92 mg, 851.06 μmol) at room temperature. The mixture was evacuated and purged with nitrogen three times. The mixture was cooled to -70°C, followed by dropwise addition of vinylmagnesium bromide (3.35 g, 25.53 mmol). After addition, the reaction mixture was gradually warmed to room temperature and stirred overnight. Upon completion, to the reaction mixture was added saturated ammonium chloride to quench, followed by extraction with EA. The EA layer was collected, washed with a saturated NaCl solution, dried over anhydrous sodium sulfate, and filtered. The filtrate was concentrated under reduced pressure to dryness. The

residue was purified by silica gel column chromatography to obtain 1.5 g (4.54 mmol) of intermediate 129-2 (yield: 53.38%). MS (ESI) m/z = 328 $[M-1]^+$.

(S3) Preparation of intermediate 129-3

To a three-neck flask were added 3-bromo-5-methyl-1H-pyrazole (20 g, 124.22 mmol) and THF (200 mL). The mixture was cooled to 0°C under an ice-water bath, followed by addition of sodium hydride (5.96 g, 248.45 mmol) in portions. After addition, the mixture was stirred under the ice-water bath for 1 h, followed by dropwise addition of 2-(trimethylsilyl)ethoxymethyl chloride (24.85 g, 149.07 mmol). After addition, the reaction mixture was warmed to room temperature and stirred overnight. Upon completion, to the reaction mixture was added saturated ammonium chloride solution to quench, followed by extraction with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 30 g (103.00 mmol) of intermediate 129-3 (yield: 82.92%). MS (ESI) m/z = 291 $[M+1]^+$.

(S4) Preparation of intermediate 129-4

To a single-neck flask were added intermediate 129-3 (6 g, 20.60 mmol), dioxane and water (40 mL/8 mL), followed by addition of allylboronic acid pinacol ester (9.52 g, 61.80 mmol), potassium carbonate (8.53 g, 61.80 mmol) and $Pd(dppf)Cl_2$ (753.66 mg, 1.03 mmol). The reaction mixture was evacuated and purged with nitrogen three times, heated to 100°C, and stirred overnight. Upon completion, the reaction mixture was cooled, diluted with water, and extracted with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 4.67 g (18.50 mmol) of intermediate 129-4 (yield: 89.81%). MS (ESI) m/z = 253 $[M+1]^+$.

(S5) Preparation of intermediate 129-5

To a single-neck flask were added intermediate 129-4 (4.5 g, 17.83 mmol) and DCM (40 mL), followed by addition of N-bromosuccinimide (3.49 g, 19.61 mmol). The reaction mixture was stirred at room temperature for 1 h. Upon completion, to the reaction mixture was added water to quench, followed by extraction with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 5.2 g (15.69 mmol) of intermediate 129-5 (yield: 88.04%). MS (ESI) m/z = 331 $[M+1]^+$.

(S6) Preparation of intermediate 129-6

To a three-neck flask were added intermediate 129-5 (4.4 g, 13.28 mmol) and dioxane (60 mL), followed by addition of triethylamine (2.02 g, 19.92 mmol, 2.78 mL), $Pd(OAc)_2$ (90.25 mg, 398.40 μmol) and CyJohnphos (279.26 mg, 796.81 μmol) at room temperature. The mixture was evacuated and purged with nitrogen three times, followed by addition of pinacolborane (4.59 g, 35.86 mmol) via a syringe. After addition, the reaction mixture was gradually warmed to 80°C and stirred overnight. Upon completion, to the reaction mixture was added water to quench, followed by extraction with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 5.2 g (15.69 mmol) of intermediate 129-6 (yield: 88.04%). MS (ESI) m/z = 379 $[M+1]^+$.

(S7) Preparation of intermediate 129-7

To a single-neck flask were added intermediate 129-6 (1.32 g, 3.49 mmol), intermediate 129-2 (1.27 g, 3.84 mmol) and dioxane and water (10 mL/2 mL), followed by addition of $Pd_2(dba)_3$ (79.86 mg, 87.21 μmol), rac-BI-DIME (57.56 mg, 174.42 μmol) and potassium phosphate (1.48 g, 6.98 mmol) at room temperature. The reaction mixture was evacuated and purged with nitrogen three times, gradually warmed to 100°C, and stirred overnight. Upon completion, to the reaction mixture was added water to quench, followed by extraction with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 1.38 g (2.74 mmol) of intermediate 129-7 (yield: 78.56%). MS (ESI) m/z = 502 $[M+1]^+$.

(S8) Preparation of intermediate 129-8

To a single-neck flask were added intermediate 129-7 (1.38 g, 2.74 mmol) and DCM (200 mL), followed by addition of Grubbs 2nd generation catalyst (116.34 mg, 137.03 μmol) at room temperature. The reaction mixture was evacuated and purged with nitrogen three times, gradually warmed to 45°C, and stirred for 48 h. Upon completion, to the reaction mixture was added water to quench, followed by separation and collection of DCM layer. The DCM layer was washed with a saturated NaCl solution, dried over anhydrous sodium sulfate, and filtered. The filtrate was concentrated under reduced pressure to dryness. The residue was purified by silica gel column chromatography to obtain 605 mg (1.28 mmol) of intermediate 129-8 (yield: 46.61%). MS (ESI) m/z = 474 $[M+1]^+$.

(S9) Preparation of intermediate 129-9

To a single-neck flask were added intermediate 129-8 (200 mg, 422.25 μmol) and EA (1 mL). A solution of 4 M HCl in EA (16.89 mg, 4.22 mmol) was added under an ice-water bath. The reaction mixture was gradually warmed to room temperature and stirred for 1 h. Upon completion, the reaction mixture was cooled under an ice-water bath and adjusted to pH 8 with triethylamine, then concentrated under reduced pressure to dryness. The residue was purified by MPLC ($CH_3CN/H_2O$, 0.05% $NH_4HCO_3$) to obtain 60 mg (160.63 μmol) of intermediate 129-9 (yield: 38.04%). MS (ESI) m/z = 374 $[M+1]^+$.

(S10-S13) Preparation of compound 129

The preparation of compound 129 was performed according to the synthesis method in Example 1, in which intermediate 1-4 in step (S5) was replaced with intermediate 129-9, and intermediate C-1 was replaced with boc-L-2-amino-3,3-dicyclopropylpropanoic acid. Moreover, the removal of Cbz group using Pd/C was replaced by the removal of Boc group using a solution of 4 M HCl in EA. This Boc-deprotection method was performed according to the synthesis method of intermediate 129-9. The remaining two steps was performed according to the synthesis method in Example 1. MS (ESI) m/z =531 $[M+1]^+$.

**Example 130 Preparation of compound 130**

A preparation of compound 130 is illustrated as follows:

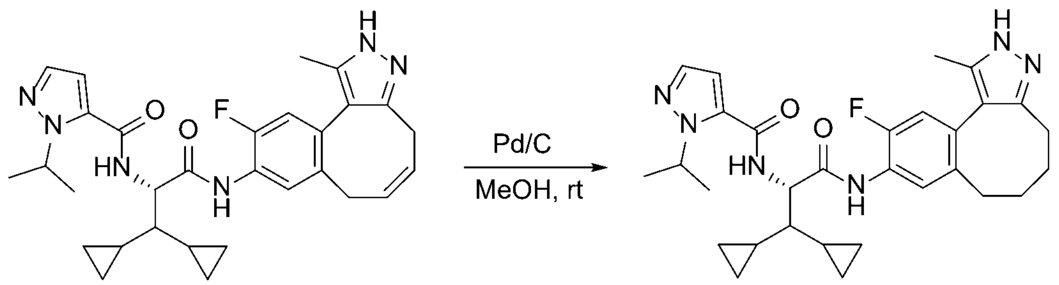


compound 129 compound 130

To a single-neck flask were added Example 129 (12 mg, 22.61 μmol) and methanol (2 mL), followed by addition of Pd/C (4.2 mg, w/w 30%). The reaction mixture was evacuated and purged with hydrogen three times, and stirred at room temperature for 2 h. Upon completion, the reaction mixture was filtered and concentrated under reduced pressure to dryness. The residue was purified by MPLC to obtain 240.00 μg (0.451 μmol) of compound 130 (yield: 1.99%). MS (ESI) m/z = 533 $[M+1]^+$.

**Example 131 Preparation of compound 131**

A preparation of compound 131 is illustrated as follows:

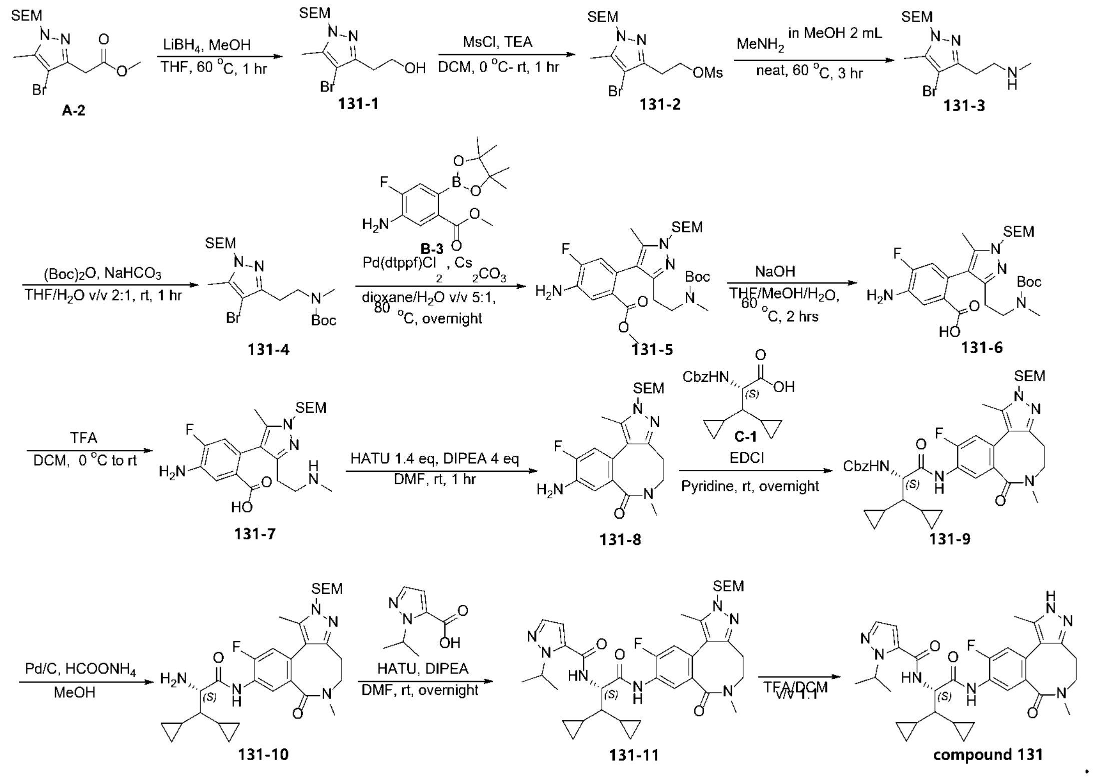


(S1) Preparation of intermediate 131-1

To a single-neck flask were added intermediate A-2 (10.9 g, 30.00 mmol), methanol (1.35 g, 42.00 mmol) and THF (110 mL), followed by addition of lithium borohydride (1.7 g, 78.05 mmol) at room temperature. The reaction mixture was heated to 60°C and stirred for 1 h. Upon completion, the reaction mixture was cooled under an ice-water bath, quenched with saturated ammonium chloride solution, and extracted with EA. The EA layer was collected, washed sequentially with water and a saturated NaCl solution, dried over anhydrous sodium sulfate, and filtered. The filtrate was concentrated under reduced pressure to dryness. The residue was purified by silica gel column chromatography to obtain 8.6 g (25.65 mmol) of intermediate 131-1 (yield: 85.49%). MS (ESI) m/z = 335 $[M+1]^+$.

(S2) Preparation of intermediate 131-2

To a three-neck flask were added intermediate 131-1 (6.6 g, 19.68 mmol) and DCM (60 mL). To the mixture was added triethylamine (3.98 g, 39.37 mmol, 5.49 mL) at room temperature, followed by dropwise addition of methanesulfonyl chloride (2.71 g, 23.62 mmol) under an ice-water bath. After addition, the reaction mixture was stirred at room temperature for 1 h. Upon completion, the reaction mixture was added with water to quench, and extracted with DCM. The DCM layer was collected, washed sequentially with water and a saturated NaCl solution, dried over anhydrous sodium sulfate, and filtered. The filtrate was concentrated under reduced pressure to dryness to obtain 7.7 g (18.63 mmol) of intermediate 131-2 (yield: 94.63%). MS (ESI) m/z = 413 $[M+1]^+$.

(S3) Preparation of intermediate 131-3

To a sealed tube were added intermediate 131-2 (7.7 g, 18.63 mmol) and 33% methylamine in methanol solution (60 mL). The mixture was heated to 60°C and stirred for 2.5 h. Upon completion, the reaction mixture was cooled under an ice-water bath and concentrated under reduced pressure to dryness to obtain 6.29 g (18.06 mmol) of intermediate 131-3 (yield: 96.94%), which was used directly in the next step without purification. MS (ESI) m/z = 348 $[M+1]^+$.

(S4) Preparation of intermediate 131-4

To a single-neck flask were added intermediate 131-3 (5.25 g, 15.07 mmol), THF and water (50 mL/25 mL), followed by addition of sodium bicarbonate (3.95 g, 18.09 mmol) and $(Boc)_2O$ (3.95 g, 18.09 mmol) at room temperature. After addition, the reaction mixture was stirred at room temperature for 2 h. Upon completion, to the reaction mixture was

added water for dilution, followed by extraction with EA. The EA layer was collected, washed sequentially with water and a saturated NaCl solution, dried over anhydrous sodium sulfate, and filtered. The filtrate was concentrated under reduced pressure to dryness. The residue was purified by silica gel column chromatography to obtain 5.57 g (12.42 mmol) of intermediate 131-4 (yield: 82.41%). MS (ESI) m/z = 448 $[M+1]^+$.

(S5) Preparation of intermediate 131-5

To a mixed solvent of 1,4-dioxane (80 mL) and water (16 mL) containing intermediate B-3 (3.99 g, 13.52 mmol), intermediate 131-4 (6.67 g, 14.87 mmol), and $Cs_2CO_3$ (8.81 g, 27.03 mmol) was added Pd(dppf)$Cl_2$ (881.18 mg, 1.35 mmol). The reaction mixture was degassed by nitrogen bubbling, stirred at 80°C overnight. Upon completion, the reaction mixture was cooled, diluted with water, and extracted with EA. The EA layer was collected, washed with a NaCl solution, dried over anhydrous sodium sulfate, and filtered. The filtrate was concentrated under reduced pressure to dryness. The residue was purified by silica gel column chromatography to obtain 4.1 g (7.64 mmol) of intermediate 131-5 (yield: 56.50%). MS (ESI) m/z = 537 $[M+1]^+$.

(S6) Preparation of intermediate 131-6

To a single-neck flask were added intermediate 131-5 (196 mg, 365.19 μmol), THF, methanol and water (2 mL/2 mL/2 mL), followed by addition of sodium hydroxide (70.04 mg, 1.83 mmol) at room temperature. After addition, the reaction mixture was heated to 60°C and stirred for 1 h. Upon completion, the reaction mixture was cooled under an ice-water bath, adjusted to pH 7 with 1 M HCl, and then concentrated under reduced pressure to dryness to obtain 190 mg (363.51 μmol) of crude intermediate 131-6. MS (ESI) m/z = 523 $[M+1]^+$.

(S7) Preparation of intermediate 131-7

To a single-neck flask were added intermediate 131-6 (190 mg, 363.51 μmol) and DCM (3 mL), followed by addition of trifluoroacetic acid (414.47 mg, 3.64 mmol) under an ice-water bath. After addition, the reaction mixture was stirred at room temperature for 0.5 h. Upon completion, the reaction mixture was cooled under an ice-water bath, adjusted to pH 7 with saturated sodium bicarbonate solution, and then concentrated under reduced pressure to dryness to obtain 153 mg (362.07 μmol) of crude intermediate 131-7. MS (ESI) m/z = 423 $[M+1]^+$.

(S8) Preparation of intermediate 131-8

To a single-neck flask were added intermediate 131-7 (153 mg, 362.07 μmol) and DMF (4 mL), followed by sequential addition of DIPEA (187.18 mg, 1.45 mmol, 252.26 μL) and HATU (178.97 mg, 470.69 μmol). After addition, the reaction mixture was stirred at room temperature for 1 h. To the reaction mixture was added water to quench, followed by extraction with EA. The EA layer was collected, washed with a saturated NaCl solution, dried over anhydrous sodium sulfate, and filtered. The filtrate was concentrated under reduced pressure to dryness. The residue was purified by silica gel column chromatography to obtain 121 mg (299.10 μmol) of intermediate 131-8 (yield: 82.61%). MS (ESI) m/z = 405 $[M+1]^+$.

(S9) Preparation of intermediate 131-9

To a single-neck flask were added intermediate 131-8 (121 mg, 299.10 μmol) and pyridine (1.5 mL), followed by addition of intermediate C-1 (108.88 mg, 358.92 μmol) and EDCI (172.01 mg, 897.29 μmol). The reaction mixture was stirred at room temperature overnight. Upon completion, the reaction mixture was concentrated under reduced pressure to dryness. The residue was purified by reverse-phase MPLC ($CH_3CN/H_2O$, 0.05% TFA) to obtain 128 mg (185.54 μmol) of intermediate 131-9 (yield: 62.03%). MS (ESI) m/z = 690 $[M+1]^+$.

(S10-S12) Preparation of compound 131

The preparation of compound 131 was performed according to steps (S6-S8) of Example 1, in which intermediate 1-5 was replaced with intermediate 131-9, with other reagents and procedures remaining identical. MS (ESI) m/z = 562 $[M+1]^+$. $^1$H NMR (400 MHz, Methanol-$d_4$) δ 8.09 (dd, *J* = 14.7, 7.8 Hz, 1H), 7.51 (t, *J* = 2.6 Hz, 1H), 7.18 - 7.10 (m, 1H), 6.78 (dd, *J* = 3.8, 2.1 Hz, 1H), 5.40 (pd, *J* = 6.7, 2.3 Hz, 1H), 5.01 (dd, *J* = 9.5, 7.1 Hz, 1H), 4.01 (tt, *J* = 14.3, 4.7 Hz, 1H), 3.40 (dt, *J* = 14.4, 5.5 Hz, 1H), 3.35-3.24 (m,1H) ,3.01 (s, 3H), 2.92 - 2.78 (m, 1H), 2.17 (s, 3H), 1.45 (td, *J* = 6.8, 3.0 Hz, 6H), 0.98 - 0.75 (m, 3H), 0.63 - 0.16 (m, 8H).

## Examples 132-181 Preparation of compounds 132-181

The preparation of compounds 132 to 181 was performed according to the synthesis method in Example 1, in which intermediate 1-4 in step (S5) was correspondingly replaced with a macrocyclic amine from the following table, and 1-isopropylpyrazole-5-carboxylic acid in step (S7) was correspondingly replaced with a pyrazolecarboxylic acid from the following table or unchanged, with other reagents and procedures remaining identical.

| Macrocyclic amine + pyrazolecarboxylic acid | Structure of compound | $^1$HNMR and/or MS (ESI) |
|---|---|---|
| 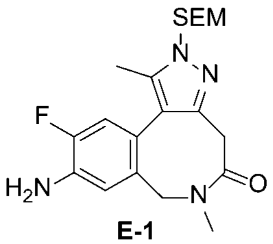<br>E-1<br>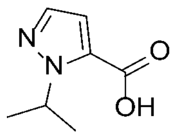 | 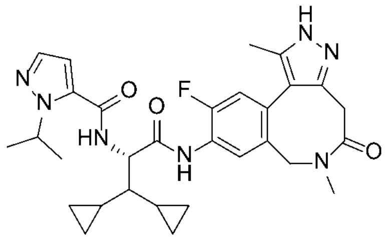<br>compound 132 | MS (ESI) m/z =562 [M+1]$^+$<br>$^1$H NMR (400 MHz, DMSO-$d_6$) $\delta$ 12.68 (d, *J* = 87.3 Hz, 1H), 10.03 (s, 1H), 8.41 (d, *J* = 8.8 Hz, 1H), 7.89 (d, *J* = 8.2 Hz, 1H), 7.51 (d, *J* = 2.0 Hz, 1H), 7.37 - 7.22 (m, 1H), 6.93 (d, *J* = 2.0 Hz, 1H), 5.41 (hept, *J* = *6.6* Hz, 1H), 4.97 (dd, *J* = 8.8, 7.5 Hz, 1H), 4.44 (q, *J* = 14.9 Hz, 2H), 4.11 - 3.78 (m, 2H), 2.72 (s, 3H), 2.37 - 2.20 (m, 3H), 1.37 (dd, *J* = 11.0, 6.6 Hz, 6H), 1.01 - 0.72 (m, 3H), 0.49 (dp, *J* = 8.1, 3.8, 3.3 Hz, 1H), 0.44 - 0.15 (m, 7H). |
| 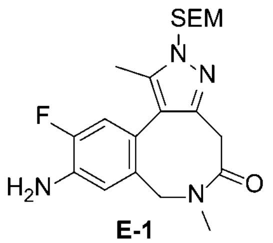<br>E-1<br>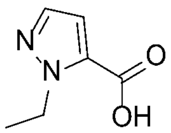 | 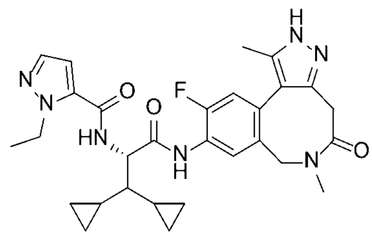<br>compound 133 | MS (ESI) m/z =548 [M+1]$^+$<br>$^1$H NMR (400 MHz, DMSO-$d_6$) $\delta$ 12.68 (d, *J* = 87.9 Hz, 1H), 10.04 (s, 1H), 8.42 (d, *J* = 8.9 Hz, 1H), 7.89 (d, *J* = 8.2 Hz, 1H), 7.49 (d, *J* = 2.0 Hz, 1H), 7.30 (dd, *J* = 23.0, 12.5 Hz, 1H), 7.01 (d, *J* = 2.0 Hz, 1H), 4.99 (dd, *J* = 8.8, 7.5 Hz, 1H), 4.56 - 4.36 (m, 4H), 4.12 - 3.80 (m, 2H), 2.73 (s, 3H), 2.36 - 2.21 (m, 3H), 1.30 (t, *J* = 7.1 Hz, 3H), 1.01 - 0.71 (m, 3H), 0.49 (tq, *J* = 8.0, 3.5 Hz, 1H), 0.44 - 0.12 (m, 7H). |
| 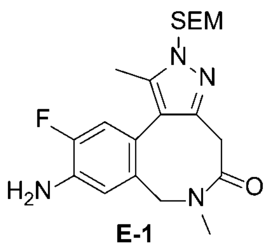<br>E-1<br>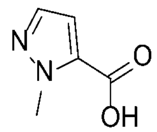 | 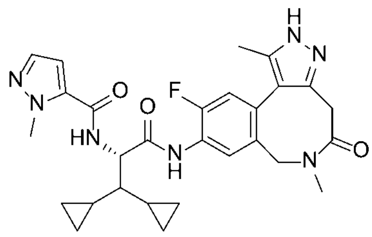<br>compound 134 | MS (ESI) m/z =534 [M+1]$^+$<br>$^1$H NMR (400 MHz, DMSO-$d_6$) $\delta$ 12.68 (d, *J* = 88.1 Hz, 1H), 10.06 (s, 1H), 8.41 (d, *J* = 8.9 Hz, 1H), 7.89 (d, *J* = 8.2 Hz, 1H), 7.47 (d, *J* = 2.0 Hz, 1H), 7.30 (dd, *J* = 24.3, 12.4 Hz, 1H), 7.06 (d, *J* = 2.1 Hz, 1H), 5.00 (dd, *J* = *8.9,* 7.6 Hz, 1H), 4.43 (d, *J* = 7.8 Hz, 2H), 4.05 (s, 3H), 4.12 - 3.83 (m, 2H), 2.72 (d, *J* = 9.3 Hz, 3H), 2.29 (d, *J* = 19.8 Hz, 3H), 1.00 - 0.72 (m, 3H), 0.49 (tq, *J* = 8.1, 3.4 Hz, 1H), 0.44 - 0.14 (m, 7H). |

(continued)

| Macrocyclic amine + pyrazolecarboxylic acid | Structure of compound | $^1$HNMR and/or MS (ESI) |
|---|---|---|
| 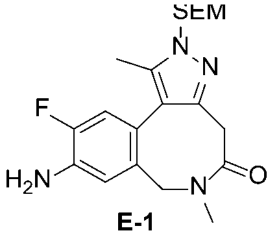 E-1 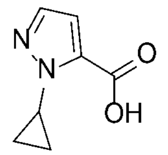 | 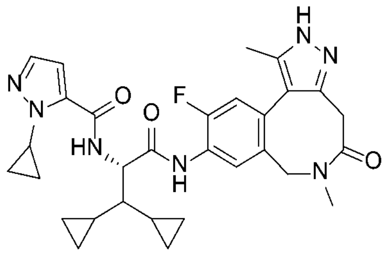 **compound 135** | MS (ESI) m/z =560 [M+1]$^+$ $^1$H NMR (400 MHz, DMSO-$d_6$) δ 12.68 (d, *J* = 88.2 Hz, 1H), 10.05 (s, 1H), 8.43 (d, *J* = 8.9 Hz, 1H), 7.90 (d, *J* = 8.2 Hz, 1H), 7.43 (d, *J* = 2.0 Hz, 1H), 7.30 (dd, *J* = 24.4, 12.4 Hz, 1H), 6.96 (d, *J* = 2.0 Hz, 1H), 5.01 (dd, *J* = 8.9, 7.4 Hz, 1H), 4.55 - 4.36 (m, 3H), 4.11 - 3.83 (m, 2H), 2.72 (d, *J* = 9.5 Hz, 3H), 2.29 (d, *J* = 20.0 Hz, 3H), 1.17 - 0.71 (m, 7H), 0.49 (dt, *J*= 9.1, 4.9 Hz, 1H), 0.45 - 0.15 (m, 7H). |
| 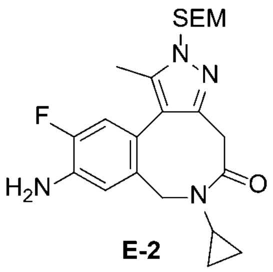 E-2 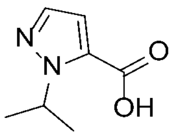 | 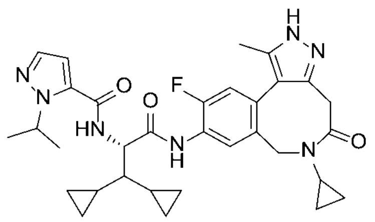 **compound 136** | MS (ESI) m/z =588 [M+1]$^+$ $^1$H NMR (400 MHz, DMSO-$d_6$) δ 12.68 (d, *J* = 87.3 Hz, 1H), 10.03 (s, 1H), 8.41 (d, *J* = 8.8 Hz, 1H), 7.89 (d, *J* = 8.2 Hz, 1H), 7.51 (d, *J* = 2.0 Hz, 1H), 7.37 - 7.22 (m, 1H), 6.93 (d, *J* = 2.0 Hz, 1H), 5.41 (hept, *J* = 6.6 Hz, 1H), 4.97 (dd, *J* = 8.8, 7.5 Hz, 1H), 4.44 (q, *J* = 14.9 Hz, 2H), 4.11 - 3.78 (m, 2H), 2.72 (s, 3H), 2.37 - 2.20 (m, 3H), 1.37 (dd, *J* = 11.0, 6.6 Hz, 6H), 1.01 - 0.72 (m, 3H), 0.49 (dp, *J* = 8.1, 3.8, 3.3 Hz, 1H), 0.44 - 0.15 (m, 7H). |
| 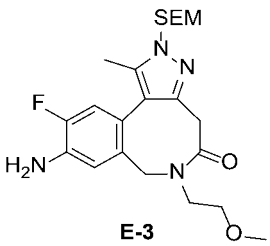 E-3 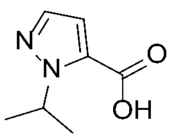 | 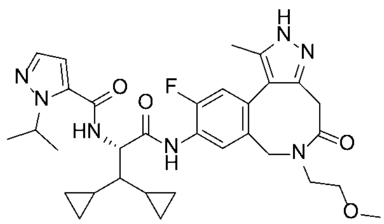 **compound 137** | MS (ESI) m/z =606 [M+1]$^+$ $^1$H NMR (400 MHz, Methanol-$d_4$) δ 8.24 - 8.07 (m, 1H), 7.52 (d, *J* = 2.0 Hz, 1H), 7.26 (t, *J* = 16.2 Hz, 1H), 6.80 (d, *J* = 2.0 Hz, 1H), 5.41 (hept, *J* = 6.7 Hz, 1H), 5.00 (d, *J* = 7.2 Hz, 1H), 4.56-4.38 (m, 2H), 4.05-3.73(m, 2H), 3.69 - 3.52 (m, 4H), 3.35 (s, 3H), 2.35 (s, 3H), 1.46 (dd, *J* = 6.7, 4.7 Hz, 6H), 1.00 - 0.76 (m, 3H), 0.63 - 0.22 (m, 8H). |
| 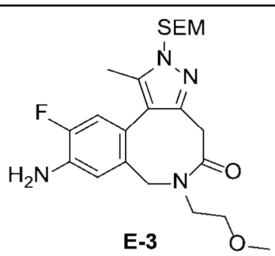 E-3 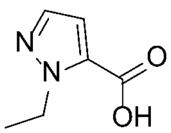 | 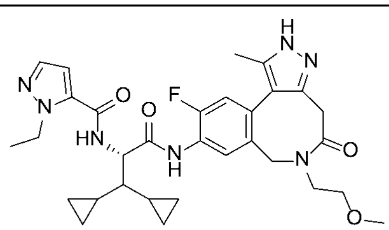 **compound 138** | MS (ESI) m/z =592 [M+1]$^+$ $^1$H NMR (600 MHz, Methanol-$d_4$) δ 8.16 (s, 1H), 7.51 (d, *J* = 2.1 Hz, 1H), 7.27 (t, *J* = 24.5 Hz, 1H), 6.87 (d, *J* = 2.1 Hz, 1H), 5.00 (d, *J* = 7.3 Hz, 1H), 4.60 - 4.51 (m, 3H), 4.45 (s, 1H), 3.99 (s, 1H), 3.79 (s, 1H), 3.69 - 3.53 (m, 4H), 3.35 (s, 3H), 2.35 (s, 3H), 1.40 (t, *J* = 7.2 Hz, 3H), 0.97 - 0.79 (m, 3H), 0.60 - 0.55 (m, 1H), 0.54 - 0.41 (m, 3H), 0.41 - 0.36 (m, 1H), 0.34 - 0.24 (m, 3H). |

(continued)

| Macrocyclic amine + pyrazolecarboxylic acid | Structure of compound | $^1$HNMR and/or MS (ESI) |
|---|---|---|
| 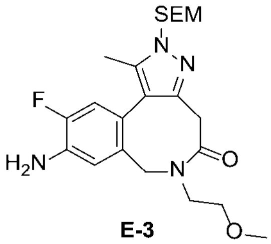 E-3 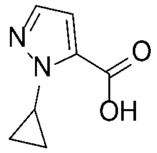 | 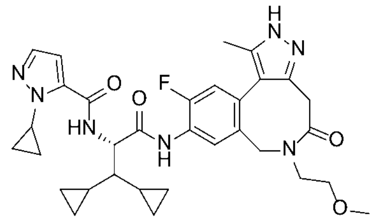 **compound 139** | MS (ESI) m/z =604 $[M+1]^+$ $^1$H NMR (600 MHz, Methanol-$d_4$) δ 8.17 (s, 1H), 7.43 (d, *J* = 2.1 Hz, 1H), 7.25 (d, *J* = 11.3 Hz, 1H), 6.79 (d, *J* = 2.0 Hz, 1H), 5.03 (d, *J* = 7.3 Hz, 1H), 4.49 (d, *J* = 41.4 Hz, 2H), 4.17 (tt, *J* = 7.5, 3.9 Hz, 1H), 4.00 (s, 1H), 3.79 (s, 1H), 3.69 - 3.54 (m, 4H), 3.36 (s, 3H), 2.35 (s, 3H), 1.19 - 1.08 (m, 2H), 1.07 - 0.98 (m, 2H), 0.96 - 0.82 (m, 3H), 0.58 (tq, *J* = 8.2, 3.9 Hz, 1H), 0.55 - 0.42 (m, 3H), 0.41 - 0.35 (m, 1H), 0.35 - 0.24 (m, 3H). |
| 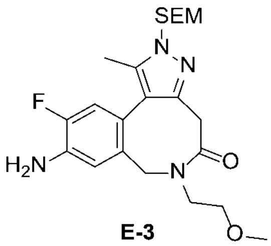 E-3 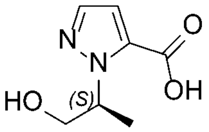 D-44 | 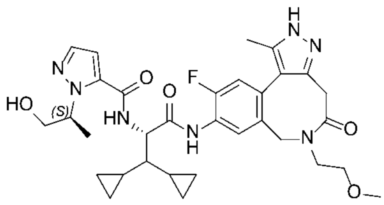 **compound 140** | MS (ESI) m/z =622 $[M+1]^+$ $^1$H NMR (600 MHz, Methanol-$d_4$) δ 8.22 - 8.07 (m, 1H), 7.56 (d, *J* = 2.0 Hz, 1H), 7.35 - 7.19 (m, 1H), 6.81 (d, *J* = 2.1 Hz, 1H), 5.38 - 5.27 (m, 1H), 4.99 (d, *J* = 7.1 Hz, 1H), 4.49 (d, *J* = 46.0 Hz, 2H), 3.99 (s, 1H), 3.86 (dd,*J* = 11.1, 8.2 Hz, 1H), 3.80 - 3.74 (m, 2H), 3.69 - 3.54 (m, 4H), 3.36 (s, 3H), 2.34 (d, *J* = 18.0 Hz, 3H), 1.45 (d, *J* = 6.8 Hz, 3H), 0.99 - 0.87 (m, 2H), 0.85 - 0.79 (m, 1H), 0.62 - 0.55 (m, 1H), 0.55 - 0.41 (m, 3H), 0.39 - 0.34 (m, 1H), 0.33 - 0.25 (m, 3H). |
| 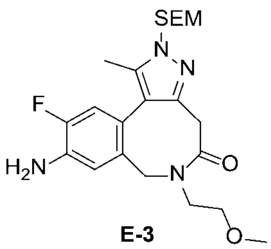 E-3 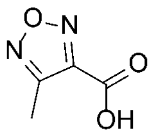 | 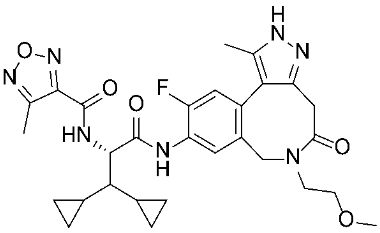 **compound 141** | MS (ESI) m/z =580 $[M+1]^+$ $^1$H NMR (600 MHz, Methanol-$d_4$) δ 8.13 (d, *J* = 23.3 Hz, 1H), 7.37 - 7.19 (m, 1H), 5.06 (d, *J* = 6.8 Hz, 1H), 4.49 (d, *J* = 44.3 Hz, 2H), 4.00 (s, 1H), 3.79 (s, 1H), 3.67 - 3.54 (m, 4H), 3.36 (s, 3H), 2.56 (s, 3H), 2.34 (d, *J* = 16.7 Hz, 3H), 0.96 - 0.80 (m, 3H), 0.62 - 0.42 (m, 4H), 0.42 - 0.35 (m, 1H), 0.35 - 0.25 (m, 3H). |
| 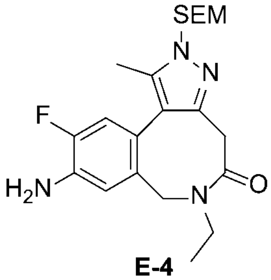 E-4 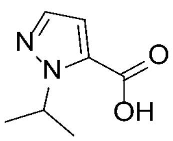 | 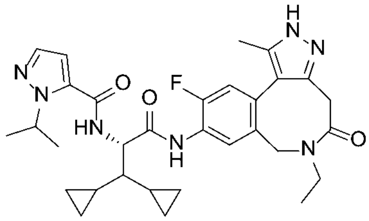 **compound 142** | MS (ESI) m/z =576 $[M+1]^+$ $^1$H NMR (400 MHz, Methanol-$d_4$) δ 8.12 (d, *J* = 7.8 Hz, 1H), 7.53 (d, *J* = 2.0 Hz, 1H), 7.27 (s, 1H), 6.80 (d, *J* = 2.0 Hz, 1H), 5.41 (hept, *J* = 6.7 Hz, 1H), 5.00 (d, *J* = 7.1 Hz, 1H), 4.40 (s, 2H), 3.90 (d, *J* = 72.3 Hz, 2H), 3.60 - 3.40 (m, 2H), 2.35 (s, 3H), 1.46 (dd, *J* = 6.6, 4.7 Hz, 6H), 1.15 (s, 3H), 0.98 - 0.78 (m, 3H), 0.58 (tq, *J* = 8.1, 3.8 Hz, 1H), 0.53 - 0.40 (m, 3H), 0.37 (p, *J* = 4.5 Hz, 1H), 0.34 - 0.23 (m, 3H). |

(continued)

| Macrocyclic amine + pyrazolecarboxylic acid | Structure of compound | $^1$HNMR and/or MS (ESI) |
|---|---|---|
| 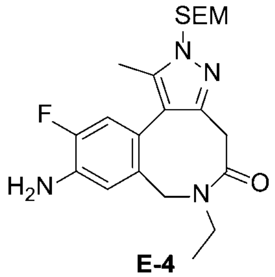 E-4 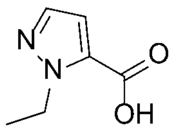 | 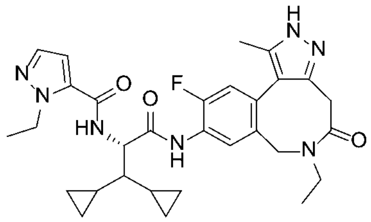 compound 143 | MS (ESI) m/z =562 [M+1]$^+$ <br> $^1$H NMR (400 MHz, Methanol-$d_4$) δ 8.12 (d, *J* = 7.8 Hz, 1H), 7.51 (d, *J* = 2.1 Hz, 1H), 7.27 (s, 1H), 6.88 (d, *J* = 2.1 Hz, 1H), 5.00 (d, *J* = 7.1 Hz, 1H), 4.55 (q, *J* = 7.1 Hz, 2H), 4.40 (s, 2H), 3.90 (d, *J* = 73.1 Hz, 2H), 3.49 (s, 2H), 2.35 (s, 3H), 1.40 (t, *J* = 7.2 Hz, 3H), 1.15 (s, 3H), 0.87 (dddd, *J* = 31.4, 18.4, 9.1, 6.1 Hz, 3H), 0.57 (qd, *J* = 7.1, 6.2, 3.5 Hz, 1H), 0.46 (dtt, *J* = 20.7, 8.2, 4.6 Hz, 3H), 0.37 (dt, *J* = 9.3, 4.5 Hz, 1H), 0.34 - 0.23 (m, 3H). |
| 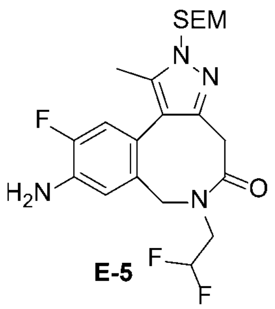 E-5 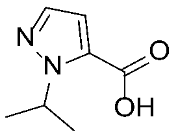 | 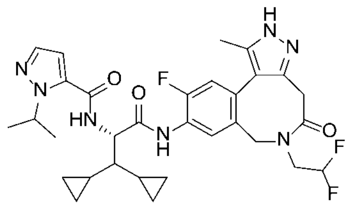 compound 144 | MS (ESI) m/z =612 [M+1]$^+$ <br> $^1$H NMR (400 MHz, DMSO-$d_6$) δ 12.72 (d, *J* = 67.9 Hz, 1H), 10.06 (s, 1H), 8.44 (d, *J* = 8.9 Hz, 1H), 8.01 (d, *J*= 9.0 Hz, 1H), 7.50 (d, *J* = 2.0 Hz, 1H), 7.33 (dd, *J* = 24.8, 12.3 Hz, 1H), 6.93 (d, *J*= *2.0* Hz, 1H), 6.18 (t, *J* = 56.1 Hz, 1H), 5.42 (p, *J* = 6.6 Hz, 1H), 4.98 (t, *J* = 8.2 Hz, 1H), 4.56 - 4.37 (m, 2H), 4.09 - 3.77 (m, 2H), 3.78 - 3.53 (m, 2H), 2.34 - 2.22 (m, 3H), 1.37 (dd, *J* = 12.5, 6.6 Hz, 6H), 1.00 - 0.73 (m, 3H), 0.53 - 0.15 (m, 8H). |
| 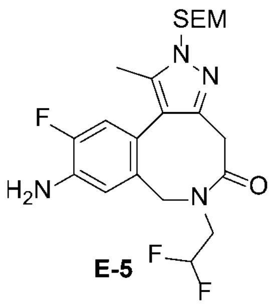 E-5 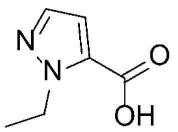 | 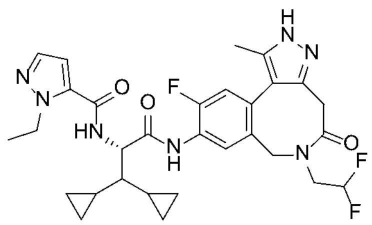 compound 145 | MS (ESI) m/z =598 [M+1]$^+$ <br> $^1$H NMR (400 MHz, DMSO-$d_6$) δ 12.72 (d, *J* = 65.8 Hz, 1H), 10.08 (s, 1H), 8.45 (d, *J* = 8.9 Hz, 1H), 8.00 (d, *J*= 8.0 Hz, 1H), 7.49 (d, *J*= 2.0 Hz, 1H), 7.31 (d, *J* = 11.5 Hz, 1H), 7.01 (d, *J* = 2.1 Hz, 1H), 6.18 (dd, *J* = 58.1, 53.8 Hz, 1H), 4.99 (t, *J* = 8.2 Hz, 1H), 4.56 - 4.40 (m, 4H), 4.14 - 3.54 (m, 4H), 2.29 (s, 3H), 1.30 (t, *J* = 7.1 Hz, 3H), 1.01 - 0.72 (m, 3H), 0.55 - 0.13 (m, 8H). |

(continued)

| Macrocyclic amine + pyrazolecarboxylic acid | Structure of compound | $^1$HNMR and/or MS (ESI) |
|---|---|---|
| 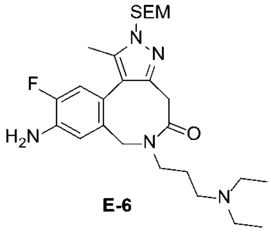 E-6 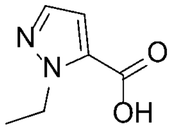 | 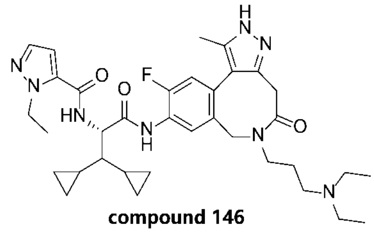 compound 146 | MS (ESI) m/z =647 [M+1]$^+$ $^1$H NMR (400 MHz, Methanol-$d_4$) δ 7.94 (t, *J* = 8.2 Hz, 1H), 7.51 (d, *J* = 2.1 Hz, 1H), 6.95 (dd, *J* = 11.4, 1.6 Hz, 1H), 6.87 (t, *J* = 2.0 Hz, 1H), 5.00 (d, *J* = 7.2 Hz, 1H), 4.59 - 4.52 (m, 2H), 4.40 - 3.40 (m, 4H), 3.27 - 2.96 (m, 4H), 2.42 (dd, *J* = 110.9, 66.9 Hz, 4H), 2.00 (s, 3H), 1.59 - 1.20 (m, 5H), 1.14 - 0.68 (m, 9H), 0.63 - 0.21 (m, 8H). |
| 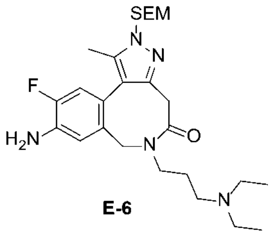 E-6 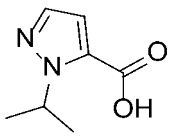 | 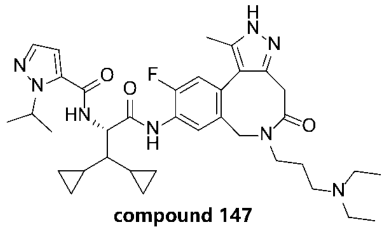 compound 147 | MS (ESI) m/z =661 [M+1]$^+$ $^1$H NMR (400 MHz, Methanol-$d_4$) δ 7.95 (t, *J* = 8.8 Hz, 1H), 7.52 (d, *J* = 2.1 Hz, 1H), 6.95 (dd, *J* = 11.4, 1.6 Hz, 1H), 6.80 (t, *J* = 2.1 Hz, 1H), 5.41 (ddq, *J* = 9.5, 6.6, 3.2 Hz, 1H), 5.00 (d, *J* = 7.2 Hz, 1H), 4.40 - 3.40 (m, 4H), 3.28 - 2.95 (m4, 4H), 2.80 - 2.13 (m, 4H), 2.00 (s, 3H), 1.61 - 1.19 (m, 8H), 1.16 - 0.66 (m, 9H), 0.63 - 0.20 (m, 8H). |
| 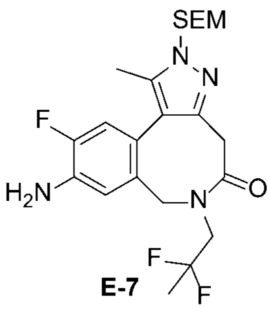 E-7 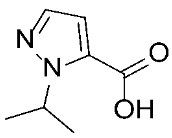 | 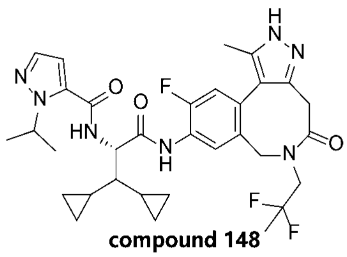 compound 148 | MS (ESI) m/z =626 [M+1]$^+$ $^1$H NMR (400 MHz, DMSO-$d_6$) δ 12.73 (d, *J* = 66.4 Hz, 1H), 10.09 (s, 1H), 8.47 (d, *J* = 8.8 Hz, 1H), 8.06 - 7.91 (m, 1H), 7.50 (d, *J* = 1.9 Hz, 1H), 7.34 (dd, *J* = 25.7, 12.1 Hz, 1H), 6.93 (d, *J* = 2.0 Hz, 1H), 5.40 (h, *J* = 6.7 Hz, 1H), 4.98 (t, *J* = 8.2 Hz, 1H), 4.45 (dq, *J* = 26.9, 15.4 Hz, 2H), 4.09 (q, *J* = 16.8 Hz, 1H), 3.93 - 3.59 (m, 3H), 2.29 (d, *J* = 18.4 Hz, 3H), 1.59 (t, *J* = 18.9 Hz, 3H), 1.37 (dd, *J* = 15.0, 6.6 Hz, 6H), 1.01 - 0.82 (m, 2H), 0.77 (q, *J* = 8.9 Hz, 1H), 0.48 (dt, *J* = 8.9, 4.8 Hz, 1H), 0.34 (dtd, *J* = 20.4, 8.1, 4.3 Hz, 4H), 0.22 (dq, *J* = 10.2, 5.2 Hz, 3H). |
| 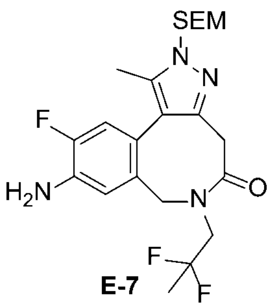 E-7 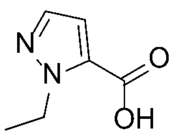 | 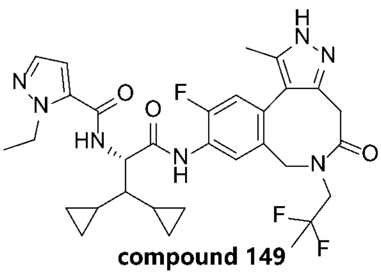 compound 149 | MS (ESI) m/z =612 [M+1]$^+$ $^1$H NMR (400 MHz, DMSO-$d_6$) δ 12.75 (s, 1H), 10.10 (s, 1H), 8.47 (d, *J* = 8.8 Hz, 1H), 7.98 (d, *J* = 8.1 Hz, 1H), 7.49 (d, *J* = 2.0 Hz, 1H), 7.33 (d, *J* = 12.2 Hz, 1H), 7.01 (d, *J* = 2.0 Hz, 1H), 5.00 (t, *J* = 8.2 Hz, 1H), 4.59 - 4.33 (m, 4H), 4.13 - 3.56 (m, 4H), 2.30 (s, 3H), 1.59 (t, *J* = 19.2 Hz, 3H), 1.29 (t, *J* = 7.1 Hz, 3H), 0.90 (dtd, *J* = 33.1, 8.6, 7.6, 4.1 Hz, 2H), 0.78 (q, *J* = 8.9 Hz, 1H), 0.48 (dt, *J* = 9.0, 4.9 Hz, 1H), 0.35 (dq, *J* = 21.1, 8.3, 7.8 Hz, 4H), 0.27 - 0.14 (m, 3H). |

(continued)

| Macrocyclic amine + pyrazolecarboxylic acid | Structure of compound | $^1$HNMR and/or MS (ESI) |
|---|---|---|
| 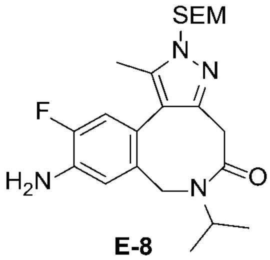 **E-8** 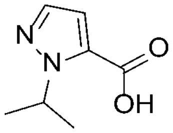 | 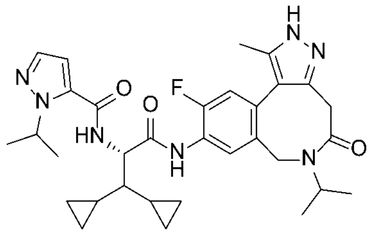 **compound 150** | MS (ESI) m/z =590 [M+1]$^+$ $^1$H NMR (400 MHz, Methanol-$d_4$) δ 8.23 (d, $J$ = 7.9 Hz, 1H), 7.52 (d, $J$ = 2.0 Hz, 1H), 7.23 (d, $J$ = 11.2 Hz, 1H), 6.80 (d, $J$ = 2.1 Hz, 1H), 5.41 (hept, $J$ = 6.7 Hz, 1H), 5.02 (d, $J$ = 7.2 Hz, 1H), 4.84 - 4.72 (m, 1H), 4.32 - 4.06 (m, 2H), 3.86 - 3.48 (m, 2H), 2.35 (s, 3H), 1.45 (t, $J$ = 6.9 Hz, 6H), 1.23 (dd, $J$ = 7.0, 3.2 Hz, 6H), 0.87 (dqd, $J$ = 30.9, 9.0, 6.0 Hz, 3H), 0.62 - 0.22 (m, 8H). |
| 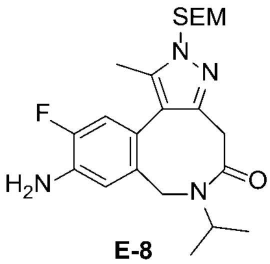 **E-8** 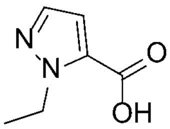 | 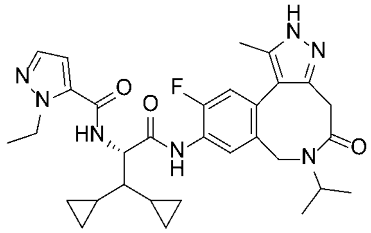 **compound 151** | MS (ESI) m/z =576 [M+1]$^+$ $^1$H NMR (400 MHz, Methanol-$d_4$) δ 8.22 (d, $J$ = 7.9 Hz, 1H), 7.50 (d, $J$ = 2.1 Hz, 1H), 7.23 (d, $J$ = 11.4 Hz, 1H), 6.87 (d, $J$ = 2.1 Hz, 1H), 5.02 (d, $J$ = 7.1 Hz, 1H), 4.85 - 4.73 (m, 1H), 4.54 (q, $J$ = 7.1 Hz, 2H), 4.35 - 4.07 (m, 2H), 3.84 - 3.52 (m, 2H), 2.35 (s, 3H), 1.39 (t, $J$ = 7.1 Hz, 3H), 1.23 (dd, $J$ = 6.9, 3.5 Hz, 6H), 1.00 - 0.76 (m, 3H), 0.64 - 0.20 (m, 8H). |
| 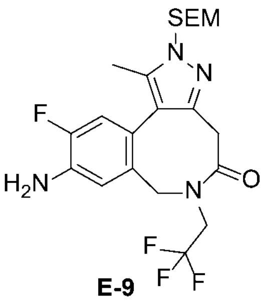 **E-9** 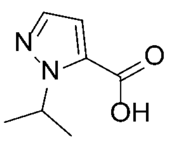 | 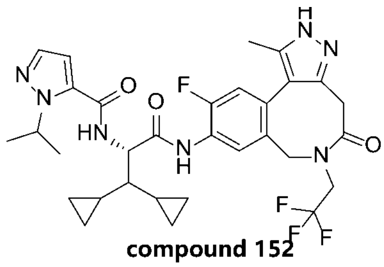 **compound 152** | MS (ESI) m/z =630 [M+1]$^+$ $^1$H NMR (600 MHz, Methanol-$d_4$) δ 8.15 (d, $J$ = 7.7 Hz, 1H), 7.53 (d, $J$ = 2.0 Hz, 1H), 7.39 - 7.23 (m, 1H), 6.80 (d, $J$ = 2.1 Hz, 1H), 5.41 (hept, $J$ = 6.7 Hz, 1H), 5.00 (d, $J$ = 7.4 Hz, 1H), 4.53 (d, $J$ = 56.6 Hz, 2H), 4.29 - 4.10 (m, 2H), 3.86 - 3.79 (m, 1H), 2.36 (s, 3H), 1.46 (dd, $J$ = 8.2, 6.7 Hz, 6H), 1.39 - 1.25 (m, 1H), 0.97 - 0.85 (m, 2H), 0.82 (td, $J$ = 9.3, 7.4 Hz, 1H), 0.57 (tt, $J$ = 8.7, 4.6 Hz, 1H), 0.55 - 0.41 (m, 3H), 0.38 (dq, $J$ = 9.7, 4.9 Hz, 1H), 0.34 - 0.24 (m, 3H). |

(continued)

| Macrocyclic amine + pyrazolecarboxylic acid | Structure of compound | $^1$HNMR and/or MS (ESI) |
|---|---|---|
| 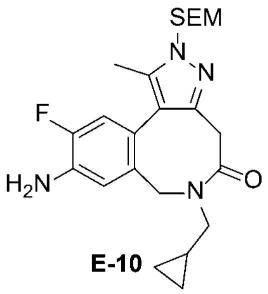 E-10 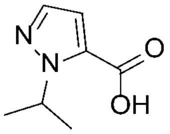 | 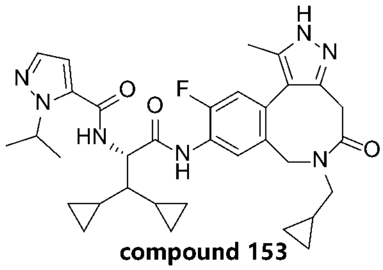 compound 153 | MS (ESI) m/z =602 [M+1]$^+$ $^1$H NMR (400 MHz, Methanol-$d_4$) δ 8.28 - 8.12 (m, 1H), 7.52 (d, $J$ = 2.1 Hz, 1H), 7.35 - 7.17 (m, 1H), 6.80 (d, $J$ = 2.0 Hz, 1H), 5.41 (hept, $J$ = 6.6 Hz, 1H), 5.01 (d, $J$ = 7.3 Hz, 1H), 4.56 - 4.35 (m, 2H), 4.08 - 3.71 (m, 2H), 3.45 - 3.32 (m, 2H), 2.34 (s, 3H), 1.45 (t, $J$ = 6.5 Hz, 6H), 1.19 - 1.04 (m, 1H), 0.99 - 0.76 (m, 3H), 0.62 - 0.21 (m, 12H). |
| 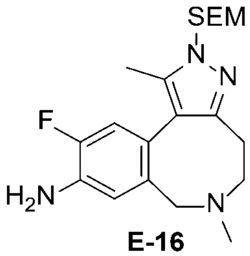 E-16 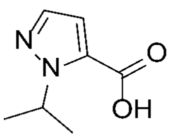 | 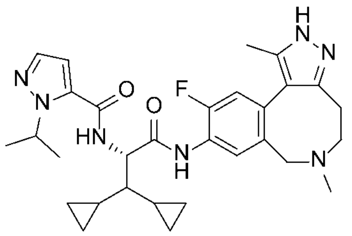 compound 154 | MS (ESI) m/z =548 [M+1]$^+$ $^1$H NMR (600 MHz, Methanol-$d_4$) δ 7.96 (d, $J$ = 8.0 Hz, 1H), 7.53 (d, $J$ = 2.0 Hz, 1H), 7.23 - 7.09 (m, 1H), 6.80 (d, $J$ = 2.0 Hz, 1H), 5.42 (hept, $J$ = 6.7 Hz, 1H), 5.01 (d, $J$ = 7.3 Hz, 1H), 3.65 (s, 2H), 3.08 - 2.54 (m, 4H), 2.41 (s, 3H), 2.24 (s, 3H), 1.47 (t, $J$ = 7.0 Hz, 6H), 0.97 - 0.79 (m, 3H), 0.58 (tq, $J$ = 8.3, 4.1 Hz, 1H), 0.54 - 0.42 (m, 3H), 0.39 (dq, $J$ = 9.7, 4.9 Hz, 1H), 0.34 - 0.25 (m, 3H). |
| 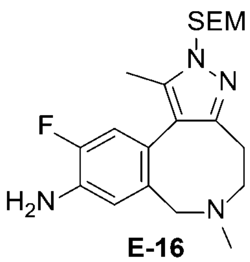 E-16 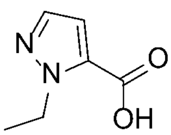 | 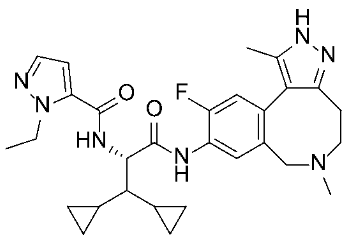 compound 155 | MS (ESI) m/z =534 [M+1]$^+$ $^1$H NMR (600 MHz, Methanol-$d_4$) δ 7.95 (d, $J$ = 7.9 Hz, 1H), 7.51 (d, $J$ = 2.1 Hz, 1H), 7.16 (d, $J$ = 9.6 Hz, 1H), 6.87 (d, $J$ = 2.1 Hz, 1H), 5.02 (d, $J$ = 7.3 Hz, 1H), 4.55 (qd, $J$ = 7.2, 1.4 Hz, 2H), 3.65 (s, 2H), 3.08 - 2.55 (m, 4H), 2.41 (s, 3H), 2.24 (s, 3H), 1.40 (t, $J$ = 7.2 Hz, 3H), 0.96 - 0.80 (m, 3H), 0.57 (tq, $J$ = 8.2, 4.0 Hz, 1H), 0.54 - 0.42 (m, 3H), 0.40 (dq, $J$ = 9.8, 4.9 Hz, 1H), 0.33 - 0.24 (m, 3H). |

(continued)

| Macrocyclic amine + pyrazolecarboxylic acid | Structure of compound | $^1$HNMR and/or MS (ESI) |
|---|---|---|
| 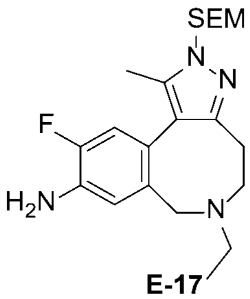 E-17 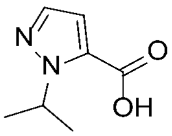 | 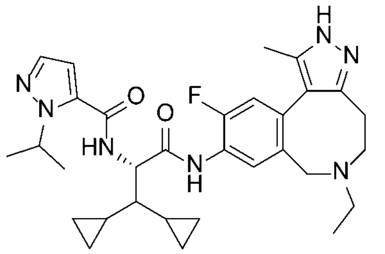 **compound 156** | MS (ESI) m/z =562 $[M+1]^+$ $^1$H NMR (400 MHz, DMSO-$d_6$) δ 12.25 (s, 1H), 9.96 (s, 1H), 8.42 (d, *J* = 8.9 Hz, 1H), 7.83 (d, *J* = 8.1 Hz, 1H), 7.51 (d, *J* = 2.0 Hz, 1H), 7.17 (d, *J* = 11.9 Hz, 1H), 6.93 (d, *J* = 2.0 Hz, 1H), 5.42 (hept, *J* = 6.5 Hz, 1H), 4.97 (dd, *J* = 8.8, 7.5 Hz, 1H), 3.51 (s, 2H), 2.67 (p, *J* = 1.9 Hz, 4H), 2.17 (s, 3H), 1.37 (dd, *J* = 13.1, 6.6 Hz, 6H), 1.24 (s, 1H), 1.09 (t, *J* = 7.1 Hz, 3H), 1.00 - 0.82 (m, 2H), 0.82 - 0.73 (m, 1H), 0.49 (tq, *J* = 8.0, 3.3 Hz, 1H), 0.44 - 0.25 (m, 5H), 0.21 (ddd, *J* = 13.0, 6.7, 4.1 Hz, 3H). |
| 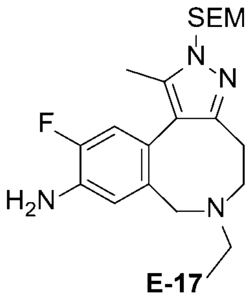 E-17 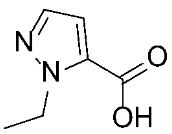 | 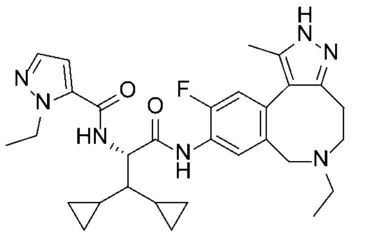 **compound 157** | MS (ESI) m/z =548 $[M+1]^+$ $^1$H NMR (400 MHz, DMSO-$d_6$) δ 12.32 (s, 1H), 9.96 (s, 1H), 8.43 (d, *J* = 8.9 Hz, 1H), 7.82 (d, *J* = 8.3 Hz, 1H), 7.49 (d, *J* = 2.0 Hz, 1H), 7.16 (d, *J* = 11.9 Hz, 1H), 7.01 (d, *J* = 2.1 Hz, 1H), 4.98 (dd, *J* = 8.8, 7.5 Hz, 1H), 4.49 (qd, *J* = 7.2, 4.0 Hz, 2H), 3.58 (d, *J* = 61.1 Hz, 2H), 2.67 (dt, *J* = 3.9, 1.9 Hz, 4H), 2.16 (s, 3H), 1.30 (t, *J* = 7.1 Hz, 3H), 1.24 (s, 1H), 1.08 (t, *J* = 7.0 Hz, 3H), 0.98 - 0.82 (m, 2H), 0.83 - 0.72 (m, 1H), 0.48 (tt, *J* = 8.1, 3.6 Hz, 1H), 0.44 - 0.25 (m, 5H), 0.25 - 0.14 (m, 3H). |
| 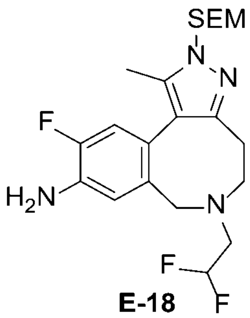 E-18 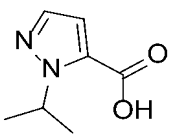 | 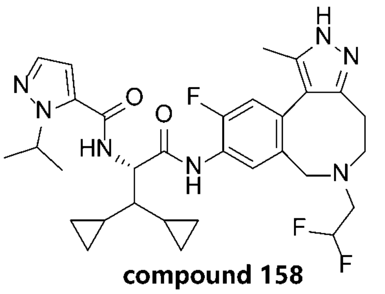 **compound 158** | MS (ESI) m/z =598 $[M+1]^+$ $^1$H NMR (400 MHz, DMSO-$d_6$) δ 12.40 (d, *J* = 47.4 Hz, 1H), 9.99 (s, 1H), 8.42 (d, *J* = 8.9 Hz, 1H), 7.84 (d, *J* = 8.2 Hz, 1H), 7.50 (d, *J* = 2.0 Hz, 1H), 7.27 - 7.12 (m, 1H), 6.92 (d, *J* = 2.0 Hz, 1H), 6.13 (tt, *J* = 56.1, 4.3 Hz, 1H), 5.41 (hept, *J* = 6.6 Hz, 1H), 4.97 (t, *J* = 8.2 Hz, 1H), 3.72 - 3.54 (m, 2H), 3.01 - 2.57 (m, 6H), 2.16 (d, *J* = 16.4 Hz, 3H), 1.37 (dd, *J* = 13.2, 6.6 Hz, 6H), 1.00 - 0.72 (m, 3H), 0.54 - 0.15 (m, 8H). |

(continued)

| Macrocyclic amine + pyrazolecarboxylic acid | Structure of compound | $^1$HNMR and/or MS (ESI) |
|---|---|---|
| 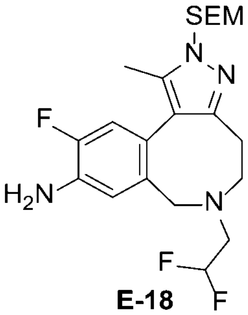 E-18 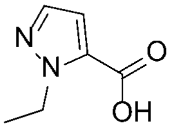 | 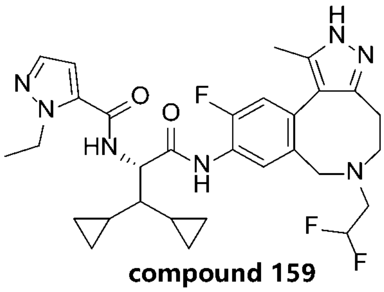 compound 159 | MS (ESI) m/z =584 [M+1]$^+$ <br> $^1$H NMR (400 MHz, DMSO-$d_6$) δ 12.40 (d, $J$ = 42.2 Hz, 1H), 10.00 (s, 1H), 8.44 (d, $J$ = 8.8 Hz, 1H), 7.83 (d, $J$ = 8.2 Hz, 1H), 7.48 (d, $J$= 2.0 Hz, 1H), 7.19 (d, $J$ = 11.7 Hz, 1H), 7.00 (d, $J$ = 2.0 Hz, 1H), 6.12 (tt, $J$ = 56.1, 4.3 Hz, 1H), 4.98 (t, $J$ = 8.1 Hz, 1H), 4.49 (qd, $J$ = 6.8, 3.1 Hz, 2H), 3.80 - 3.50 (m, 2H), 3.03 - 2.59 (m, 6H), 2.16 (s, 3H), 1.29 (t, $J$ = 7.1 Hz, 3H), 1.07 - 0.71 (m, 3H), 0.59 - 0.12 (m, 8H). |
| 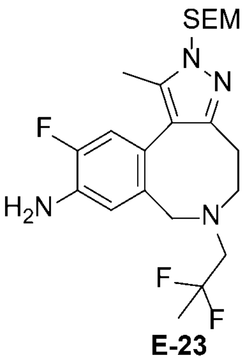 E-23 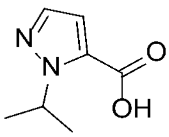 | 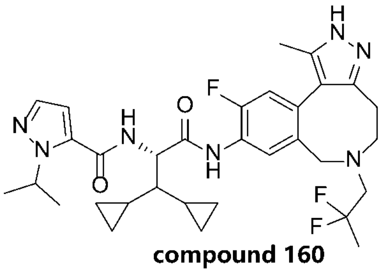 compound 160 | MS (ESI) m/z =612 [M+1]$^+$ <br> $^1$H NMR (400 MHz, Methanol-$d_4$) δ 8.22 (d, $J$ = 7.7 Hz, 1H), 7.53 (d, $J$ = 2.0 Hz, 1H), 7.36 (d, $J$ = 11.4 Hz, 1H), 6.80 (d, $J$ = 2.1 Hz, 1H), 5.41 (p, $J$ = 6.7 Hz, 1H), 4.99 (d, $J$ = 7.2 Hz, 1H), 4.33 (d, $J$ = 118.2 Hz, 2H), 3.90 (s, 2H), 3.59 - 3.38 (m, 2H), 3.13 (q, $J$ = 1.6 Hz, 2H), 2.30 (s, 3H), 1.82 (t, $J$ = 19.2 Hz, 3H), 1.46 (t, $J$ = 6.5 Hz, 6H), 1.00 - 0.79 (m, 3H), 0.59 (tq, $J$ = 8.5, 4.4 Hz, 1H), 0.54 - 0.42 (m, 3H), 0.39 (tt, $J$ = 9.3, 4.3 Hz, 1H), 0.36 - 0.22 (m, 3H). |
| 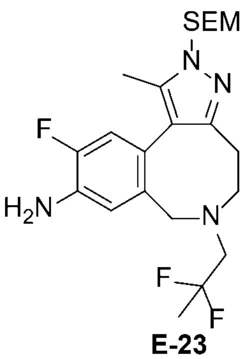 E-23 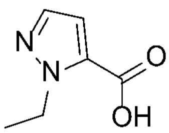 | 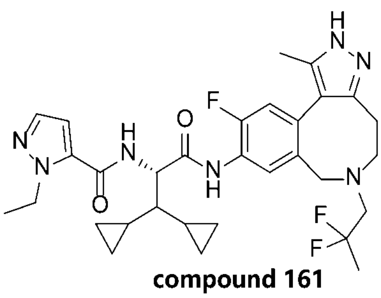 compound 161 | MS (ESI) m/z =598 [M+1]$^+$ <br> $^1$H NMR (400 MHz, Methanol-$d_4$) δ 8.21 (d, $J$ = 7.7 Hz, 1H), 7.52 (d, $J$ = 2.1 Hz, 1H), 7.36 (d, $J$ = 11.4 Hz, 1H), 6.87 (d, $J$ = 2.1 Hz, 1H), 5.03 - 4.96 (m, 1H), 4.55 (q, $J$ = 7.2 Hz, 2H), 4.18 (s, 2H), 3.87 (s, 2H), 3.48 (dt, $J$ = 3.3, 1.7 Hz, 2H), 3.25 - 2.92 (m, 2H), 2.29 (s, 3H), 1.81 (t, $J$ = 19.2 Hz, 3H), 1.39 (t, $J$ = 7.1 Hz, 3H), 1.00 - 0.80 (m, 3H), 0.59 (tt, $J$ = 8.7, 4.9 Hz, 1H), 0.48 (dtd, $J$ = 17.6, 8.6, 4.2 Hz, 3H), 0.38 (p, $J$ = 4.4 Hz, 1H), 0.36 - 0.22 (m, 3H). |

(continued)

| Macrocyclic amine + pyrazolecarboxylic acid | Structure of compound | $^1$HNMR and/or MS (ESI) |
|---|---|---|
| 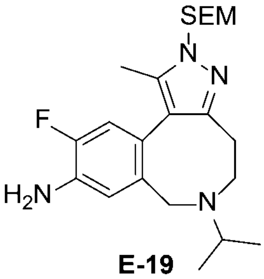 **E-19** 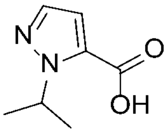 | 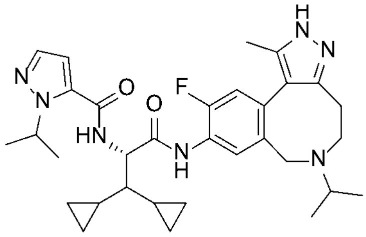 **compound 162** | MS (ESI) m/z =576 [M+1]$^+$ $^1$H NMR (400 MHz, Methanol-$d_4$) δ 8.06 (d, *J* = 8.0 Hz, 1H), 7.52 (d, *J* = 2.0 Hz, 1H), 7.11 (d, *J* = 11.4 Hz, 1H), 6.80 (d, *J* = 2.0 Hz, 1H), 5.41 (hept, *J* = 6.7 Hz, 1H), 5.00 (d, *J* = 7.1 Hz, 1H), 3.96 - 3.39 (m, 1H), 3.22 - 2.45 (m, 6H), 2.26 (s, 3H), 1.46 (t, *J* = 6.5 Hz, 6H), 1.17 (s, 6H), 0.99 - 0.74 (m, 3H), 0.62 - 0.23 (m, 8H). |
| 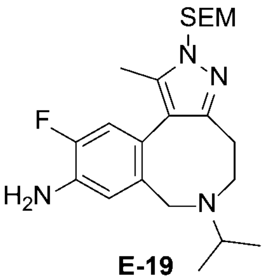 **E-19** 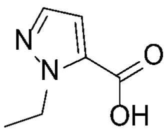 | 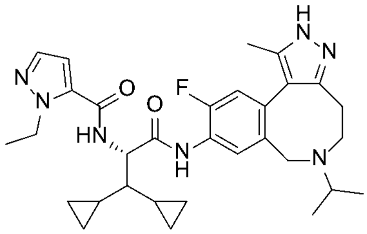 **compound 163** | MS (ESI) m/z =562 [M+1]$^+$ $^1$H NMR (400 MHz, Methanol-$d_4$) δ 8.05 (d, *J* = 8.0 Hz, 1H), 7.50 (d, *J* = 2.1 Hz, 1H), 7.11 (d, *J* = 11.4 Hz, 1H), 6.87 (d, *J* = 2.1 Hz, 1H), 4.55 (q, *J* = 7.2 Hz, 2H), 3.93 - 3.39 (m, 1H), 3.17 - 2.48 (m, 6H), 2.26 (s, 3H), 1.39 (t, *J* = 7.1 Hz, 3H), 1.17 (s, 6H), 0.98 - 0.78 (m, 3H), 0.63 - 0.21 (m, 8H). |
| 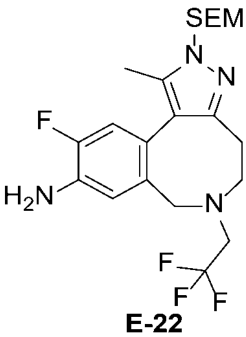 **E-22** 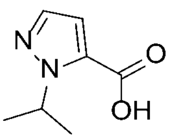 | 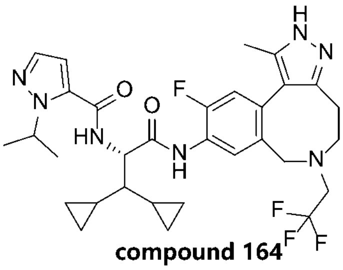 **compound 164** | MS (ESI) m/z =616 [M+1]$^+$ $^1$H NMR (600 MHz, Methanol-$d_4$) δ 7.93 (d, *J* = 7.9 Hz, 1H), 7.53 (d, *J* = 2.1 Hz, 1H), 7.20 - 7.12 (m, 1H), 6.80 (d, *J* = 2.0 Hz, 1H), 5.40 (h, *J* = 6.6 Hz, 1H), 4.99 (d, *J* = 7.4 Hz, 1H), 3.74 (s, 2H), 3.19 (d, *J* = 1.6 Hz, 2H), 2.99 (s, 2H), 2.69 (s, 2H), 2.24 (s, 3H), 1.46 (dd, *J* = 9.0, 6.7 Hz, 6H), 0.90 (dddd, *J* = 25.9, 9.8, 8.3, 4.3 Hz, 2H), 0.82 (td, *J* = 9.3, 7.3 Hz, 1H), 0.57 (tt, *J* = 8.4, 4.7 Hz, 1H), 0.55 - 0.41 (m, 3H), 0.38 (dq, *J* = 9.7, 4.9 Hz, 1H), 0.29 (ddp, *J* = 18.5, 9.3, 4.6 Hz, 3H). |

(continued)

| Macrocyclic amine + pyrazolecarboxylic acid | Structure of compound | $^1$HNMR and/or MS (ESI) |
| --- | --- | --- |
| 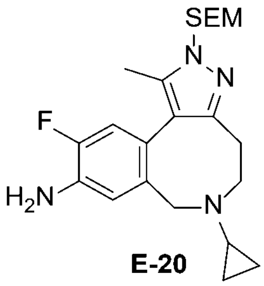 E-20 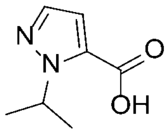 | 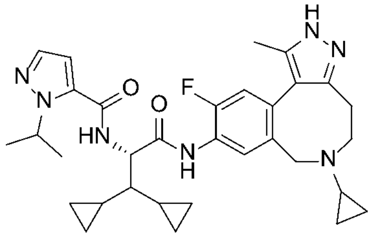 compound 165 | MS (ESI) m/z =574 [M+1]$^+$ $^1$H NMR (400 MHz, Methanol-$d_4$) δ 8.05 (d, $J$ = 8.1 Hz, 1H), 7.51 (d, $J$ = 2.1 Hz, 1H), 7.13 (d, $J$ = 11.5 Hz, 1H), 6.80 (d, $J$ = 2.0 Hz, 1H), 5.41 (hept, $J$ = 6.7 Hz, 1H), 5.03 (d, $J$ = 7.2 Hz, 1H), 3.97 - 3.49 (m, 2H), 3.19 - 2.53 (m, 4H), 2.24 (s, 3H), 1.91 (tt, $J$ = 6.2, 3.9 Hz, 1H), 1.45 (t, $J$ = 6.8 Hz, 6H), 1.00 - 0.75 (m, 3H), 0.67 - 0.36 (m, 9H), 0.36 - 0.21 (m, 3H). |
| 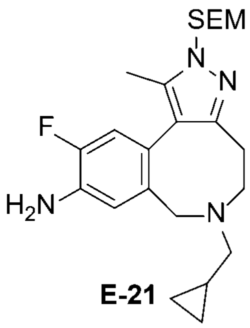 E-21 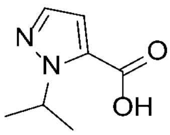 | 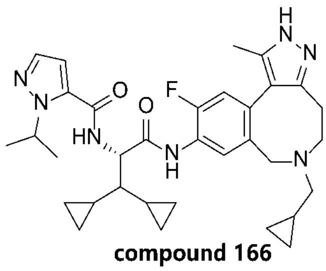 compound 166 | MS (ESI) m/z =588 [M+1]$^+$ $^1$H NMR (400 MHz, Methanol-$d_4$) δ 8.04 (d, $J$ = 8.0 Hz, 1H), 7.52 (d, $J$ = 2.0 Hz, 1H), 7.13 (d, $J$ = 11.6 Hz, 1H), 6.80 (d, $J$ = 2.0 Hz, 1H), 5.42 (hept, $J$ = 6.7 Hz, 1H), 5.03 (d, $J$ = 7.1 Hz, 1H), 4.07 - 3.49 (m, 2H), 3.21 - 2.31 (m, 6H), 2.24 (s, 3H), 1.45 (t, $J$ = 6.9 Hz, 6H), 1.07 - 0.77 (m, 4H), 0.65 - 0.35 (m, 7H), 0.35 - 0.23 (m, 3H), 0.23 - 0.15 (m, 2H). |
| 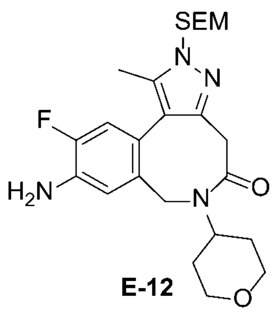 E-12 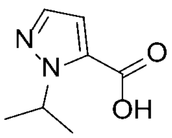 | 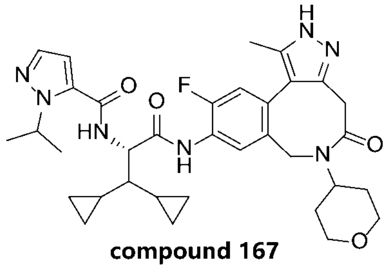 compound 167 | MS (ESI) m/z =632 [M+1]$^+$ $^1$H NMR (400 MHz, Methanol-$d_4$) δ 8.22 (d, $J$ = 7.9 Hz, 1H), 7.53 (d, $J$ = 2.1 Hz, 1H), 7.26 (s, 1H), 6.80 (d, $J$ = 2.1 Hz, 1H), 5.49 - 5.34 (m, 1H), 5.04 - 4.97 (m, 1H), 4.58 (s, 1H), 4.25 (s, 2H), 3.97 (s, 2H), 3.66 (s, 2H), 3.48 (t, $J$ = 11.6 Hz, 2H), 2.35 (s, 3H), 2.08 (s, 2H), 1.55 (s, 2H), 1.46 (t, $J$ = 6.4 Hz, 6H), 0.96 - 0.76 (m, 3H), 0.63 - 0.35 (m, 5H), 0.35 - 0.21 (m, 3H). |

(continued)

| Macrocyclic amine + pyrazolecarboxylic acid | Structure of compound | $^1$HNMR and/or MS (ESI) |
|---|---|---|
| 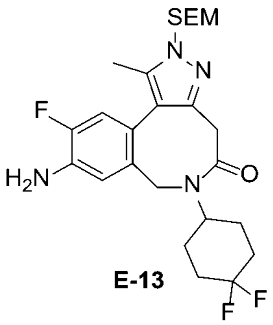 E-13 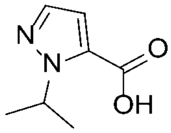 | 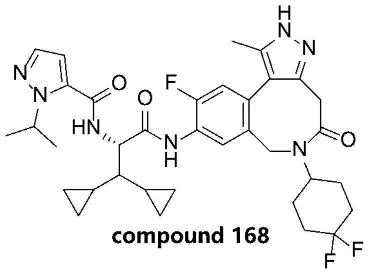 compound 168 | MS (ESI) m/z =666 [M+1]$^+$ <br> $^1$H NMR (600 MHz, Methanol-$d_4$) δ 8.20 (d, *J* = 7.8 Hz, 1H), 7.53 (d, *J* = 2.1 Hz, 1H), 7.37 - 7.22 (m, 1H), 6.80 (d, *J* = 2.1 Hz, 1H), 5.41 (hept, *J* = 6.7 Hz, 1H), 5.00 (d, *J* = 7.5 Hz, 1H), 4.45 - 4.18 (m, 3H), 3.67 (q, *J* = 16.3 Hz, 2H), 2.35 (s, 3H), 2.08 (s, 4H), 1.94 (dd, *J* = 33.5, 14.5 Hz, 2H), 1.62 (d, *J* = 48.7 Hz, 2H), 1.46 (dd, *J* = 9.0, 6.7 Hz, 6H), 0.97 - 0.85 (m, 2H), 0.82 (td, *J* = 9.3, 7.4 Hz, 1H), 0.57 (tt, *J* = 9.0, 4.6 Hz, 1H), 0.54 - 0.35 (m, 4H), 0.31 (tdd, *J* = 8.9, 5.0, 3.3 Hz, 2H), 0.25 (dq, *J* = 9.5, 4.9 Hz, 1H). |
| 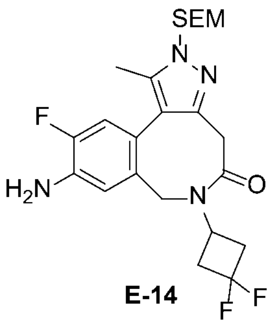 E-14 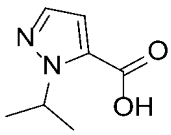 | 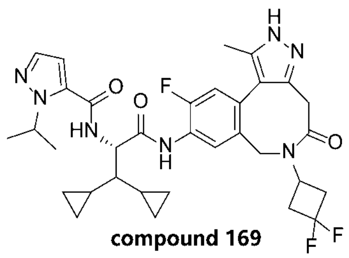 compound 169 | MS (ESI) m/z =638 [M+1]$^+$ <br> $^1$H NMR (600 MHz, DMSO-$d_6$) δ 8.05 (d, *J* = 8.1 Hz, 1H), 7.53 (d, *J* = 2.3 Hz, 1H), 7.35 - 7.20 (m, 1H), 6.80 (d, *J* = 2.4 Hz, 1H), 5.49 - 5.33 (m, 1H), 4.99 (s, 1H), 4.58 (s, 1H), 4.46 (s, 1H), 3.98 (s, 2H), 3.67 (d, *J* = 66.7 Hz, 1H), 3.05 - 2.90 (m, 2H), 2.88 - 2.73 (m, 2H), 2.36 (s, 3H), 1.49 - 1.41 (m, 6H), 0.98 - 0.76 (m, 3H), 0.60 - 0.54 (m, 1H), 0.54 - 0.44 (m, 2H), 0.44 - 0.34 (m, 2H), 0.34 - 0.22 (m, 3H). |
| 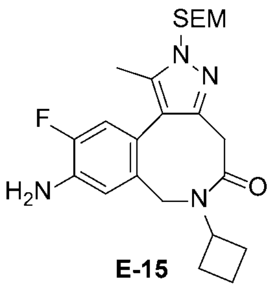 E-15 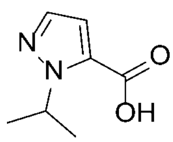 | 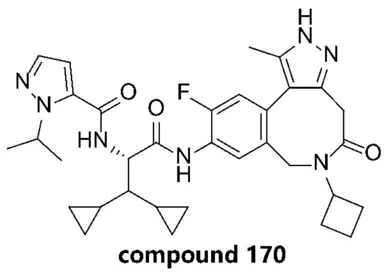 compound 170 | MS (ESI) m/z =602 [M+1]$^+$ <br> $^1$H NMR (600 MHz, Methanol-$d_4$) δ 8.00 (d, *J* = 7.9 Hz, 1H), 7.43 (d, *J* = 2.0 Hz, 1H), 7.15 (d, *J*= 11.7 Hz, 1H), 6.71 (d, *J* = 2.0 Hz, 1H), 5.32 (p, *J* = 6.7 Hz, 1H), 4.92 (d, *J* = 7.5 Hz, 1H), 4.30 (q, *J* = 15.2 Hz, 2H), 3.76 (s, 2H), 3.57 (s, 1H), 2.30 - 2.22 (m, 5H), 2.09 (qt, *J* = 10.2, 5.1 Hz, 2H), 1.69 - 1.56 (m, 2H), 1.37 (dd, *J* = 10.4, 6.6 Hz, 6H), 0.81 (dtq, *J* = 25.8, 8.3, 5.0, 4.3 Hz, 2H), 0.75 - 0.69 (m, 1H), 0.48 (tt, *J* = 8.8, 4.7 Hz, 1H), 0.44 - 0.27 (m, 4H), 0.20 (ddt, *J* = 23.0, 9.4, 4.7 Hz, 3H). |

(continued)

| Macrocyclic amine + pyrazolecarboxylic acid | Structure of compound | $^1$HNMR and/or MS (ESI) |
|---|---|---|
| 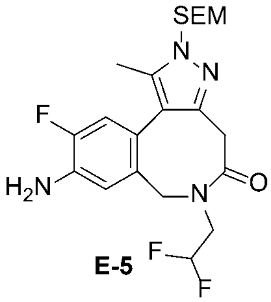 E-5 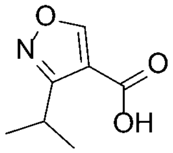 | 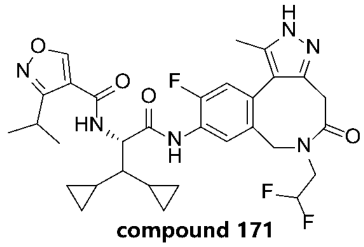 compound 171 | MS (ESI) m/z =613 $[M+1]^+$ $^1$H NMR (400 MHz, DMSO-$d_6$) δ 12.72 (d, *J* = 67.5 Hz, 1H), 10.05 (s, 1H), 9.37 (s, 1H), 8.47 (d, *J* = 8.9 Hz, 1H), 7.97 (s, 1H), 7.31 (s, 1H), 6.18 (t, *J* = 56.0 Hz, 1H), 4.99 (t, *J* = 8.0 Hz, 1H), 4.47 (s, 2H), 4.11 - 3.54 (m, 4H), 3.53 - 3.42 (m, 1H), 2.29 (s, 3H), 1.32 - 1.17 (m, 6H), 1.02 - 0.80 (m, 2H), 0.78 - 0.63 (m, 1H), 0.55 - 0.14 (m, 7H). |
| 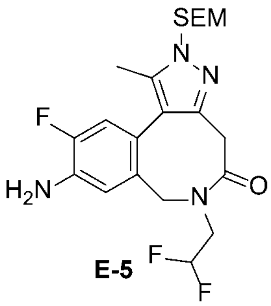 E-5 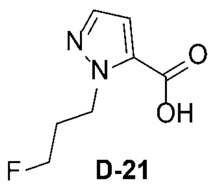 D-21 | 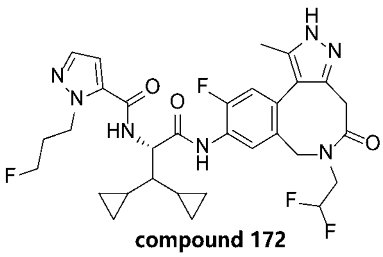 compound 172 | MS (ESI) m/z =630 $[M+1]^+$ $^1$H NMR (400 MHz, DMSO-$d_6$) δ 12.73 (d, *J* = 60.8 Hz, 1H), 10.09 (s, 1H), 8.50 (d, *J* = 8.9 Hz, 1H), 8.00 (d, *J* = 8.0 Hz, 1H), 7.53 (d, *J* = 2.0 Hz, 1H), 7.32 (d, *J* = 11.8 Hz, 1H), 7.07 (d, *J* = 2.1 Hz, 1H), 6.35 - 6.02 (m, 1H), 5.01 (t, *J* = 8.2 Hz, 1H), 4.59 (t, *J* = 7.0 Hz, 2H), 4.49 (d, *J* = 5.8 Hz, 3H), 4.37 (t, *J* = 5.9 Hz, 1H), 4.16 - 3.53 (m, 4H), 2.29 (s, 3H), 2.12 (dp, *J* = 25.9, 6.3 Hz, 2H), 1.00 - 0.73 (m, 3H), 0.56 - 0.14 (m, 8H). |
| 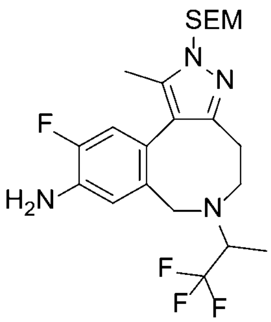 E-24 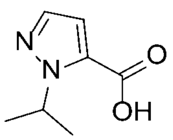 | 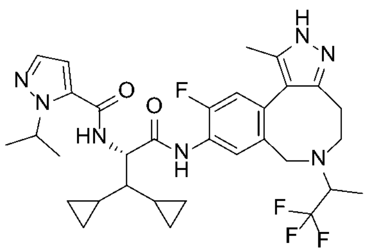 compound 173 (racemic modification) | MS (ESI) m/z =630 $[M+1]^+$ $^1$H NMR (400 MHz, Methanol-$d_4$) δ 8.04 (dd, *J* = 8.0, 1.3 Hz, 1H), 7.53 (d, *J* = 2.0 Hz, 1H), 7.14 (d, *J* = 11.4 Hz, 1H), 6.80 (d, *J* = 2.1 Hz, 1H), 5.41 (hept, *J* = 6.7 Hz, 1H), 5.01 (dd, *J* = 7.8, 5.2 Hz, 1H), 3.73 (d, *J* = 11.4 Hz, 3H), 3.18 - 2.99 (m, 1H), 2.87 (d, *J* = 7.7 Hz, 1H), 2.69 (d, *J* = 49.9 Hz, 2H), 2.29 (s, 3H), 1.46 (dd, *J* = 6.6, 5.3 Hz, 6H), 1.31 (t, *J* = 6.8 Hz, 3H), 0.99 - 0.78 (m, 3H), 0.58 (dq, *J* = 9.1, 4.8 Hz, 1H), 0.48 (ddt, *J* = 19.1, 7.7, 4.0 Hz, 3H), 0.38 (dq, *J* = 9.4, 4.9, 4.3 Hz, 1H), 0.30 (tt, *J* = 9.0, 4.4 Hz, 3H). |

(continued)

| Macrocyclic amine + pyrazolecarboxylic acid | Structure of compound | $^1$HNMR and/or MS (ESI) |
|---|---|---|
| 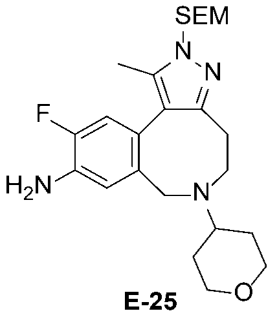 E-25 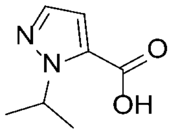 | 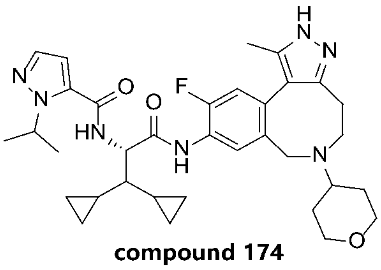 **compound 174** | MS (ESI) m/z =618 [M+1]$^+$ $^1$H NMR (400 MHz, Methanol-$d_4$) δ 8.32 (dd, *J* = 21.3, 7.7 Hz, 1H), 7.53 (t, *J* = 1.5 Hz, 1H), 7.36 (d, *J*= 11.2 Hz, 1H), 6.80 (t, *J* = 2.1 Hz, 1H), 5.50 - 5.34 (m, 1H), 4.99 (d, *J* = 7.1 Hz, 1H), 4.64 (s, 1H), 4.11 (d, *J* = 11.7 Hz, 2H), 3.76 (s, 2H), 3.52 (d, *J* = 12.7 Hz, 3H), 3.36 (s, 2H), 2.80 (s, 1H), 2.32 (d, *J* = 1.6 Hz, 3H), 2.13 (d, *J* = 41.4 Hz, 3H), 1.88 (d, *J* = 12.3 Hz, 1H), 1.53 - 1.39 (m, 6H), 0.97 - 0.80 (m, 3H), 0.59 (tt, *J* = 8.5, 4.8 Hz, 1H), 0.54 - 0.36 (m, 4H), 0.35 - 0.19 (m, 3H). |
| 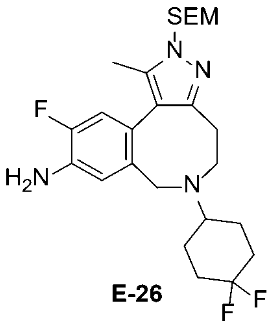 E-26 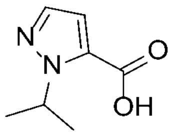 | 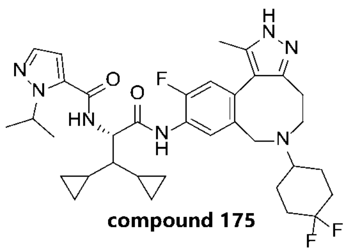 **compound 175** | MS (ESI) m/z =652 [M+1]$^+$ |
| 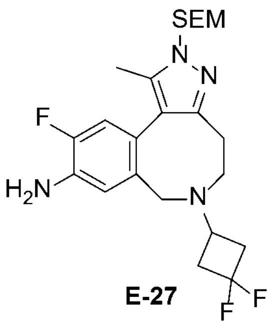 E-27 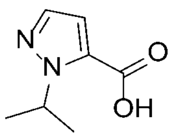 | 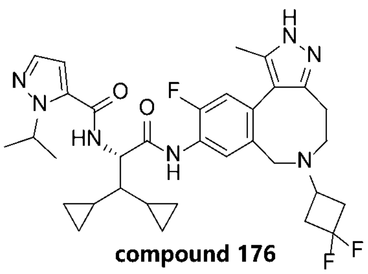 **compound 176** | MS (ESI) m/z =624 [M+1]$^+$ $^1$H NMR (600 MHz, DMSO-$d_6$) δ 7.95 (s, 1H), 7.58 - 7.47 (m, 1H), 7.16 (s, 1H), 6.86 - 6.75 (m, 1H), 5.50 - 5.37 (m, 1H), 5.00 (t, *J* = 7.0 Hz, 1H), 4.58 (s, 1H), 3.67 (s, 2H), 3.48 - 3.41 (m, 2H), 2.91 (d, *J* = 103.4 Hz, 4H), 2.50 (s, 2H), 2.23 (s, 3H), 1.47 (dd, *J* = 9.6, 6.7 Hz, 6H), 0.97 - 0.81 (m, 3H), 0.62 - 0.41 (m, 4H), 0.41 - 0.25 (m, 4H). |

(continued)

| Macrocyclic amine + pyrazolecarboxylic acid | Structure of compound | $^1$HNMR and/or MS (ESI) |
|---|---|---|
| 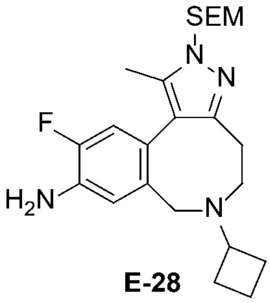 E-28 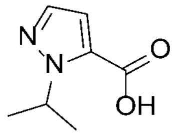 | 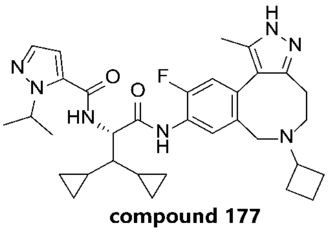 compound 177 | MS (ESI) m/z =588 [M+1]$^+$ 1H NMR (600 MHz, Methanol-$d_4$) δ 7.95 (s, 1H), 7.53 (d, *J* = 2.0 Hz, 1H), 7.15 (d, *J* = 11.5 Hz, 1H), 6.80 (d, *J* = 2.1 Hz, 1H), 5.41 (hept, *J* = 6.6 Hz, 1H), 5.01 (d, *J* = 7.4 Hz, 1H), 3.66 (s, 2H), 3.14 - 2.91 (m, 2H), 2.70 (s, 2H), 2.47 (s, 1H), 2.23 (s, 5H), 1.97 (s, 2H), 1.79 - 1.66 (m, 2H), 1.46 (dd, *J* = 9.6, 6.7 Hz, 6H), 0.95 - 0.79 (m, 3H), 0.60 - 0.37 (m, 5H), 0.30 (dhept, *J* = 18.1, 4.7 Hz, 3H). |
| 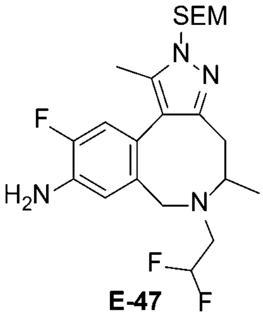 E-47 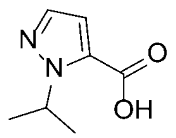 | 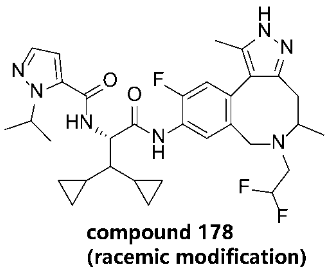 compound 178 (racemic modification) | MS (ESI) m/z =612 [M+1]$^+$ $^1$H NMR (600 MHz, Methanol-$d_4$) δ 7.95 (dd, *J* = 20.3, 7.9 Hz, 1H), 7.53 (d, *J* = 2.0 Hz, 1H), 7.13 (s, 1H), 6.80 (t, *J* = 1.8 Hz, 1H), 5.90 (tt, *J* = 56.5, 4.3 Hz, 1H), 5.41 (p, *J* = 6.6 Hz, 1H), 5.00 (dd, *J* = 7.4, 3.7 Hz, 1H), 3.63 (s, 2H), 3.23 - 3.11 (m, 2H), 2.82 - 2.60 (m, 2H), 2.51 (d, *J* = 14.6 Hz, 1H), 2.25 (s, 3H), 1.46 (dd, *J* = 8.8, 6.7 Hz, 6H), 1.06 (s, 3H), 0.90 (tdd, *J* = 15.0, 7.8, 4.4 Hz, 2H), 0.82 (tdd, *J* = 9.4, 7.3, 4.0 Hz, 1H), 0.57 (tt, *J* = 8.0, 4.1 Hz, 1H), 0.53 - 0.42 (m, 3H), 0.38 (dt, *J* = 9.6, 4.6 Hz, 1H), 0.30 (tq, *J* = 9.4, 4.9 Hz, 3H). |
| 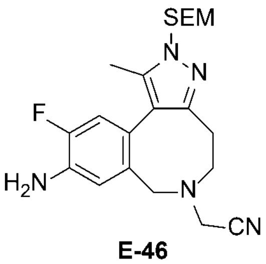 E-46 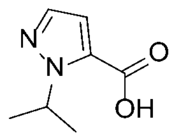 | 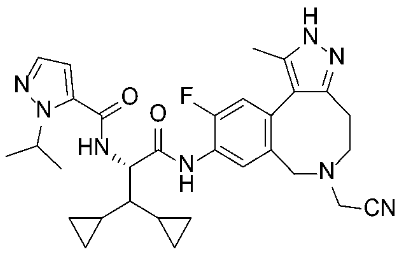 compound 179 | MS (ESI) m/z =573 [M+1]$^+$ $^1$H NMR (400 MHz, Methanol-$d_4$) δ 8.02 (d, *J* = 8.0 Hz, 1H), 7.52 (d, *J* = 2.1 Hz, 1H), 7.15 (d, *J* = 11.3 Hz, 1H), 6.80 (d, *J* = 2.1 Hz, 1H), 5.41 (hept, *J* = 6.7 Hz, 1H), 5.01 (d, *J* = 7.1 Hz, 1H), 3.78 (s, 2H), 3.65 (s, 2H), 3.02 - 2.70 (m, 4H), 2.26 (s, 3H), 1.46 (dd, *J* = 6.7, 4.7 Hz, 6H), 0.99 - 0.78 (m, 3H), 0.61 - 0.35 (m, 5H), 0.35 - 0.24 (m, 3H). |

(continued)

| Macrocyclic amine + pyrazolecarboxylic acid | Structure of compound | $^1$HNMR and/or MS (ESI) |
|---|---|---|
| 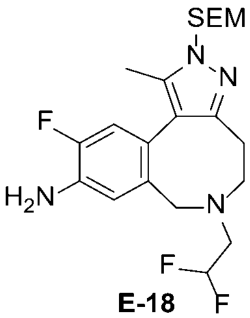 E-18 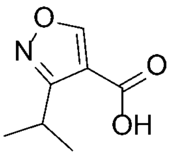 | 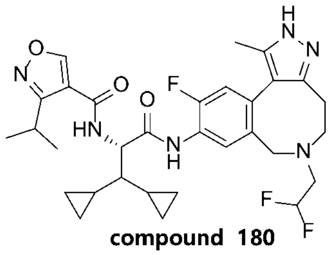 compound 180 | MS (ESI) m/z =599 [M+1]$^+$ $^1$H NMR (600 MHz, DMSO-$d_6$) δ 12.18 (d, *J* = 60.3 Hz, 1H), 9.76 (s, 1H), 9.15 (s, 1H), 8.25 (d, *J* = 8.9 Hz, 1H), 7.60 (d, *J* = 8.2 Hz, 1H), 6.98 (d, *J* = 11.6 Hz, 1H), 5.91 (tt, *J* = 56.1, 4.3 Hz, 1H), 4.78 (dd, *J* = 8.9, 7.2 Hz, 1H), 3.41 (s, 1H), 3.25 (p, *J* = 7.0 Hz, 1H), 3.15 (dd, *J* = 4.9, 2.6 Hz, 2H), 2.77 - 2.36 (m, 5H), 1.95 (s, 3H), 1.05 (d, *J* = 6.9 Hz, 3H), 1.02 (d, *J* = 6.9 Hz, 3H), 0.78 - 0.61 (m, 2H), 0.48 (td, *J* = 9.6, 7.2 Hz, 1H), 0.30 - -0.04 (m, 8H). |
| 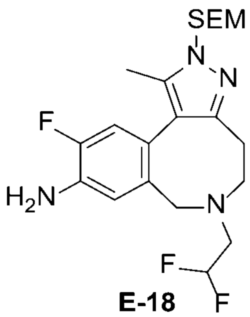 E-18 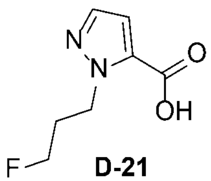 D-21 | 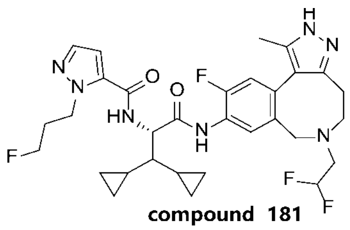 compound 181 | MS (ESI) m/z =616 [M+1]$^+$ $^1$H NMR (600 MHz, DMSO-$d_6$) δ 12.38 (d, *J* = 62.9 Hz, 1H), 10.00 (s, 1H), 8.47 (d, *J* = 9.0 Hz, 1H), 7.84 (d, *J* = 8.2 Hz, 1H), 7.52 (d, *J* = 2.0 Hz, 1H), 7.19 (d, *J* = 11.6 Hz, 1H), 7.06 (d, *J* = 2.1 Hz, 1H), 6.13 (tt, *J* = 56.2, 4.3 Hz, 1H), 5.00 (t, *J* = 8.3 Hz, 1H), 4.59 (t, *J* = 7.0 Hz, 2H), 4.46 (t, *J* = 5.8 Hz, 1H), 4.38 (t, *J* = 5.9 Hz, 1H), 3.48 (d, *J* = 169.1 Hz, 3H), 3.00 - 2.56 (m, 5H), 2.12 (ddd, *J* = 26.2, 13.9, 7.3 Hz, 5H), 0.97 - 0.75 (m, 3H), 0.56 - 0.13 (m, 8H). |

**Example 182 Preparation of compound 182**

A preparation of compound 182 is illustrated as follows:

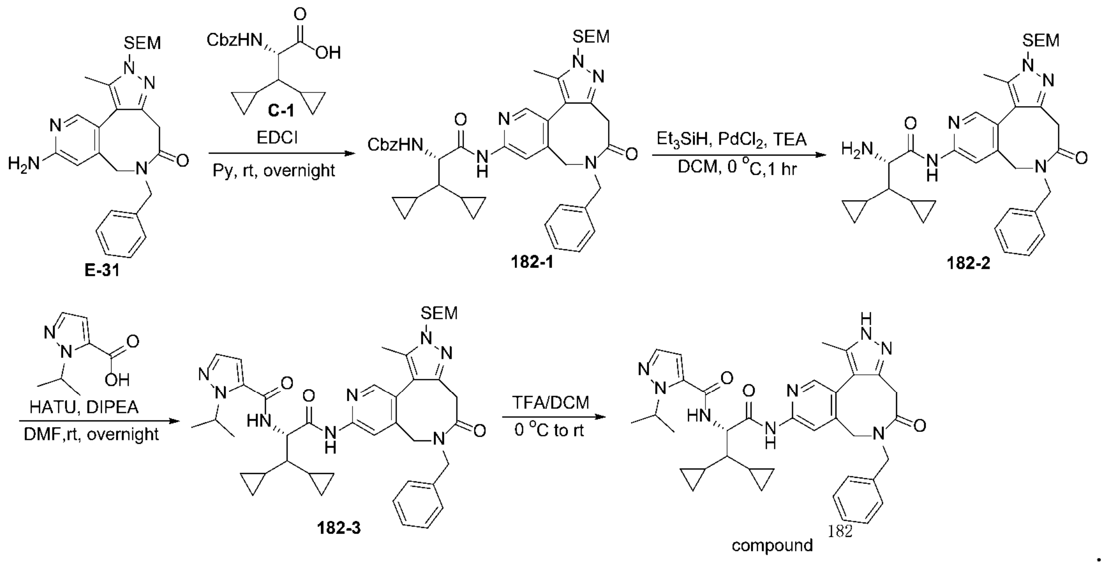


E-31 182-1 182-2

182-3 compound 182

.

(S1) Preparation of intermediate 182-1

The preparation of intermediate 182-1 was performed according to the synthesis method in Example 12, in which intermediate 12-5 in step (S6) was replaced with intermediate E-31, with other reagents and procedures remaining identical. MS (ESI) m/z = 749 $[M+1]^+$.

(S2) Preparation of intermediate 182-2

To a single-neck flask were added intermediate 182-1 (300 mg, 401.07 μmol) and DCM (4 mL), followed by addition of palladium dichloride (14.12 mg, 80.21 μmol) and triethylamine (28.28 mg, 280.75 μmol) and dropwise addition of triethylsilane (232.62 mg, 2.01 mmol) under an ice-water bath. The mixture was warmed to room temperature and stirred for 1 h. Upon completion, the reaction mixture was filtered through Celite. The filtrate was concentrated under reduced pressure to dryness. The residue was purified by reverse-phase MPLC to obtain 210.87 mg (343.44 μmol) of intermediate 182-2 (yield: 85.63%). MS (ESI) m/z = 615 $[M+1]^+$.

(S3-S4) Preparation of compound 182

The preparation of compound 182 was performed according to the synthesis method in Example 12, in which intermediate 12-7 in step (S8) was replaced with intermediate 182-2, with other reagents and procedures remaining identical. MS (ESI) m/z = 621 $[M+1]^+$.

**Examples 183-185 Preparation of compound 183-185**

The preparation of compounds 183-185 was performed according to the synthesis method in Example 182, in which intermediate E-31 in step (S1) was correspondingly replaced with a macrocyclic amine from the following table, with other reagents and procedures remaining identical.

| Macrocyclic amine | Structure of compound | 1HNMR and/or MS (ESI) |
|---|---|---|
| 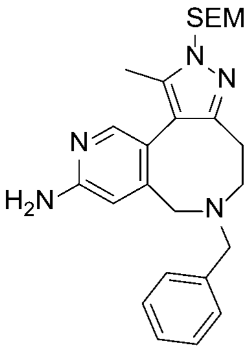 E-37 | 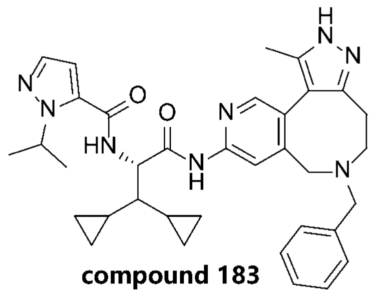 compound 183 | MS (ESI) m/z =607 $[M+1]^+$ |
| 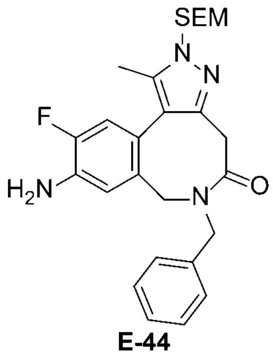 E-44 | 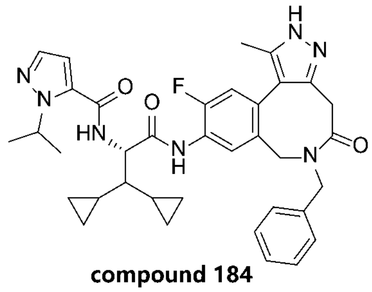 compound 184 | MS (ESI) m/z =638 $[M+1]^+$ |
| 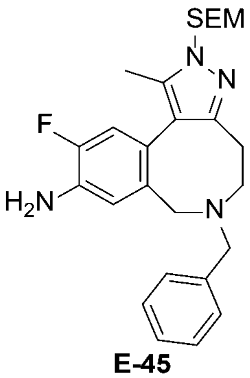 E-45 | 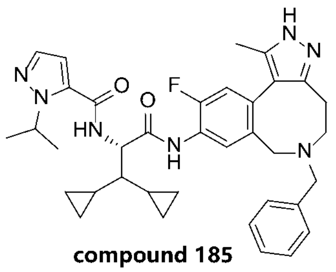 compound 185 | MS (ESI) m/z =624 $[M+1]^+$ |

**Example 186 Preparation of compound 186a and 186b**

A structure of compound 186a/186b is as follows:

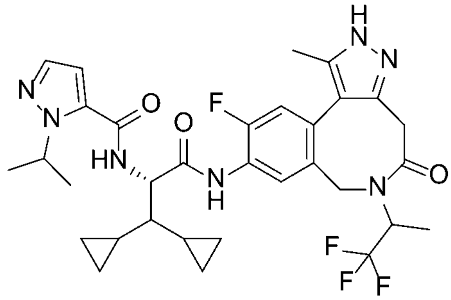

**compound 186a & compound 186b**.

The preparation of compounds 186a and 186b was performed according to the synthesis method in Example

118, in which intermediate 118-8 in step (S9) was replaced with intermediate E-11, with all other reagents and procedures remaining identical. After SEM group was removed, the crude product was subjected to purification by MPLC ($H_2O/CH_3CN$, 0.05% $NH_4HCO_3$) and separation by SFC to obtain compounds 186a and 186b, in which SFC was performed using a Daicel CHIRALPAK AD-3 column (3 μm, 150 mm × 3 mm) with $CO_2$ (as mobile phase A) and 0.1% DEA/ethanol (as mobile phase B) (A/B = 75/25), at a flow rate of 1 mL/min and a column temperature of 40°C. For compound 186a (P1, SFC $t_R$ = 2.533), MS (ESI) m/z = 644 $[M+1]^+$. $^1H$ NMR (600 MHz, Methanol-$d_4$) δ 8.24 (d, *J* = 7.7 Hz, 1H), 7.52 (d, *J* = 2.0 Hz, 1H), 7.25 (d, *J* = 10.8 Hz, 1H), 6.79 (d, *J* = 2.0 Hz, 1H), 5.53 (d, *J* = 8.1 Hz, 1H), 5.40 (hept, *J* = 6.6 Hz, 1H), 5.00 (d, *J* = 7.4 Hz, 1H), 4.21 (t, *J* = 12.8 Hz, 2H), 3.72 - 3.51 (m, 2H), 2.36 (s, 3H), 1.53 (d, *J* = 7.2 Hz, 3H), 1.46 (dd, *J* = 11.3, 6.6 Hz, 6H), 0.96 - 0.86 (m, 2H), 0.82 (td, *J* = 9.3, 7.4 Hz, 1H), 0.61 - 0.55 (m, 1H), 0.54 - 0.41 (m, 3H), 0.37 (dt, *J* = 9.6, 4.7 Hz, 1H), 0.30 (dhept, *J* = 18.4, 4.8 Hz, 3H).

For compound 186b (P2, SFC $t_R$ = 4.165), MS (ESI) m/z = 644 $[M+1]^+$. $^1H$ NMR (600 MHz, Methanol-$d_4$) δ 8.23 (d, *J* = 7.7 Hz, 1H), 7.53 (d, *J= 2.0* Hz, 1H), 7.25 (d, *J* = 10.9 Hz, 1H), 6.80 (d, *J* = 2.0 Hz, 1H), 5.53 (s, 1H), 5.41 (hept, *J* = 6.7 Hz, 1H), 4.99 (d, *J* = 7.4 Hz, 1H), 4.35 - 4.15 (m, 2H), 3.76 - 3.49 (m, 2H), 2.36 (s, 3H), 1.54 (d, *J* = 7.2 Hz, 3H), 1.46 (dd, *J* = 10.4, 6.7 Hz, 6H), 0.90 (dddt, *J* = 22.6, 13.0, 8.0, 5.0 Hz, 2H), 0.81 (td, *J* = 9.3, 7.4 Hz, 1H), 0.57 (tt, *J* = 9.0, 4.3 Hz, 1H), 0.54 - 0.40 (m, 3H), 0.37 (dq, *J* = 9.7, 4.9 Hz, 1H), 0.31 (ttd, *J* = 7.2, 5.0, 2.6 Hz, 2H), 0.25 (dq, *J* = 9.6, 4.9 Hz, 1H).

## Example 187 Preparation of compound 187a and 187b

A structure of compound 187a/187b is as follows:

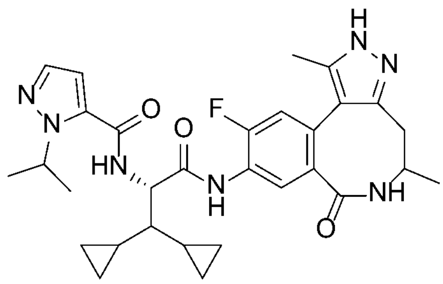

compound 187a & compound 187b

The preparation of compounds 187a and 187b was performed according to the synthesis method in Example 118, in which intermediate 118-8 in step (S9) was replaced with intermediate E-29, with all other reagents and procedures remaining identical. After SEM group was removed, the crude product was subjected to purification by MPLC ($H_2O/CH_3CN$, 0.05% $NH_4HCO_3$) and separation by SFC to obtain compounds 187a and 187b, in which SFC was performed using a Daicel CHIRALPAK AD-3 column (3 μm, 150 mm × 3 mm) with $CO_2$ (as mobile phase A) and ethanol (as mobile phase B) (A/B = 75/25), at a flow rate of 1 mL/min and a column temperature of 40°C.

For compound 187a (P1, SFC $t_R$ = 2.677), MS (ESI) m/z = 562 $[M+1]^+$. $^1H$ NMR (600 MHz, DMSO-$d_6$) δ 12.22 (d, *J* = 109.0 Hz, 1H), 9.86 (s, 1H), 8.21 (d, *J* = 8.6 Hz, 1H), 7.86 - 7.58 (m, 2H), 7.28 (d, *J* = 2.0 Hz, 1H), 6.95 (d, *J* = 11.8 Hz, 1H), 6.69 (d, *J= 2.0* Hz, 1H), 5.17 (p, *J* = 6.6 Hz, 1H), 4.74 (t, *J* = 8.2 Hz, 1H), 3.51 (dddd, *J* = 13.0, 10.1, 6.4, 3.9 Hz, 1H), 2.64 (d, *J* = 32.0 Hz, 1H), 2.47 (dd, *J* = 16.4, 12.5 Hz, 1H), 1.85 (s, 3H), 1.14 (dd, *J* = 15.3, 6.6 Hz, 6H), 0.93 (d, *J* = 6.5 Hz, 3H), 0.76 - 0.69 (m, 1H), 0.68 - 0.62 (m, 1H), 0.55 (td, *J* = 9.4, 7.8 Hz, 1H), 0.30 - 0.22 (m, 1H), 0.19 - -0.03 (m, 6H), -0.04 - -0.09 (m, 1H).

For compound 187b (P2, SFC $t_R$ = 4.627), MS (ESI) m/z = 562 $[M+1]^+$. $^1H$ NMR (600 MHz, DMSO-$d_6$) δ 12.45 (d, *J* = 102.7 Hz, 1H), 10.03 (s, 1H), 8.45 (d, *J* = 8.6 Hz, 1H), 7.88 (t, *J* = 11.6 Hz, 2H), 7.51 (d, *J* = 1.9 Hz, 1H), 7.17 (d, *J* = 11.7 Hz, 1H), 6.90 (d, *J* = 2.0 Hz, 1H), 5.39 (p, *J* = 6.6 Hz, 1H), 5.02 - 4.86 (m, 1H), 3.74 (dtd, *J* = 9.1, 6.3, 2.6 Hz, 1H), 2.85 (s, 1H), 2.69 (dd, *J* = 16.3, 12.4 Hz, 1H), 2.08 (s, 3H), 1.37 (dd, *J* = 19.7, 6.6 Hz, 6H), 1.16 (d, *J* = 6.5 Hz, 3H), 0.97 - 0.83 (m, 2H), 0.79 (td, *J* = 9.4, 7.6 Hz, 1H), 0.49 (dt, *J* = 13.2, 6.4 Hz, 1H), 0.42 - 0.15 (m, 7H).

## Example 188 Preparation of compound 188a and 188b

A structure of compound 188a/188b is as follows:

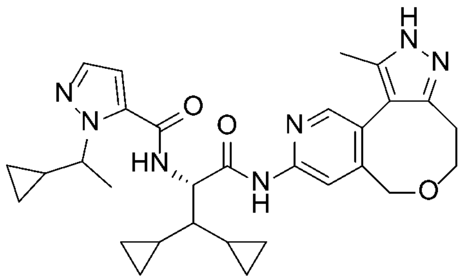

compound 188a & compound188b

The preparation of compounds 188a and 188b was performed according to the synthesis method in Example 12, in which 1-isopropylpyrazole-5-carboxylic acid in step (S8) was replaced with intermediate D-41, with all other reagents and procedures remaining identical. After SEM group was removed, the crude product was subjected to purification by MPLC ($H_2O/CH_3CN$, 0.05% $NH_4HCO_3$) and separation by SFC to obtain compounds 188a and 188b, in which SFC was performed using a Daicel CHIRALPAK AD-3 column (3 μm, 150 mm × 3 mm) with $CO_2$ (as mobile phase A) and 0.1% DEA/ethanol (as mobile phase B) (A/B = 60/40), at a flow rate of 1 mL/min and a column temperature of 40°C.

For compound 188a (P1, SFC $t_R$ = 2.850), MS (ESI) m/z = 544 $[M+1]^+$. $^1H$ NMR (600 MHz, Methanol-$d_4$) δ 8.32 (s, 1H), 8.22 (s, 1H), 7.56 (d, *J* = 2.0 Hz, 1H), 6.80 (d, *J* = 2.0 Hz, 1H), 4.93 (d, *J* = 7.1 Hz, 1H), 4.52 (dq, *J* = 9.6, 6.7 Hz, 3H), 3.68 (d, *J* = 96.4 Hz, 2H), 2.68 (d, *J* = 35.9 Hz, 2H), 2.30 (s, 3H), 1.58 (d, *J* = 6.8 Hz, 3H), 1.33 (dddd, *J* = 12.9, 9.7, 8.0, 4.9 Hz, 1H), 0.94 - 0.80 (m, 3H), 0.62 - 0.53 (m, 2H), 0.53 - 0.43 (m, 2H), 0.40 - 0.32 (m, 3H), 0.32 - 0.27 (m, 4H), 0.24 (dt, *J* = 9.2, 4.7 Hz, 1H).

For compound 188b (P2, SFC $t_R$ = 5.574), MS (ESI) m/z = 544 $[M+1]^+$. $^1H$ NMR (600 MHz, Methanol-$d_4$) δ 8.31 (s, 1H), 8.22 (s, 1H), 7.56 (d, *J* = 2.0 Hz, 1H), 6.84 (d, *J* = 2.1 Hz, 1H), 4.94 (d, *J* = 6.7 Hz, 1H), 4.58 (dq, *J* = 9.5, 6.7 Hz, 1H), 4.35 (s, 2H), 3.75 (s, 2H), 2.71 (s, 2H), 2.30 (s, 3H), 1.56 (d, *J* = 6.8 Hz, 3H), 1.38 (dddd, *J* = 12.8, 9.8, 8.0, 4.9 Hz, 1H), 0.92 (dtd, *J* = 13.2, 8.3, 4.9 Hz, 1H), 0.88 - 0.82 (m, 2H), 0.62 (tdd, *J* = 8.8, 5.4, 3.6 Hz, 1H), 0.56 (dq, *J* = 8.1, 3.6 Hz, 1H), 0.52 - 0.43 (m, 2H), 0.36 (dddd, *J* = 21.3, 10.7, 5.2, 3.5 Hz, 4H), 0.30 (dt, *J* = 9.2, 4.9 Hz, 3H), 0.24 (dt, *J* = 9.3, 4.8 Hz, 1H).

## Example 189 Preparation of compound 189a and 189b

A structure of compound 189a/189b is as follows:

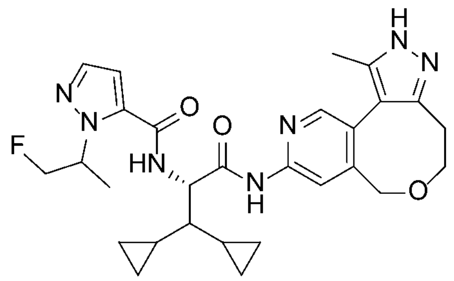

compound 189a & compound 189b

The preparation of compounds 189a and 189b was performed according to the synthesis method in Example 12, in which 1-isopropylpyrazole-5-carboxylic acid in step (S8) was replaced with intermediate D-40, with all other reagents and procedures remaining identical. After SEM group was removed, the crude product was subjected to purification by MPLC ($H_2O/CH_3CN$, 0.05% $NH_4HCO_3$) and separation by SFC to obtain compounds 189a and 189b, in which SFC was performed using a Daicel CHIRALCEL OX-3 column (3 μm, 150 mm × 3 mm) with $CO_2$ (as mobile phase A) and ethanol (as mobile phase B) (A/B = 80/20), at a flow rate of 1 mL/min and a column temperature of 40°C.

For compound 189a (P1, SFC $t_R$ = 3.813), MS (ESI) m/z = 536 $[M+1]^+$. $^1H$ NMR (600 MHz, Methanol-$d_4$) δ 8.33 (d, *J* = 29.6 Hz, 1H), 8.22 (s, 1H), 7.58 (d, *J* = 2.0 Hz, 1H), 6.86 (d, *J* = 2.1 Hz, 1H), 5.64 (dddd, *J* = 13.1, 8.0, 6.7, 4.6 Hz, 1H), 4.98 (d, *J* = 7.1 Hz, 1H), 4.69 (ddd, *J* = 47.7, 9.4, 8.1 Hz, 1H), 4.61 - 4.59 (m, 0.5H), 4.52 (dd, *J* = 9.4, 4.7 Hz, 0.5H), 4.35 (s, 2H), 3.69 (d, *J* = 72.1 Hz, 2H), 2.74 (d, *J* = 35.5 Hz, 2H), 2.31 (s, 3H), 1.48 (dd, *J* = 6.9, 1.6 Hz, 3H), 0.94 - 0.81 (m, 3H), 0.58 - 0.43 (m, 3H), 0.41 - 0.33 (m, 2H), 0.30 (dqt, *J* = 9.3, 4.7, 2.9 Hz, 2H), 0.24 (dq, *J* = 9.4, 4.9 Hz, 1H).

For compound 189b (P2, SFC $t_R$ = 4.502), MS (ESI) m/z = 536 $[M+1]^+$. $^1H$ NMR (600 MHz, Methanol-$d_4$) δ 8.32 (s, 1H), 8.23 (s, 1H), 7.57 (d, *J* = 2.0 Hz, 1H), 6.88 (d, *J* = 2.0 Hz, 1H), 5.66 (dddd, *J* = 13.2, 8.2, 6.8, 4.6 Hz, 1H), 5.00 (d, *J* = 7.1 Hz, 1H), 4.70 (ddd, *J* = 47.8, 9.4, 8.1 Hz, 1H), 4.61 (dd, *J* = 9.3, 4.6 Hz, 0.5H), 4.53 (dd, *J* = 9.4, 4.6 Hz, 0.5H), 4.35 (s, 2H),

3.94 - 3.57 (m, 2H), 2.65 (s, 2H), 2.30 (s, 3H), 1.46 (dd, *J* = 6.9, 1.6 Hz, 3H), 0.94 - 0.81 (m, 3H), 0.55 (tq, *J* = 8.7, 4.1 Hz, 1H), 0.51 - 0.42 (m, 2H), 0.37 (dddd, *J* = 9.3, 7.5, 5.2, 4.0 Hz, 1H), 0.34 - 0.22 (m, 4H).

**Example 190 Preparation of compound 190a and 190b**

A structure of compound 190a/190b is as follows:

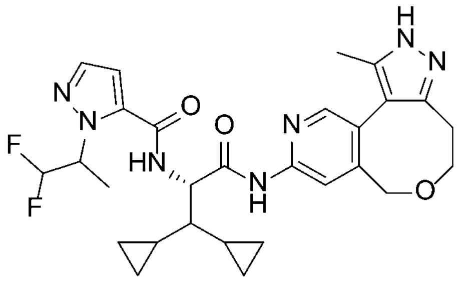

**compound 190a & compound 190b**

The preparation of compounds 190a and 190b was performed according to the synthesis method in Example 12, in which 1-isopropylpyrazole-5-carboxylic acid in step (S8) was replaced with intermediate D-1, with all other reagents and procedures remaining identical. After SEM group was removed, the crude product was subjected to purification by MPLC ($H_2O/CH_3CN$, 0.05% $NH_4HCO_3$) and separation by SFC to obtain compounds 190a and 190b, in which SFC was performed using a Daicel CHIRALPAK AD-3 column (3 μm, 150 mm × 3 mm) with $CO_2$ (as mobile phase A) and ethanol (as mobile phase B) (A/B = 80/20), at a flow rate of 1 mL/min and a column temperature of 40°C.

For compound 190a (P1, SFC $t_R$ = 2.627), MS (ESI) m/z = 554 $[M+1]^+$. $^1H$ NMR (600 MHz, Methanol-$d_4$) δ 8.35 (d, *J* = 33.5 Hz, 1H), 8.23 (s, 1H), 7.62 (d, *J* = 2.0 Hz, 1H), 6.95 (d, *J* = 2.0 Hz, 1H), 6.10 (td, *J* = 56.2, 5.3 Hz, 1H), 5.70 (ddd, *J* = 10.6, 5.3, 1.7 Hz, 1H), 5.01 (d, *J* = 7.2 Hz, 1H), 4.36 (s, 2H), 3.77 (s, 2H), 2.70 (d, *J* = 32.6 Hz, 2H), 2.33 (s, 3H), 1.62 (dd, *J* = 7.1, 1.2 Hz, 3H), 0.96 - 0.82 (m, 3H), 0.60 - 0.54 (m, 1H), 0.54 - 0.49 (m, 1H), 0.49 - 0.44 (m, 1H), 0.42 - 0.34 (m, 2H), 0.34 - 0.29 (m, 2H), 0.26 (dq, *J* = 9.3, 4.9 Hz, 1H).

For compound 190b (P2, SFC $t_R$ = 5.788), MS (ESI) m/z = 554 $[M+1]^+$. $^1H$ NMR (600 MHz, Methanol-$d_4$) δ 8.33 (s, 1H), 8.24 (s, 1H), 7.62 (d, *J* = 2.0 Hz, 1H), 6.97 (d, *J* = 2.0 Hz, 1H), 6.10 (td, *J* = 56.4, 5.4 Hz, 1H), 5.71 (dd, *J* = 7.1, 5.4 Hz, 1H), 5.02 (d, *J* = 7.1 Hz, 1H), 4.36 (s, 2H), 3.77 (s, 2H), 2.73 (s, 2H), 2.32 (s, 3H), 1.61 (dd, *J* = 7.0, 1.2 Hz, 3H), 0.96 - 0.83 (m, 3H), 0.57 (tq, *J* = 8.8, 5.2, 4.6 Hz, 1H), 0.53 - 0.43 (m, 2H), 0.42 - 0.36 (m, 1H), 0.30 (dddt, *J* = 26.5, 22.1, 9.2, 4.4 Hz, 4H).

**Examples 191-213 Preparation of compounds 191-213**

The preparation of compounds 191 to 213 was performed according to steps (S6-S9) of Example 12, in which intermediate 12-5 in step (S6) was correspondingly replaced with a macrocyclic amine from the following table, with all other reagents and procedures remaining identical.

| Macrocyclic amine | Structure of compound | $^1$HNMR and/or MS (ESI) |
|---|---|---|
| 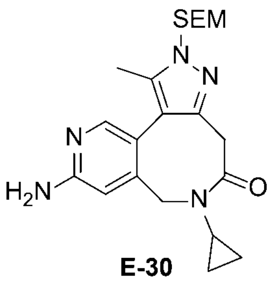 **E-30** | 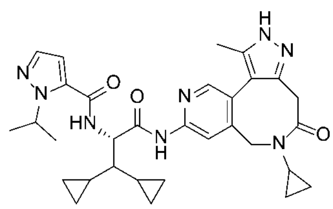 **compound 191** | MS (ESI) m/z =571 $[M+1]^+$<br>$^1H$ NMR (600 MHz, Methanol-$d_4$) δ 8.41 (s, 1H), 8.15 (s, 1H), 7.53 (d, *J* = 2.0 Hz, 1H), 6.82 (d, *J* = 2.1 Hz, 1H), 5.47 - 5.34 (m, 1H), 4.95 (d, *J* = 7.2 Hz, 1H), 4.71 - 4.53 (m, 2H), 4.13 - 3.99 (m, 2H), 2.41 (s, 3H), 2.18 (tt, *J* = 7.2, 4.0 Hz, 1H), 1.45 (dd, *J* = 11.2, 6.7 Hz, 6H), 0.95 - 0.82 (m, 5H), 0.77 - 0.72 (m, 2H), 0.57 (tq, *J* = 8.7, 4.6 Hz, 1H), 0.53 - 0.48 (m, 1H), 0.48 - 0.42 (m, 1H), 0.39 - 0.27 (m, 4H), 0.22 (dq, *J* = 9.3, 4.8 Hz, 1H). |

(continued)

| Macrocyclic amine | Structure of compound | $^1$HNMR and/or MS (ESI) |
|---|---|---|
| 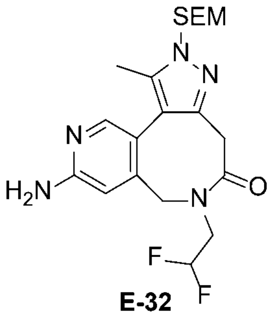 E-32 | 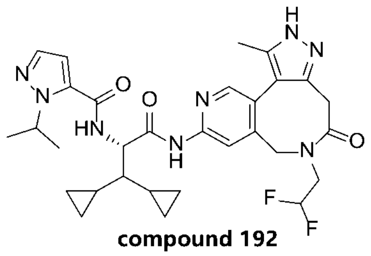 compound 192 | MS (ESI) m/z =595 [M+1]$^+$ $^1$H NMR (600 MHz, Methanol-$d_4$) δ 8.21 (d, *J* = 47.8 Hz, 1H), 8.03 (s, 1H), 7.30 (d, *J* = 2.0 Hz, 1H), 6.59 (d, *J* = 2.0 Hz, 1H), 5.81 (tt, *J* = 56.1, 4.2 Hz, 1H), 5.18 (p, *J* = 6.6 Hz, 1H), 4.76 (d, *J* = 7.3 Hz, 1H), 4.37 (d, *J* = 18.2 Hz, 2H), 4.02 - 3.72 (m, 2H), 3.51 (s, 2H), 2.15 (d, *J* = 20.8 Hz, 3H), 1.22 (dd, *J* = 8.9, 6.7 Hz, 6H), 0.74 - 0.57 (m, 3H), 0.36 - 0.20 (m, 3H), 0.16 - -0.01 (m, 5H). |
| 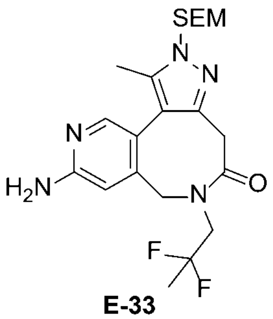 E-33 | 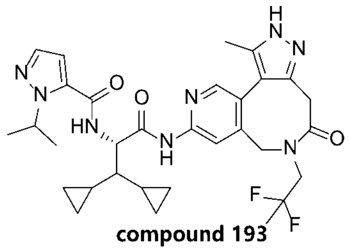 compound 193 | MS (ESI) m/z =609 [M+1]$^+$ $^1$H NMR (600 MHz, Methanol-$d_4$) δ 8.42 (s, 1H), 8.23 (s, 1H), 7.53 (d, *J* = 2.0 Hz, 1H), 6.82 (d, *J* = 2.0 Hz, 1H), 5.40 (hept, *J* = 6.7 Hz, 1H), 4.99 (t, *J* = 6.8 Hz, 1H), 4.69 - 4.58 (m, 2H), 4.09 (q, *J* = 16.5 Hz, 2H), 3.78 (dq,*J* = 45.6, 13.9 Hz, 2H), 2.39 (s, 3H), 1.60 (t, *J* = 18.9 Hz, 3H), 1.45 (dd, *J* = 8.8, 6.7 Hz, 6H), 0.98 - 0.79 (m, 3H), 0.60 - 0.42 (m, 3H), 0.33 (dddd, *J* = 31.9, 14.4, 8.9, 4.4 Hz, 4H), 0.24 (dq, *J* = 9.4, 4.9 Hz, 1H). |
| 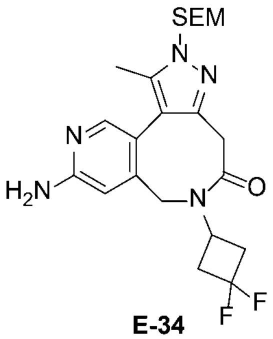 E-34 | 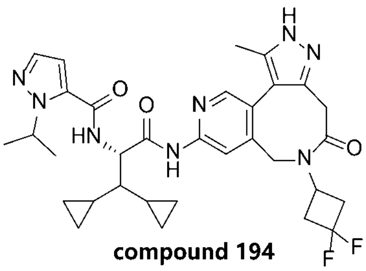 compound 194 | MS (ESI) m/z =621 [M+1]$^+$ $^1$H NMR (600 MHz, Methanol-$d_4$) δ 8.43 (d, *J* = 45.0 Hz, 1H), 8.17 (s, 1H), 7.55 (d, *J* = 2.0 Hz, 1H), 6.84 (d, *J* = 2.0 Hz, 1H), 5.42 (p, *J* = 6.7 Hz, 1H), 5.00 (d, *J* = 7.4 Hz, 1H), 4.70 (d, *J* = 15.1 Hz, 1H), 4.54 (d, *J* = 15.1 Hz, 1H), 4.13 (d, *J* = 15.7 Hz, 1H), 4.02 (d, *J* = 16.4 Hz, 1H), 3.56 (s, 1H), 3.02 - 2.91 (m, 2H), 2.83 (td, *J* = 21.0, 20.1, 10.2 Hz, 2H), 2.40 (d, *J* = 25.1 Hz, 3H), 1.47 (dd,*J* = 10.5, 6.7 Hz, 6H), 0.98 - 0.82 (m, 3H), 0.60 - 0.44 (m, 3H), 0.34 (dtd, *J* = 20.7, 9.6, 9.0, 4.4 Hz, 4H), 0.25 (dq, *J* = 9.3, 4.9 Hz, 1H). |
| 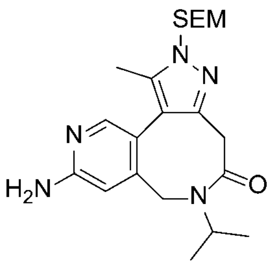 E-35 | 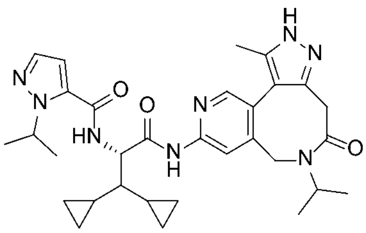 compound 195 | MS (ESI) m/z =573 [M+1]$^+$ $^1$H NMR (400 MHz, Methanol-$d_4$) δ 8.52 - 8.29 (m, 2H), 7.52 (d, *J* = 2.1 Hz, 1H), 6.82 (d, *J* = 2.1 Hz, 1H), 5.41 (hept, *J* = 6.6 Hz, 1H), 5.00 (d, *J* = 7.0 Hz, 1H), 4.68 - 4.26 (m, 3H), 3.85 (dq, *J* = 65.8, 16.2 Hz, 2H), 2.39 (s, 3H), 1.45 (t, *J* = 6.6 Hz, 6H), 1.19 (d, *J* = 6.9 Hz, 6H), 1.01 - 0.75 (m, 3H), 0.61 - 0.40 (m, 3H), 0.40 - 0.18 (m, 5H). |
| 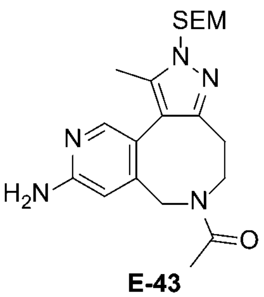 E-43 | 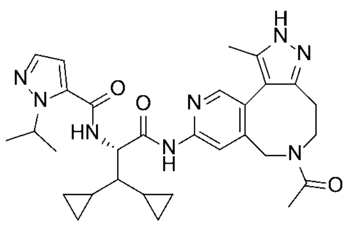 compound 196 | MS (ESI) m/z =559 [M+1]$^+$ |

(continued)

| Macrocyclic amine | Structure of compound | $^1$HNMR and/or MS (ESI) |
|---|---|---|
| 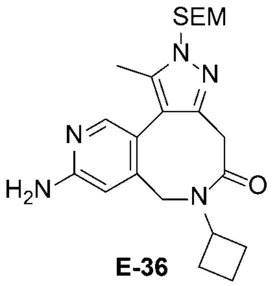 E-36 | 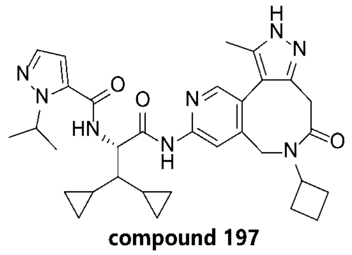 compound 197 | MS (ESI) m/z =585 [M+1]$^+$ $^1$H NMR (600 MHz, Methanol-$d_4$) δ 8.37 (s, 1H), 8.21 (s, 1H), 7.55 (d, $J$ = 2.0 Hz, 1H), 6.84 (d, $J$ = 2.1 Hz, 1H), 5.53 - 5.29 (m, 1H), 4.99 (d, $J$ = 7.4 Hz, 1H), 4.59 (d, $J$ = 14.8 Hz, 1H), 4.48 (d, $J$ = 15.0 Hz, 1H), 3.99 (q, $J$ = 45.5, 36.0 Hz, 3H), 2.40 (s, 5H), 2.19 (q, $J$ = 9.6 Hz, 2H), 1.85 - 1.63 (m, 2H), 1.47 (dd, $J$ = 9.6, 6.6 Hz, 6H), 1.02 - 0.79 (m, 3H), 0.63 - 0.43 (m, 3H), 0.35 (dddd, $J$ = 26.7, 14.2, 9.0, 4.4 Hz, 4H), 0.26 (dd, $J$ = 9.4, 4.6 Hz, 1H). |
| 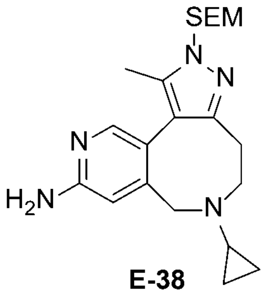 E-38 | 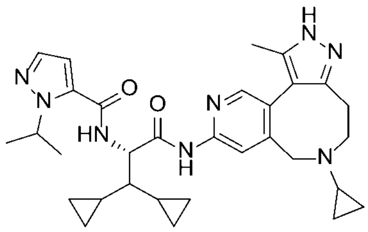 compound 198 | MS (ESI) m/z =557 [M+1]$^+$ $^1$H NMR (600 MHz, Methanol-$d_4$) δ 8.30 (d, $J$ = 23.2 Hz, 2H), 7.53 (d, $J$ = 2.0 Hz, 1H), 6.82 (d, $J$ = 2.1 Hz, 1H), 5.40 (hept, $J$ = 6.7 Hz, 1H), 4.97 (d, $J$ = 7.1 Hz, 1H), 4.28 (d, $J$ = 437.1 Hz, 2H), 3.28 - 2.50 (m, 4H), 2.29 (s, 3H), 1.45 (dd, $J$ = 9.2, 6.7 Hz, 6H), 0.96 - 0.91 (m, 1H), 0.90 - 0.85 (m, 1H), 0.83 (dd, $J$ = 9.3, 7.4 Hz, 1H), 0.69 (s, 4H), 0.55 (dq, $J$ = 9.2, 4.7, 4.2 Hz, 1H), 0.51 (tq, $J$ = 9.2, 4.1 Hz, 1H), 0.44 (dq, $J$ = 8.8, 4.5 Hz, 1H), 0.39 - 0.27 (m, 4H), 0.24 (dq, $J$ = 9.4, 4.9 Hz, 1H). |
| 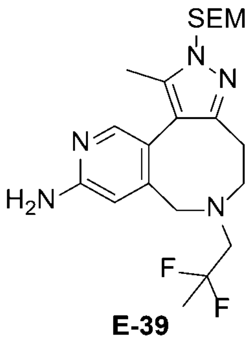 E-39 | 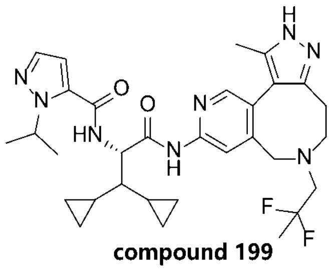 compound 199 | MS (ESI) m/z =595 [M+1]$^+$ $^1$H NMR (600 MHz, Methanol-$d_4$) δ 8.28 (s, 1H), 8.16 (s, 1H), 7.52 (d, $J$ = 2.0 Hz, 1H), 6.82 (d, $J$ = 2.2 Hz, 1H), 5.40 (p, $J$ = 6.7 Hz, 1H), 4.99 (d, $J$ = 7.2 Hz, 1H), 3.77 (s, 2H), 2.96 (s, 4H), 2.83 - 2.62 (m, 2H), 2.26 (s, 3H), 1.64 (t, $J$ = 18.6 Hz, 3H), 1.45 (t, $J$ = 7.7 Hz, 6H), 0.96 - 0.80 (m, 3H), 0.58 - 0.43 (m, 3H), 0.32 (tddd, $J$ = 29.5, 24.8, 8.8, 4.3 Hz, 5H). |
| 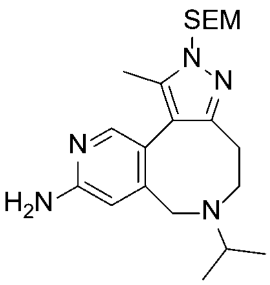 E-40 | 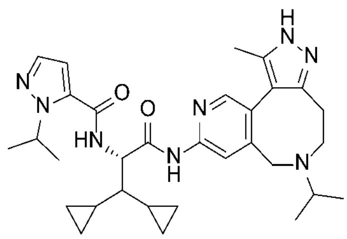 compound 200 | MS (ESI) m/z =559 [M+1]$^+$ $^1$H NMR (400 MHz, Methanol-$d_4$) δ 8.26 (s, 2H), 7.53 (d, $J$ = 2.1 Hz, 1H), 6.82 (d, $J$ = 2.1 Hz, 1H), 5.41 (hept, $J$ = 6.7 Hz, 1H), 4.98 (d, $J$ = 6.7 Hz, 1H), 3.84 - 3.29 (m, 2H), 3.11 (hept, $J$ = 6.5 Hz, 1H), 2.96 - 2.46 (m, 4H), 2.28 (s, 3H), 1.45 (t, $J$ = 6.3 Hz, 6H), 1.17 (s, 6H), 1.00 - 0.76 (m, 3H), 0.61 - 0.20 (m, 8H). |

(continued)

| Macrocyclic amine | Structure of compound | $^1$HNMR and/or MS (ESI) |
|---|---|---|
| 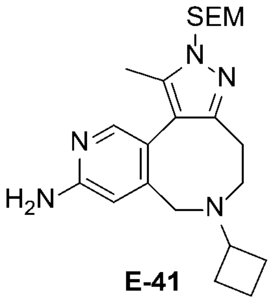 E-41 | 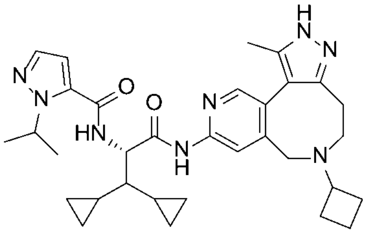 **compound 201** | MS (ESI) m/z =571 [M+1]$^+$ $^1$H NMR (600 MHz, Methanol-$d_4$) δ 8.30 (s, 1H), 7.54 (d, $J$ = 2.1 Hz, 1H), 7.40 (t, $J$ = 7.7 Hz, 1H), 7.28 (d, $J$ = 7.8 Hz, 1H), 6.83 (d, $J$= 2.0 Hz, 1H), 5.42 (p, $J$ = 6.7 Hz, 1H), 3.54 - 3.41 (m, 3H), 3.06 (s, 1H), 2.27 (s, 5H), 1.98 (d, $J$ = 8.2 Hz, 2H), 1.82 - 1.66 (m, 2H), 1.63 (t, $J$ = 2.9 Hz, 2H), 1.47 (dd, $J$ = 8.8, 6.6 Hz, 6H), 1.40 - 1.27 (m, 2H), 0.99 - 0.81 (m, 3H), 0.63 - 0.44 (m, 3H), 0.44 - 0.24 (m, 4H). |
| 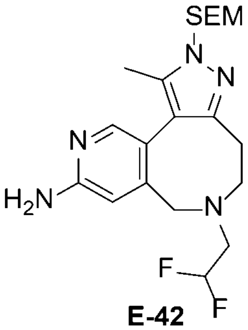 E-42 | 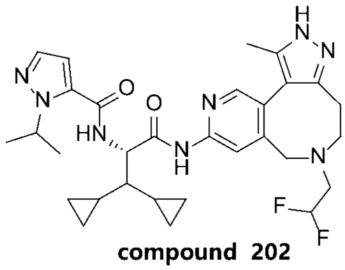 **compound 202** | MS (ESI) m/z =581 [M+1]$^+$ $^1$H NMR (600 MHz, Methanol-$d_4$) δ 8.28 (s, 1H), 8.16 (s, 1H), 7.53 (d, $J$ = 2.0 Hz, 1H), 6.82 (d, $J$ = 2.0 Hz, 1H), 5.98 (tt, $J$ = 56.2, 4.2 Hz, 1H), 5.40 (p, $J$ = 6.6 Hz, 1H), 4.98 (d, $J$ = 7.3 Hz, 1H), 3.76 (s, 2H), 2.86 (d, $J$ = 138.7 Hz, 6H), 2.27 (s, 3H), 1.46 (dd, $J$ = 8.2, 6.5 Hz, 6H), 0.97 - 0.80 (m, 3H), 0.60 - 0.43 (m, 3H), 0.41 - 0.22 (m, 5H). |
| 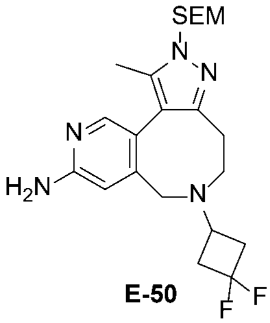 E-50 | 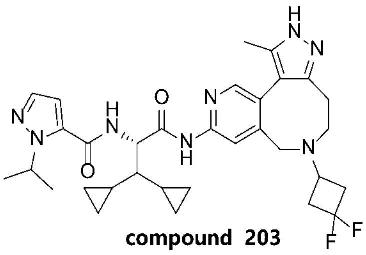 **compound 203** | MS (ESI) m/z =607 [M+1]$^+$ |
| 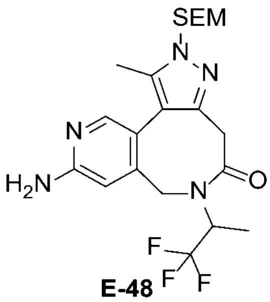 E-48 | 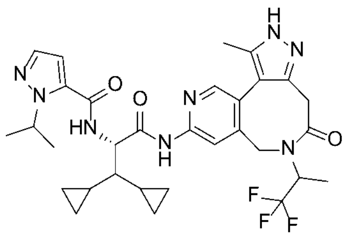 **compound 204 (racemic)** | MS (ESI) m/z =627 [M+1]$^+$ |
| 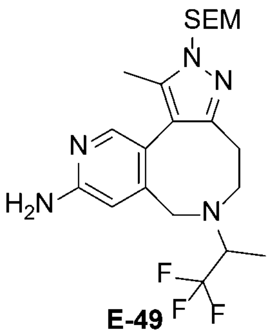 E-49 | 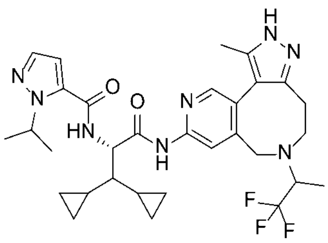 **compound 205 (racemic)** | MS (ESI) m/z =613 [M+1]$^+$ |

(continued)

| Macrocyclic amine | Structure of compound | 1HNMR and/or MS (ESI) |
|---|---|---|
| 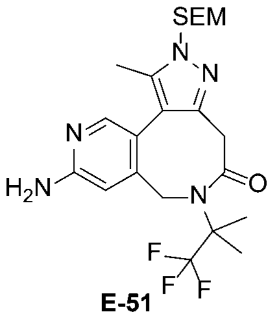 E-51 | 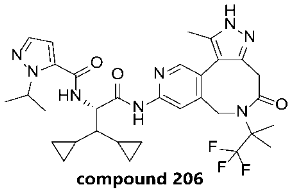 compound 206 | MS (ESI) m/z =641 [M+1]+ |
| 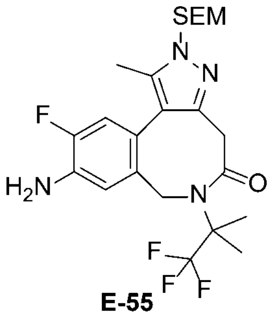 E-55 | 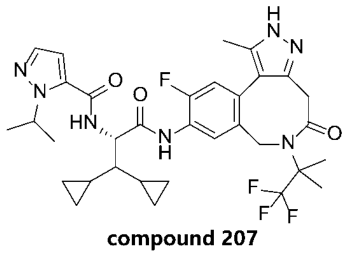 compound 207 | MS (ESI) m/z =658 [M+1]+ |
| 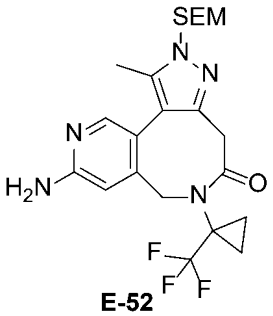 E-52 | 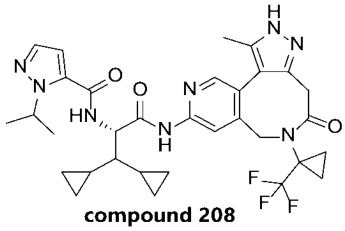 compound 208 | MS (ESI) m/z =639 [M+1]+ |
| 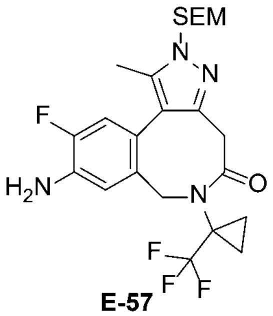 E-57 | 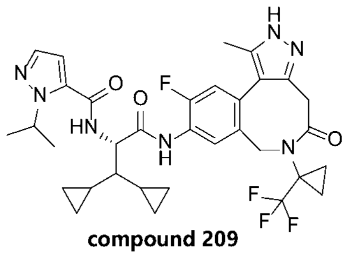 compound 209 | MS (ESI) m/z =656 [M+1]+ |
| 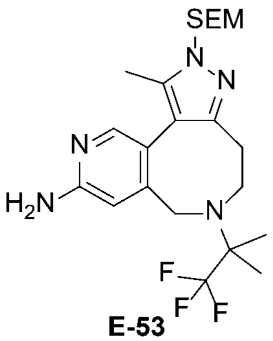 E-53 | 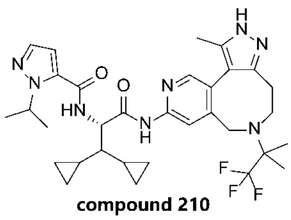 compound 210 | MS (ESI) m/z =627 [M+1]+ |

(continued)

| Macrocyclic amine | Structure of compound | $^1$HNMR and/or MS (ESI) |
|---|---|---|
| 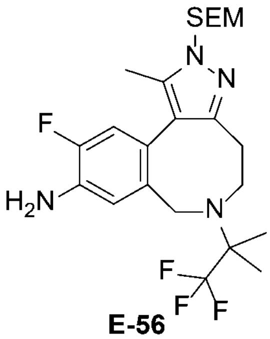 E-56 | 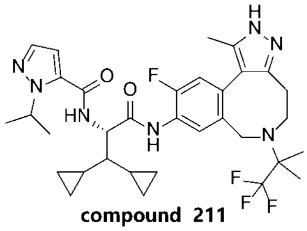 compound 211 | MS (ESI) m/z =644 [M+1]$^+$ |
| 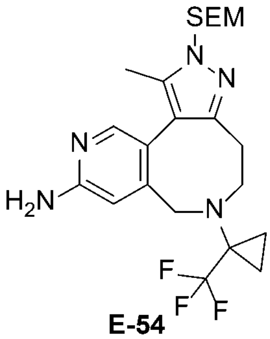 E-54 | 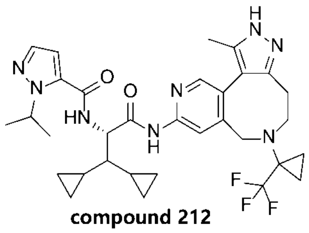 compound 212 | MS (ESI) m/z =625 [M+1]$^+$ |
| 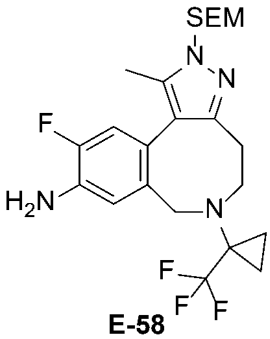 E-58 | 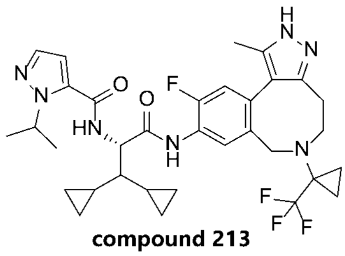 compound 213 | MS (ESI) m/z =642 [M+1]$^+$ |

**Examples 214-233 Preparation of compounds 214-233**

The preparation of compounds 214 to 233 was performed according to steps (S6-S9) of Example 12, in which intermediate 12-5 in step (S6) was correspondingly replaced with a macrocyclic amine from the following table, and 1-isopropylpyrazole-5-carboxylic acid in step (S8) was correspondingly replaced with a carboxylic acid from the following table, with all other reagents and procedures remaining identical.

| Macrocyclic amine + carboxylic acid | Structure of compound | ¹HNMR and/or MS (ESI) |
|---|---|---|
| 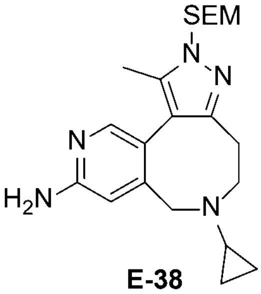 E-38 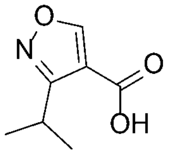 | 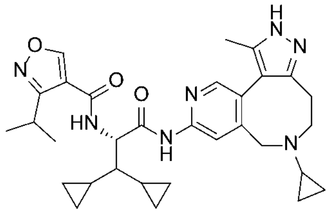 compound 214 | MS (ESI) m/z =558 $[M+1]^+$<br>$^1H$ NMR (600 MHz, Methanol-$d_4$) δ 9.11 (s, 1H), 8.48 (s, 1H), 8.44 (s, 1H), 4.94 (d, *J* = 7.0 Hz, 1H), 4.44 (d, *J* = 119.2 Hz, 2H), 3.55 (s, 2H), 3.48 (p, *J* = 6.9 Hz, 1H), 3.01 (q, *J* = 6.9 Hz, 2H), 2.33 (s, 3H), 1.31 (dd, *J* = 9.5, 6.9 Hz, 6H), 1.24 - 1.05 (m, 5H), 0.96 (td, *J* = 8.6, 4.1 Hz, 1H), 0.93 - 0.86 (m, 1H), 0.81 (td, *J* = 9.4, 7.0 Hz, 1H), 0.62 - 0.54 (m, 1H), 0.53 - 0.48 (m, 1H), 0.44 (t, *J* = 9.0 Hz, 1H), 0.37 (dt, *J* = 8.9, 4.4 Hz, 1H), 0.33 - 0.17 (m, 4H). |
| 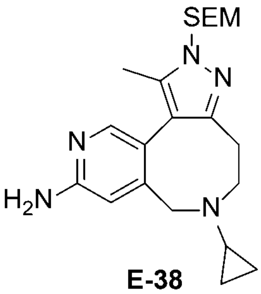 E-38 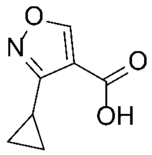 | 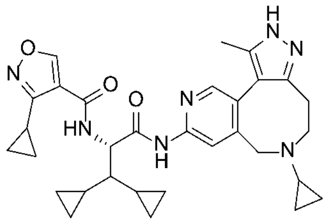 compound 215 | MS (ESI) m/z =556 $[M+1]^+$<br>$^1H$ NMR (600 MHz, Methanol-$d_4$) δ 9.10 (s, 1H), 8.48 (s, 1H), 8.44 (s, 1H), 4.98 (d, *J* = 6.9 Hz, 1H), 4.44 (d, *J* = 119.3 Hz, 2H), 3.54 (s, 2H), 3.01 (s, 2H), 2.38 (tt, *J* = 8.4, 5.1 Hz, 1H), 2.33 (s, 3H), 1.16 (dt, *J* = 18.8, 5.4 Hz, 4H), 1.09 - 0.86 (m, 7H), 0.82 (td, *J* = 9.4, 6.9 Hz, 1H), 0.57 (tq, *J* = 9.0, 4.3 Hz, 1H), 0.54 - 0.48 (m, 1H), 0.45 (t, *J* = 8.9 Hz, 1H), 0.38 (tt, *J* = 9.1, 4.6 Hz, 1H), 0.34 - 0.28 (m, 3H), 0.26 (dq, *J* = 9.7, 4.8, 4.0 Hz, 1H). |
| 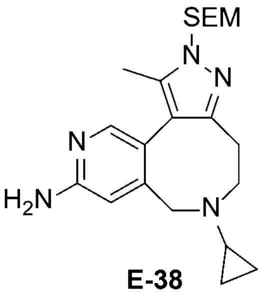 E-38 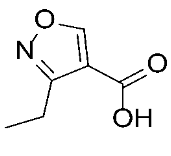 | 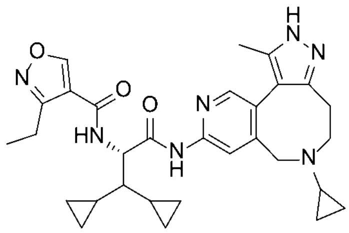 compound 216 | MS (ESI) m/z =544 $[M+1]^+$<br>$^1H$ NMR (600 MHz, Methanol-$d_4$) δ 9.15 (s, 1H), 8.48 (s, 1H), 8.44 (s, 1H), 4.94 (d, *J* = 7.0 Hz, 1H), 4.44 (d, *J* = 120.6 Hz, 2H), 3.55 (s, 2H), 3.11 - 2.83 (m, 4H), 2.33 (s, 3H), 1.26 (td, *J* = 7.6, 1.6 Hz, 3H), 1.20 - 1.11 (m, 4H), 0.96 (tq, *J* = 8.4, 5.1, 4.3 Hz, 1H), 0.89 (ddt, *J* = 13.3, 8.3, 4.2 Hz, 1H), 0.82 (td, *J* = 9.4, 6.9 Hz, 1H), 0.57 (tt, *J* = 8.8, 4.9 Hz, 1H), 0.51 (ddd, *J* = 10.0, 6.3, 2.5 Hz, 1H), 0.44 (t, *J* = 8.8 Hz, 1H), 0.41 - 0.19 (m, 6H). |

(continued)

| Macrocyclic amine + carboxylic acid | Structure of compound | $^1$HNMR and/or MS (ESI) |
|---|---|---|
| 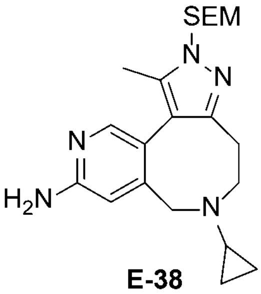 E-38 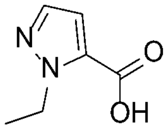 | 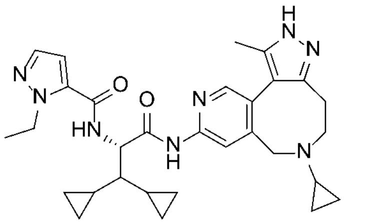 compound 217 | MS (ESI) m/z =543 [M+1]$^+$ $^1$H NMR (400 MHz, Methanol-$d_4$) δ 8.27 (d, *J* = 15.0 Hz, 2H), 7.51 (d, *J* = 2.1 Hz, 1H), 6.89 (d, *J* = 2.1 Hz, 1H), 4.97 (d, *J* = 6.7 Hz, 1H), 4.54 (q, *J* = 7.2 Hz, 2H), 3.71 (d, *J* = 60.5 Hz, 2H), 2.81 (s, 4H), 2.28 (s, 3H), 1.89 (tt, *J* = 6.2, 3.8 Hz, 1H), 1.38 (t, *J* = 7.2 Hz, 3H), 0.98 - 0.79 (m, 3H), 0.67 - 0.53 (m, 5H), 0.52 - 0.42 (m, 2H), 0.39 - 0.22 (m, 5H). |
| 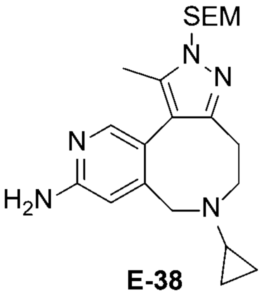 E-38 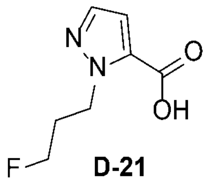 D-21 | 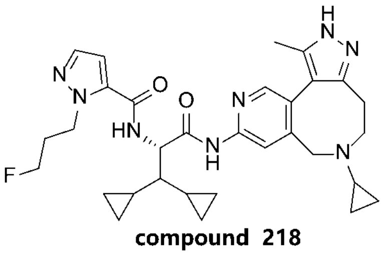 compound 218 | MS (ESI) m/z =575 [M+1]$^+$ $^1$H NMR (600 MHz, Methanol-$d_4$) δ 8.49 (s, 1H), 8.44 (s, 1H), 7.54 (d, *J* = 2.1 Hz, 1H), 6.91 (d, *J* = 2.1 Hz, 1H), 4.94 (d, *J* = 6.8 Hz, 1H), 4.67 - 4.35 (m, 6H), 3.54 (s, 2H), 3.02 (s, 2H), 2.33 (s, 3H), 2.19 (dp, *J* = 25.6, 6.3 Hz, 2H), 1.15 (s, 5H), 0.94 (dq, *J* = 8.3, 4.2, 3.2 Hz, 1H), 0.90 - 0.82 (m, 2H), 0.57 (tt, *J* = 8.9, 5.0 Hz, 1H), 0.51 (td, *J* = 8.9, 4.1 Hz, 1H), 0.45 (dt, *J* = 8.7, 4.6 Hz, 1H), 0.41 - 0.36 (m, 1H), 0.36 - 0.28 (m, 3H), 0.27 - 0.20 (m, 1H). |
| 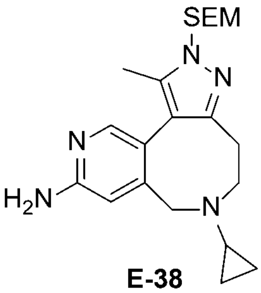 E-38 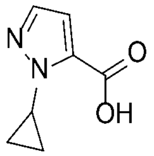 | 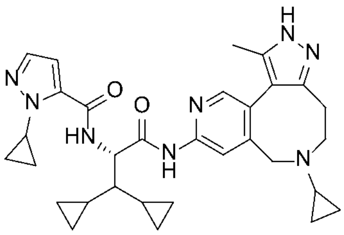 compound 219 | MS (ESI) m/z =555 [M+1]$^+$ $^1$H NMR (600 MHz, Methanol-$d_4$) δ 8.26 (s, 2H), 7.43 (d, *J* = 2.1 Hz, 1H), 6.81 (d, *J* = 2.0 Hz, 1H), 5.00 (d, *J* = 7.0 Hz, 1H), 4.16 (tt, *J* = 7.6, 3.9 Hz, 1H), 4.04 - 3.53 (m, 2H), 2.74 (d, *J* = 104.1 Hz, 4H), 2.28 (s, 3H), 1.89 (tt, *J* = 6.6, 3.9 Hz, 1H), 1.13 (dq, *J* = 3.9, 1.9 Hz, 2H), 1.04 - 0.97 (m, 2H), 0.97 - 0.86 (m, 2H), 0.83 (td, *J* = 9.3, 7.1 Hz, 1H), 0.67 - 0.60 (m, 2H), 0.60 - 0.54 (m, 3H), 0.53 - 0.48 (m, 1H), 0.48 - 0.42 (m, 1H), 0.40 - 0.28 (m, 4H), 0.25 (dq, *J* = 9.3, 4.9 Hz, 1H). |

(continued)

| Macrocyclic amine + carboxylic acid | Structure of compound | ¹HNMR and/or MS (ESI) |
|---|---|---|
| 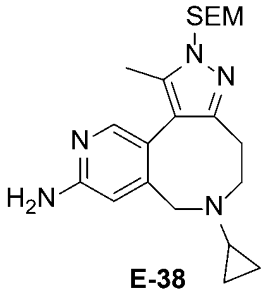 E-38 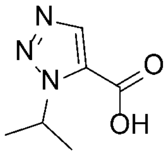 | 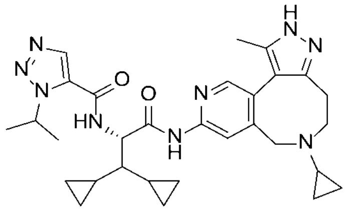 compound 220 | MS (ESI) m/z =558 [M+1]$^+$ |
| 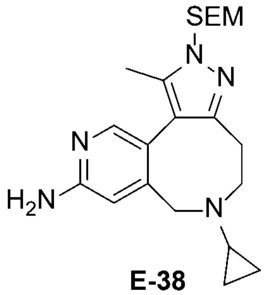 E-38 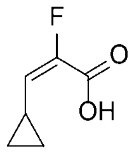 D-53 | 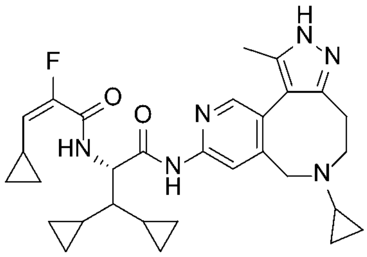 compound 221 | MS (ESI) m/z =533 [M+1]$^+$ |
| 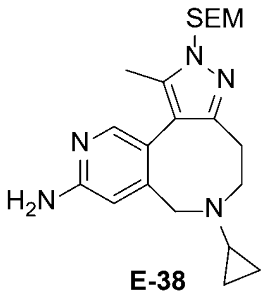 E-38 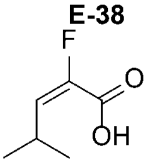 D-52 | 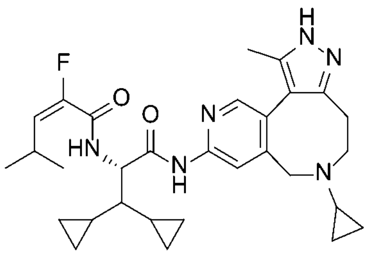 compound 222 | MS (ESI) m/z =535 [M+1]$^+$ |

(continued)

| Macrocyclic amine + carboxylic acid | Structure of compound | $^1$HNMR and/or MS (ESI) |
|---|---|---|
| 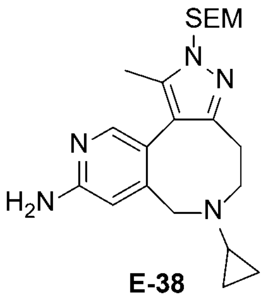 E-38 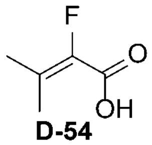 D-54 | 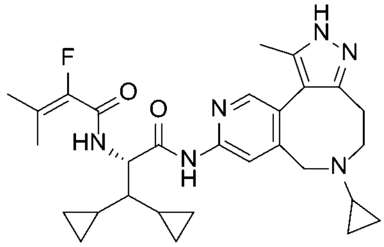 compound 223 | MS (ESI) m/z =521 [M+1]$^+$ |
| 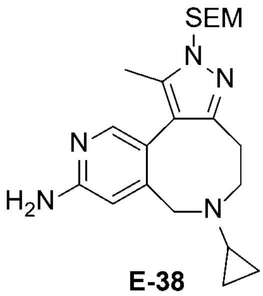 E-38 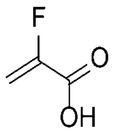 | 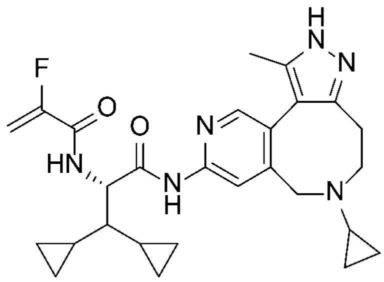 compound 224 | MS (ESI) m/z =493 [M+1]$^+$ |
| 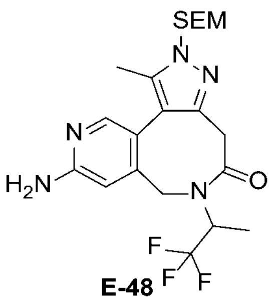 E-48 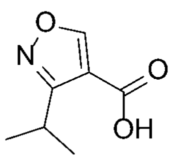 | 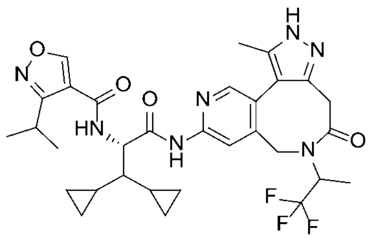 compound 225 (racemic) | MS (ESI) m/z =628 [M+1]$^+$ |

(continued)

| Macrocyclic amine + carboxylic acid | Structure of compound | $^1$HNMR and/or MS (ESI) |
|---|---|---|
| 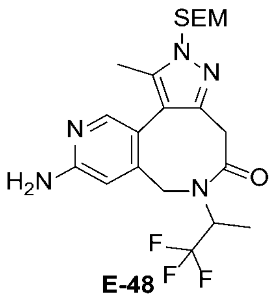 E-48 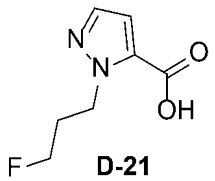 D-21 | 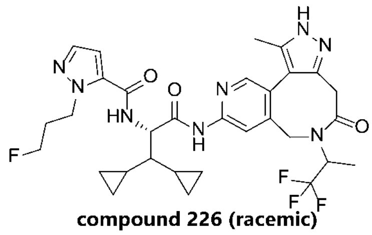 compound 226 (racemic) | MS (ESI) m/z =645 [M+1]$^+$ |
| 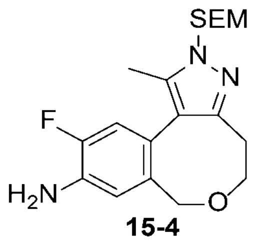 15-4 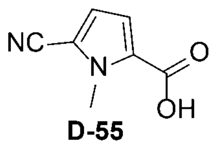 D-55 | 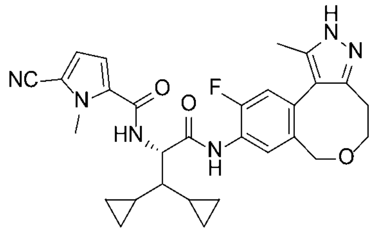 compound 227 | MS (ESI) m/z =531 [M+1]$^+$ $^1$H NMR (600 MHz, Methanol-$d_4$) δ 8.03 (d, *J* = 7.9 Hz, 1H), 7.19 (d, *J* = 11.3 Hz, 1H), 6.93 - 6.79 (m, 2H), 4.99 (d, *J* = 7.2 Hz, 1H), 4.22 (d, *J* = 111.0 Hz, 2H), 4.01 (s, 3H), 3.71 (s, 2H), 2.70 (s, 2H), 2.28 (s, 3H), 0.94 - 0.79 (m, 3H), 0.59 - 0.41 (m, 4H), 0.38 (dq, *J* = 9.8, 5.0 Hz, 1H), 0.28 (ddq, *J* = 18.5, 9.4, 4.7 Hz, 3H). |
| 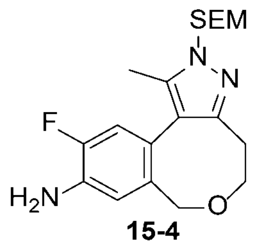 15-4 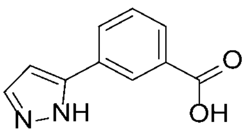 | 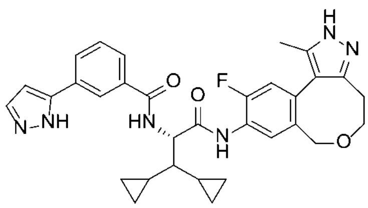 compound 228 | MS (ESI) m/z =569 [M+1]$^+$ $^1$H NMR (600 MHz, Methanol-$d_4$) δ 8.29 (d, *J* = 69.2 Hz, 1H), 8.07 (d, *J* = 7.9 Hz, 1H), 8.02 (d, *J* = 7.8 Hz, 1H), 7.85 (d, *J* = 7.8 Hz, 1H), 7.74 (s, 1H), 7.65 - 7.51 (m, 1H), 7.20 (s, 1H), 6.79 (s, 1H), 5.11 (d, *J* = 7.1 Hz, 1H), 4.33 (s, 2H), 3.73 (s, 2H), 2.72 (s, 2H), 2.29 (d, *J* = 24.2 Hz, 3H), 1.05 - 0.83 (m, 3H), 0.60 (tdd, *J* = 7.7, 4.5, 3.3 Hz, 1H), 0.57 - 0.46 (m, 3H), 0.43 (dq, *J* = 9.6, 4.7 Hz, 1H), 0.34 (ddt, *J* = 16.4, 8.7, 4.9 Hz, 3H). |

(continued)

| Macrocyclic amine + carboxylic acid | Structure of compound | $^1$HNMR and/or MS (ESI) |
|---|---|---|
| 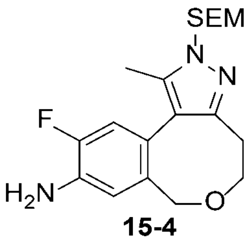 **15-4** 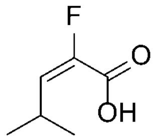 **D-52** | 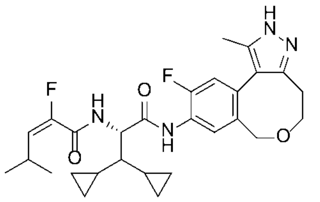 **compound 229** | MS (ESI) m/z =513 [M+1]$^+$ $^1$H NMR (600 MHz, Methanol-$d_4$) $\delta$ 8.03 (d, *J*= 7.9 Hz, 1H), 7.22 (d, *J* = 11.4 Hz, 1H), 5.66 (dd, *J* = 24.5, 10.4 Hz, 1H), 4.91 (dd, *J* = 6.7, 1.1 Hz, 1H), 4.58 - 4.06 (m, 2H), 3.83 (d, *J* = 112.9 Hz, 2H), 3.54 (tdd, *J* = 13.4, 6.7, 3.4 Hz, 1H), 2.79 (d, *J* = 78.0 Hz, 2H), 2.31 (s, 3H), 1.05 (ddd, *J* = 6.7, 3.9, 0.9 Hz, 6H), 0.83 (ddt, *J* = 12.3, 7.9, 4.3 Hz, 2H), 0.77 (td, *J* = 9.2, 6.8 Hz, 1H), 0.58 - 0.41 (m, 4H), 0.34 (dq, *J* = 9.5, 4.8 Hz, 1H), 0.32 - 0.27 (m, 2H), 0.24 (dq, *J* = 9.4, 4.8 Hz, 1H). |
| 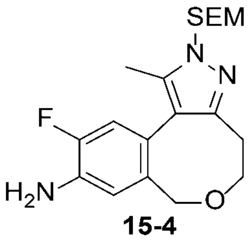 **15-4** 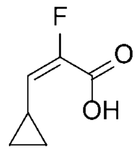 **D-53** | 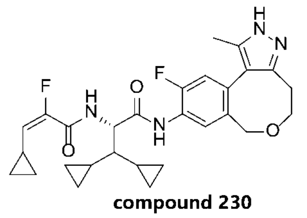 **compound 230** | MS (ESI) m/z =511 [M+1]$^+$ $^1$H NMR (600 MHz, Methanol-$d_4$) $\delta$ 8.05 (d, *J* = 7.9 Hz, 1H), 7.24 (d, *J* = 11.3 Hz, 1H), 5.30 (dd, *J* = 22.9, 10.9 Hz, 1H), 4.93 (dd, *J* = 6.7, 1.2 Hz, 1H), 4.10 (s, 2H), 3.74 (s, 2H), 2.85 - 2.55 (m, 3H), 2.33 (s, 3H), 0.91 (dt, *J* = 7.4, 3.7 Hz, 2H), 0.88 - 0.81 (m, 2H), 0.79 (td, *J* = 9.2, 6.7 Hz, 1H), 0.55 (ddq, *J* = 12.7, 8.6, 4.1 Hz, 1H), 0.50 (qt, *J* = 4.3, 2.6 Hz, 4H), 0.44 (tdd, *J* = 7.7, 5.2, 4.0 Hz, 1H), 0.38 - 0.33 (m, 1H), 0.33 - 0.27 (m, 2H), 0.27 - 0.22 (m, 1H). |
| 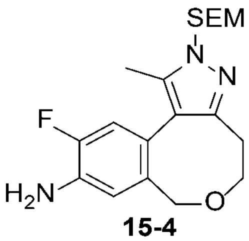 **15-4** 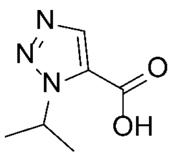 | 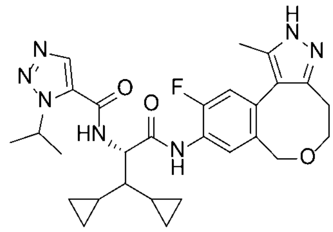 **compound 231** | MS (ESI) m/z =536 [M+1]$^+$ $^1$H NMR (600 MHz, Methanol-$d_4$) $\delta$ 8.19 (s, 1H), 8.03 (d, *J* = 7.9 Hz, 1H), 7.19 (s, 1H), 5.48 (p, *J* = 6.7 Hz, 1H), 5.04 (d, *J* = 7.5 Hz, 1H), 4.31 (s, 2H), 3.65 (d, *J*= 74.1 Hz, 2H), 2.70 (s, 2H), 2.29 (s, 3H), 1.61 (dd, *J* = 6.7, 1.4 Hz, 6H), 0.97 - 0.86 (m, 2H), 0.82 (td, *J* = 9.4, 7.5 Hz, 1H), 0.57 (tt, *J* = 8.3, 4.4 Hz, 1H), 0.51 (ddt, *J* = 10.9, 6.4, 3.4 Hz, 1H), 0.45 (qt, *J* = 9.6, 4.7 Hz, 2H), 0.37 (dq, *J* = 9.7, 4.9 Hz, 1H), 0.29 (ddp, *J* = 18.3, 9.3, 4.7 Hz, 3H). |

(continued)

| Macrocyclic amine + carboxylic acid | Structure of compound | $^1$HNMR and/or MS (ESI) |
|---|---|---|
| 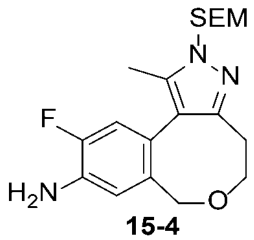 15-4 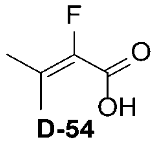 D-54 | 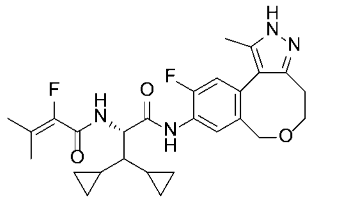 compound 232 | MS (ESI) m/z =499 [M+1]$^+$ $^1$H NMR (600 MHz, Methanol-$d_4$) δ 7.99 (d, *J* = 7.9 Hz, 1H), 7.18 (s, 1H), 4.88 (dd, *J* = 6.6, 1.2 Hz, 1H), 4.30 (s, 2H), 3.71 (s, 2H), 2.70 (s, 2H), 2.28 (s, 3H), 2.15 (d, *J*= 3.0 Hz, 3H), 1.87 (d, *J* = 4.0 Hz, 3H), 0.87 - 0.80 (m, 2H), 0.77 (td, *J* = 9.3, 6.6 Hz, 1H), 0.58 - 0.41 (m, 4H), 0.34 (dq, *J* = 9.5, 4.8 Hz, 1H), 0.29 (dq, *J* = 9.5, 4.8 Hz, 2H), 0.27 - 0.22 (m, 1H). |
| 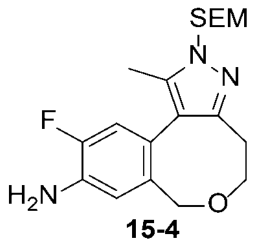 15-4 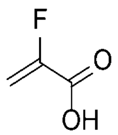 | 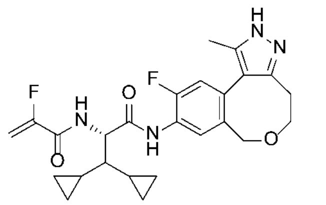 compound 233 | MS (ESI) m/z =471 [M+1]$^+$ $^1$H NMR (600 MHz, Methanol-$d_4$) δ 8.02 (d, *J* = 8.0 Hz, 1H), 7.20 (s, 1H), 5.67 (dd, *J* = 47.0, 3.5 Hz, 1H), 5.29 (dd, *J* = 15.3, 3.5 Hz, 1H), 4.96 (d, *J* = 6.8 Hz, 1H), 4.32 (s, 2H), 3.73 (s, 2H), 2.70 (d, *J* = 28.7 Hz, 2H), 2.30 (s, 3H), 0.88 - 0.77 (m, 3H), 0.61 - 0.42 (m, 4H), 0.37 (dq, *J* = 9.6, 4.9 Hz, 1H), 0.29 (dtd, *J* = 20.6, 9.1, 4.8 Hz, 3H). |

**Examples 234-245 Preparation of compounds 234-245**

The preparation of compounds 234 to 245 was performed according to the synthesis method in Example 12, in which 1-isopropylpyrazole-5-carboxylic acid in step (S8) was correspondingly replaced with a carboxylic acid from the following table, with all other reagents and procedures remaining identical.

| Carboxylic acid | Structure of compound | $^1$HNMR and/or MS (ESI) |
|---|---|---|
| 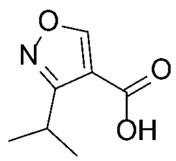 | 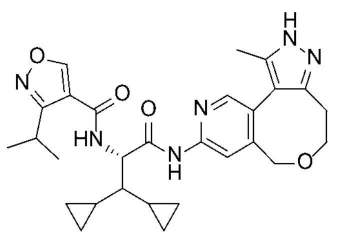 compound 234 | MS (ESI) m/z =519 [M+1]$^+$ $^1$H NMR (600 MHz, Methanol-$d_4$) δ 9.12 (s, 1H), 8.35 (s, 1H), 8.14 (s, 1H), 4.97 (d, *J* = 7.1 Hz, 1H), 4.35 (d, *J* = 7.0 Hz, 2H), 3.79 (s, 2H), 3.50 (p, *J* = 6.9 Hz, 1H), 2.75 (s, 2H), 2.33 (s, 3H), 1.33 (dd, *J* = 6.9, 5.5 Hz, 6H), 1.01 - 0.93 (m, 1H), 0.89 (tdd, *J* = 13.0, 6.4, 4.1 Hz, 1H), 0.83 (td, *J* = 9.4, 7.1 Hz, 1H), 0.58 (tq, *J* = 9.0, 4.2 Hz, 1H), 0.55 - 0.43 (m, 2H), 0.43 - 0.36 (m, 1H), 0.35 - 0.29 (m, 3H), 0.27 (dt, *J* = 9.4, 4.8 Hz, 1H). |
| 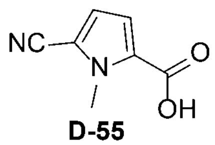 D-55 | 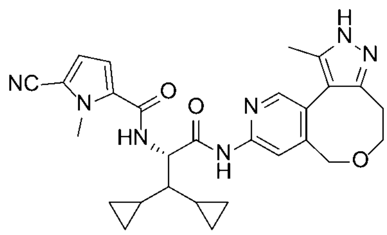 compound 235 | MS (ESI) m/z =514 [M+1]$^+$ $^1$H NMR (600 MHz, Methanol-$d_4$) δ 8.31 (s, 1H), 8.22 (s, 1H), 6.89 (d, *J* = 4.3 Hz, 1H), 6.85 (d, *J* = 4.3 Hz, 1H), 4.96 (d, *J* = 6.9 Hz, 1H), 4.46 (d, *J* = 143.1 Hz, 2H), 4.00 (s, 3H), 3.82 (d, *J*= 77.5 Hz, 2H), 2.71 (s, 2H), 2.30 (s, 3H), 0.96 - 0.80 (m, 3H), 0.60 - 0.42 (m, 3H), 0.40 - 0.21 (m, 5H). |

(continued)

| Carboxylic acid | Structure of compound | 1HNMR and/or MS (ESI) |
|---|---|---|
| 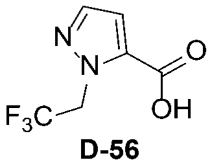<br>**D-56** | 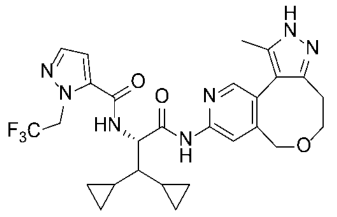<br>**compound 236** | MS (ESI) m/z =558 [M+1]+<br>$^1$H NMR (600 MHz, Methanol-$d_4$) δ 8.32 (s, 1H), 8.24 (s, 1H), 7.66 (d, *J* = 2.1 Hz, 1H), 7.07 (d, *J* = 2.1 Hz, 1H), 5.49 (dd, *J* = 15.0, 8.7 Hz, 1H), 5.43 - 5.30 (m, 1H), 5.03 (d, *J* = 6.6 Hz, 1H), 4.36 (s, 2H), 3.77 (s, 2H), 2.73 (s, 2H), 2.32 (s, 3H), 0.99 - 0.81 (m, 3H), 0.56 (dddd, *J* = 11.9, 8.7, 5.4, 4.0 Hz, 1H), 0.53 - 0.42 (m, 2H), 0.42 - 0.33 (m, 2H), 0.33 - 0.22 (m, 3H). |
| 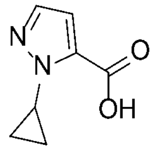 | 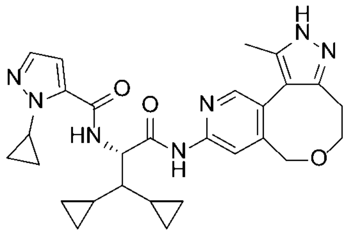<br>**compound 237** | MS (ESI) m/z =516 [M+1]+<br>$^1$H NMR (600 MHz, Methanol-$d_4$) δ 8.35 (d, *J* = 31.3 Hz, 1H), 8.25 (s, 1H), 7.45 (d, *J* = 2.0 Hz, 1H), 6.82 (d, *J* = 2.0 Hz, 1H), 5.03 (d, *J* = 7.0 Hz, 1H), 4.33 (s, 2H), 4.18 (tt, *J* = 7.5, 3.9 Hz, 1H), 3.77 (s, 2H), 2.72 (s, 2H), 2.33 (s, 3H), 1.15 (tt, *J* = 5.2, 2.5 Hz, 2H), 1.07 - 0.98 (m, 2H), 0.98 - 0.81 (m, 3H), 0.61 - 0.55 (m, 1H), 0.50 (dddt, *J* = 17.1, 10.4, 9.1, 4.3 Hz, 2H), 0.44 - 0.37 (m, 1H), 0.37 - 0.25 (m, 4H). |
| 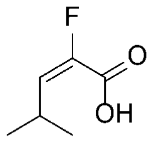<br>**D-52** | 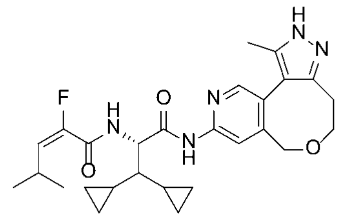<br>**compound 238** | MS (ESI) m/z =496 [M+1]+<br>$^1$H NMR (600 MHz, Methanol-$d_4$) δ 8.32 (s, 1H), 8.20 (s, 1H), 5.66 (dd, *J* = 24.5, 10.4 Hz, 1H), 4.87 (d, *J* = 7.3 Hz, 1H), 4.35 (s, 2H), 3.73 (d, *J* = 6.7 Hz, 2H), 3.53 (ddq, *J* = 13.9, 6.9, 4.1, 3.4 Hz, 1H), 2.71 (s, 2H), 2.29 (s, 3H), 1.04 (t, *J* = 6.7 Hz, 6H), 0.80 (dqd, *J* = 29.1, 9.3, 5.7 Hz, 3H), 0.57 - 0.42 (m, 3H), 0.38 (tt, *J* = 9.3, 4.8 Hz, 1H), 0.29 (ddq, *J* = 17.3, 9.3, 4.6 Hz, 3H), 0.22 (dq, *J* = 9.4, 4.8 Hz, 1H). |
| 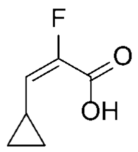<br>**D-53** | 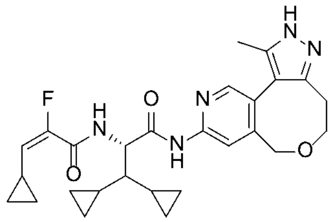<br>**compound 239** | MS (ESI) m/z =494 [M+1]+<br>$^1$H NMR (600 MHz, Methanol-$d_4$) δ 8.33 (s, 1H), 8.21 (s, 1H), 5.29 (dd, *J* = 22.9, 10.8 Hz, 1H), 4.90 (d, *J* = 6.6 Hz, 1H), 4.35 (s, 2H), 3.75 (s, 2H), 2.64 (dddd, *J* = 15.4, 8.1, 6.6, 4.0 Hz, 3H), 2.29 (s, 3H), 0.94 - 0.88 (m, 2H), 0.87 - 0.78 (m, 3H), 0.55 (tq, *J* = 9.5, 5.3, 4.8 Hz, 1H), 0.53 - 0.46 (m, 4H), 0.42 - 0.36 (m, 1H), 0.29 (tdd, *J* = 11.3, 9.1, 4.4 Hz, 3H), 0.23 (dq, *J* = 9.4, 4.8 Hz, 1H). |
| 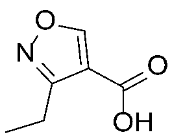 | 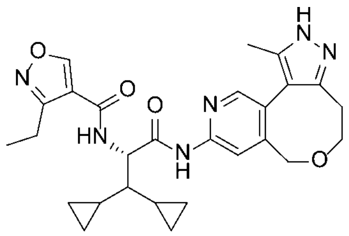<br>**compound 240** | MS (ESI) m/z =505 [M+1]+<br>$^1$H NMR (400 MHz, Methanol-$d_4$) δ 9.17 (d, *J* = 0.6 Hz, 1H), 8.33 (s, 1H), 8.24 (s, 1H), 4.99 (d, *J* = 6.9 Hz, 1H), 4.36 (s, 2H), 3.77 (s, 2H), 2.95 (q, *J* = 7.5 Hz, 2H), 2.72 (s, 2H), 2.31 (s, 3H), 1.29 (t, *J* = 7.5 Hz, 3H), 1.02 - 0.76 (m, 3H), 0.62 - 0.53 (m, 1H), 0.53 - 0.44 (m, 2H), 0.44 - 0.35 (m, 1H), 0.30 (dtd, *J* = 10.5, 5.2, 3.6 Hz, 4H). |

(continued)

| Carboxylic acid | Structure of compound | $^1$HNMR and/or MS (ESI) |
|---|---|---|
| 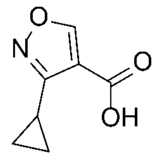 | 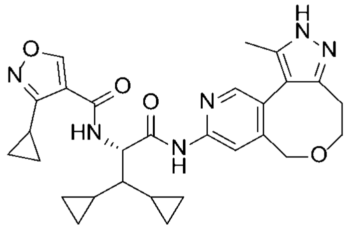 compound 241 | MS (ESI) m/z =517 [M+1]$^+$ $^1$H NMR (400 MHz, Methanol-$d_4$) δ 9.11 (s, 1H), 8.33 (s, 1H), 8.24 (s, 1H), 5.04 (d, *J* = 6.7 Hz, 1H), 4.36 (s, 2H), 3.77 (s, 2H), 2.72 (s, 2H), 2.44 - 2.36 (m, 1H), 2.31 (s, 3H), 1.13 - 1.05 (m, 2H), 1.05 - 0.99 (m, 2H), 0.99 - 0.87 (m, 2H), 0.82 (td, *J* = 9.3, 6.7 Hz, 1H), 0.62 - 0.53 (m, 1H), 0.53 - 0.44 (m, 2H), 0.40 (dddd, *J* = 10.2, 7.9, 4.2, 2.9 Hz, 1H), 0.36 - 0.23 (m, 4H). |
| 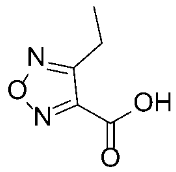 | 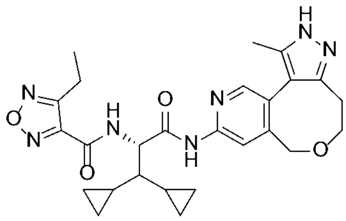 compound 242 | MS (ESI) m/z =506 [M+1]$^+$ $^1$H NMR (600 MHz, Methanol-$d_4$) δ 8.32 (s, 1H), 8.22 (s, 1H), 5.04 (d, *J* = 6.3 Hz, 1H), 4.34 (s, 2H), 3.70 (d, *J* = 56.7 Hz, 2H), 3.01 (qd,*J* = 7.5, 1.0 Hz, 2H), 2.78 (d, *J* = 90.6 Hz, 2H), 2.30 (s, 3H), 1.33 (t, *J* = 7.5 Hz, 3H), 0.95 - 0.82 (m, 3H), 0.59 - 0.54 (m, 1H), 0.53 - 0.49 (m, 1H), 0.45 (dddd, *J* = 8.9, 7.8, 5.3, 4.0 Hz, 1H), 0.39 (tdd, *J* = 7.3, 5.2, 3.8 Hz, 1H), 0.37 - 0.24 (m, 4H). |
| 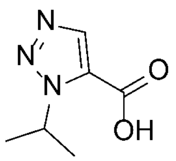 | 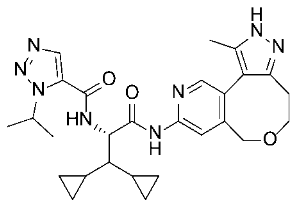 compound 243 | MS (ESI) m/z =519 [M+1]$^+$ $^1$H NMR (600 MHz, Methanol-$d_4$) δ 8.31 (s, 1H), 8.22 (s, 1H), 8.20 (s, 1H), 5.47 (p, *J* = 6.7 Hz, 1H), 5.01 (d, *J* = 7.3 Hz, 1H), 4.34 (s, 2H), 3.75 (s, 2H), 2.71 (s, 2H), 2.31 (s, 3H), 1.60 (dd, *J* = 6.7, 2.6 Hz, 6H), 0.98 - 0.86 (m, 2H), 0.83 (td, *J* = 9.3, 7.4 Hz, 1H), 0.56 (tq, *J* = 8.9, 4.2 Hz, 1H), 0.53 - 0.47 (m, 1H), 0.47 - 0.42 (m, 1H), 0.40 - 0.35 (m, 1H), 0.34 - 0.28 (m, 3H), 0.25 (dq, *J* = 9.4, 5.0 Hz, 1H). |
| 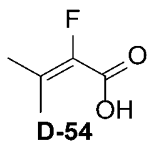 D-54 | 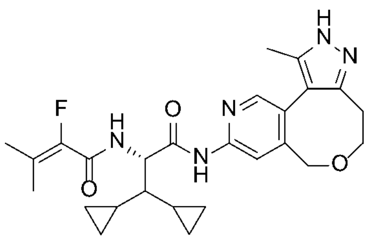 compound 244 | MS (ESI) m/z =482 [M+1]$^+$ 1H NMR (600 MHz, Methanol-$d_4$) δ 8.31 (s, 1H), 8.21 (s, 1H), 4.34 (s, 2H), 3.75 (s, 2H), 2.71 (s, 2H), 2.30 (s, 3H), 2.14 (d, *J* = 3.0 Hz, 3H), 1.87 (d, *J* = 4.0 Hz, 3H), 0.87 - 0.75 (m, 3H), 0.57 - 0.43 (m, 3H), 0.41 - 0.35 (m, 1H), 0.32 - 0.25 (m, 3H), 0.22 (dq, *J* = 9.4, 4.9 Hz, 1H). |
| 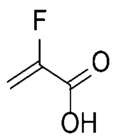 | 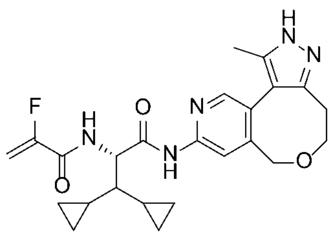 compound 245 | MS (ESI) m/z =454 [M+1]$^+$ $^1$H NMR (600 MHz, Methanol-$d_4$) δ 8.34 (s, 1H), 8.22 (s, 1H), 5.66 (dd, *J* = 47.0, 3.5 Hz, 1H), 5.29 (dd, *J* = 15.3, 3.5 Hz, 1H), 4.93 (d, *J* = 6.6 Hz, 1H), 4.39 (s, 2H), 3.77 (s, 2H), 2.74 (s, 2H), 2.31 (s, 3H), 0.83 (dddd, *J* = 17.6, 11.2, 9.0, 3.6 Hz, 3H), 0.59 - 0.44 (m, 3H), 0.42 - 0.36 (m, 1H), 0.36 - 0.27 (m, 3H), 0.24 (dt, *J* = 9.2, 4.7 Hz, 1H). |

## Example 246 Preparation of compound 246

A preparation of compound 246 is illustrated as follows:

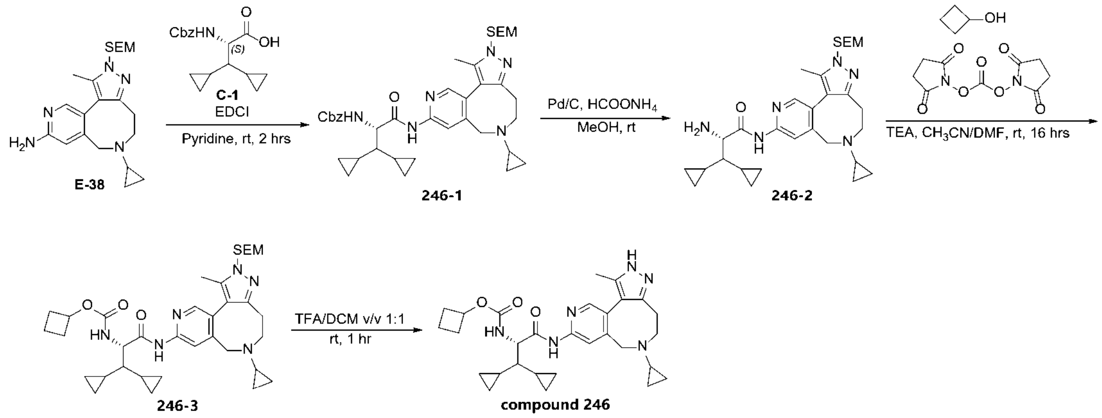


E-38, 246-1, 246-2, 246-3, compound 246

.

(S1-S2) Preparation of intermediate 246-2

The preparation of intermediate 246-2 was performed according to steps (S6-S7) of Example 12, in which intermediate 12-5 in step (S6) was replaced with intermediate E-38, with other reagents and procedures remaining identical. MS (ESI) m/z = 551 $[M+1]^+$.

(S3-S4) Preparation of compound 246

The preparation of compound 246 was performed according to the synthesis method in Example 32, in which cyclopropanol in step (S1) was replaced with cyclobutanol, and intermediate 15-6 was replaced with intermediate 246-2, with other reagents and procedures remaining identical. MS (ESI) m/z = 519 $[M+1]^+$. $^1$H NMR (600 MHz, Methanol-$d_4$) δ 8.45 (d, *J* = 34.0 Hz, 2H), 4.92 (d, *J* = 7.6 Hz, 2H), 4.65 - 4.14 (m, 3H), 3.55 (s, 2H), 3.17 - 2.84 (m, 2H), 2.33 (s, 5H), 2.11 (dq, *J* = 19.2, 9.4 Hz, 2H), 1.79 (q, *J* = 9.8, 8.9 Hz, 1H), 1.63 (dtd, *J* = 18.3, 10.3, 7.9 Hz, 1H), 1.17 (dt, *J* = 20.8, 4.5 Hz, 4H), 0.93 - 0.82 (m, 2H), 0.69 (td, *J* = 9.5, 6.0 Hz, 1H), 0.56 (tt, *J* = 9.0, 4.7 Hz, 1H), 0.49 (tt, *J* = 8.9, 4.7 Hz, 1H), 0.44 - 0.34 (m, 2H), 0.25 (dddt, *J* = 38.0, 14.0, 9.2, 4.7 Hz, 4H).

**Example 247 Preparation of compound 247**

A structure of compound 247 is as follows:

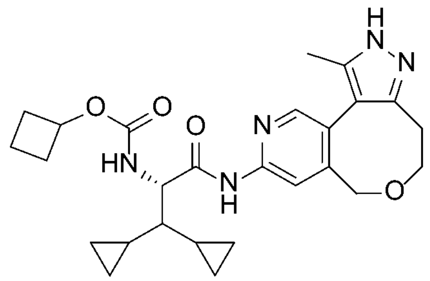

compound 247

.

The preparation of compound 247 was performed according to the synthesis method in Example 32, in which cyclopropanol in step (S1) was replaced with cyclobutanol, and intermediate 15-6 was replaced with intermediate 12-7, with other reagents and procedures remaining identical. MS (ESI) m/z = 480 $[M+1]^+$. $^1$H NMR (600 MHz, Methanol-$d_4$) δ 8.34 (s, 1H), 8.06 (s, 1H), 4.96 - 4.89 (m, 1H), 4.59 - 4.15 (m, 3H), 3.78 (s, 2H), 2.80 (d, *J* = 66.9 Hz, 2H), 2.32 (s, 5H), 2.11 (p, *J* = 9.8 Hz, 2H), 1.79 (q, *J* = 10.6 Hz, 1H), 1.63 (h, *J* = 9.8 Hz, 1H), 0.85 (dddd, *J* = 13.2, 9.9, 8.1, 4.9 Hz, 2H), 0.70 (q, *J* = 8.9 Hz, 1H), 0.57 (dq, *J* = 13.6, 8.6, 6.9 Hz, 1H), 0.49 (tt, *J* = 8.6, 4.6 Hz, 1H), 0.39 (dtd, *J* = 17.8, 8.7, 4.4 Hz, 2H), 0.30 (dq, *J* = 9.9, 5.0 Hz, 1H), 0.24 (dp, *J* = 14.4, 4.6 Hz, 3H).

**Examples 248-257 Preparation of compounds 248-257**

The preparation of compounds 248 to 257 was performed according to steps (S6-S9) of Example 12, in which

intermediate 12-5 in step (S6) was correspondingly replaced with a macrocyclic amine from the following table, and 1-isopropylpyrazole-5-carboxylic acid in step (S8) was correspondingly replaced with a carboxylic acid from the following table, with all other reagents and procedures remaining identical.

| Macrocyclic amine + carboxylic acid | Structure of compound | $^1$HNMR and/or MS (ESI) |
|---|---|---|
| 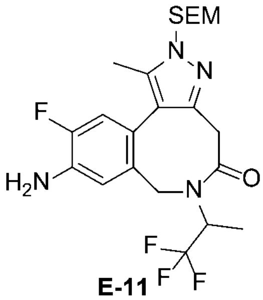 E-11 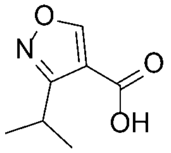 | 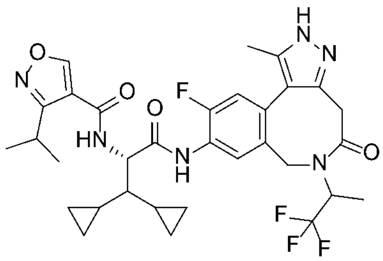 **Compound 248 (racemic)** | MS (ESI) m/z =645 $[M+1]^+$ |
| 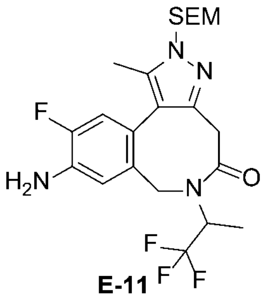 E-11 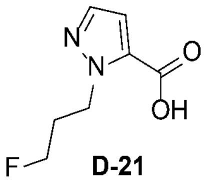 D-21 | 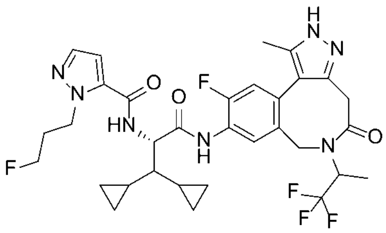 **Compound 249 (racemic)** | MS (ESI) m/z =662 $[M+1]^+$ |
| 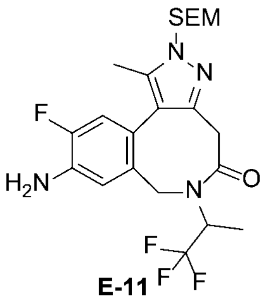 E-11 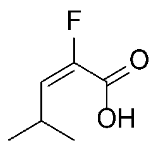 D-52 | 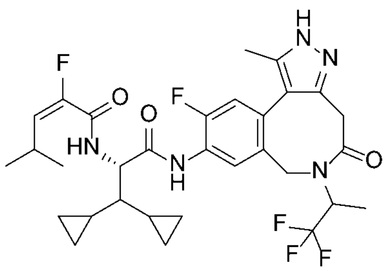 **Compound 250 (racemic)** | MS (ESI) m/z =622 $[M+1]^+$ |

(continued)

| Macrocyclic amine + carboxylic acid | Structure of compound | ¹HNMR and/or MS (ESI) |
|---|---|---|
| 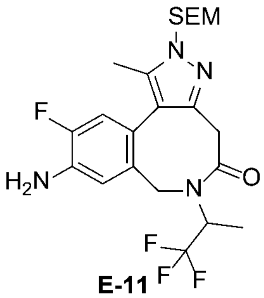 E-11 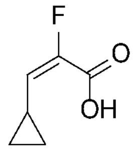 D-53 | 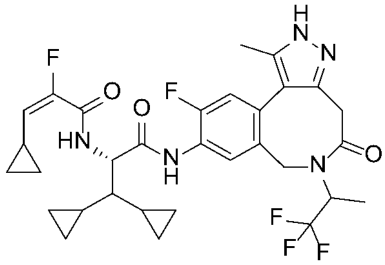 **Compound 251 (racemic)** | MS (ESI) m/z =620 $[M+1]^+$ |
| 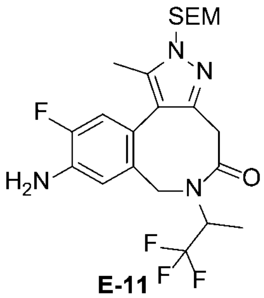 E-11 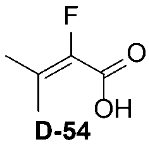 D-54 | 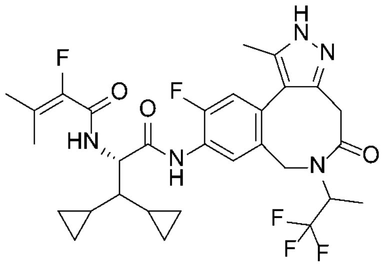 **Compound 252(racemic)** | MS (ESI) m/z =608 $[M+1]^+$ |
| 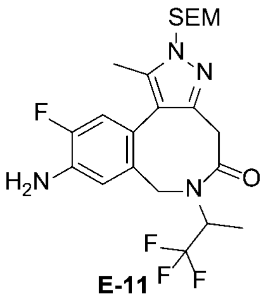 E-11 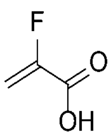 | 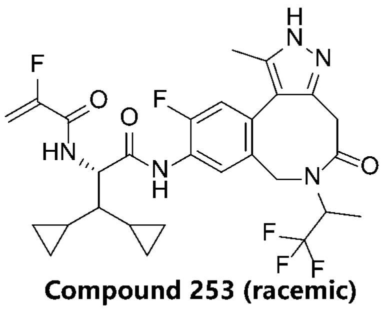 **Compound 253 (racemic)** | MS (ESI) m/z =580 $[M+1]^+$ |

(continued)

| Macrocyclic amine + carboxylic acid | Structure of compound | $^1$HNMR and/or MS (ESI) |
|---|---|---|
| 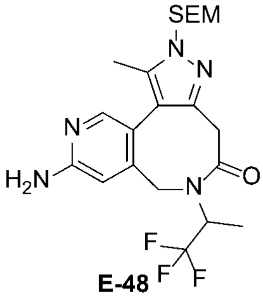 E-48 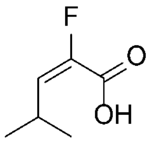 D-52 | 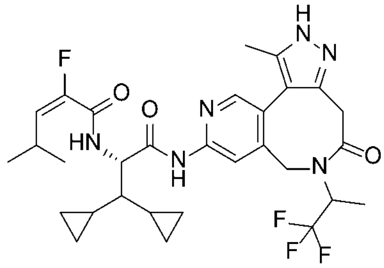 Compound 254 (racemic) | MS (ESI) m/z =605 [M+1]+ |
| 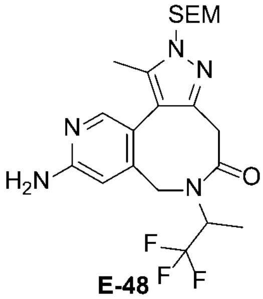 E-48 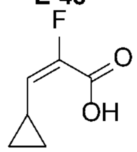 D-53 | 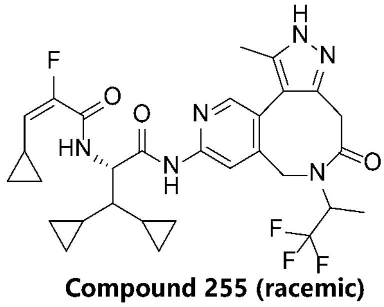 Compound 255 (racemic) | MS (ESI) m/z =603 [M+1]+ |
| 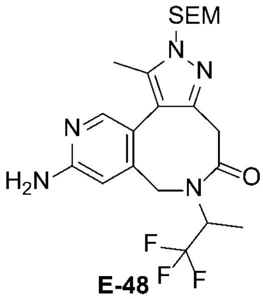 E-48 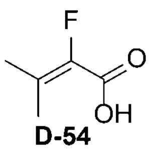 D-54 | 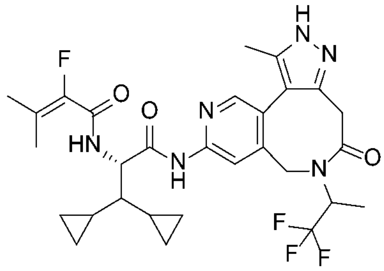 Compound 256 (racemic) | MS (ESI) m/z =591 [M+1]+ |

(continued)

| Macrocyclic amine + carboxylic acid | Structure of compound | $^1$HNMR and/or MS (ESI) |
|---|---|---|
| 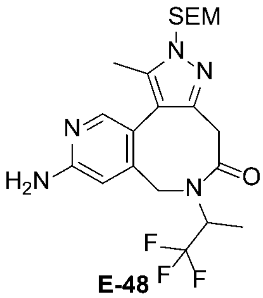 E-48 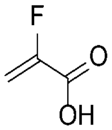 | 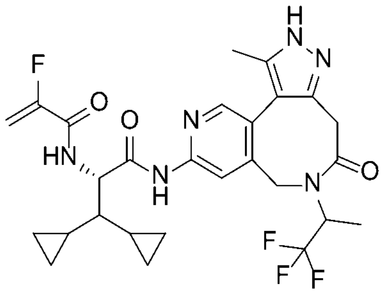 **Compound 257 (racemic)** | MS (ESI) m/z =563 [M+1]$^+$ |

In order to illustrate the beneficial effects of this closure, this disclosure had the following experiment examples.

**Experimental example 1 IL-17A enzyme-linked immunosorbent assay (ELISA)**

The inhibitory effects of human IL-17A inhibitors on receptor-ligand binding were quantitatively determined by a competitive enzyme-linked immunosorbent assay (ELISA). The ELISA was performed through the following steps.

(S1) Plate coating

Human IL-17A (Sino Biological Inc., Cat# 12047-H07B) was diluted with coating buffer (50 mM phosphate-buffered saline (PBS), pH 7.4) to give an IL-17A concentration of 0.2 μg/ml. 100 μL of the resulting solution was added to each well of a 96-well plate, and the plate was incubated at 37°C for 30 min. In a blank group, 100 100 μL of the coating buffer was added instead of the IL-17A solution.

(S2) Washing

The plate was washed four times with 10 mM phosphate-buffered saline containing 0.05% Tween-20 (PBST, pH 7.4). 200 μL of PBST was used per well for each wash. After the final wash, the plate was tapped dry on absorbent paper.

(S3) Blocking

Fresh 5% (w/v) skim milk solution was prepared before the experiment. For example, 5 g of skim milk powder (Bio-Rad, 170-6404) was completely dissolved in 100 mL of 10 mM PBST. 200 μL of the 5% (w/v) skim milk solution was added to each well, and the plate was incubated on a shaker at 25°C for 30 min.

(S4) Preparation of 100× to-be-tested compound solutions

During step (S3), the to-be-tested compound solutions were prepared, respectively. Each powdered compound was dissolved in dimethyl sulfoxide (DMSO) to reach a storage concentration of 10 mM. The to-be-tested compound solutions were then serially diluted four-fold with DMSO to obtain nine storage concentrations ranging from 0.061 μM to 4,000 μM.

(S5) Washing

Step (S2) was repeated.

(S6) Pre-incubation of IL-17A with to-be-tested compound solutions

89 μL of PBST and 1 μL of each of the to-be-tested compound solutions were added to each well and mixed. The

plate was pre-incubated at 25°C for 10 min. In the blank group and the control group, 1 μL of DMSO was added instead of the to-be-tested compound solutions.

(S7) Incubation with human interleukin-17 receptor a (IL-17Ra)

10 μL of 16 nM human IL-17Ra (Sino Biological Inc., Cat# 10895-H03H) was added, followed by incubation on a shaker at 25°C for 30 min. The receptor solution was prepared in 10 mM PBST.

(S8) Washing

Step (S2) was repeated.

(S9) Incubation with horseradish peroxidase-conjugated antibody

100 μL of mouse anti-Fc tag horseradish peroxidase (HRP)-conjugated antibody (Sino Biological Inc., Cat# 10702-MM01T-H) was added to each well. The plate was incubated on a shaker at 25°C for 30 min. The antibody was diluted with 5% (w/v) skim milk solution at 1:5,000 (v/v).

(S10) Washing

Step (S2) was repeated.

(S11) Color development

100 μL of 3,3',5,5'-tetramethylbenzidine (TMB) substrate solution (BD, Cat# 555214) was added to each well. Substrate A and substrate B were freshly mixed at a volume ratio of 1:1 before use. The plate was incubated in the dark at 25°C for 5-15 min. When the room temperature was relatively low, the incubation time was appropriately extended until an obvious color change was observed in the control group.

(S12) Termination and detection

The color reaction was terminated by addition of 2.5 M hydrochloric acid (HCl). Absorbance was measured at 450 nm using a BMG PHERStar microplate reader.

(S13) Calculation of half-maximal inhibitory concentration ($IC_{50}$)

The relative percent residual activity at each compound concentration was calculated according to Equation 1. The $IC_{50}$ value of each test compound was then calculated in GraphPad 6 using the equation for log(inhibitor) versus normalized response with variable slope, as shown in Equation 2.

relative percent residual activity (%) = [(absorbance of sample - absorbance of blank group) / (absorbance of control group - absorbance of blank group)] %. Equation 1:

$$\text{Equation 2: } Y = 100 / (1 + 10^\wedge((\text{LogIC}_{50} - X) \times \text{HillSlope})).$$

The compounds prepared in the above examples were tested for IL-17A inhibitory activity according to the above method. The results were shown in Table 1. The measured $IC_{50}$ values of the compounds were classified as follows:

"+" indicated that an $IC_{50}$ value was less than or equal to 1 μM and greater than 150 nM;
"++" indicated that an $IC_{50}$ value was less than or equal to 150 nM and greater than 50 nM; and
"+++" indicated that an $IC_{50}$ value was less than or equal to 50 nM.

Table 1 Inhibitory activity of compounds on human IL-17A

| Compound number | $IC_{50}$ | Compound number | $IC_{50}$ |
|---|---|---|---|
| compound 1 | +++ | compound 128 | +++ |
| compound 2 | +++ | compound 129 | +++ |
| compound 3 | +++ | compound 130 | +++ |
| compound 4 | +++ | compound 132 | +++ |
| compound 5 | +++ | compound 133 | +++ |
| compound 6 | +++ | compound 134 | ++ |
| compound 7 | ++ | compound 135 | +++ |
| compound 8 | ++ | compound 136 | +++ |
| compound 9 | +++ | compound 140 | +++ |
| compound 10 | +++ | compound 141 | +++ |
| compound 11a | ++ | compound 142 | +++ |
| compound 12 | +++ | compound 143 | +++ |
| compound 13 | ++ | compound 144 | +++ |
| compound 14 | ++ | compound 145 | +++ |
| compound 15 | +++ | compound 148 | +++ |
| compound 16 | +++ | compound 149 | +++ |
| compound 17 | +++ | compound 150 | +++ |
| compound 18 | ++ | compound 151 | +++ |
| compound 19 | +++ | compound 152 | +++ |
| compound 20 | +++ | compound 153 | +++ |
| compound 21 | +++ | compound 154 | +++ |
| compound 22 | +++ | compound 155 | ++ |
| compound 23 | +++ | compound 156 | +++ |
| compound 24 | ++ | compound 157 | +++ |
| compound 25 | ++ | compound 158 | +++ |
| compound 26 | ++ | compound 159 | +++ |
| compound 27 | +++ | compound 160 | +++ |
| compound 28 | +++ | compound 161 | +++ |
| compound 29 | ++ | compound 162 | ++ |
| compound 30 | ++ | compound 164 | +++ |
| compound 31 | + | compound 165 | +++ |
| compound 32 | +++ | compound 166 | +++ |
| compound 33 | +++ | compound 167 | +++ |
| compound 34 | +++ | compound 168 | +++ |
| compound 35 | +++ | compound 169 | +++ |
| compound 37a | +++ | compound 170 | +++ |
| compound 38a | +++ | compound 171 | +++ |
| compound 39a | ++ | compound 172 | +++ |
| compound 39b | ++ | compound 173 | +++ |

(continued)

| Compound number | $IC_{50}$ | Compound number | $IC_{50}$ |
|---|---|---|---|
| compound 40a | ++ | compound 174 | +++ |
| compound 40b | +++ | compound 175 | +++ |
| compound 41a | +++ | compound 176 | +++ |
| compound 41b | +++ | compound 177 | +++ |
| compound 42a | +++ | compound 178 | +++ |
| compound 42b | +++ | compound 179 | +++ |
| compound 43a | ++ | compound 180 | +++ |
| compound 43b | ++ | compound 181 | +++ |
| compound 44a | +++ | compound 182 | +++ |
| compound 45 | +++ | compound 186a | +++ |
| compound 46 | +++ | compound 186b | +++ |
| compound 47 | +++ | compound 188a | +++ |
| compound 48 | +++ | compound 188b | +++ |
| compound 49 | +++ | compound 189a | +++ |
| compound 50 | ++ | compound 189b | +++ |
| compound 51 | +++ | compound 190a | +++ |
| compound 52 | +++ | compound 190b | ++ |
| compound 53 | +++ | compound 191 | +++ |
| compound 54 | +++ | compound 192 | +++ |
| compound 55 | +++ | compound 193 | +++ |
| compound 56 | +++ | compound 194 | +++ |
| compound 57 | +++ | compound 195 | +++ |
| compound 58 | +++ | compound 196 | +++ |
| compound 59 | +++ | compound 197 | +++ |
| compound 60 | +++ | compound 198 | +++ |
| compound 61 | +++ | compound 199 | +++ |
| compound 62 | +++ | compound 200 | +++ |
| compound 63 | +++ | compound 201 | +++ |
| compound 64 | +++ | compound 202 | +++ |
| compound 65 | +++ | compound 203 | +++ |
| compound 72 | +++ | compound 204 | +++ |
| compound 73 | ++ | compound 205 | +++ |
| compound 74 | ++ | compound 206 | +++ |
| compound 76 | +++ | compound 207 | +++ |
| compound 77 | ++ | compound 208 | +++ |
| compound 78 | +++ | compound 209 | +++ |
| compound 79 | ++ | compound 210 | +++ |
| compound 81 | +++ | compound 211 | +++ |
| compound 82 | ++ | compound 212 | +++ |

(continued)

| Compound number | $IC_{50}$ | Compound number | $IC_{50}$ |
|---|---|---|---|
| compound 83 | ++ | compound 213 | +++ |
| compound 84 | +++ | compound 214 | +++ |
| compound 85 | ++ | compound 215 | +++ |
| compound 86 | +++ | compound 216 | +++ |
| compound 87 | +++ | compound 217 | +++ |
| compound 88 | ++ | compound 218 | +++ |
| compound 89 | +++ | compound 219 | +++ |
| compound 90 | +++ | compound 220 | +++ |
| compound 91 | ++ | compound 221 | +++ |
| compound 92 | +++ | compound 222 | +++ |
| compound 93 | ++ | compound 223 | +++ |
| compound 94 | +++ | compound 224 | +++ |
| compound 95 | +++ | compound 225 | +++ |
| compound 97 | ++ | compound 226 | +++ |
| compound 98 | +++ | compound 227 | +++ |
| compound 99 | ++ | compound 228 | +++ |
| compound 100 | +++ | compound 229 | +++ |
| compound 101 | +++ | compound 230 | +++ |
| compound 102 | +++ | compound 231 | +++ |
| compound 103 | ++ | compound 232 | +++ |
| compound 104 | +++ | compound 233 | +++ |
| compound 107 | +++ | compound 234 | +++ |
| compound 108 | +++ | compound 235 | +++ |
| compound 109 | ++ | compound 236 | +++ |
| compound 112 | +++ | compound 237 | +++ |
| compound 113 | +++ | compound 238 | +++ |
| compound 114 | +++ | compound 239 | +++ |
| compound 115 | +++ | compound 240 | +++ |
| compound 116 | +++ | compound 241 | +++ |
| compound 117 | +++ | compound 243 | +++ |
| compound 118a | +++ | compound 244 | +++ |
| compound 118b | +++ | compound 245 | +++ |
| compound 119a | +++ | compound 246 | +++ |
| compound 119b | +++ | compound 247 | ++ |
| compound 120a | ++ | compound 248 | +++ |
| compound 120b | +++ | compound 249 | +++ |
| compound 121 | ++ | compound 250 | +++ |
| compound 122 | ++ | compound 251 | +++ |
| compound 123a | +++ | compound 252 | +++ |

(continued)

| Compound number | $IC_{50}$ | Compound number | $IC_{50}$ |
|---|---|---|---|
| compound 123b | +++ | compound 253 | +++ |
| compound 124 | +++ | compound 254 | +++ |
| compound 125 | +++ | compound 255 | +++ |
| compound 256 | +++ | compound 257 | +++ |

**Experiment example 2 IL-17AF ELISA**

The inhibitory effects of human IL-17AF inhibitors on receptor-ligand binding were quantitatively determined by a competitive ELISA.

The ELISA was performed through the following steps.

(S1) Plate coating

Human IL-17AF (Sino Biological Inc., Cat# CT047-H08H) was diluted with coating buffer (50 mM PBS, pH 7.4) to reach a concentration of 0.625 μg/ml. 100 μL of the resulting solution was added to each well of a 96-well plate, and the plate was incubated at 37°C for 30 min. In the blank group, 100 μL of coating buffer was added instead of the IL-17AF solution.

(S2) Washing

The plate was washed four times with 10 mM PBST (pH 7.4). 200 μL was used per well for each wash. After the final wash, the plate was tapped dry on absorbent paper.

(S3) Blocking

Fresh 5% (w/v) skim milk solution was prepared before the experiment. For example, 5 grams of skim milk powder (Bio-Rad, Cat# 170-6404) was completely dissolved in 100 mL of 10 mM PBST. 200 μL of the 5% (w/v) skim milk solution was added to each well, and the plate was incubated on a shaker at 25°C for 30 min.

(S4) Preparation of 100× to-be-tested compound solutions

During step (S3), the to-be-tested compound solutions were prepared. Each powdered compound was dissolved in dimethyl sulfoxide (DMSO) to reach a stock concentration of 10 mM. The to-be-tested compound solutions were then serially diluted four-fold with DMSO to obtain nine concentrations ranging from 0.061 μM to 4,000 μM.

(S5) Washing

Step (S2) was repeated.

(S6) Pre-incubation of IL-17AF with to-be-tested compound solutions

89 μL of PBST and 1 μL of each of the to-be-tested compound solutions were added to each well and mixed. The plate was pre-incubated at 25°C for 10 min. In the blank group and the control group, 1 μL of DMSO was added instead of the to-be-tested compound solutions.

(S7) Incubation with human IL-17Ra

10 μL of 100 nM IL-17Ra (Sino Biological Inc., Cat# 10895-H03H) was added to each well, followed by incubation on a shaker at 25°C for 30 min. The receptor solution was prepared in 10 mM PBST.

(S8) Washing

Step (S2) was repeated.

(S9) Incubation with horseradish peroxidase-conjugated antibody

100 μL of mouse anti-Fc tag HRP-conjugated antibody (Sino Biological Inc., Cat# 10702-MM01T-H) was added to each well. The plate was incubated on a shaker at 25°C for 30 min. The antibody was diluted with 5% (w/v) skim milk solution at 1:5,000 (v/v).

(S10) Washing

Step (S2) was repeated.

(S11) Color development

100 μL of TMB substrate solution (BD, Cat# 555214) was added to each well. Substrate A and substrate B were freshly mixed at a volume ratio of 1:1 before use. The plate was incubated in the dark at 25°C for 5-15 min. When the room temperature was relatively low, the incubation time was appropriately extended until an obvious color change was observed in the control group.

(S12) Termination and detection

The color reaction was terminated by addition of 2.5 M HCl. Absorbance was measured at 450 nm using a BMG PHERStar microplate reader.

(S13) Calculation of half-maximal inhibitory concentration ($IC_{50}$)

The relative percent residual activity at each compound concentration was calculated according to Equation 1. The $IC_{50}$ value of each test compound was then calculated in GraphPad 6 using the equation for log(inhibitor) versus normalized response with variable slope, as shown in Equation 2.

relative percent residual activity (%) = [(absorbance of sample - absorbance of blank group) / (absorbance of control group - absorbance of blank group)] %. Equation 1:

$$\text{Equation 2: } Y = 100 / (1 + 10\text{^}((\text{Log}IC_{50} - X) \times \text{HillSlope})).$$

The compounds prepared in the Examples were tested for IL-17AF inhibitory activity according to the above method. The results were shown in Table 2. The measured $IC_{50}$ values of the compounds were classified as follows:

"+" indicated that an $IC_{50}$ value was less than or equal to 1 μM and greater than 500 nM;
"++" indicated that the $IC_{50}$ value was less than or equal to 500 nM and greater than 100 nM; and
"+++" indicated that the $IC_{50}$ value was less than or equal to 100 nM.

Table 2 Inhibitory activity of compounds on human IL-17AF

| Compound number | $IC_{50}$ | Compound number | $IC_{50}$ |
|---|---|---|---|
| compound 1 | +++ | compound 133 | ++ |
| compound 2 | +++ | compound 134 | ++ |
| compound 3 | +++ | compound 136 | +++ |
| compound 6 | ++ | compound 137 | +++ |
| compound 7 | ++ | compound 138 | +++ |
| compound 8 | ++ | compound 139 | ++ |
| compound 9 | ++ | compound 142 | +++ |
| compound 10 | ++ | compound 143 | +++ |

(continued)

| Compound number | $IC_{50}$ | Compound number | $IC_{50}$ |
|---|---|---|---|
| compound 12 | +++ | compound 144 | ++ |
| compound 15 | +++ | compound 145 | +++ |
| compound 16 | ++ | compound 148 | ++ |
| compound 17 | ++ | compound 149 | +++ |
| compound 19 | ++ | compound 150 | +++ |
| compound 22 | +++ | compound 151 | +++ |
| compound 23 | +++ | compound 152 | ++ |
| compound 24 | ++ | compound 153 | +++ |
| compound 25 | ++ | compound 154 | ++ |
| compound 26 | ++ | compound 156 | ++ |
| compound 27 | ++ | compound 158 | ++ |
| compound 28 | +++ | compound 159 | +++ |
| compound 33 | +++ | compound 160 | ++ |
| compound 34 | +++ | compound 161 | +++ |
| compound 35 | ++ | compound 162 | ++ |
| compound 39a | ++ | compound 164 | ++ |
| compound 39a | ++ | compound 165 | ++ |
| compound 41a | ++ | compound 166 | ++ |
| compound 41b | +++ | compound 167 | ++ |
| compound 42a | +++ | compound 168 | ++ |
| compound 42b | +++ | compound 169 | +++ |
| compound 46 | +++ | compound 170 | +++ |
| compound 48 | ++ | compound 173 | ++ |
| compound 52 | ++ | compound 174 | ++ |
| compound 54 | +++ | compound 176 | +++ |
| compound 57 | ++ | compound 182 | ++ |
| compound 58 | +++ | compound 184 | ++ |
| compound 59 | ++ | compound 185 | ++ |
| compound 60 | +++ | compound 186a | ++ |
| compound 61 | +++ | compound 186b | ++ |
| compound 62 | +++ | compound 188a | ++ |
| compound 65 | +++ | compound 188b | ++ |
| compound 76 | +++ | compound 189a | ++ |
| compound 81 | +++ | compound 189b | ++ |
| compound 82 | ++ | compound 191 | +++ |
| compound 84 | ++ | compound 192 | +++ |
| compound 86 | +++ | compound 193 | +++ |
| compound 87 | ++ | compound 194 | ++ |
| compound 88 | ++ | compound 195 | ++ |

(continued)

| Compound number | $IC_{50}$ | Compound number | $IC_{50}$ |
|---|---|---|---|
| compound 89 | ++ | compound 196 | ++ |
| compound 90 | ++ | compound 198 | ++ |
| compound 94 | ++ | compound 199 | +++ |
| compound 97 | ++ | compound 200 | ++ |
| compound 98 | ++ | compound 201 | ++ |
| compound 99 | ++ | compound 202 | ++ |
| compound 100 | +++ | compound 214 | +++ |
| compound 101 | +++ | compound 215 | +++ |
| compound 102 | ++ | compound 216 | ++ |
| compound 104 | ++ | compound 217 | +++ |
| compound 108 | ++ | compound 218 | +++ |
| compound 113 | ++ | compound 227 | ++ |
| compound 114 | +++ | compound 229 | ++ |
| compound 115 | ++ | compound 230 | ++ |
| compound 116 | ++ | compound 234 | ++ |
| compound 117 | ++ | compound 235 | ++ |
| compound 118a | ++ | compound 236 | ++ |
| compound 118b | ++ | compound 237 | ++ |
| compound 119a | ++ | compound 238 | ++ |
| compound 119b | ++ | compound 239 | ++ |
| compound 124 | ++ | compound 240 | ++ |
| compound 125 | ++ | compound 241 | ++ |
| compound 132 | ++ | compound 246 | ++ |

**Experiment example 3 Inhibition of human IL-17A protein-induced GROα/CXCL1 production in HT-29 cells by compounds**

This assay was conducted to evaluate the ability of the compounds to inhibit human IL-17A protein-induced production of the chemokine growth-regulated oncogene alpha/C-X-C motif chemokine ligand 1 (GROα/CXCL1) in HT-29 cells.

Human colorectal adenocarcinoma epithelial HT-29 cells (Chengdu Zhongyuan Gongchuang Technology Co., Ltd.) were seeded into a 96-well plate at a density of $5 \times 10^4$ cells per well and were cultured overnight in an incubator at 37°C.

30ng/mL of human IL-17A protein (R&D, #317-ILB) was mixed with gradient concentrations of the IL-17A small-molecule inhibitor, or with 0.3 μg/mL of a positive control IL-17A antibody (R&D, #AF-317-NA). These mixtures were incubated at 37°C for 1 h and were then added to the plate. The mixtures were co-incubated with the HT-29 cells at 37°C for 48 h. Thereafter, the level of GROα in the cell culture supernatant was determined using a GROα ELISA kit (Cisbio, #62HCXC1PEG).

According to the methods of Experiment examples 1-2, the compounds prepared in the above Examples were tested for inhibition of human IL-17A protein-induced production of GROα/CXCL1 in HT-29 cells. The results were shown in Table 3. The inhibitory activity of each compound was expressed as the half-maximal inhibitory concentration ($IC_{50}$) for inhibition of GROα/CXCL1 production in HT-29 cells. The measured $IC_{50}$ values of the compounds were classified as follows:

"+" indicated that an $IC_{50}$ value was less than or equal to 1 μM and greater than 500 nM;
"++" indicated that the $IC_{50}$ value was less than or equal to 500 nM and greater than 100 nM; and

"+++" indicated that the $IC_{50}$ value was less than or equal to 100 nM.

Table 3. Inhibitory $IC_{50}$ of compounds on HT-29 cell GROα/CXCL1

| Compound number | $IC_{50}$ | Compound number | $IC_{50}$ |
|---|---|---|---|
| compound 1 | +++ | compound 150 | +++ |
| compound 2 | ++ | compound 151 | +++ |
| compound 3 | +++ | compound 152 | +++ |
| compound 6 | ++ | compound 153 | +++ |
| compound 12 | +++ | compound 156 | ++ |
| compound 15 | +++ | compound 157 | ++ |
| compound 16 | ++ | compound 158 | +++ |
| compound 17 | ++ | compound 159 | +++ |
| compound 22 | +++ | compound 160 | +++ |
| compound 23 | +++ | compound 161 | +++ |
| compound 27 | ++ | compound 164 | +++ |
| compound 28 | ++ | compound 165 | +++ |
| compound 40a | ++ | compound 166 | ++ |
| compound 40b | ++ | compound 167 | +++ |
| compound 41a | +++ | compound 168 | +++ |
| compound 41b | +++ | compound 169 | +++ |
| compound 42a | +++ | compound 170 | +++ |
| compound 42b | ++ | compound 171 | +++ |
| compound 45 | ++ | compound 172 | +++ |
| compound 46 | ++ | compound 173 | +++ |
| compound 48 | ++ | compound 174 | +++ |
| compound 49 | ++ | compound 175 | +++ |
| compound 52 | +++ | compound 176 | +++ |
| compound 53 | ++ | compound 178 | +++ |
| compound 54 | +++ | compound 180 | +++ |
| compound 58 | ++ | compound 181 | +++ |
| compound 59 | ++ | compound 182 | ++ |
| compound 60 | ++ | compound 183 | ++ |
| compound 61 | ++ | compound 186a | +++ |
| compound 62 | ++ | compound 186b | +++ |
| compound 64 | ++ | compound 187a | +++ |
| compound 65 | ++ | compound 187b | +++ |
| compound 76 | ++ | compound 188a | ++ |
| compound 79 | ++ | compound 188b | +++ |
| compound 81 | ++ | compound 189a | +++ |
| compound 84 | +++ | compound 189b | ++ |
| compound 86 | +++ | compound 190a | ++ |

(continued)

| Compound number | $IC_{50}$ | Compound number | $IC_{50}$ |
|---|---|---|---|
| compound 87 | ++ | compound 190b | ++ |
| compound 88 | +++ | compound 191 | +++ |
| compound 89 | +++ | compound 192 | +++ |
| compound 90 | ++ | compound 193 | +++ |
| compound 92 | +++ | compound 194 | +++ |
| compound 98 | ++ | compound 195 | +++ |
| compound 99 | ++ | compound 196 | ++ |
| compound 100 | ++ | compound 197 | +++ |
| compound 101 | +++ | compound 198 | +++ |
| compound 102 | +++ | compound 199 | +++ |
| compound 104 | +++ | compound 200 | ++ |
| compound 108 | ++ | compound 201 | +++ |
| compound 113 | ++ | compound 202 | +++ |
| compound 114 | ++ | compound 214 | +++ |
| compound 115 | ++ | compound 215 | +++ |
| compound 118a | ++ | compound 216 | +++ |
| compound 120a | ++ | compound 217 | +++ |
| compound 120b | ++ | compound 218 | +++ |
| compound 123a | ++ | compound 219 | +++ |
| compound 123b | ++ | compound 229 | +++ |
| compound 124 | +++ | compound 230 | +++ |
| compound 125 | +++ | compound 232 | +++ |
| compound 130 | ++ | compound 233 | +++ |
| compound 132 | +++ | compound 234 | ++ |
| compound 133 | ++ | compound 235 | ++ |
| compound 136 | +++ | compound 236 | ++ |
| compound 137 | ++ | compound 237 | ++ |
| compound 138 | ++ | compound 238 | +++ |
| compound 142 | +++ | compound 239 | +++ |
| compound 143 | +++ | compound 240 | ++ |
| compound 144 | +++ | compound 241 | ++ |
| compound 145 | +++ | compound 244 | +++ |
| compound 148 | +++ | compound 245 | +++ |
| compound 149 | +++ | compound 246 | ++ |

It was shown that these compounds provided by the present disclosure had good inhibitory activity against human IL-17A and could be effectively used in the treatment of diseases with abnormal IL-17A activity.

**Claims**

1. A compound of formula (I), or a deuterated compound, a stereoisomer or a pharmaceutically acceptable salt thereof:

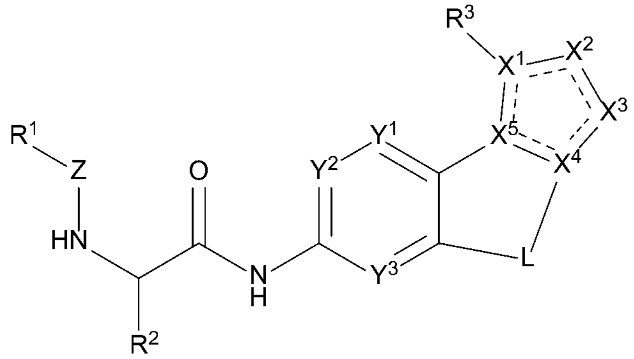

(I),

**characterized in that** --- represents a single bond or a double bond;
$X^1$ is C or N;
$X^2$ and $X^3$ are independently selected from the group consisting of O, N, $NR^X$ and $CR^X$;
$X^4$ and $X^5$ are each independently C or N;
each $R^X$ is independently selected from the group consisting of hydrogen, deuterium, halogen, cyano, $-C_{1-6}$ alkyl, $-OC_{1-6}$ alkyl, $-C_{0-2}$ alkylene-(3 to 10-membered cycloalkyl), -O(3 to 10-membered cycloalkyl), $-C_{0-2}$ alkylene-(4 to 10-membered heterocycloalkyl), -O(4 to 10-membered heterocycloalkyl), deuterium-substituted $-C_{1-6}$ alkyl and halogen-substituted $-C_{1-6}$ alkyl;
$Y^1$, $Y^2$ and $Y^3$ are each independently N or $CR^Y$;
each $R^Y$ is independently selected from the group consisting of hydrogen, deuterium, halogen, cyano, $-C_{1-6}$ alkyl, deuterium-substituted $-C_{1-6}$ alkyl, halogen-substituted $-C_{1-6}$ alkyl, $-C_{0-2}$ alkylene-(3 to 10-membered cycloalkyl), $-C_{0-2}$ alkylene-(4 to 10-membered heterocycloalkyl), $-C_{0-2}$ alkylene-$OC_{1-6}$ alkyl, $-C_{0-2}$ alkylene-O(halogen-substituted $-C_{1-6}$ alkyl), $-C_{0-2}$ alkylene-O(3 to 10-membered cycloalkyl) and $-C_{0-2}$ alkylene-O(4 to 10-membered heterocycloalkyl);
Z is C(O), S(O) or $S(O)_2$;
$R^1$ is selected from the group consisting of $-C_{1-6}$ alkyl, deuterium-substituted $-C_{1-6}$ alkyl, halogen-substituted $-C_{1-6}$ alkyl, $-C_{2-6}$ alkenyl, halogen-substituted $-C_{2-6}$ alkenyl, $-C_{2-6}$ alkynyl, halogen-substituted $-C_{2-6}$ alkynyl, $-C_{0-2}$ alkylene-(3 to 10-membered cycloalkyl), $-C_{0-2}$ alkylene-(4 to 10-membered heterocycloalkyl), $-C_{0-2}$ alkylene-(5 to 10-membered aromatic ring), $-C_{0-2}$ alkylene-(5 to 10-membered heteroaromatic ring), - $C_{0-2}$ alkylene-$OR^{11}$ and $-C_{0-2}$ alkylene-$NR^{11}R^{12}$, wherein alkylene, alkyl, alkenyl, alkynyl, cycloalkyl, heterocycloalkyl, aromatic ring and heteroaromatic ring are unsubstituted or substituted by one, two or three $R^{1a}$;
$R^{11}$ and $R^{12}$ are independently selected from the group consisting of hydrogen, deuterium, $-C_{1-6}$ alkyl, deuterium-substituted $-C_{1-6}$ alkyl, halogen-substituted $-C_{1-6}$ alkyl, $-C_{0-2}$ alkylene-(3 to 10-membered cycloalkyl), $-C_{0-2}$ alkylene-(4 to 10-membered heterocycloalkyl), $-C_{0-2}$ alkylene-(5 to 10-membered aromatic ring) and $-C_{0-2}$ alkylene-(5 to 10-membered heteroaromatic ring), wherein alkyl, alkylene, cycloalkyl, heterocycloalkyl, aromatic ring and heteroaromatic ring are unsubstituted or substituted by one, two or three $R^{1a}$;
each $R^{1a}$ is independently selected from the group consisting of hydrogen, deuterium, halogen, cyano, $-C_{1-6}$ alkyl, deuterium-substituted $-C_{1-6}$ alkyl, halogen-substituted $-C_{1-6}$ alkyl, $-C_{0-4}$ alkylene-$OR^{1b}$, $-C_{0-4}$ alkylene-$SR^{1b}$, $-C_{0-2}$ alkylene-$C(O)R^{1b}$, $-C_{0-2}$ alkylene-$C(O)NR^{1b}R^{1c}$, $-C_{0-4}$ alkylene-$NR^{1b}R^{1c}$, $-C_{0-2}$ alkylene-$NR^{1b}C(O)R^{1c}$, $-C_{0-4}$ alkylene-$S(O)_2R^{1b}$, $-C_{0-4}$ alkylene-$NHS(O)_2R^{1b}$, $-C_{0-4}$ alkyleneS(O)$R^{1b}$, $-C_{0-4}$ alkylene-(3 to 10-membered cycloalkyl), $-C_{0-4}$ alkylene-(4 to 10-membered heterocycloalkyl), $-C_{0-4}$ alkylene-(5 to 10-membered aromatic ring) and $-C_{0-4}$ alkylene-(5 to 10-membered heteroaromatic ring), wherein alkyl, alkylene, cycloalkyl, heterocycloalkyl, aromatic ring and heteroaromatic ring are unsubstituted or substituted by one, two or three $R^{1d}$; or two $R^{1a}$ linked to the same carbon atom form 3 to 10-membered cycloalkyl or 4 to 10-membered heterocycloalkyl with the carbon atom, wherein cycloalkyl and heterocycloalkyl are unsubstituted or substituted by one, two or three $R^{1e}$;
$R^{1b}$, $R^{1c}$ and $R^{1d}$ are independently selected from the group consisting of hydrogen, deuterium, $-C_{1-6}$ alkyl, deuterium-substituted $-C_{1-6}$ alkyl, halogen-substituted $-C_{1-6}$ alkyl, halogen, cyano, =O, =S, nitro, -OH, -SH, $-S(C_{1-6}$ alkyl), $-C_{0-2}$ alkylene-$O(C_{1-6}$ alkyl), $-C(O)NH_2$, $-NH_2$, $-NH(C_{1-6}$ alkyl), $-N(C_{1-6}$ alkyl)$(C_{1-6}$ alkyl), $-C_{0-2}$

alkylene-(3 to 10-membered cycloalkyl), -$C_{0-2}$ alkylene-(4 to 10-membered heterocycloalkyl), -$C_{0-2}$ alkylene-(5 to 10-membered aromatic ring) and -$C_{0-2}$ alkylene-(5 to 10-membered heteroaromatic ring);

each $R^{1e}$ is independently selected from the group consisting of hydrogen, deuterium, -$C_{1-6}$ alkyl, deuterium-substituted -$C_{1-6}$ alkyl, halogen-substituted -$C_{1-6}$ alkyl, halogen, cyano, =O, =S, nitro, -OH, -SH, -S($C_{1-6}$ alkyl), -$C_{0-2}$ alkylene-O($C_{1-6}$ alkyl), -C(O)$NH_2$, -$NH_2$, -NH($C_{1-6}$ alkyl), -N($C_{1-6}$ alkyl)($C_{1-6}$ alkyl), -$C_{0-2}$ alkylene-(3 to 10-membered cycloalkyl), -$C_{0-2}$ alkylene-(4 to 10-membered heterocycloalkyl), -$C_{0-2}$ alkylene-(6 to 10-membered aromatic ring) and -$C_{0-2}$ alkylene-(5 to 10-membered heteroaromatic ring);

$R^2$ is selected from the group consisting of -$C_{0-2}$ alkylene-(3 to 10-membered cycloalkyl), -$C_{0-2}$ alkylene-(4 to 10-membered heterocycloalkyl), -$C_{0-2}$ alkylene-(5 to 12-membered bridged cycloalkyl), -$C_{0-2}$ alkylene-(5 to 12-membered bridged heterocycloalkyl), -$C_{0-2}$ alkylene-(5 to 12-membered spiro cycloalkyl), -$C_{0-2}$ alkylene-(5 to 12-membered spiro heterocycloalkyl), -$C_{0-2}$ alkylene-(5 to 10-membered aromatic ring), -$C_{0-2}$ alkylene-(5 to 10-membered heteroaromatic ring), -$C_{0-2}$ alkylene-$OR^{21}$, -$C_{0-2}$ alkylene-$NR^{21}R^{22}$ and -$C_{0-2}$ alkylene-CN, wherein alkylene, cycloalkyl, heterocycloalkyl, bridged cycloalkyl, bridged heterocycloalkyl, spiro cycloalkyl, spiro heterocycloalkyl, aromatic ring and heteroaromatic ring are unsubstituted or substituted by one, two, three or four $R^{2a}$;

$R^{21}$ and $R^{22}$ are independently selected from the group consisting of hydrogen, deuterium, -$C_{1-6}$ alkyl, deuterium-substituted -$C_{1-6}$ alkyl, halogen-substituted -$C_{1-6}$ alkyl, -$C_{0-2}$ alkylene-(3 to 10-membered cycloalkyl), -$C_{0-2}$ alkylene-(4 to 10-membered heterocycloalkyl), -$C_{0-2}$ alkylene-(5 to 10-membered aromatic ring) and -$C_{0-2}$ alkylene-(5 to 10-membered heteroaromatic ring), wherein alkyl, alkylene, cycloalkyl, heterocycloalkyl, aromatic ring and heteroaromatic ring are unsubstituted or substituted by one, two or three $R^{2a}$; and

each $R^{2a}$ is independently selected from the group consisting of hydrogen, deuterium, halogen, cyano, =O, =S, nitro, -$C_{1-6}$ alkyl, deuterium-substituted -$C_{1-6}$ alkyl, halogen-substituted -$C_{1-6}$ alkyl, -$C_{0-2}$ alkylene-$OR^{2b}$, -$C_{0-2}$ alkylene-C(O)$R^{2b}$, -$C_{0-2}$ alkylene-C(O)$NR^{2b}R^{2c}$, -$C_{0-2}$ alkylene-$NR^{2b}R^{2c}$, -$C_{0-2}$ alkylene-$NR^{2b}$C(O)$R^{2c}$, -$C_{0-4}$ alkylene-S(O)$_2R^{2b}$, -$C_{0-4}$ alkylene-CN, -$C_{0-2}$ alkylene-(3 to 10-membered cycloalkyl), - $C_{0-2}$ alkylene-(4 to 10-membered heterocycloalkyl), -$C_{0-2}$ alkylene-(5 to 12-membered bridged cycloalkyl), -$C_{0-2}$ alkylene-(5 to 12-membered bridged heterocycloalkyl), -$C_{0-2}$ alkylene-(5 to 12-membered spiro cycloalkyl), -$C_{0-2}$ alkylene-(5 to 12-membered spiro heterocycloalkyl), -$C_{0-2}$ alkylene-(5 to 10-membered aromatic ring) and -$C_{0-2}$ alkylene-(5 to 10-membered heteroaromatic ring), wherein alkyl, alkylene, cycloalkyl, heterocycloalkyl, bridged cycloalkyl, bridged heterocycloalkyl, spiro cycloalkyl, spiro heterocycloalkyl, aromatic ring and heteroaromatic ring are unsubstituted or substituted by one, two or three $R^{2d}$; or two $R^{2a}$ linked to the same carbon atom form 3 to 10-membered cycloalkyl or 4 to 10-membered heterocycloalkyl with the carbon atom;

$R^{2b}$ and $R^{2C}$ are independently selected from the group consisting of hydrogen, deuterium, -$C_{1-6}$ alkyl, deuterium-substituted -$C_{1-6}$ alkyl, halogen-substituted -$C_{1-6}$ alkyl, -$C_{0-2}$ alkylene-(3 to 10-membered cycloalkyl), -$C_{0-2}$ alkylene-(4 to 10-membered heterocycloalkyl), -$C_{0-2}$ alkylene-(5 to 10-membered aromatic ring) and -$C_{0-2}$ alkylene-(5 to 10-membered heteroaromatic ring), wherein alkyl, alkylene, cycloalkyl, heterocycloalkyl, aromatic ring and heteroaromatic ring are unsubstituted or substituted by one, two or three $R^{2d}$;

each $R^{2d}$ is independently selected from the group consisting of hydrogen, deuterium, -$C_{1-6}$ alkyl, deuterium-substituted -$C_{1-6}$ alkyl, halogen-substituted -$C_{1-6}$ alkyl, halogen, cyano, =O, =S, nitro, -OH, -O($C_{1-6}$ alkyl), -$NH_2$, -NH($C_{1-6}$ alkyl) and - N($C_{1-6}$ alkyl)($C_{1-6}$ alkyl);

$R^3$ is selected from the group consisting of hydrogen, deuterium, halogen, cyano, -$C_{1-6}$ alkyl, deuterium-substituted -$C_{1-6}$ alkyl, halogen-substituted -$C_{1-6}$ alkyl, -$C_{0-2}$ alkylene-(3 to 10-membered cycloalkyl), -$C_{0-2}$ alkylene-(4 to 10-membered heterocycloalkyl), -$C_{0-2}$ alkylene-(5 to 10-membered aromatic ring), -$C_{0-2}$ alkylene-(5 to 10-membered heteroaromatic ring), -$C_{0-2}$ alkylene-$OR^{31}$ and -$C_{0-2}$ alkylene-$NR^{31}R^{32}$, wherein alkylene, cycloalkyl, heterocycloalkyl, aromatic ring and heteroaromatic ring are unsubstituted or substituted by one, two or three $R^{3a}$;

$R^{31}$ and $R^{32}$ are independently selected from the group consisting of hydrogen, deuterium, -$C_{1-6}$ alkyl, deuterium-substituted -$C_{1-6}$ alkyl, halogen-substituted -$C_{1-6}$ alkyl, -$C_{0-2}$ alkylene-(3 to 10-membered cycloalkyl), -$C_{0-2}$ alkylene-(4 to 10-membered heterocycloalkyl), -$C_{0-2}$ alkylene-(5 to 10-membered aromatic ring) and -$C_{0-2}$ alkylene-(5 to 10-membered heteroaromatic ring);

each $R^{3a}$ is independently selected from the group consisting of hydrogen, deuterium, halogen, cyano, =O, =S, nitro, -$C_{1-6}$ alkyl, deuterium-substituted -$C_{1-6}$ alkyl and halogen-substituted -$C_{1-6}$ alkyl;

L is -$C_{2-6}$ alkylene or -$C_{2-6}$ alkenylene, wherein one, two or three methylene groups in -$C_{2-6}$ alkylene and -$C_{2-6}$ alkenylene are unsubstituted or substituted by O, $NR^{L1}$, C(O), S(O) or S(O)$_2$, and/or -$C_{2-6}$ alkylene and -$C_{2-6}$ alkenylene are unsubstituted or substituted by one, two, three or four $R^L$;

each $R^{L1}$ is selected from the group consisting of hydrogen, deuterium, -$C_{1-6}$ alkyl, deuterium-substituted -$C_{1-6}$ alkyl, halogen-substituted -$C_{1-6}$ alkyl, -$C_{0-2}$ alkylene-O$C_{1-6}$ alkyl, -$C_{0-2}$ alkylene-CN, -$C_{0-2}$ alkylene-C(O)($C_{1-6}$ alkyl), -$C_{0-2}$ alkylene-O(3 to 10-membered cycloalkyl), -$C_{0-2}$ alkylene-O(4 to 10-membered heterocycloalkyl), -$C_{0-2}$ alkylene-(3 to 10-membered cycloalkyl), -$C_{0-2}$ alkylene-(3 to 10-membered heterocycloalkyl), -$C_{0-2}$

alkylene-(5 to 10-membered aromatic ring), $-C_{0-2}$ alkylene-(5 to 10-membered heteroaromatic ring) and $-C_{0-4}$ alkylene-N($C_{1-6}$ alkyl)($C_{1-6}$ alkyl), wherein alkyl, alkylene, cycloalkyl, heterocycloalkyl, aromatic ring and heteroaromatic ring are unsubstituted or substituted by one, two or three $R^{L2}$;
each $R^{L2}$ is independently selected from the group consisting of hydrogen, deuterium, halogen, cyano, =O, =S, nitro, $-C_{1-6}$ alkyl, deuterium-substituted $-C_{1-6}$ alkyl and halogen-substituted $-C_{1-6}$ alkyl; and
each $R^L$ is independently selected from the group consisting of hydrogen, deuterium, halogen, cyano, =O, =S, nitro, $-C_{1-6}$ alkyl, deuterium-substituted $-C_{1-6}$ alkyl, halogen-substituted $-C_{1-6}$ alkyl, $-C_{0-2}$ alkylene-$OC_{1-6}$ alkyl, $-C_{0-2}$ alkylene-O(3 to 10-membered cycloalkyl) and $-C_{0-2}$ alkylene-O(4 to 10-membered heterocycloalkyl); or two $R^L$ linked to the same carbon atom form 3 to 10-membered cycloalkyl or 4 to 10-membered heterocycloalkyl with the carbon atom.

2. The compound according to claim 1, **characterized in that** $X^1$ is C, $X^2$ is $NR^X$, $X^3$ is N, $X^4$ is C and $X^5$ is C;

$X^1$ is C, $X^2$ is N, $X^3$ is N, $X^4$ is C and $X^5$ is N;
$X^1$ is C, $X^2$ is N, $X^3$ is $CR^X$, $X^4$ is N and $X^5$ is C;
$X^1$ is N, $X^2$ is N, $X^3$ is $CR^X$, $X^4$ is C and $X^5$ is C; or
$X^1$ is C, $X^2$ is O, $X^3$ is N, $X^4$ is C and $X^5$ is C.

3. The compound according to any one of claims 1-2, **characterized in that** the compound is selected from the group consisting of:

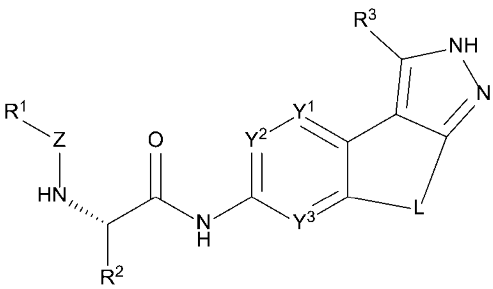
,
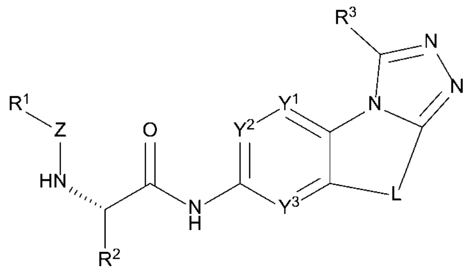
,
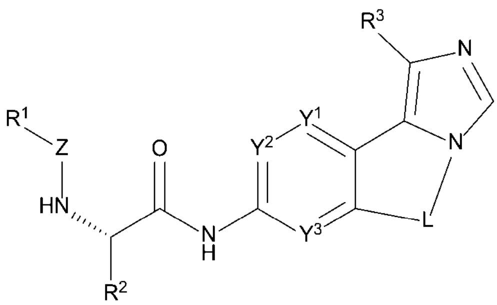
,
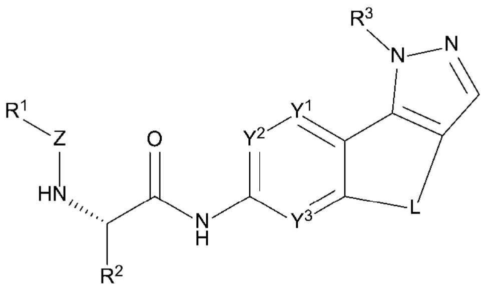

and

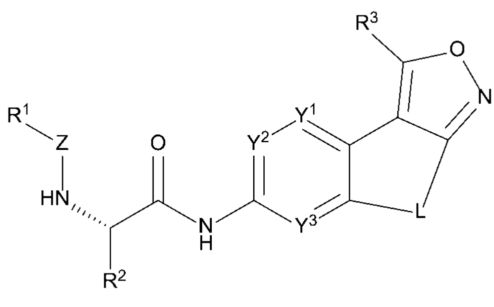
;

wherein $R^1$, $R^2$, $R^3$, $Y^1$, $Y^2$, $Y^3$, Z and L are as defined in claim 1.

4. The compound according to any one of claims 1-3, **characterized in that** $Y^1$, $Y^2$ and $Y^3$ are independently N or $CR^Y$;

each $R^Y$ is independently selected from the group consisting of hydrogen, deuterium, fluoro, chloro, bromo, cyano, methyl, ethyl, n-propyl, isopropyl, cyclopropyl, trifluoromethyl, methoxy, ethoxy and trifluoromethoxy; and Z is C(O) or $S(O)_2$.

5. The compound according to any one of claims 1-4, **characterized in that** $R^3$ is selected from the group consisting of methyl, deuteromethyl, ethyl, perdeuteroethyl, n-propyl, isopropyl, trifluoromethyl, cyclopropyl, cyclobutyl, hydroxymethyl, methoxymethyl, deuteromethoxymethyl, amino, methylamino, dimethylamino and deuteromethylamino.

6. The compound according to any one of claims 1-5, **characterized in that** $R^1$ is selected from the group consisting of:

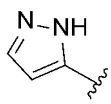, 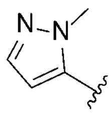, 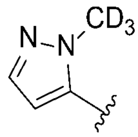, 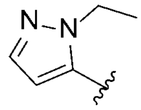,

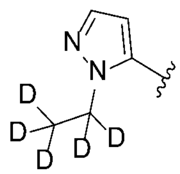, 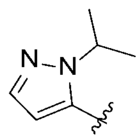, 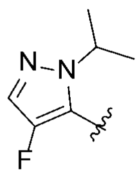, 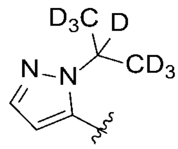, 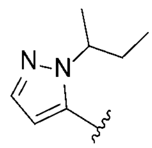, 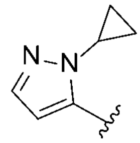,

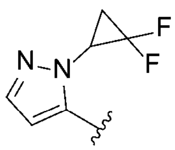, 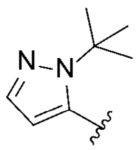, 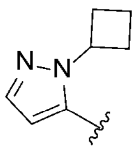, 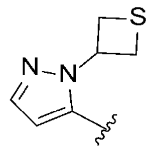, 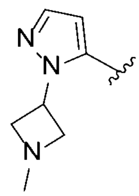, 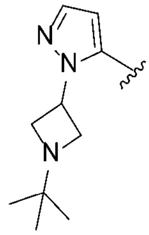,

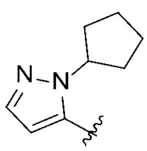, 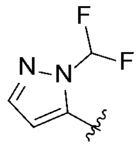, 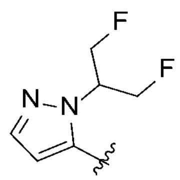, 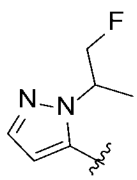, 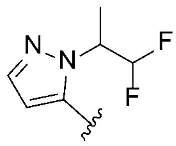, 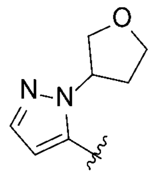,

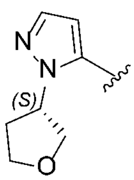, 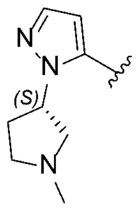, 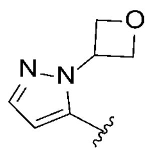, 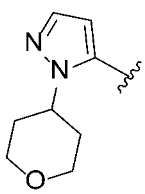, 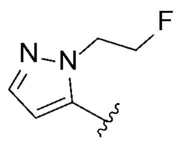, 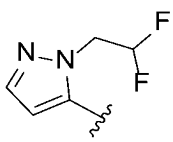,

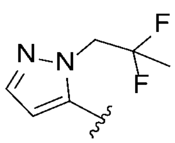, 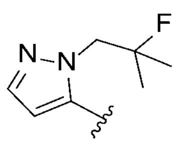, 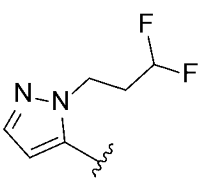, 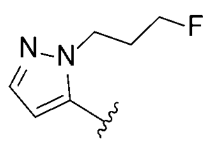, 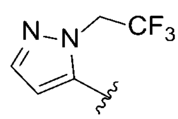,

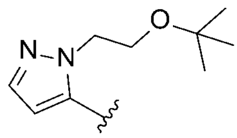 , 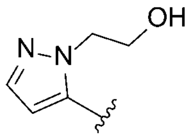 , 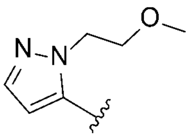 , 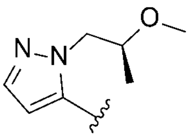 , 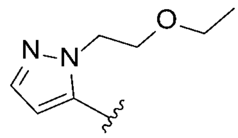

, 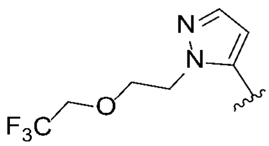 , 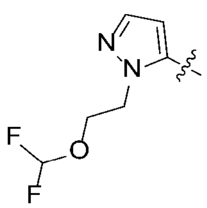 , 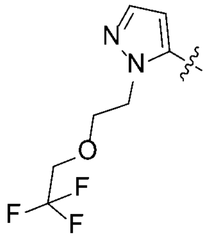 , 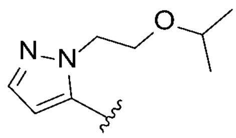 ,

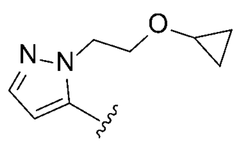 , 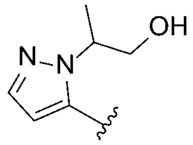 , 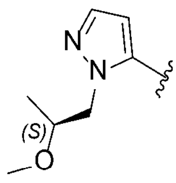 , 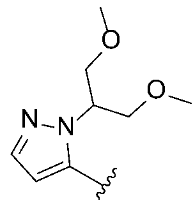 , 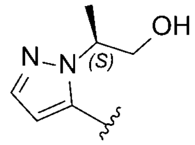 ,

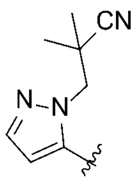 , 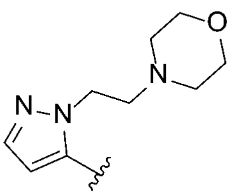 , 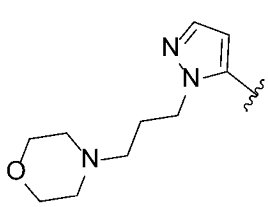 , 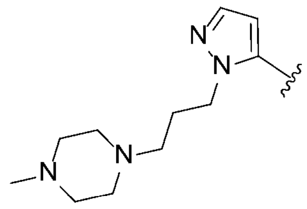 ,

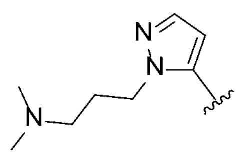 , 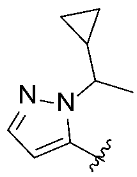 , 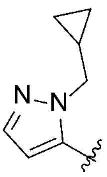 , 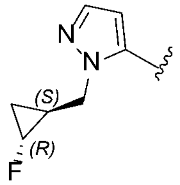 , 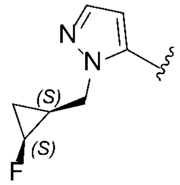 , 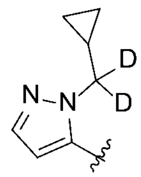 ,

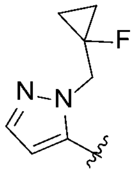 , 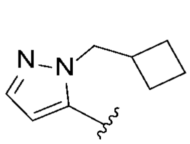 , 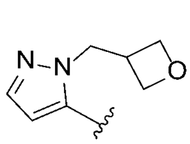 , 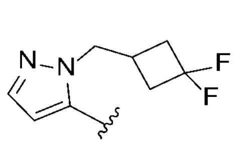 , 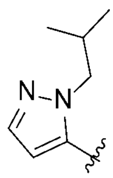 , 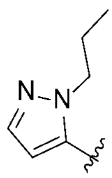 ,

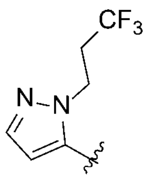 , 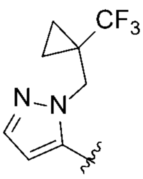 , 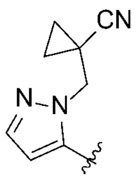 , 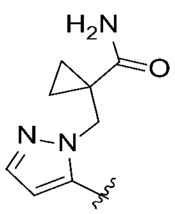 , 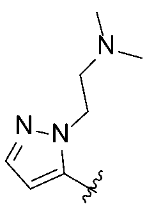 , 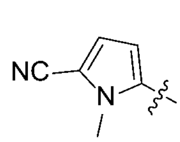 ,

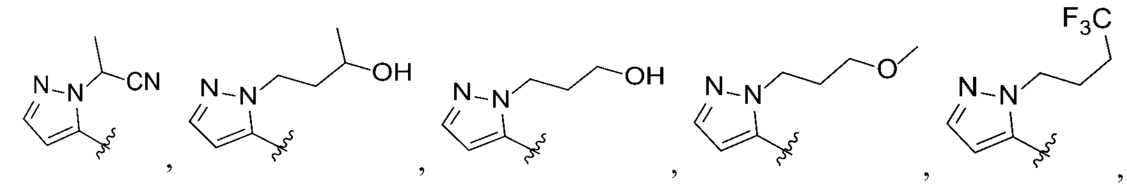
, , , , ,

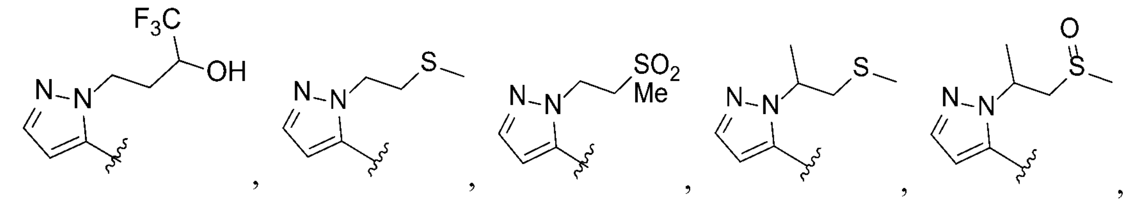
, , , , ,

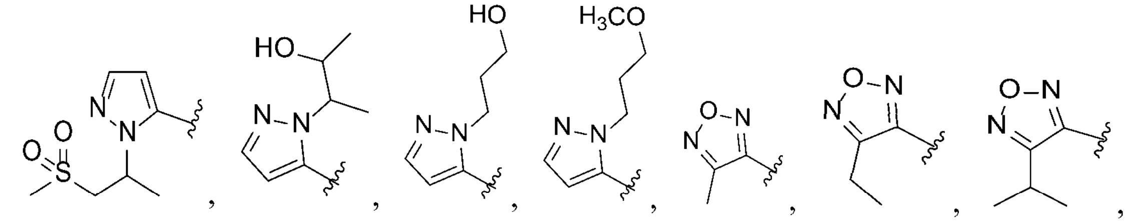
, , , , , , ,

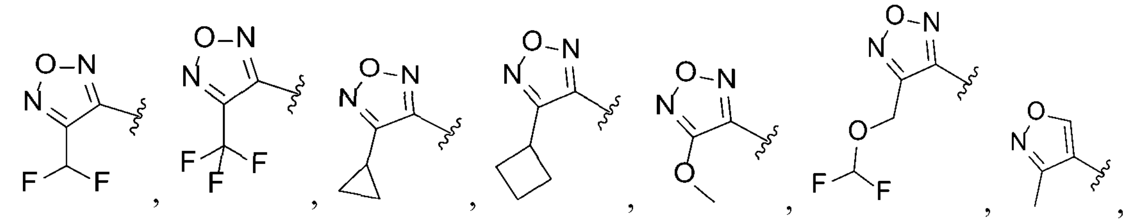
, , , , , , ,

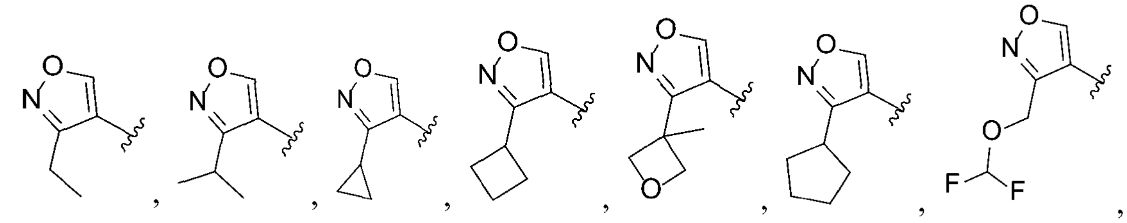
, , , , , , ,

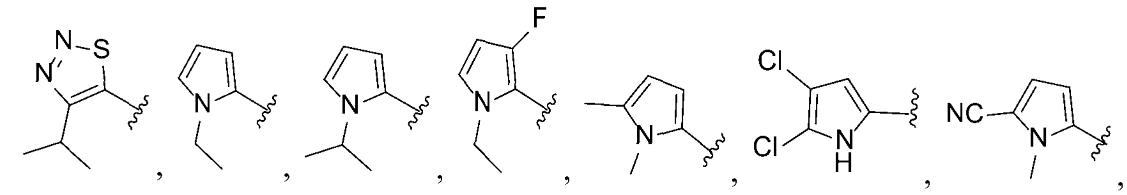
, , , , , , ,

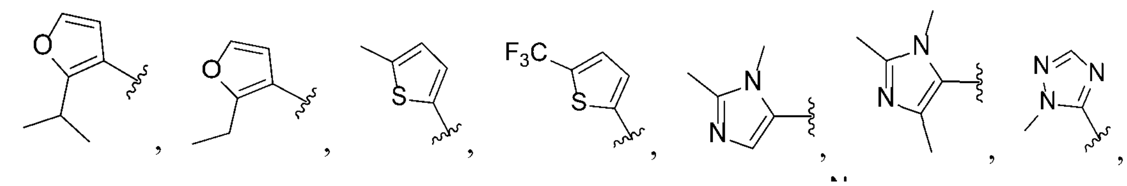
, , , , , , ,

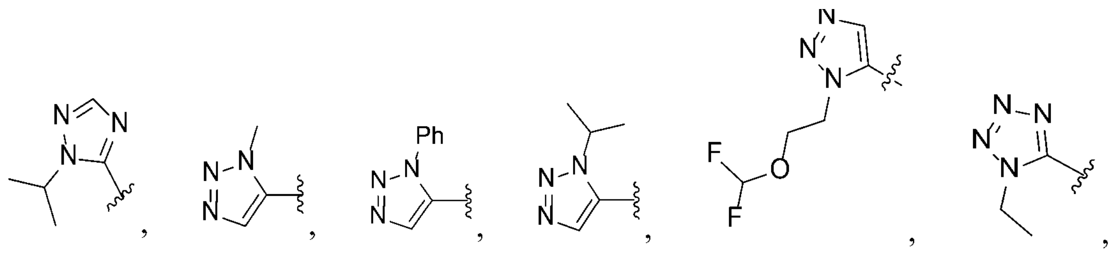

, , , , , ,

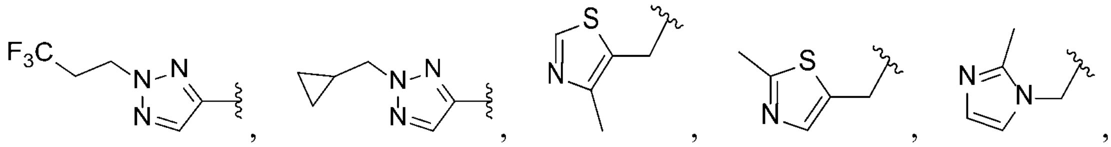

, , , , ,

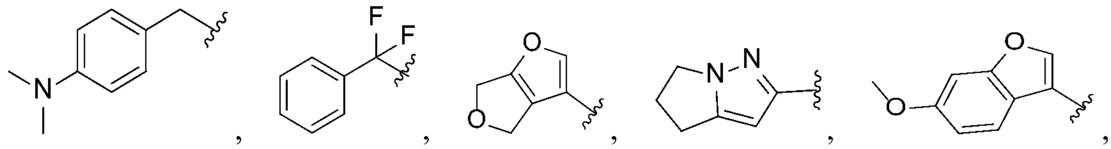

, , , , ,

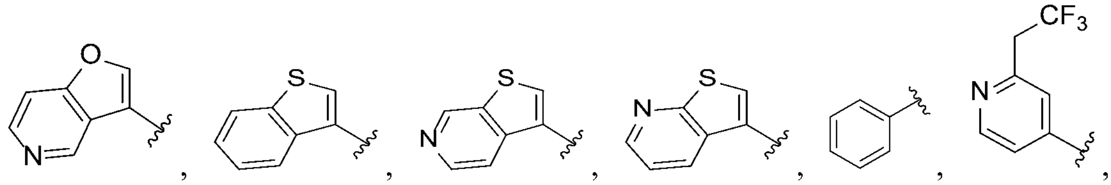

, , , , , ,

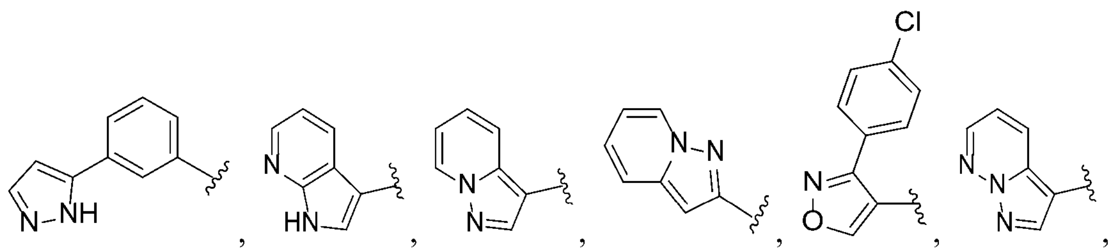

, , , , , ,

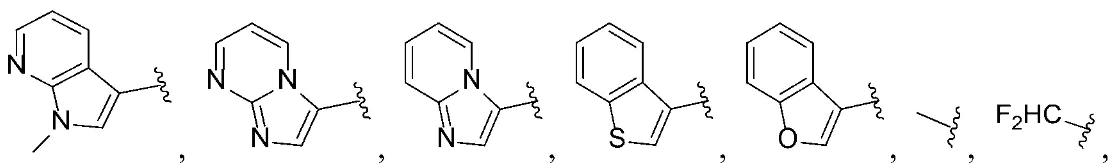

, , , , , , , ,

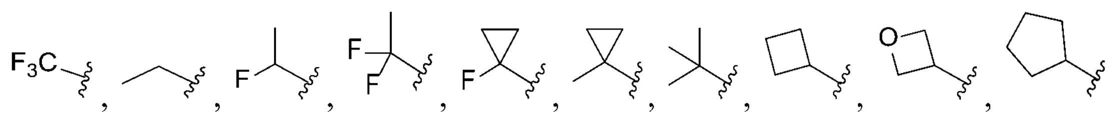

, , , , , , , , ,

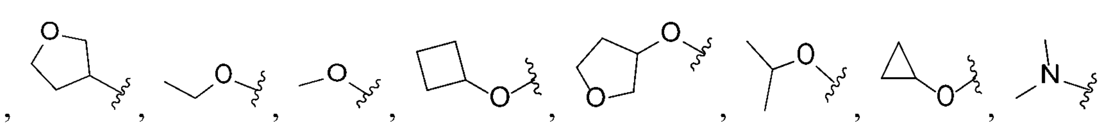

, , , , , , , , ,

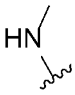, 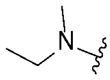, 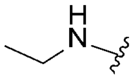, 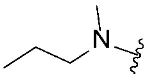, 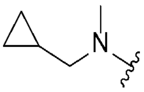, 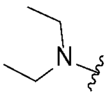, 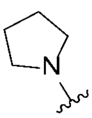, 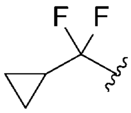,

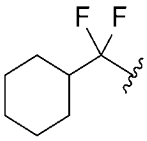, 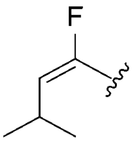, 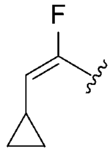, 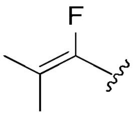, 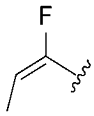, 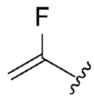, 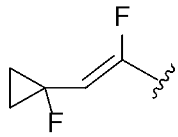,

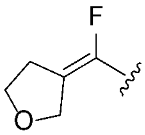, 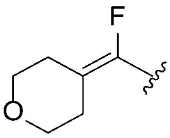, 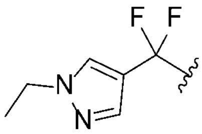, 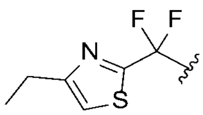, 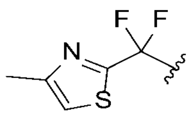,

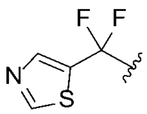, 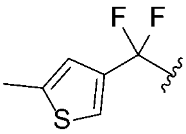, 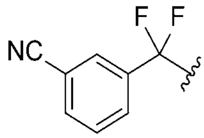, 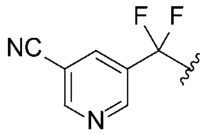, 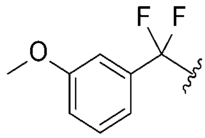,

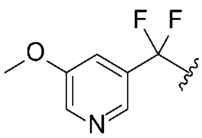, 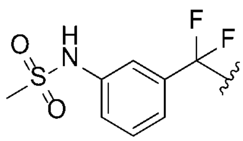, 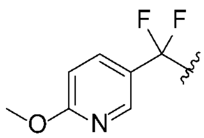, 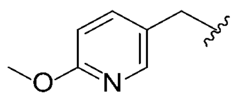,

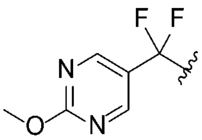, 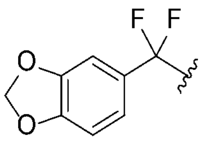 and 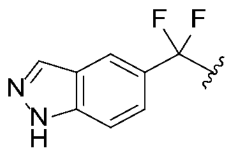.

7. The compound according to any one of claims 1-6, **characterized in that** $R^2$ is selected from the group consisting of:

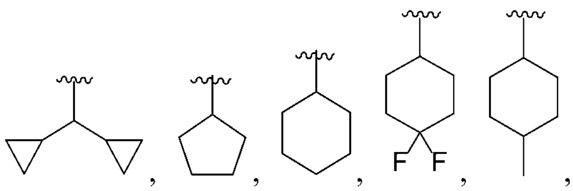

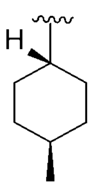, 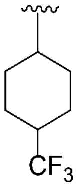, 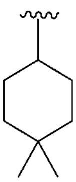, 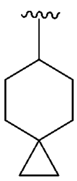, 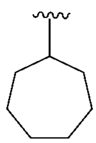, 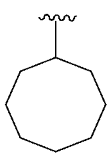, 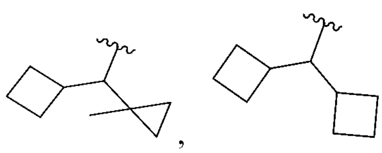

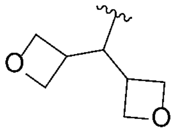, 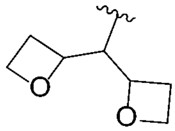, 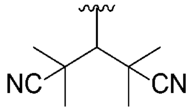, 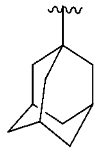, 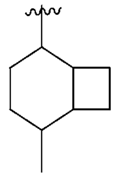, 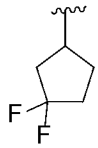, 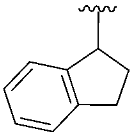,

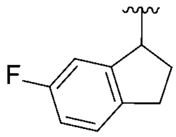, 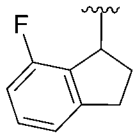, 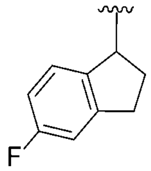, 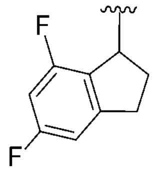, 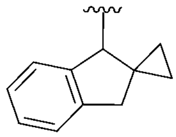, 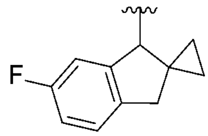,

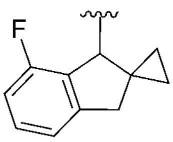, 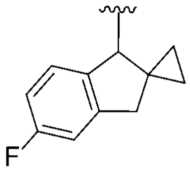, 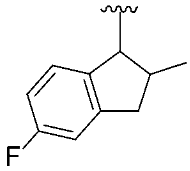, 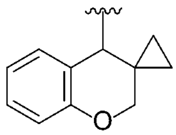, 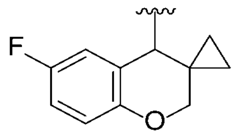,

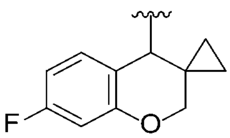, 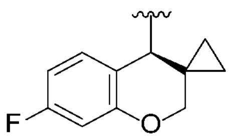, 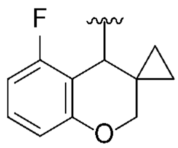, 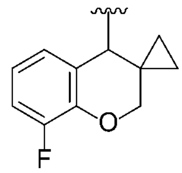,

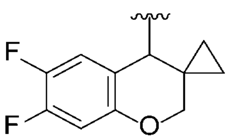, 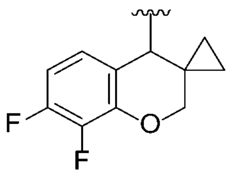, 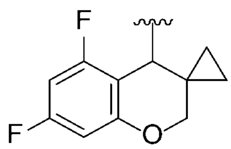, 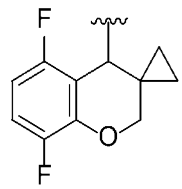,

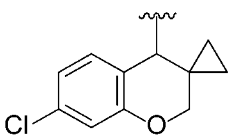, 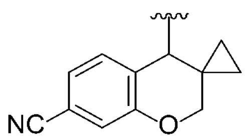, 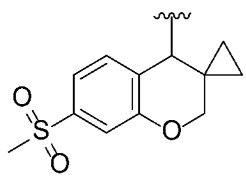, 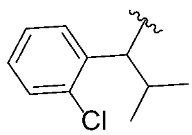,

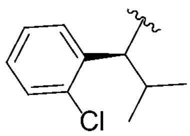, 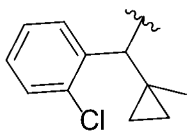, 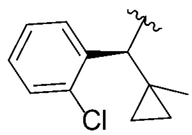, 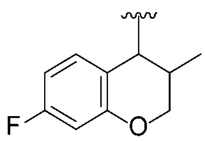, 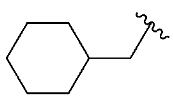,

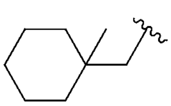, 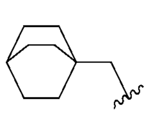, 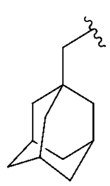, 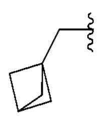, 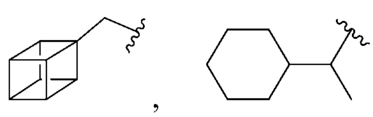, ,

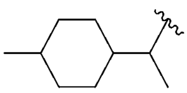, 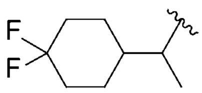, 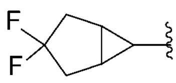, 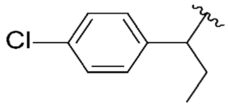, 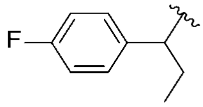,

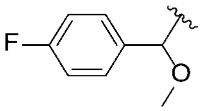, 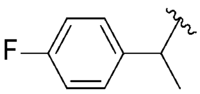, 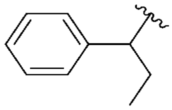, 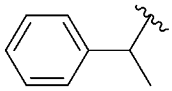, 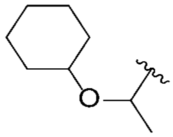,

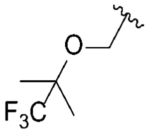, 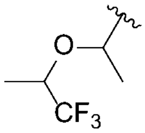, 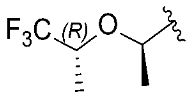, 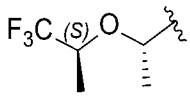, 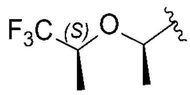,

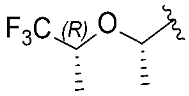, 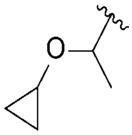, 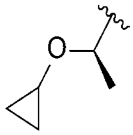, 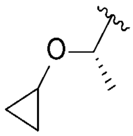, 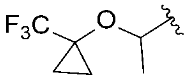, 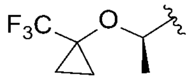

and

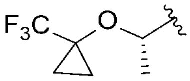.

**8.** The compound according to any one of claims 1-7, **characterized in that** L is selected from the group consisting of:

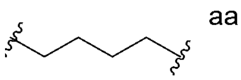, 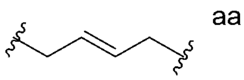,

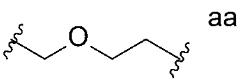, 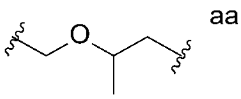, 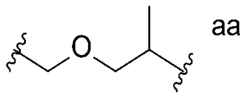, 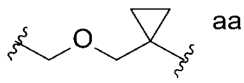,

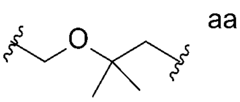, 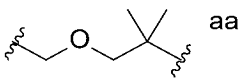, 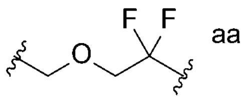, 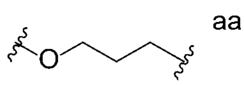,

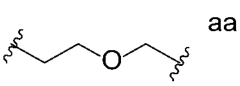, 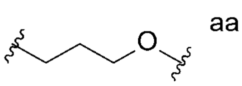, 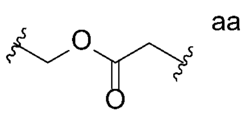, 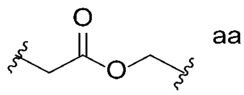,

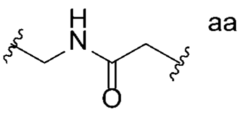, 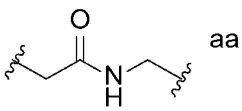, 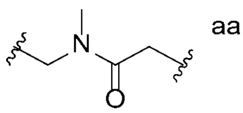, 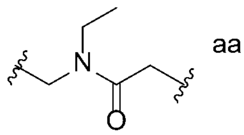,

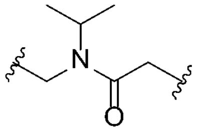 aa , 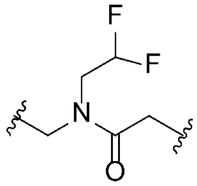 aa , 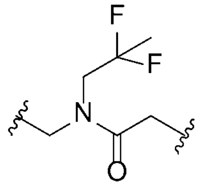 aa , 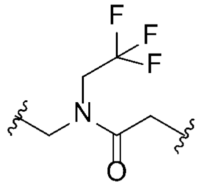 aa ,

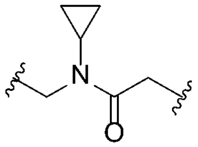 aa , 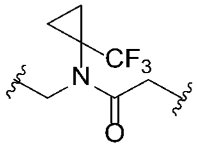 aa , 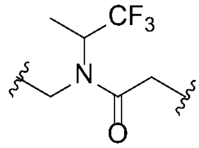 aa , 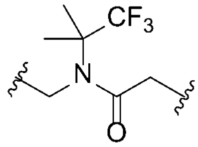 aa ,

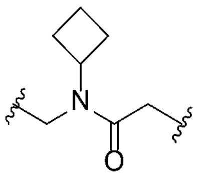 aa , 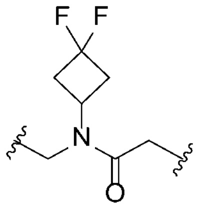 aa , 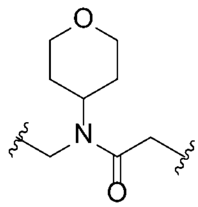 aa , 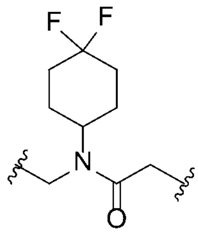 aa ,

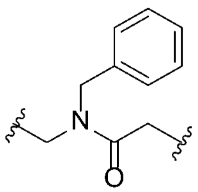 aa , 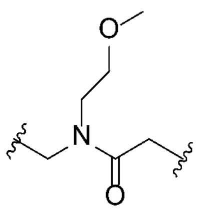 aa , 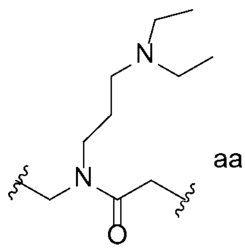 aa , 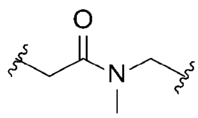 aa ,

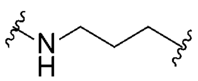 aa , 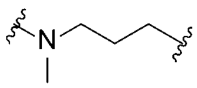 aa , 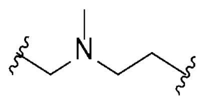 aa , 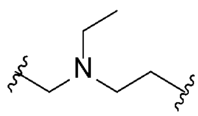 aa ,

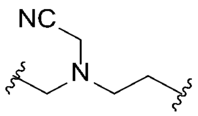 aa , 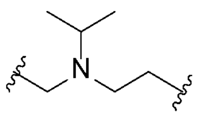 aa , 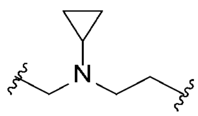 aa , 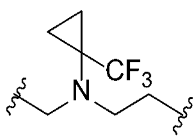 aa ,

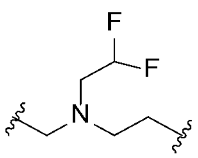 aa , 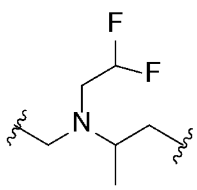 aa , 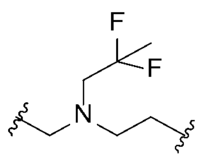 aa , 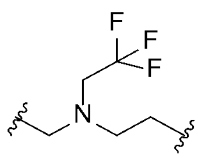 aa ,

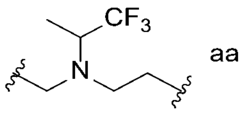, 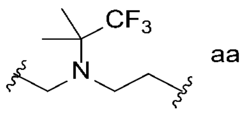, 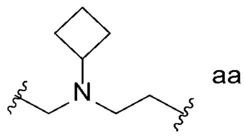, 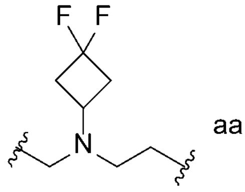,

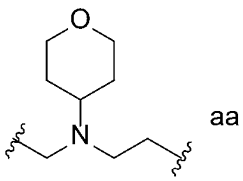, 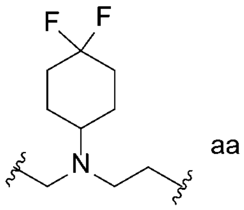, 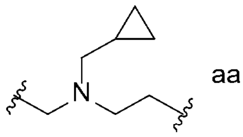, 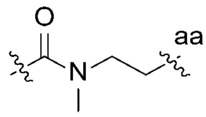,

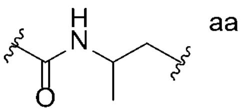, 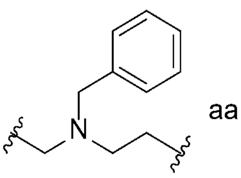 and 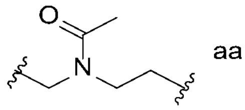;

wherein aa indicates an end of L linked to $X^4$.

**9.** The compound according to any one of claims 1-8, **characterized in that** the compound is selected from the group consisting of:

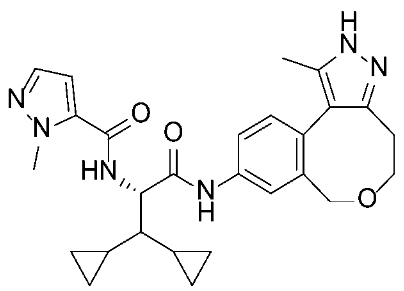, 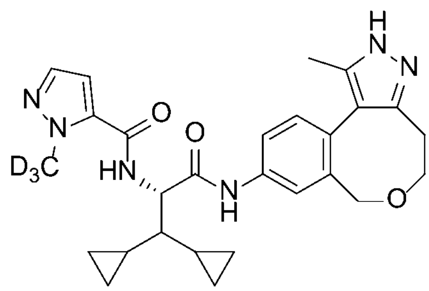,

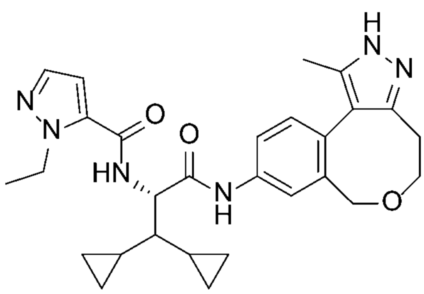, 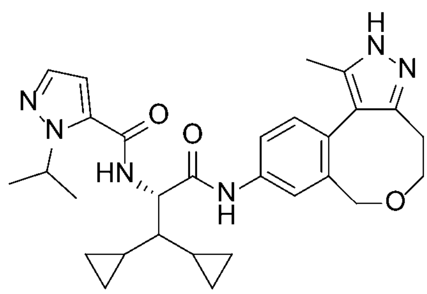,

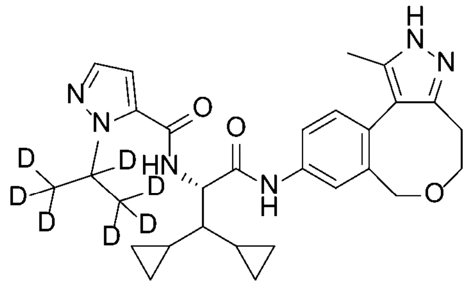

,

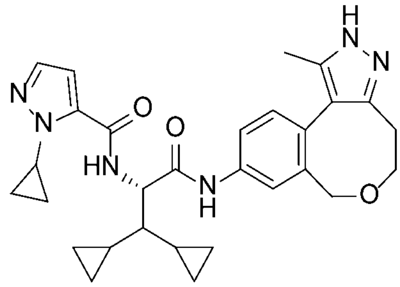

,

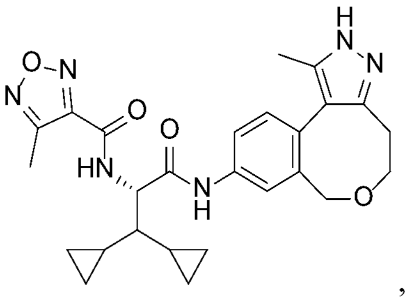

,

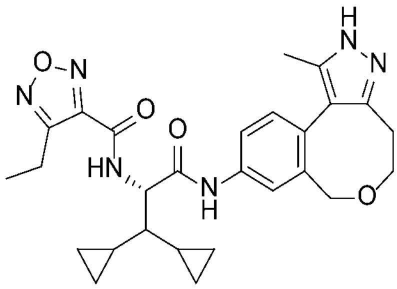

,

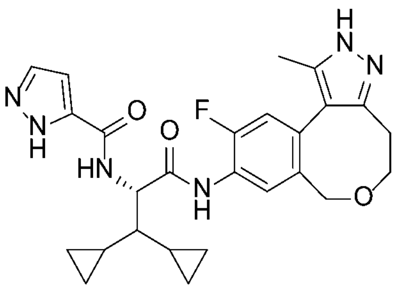

,

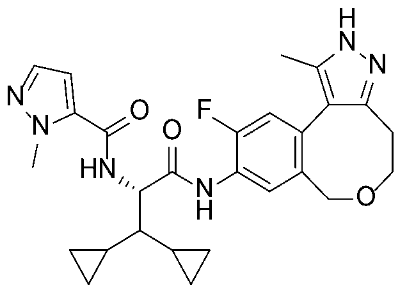

,

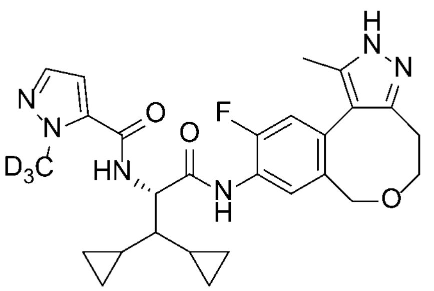

,

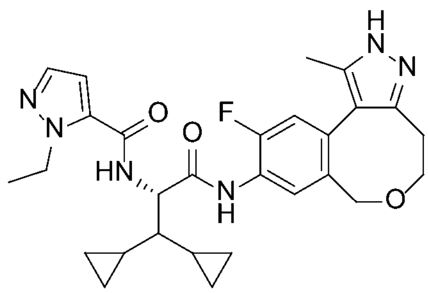

,

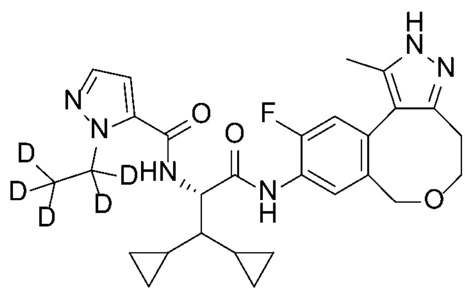

,

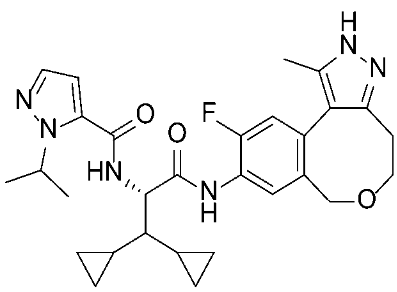

,

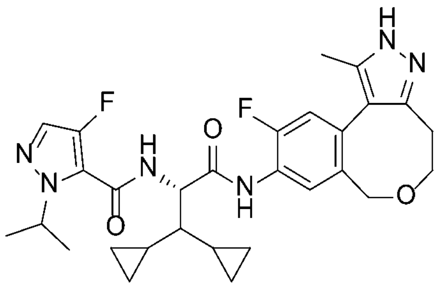,

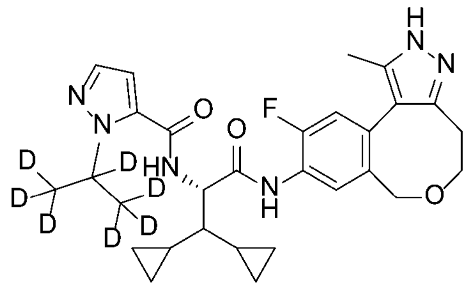,

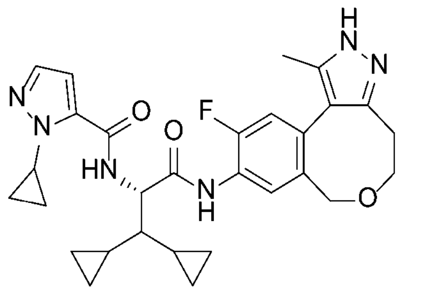,

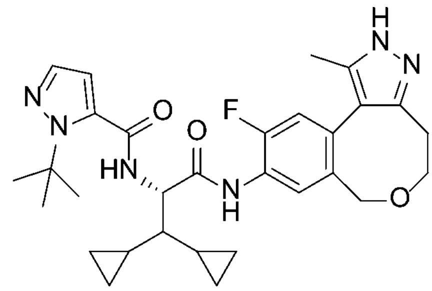,

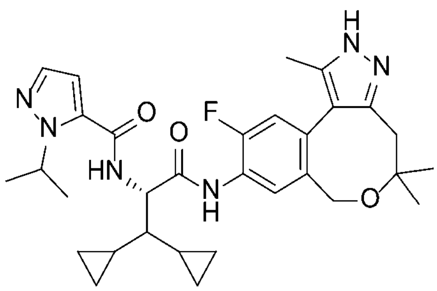,

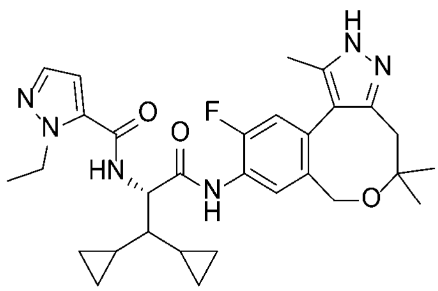,

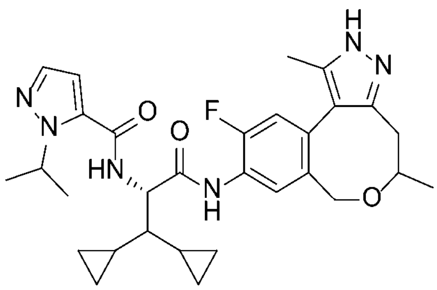,

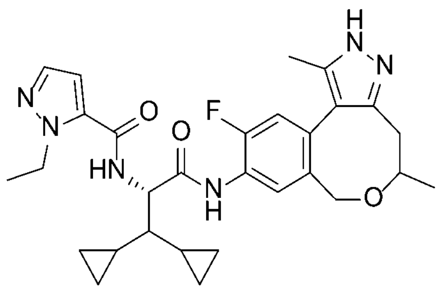,

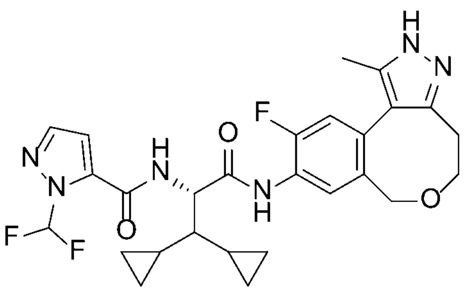,

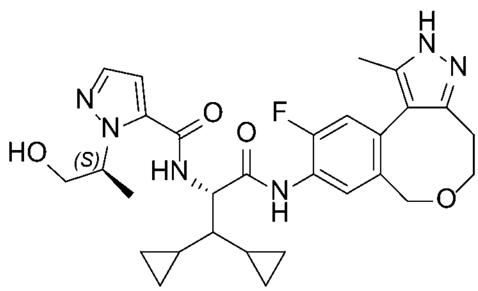,

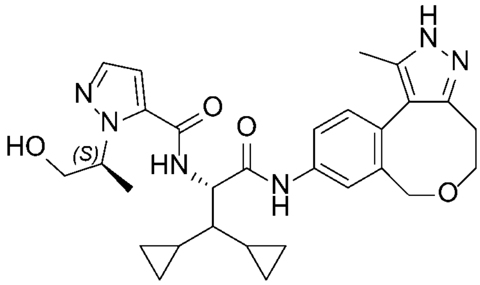,

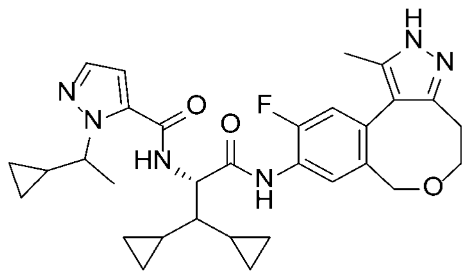,

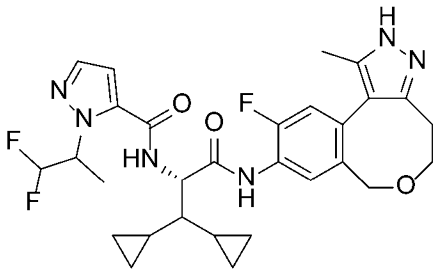,

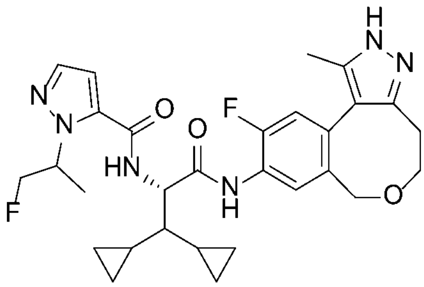,

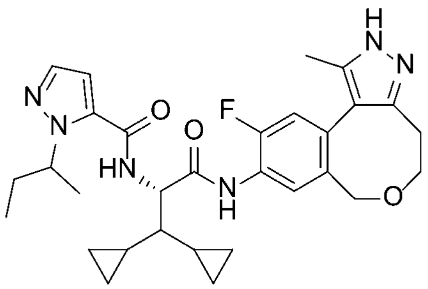,

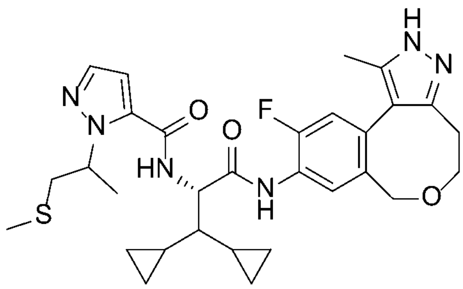,

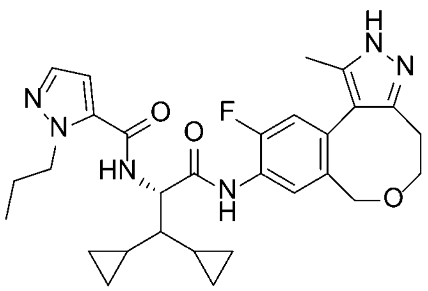,

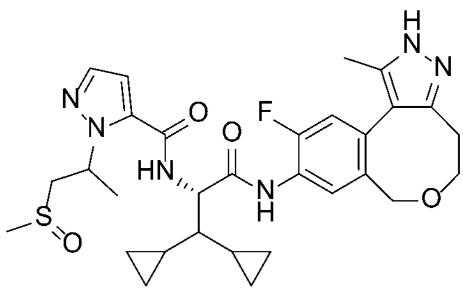,

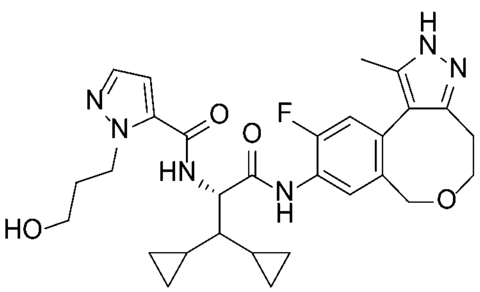,

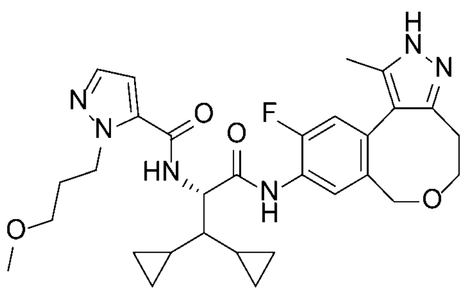,

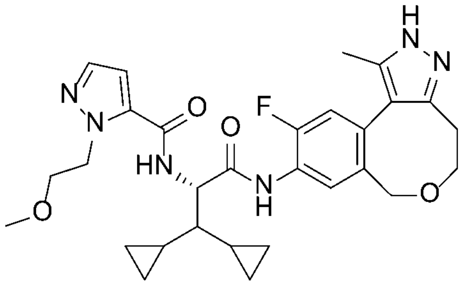,

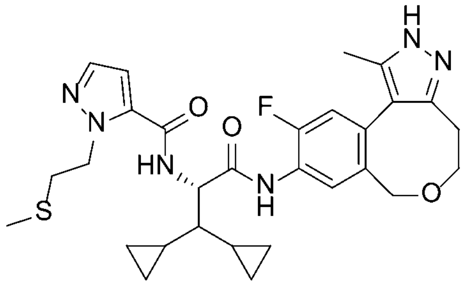,

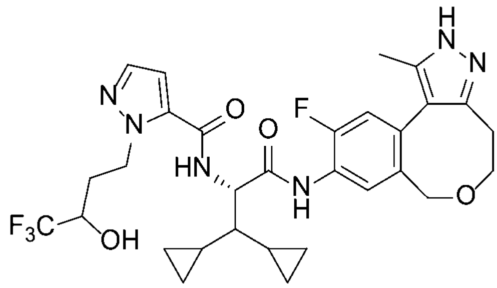,

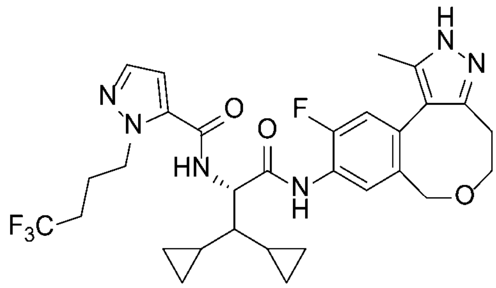,

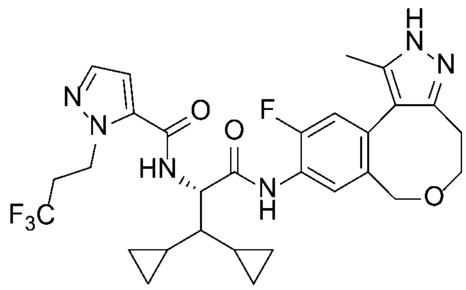,

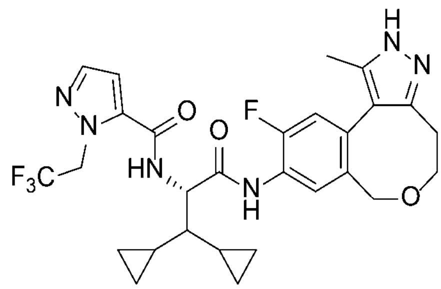,

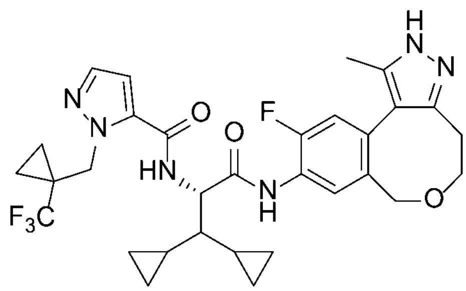,

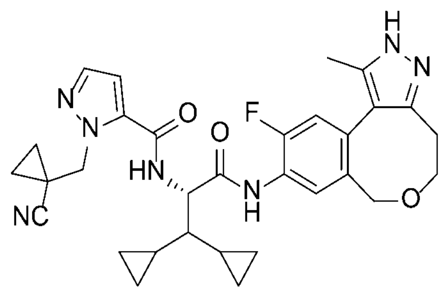,

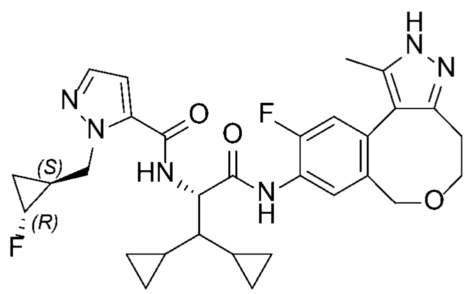,

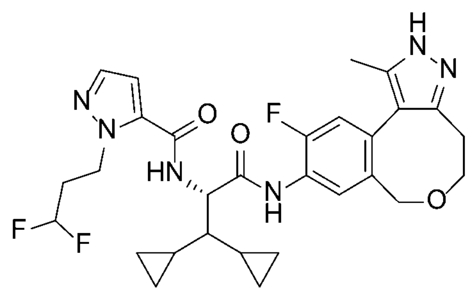,

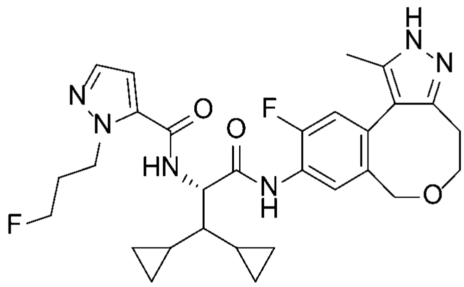,

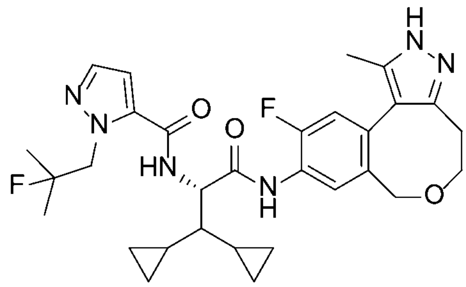,

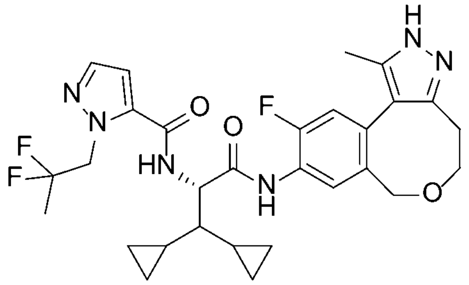,

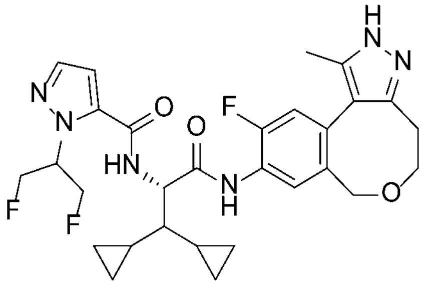,

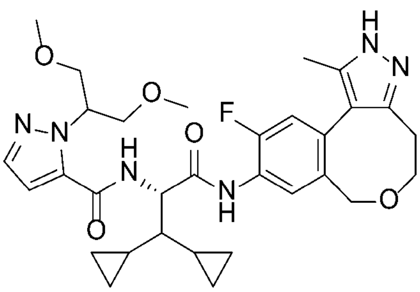,

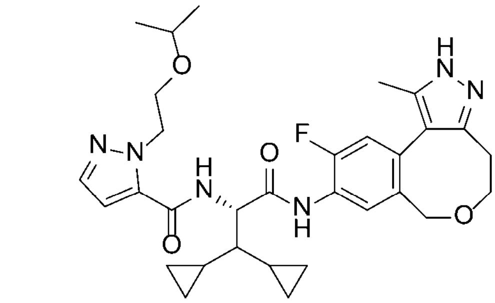,

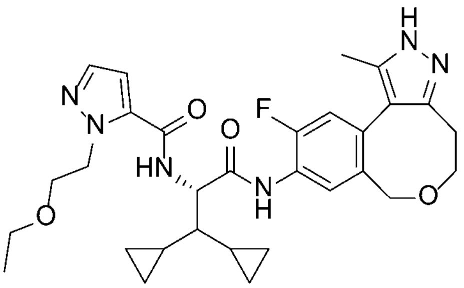,

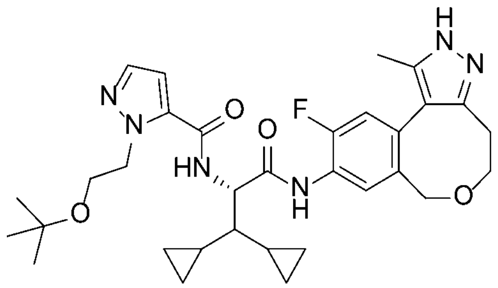,

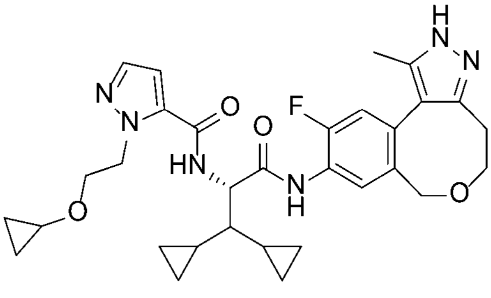,

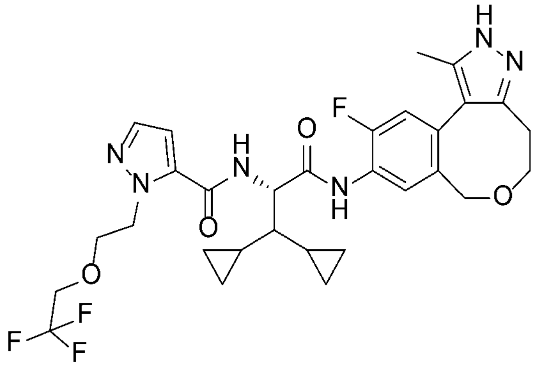,

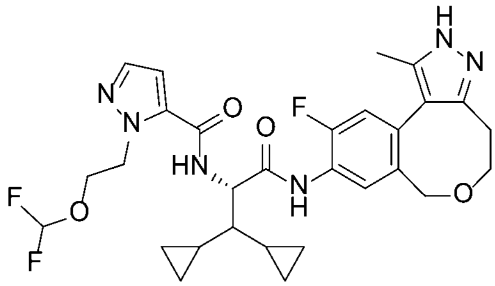
,
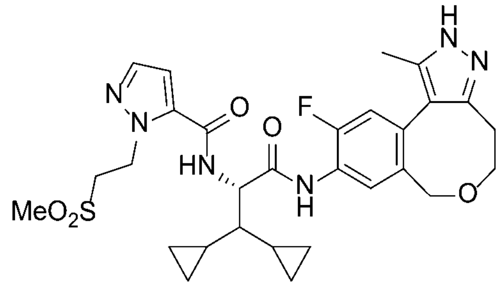
,
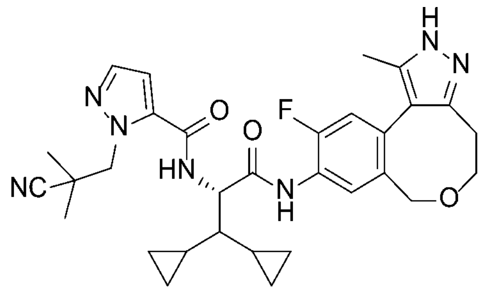
,
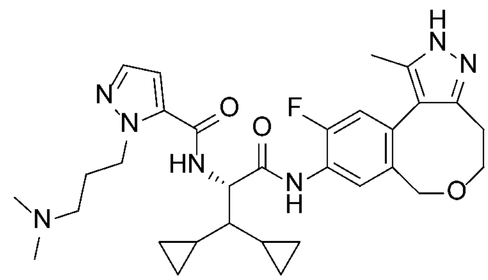
,
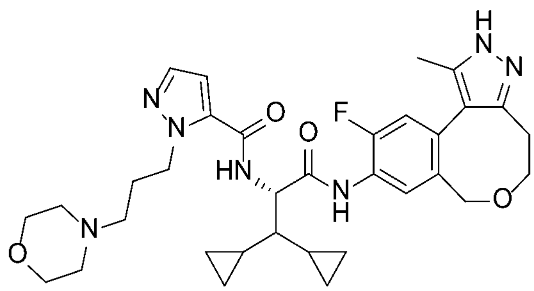
,
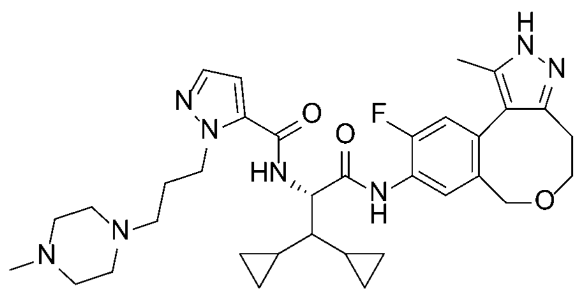
,
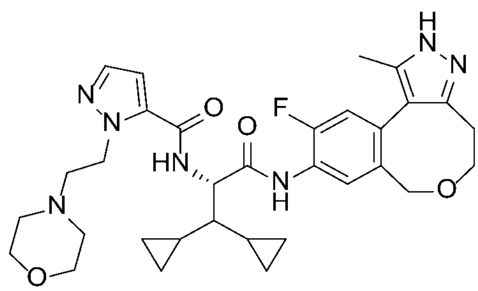
,
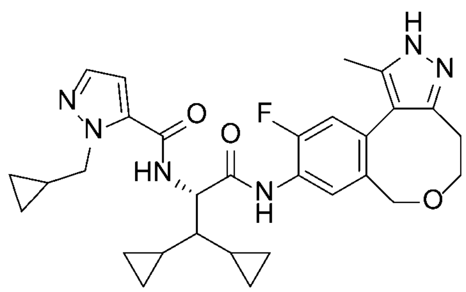
,
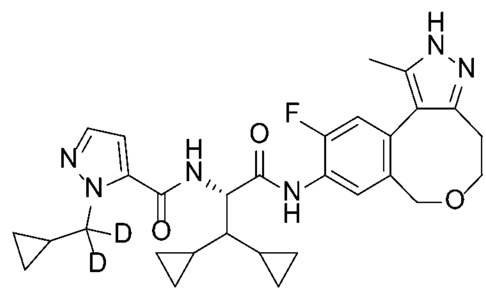
,

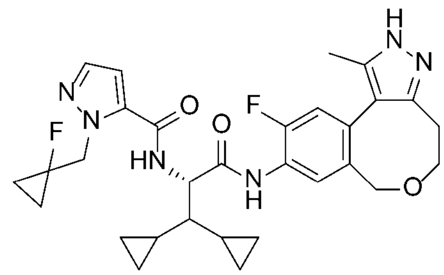
,
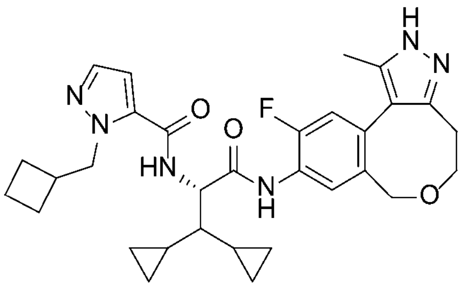
,
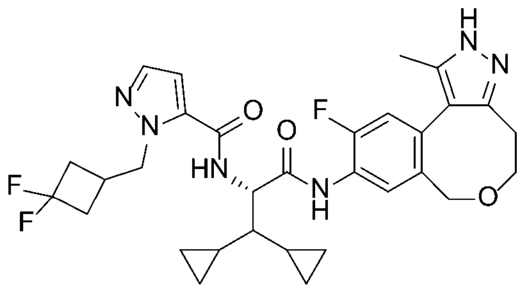
,
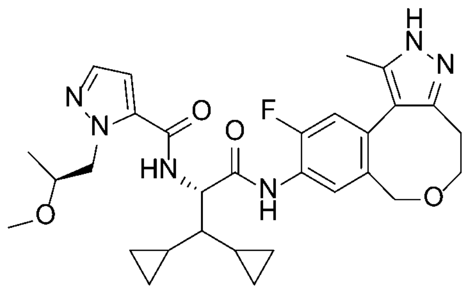
,
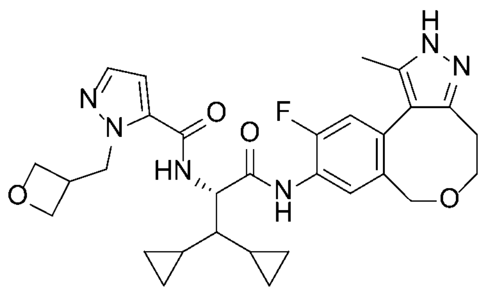
,
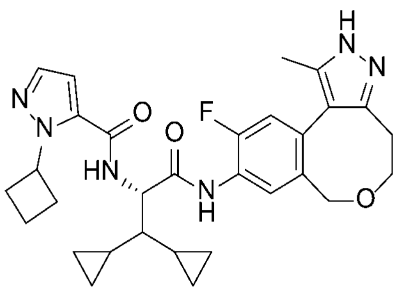
,
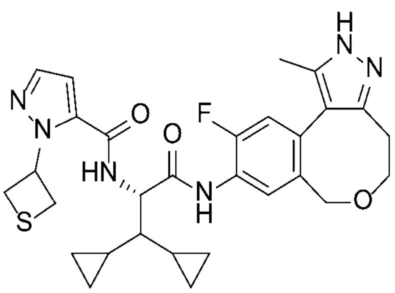
,
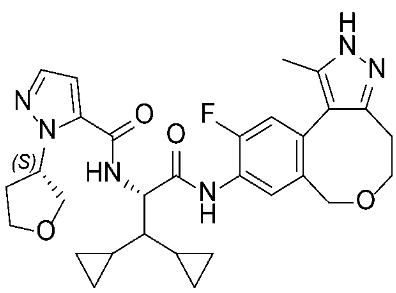
,
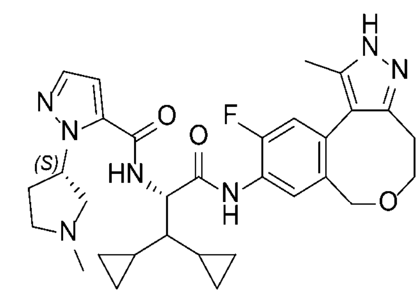
,
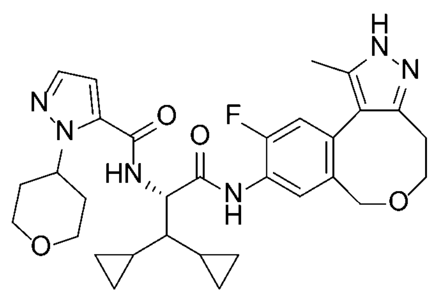
,

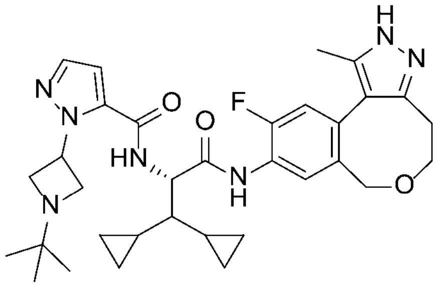

,

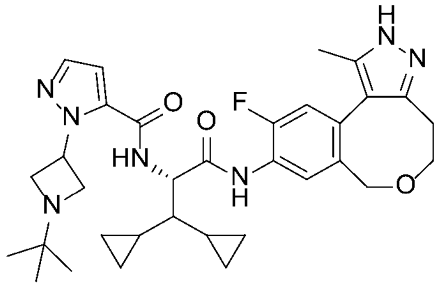

,

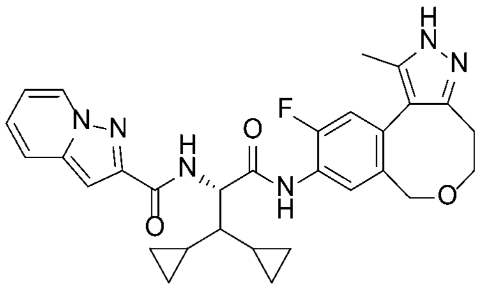

,

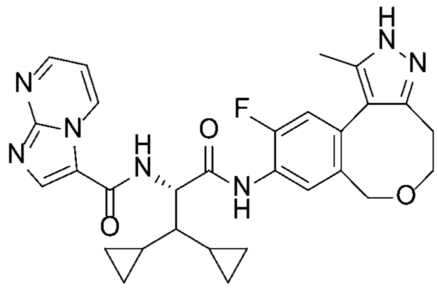

,

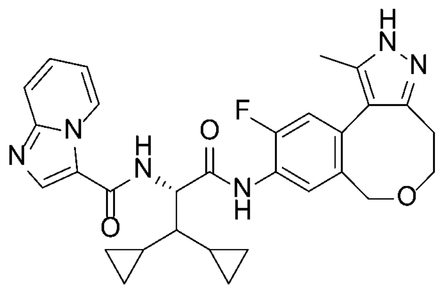

,

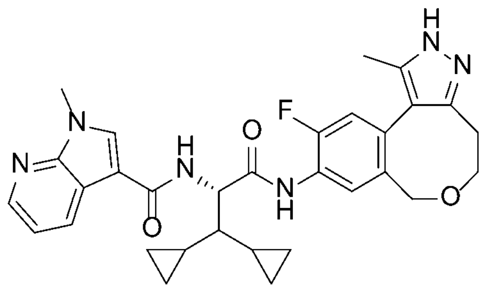

,

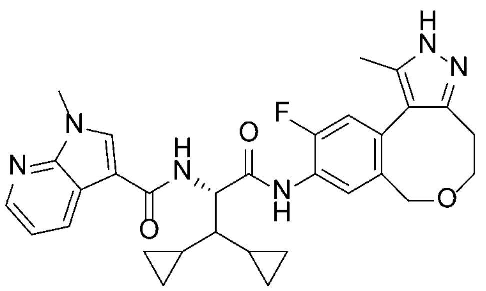

,

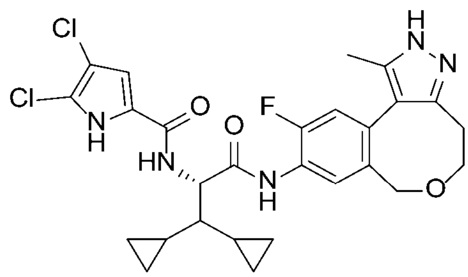

,

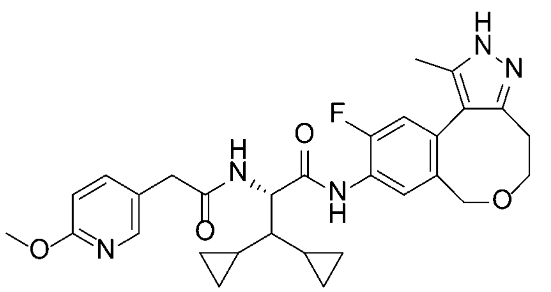

,

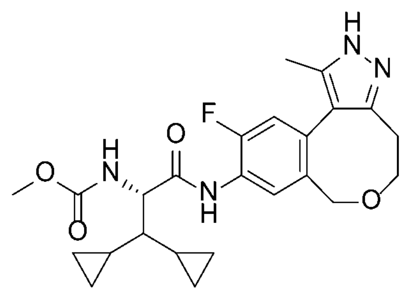

,

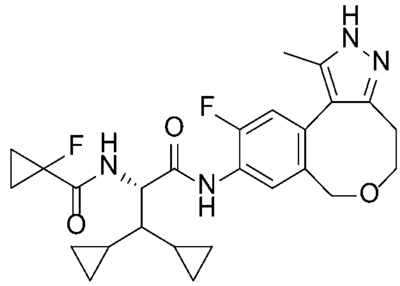
,

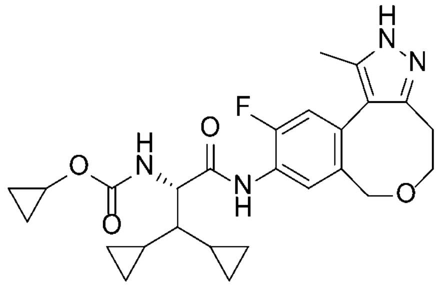
,

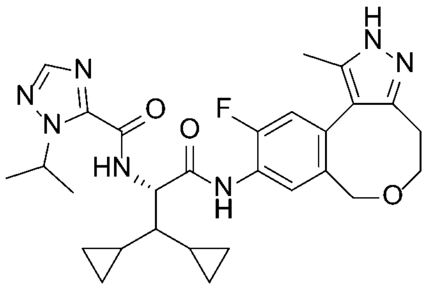
,

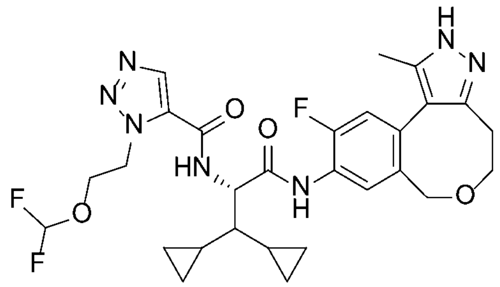
,

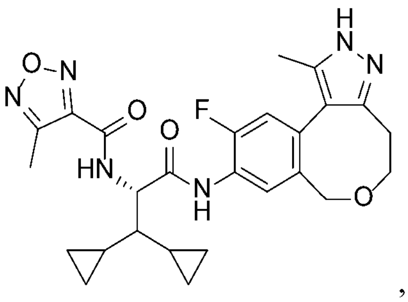
,

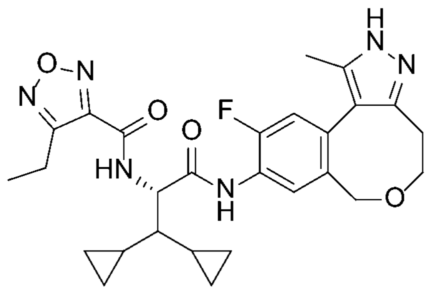
,

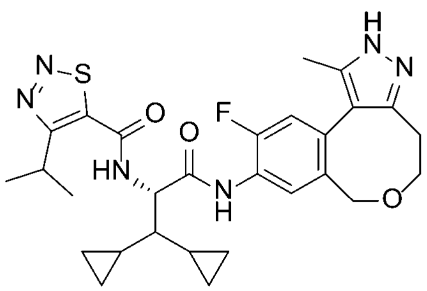
,

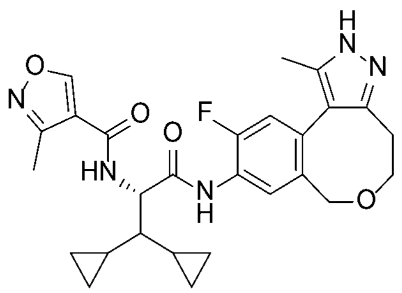
,

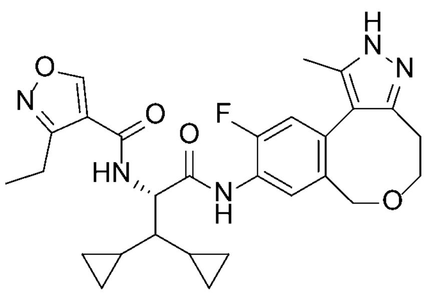
,

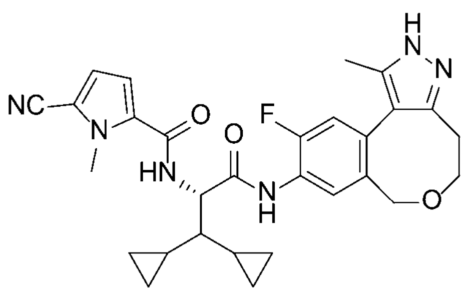
,

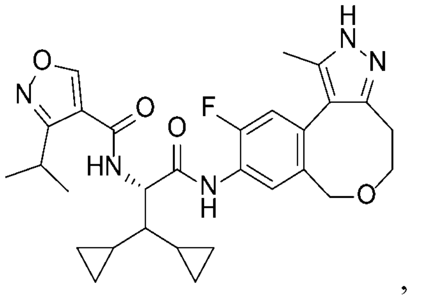,

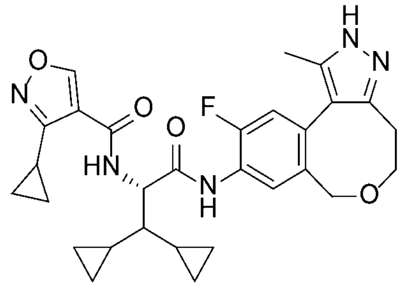,

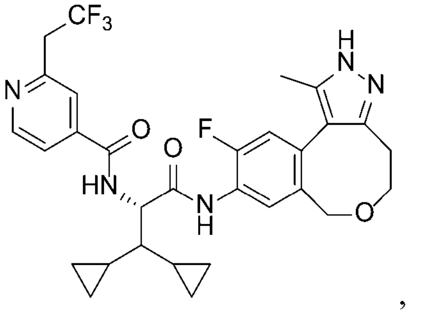,

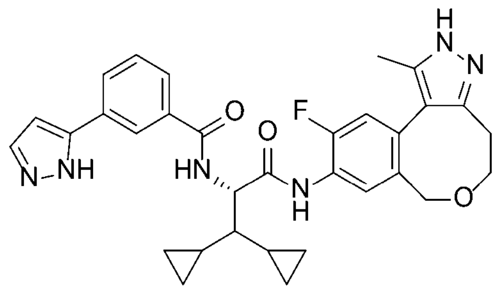,

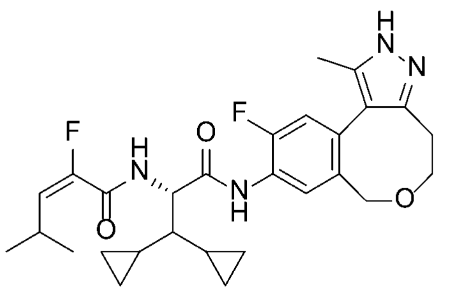,

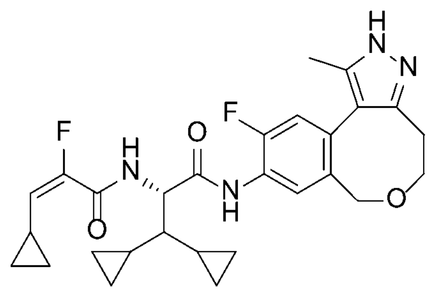,

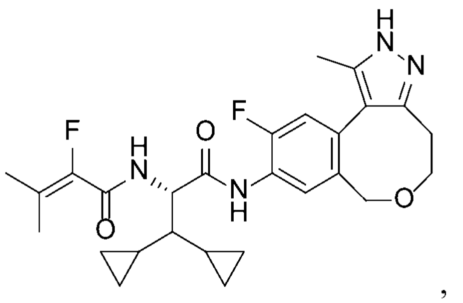,

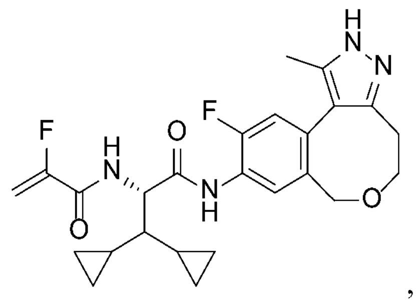,

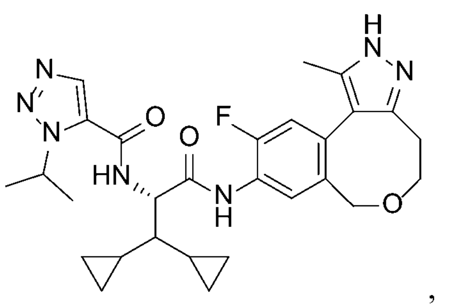,

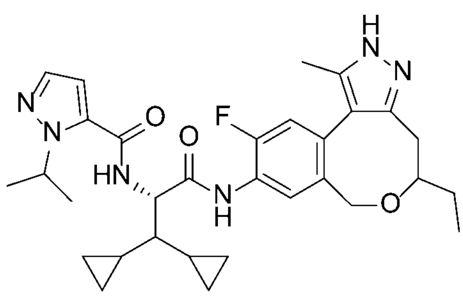,

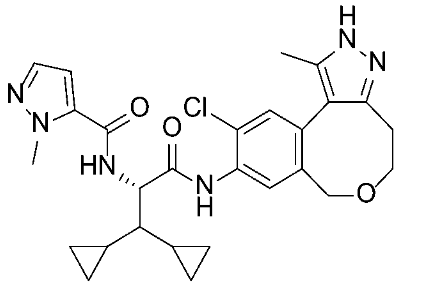

,

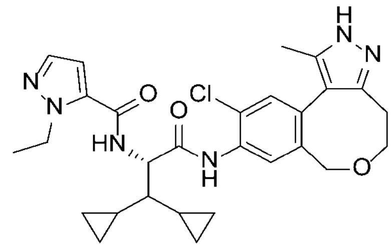

,

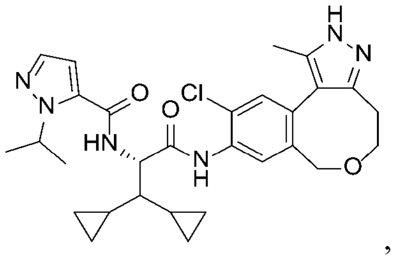

,

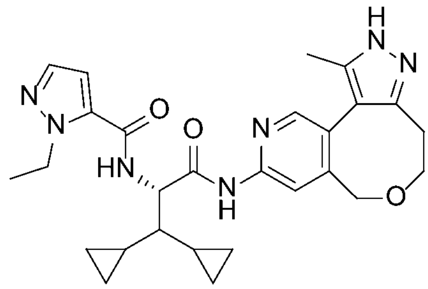

,

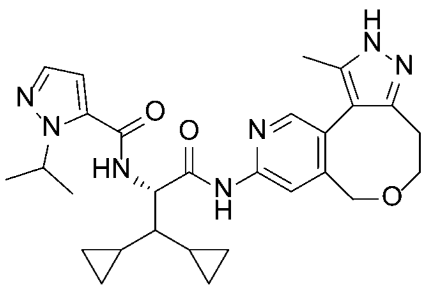

,

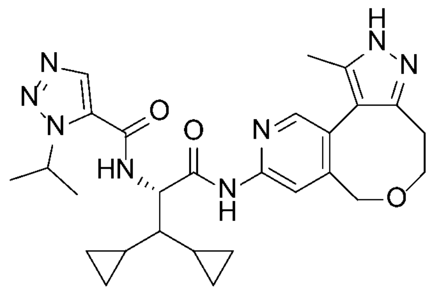

,

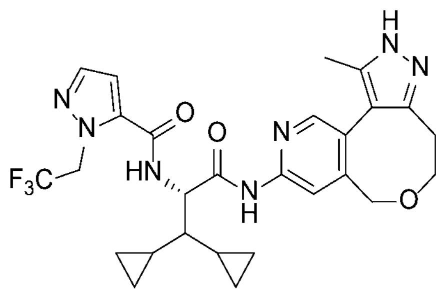

,

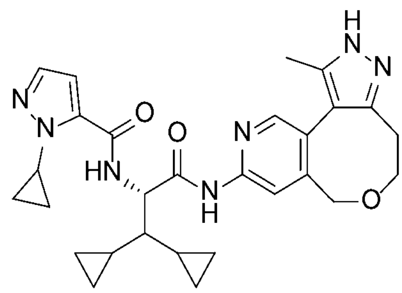

,

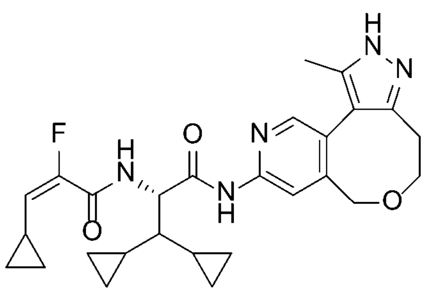

,

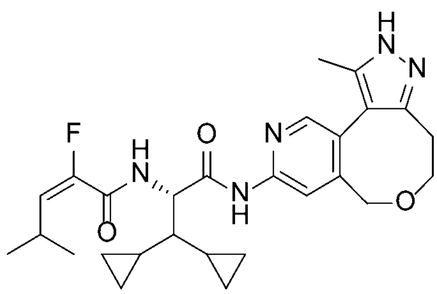

,

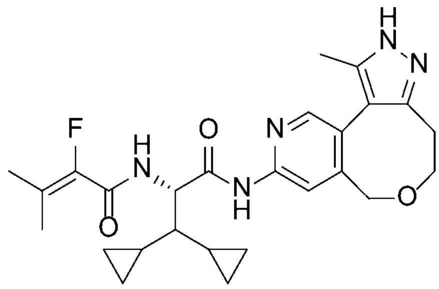
,
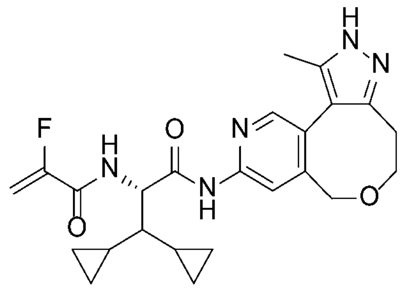
,
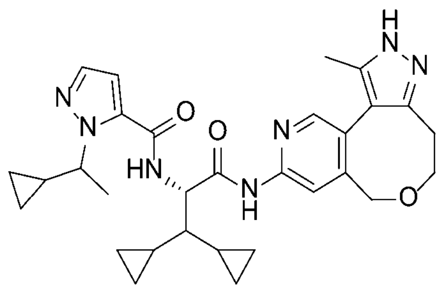
,
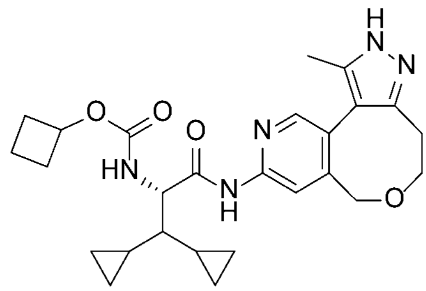
,
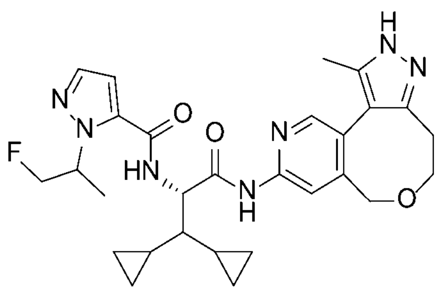
,
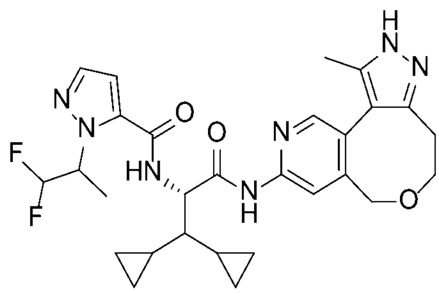
,
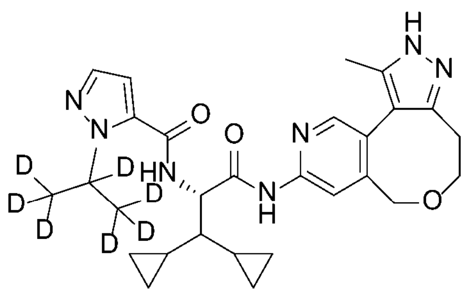
,
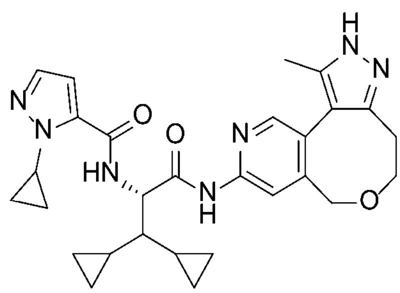
,
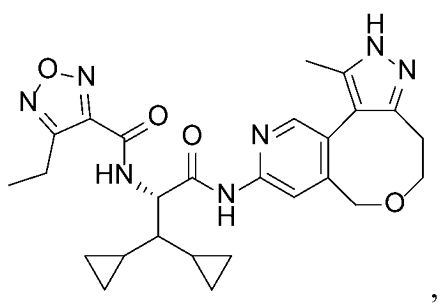
,
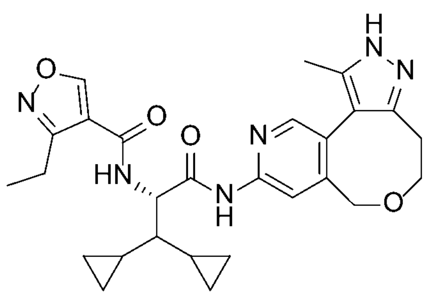
,

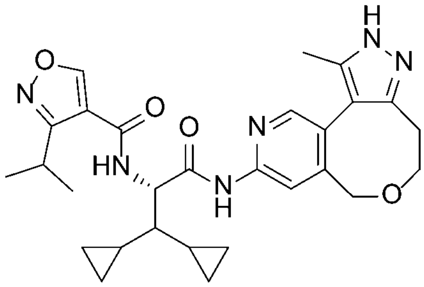
,
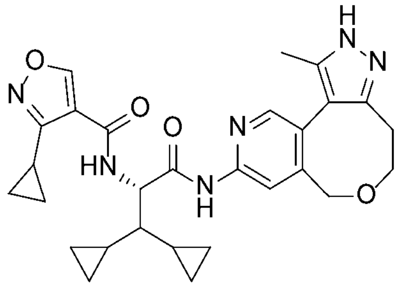
,
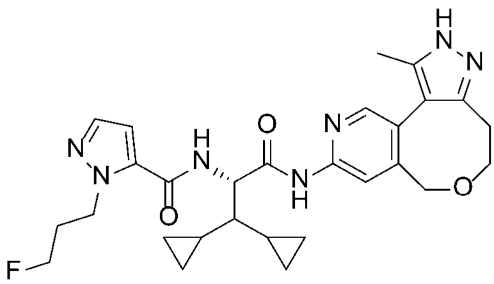
,
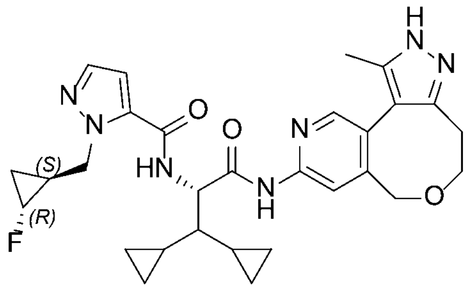
,
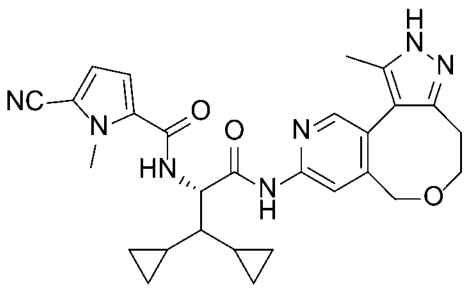
,
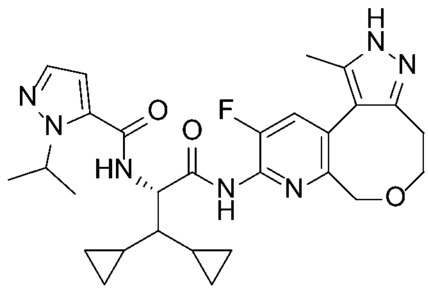
,
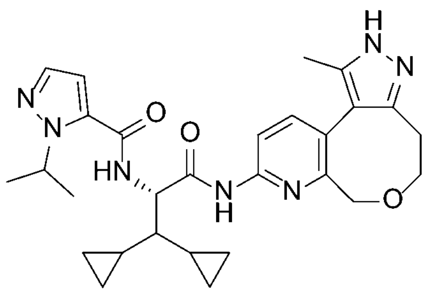
,
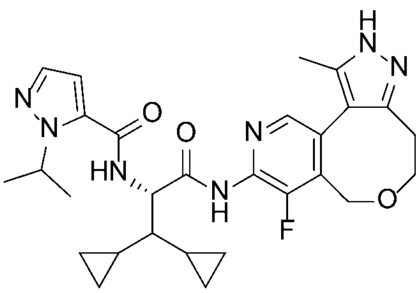
,
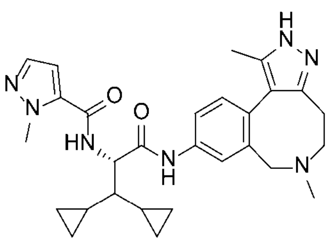
,
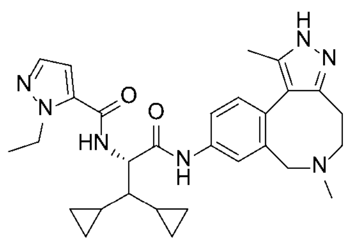
,

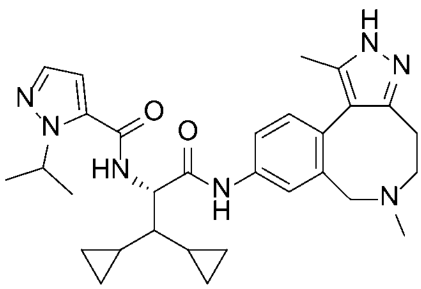,

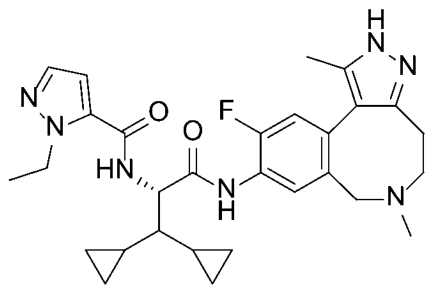,

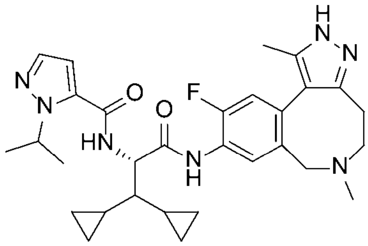,

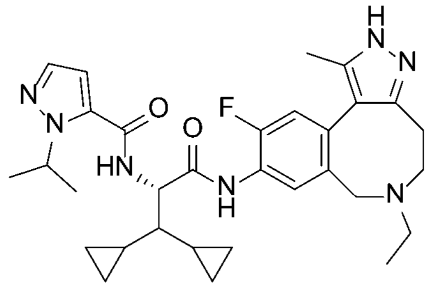,

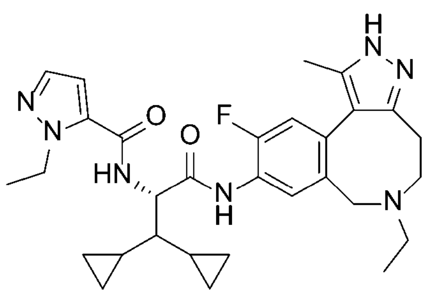,

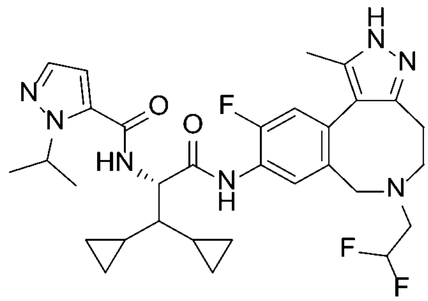,

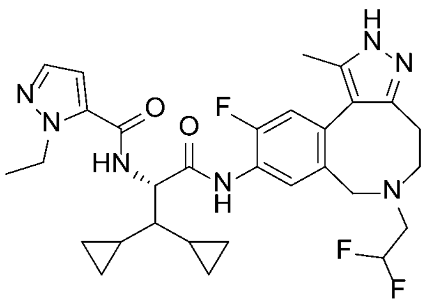,

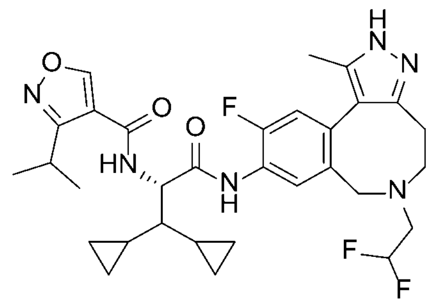,

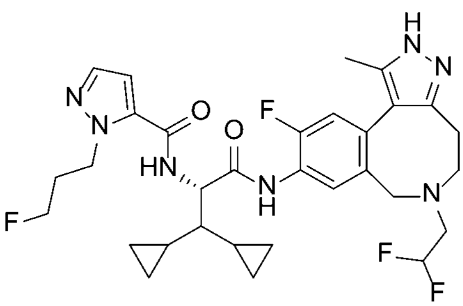,

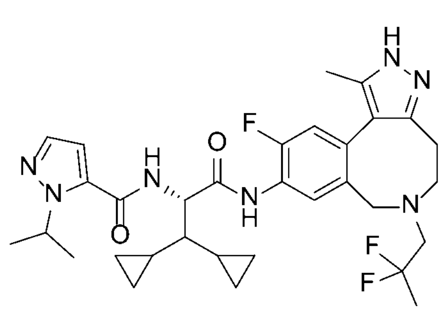,

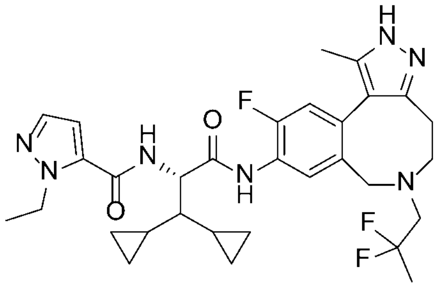,

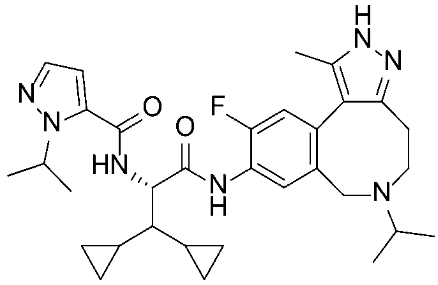,

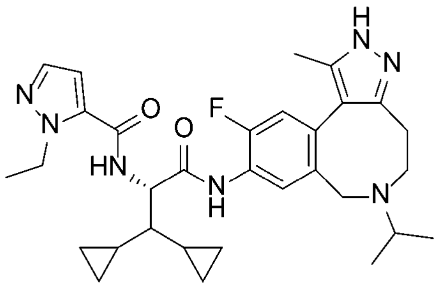,

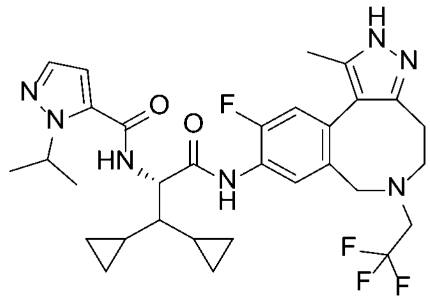,

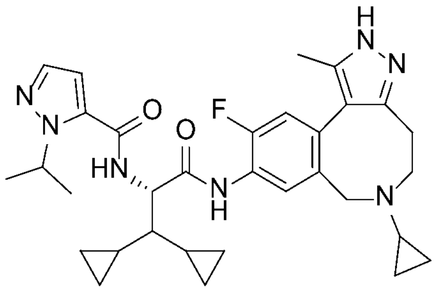,

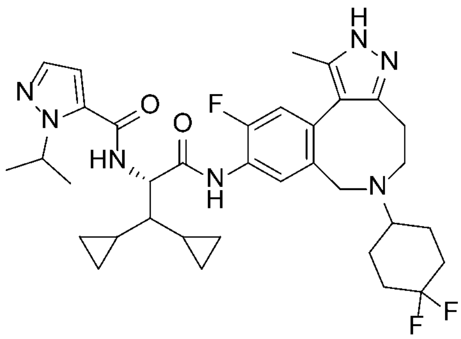,

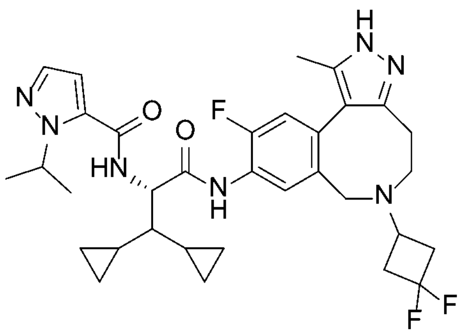.

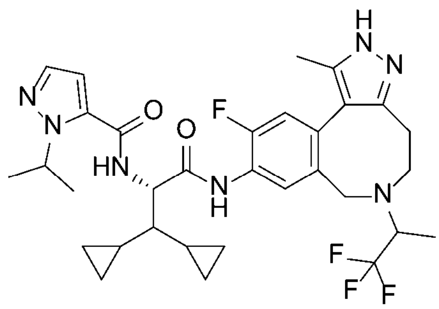.

,

,

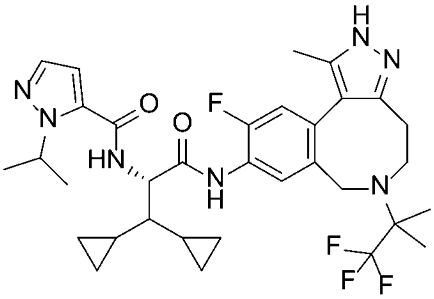

,

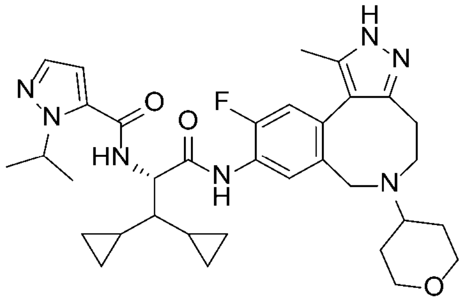

,

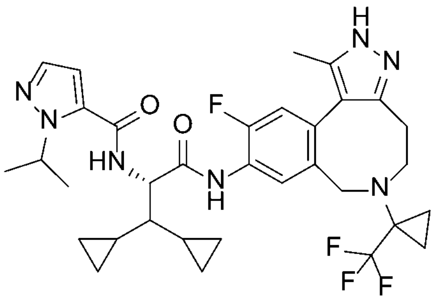

,

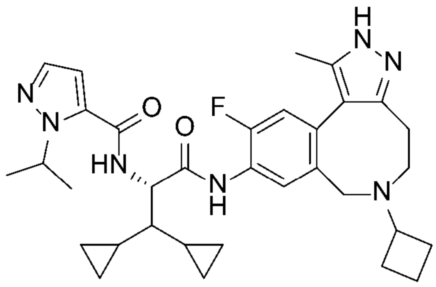

,

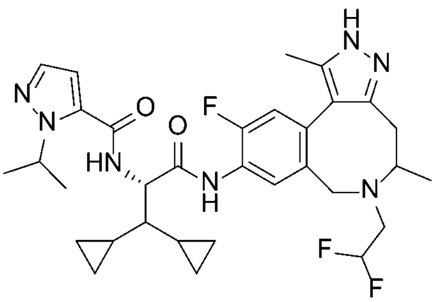

,

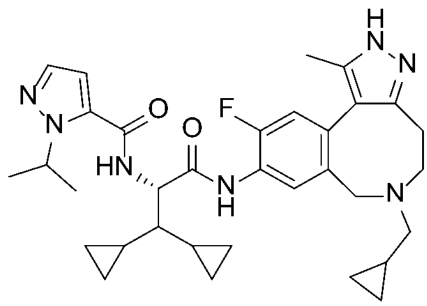

,

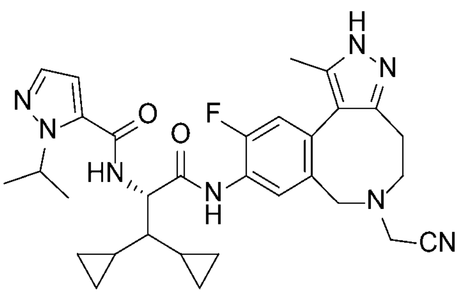

,

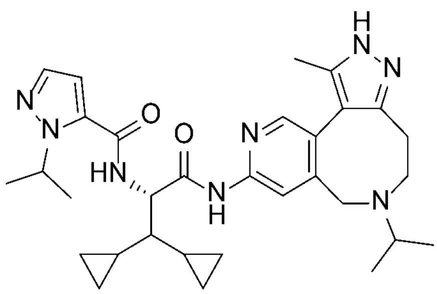

,

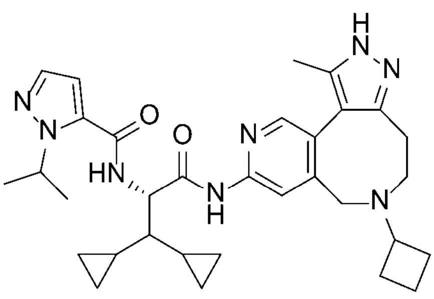

,

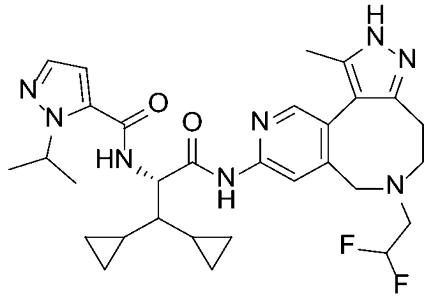

,

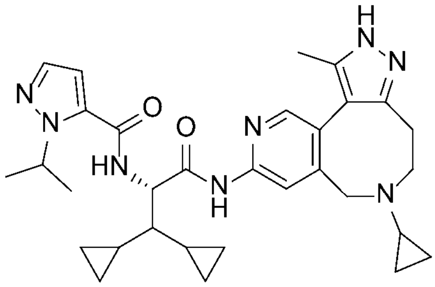,

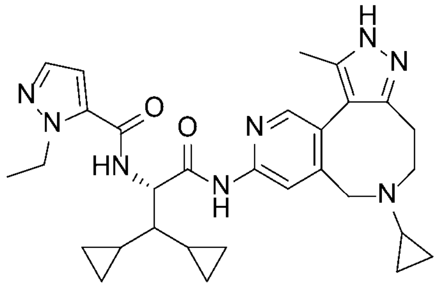,

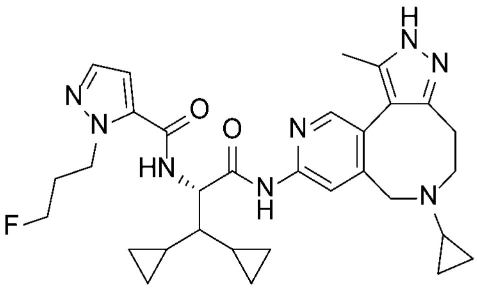,

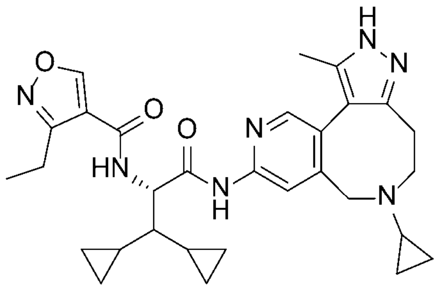,

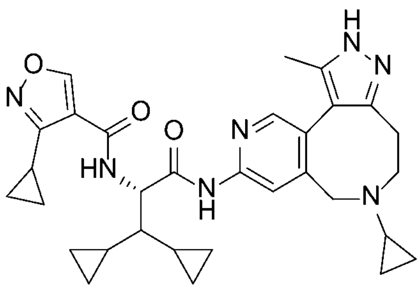,

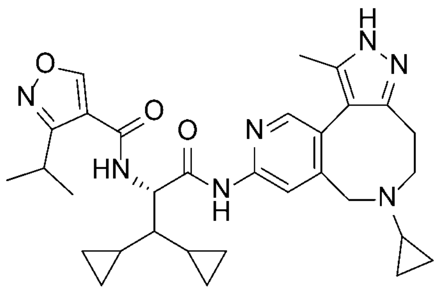,

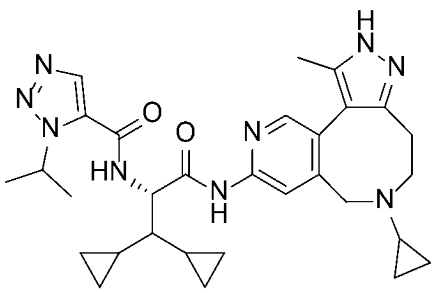,

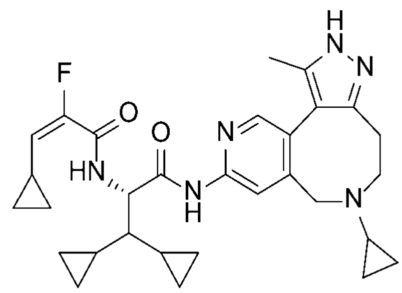,

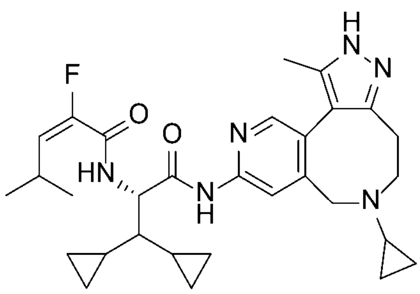,

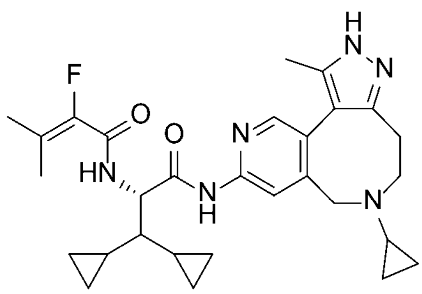,

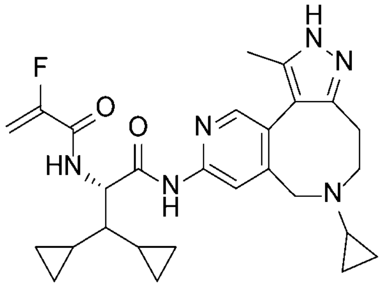
,

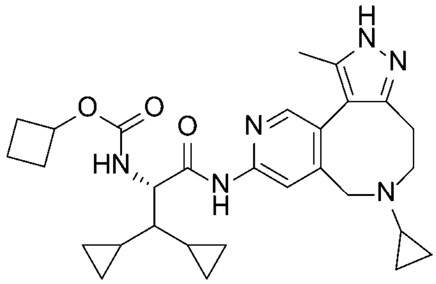
,

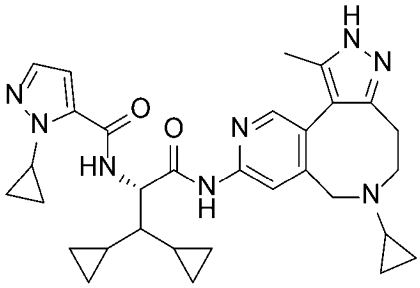
,

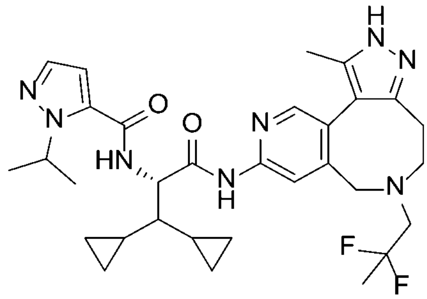
,

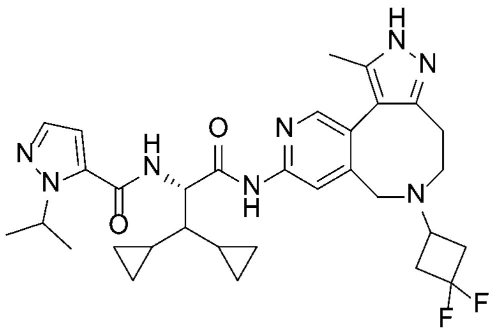
,

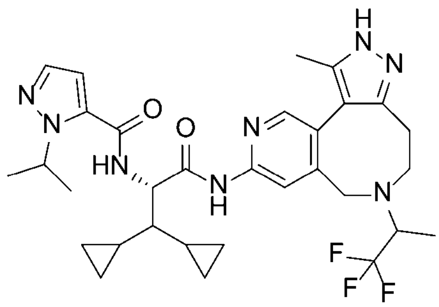
,

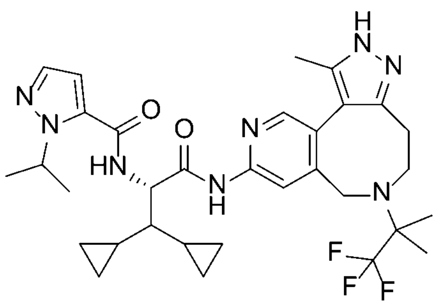
,

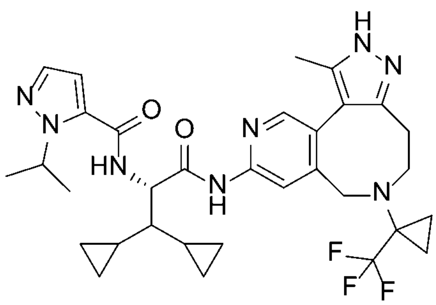
,

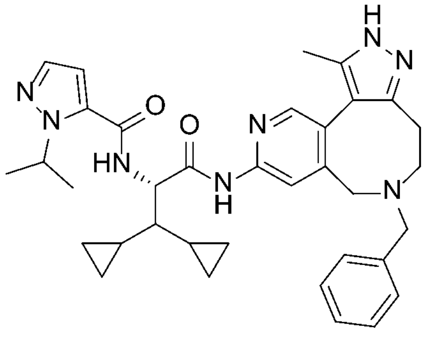
,

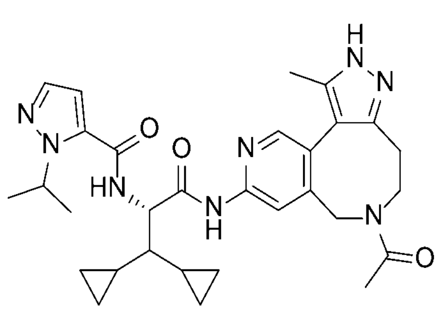
,

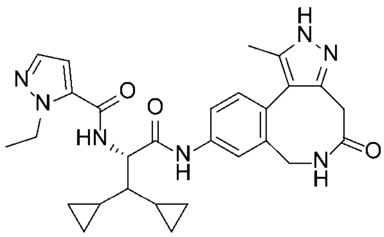,

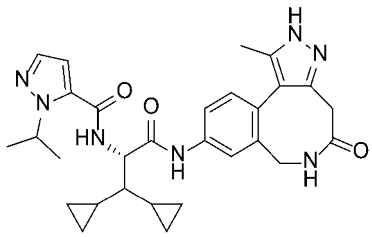,

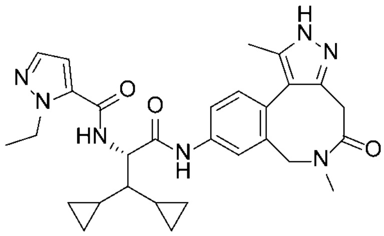,

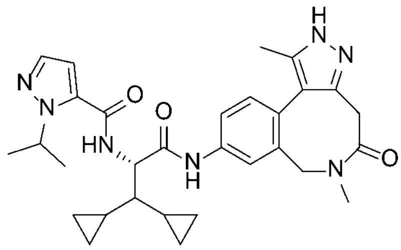,

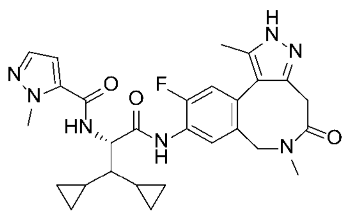,

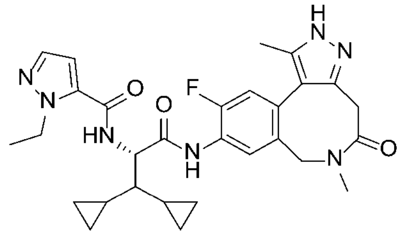,

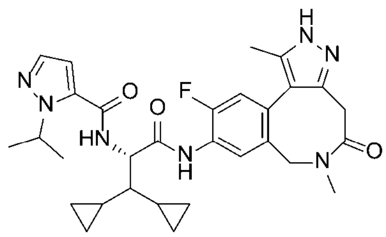,

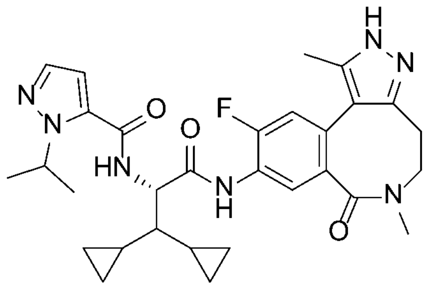,

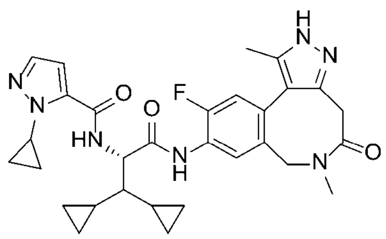,

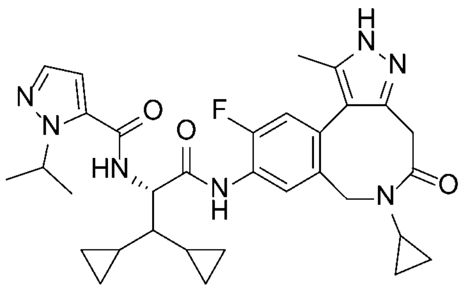,

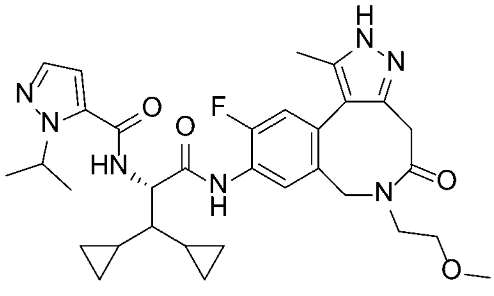,

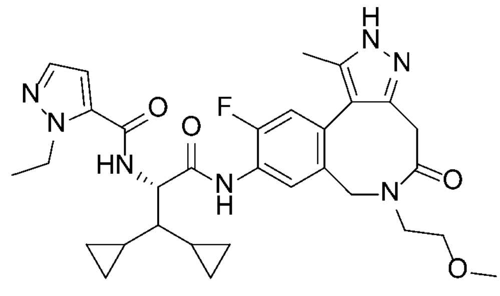,

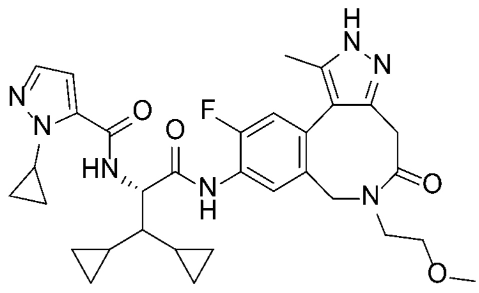,

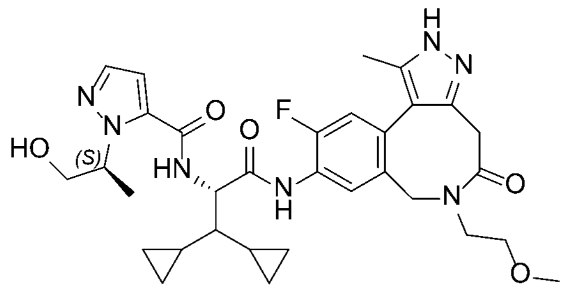,

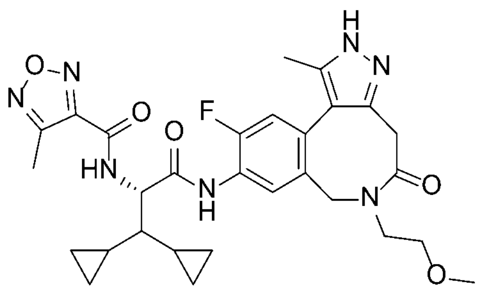,

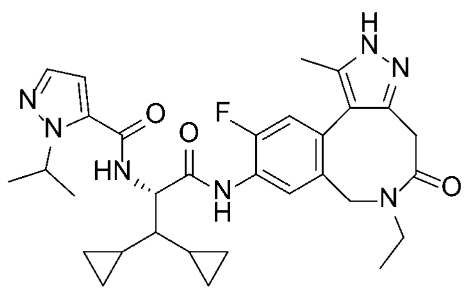,

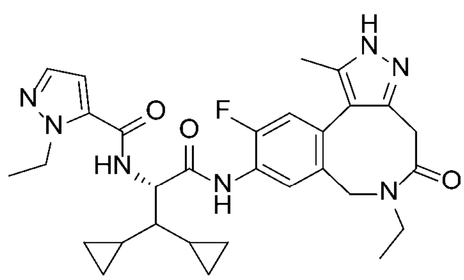,

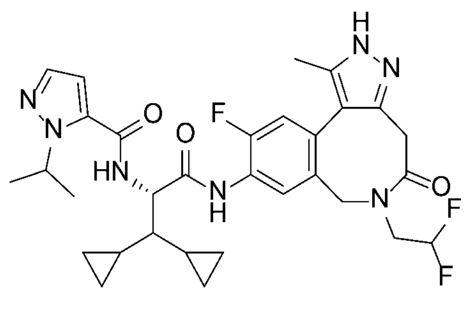,

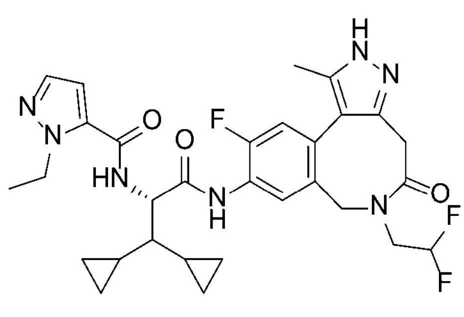,

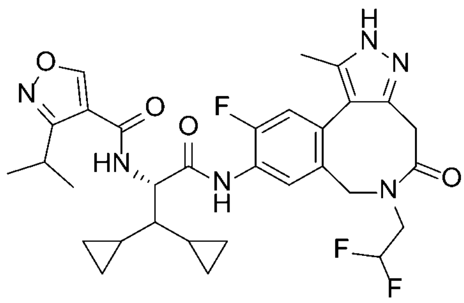,

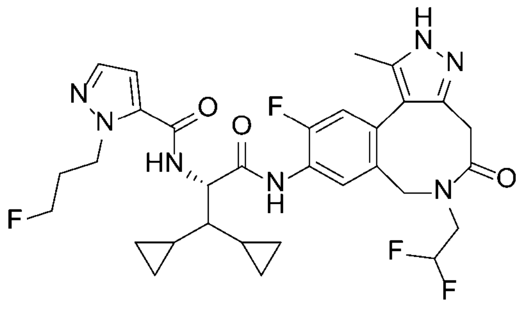
,

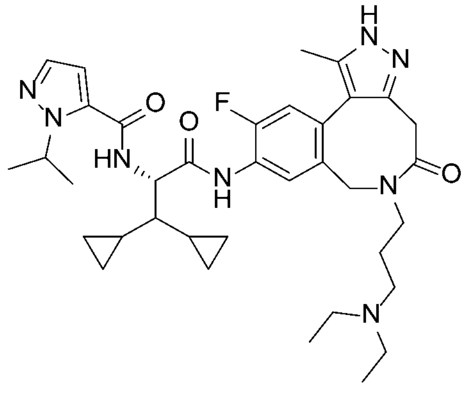
,

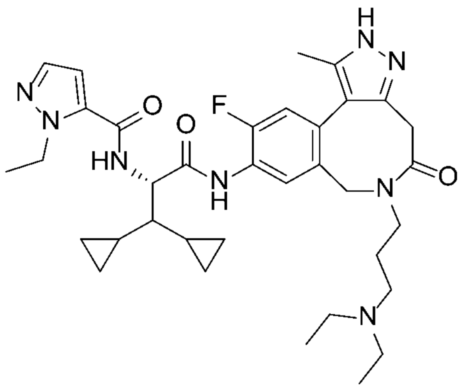
,

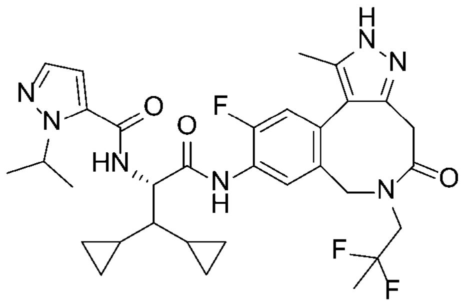
,

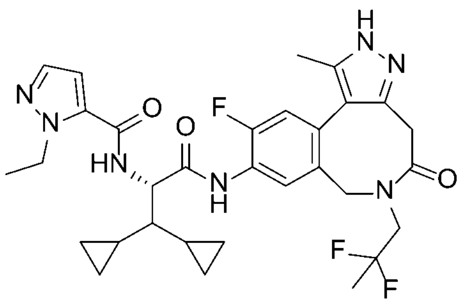
,

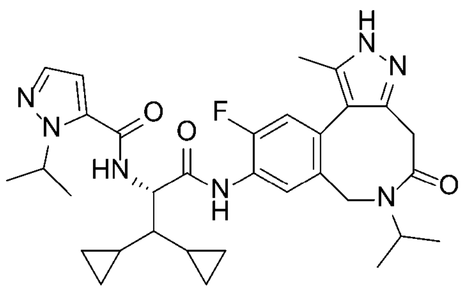
,

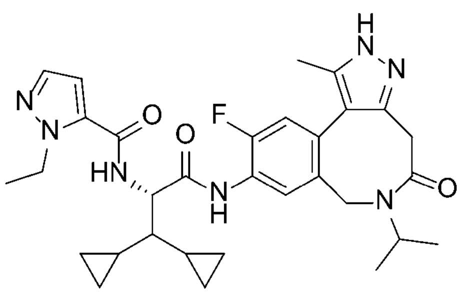
,

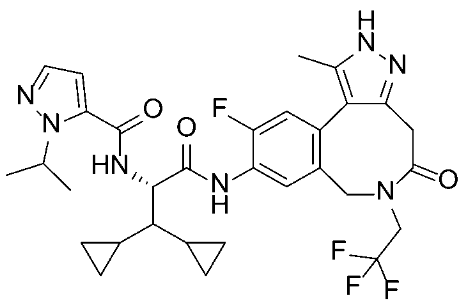
,

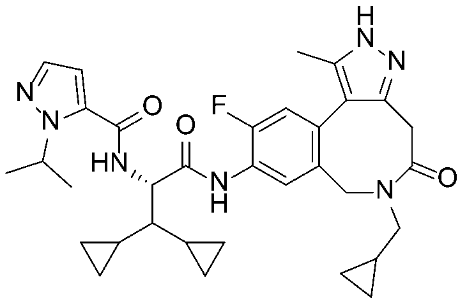
,

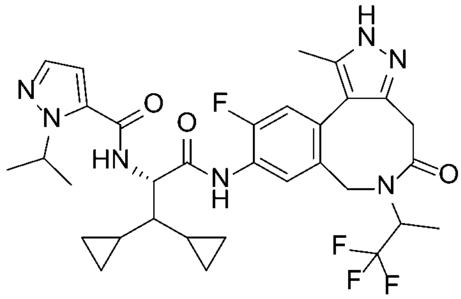
,

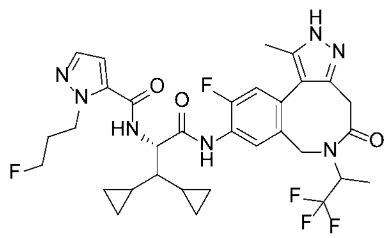
,

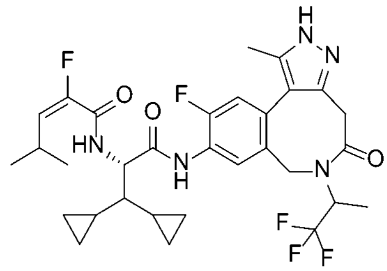
,

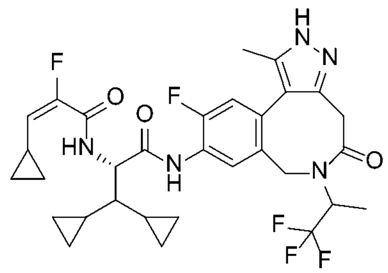
,

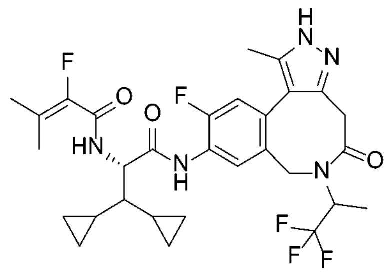
,

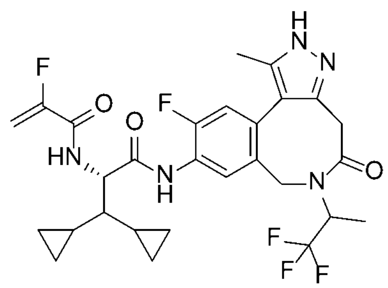
,

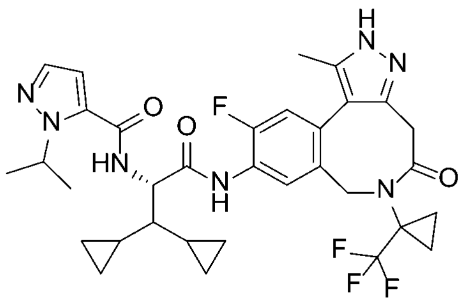
,

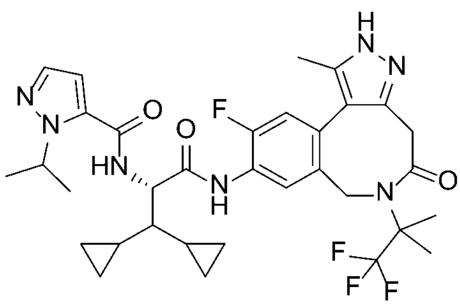
,

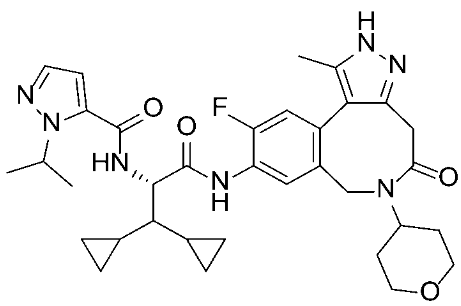
,

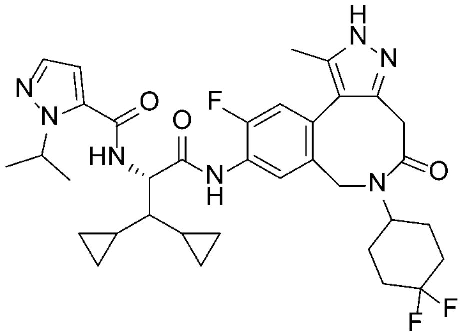
,
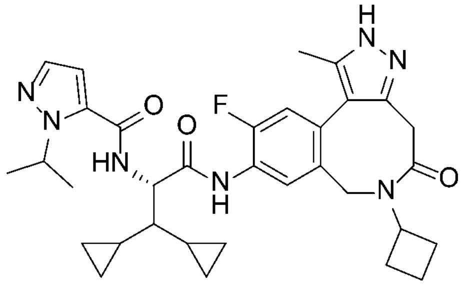
,
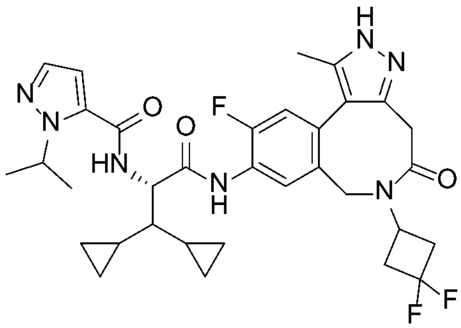
,
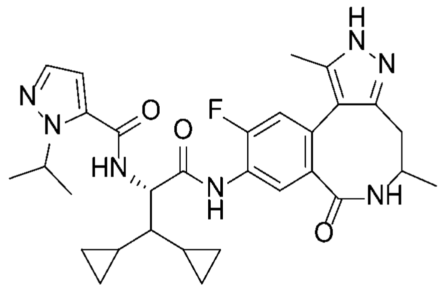
,
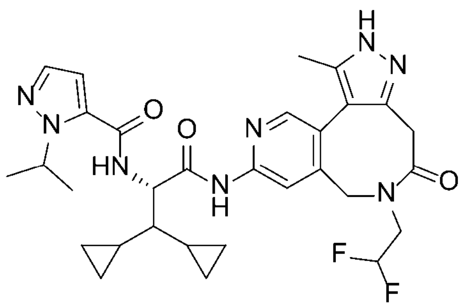
,
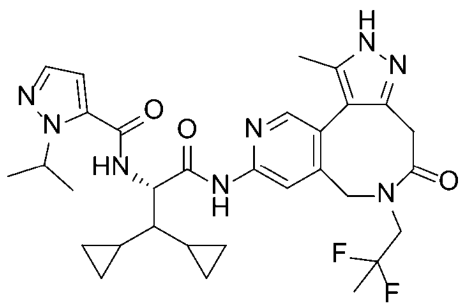
,
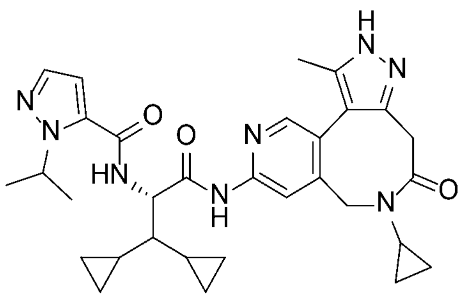
,
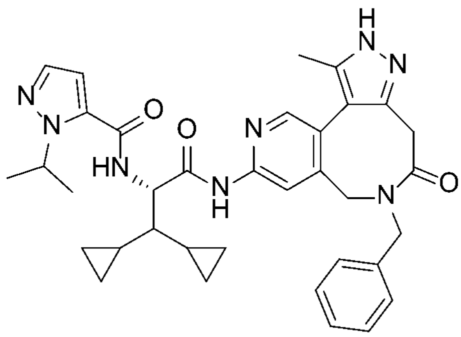
,

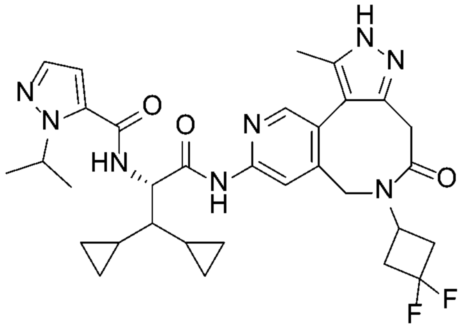,

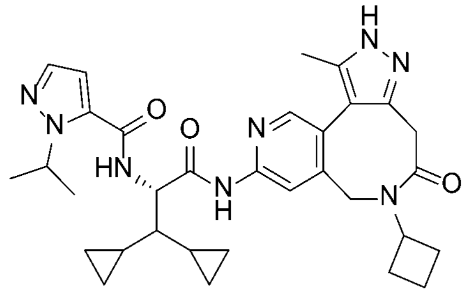,

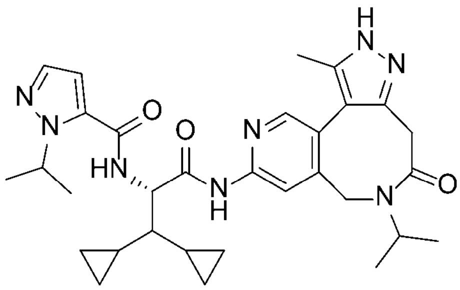,

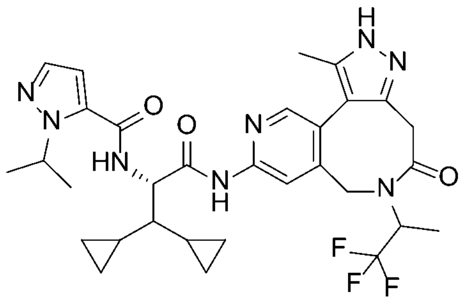,

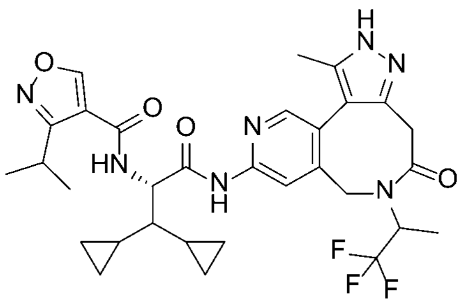,

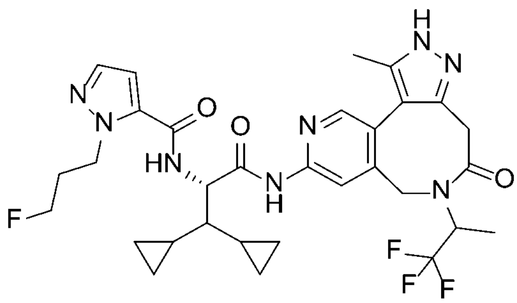,

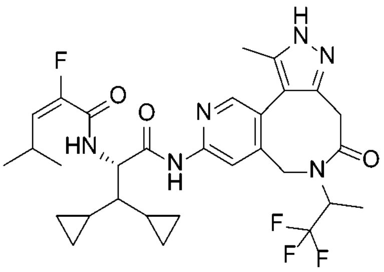,

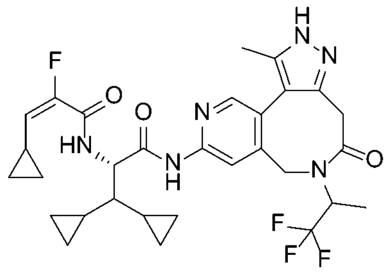,

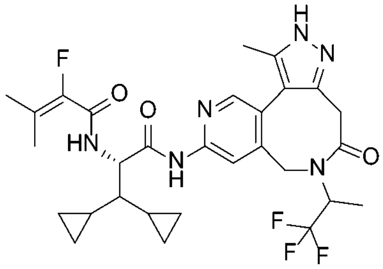,

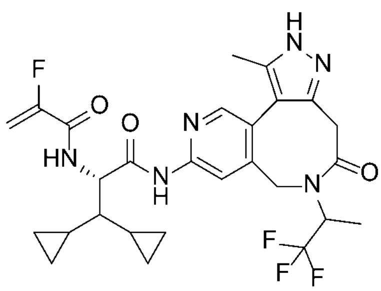,

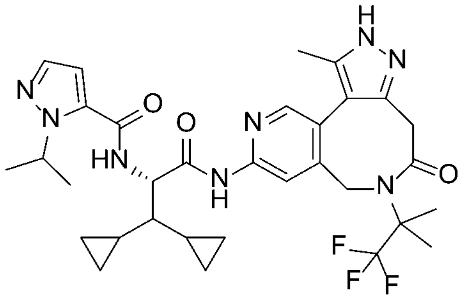,

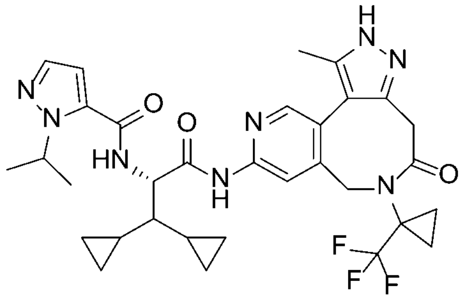,

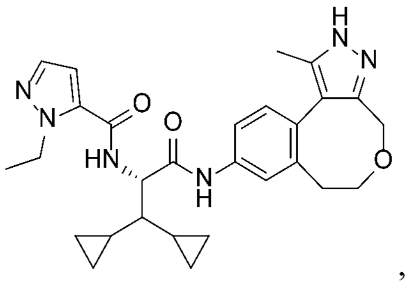,

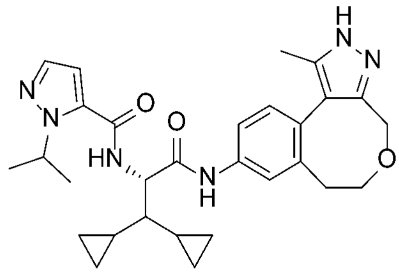,

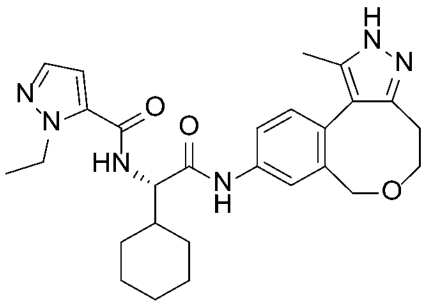,

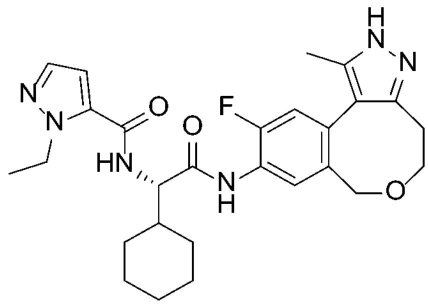,

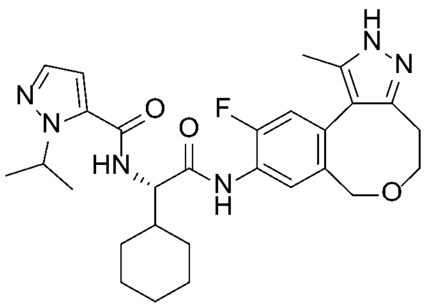,

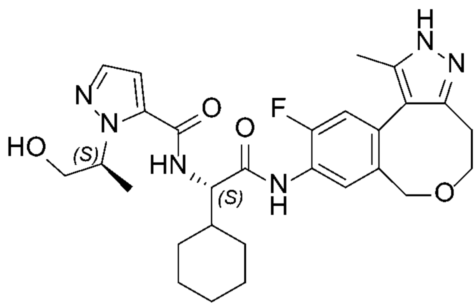,

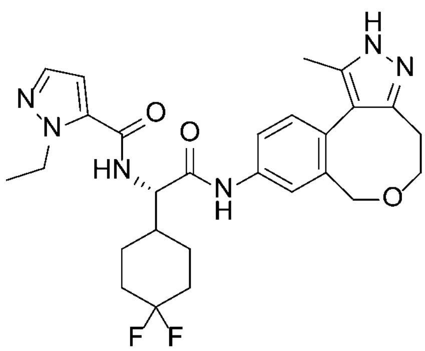,

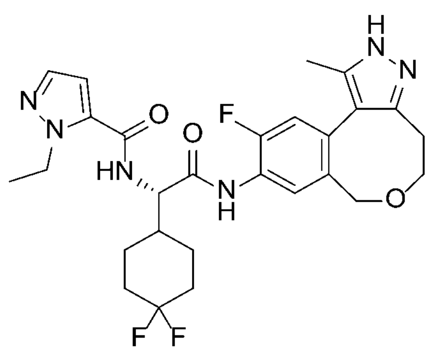,

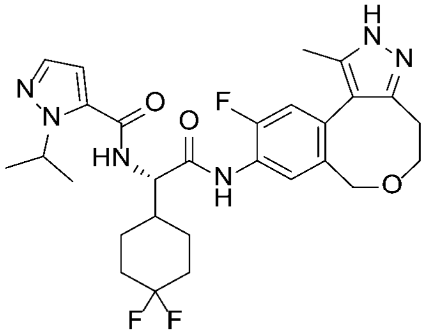

,

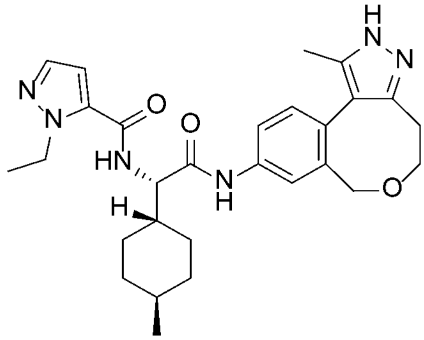

,

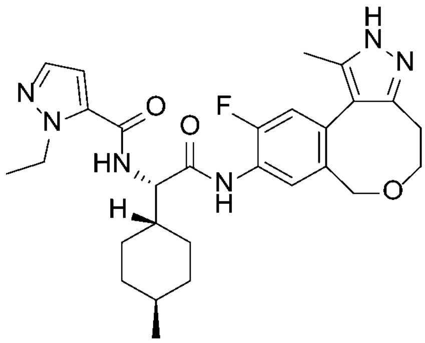

,

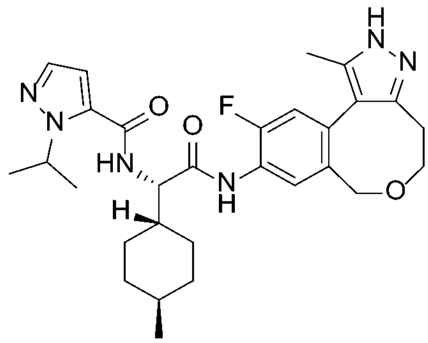

,

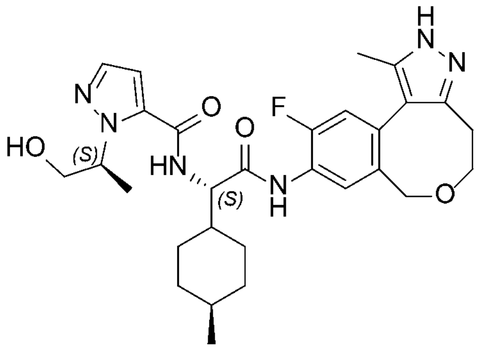

,

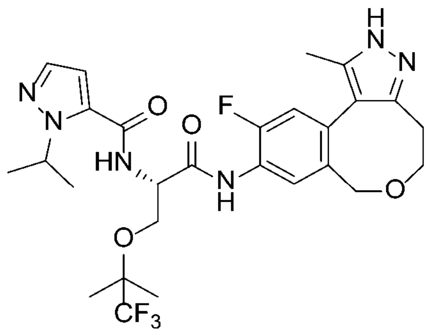

,

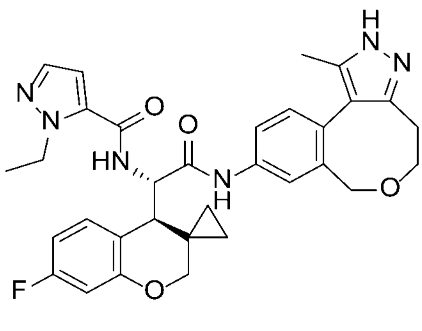

,

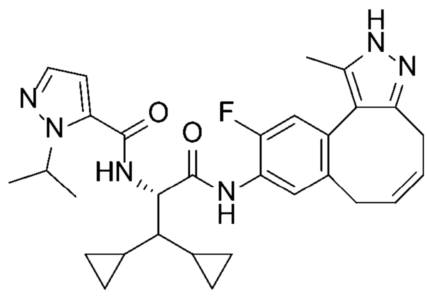

,

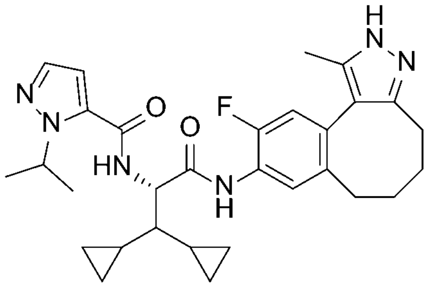,

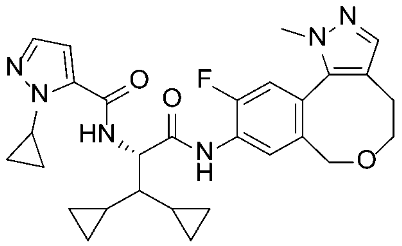

and

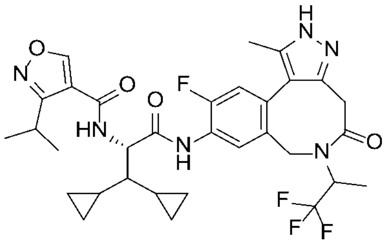.

**10.** The compound according to any one of claims 1-9, or a deuterated compound, a stereoisomer or a pharmaceutically acceptable salt thereof for use in the treatment of an interleukin-17A (IL-17A)-mediated disease.

**11.** The compound for use according to claim 10, **characterized in that** the IL-17A-mediated disease is selected from the group consisting of inflammation, autoimmune disease, infectious disease, cancer, precancerous syndrome and a combination thereof.

**12.** A pharmaceutical composition, comprising:

the compound according to any one of claims 1-9, or a deuterated compound, a stereoisomer or a pharmaceuticall acceptable salt thereof; and
a pharmaceutically acceptable excipient.

**13.** The compound according to any one of claims 1-9 for use in the treatment of psoriasis, rheumatoid arthritis, spondyloarthritis, multiple sclerosis, psoriatic arthritis, axial spondyloarthritis, ankylosing spondylitis, hidradenitis suppurativa, systemic lupus erythematosus, palmoplantar pustulosis, atopic dermatitis, asthma and/or chronic obstructive pulmonary disease.

**INTERNATIONAL SEARCH REPORT**

International application No.
**PCT/CN2024/125698**

**A. CLASSIFICATION OF SUBJECT MATTER**

C07D403/12(2006.01)i; C07D401/10(2006.01)i; C07D405/12(2006.01)i; C07D491/04(2006.01)i; C07D471/04(2006.01)i; A61K31/407(2006.01)i; A61P35/00(2006.01)i; A61P35/02(2006.01)i; A61K31/506(2006.01)i; A61K31/4025(2006.01)i

According to International Patent Classification (IPC) or to both national classification and IPC

**B. FIELDS SEARCHED**

Minimum documentation searched (classification system followed by classification symbols)

IPC:C07D403/-,C07D401/-,C07D405/-,C07D491/-,C07D471/-,A61K31/-,A61P35/-

Documentation searched other than minimum documentation to the extent that such documents are included in the fields searched

Electronic data base consulted during the international search (name of data base and, where practicable, search terms used)

DWPI, VEN, CNKI, CNTXT, STN(Reg, Caplus, Marpat): 多环, 稠环, 三环, 免疫, 炎症, 发炎, 癌症, 肿瘤, fused w ring, tricyclic, polycyclic, cancer, tumour, immune, inflammatory, 针对权利要求1的structural formula (I)进行了结构检索, structure search for structural formula (I) of claim 1

**C. DOCUMENTS CONSIDERED TO BE RELEVANT**

| Category* | Citation of document, with indication, where appropriate, of the relevant passages | Relevant to claim No. |
|---|---|---|
| A | WO 2008039520 A2 (SYNTA PHARMACEUTICALS CORP. et al.) 03 April 2008 (2008-04-03)<br>claims 1-339, and description, page 14, lines 16-35, and table 1 | 1-13 |
| A | CN 112789279 A (SHANGHAI HANSOH BIOMEDICAL CO., LTD.; JIANGSU HANSOH PHARMACEUTICAL GROUP CO., LTD.) 11 May 2021 (2021-05-11)<br>claims 1-26, and description, pages 21-28 | 1-13 |
| A | WO 2009123971 A1 (GENENTECH INC. et al.) 08 October 2009 (2009-10-08)<br>claims 1-50 | 1-13 |
| A | CN 105209448 A (SK CHEMICALS CO., LTD.) 30 December 2015 (2015-12-30)<br>claims 1-10 | 1-13 |
| A | CN 106349241 A (SHANGHAI HANSOH BIOMEDICAL CO., LTD.; JIANGSU HANSOH PHARMACEUTICAL GROUP CO., LTD.) 25 January 2017 (2017-01-25)<br>entire document | 1-13 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 December 2024** | **24 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/125698**

**C. DOCUMENTS CONSIDERED TO BE RELEVANT**

| Category* | Citation of document, with indication, where appropriate, of the relevant passages | Relevant to claim No. |
|---|---|---|
| A | CN 102333779 A (GENENTECH INC. et al.) 25 January 2012 (2012-01-25)<br>entire document | 1-13 |
| A | CN 115894425 A (JIANGSU HENGRUI MEDICINE CO., LTD.; SHANGHAI HENGRUI PHARMACEUTICAL CO., LTD.) 04 April 2023 (2023-04-04)<br>entire document | 1-13 |
| A | WO 2021115225 A1 (SHANGHAI HANSOH BIOMEDICAL CO., LTD. et al.) 17 June 2021 (2021-06-17)<br>entire document | 1-13 |
| A | WO 9809969 A1 (ASTRA PHARMACEUTICALS LTD. et al.) 12 March 1998 (1998-03-12)<br>entire document | 1-13 |

Form PCT/ISA/210 (second sheet) (July 2022)

**INTERNATIONAL SEARCH REPORT**
**Information on patent family members**

International application No.
**PCT/CN2024/125698**

| Patent document cited in search report | Publication date (day/month/year) | Patent family member(s) | Publication date (day/month/year) |
|---|---|---|---|
| WO 2008039520 A2 | 03 April 2008 | US 2008132513 A1 | 05 June 2008 |
| | | US 8779154 B2 | 15 July 2014 |
| | | CA 2664301 A1 | 03 April 2008 |
| | | AU 2007300450 A1 | 03 April 2008 |
| | | AU 2007300450 A2 | 04 June 2009 |
| | | AU 2007300450 B2 | 23 January 2014 |
| | | WO 2008039520 A3 | 13 November 2008 |
| | | EP 2086983 A2 | 12 August 2009 |
| | | EP 2086983 A4 | 21 December 2011 |
| | | JP 2010504913 A | 18 February 2010 |
| CN 112789279 A | 11 May 2021 | WO 2020020385 A2 | 03 April 2008 |
| WO 2009123971 A1 | 08 October 2009 | None | |
| CN 105209448 A | 30 December 2015 | KR 20140108179 A | 05 September 2014 |
| | | KR 101735787 B1 | 16 May 2017 |
| | | AU 2014221489 A1 | 08 October 2015 |
| | | AU 2014221489 B2 | 08 March 2018 |
| | | ES 2709195 T3 | 15 April 2019 |
| | | EP 2963027 A1 | 06 January 2016 |
| | | EP 2963027 A4 | 20 July 2016 |
| | | EP 2963027 B1 | 26 December 2018 |
| | | WO 2014133361 A1 | 04 September 2014 |
| | | CA 2900348 A1 | 04 September 2014 |
| | | CA 2900348 C | 22 June 2021 |
| | | JP 2016513135 A | 12 May 2016 |
| | | JP 6387023 B2 | 05 September 2018 |
| | | US 2016009677 A1 | 14 January 2016 |
| | | US 9643946 B2 | 09 May 2017 |
| CN 106349241 A | 25 January 2017 | None | |
| CN 102333779 A | 25 January 2012 | AU 2009231885 A1 | 08 October 2009 |
| | | AU 2009231885 B2 | 03 April 2014 |
| | | WO 2009123971 A1 | 08 October 2009 |
| | | JP 2011517457 A | 09 June 2011 |
| | | JP 5511786 B2 | 04 June 2014 |
| | | KR 20100137551 A | 30 December 2010 |
| | | KR 101626996 B1 | 02 June 2016 |
| | | AR 071112 A1 | 26 May 2010 |
| | | US 2014336154 A1 | 13 November 2014 |
| | | US 9309265 B2 | 12 April 2016 |
| | | IL 208359 A0 | 30 December 2010 |
| | | IL 208359 A | 31 July 2016 |
| | | TW 200944531 A | 01 November 2009 |
| | | TWI 443102 B | 01 July 2014 |
| | | EP 2276767 A1 | 26 January 2011 |
| | | EP 2276767 B1 | 07 May 2014 |
| | | US 2009247567 A1 | 01 October 2009 |
| | | US 7928248 B2 | 19 April 2011 |
| | | CA 2719032 A1 | 08 October 2009 |
| | | CA 2719032 C | 08 November 2016 |
| | | ZA 201007218 B | 27 June 2012 |

Form PCT/ISA/210 (patent family annex) (July 2022)

**INTERNATIONAL SEARCH REPORT**
**Information on patent family members**

International application No.
**PCT/CN2024/125698**

| Patent document cited in search report | Publication date (day/month/year) | Patent family member(s) | Publication date (day/month/year) |
|---|---|---|---|
| | | MX 2010010659 A | 26 October 2010 |
| | | CL 2009000780 A1 | 15 January 2010 |
| | | ES 2480994 T3 | 29 July 2014 |
| | | HK 1147741 A1 | 19 August 2011 |
| | | BRPI 0910346 A2 | 06 October 2015 |
| | | US 2013123263 A1 | 16 May 2013 |
| | | US 8846762 B2 | 30 September 2014 |
| | | RU 2010143319 A | 10 May 2012 |
| | | RU 2506267 C2 | 10 February 2014 |
| | | PL 2276767 T3 | 30 September 2014 |
| | | PE 20091720 A1 | 26 November 2009 |
| | | DK 2276767 T3 | 14 July 2014 |
| | | US 2011130363 A1 | 02 June 2011 |
| | | US 8399690 B2 | 19 March 2013 |
| | | SI 2276767 T1 | 29 August 2014 |
| CN 115894425 A | 04 April 2023 | None | |
| WO 2021115225 A1 | 17 June 2021 | CA 3160875 A1 | 17 June 2021 |
| | | KR 20220113385 A | 12 August 2022 |
| | | US 2023118497 A1 | 20 April 2023 |
| | | TW 202128693 A | 01 August 2021 |
| | | AU 2020401668 A1 | 21 July 2022 |
| | | JP 2023506436 A | 16 February 2023 |
| | | EP 4074714 A1 | 19 October 2022 |
| | | EP 4074714 A4 | 27 December 2023 |
| WO 9809969 A1 | 12 March 1998 | AR 009322 A1 | 12 April 2000 |
| | | CA 2263586 A1 | 12 March 1998 |
| | | ID 17393 A | 24 December 1997 |
| | | JP 2001501180 A | 30 January 2001 |
| | | AU 3873697 A | 26 March 1998 |
| | | AU 711539 B2 | 14 October 1999 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 202011731 A1
- WO 2020146194 A1


### Non-patent literature cited in the description


- **WRIGHT et al.** *Journal of immunology*, 2008, vol. 181, 2799-2805
- **AL-RAMLI et al.** *J Allergy Clin Immunol*, 2009, vol. 123, 1185-1187
- **GAFFEN, SL et al.** *Arthritis Research & Therapy*, 2004, vol. 6, 240-247
- **KOMIYAMA Y et al.** *J. Immunol.*, 2006, vol. 177, 566-573